# EUROPEAN PATENT APPLICATION

(11) **EP 4 194 457 A1**
(43) Date of publication of application: **14.06.2023**
(21) Application number: 21852332.2
(22) Date of filing: 05.08.2021
(51) Int. Cl.: C07D 471/10

(54) **COMPOUND FOR TARGETING AND DEGRADING PROTEIN, AND PREPARATION METHOD THEREFOR AND USE THEREOF**

(30) Priority: 05.08.2020 CN 202010785541; 18.12.2020 CN 202011513669
(71) Applicant: Shanghai Leadingtac Pharmaceutical Co., Ltd., Shanghai 201203 (CN)
(72) Inventor: FENG, Yan, Shanghai 201203 (CN); LI, Shiqiang, Shanghai 201203 (CN)
(74) Representative: Cabinet Laurent & Charras
(86) International application number: PCT/CN2021/110990
(87) International publication number: WO 2022/028547

(57) **Abstract**

Provided in the present invention are a class of bifunctional compounds for targeting and degrading an IRAK4 kinase protein, a pharmaceutical composition and a preparation method therefor. The compound of the present invention can not only effectively inhibit and/or degrade the IRAK4 kinase protein in cells, but also effectively inhibit the production of IL-6 by immune cells, and has good degradation selectivity. The compound can be used in the preparation of a drug for treating and/or preventing IRAK4-mediated related diseases or conditions, such as cancer, immunological diseases and inflammatory diseases.

## Description

Field of the invention: The present invention belongs to the field of pharmaceuticals, and in particular, the present invention relates to compounds for targeting and degrading IRAK4 proteins, pharmaceutical compositions and methods therefor and their use in the preparation of medicaments for the treatment and/or prevention of related diseases or conditions mediated by IRAK4, such as cancer, immune diseases and inflammatory diseases.

Background: Interleukin-1 receptor kinase 4(IRAK4) is a serine/threonine-specific protein kinase with biologically important kinase activity and plays an important role in activating the immune system. Studies have shown that IRAK4 is a key factor downstream of IL-1β family receptors (including IL-1R, IL-18R, IL-33R, IL-36R) and Toll-like receptor (TLR) signaling pathways. Both IRAK4-deficient mice and IRAK4-deficient patients do not respond to TLR (except TLR3) and IL-1β family stimulation.

According to the presence or absence of MyD88, TLR/IL-1α and IL-1β mediated signaling pathways can be divided into MyD88-dependent signaling pathways and MyD88-independent pathways, in which IL-1R and TLR2, TLR4, TLR7/8, TLR9 mediated signal transduction pathways rely on MyD88 as a regulator to activate downstream inflammatory signaling pathways. After TLR/IL-1β binds to the ligand, MyD88 molecules are recruited, then MyD88 further recruits IRAK4 into TLR/IL-1β complex through its N-terminal death domain, and interacts with IRAK1 or IRAK2 and activates it, thus transmitting signals to E3 ubiquitin ligase TNF receptor related factor (TRAF6) downstream, activating serine/threonine kinase TAK1, and then activating NF-κB and MAPK signal pathways, thus causing the release of a variety of inflammatory cytokines and anti-apoptotic molecules. The IRAK4-dependent TLR/IL-1β signaling pathway has been shown to be associated with a variety of diseases: such as multiple sclerosis, atherosclerosis, myocardial infarction, myocarditis, Vogt-Koyanagi-Harada syndrome, systemic lupus erythematosus (SLE), obesity, type 1 diabetes, rheumatoid arthritis, spondyloarthritis (especially psoriatic spondyloarthritis and Bekhterev's disease), lupus erythematosus, psoriasis, vitiligo, giant cell arteritis, chronic inflammatory intestinal diseases and viral diseases, for example, HIV (human immunodeficiency virus), hepatitis virus; Skin diseases such as psoriasis, atopic dermatitis, Kindler's syndrome, bullous pemphigoid, allergic contact dermatitis, alopecia areata, acneinversa and acne vulgaris; other inflammatory diseases such as allergy, Bechet's disease, gout, adult-onset Still's disease, pericarditis and chronic inflammatory intestinal diseases such as ulcerative colitis and Crohn's disease, transplant rejection and graft-versushost reactions; Gynecological diseases such as adenomyosis, dysmenorrhea, dyspareunia and endometriosis, especially pain related to endometriosis and other symptoms related to endometriosis such as dysmenorrhea, dyspareunia, dysuria and difficulty defecating; eye diseases such as retinal ischemia, keratitis, allergic conjunctivitis, keratoconjunctivitis sicca, macular degeneration and ocular pigment layer inflammation; fibrotic diseases such as liver fibrosis, myocarditis, primary biliary cirrhosis, cystic fibrosis; chronic liver diseases such as fatty liver hepatitis, especially non-alcoholic fatty liver disease (NAFLD) and/or non-alcoholic steatohepatitis (NASH), alcoholic steatohepatitis (ASH); cardiovascular diseases and neurological disorders, such as myocardial reperfusion injury, myocardial infarction, hypertension, and Alzheimer's disease, stroke, craniocerebral trauma, amyotrophic lateral sclerosis (ALS) and Parkinson's disease; pruritus and pain (including acute, chronic, inflammatory and neuropathic pain) such as hyperalgesia, allodynia, premenstrual pain, pain related to endometriosis, postoperative pain, interstitial cystitis, CRPS (complex local pain syndrome), trigeminal neuralgia, prostatitis, pain caused by spinal cord injury, pain caused by inflammation, low back pain, cancer pain, chemotherapy-related pain, HIV treatmentinduced neuropathy, burns pain and chronic pain; tumor diseases such as certain lymphomas: ABC-DLBCL (activated B- cell diffuse large cell B- cell lymphoma), mantle cell lymphoma and Waldanstrom disease, as well as chronic lymphocytic leukemia, melanoma, pancreatic tumors and hepatocellular carcinoma, ras-dependent tumors, breast cancer, ovarian cancer, colorectal cancer, head and neck cancer, lung cancer, and prostate cancer.

The regulation of IRAK4-mediated signaling pathway is mainly related to its kinase function. However, there are also some reports indicating in some cell types, the signal regulation of downstream processes by IRAK4 is related to the non-kinase function of IRAK4. Cushing et al. indicated that although the phosphorylation level of IRAK4 was reduced in human skin fibroblasts stimulated by IL-1β, the pharmacological inhibition of IRAK4 does not lead to the inhibition of IL-6 and TNF-α. In support of these results, the scaffold function of IRAK4 is important for IL1 signaling in IRAK4-deficient fibroblasts compared with wild-type cells, but its kinase effect is redundant. At the same time, Chiang and his colleagues also said that IRAK4 kinase activity was not necessary in human B cells, T cells, dendritic cells and monocytes, and siRNA gene excision also showed that IRAK4 had a scaffold function in these cells. A variety of potent selective inhibitors against IRAK4 have been reported, such as CA-4948, BAY-1834845, BMS-986126 and PF-06650833. These inhibitors can selectively inhibit the kinase activity of IRAK4 and are mainly used for the prevention and treatment of autoimmune diseases, inflammatory diseases and tumor diseases. However, on the one hand, IRAK4 has the function of scaffold protein and active kinase, and on the other hand, traditional small molecule inhibitors are prone to drug resistance, therefore, only inhibition of IRAK4 kinase activity may not be sufficient to produce therapeutic effect.

Proteolysis Targeting Chimera(PROTAC) is a technology different from traditional small molecule inhibitors. Traditional small molecule inhibitors usually need to act on the active site of the target protein to inhibit its activity. PROTAC is a heterogeneous bifunctional molecule, one end of which is a small molecule inhibitor that can recognize the target protein. Through the connection chain, the other end is an E3 ubiquitin ligase ligand that can recognize E3 ubiquitin ligase, this bifunctional molecule recognizes the target protein and E3 ubiquitin ligase in the body, and draws the target protein and E3 ubiquitin ligase closer to form a ternary complex. After the target protein is ubiquitinated, the target protein is degraded through the ubiquitin-proteasome pathway in the body. Compared with traditional small molecule inhibitors, on the one hand, PROTAC only needs to bring the target protein closer to E3 ubiquitin ligase to degrade the substrate, and this mode of action makes this technology applicable to some non-druggable targets; on the other hand, after the target protein is degraded, the PROTAC molecules can be released to continue to participate in the degradation process of the next protein, so this degradation has a catalytic effect, so that less dose of PROTAC drug can achieve efficient degradation; on the other hand, traditional small molecule inhibitors are often prone to drug resistance because of point mutations, which makes small molecule inhibitors lose the inhibitory effect on the target. PROTAC can directly degrade the target protein, which can avoid the drug resistance caused by point mutations to a certain extent. Therefore, compared with traditional small molecule inhibitors, the use of PROTAC technology for new drug small molecule research and development has high advantages and feasibility, and is expected to become the next generation of promising new drugs. PROTAC technology has also been applied to the modification of various target drugs, such as androgen receptor, estrogen protein receptor, BTK, etc. Several types of degradation agents targeting IRAK4 are disclosed in US2019/0151295, US2019/0192688, WO2019/160915 and WO2020/113233, and more degradation agents targeting IRAK4 are to be developed.

### SUMMARY OF THE INVENTION

The invention provides a compound of formula I, and/or a stereoisomer, an enantiomer, a diastereomer, a deuterate, a hydrate, a solvate, a prodrug and/or a pharmaceutically acceptable salt thereof:

PTM-L-ULM I

wherein:
PTM is a small molecule compound that can inhibit IRAK4 kinase protein or bind to IRAK4 kinase protein;
L is a connecting chain, which connects PTM and ULM through a covalent bond;
ULM is a small molecule ligand in E3 ubiquitin ligase complex, and the ULM has the following structure: 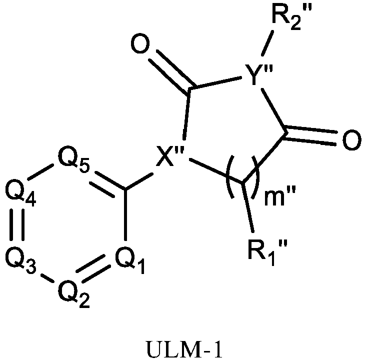
wherein, in ULM-1:
   X" is CH or N;
   Y" is CH, N, O or S;
   Q₁, Q₂, Q₃, Q₄ and Q₅ are each independently CR₃" or N;
   R₃" are each independently hydrogen, deuterium, hydroxyl, amino, cyano, halogen, C1-C6 alkyl, C3-C8 cycloalkyl, 3-8-membered heterocycloalkyl, 6-10-membered aryl, 5-10-membered heteroaryl, -O(C1-C6 alkyl), -O-(C3-C8 cycloalkyl), -O-(3-8-membered heterocycloalkyl), N(C1-C6 alkyl)₂, NH(C3-C8 cycloalkyl), NH(3-8-membered heterocycloalkyl), -O-(6-10-membered aryl), or -O-(5-10-membered heteroaryl); and the alkyl, cycloalkyl, heterocycloalkyl, aryl and heteroaryl are optionally substituted by 1-3 groups independently selected from hydroxyl, halogen, cyano, or amino; or R₃" together with its attached atoms to form a cycloalkyl, heterocycloalkyl, heteroaryl or aryl;
   m" is 1, 2 or 3;
   R₁" are each independently hydrogen, deuterium, hydroxyl, amino, cyano, halogen, C1-C6 alkyl, C3-C8 cycloalkyl, 3-8-membered heterocycloalkyl, 6-10-membered aryl, 5-10-membered heteroaryl, or -O(C1-C6 alkyl); and the alkyl, cycloalkyl, heterocycloalkyl, aryl and heteroaryl are optionally substituted by 1-3 groups independently selected from hydroxyl, halogen, cyano, or amino;
   R₂" is absent, hydrogen, deuterium, C1-C6 alkyl, or C3-C6 cycloalkyl, the C1-C6 alkyl and C3-C6 cycloalkyl are optionally substituted by 1-3 groups independently selected from hydroxyl, halogen, -O-(C=O)-(C1-C6 alkyl), cyano or amino.

In some embodiments of the present invention, in ULM-1,
X" is CH or N;
Y" is CH, N, O or S;
Q₁, Q₂, Q₃, Q₄ and Q₅ are each independently CR₃" or N;
R₃" are each independently hydrogen, deuterium, hydroxyl, amino, cyano, halogen, C1-C6 alkyl, C3-C8 cycloalkyl, 3-8-membered heterocycloalkyl, 6-10-membered aryl, 5-10-membered heteroaryl, -O(C1-C6 alkyl), -O-(C3-C8 cycloalkyl), -O-(3-8-membered heterocycloalkyl), N(C1-C6 alkyl)₁₋₂, NH(C3-C8 cycloalkyl), NH (3-8-membered heterocycloalkyl), -O-(6-10 membered aryl), -O-(5-10 membered heteroaryl); the alkyl, cycloalkyl, heterocycloalkyl, aryl and heteroaryl are optionally substituted by 1-3 groups independently selected from hydroxyl, halogen or amino;
m" is 1, 2 or 3;
R₁" is each independently hydrogen, deuterium, hydroxyl, amino, cyano, halogen, C1-C6 alkyl, C3-C8 cycloalkyl, 3-8-membered heterocycloalkyl, 6-10-membered aryl, 5-10-membered heteroaryl, or -O(C1-C6 alkyl); the alkyl, cycloalkyl, heterocycloalkyl, aryl, and heteroaryl are optionally substituted by 1-3 groups independently selected from hydroxyl, halogen, or amino; and
R₂" is hydrogen, deuterium, C1-C6 alkyl or C3-C6 cycloalkyl, the C1-C6 alkyl and C3-C6 cycloalkyl are optionally substituted by 1-3 groups independently selected from hydroxyl, halogen, or amino.

In some embodiments of the present invention, one or two of Q₁, Q₂, Q₃, Q₄ and Q₅ in ULM-1 are N, and the rest are each independently CR₃".

In some embodiments of the present invention, Q₁, Q₂, Q₃, Q₄ and Q₅ in ULM-1 are each independently CR₃".

In some embodiments of the present invention, X" in ULM-1 is N.

In some embodiments of the present invention, X" in ULM-1 is CH.

In some embodiments of the present invention, Y" in ULM-1 is N.

In some embodiments of the present invention, R₁" in ULM-1 is each independently hydrogen, deuterium, -F, -Cl, or C1-C6 alkyl, the alkyl is optionally substituted by 1-3 halogens; preferably R₁" is hydrogen.

In some embodiments of the present invention, R₂" in ULM-1 is hydrogen or C1-C6 alkyl, the alkyl is optionally substituted by 1-3 halogens; preferably R₂" is hydrogen.

In some embodiments of the present invention, R₃"in ULM-1 is each independently hydrogen, deuterium, halogen, -O(C1-C6 alkyl), or C1-C6 alkyl, the alkyl is optionally substituted by 1-3 halogens; preferably R₃" is each independently hydrogen, deuterium, F, Cl, methyl, methoxy, ethoxy, trifluoromethoxy, 2-hydroxypropyl-2-yl or trifluoromethyl.

In some embodiments of the present invention, the ULM-1 is connected to L via Q₁, Q₂, Q₃, Q₄ or Q₅.

In some embodiments of the present invention, the ULM has the following structure: 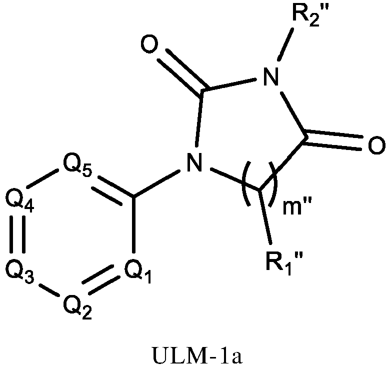 wherein, Q₁, Q₂, Q₃, Q₄, Q₅, R₁", R₂"and m" are as defined in the above ULM-1.

In some embodiments of the present invention, the ULM has the following structure: 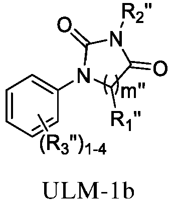 wherein, R₁", R₂", R₃", and m" are as defined in the above ULM-1.

In some embodiments of the present invention, the ULM has the following structure: 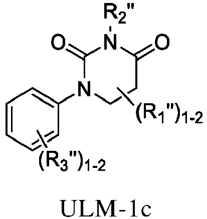 wherein, R₁", R₂" and R₃" are as defined in the above ULM-1.

In some embodiments of the present invention, the ULM has the following structure: 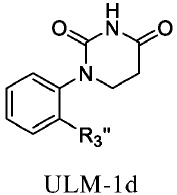 wherein, R₃" is as defined in the above ULM-1.

In some embodiments of the present invention, the ULM has the following structure: 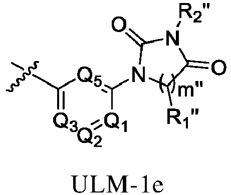 wherein, Q₁, Q₂, Q₃, Q₅, R₁", R₂" and m" are as defined in the above ULM-1.

In some embodiments of the present invention, the ULM has the following structure: 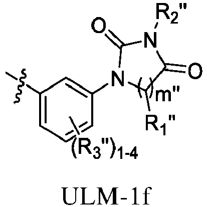 wherein, R₁", R₂", R₃", and m" are as defined in the above ULM-1.

In some embodiments of the present invention, the ULM has the following structure: 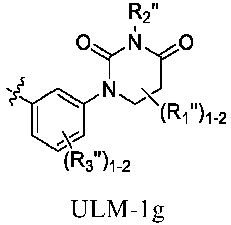 wherein, R₁", R₂", R₃" are as defined in the above ULM-1. 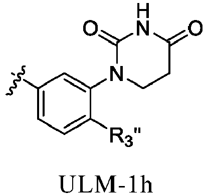 wherein, R₃" is as defined in the above ULM-1.

In some embodiments of the present invention, the ULM is selected from 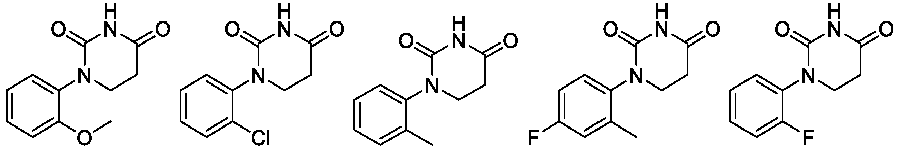 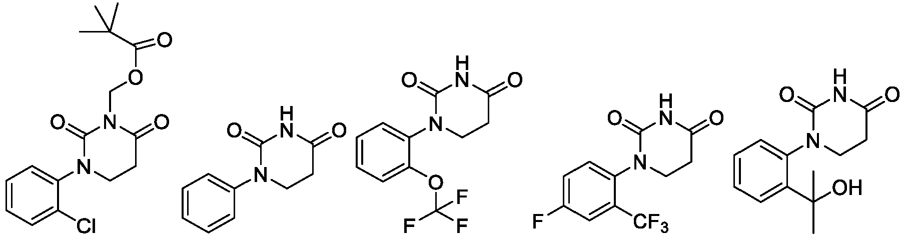 or

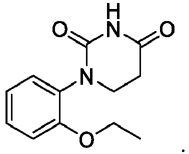

In some embodiments of the present invention, the ULM is selected from

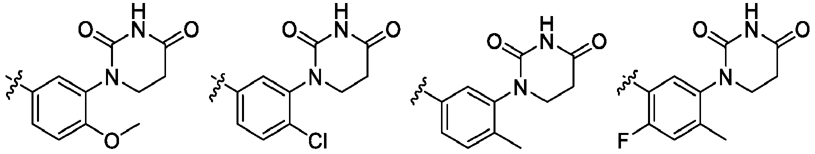

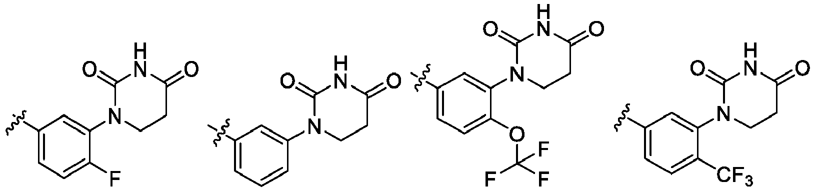

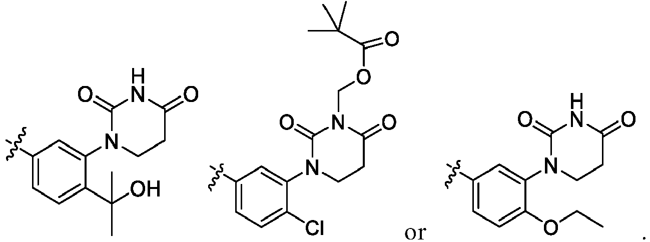

In some embodiments of the present invention, the ULM has the following structure: 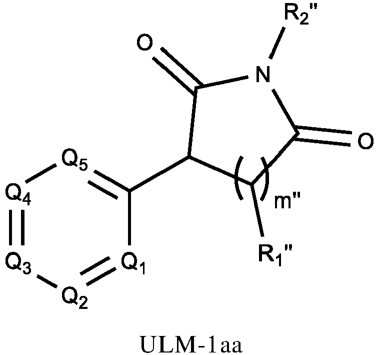 wherein, Q₁, Q₂, Q₃, Q₄, Qs, R₁", R₂"and m" are as defined in the above ULM-1.

In some embodiments of the present invention, the ULM has the following structure: 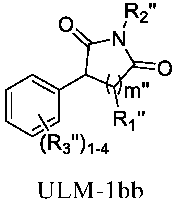 wherein, R₁", R₂", R₃", and m" are as defined in the above ULM-1.

In some embodiments of the present invention, the ULM has the following structure: 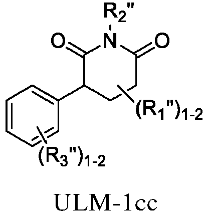 wherein, R₁", R₂" and R₃" are as defined in the above ULM-1.

In some embodiments of the present invention, the ULM has the following structure: 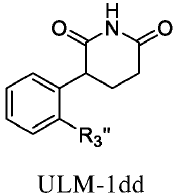 wherein, R₃" is as defined in the above ULM-1.

In some embodiments of the present invention, the ULM has the following structure: 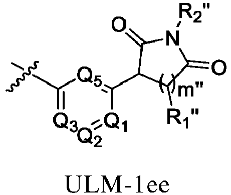 wherein, Q₁, Q₂, Q₃, Q₅, R₁", R₂" and m" are as defined in the above ULM-1.

In some embodiments of the present invention, the ULM has the following structure: 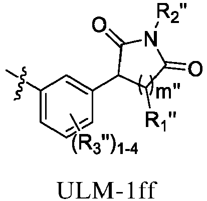 wherein, R₁", R₂", R₃", and m" are as defined in the above ULM-1.

In some embodiments of the present invention, the ULM has the following structure: 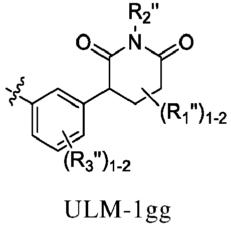 wherein, R₁", R₂" and R₃" are as defined in the above ULM-1. 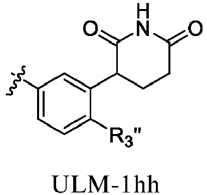 wherein, R₃" is as defined in the above ULM-1.

In some embodiments of the present invention, the ULM is selected from 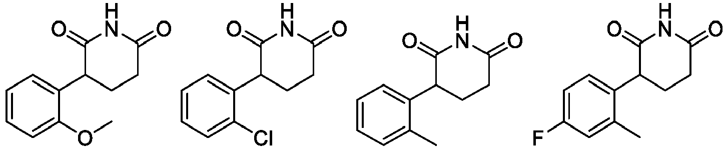 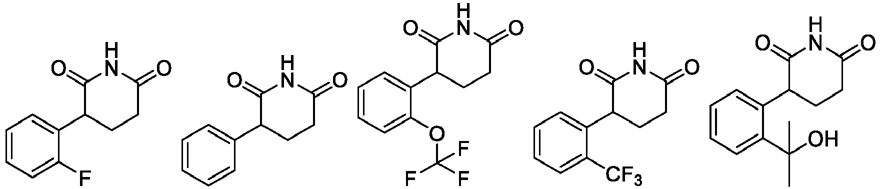 or

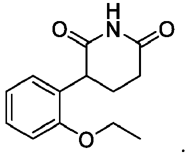

In some embodiments of the present invention, the ULM is selected from

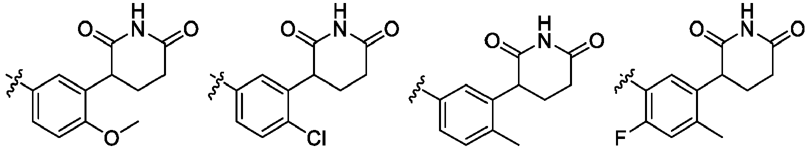

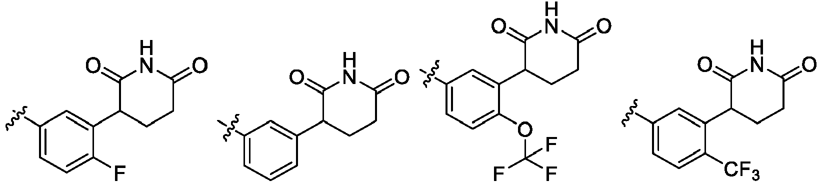

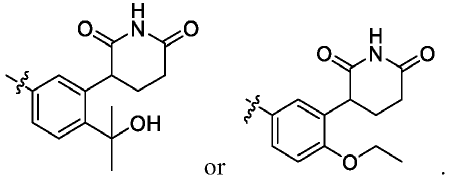

In some embodiments of the present invention, the compound of formula I, and/or stereoisomer, enantiomer, diastereomer, deuterate, metabolite, hydrate, solvate, prodrug and/or pharmaceutically acceptable salt thereof is a compound of formula Ia, and/or a stereoisomer, an enantiomer, a diastereomer, a deuterate, a metabolite, a hydrate, a solvate, a prodrug and/or a pharmaceutically acceptable salts thereof, 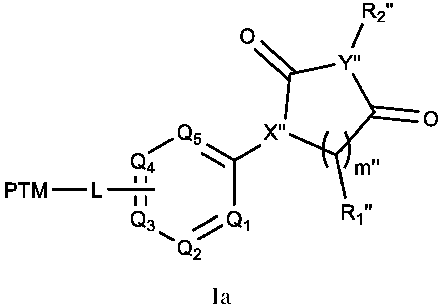 wherein, Q₁, Q₂, Q₃, Q₄, Q₅, R₁", R₂", m", X", and Y" are as defined in ULM-1, and L, PTM are as defined herein.

In some embodiments of the present invention, the PTM has the following structure: 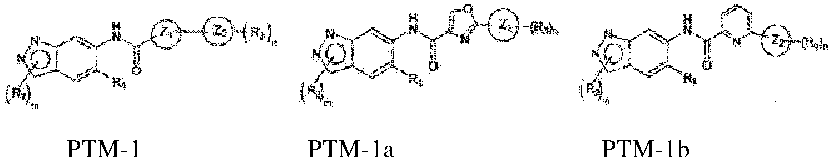
or a pharmaceutically acceptable salt or a stereoisomer thereof; wherein, in PTM-1, PTM-1a or PTM-1b:
   Z₁ is hydrogen, deuterium, optionally substituted alkyl, optionally substituted cycloalkyl, optionally substituted aryl, optionally substituted heterocycloalkyl or absent;
   Z₂ is hydrogen, deuterium, optionally substituted alkyl, optionally substituted cycloalkyl, optionally substituted aryl or optionally substituted heteroaryl;
   R₁ is hydrogen, deuterium, optionally substituted alkyl, amino, halogen, cyano, optionally substituted aryl, optionally substituted heteroaryl, optionally substituted (aryl) alkyl- or optionally substituted (heterocycloalkyl) alkyl-;
   R₂ is hydrogen, deuterium, halogen, amino, optionally substituted alkyl, optionally substituted cycloalkyl, optionally substituted aryl, optionally substituted heteroaryl, optionally substituted (aryl) alkyl- or optionally substituted (heterocycloalkyl) alkyl- at each occurrence;
   R₃ is hydroxyl, halogen, optionally substituted alkyl, optionally substituted alkoxy, optionally substituted cycloalkyl or -NRₐR_{b} at each occurrence;
   Rₐ and R_{b} are independently hydrogen, deuterium, optionally substituted alkyl, optionally substituted acyl, optionally substituted cycloalkyl, optionally substituted aryl, optionally substituted heterocycloalkyl, optionally substituted (aryl) alkyl- or optionally substituted (heterocycloalkyl) alkyl- at each occurrence;
   m is 0, 1 or 2 at each occurrence; and
   n is 0, 1 or 2 at each occurrence; or,
   in some embodiments of the present invention, the PTM has the following structure: 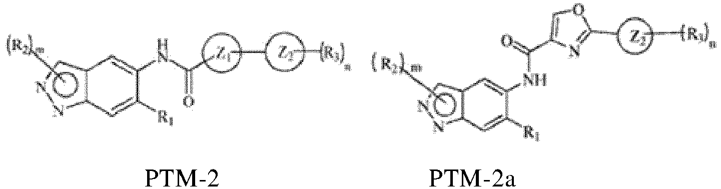 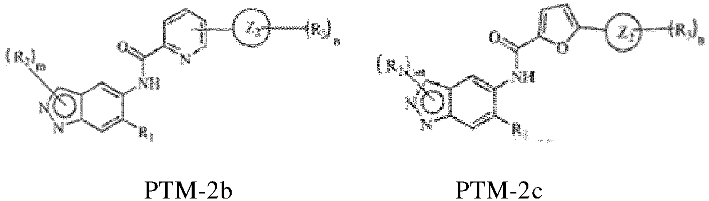
or a pharmaceutically acceptable salt or a stereoisomer thereof; wherein, in PTM-2, PTM-2a, PTM-2b or PTM-2c:
   Z₁ is optionally substituted heteroaryl;
   Z₂ is optionally substituted heterocycloalkyl, optionally substituted heteroaryl or bond;
   R₁ is alkyl, cyano, -NRₐR_{b} or optionally substituted cycloalkyl, optionally substituted aryl, or optionally substituted heterocycloalkyl; wherein the substituents are independently alkyl, alkoxy, halogen, hydroxyl, hydroxyalkyl, amino, aminoalkyl, nitro, cyano, haloalkyl, haloalkoxy, -OC(O)CH₂O-alkyl, -OP(O)(O-alkyl)₂ or -CH₂OP(O)(O-alkyl)₂ at each occurrence;
   R₂ is independently optionally substituted alkyl, or optionally substituted cycloalkyl at each occurrence, wherein the substituent is independently halogen, alkoxy, hydroxyl, hydroxyalkyl, haloalkyl or haloalkoxy at each occurrence;
   R₃ is independently hydrogen, deuterium, halogen, alkyl, haloalkyl, haloalkoxy, alkoxy, hydroxyl, hydroxyalkyl or -NRₐR_{b} at each occurrence;
   Rₐ is hydrogen or alkyl;
   R_{b} is hydrogen, deuterium, alkyl, acyl, hydroxyalkyl, -S(O)₂alkyl or optionally substituted cycloalkyl;
   m or n is independently 1 or 2; or
   in some embodiments of the present invention, the PTM has the following structure: 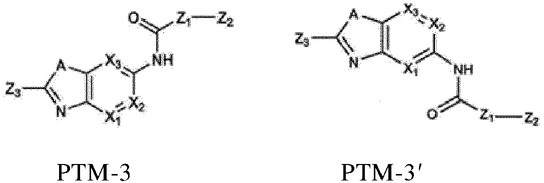 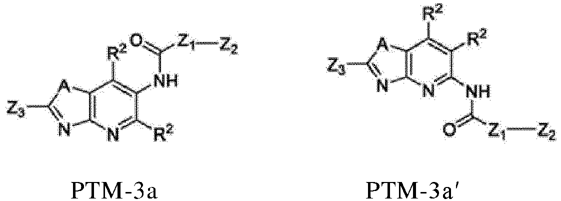
or a pharmaceutically acceptable salt or a stereoisomer thereof; wherein, in PTM-3, PTM-3', PTM-3a or PTM-3a':
   X₁, X₂ and X₃ are each independently CR² or N;
   A is O, S, S(O) or S(O)₂;
   Z₁ is optionally substituted heteroaryl, optionally substituted heterocycloalkyl, optionally substituted aryl, optionally substituted cycloalkyl, optionally substituted (heterocycloalkyl) alkyl-, optionally substituted (aryl) alkyl-, optionally substituted (heteroaryl) alkyl-, optionally substituted (cycloalkyl) alkyl-, optionally substituted aryl-O-, optionally substituted (heteroaryl) alkyl-, optionally substituted heterocycloalkyl-O-, optionally substituted cycloalkyl-O-, optionally substituted aryl-NR'-, optionally substituted heteroaryl-NR'-, optionally substituted heteroaryl-S-, optionally substituted heteroaryl -S-, optionally substituted heterocycloalkyl-NR'-, optionally substituted cycloalkyl-NR'-, optionally substituted heterocycloalkyl-S-, optionally substituted cycloalkyl-S-, optionally substituted (cycloalkyl) alkyl -NR'-, optionally substituted (aryl) alkyl- NR'-, optionally substituted (heterocycloalkyl) alkyl -NR'-, optionally substituted (heteroaryl) alkyl -NR', optionally substituted (cycloalkyl) alkyl -S-, optionally substituted (aryl) alkyl-S-, optionally substituted (heterocycloalkyl) alkyl-S-, optionally substituted (heteroaryl) alkyl-S-, optionally substituted (cycloalkyl) alkyl-O-, optionally substituted (aryl) alkyl-O-, optionally substituted (heterocycloalkyl) alkyl-O-, optionally substituted (heteroaryl) alkyl-O-; wherein the optional substituent is R^{X};
   Z₂ is absent or optionally substituted heteroaryl, optionally substituted heterocycloalkyl, optionally substituted aryl, optionally substituted cycloalkyl, optionally substituted (heterocycloalkyl) alkyl-, optionally substituted (aryl) alkyl-, optionally substituted (heteroaryl) alkyl-, optionally substituted (cycloalkyl) alkyl-, optionally substituted aryl-O-, optionally substituted heteroaryl-O-, optionally substituted heterocycloalkyl-O-, optionally substituted cycloalkyl-O-, optionally substituted (cycloalkyl) alkyl-, optionally substituted (aryl) alkyl-, optionally substituted (heterocycloalkyl) alkyl-, optionally substituted (heteroaryl) alkyl-, optionally substituted (cycloalkyl) alkyl-NR"-, optionally substituted (heterocycloalkyl) alkyl-NR"-, optionally substituted (heteroaryl) alkyl-NR"-, optionally substituted (aryl) alkyl-NR"-, optionally substituted (cycloalkyl) alkyl-S-, optionally substituted (aryl) alkyl-S-, optionally substituted (heterocycloalkyl) alkyl-S-, optionally substituted (heteroaryl) alkyl-S-, optionally substituted (cycloalkyl) alkyl-O-, optionally substituted (aryl) alkyl-O-, optionally substituted (heterocycloalkyl) alkyl-O-, optionally substituted (heteroaryl) alkyl-O-; wherein the optional substituent is R^{y};
   Z₃ is optionally substituted cycloalkyl, optionally substituted aryl, optionally substituted heteroaryl, optionally substituted heterocycloalkyl, optionally substituted aryl-O-, optionally substituted heteroaryl-O-, optionally substituted cycloalkyl-O-, optionally substituted heterocycloalkyl-O-, optionally substituted (cycloalkyl) alkyl-, optionally substituted (aryl) alkyl-, optionally substituted (heterocycloalkyl) alkyl-, optionally substituted (heteroaryl) alkyl-, optionally substituted cycloalkyl-NR"'-, optionally substituted aryl-NR"'-, optionally substituted heteroaryl-NR"'-, optionally substituted heterocycloalkyl-NR"'-, optionally substituted (cycloalkyl) alkyl-NR"'-, optionally substituted (aryl) alkyl-NR"'-, optionally substituted (heteroaryl) alkyl-NR‴-, optionally substituted (heterocycloalkyl) alkyl-NR"'-, optionally substituted cycloalkyl-S-, optionally substituted aryl-S-, optionally substituted heteroaryl-S-, optionally substituted heterocycloalkyl-S-, optionally substituted (cycloalkyl)) alkyl-O-, optionally substituted (aryl) alkyl-O-, optionally substituted (heteroaryl) alkyl-O-, optionally substituted (heterocycloalkyl) alkyl-O-, optionally substituted (cycloalkyl) alkyl-S-, optionally substituted (aryl) alkyl-S-, optionally substituted (heteroaryl) alkyl-S-, optionally substituted (heterocycloalkyl) alkyl-S-; wherein the optional substituent is R^{z};
   R² is selected from hydrogen, deuterium, alkyl, haloalkyl, halogen, cyano, optionally substituted alkoxy, optionally substituted cycloalkyl, optionally substituted (cycloalkyl) alkyl, optionally substituted cycloalkyl-O-, optionally substituted aryl, optionally substituted (aryl) alkyl-, optionally substituted heterocycloalkyl, optionally substituted heteroaryl, optionally substituted (heterocycloalkyl) alkyl-, optionally substituted (heteroaryl) alkyl-, - NR^{a}R^{b},-OR³and -SR³; wherein the optional substituent is alkyl, alkoxy, halogen, haloalkyl, hydroxyl, hydroxyalkyl, -SH, -S(alkyl), cyano, amide, amino, carboxylic acid, glycine ester, alanine ester, oxo, aryl, cycloalkyl, heterocycloalkyl or heteroaryl;
   each R', R" and R"' is independently selected from hydrogen, deuterium, alkyl, hydroxyl, hydroxyalkyl, acyl, or cycloalkyl;
   each R^{x}, R^{y}, and R^{z} are independently selected from alkyl, alkenyl, alkynyl, halogen, hydroxyl, haloalkyl, hydroxyalkyl, aminoalkyl, alkoxy, -SH, -S(alkyl), cyano, amide, carboxylic acid, carboxylate, ester, thioester, alkoxycarbonyl, -C(O)NH(alkyl), oxo, cycloalkyl, cycloalkyl-O-, (cycloalkyl) alkyl-, aryl, (aryl) alkyl-, heterocycloalkyl, heteroaryl, (heterocycloalkyl) alkyl-, (heteroaryl) alkyl-, -NR^{a}R^{b}, -OR⁴ and -SR⁴; wherein the cycloalkyl, aryl, heteroaryl and heterocycloalkyl are optionally further substituted by one or more substituents selected from halogen, haloalkyl, amino, hydroxyl, alkyl, cyano, nitro, alkenyl, aminoalkyl, hydroxyalkyl or haloalkoxy;
   each R^{a} and R^{b} is independently selected from hydrogen, deuterium, alkyl, aminoalkyl, acyl, aminoacyl, halogen, haloalkyl, hydroxyl, haloalkoxy, hydroxyalkyl, nitro, cyano, cycloalkyl, heterocycloalkyl, aryl, heteroaryl, (cycloalkyl) alkyl-, (heterocycloalkyl) alkyl, (aryl) alkyl-, (heteroaryl) alkyl-; wherein the cycloalkyl, heterocycloalkyl, aryl and heteroaryl are optionally further substituted by one or more substituents selected from alkyl, halogen, alkenyl, cyano, hydroxyl, hydroxyalkyl, alkoxy, amino or nitro; or
   R^{a} and R^{b} together with the N atoms to which they are attached form a 3 to 8 membered optionally substituted ring; and
   each R³ and R⁴ is independently selected from hydrogen, deuterium, alkyl, aminoacyl, phosphate, phosphonate, alkyl phosphate, alkoxycarbonyl, cycloalkyl, (cycloalkyl) alkyl-, aryl, heteroaryl, heterocycloalkyl, (aryl) alkyl-, (heteroaryl) alkyl-, or (heterocycloalkyl) alkyl-; or,
   in some embodiments of the present invention, the PTM has the following structure: 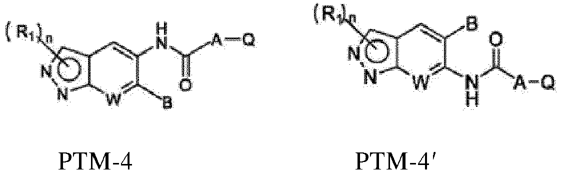 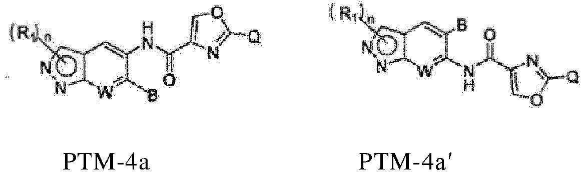 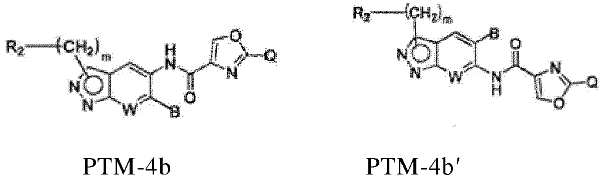 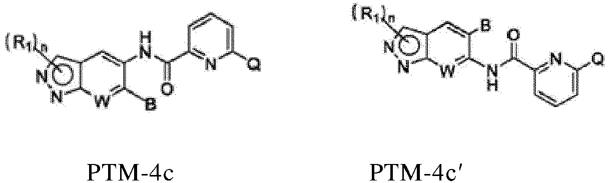
or a pharmaceutically acceptable salt or a stereoisomer thereof; wherein in the PTM-4, PTM-4a, PTM-4b, PTM-4c, PTM-4a', PTM-4b', PTM-4c', and PTM-4d',
A is optionally substituted heteroaryl, optionally substituted aryl, optionally substituted heterocycloalkyl, optionally substituted cycloalkyl, optionally substituted (cycloalkyl) alkyl, optionally substituted (heterocycloalkyl) alkyl, optionally substituted (aryl) alkyl-, optionally substituted (heteroaryl) alkyl-, optionally substituted cycloalkyl-NR^{X}-, optionally substituted heterocycloalkyl-NR^{X}-, optionally substituted aryl-NR^{X}-, optionally substituted heteroaryl-NR^{X}-, optionally substituted cycloalkyl-O-, optionally substituted heterocycloalkyl-O-, optionally substituted aryl-O-, or optionally substituted heteroaryl-O-; wherein the optional substituent is R^{X};
B is hydrogen, deuterium, halogen, cyano, optionally substituted alkyl, alkenyl, optionally substituted alkoxy,-NR^{a}R^{b}, optionally substituted cycloalkyl, optionally substituted aryl, optionally substituted heterocycloalkyl, optionally substituted heteroaryl, optionally substituted (cycloalkyl) alkyl, optionally substituted (heterocycloalkyl) alkyl, optionally substituted (aryl) alkyl-, optionally substituted (heteroaryl) alkyl-, optionally substituted cycloalkyl-NR^{x}-, optionally substituted heterocycloalkyl-NR^{x}-, optionally substituted aryl-NR^{x}-, optionally substituted heteroaryl-NR^{x}-, optionally substituted cycloalkyl-O-, optionally substituted heterocycloalkyl-O-, optionally substituted heteroaryl-O-, optionally substituted aryl-O-; wherein the optional substituent is R^{y};
Q is absent or optionally substituted heterocycloalkyl, optionally substituted heteroaryl, optionally substituted aryl, optionally substituted cycloalkyl, optionally substituted (heterocycloalkyl) alkyl, optionally substituted (heteroaryl) alkyl, optionally substituted (aryl) alkyl-, optionally substituted (cycloalkyl) alkyl, -NR³R⁴, -OR³ or -SR³; wherein the optional substituent is R^{z};
W is N or CH;
R₁ is hydrogen, deuterium, optionally substituted alkyl, optionally substituted cycloalkyl, optionally substituted (cycloalkyl) alkyl, optionally substituted (heterocycloalkyl) alkyl, optionally substituted heterocycloalkyl, optionally substituted (aryl) alkyl-, optionally substituted (heteroaryl) alkyl-, optionally substituted alkoxyalkyl, optionally substituted aminoalkyl or -(CH2)ₘR₂; wherein the optional substituent is each independently selected from halogen, hydroxyl, alkoxy, amino, nitro, cycloalkyl, aryl, heteroaryl or heterocycloalkyl;
R₂ is hydrogen, deuterium, -NR^{a}R^{b}, alkoxy, hydroxyl, optionally substituted heteroaryl or optionally substituted heterocycloalkyl; wherein the optional substituent is R^{y};
R₃ and R₄ are each independently selected from optionally substituted aryl, optionally substituted cycloalkyl, optionally substituted heteroaryl, optionally substituted heterocycloalkyl, optionally substituted (aryl) alkyl-, optionally substituted (cycloalkyl) alkyl, optionally substituted (heteroaryl) alkyl, or optionally substituted (heterocycloalkyl) alkyl; wherein the optional substituent is each independently selected from alkyl, halogen, haloalkyl, hydroxyl, hydroxyalkyl, alkoxy, alkoxy alkyl, amino, nitro, cycloalkyl, (cycloalkyl) alkyl, aryl, (aryl) alkyl-, (heteroaryl) alkyl-, (heterocycloalkyl) alkyl, heteroaryl and (heteroaryl) alkyl;
each R^{a} and R^{b} are independently selected from hydrogen, deuterium, alkyl, aminoalkyl, acyl, or heterocycloalkyl; or R^{a} and R^{b} together with the nitrogen to which they are attached form an optionally substituted ring;
R^{X} is hydrogen, deuterium, alkyl, hydroxyl, hydroxyalkyl, acyl or cycloalkyl;
each R^{y} and R^{z} are independently selected from hydroxyl, hydroxyalkyl, halogen, alkyl, oxo, haloalkyl, alkoxy, alkenyloxy, amino, nitro, cyano, -SH, -S (alkyl), glycine ester, ester, thioester, cycloalkyl, heterocycloalkyl, aryl, heteroaryl, (cycloalkyl) alkyl, (heterocycloalkyl) alkyl, (aryl) alkyl- and (heteroaryl) alkyl; wherein the hydroxyl, hydroxyalkyl, alkoxy, cycloalkyl, heterocycloalkyl, aryl and heteroaryl are optionally further substituted by one or more substituents selected from alkyl, halogen, alkenyl, amino, nitro, cycloalkyl or (cycloalkyl) alkyl; or
R^{y} and R^{z} together with the atoms to which they are attached form an alkyl chain with 1-10 carbon atoms; 1-3 carbon atoms of which are optionally substituted by O, NH or S;
m is 1, 2 or 3; and n is 1 or 2; or
in some embodiments of the present invention, the PTM has the following structure: 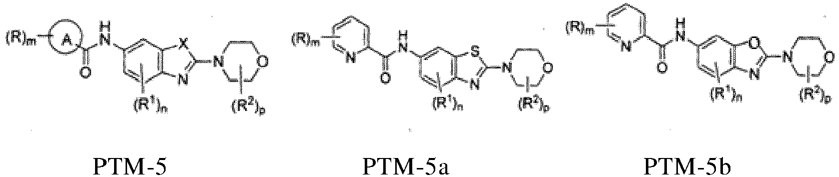
or prodrugs, nitrogen oxides, stereoisomers, solvates, pharmaceutically acceptable salts thereof; wherein in PTM-5, PTM-5a, PTM-5b:
   X is selected from O, S or NH;
   A is selected from aryl or heteroaryl;
   R is independently selected from hydrogen, deuterium, cyano, halogen, hydroxyl, nitro, -NR³R⁴, optionally substituted alkyl, optionally substituted aryl, optionally substituted cycloalkyl, optionally substituted heterocycloalkyl, optionally substituted heteroaryl; the optional substituent is independently selected from halogen, alkyl, haloalkyl, cyano, - NR⁵NR⁶ or -C(O)OR⁷;
   R¹ is independently selected from hydrogen, deuterium, halogen, alkyl, aryl, heterocycloalkyl, (heterocycloalkyl) alkyl-, heteroaryl, -Y-(aryl) alkyl- or -Y-cycloalkyl; the cycloalkyl, aryl, heterocycloalkyl, (heterocycloalkyl) alkyl-, heteroaryl or (aryl) alkyl- is optionally substituted by hydroxyl, alkyl, haloalkyl, cyano or halogen;
   Y is selected from bond, -O-, -C(O)- or -NR⁷-;
   R² is independently selected from hydrogen, deuterium, carboxyl, cyano, hydroxyl, hydroxyalkyl, alkyl, aryl, heteroaryl, -S(O)₂R⁵ or oxo;
   R³ and R⁴ are independently selected from hydrogen, deuterium, hydroxyalkyl, aminoalkyl, optionally substituted alkyl, optionally substituted heterocycloalkyl, or optionally substituted aryl; the optional substituent is independently selected from halogen, haloalkyl or -C(O)OR⁷;
   each R⁵ and R⁶ are independently selected from hydrogen, deuterium, alkyl, -C(O)R⁷ or -C(O)OR⁷;
   R⁷ is selected from hydrogen and alkyl;
   and m, n, and p are each independently selected from 1, 2, or 3; or
   in some embodiments of the present invention, the PTM has the following structure: 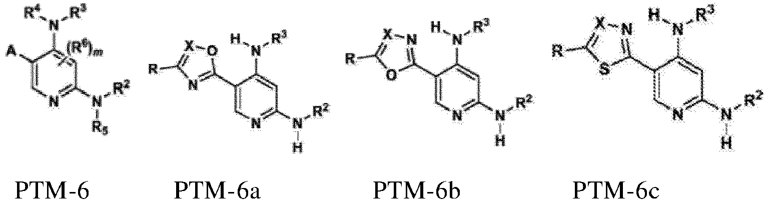
or pharmaceutically acceptable salt thereof; wherein, in PTM-6, PTM-6a, PTM-6b, PTM-6c:
   A is triazole substituted by 0-2 R or 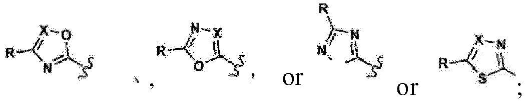
   X is N or CR⁷;
   R is hydrogen, deuterium, R¹, halogen, cyano, nitro, -OR¹-, -C(O)R¹, -C(O)OR¹, - C(O)NR¹¹R¹, -S(O)₂R¹, -NR¹¹C(O)R¹, -NR¹¹C(O)NR¹¹R¹, -NR¹¹C(O)OR¹, -NR¹¹S(O)₂R¹ or - NR¹¹R¹;
   R¹ is hydrogen, deuterium, 0-4 R^{1a} substituted C1-C6 alkyl, C1-C6 haloalkyl, 0-4 R^{1a}substituted C2-6 alkenyl, 0-3 R^{1a} substituted C2-6 alkynyl, 0-3 R^{1a} substituted C3-C10 cycloalkyl, 0-3 R^{1a} substituted 6-10 membered aryl, 0-3 R^{1a}substituted 5-10-membered heteroaryls containing 1-4 heteroatoms selected from N, O or S, or 0-3 R^{1a} substituted 5-10-membered heterocycloalkyl containing 1-4 heteroatoms selected from N, O or S;
   R² is 0-4 R^{2a} substituted 6-10-membered aryl , 1-4 R^{2a} substituted 5-10-membered heterocycloalkyl containing 1-4 heteroatoms selected from N, O or S, or 0-4 R^{2a} substituted 5-10-membered heterocycloalkyl containing 1-4 heteroatoms selected from N, O or S;
   R^{1a} is hydrogen, deuterium, oxo, F, Cl, Br, OCF₃, CN, NO₂, -(CH₂)ᵣOR^{b}, -(CH₂)ᵣSR^{b}, - (CH₂)ᵣC(O)R^{b}, -(CH₂)ᵣC(O)OR^{b}, -(CH₂)ᵣOC(O)R^{b}, -(CH₂)ᵣNR¹¹R¹¹, -(CH₂)ᵣC(O)NR¹¹R¹¹, - (CH₂)ᵣNR^{b}C(O)R^{c}, -(CH₂)ᵣNR^{b}C(O)OR^{c}, -NR^{b}C(O)NR¹¹R¹¹, -S(O)ₚNR¹¹R¹¹, -NR^{b}S(O)ₚR^{c}, - S(O)₂R^{c}, -S(O)R^{c}, 0-2 R^{a} substituted C1-C6 alkyl, C1-C6 haloalkyl, 0-3 R^{a} substituted-(CH₂)ᵣ-3-14 membered carbocyclic, or 0-3 R^{a} substituted -(CH₂)ᵣ-5-7 membered heterocycloalkyl or heteroaryl, the heterocycloalkyl or heteroaryl each contains carbon atoms and 1-4 heteroatoms selected from N, O or S(O)ₚ;
   R^{2a} is hydrogen, deuterium, oxo, halogen, OCF₃, CN, NO₂, -(CH₂)ᵣOR^{b}, -(CH₂)ᵣSR^{b}, - (CH₂)ᵣC(O)R^{b}, -(CH₂)ᵣC(O)OR^{b}, -(CH₂)ᵣOC(O)R^{b}, -(CH₂)ᵣNR¹¹R¹¹, -(CH₂)ᵣC(O)NR¹¹R¹¹, - (CH₂)ᵣNR^{b}C(O)R^{c}, -(CH₂)ᵣNR^{b}C(O)OR^{c}, -NR^{b}C(O)NR¹¹R¹¹, -S(O)ₚNR¹¹R¹¹, -NR^{b}S(O)ₚR^{c}, - S(O)₂R^{c}, -S(O)R^{c}, 0-2 R^{a} substituted C1-C6 alkyl, C1-C6 haloalkyl, 0-1 R^{a} substituted - (CH₂)ᵣ-3-14 membered carbocyclic, or 0-2 R^{a} substituted -(CH₂)ᵣ-5-7 membered heterocycloalkyl or heteroaryl, the heterocycloalkyl or heteroaryl each contains carbon atoms and 1-4 heteroatoms selected from N, O or S(O)ₚ;
   R³ is 0-3 R^{3a} substituted C1-C6 alkyl , C1-C6 haloalkyl, 0-3 R^{3a} substituted C2-6 alkenyl, 0-3 R^{3a} substituted C2-6 alkynyl, 0-3 R^{3a} substituted C3-C10 cycloalkyl, 0-3 R^{3a} substituted 6-10-membered aryl, 0-3 R^{3a} substituted 5-10-membered heteroaryl containing 1-4 heteroatoms selected from N, O or S, or 0-3 R^{3a} substituted 5-10-membered heterocycloalkyl containing 1-4 heteroatoms selected from N, O or S;
   R^{3a} is hydrogen, deuterium, oxo, F, Cl, Br, OCF₃, CN, NO₂, -(CH₂)ᵣOR^{b}, -(CH₂)ᵣSR^{b}, - (CH₂)ᵣC(O)R^{b}, -(CH₂)ᵣC(O)OR^{b}, -(CH₂)ᵣOC(O)R^{b}, -(CH₂)ᵣNR¹¹R¹¹, -(CH₂)ᵣC(O)NR¹¹R¹¹, - (CH₂)ᵣNR^{b}C(O)R^{c}, -(CH₂)ᵣNR^{b}C(O)OR^{c}, -NR^{b}C(O)NR¹¹R¹¹, -S(O)ₚNR¹¹R¹¹, -NR^{b}S(O)ₚR^{c}, - S(O)₂R^{c}, -S(O)R^{c}, 0-2 R^{a} substituted C1-C6 alkyl, C1-C6 haloalkyl, 0-3 R^{a} substituted - (CH₂)ᵣ-3-14 membered carbocyclic, or 0-3 R^{a} substituted -(CH₂)ᵣ-5-7 membered heterocycloalkyl or heteroaryl, the heterocycloalkyl or heteroaryl each contains carbon atoms and 1-4 heteroatoms selected from N, O or S(O)ₚ;
   R⁴ and R⁵ are each independently selected from hydrogen, deuterium, 0-1 R^{f} substituted C1-C4 alkyl, 0-3 R^{d} substituted (CH₂)-phenyl or -(CH₂)-5-7-membered heterocycloalkyl containing a carbon atom and heteroatoms selected from N, O or S(O)ₚ;
   each R⁶ and R⁷ are independently hydrogen, deuterium, oxo, F, Cl, Br, OCF₃, CN, NO₂, -(CH₂)ᵣOR^{b}, -(CH₂)ᵣSR^{b}, -(CH₂)ᵣC(O)R^{b}, -(CH₂)ᵣC(O)OR^{b}, -(CH₂)ᵣOC(O)R^{b}, - (CH₂)ᵣNR¹¹R¹¹, -(CH₂)ᵣC(O)NR¹¹R¹¹, -(CH₂)ᵣNR^{b}C(O)R^{c}, -(CH₂)ᵣNR^{b}C(O)OR^{c}, - NR^{b}C(O)NR¹¹R¹¹, -S(O)ₚNR¹¹R¹¹, -NR^{b}S(O)ₚR^{c}, -S(O)₂R^{c}, -S(O)R^{c}, 0-2 R^{a} substituted C1-C6 alkyl, C1-C6 haloalkyl, 0-3 R^{a} substituted -(CH₂)ᵣ-3-14 membered carbocyclic, or 0-3 R^{a} substituted -(CH₂)ᵣ-5-7 membered heterocycloalkyl or heteroaryl, the heterocycloalkyl or heteroaryl each contains carbon atoms and 1-4 heteroatoms selected from N, O or S(O)ₚ, provided that R⁶ and R⁷are not hydrogen at the same time;
   R¹¹ is independently hydrogen, deuterium, R^{e}, 0-1 R^{f} substituted C1-C4 alkyl, 0-3 R^{d} substituted CH₂-phenyl, or 0-3 R^{d} substituted -(CH₂)-5-7-membered heterocyclyl containing carbon atoms and 1-4 heteroatoms selected from N, O or S(O)ₚ in various cases; or
   R¹¹ and another R¹¹ on the same nitrogen atom, R¹ or R² together form an optionally substituted heterocycloalkyl;
   R^{a} is hydrogen, deuterium, F, Cl, Br, OCF₃, CF₃, CHF₂, CN, NO₂, -(CH₂)ᵣOR^{b}, - (CH₂)ᵣSR^{b}, -(CH₂)ᵣC(O)R^{b}, -(CH₂)ᵣC(O)OR^{b}, -(CH₂)ᵣOC(O)R^{b}, -(CH₂)ᵣNR¹¹R¹¹, - (CH₂)ᵣC(O)NR¹¹R¹¹, -(CH₂)ᵣNR^{b}C(O)R^{c}, -(CH₂)ᵣNR^{b}C(O)OR^{c}, -NR^{b}C(O)NR¹¹R¹¹, - S(O)ₚNR¹¹R¹¹, -NR^{b}S(O)ₚR^{c}, -S(O)₂R^{c}, -S(O)R^{c}, 0-1 R^{f} substituted C1-C6 alkyl, C1-C6 haloalkyl, -(CH₂)ᵣ-3-14 membered carbocyclic, or -(CH₂)ᵣ-5-7 membered heterocyclyl or heteroaryl, the heterocyclyl or heteroaryl each contains carbon atoms and 1-4 heteroatoms selected from N, O or S(O)ₚ; or two R^{a} on adjacent carbon atoms or on the same carbon atom together with the carbon atom to which they are attached form a cyclic acetal of formula -O-(CH₂)ₙ-O- or -O-CF₂-O-, wherein n is selected from 1 or 2;
   R^{b} is hydrogen, deuterium, R^{e}, 0-2 R^{d} substituted C1-C6 alkyl, C1-C6 haloalkyl, C3-C6 cycloalkyl or 0-3 R^{d} substituted (CH₂)ᵣ-phenyl;
   R^{c} is 0-1 R^{f} substituted C1-C6 alkyl, C3-C6 cycloalkyl or 0-3 R^{f} substituted (CH₂)ᵣ-phenyl;
   R^{d} is hydrogen, deuterium, F, Cl, Br, OCF₃, CF₃, CN, NO₂, -OR^{e}, -(CH₂)ᵣC(O)R^{c}, - NR^{e}R^{e}, NR^{e}C(O)OR^{c}, C1-C6 alkyl, or 0-3 R^{f}-substituted (CH₂)ᵣ-phenyl;
   R^{e} is selected from hydrogen, deuterium, C1-C6 alkyl, C3-C6 cycloalkyl and 0-3 R^{f}-substituted (CH₂)ᵣ-phenyl;
   R^{f} is hydrogen, deuterium, halogen, NH₂, OH or O(C1-C6 alkyl);
   p is 0, 1 or 2;
   r is 0, 1, 2, 3 or 4;
   or m is 0, 1, or 2; or
   in some embodiments of the present invention, the PTM has the following structure: 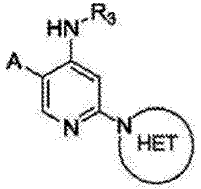 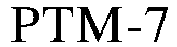
or pharmaceutically acceptable salt thereof; wherein, in PTM-7:
   HET is heteroaryl selected from pyrrolo [2,3-b] pyridyl, pyrrolo [2,3-b] pyrimidinyl, pyrazolo [3,4-b] pyridyl, pyrazolo [3,4-b] pyrimidinyl, imidazo [4,5-b] pyridyl or imidazo[4,5-b]pyrimidinyl, wherein the heteroaryl is connected to the pyridyl in the compound by the nitrogen ring atom in the heteroaryl and wherein the heteroaryl is substituted by 0-2 R_{b};
   A is pyrazolyl, imidazolyl, triazolyl, isoxazolyl, oxadiazolyl or dihydroisoxolyl, each of which is independently substituted by Rₐ;
   R₃ is a cyclic group selected from C2-C3 alkyl, C2-C3 fluoroalkyl, C3-C4 hydroxyalkyl, C3-C6 cycloalkyl, oxetane, tetrahydrofuryl, tetrahydropyran, or pyrazole, wherein the cyclic group is substituted by 0-2 substituents independently selected from F, -OH, C1-C2 alkyl, or -CH₂CHF₂;
   Rₐ is: (i) hydrogen, deuterium, F, Cl,-OH,-CN, C1-C6 alkyl, C1-C6 fluoroalkyl, C1-C6 cyanoalkyl, C1-C6 hydroxyl alkyl, C1-C5 hydroxyl-fluoroalkyl, C2-6 alkenyl, C1-C6aminoalkyl, -(CH₂)₁₋₃NHR_{y}, -(CH₂)₁₋₃NHR_{y}R_{y}, -CH₂CH(OH)(phenyl), - CH₂(CH₂OH)(phenyl), -CH₂CH(OH)CH₂(phenyl), -CH₂CH(OH)CH₂O (methoxyphenyl), - CH₂CH(NH₂)(phenyl), -(CH₂CH₂O)₄H, -(CH₂)₁₋₃O(C1-C3 alkyl), -CH₂CH(OH)CH₂O(C1-C3 alkyl), -CH2C(O)(C1-C3 alkyl), -CH₂C(O)NR_{y}R_{y}, -(CH₂)₁₋₃NR_{y}C(O)(C1-C3 alkyl), - C(O)O(C1-C3 alkyl), -C(O)NH₂, -CH₂NR_{y}C(O)NH₂, -(CH₂)₁₋₂NR_{y}C(O)O(C1-C2 alkyl), - (CR_{y}R_{y})₁₋₅OC(O)CH₂NR_{y}R_{y}, -CH₂CH₂S(O)₂CH₃, -CH₂S(O)₂(C1-C3 alkyl), - CH₂S(O)₂(phenyl), or -NH(aminocyclohexane); or
   (ii) -(CH₂)₀₋₃NHR_{z}, or -(CH₂)₀₋₁C(O)_{z}, wherein, R_{z} is C3-C6 cycloalkyl, azetidinyl, oxetanyl, tetrahydrofuryl, tetrahydropyranyl, piperidinyl, piperazinyl, pyrryl, pyrrolidonyl, morpholinyl, pyrrolidinyl, phenyl, pyrazolyl, imidazolyl, pyridyl, pyrimidinyl, dioxopyrimidinyl, benzo [d] imidazolyl, benzo [d] thiazolyl, 1, 3-dioxocyclopentyl or 8-azabicyclo [3.2.1] octyl, each of which is independently substituted by 0-4 substituents independently selected from the following: F,-OH,-CN, NR_{y}R_{y}, C1-C3 alkyl, C1-C3 fluoroalkyl, C1-C6 hydroxyalkyl,-CH (phenyl)₂,-O(C1-C4 alkyl),-C(O)(C1-C4 alkyl),-C(O)(C1-C4 deuterated alkyl),-C(O)(C1-5 hydroxyalkyl),-C(O))( C1-C3 fluoroalkyl),-C(O)(C3-C6 cycloalkyl),-C(O)O(C1-C4 alkyl), -C(O)NR_{y}R_{y}, -C(O)(phenyl), -C(O)(pyridyl), -C(O)CH₂(C3-C6 cycloalkyl), -NH(C1-C4 alkyl), -NH(C1-C3 fluoroalkyl), -NHC(O)CH₃, - NHC(O)O(C1-C3 alkyl), -NHC(O)O(CH3)₃, -S(O)₂(C1-C3 alkyl), -OS(O)₂(C1-C3 alkyl), methyloxadiazole or pyrimidinyl;
   each R_{b} is independently selected from hydrogen, deuterium, Cl, -CN, -NH₂ or - C(O)NH₂, wherein the heteroaryl is connected to the pyridyl by a nitrogen atom in the heteroaryl; and
   each R_{y} is independently hydrogen or C1-2 alkyl; or
   in some embodiments of the present invention, the PTM has the following structure: 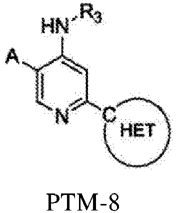
or a pharmaceutically acceptable salt thereof; wherein, in PTM-8:
   HET is a heteroaryl selected from imidazo [1,2-b] pyridazinyl or pyrazolo [1,5-a] pyrimidinyl, wherein the heteroaryl is connected to the pyridyl in the compound by a carbon ring atom in the heteroaryl, wherein the heteroaryl is substituted by 0-2 R_{b};
   A is pyrazolyl, imidazolyl or triazolyl, each of which is substituted by 0-1 Rₐ;
   R₃: (i) -CH₂CH₃, -CH(CH₃)₂, -CH₂CHF₂, -CH(CH₃)CH₂OH, oxetanyl, tetrahydrofuryl-or 0-2 F substituted C3-5 cyclopropyl;
   (ii) pyrazolyl substituted by 0-2 substituents independently selected from the following: C1-C3 alkyl, C1-C3 hydroxyalkyl, C1-C3 fluoroalkyl, oxetanyl, tetrahydrofuryl or tetrahydropyranyl;
   Rₐ is: (i) F, Cl,-OH, -CN, C1-C6 alkyl, C1-C4 fluoroalkyl, C1-C4 cyanoalkyl or C1-C6 hydroxyalkyl; or
   (ii) C3-C6 alkyl, azetidinyl, oxetanyl, tetrahydrofuryl-, tetrahydropyranyl, piperidinyl, piperazinyl, pyrryl, pyrrolidonyl, morpholinyl, pyrrolidyl, phenyl, pyrazolyl, imidazolyl, pyridyl or pyrimidinyl, each of which is independently substituted by 0-4 substituents selected from the following: F,-OH,-CN, NR_{y}R_{y}, C1-C3 alkyl, fluorinated C1-C3 alkyl, - CH(phenyl)₂, -O(C1-C4 alkyl), -C(O)(C1-C4 alkyl), -C(O)(C1-C4 deuterated alkyl), - C(O)(C3-C6 cycloalkyl), -C(O)O(C1-C4 alkyl), -C(O)NR_{y}R_{y}, -C(O)(phenyl), -C(O)(pyridyl), -C(O)CH₂(C3-C6 cycloalkyl), -NHC(O)CH₃, -NHC(O)OCH₃, -NHC(O)O(CH₃)₃, -S(O)₂(C1-C3 alkyl), or -OS(O)₂(C1-C3 alkyl);
   R_{b} is selected from F, Cl, -CN, -NH₂, -CH₃, -OCH₃ or cyclopropyl;
   R_{y} is hydrogen or C1-C3 alkyl; or
   in some embodiments of the present invention, the PTM has the following structure: 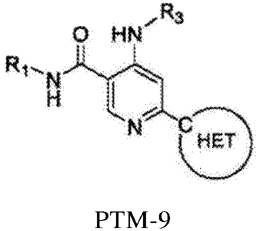
or a pharmaceutically acceptable salt thereof; wherein, in PTM-9:
   HET is a heteroaryl selected from oxazolyl, pyrazolyl, imidazo [1,2-b] pyridazin-3-yl or pyrazolo [1.5-a] pyrimidin-3-yl, wherein the heteroaryl is attached to the pyridyl of the compound through a carbon ring atom in the heteroaryl, and wherein the heteroaryl is substituted by 0-2 R_{b};
   each R_{b} is independently selected from hydrogen, deuterium, F, Cl, -CN, -NH₂, C1-C3 alkyl, C1-2 fluoroalkyl, C1-C3 alkoxy, C3-C6 cycloalkyl,-NH(C1-C3 alkyl), -NH(C1-C4 hydroxyalkyl), cyanophenyl, pyridyl or hydroxypyrrolidyl;
   R1 is: (i) a C3-C6 alkyl substituted by 1-4 substituents independently selected from F,-CN, -OH, -OCH₃, -OCD₃, -NHC(O)(C1-C3 alkyl), -S(O)₂(C1-C3 alkyl), or C1-C3 fluoroalkoxy;
   (ii) -(CR_{y}R_{y})₁₋₃Rₓ or -(CH₂)₁₋₃C(O)Rₓ, wherein Rₓ is phenyl, oxetanyl, tetrahydropyranyl, morpholinyl, piperidinyl, imidazolyl, pyridyl, thienyl, or C4-6 cycloalkyl, each of which is substituted by 0-3 substituents independently selected from F, Cl, -OH, C1-C3 alkyl, C1-2 alkoxy or -S(O)₂NH₂;
   (iii) C4-6 cycloalkyl substituted by 0-2 substituents independently selected from -OH, C1-C4 hydroxyalkyl, C1-C3 alkoxy, -(CH₂)₁₋₃O(C1-C3 alkyl), -C(O)NH(C1-C4 alkyl), - C(O)NH(C3-C6 cycloalkyl), -N(C1-C3 alkyl)₂, -NHC(O)(C1-C3 alkyl), -NHC(O)O(C1-C3 alkyl) or -NHC(O)(C1-C4 hydroxyalkyl);
   (iv) tetrahydrofuryl, tetrahydropyranyl, piperidinyl, piperazinyl, pyrrolyl, pyrazolyl, imidazolyl or triazolyl, each of which is substituted by 0-2 substitutions independently selected from C1-C4 hydroxyalkyl, -S(O)₂(C1-C3 alkyl), -CH₂C(O)NH(C1-C3 alkyl), - CH₂C(O)NH(C1-C6 hydroxyalkyl), -CH₂C(O)NH(C1-C6 fluoroalkyl) or -CH₂C(O)NH(C1-C6 hydroxyl-fluoroalkyl) ; or
   (v) 1-oxa-7-aza spiro [3.5] nonyl;
   R_{y} is independently hydrogen, deuterium, F or -OH; and
   R₃ is: (i) C2-5 alkyl, C2-5 fluoroalkyl, C2-5 hydroxyalkyl, -(CH₂)₁₋₃R_{z} or - CH(CH₂OH)CH₂R_{z}, wherein R_{z} is C4-6 cycloalkyl, oxetanyl, tetrahydrofuryl, tetrahydropyranyl or phenyl, each of which is substituted by 0-1 substituents independently selected from -OH or methyl;
   (ii) C3-C6 cycloalkyl substituted by 0-3 substituents independently selected from F, Cl, -CN, -OH, C1-C3 alkyl, C1-C3 fluoroalkyl, C1-C4 hydroxyalkyl, C1-C4 alkoxy,-C(O)NH₂,-C(O)NH(C1-C3 alkyl) or -C(O)NH(C1-C3 fluoroalkyl);
   (iii) oxetanyl, tetrahydrofuryl, tetrahydropyranyl, pyrrolidyl, piperidinyl, pyrazolyl, thiazolyl, bicyclo [1.1.1] pentyl, bicyclo [2.2.1] heptyl or 4,5, 6,7-tetrahydropyrazolo [1,5-a] pyrazinyl, each of which is substituted by 0-2 substituents independently selected from F, - OH, C1-C4 alkyl, C1-C3 hydroxyalkyl, C1-C4 fluoroalkyl, -CH₂(C3-C6 cycloalkyl), -(CH₂)₁₋₃(C1-C3 alkyl), -C(O)(C1-C3 fluoroalkyl), -S(O)₂(C1-C3 alkyl), C3-C6 cycloalkyl, C3-C6 fluorocycloalkyl, oxetanyl , tetrahydrofuryl, tetrahydropyranyl, pyrimidinyl, fluoropyrimidinyl or methoxypyrimidinyl; or
   (iv) phenyl substituted by 0-3 substituents independently selected from F, Cl, -CN, -OH, -C(O)NH₂, -C(O)NH(C1-C3 alkyl), or -C(O)NH(C1-C3 fluoroalkyl); or
   in some embodiments of the present invention, the PTM has the following structure: 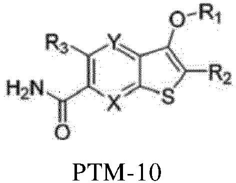
or a pharmaceutically acceptable salt or a prodrug thereof; wherein, in PTM-10:
   X is CR₄ or N;
   Y is CR₅ or N; provided that only one of X and Y is N;
   R₁ is 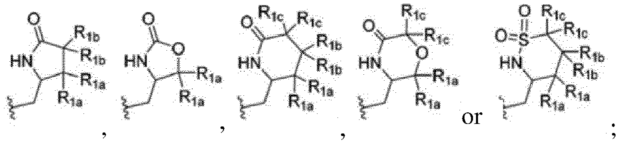
   R₁ₐ, R_{1b}, and R_{1c} are each independently hydrogen, deuterium,-OH, F, C1-C3 alkyl, C1-C3 fluoroalkyl, C1-C3 alkoxy, C1-C3 fluoroalkoxy, or C3-C6 cycloalkyl; or two R₁ₐ together with the carbon atoms to which they are attached can form a 3-4-membered spiro-cycloalkyl ring; or
   R₁ₐ and R_{1b} together with the carbon atoms to which they are attached can form a 3-4 membered cycloalkyl ring;
   R₁ₐ and R_{1c} together with the carbon atoms to which they are attached can form a 3-4 membered cycloalkyl ring;
   R₂ is hydrogen, deuterium, halogen, C1-C3 alkyl or C3-C6 cycloalkyl;
   R₃ is C1-C4 alkoxy, C1-C4 fluoroalkyl or C3-C6 cycloalkyl;
   R₄ and R₅ are each independently hydrogen, deuterium, halogen, C1-C4 alkyl, C1-C4 fluoroalkyl or C3-C6 cycloalkyl; or
   in some embodiments of the present invention, the PTM has the following structure: 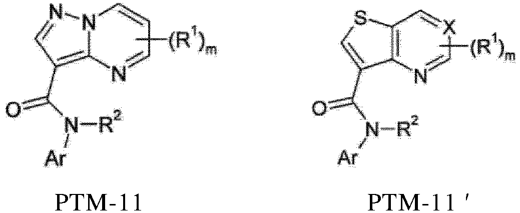
or a pharmaceutically acceptable salt thereof; wherein, in PTM-11, PTM-11':
   X is N or CH;
   m is 1 or 2;
   Ar is optionally substituted aryl or optionally substituted heteroaryl;
   R₁ is hydrogen, deuterium, C1-C6 alkyl, C1-C6 alkoxy, hydroxyl, hydroxyl-C1-C6 alkyl, C1-C6 alkyl-amino, amino-C1-C6 alkyl, amino-C1-C6 alkylamino, hydroxyl-C1-C6 alkylamino, C3-C6 cycloalkylamino, amino-C3-C6 cycloalkylamino, amino-C3-C6 heterocycloalkylamino, aminocarbonyl, halogen or hydroxyl-C1-C6 alkoxy; and
   R₂ is hydrogen or C1-C6 alkyl; or,
   in some embodiments of the present invention, the PTM has the following structure: 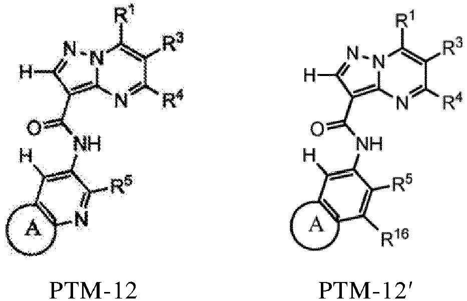
or a stereoisomer or a pharmaceutically acceptable salt thereof; wherein, in PTM-12 or PTM-12':
   R¹ is hydrogen or halogen;
   R³ is hydrogen, deuterium, halogen,-CN, -OH, C1-C3 alkyl, C2-3 alkenyl, C3-7 cycloalkyl, C1-C3 alkyl acyl,-(C0-3 alkyl) C(O)NR⁶R⁷,-(C2-3 alkenyl) C(O)NR⁶R⁷, -S(O)₁₋₂NR⁶R⁷, -NR⁸R⁹, -OC1-C3 alkyl, 3-7-membered monocyclic saturated or partially saturated heterocycloalkyl, 5-6-membered monocyclic heteroaryl or 5-6-membered monocyclic aryl; wherein the alkyl, alkylacyl or alkenyl are optionally substituted by halogen, oxo,-CN,-OH, C1-C3 alkoxy or C1-C3 haloalkoxy; and the cycloalkyl, heterocycloalkyl, heteroaryl or aryl are each independently optionally substituted by halogen, oxo,-CN,-OH, C1-C3 alkyl or C1-C3 haloalkyl;
   R₄ is hydrogen, deuterium, halogen, C1-C3 alkyl, C2-3 alkenyl, -(C0-3 alkyl) C(O)R¹³, - (C2-3 alkenyl) C(O)NR¹⁰R¹¹, -S(O)₁₋₂NR¹⁰R¹¹, 3-7-membered monocyclic saturated or partially saturated heterocycloalkyl,-(CO-3 alkyl) C(O)NR¹⁰R¹¹, -C(O)NR⁸R⁹, or -NR⁸R⁹; wherein, the alkyl, heterocycloalkyl or alkenyl is each independently optionally substituted by halogen, oxo,-CN, -OH, C1-C3 alkoxy, C1-C3 haloalkoxy or optionally oxo substituted 3-7 monocyclic saturated or partially saturated heterocycloalkyl ring;
   R⁵ is hydrogen, deuterium,-CN, C1-C6 alkyl, C1-C6 alkoxy, C3-C10 cycloalkyl, - NR⁸R⁹, -C(O)NR⁸R⁹, -O(C3-7 cycloalkyl), -O(C1-C3 alkyl)-3-8-membered cycloalkyl, - O(C0-3 alkyl)-3-8-membered saturated or partially unsaturated heterocycloalkyl, -O(C1-C3 alkyl) phenyl, -O(C1-C3 alkyl)-5-6-membered heteroaryl, -3-11membered saturated or partially saturated heterocycloalkyl, or 5-6-membered monocyclic heteroaryl; wherein, the alkyl or alkoxy is each independently optionally substituted by halogen, oxo,-CN,-OH, C3-7 cycloalkyl, C1-C3 alkoxy, C1-C3 haloalkoxy, or 3-11 membered saturated or partially saturated heterocycloalkyl optionally substituted by halogen optionally substituted -C(O)(C1-C3 alkyl) or C1-C3 alkyl; and the cycloalkyl, heterocycloalkyl, phenyl or heteroaryl is optionally substituted by halogen, oxo, -CN, -OH, C1-C6 alkoxy, -NR⁸R⁹, -C(O)(C1-C3 alkyl), -(C0-3 alkyl) C(O)NR¹⁰R¹¹, -S(O)₁₋₂NR⁸R⁹, -OP(O)(OC1-C3 alkyl)₂, C3-C10 cycloalkyl optionally substituted by -OH or halogen, 3-11membered saturated or partially saturated heterocycloalkyl optionally substituted by oxo or C1-C3 alkyl, 5-6-membered monocyclic heteroaryl optionally substituted by halogen, oxo,-CN,-OH, C1-C3 alkyl or C1-C3 haloalkyl, or optionally substituted by halogen, oxo,-CN,-OH,-O(C1-C3 alkyl),-S(C1-C3 alkyl),-S(O)₂(C1-C3 alkyl),-NR⁸R⁹,-C(O)NR⁸R⁹, phenyl, C3-C10 cycloalkyl, saturated or partially saturated 3-11 membered heterocycloalkyl ring optionally substituted by oxo or C1-C3 alkyl, or C1-C4 alkyl substituted by 5-6-membered monocyclic heteroaryl optionally substituted by oxo, halogen or C1-C3 alkyl;
   A is 3-11-membered heterocycloalkyl, it is optionally substituted by halogen, oxo, -CN, -OH, C1-C6 alkyl, -(C0-3 alkyl)-C3-C6 cycloalkyl, -(C0-3 alkyl)-3-11membered heterocycloalkyl, -NR⁸R⁹, -NR¹²C(O)R¹³, -NR¹²S(O)₁₋₂R¹³, -C(O)(C1-C3 alkyl), - C(O)NR¹⁰R¹¹, -C(O)OR¹³, -S(O)₁₋₂NR¹⁰R¹¹, or -(C0-3 alkyl)-OP(O)(OC1-C3 alkyl)₂; the alkyl, cycloalkyl, or heterocycloalkyl is each independently optionally substituted by halogen, oxo,-CN,-OR¹³, C1-C3 alkoxy, C1-C3 haloalkoxy, -C(O)(C1-C3 alkyl), -S(C1-C3 alkyl), or substituted C1-C3 alkyl optionally substituted by -OH, halogen, C1-C3 haloalkyl, C1-C3 alkoxy, C1-C3 haloalkoxy, or 3-8-membered heterocycloalkyl;
   when A is a 5-membered azacycloalkyl, the nitrogen heteroatom is substituted;
   R⁶ and R⁷ are independently hydrogen, deuterium, C1-C3 alkyl, or C3-C6 cycloalkyl at each occurrence; wherein, the alkyl or cycloalkyl is each independently optionally substituted by halogen, oxo,-CN,-OH, C1-C3 alkyl, C1-C3 haloalkyl, C1-C3 alkoxy, or C1-C3 haloalkoxy;
   R⁸, R⁹, R¹⁰ or R¹¹ is each independently hydrogen, deuterium, C1-C6 alkyl, C3-C6 cycloalkyl, -(C0-3 alkyl)-phenyl, 3-11 membered saturated heterocycloalkyl, 5-6-membered monocyclic heteroaryl, -C(O)R¹³, -C(O)OR¹³, -S(O)₁₋₂R¹³ or -C (O) NR⁶NR⁷ at each occurrence; or R¹¹ and R¹⁰ together with the atoms to which they are attached form 5-8-membered heterocycloalkyl; wherein, the alkyl, cycloalkyl, phenyl, heterocycloalkyl are each independently optionally substituted by halogen, oxo,-CN, C1-C3 alkyl, C1-C3 haloalkyl, C1-C3 alkoxy, C1-C3 haloalkoxy, -OR¹³, -NR⁶NR⁷, or 5-8-membered heteroaryl;
   R¹² is independently hydrogen, deuterium, C1-C6 alkyl, or C3-C6 cycloalkyl at each occurrence; the alkyl or cycloalkyl is each independently optionally substituted by halogen, oxo,-CN, -OH, C1-C3 alkyl, C1-C3 haloalkyl, C1-C3 alkoxy, or C1-C3 haloalkoxy; and
   R¹³ is independently hydrogen, deuterium, C1-C6 alkyl, or C3-C10 cycloalkyl or 3-11 membered saturated heterocycloalkyl at each occurrence; wherein, the alkyl, cycloalkyl, or heterocycloalkyl is each independently optionally substituted by halogen, oxo, -CN, -OH, C1-C3 alkyl, C1-C3 haloalkyl, C1-C3 alkoxy, C1-C3 haloalkoxy, -OR¹² or -NR⁶NR⁷;
   R16 is hydrogen, deuterium, halogen,-CN or C1-C3 alkyl optionally substituted by - NH₂, halogen or -CN; or
   in some embodiments of the present invention, the PTM has the following structure: 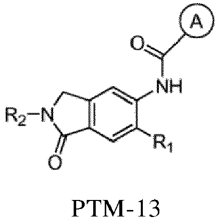
or a stereoisomer or a pharmaceutically acceptable salt thereof; wherein, in PTM-13:
   R₁ is C1-C6 alkoxy, oxetanyl, -NRₓR_{y}, or 6-membered heteroaryl optionally substituted by Rz;
   R₂ is hydrogen, deuterium, oxo, -CN, -OH, C1-C6 alkyl, -(C0-3 alkyl)-C3-C6 cycloalkyl, -(C0-3 alkyl)-3-11membered heterocycloalkyl, -NRₐR_{b}, -NRₑC(O)R_{f}, -NRₑS(O)₁₋₂Rf, -C(O)(C1-C3 alkyl), -C(O)NR_{c}R_{d}, -C(O)ORε, -S(O)₁₋₂R_{c}R_{d}, -(C0-3 alkyl)-OP(O)(OC1-C3 alkyl)₂; the alkyl, cycloalkyl or heterocycloalkyl is each independently optionally substituted by halogen, oxo, -CN, -OR_{f}, C1-C3 haloalkoxy, -C(O)(C1-C3 alkyl), -S(C1-C3 alkyl), or C1-C3 alkyl optionally substituted by -OH, halogen, C1-C3 haloalkyl, C1-C3 alkoxy, C1-C3 haloalkoxy or 3-8-membered heterocycloalkyl;
   ring A is a 5-membered or 6-membered heteroaryl, a 6-membered saturated or partially saturated heterocycloalkyl, and a 9-membered bicyclic heteroaryl containing at least two heteroatoms selected from N, O or S; the ring A is optionally substituted by Rₘ, provided that ring A does not contain an optionally substituted 9-membered bicyclic heteroaryl of the following structure: 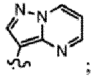
   Rₐ, R_{b}, R_{c} and R_{d} are each independently hydrogen, deuterium, C1-C6 alkyl, C3-C6 cycloalkyl,-(C0-3 alkyl)-phenyl, 3-11-membered saturated heterocycloalkyl, 5-6-membered monocyclic heteroaryl,-C(O)R_{f},-C(O)NR_{g}Rₕ,-C(O)OR_{f}, or -S(O)₁₋₂R_{f}, or R_{c} and R_{d} together with their attached atoms to form a 5-8-membered heterocycloalkyl; the alkyl, cycloalkyl, phenyl, heterocycloalkyl, or heteroaryl is each independently optionally substituted by a halogen, oxo,-CN, C1-C3 alkyl, C1-C3 haloalkyl, C1-C3 alkoxy,-ORε, C1-C3 haloalkoxy, - NR_{g}Rₕ, or 5-6-membered monocyclic heteroaryl;
   Rₑ is hydrogen, deuterium, C1-C6 alkyl or C3-C6 cycloalkyl; the alkyl or cycloalkyl is each independently optionally substituted by halogen, oxo,-CN,-OH, C1-C3 alkyl, C1-C3 haloalkyl, C1-C3 alkoxy or C1-C3 haloalkoxy;
   Rε is hydrogen, deuterium, C1-6 alkyl, C3-C10 cycloalkyl or 3-11membered saturated heterocycloalkyl; the alkyl, cycloalkyl or heterocycloalkyl is each independently optionally substituted by halogen, oxo,-CN,-OH, C1-C3 alkyl, C1-C3 haloalkyl, C1-C3 alkoxy or C1-C3 haloalkoxy,-ORₑ or -NR_{g}Rₕ;
   R_{g} and Rₕ are each independently selected from hydrogen, deuterium, C1-C6 alkyl or C3-C6 cycloalkyl; the alkyl or cycloalkyl is each independently optionally substituted by halogen, oxo,-CN,-OH, C1-C3 alkyl, C1-C3 haloalkyl, C1-C3 alkoxy or C1-C3 haloalkoxy;
   Rₘ is selected from halogen, oxo, -CN, -ORv, -S(O)₁₋₂Rᵥ, -OH, C1-C6 alkoxy, -NRₙRₚ, -C(O)( C1-C3 alkyl), -(C0-3 alkyl) C(O)NRᵣRₛ, -S(O)₁₋₂NRₙRₚ, -OP(O)(OC1-C3 alkyl), C3-C10 cycloalkyl optionally substituted by -OH or halogen, saturated or partially saturated 3-11 membered heterocycloalkyl optionally substituted by halogen, oxo,-CN,-OH, C1-C4 alkoxy,-NRₙRₚ or C1-C4 alkyl optionally substituted by halogen or -OH, C1-C4 alkyl substituted by 3-11-membered saturated or partially saturated heterocycloalkyl substituted by 5-6-membered monocyclic heteroaryl optionally substituted by halogen, oxo, -CN, -OH, - O(C1-C3 alkyl), -S(C1-C3 alkyl), -S(O)₂(C1-C3 alkyl), -NRₙRₚ, -C(O)NRₙRₚ, phenyl, C3-C10 cycloalkyl, optionally substituted by oxo, C1-C3 alkyl or optionally substituted by oxo, halogen or C1-C3 alkyl;
   Rₙ, Rₚ, Rᵣ and Rₛ are each independently selected from hydrogen, deuterium, C1-C6 alkyl, C3-C6 cycloalkyl,-(C0-3 alkyl)-phenyl, 3-11membered saturated heterocycloalkyl, 5-6-membered monocyclic heteroaryl, -C(O)Rᵥ, -C(O)ORᵥ, -C(O)) NRₜRᵤ, or -S(O)₁₋₂Rᵥ; or Rᵣ and Rₛ together with the atom to which they are attached form a heterocycloalkyl; the alkyl, cycloalkyl, phenyl, heterocycloalkyl or heteroaryl is each independently optionally substituted by halogen, oxo,-CN, C1-C3 alkyl, C1-C3 haloalkyl, C1-C3 alkoxy, C1-C3 haloalkoxy, -ORᵥ, -NRₜRᵤ, or 5-6-membered monocyclic heteroaryl;
   Rₜ and Rᵤ are each independently selected from hydrogen, deuterium, C1-C3 alkyl, C3-C6 cycloalkyl; the alkyl or cycloalkyl are each independently optionally substituted by a substituent selected from halogen, oxo, -CN, -OH, C1-C3 alkyl, C1-C3 haloalkyl, C1-C3 alkoxy, C1-C3 haloalkoxy;
   R^{v} is hydrogen, deuterium, C1-C3 alkyl, C3-C10 cycloalkyl, or 3-11 membered saturated heterocycloalkyl; the alkyl, cycloalkyl or heterocycloalkyl is each independently optionally substituted by halogen, oxo,-CN,-OH, C1-C3 alkyl, C1-C3 haloalkyl, C1-C3 alkoxy, C1-C3 haloalkoxy, -OR_{w} or -NRₜRᵤ;
   R_{w} is selected from hydrogen, deuterium, C1-C6 alkyl, and C3-C6 cycloalkyl; the alkyl and cycloalkyl are each independently optionally substituted by halogen, oxo, -OH, C1-C3 alkyl, C1-C3 haloalkyl, C1-C3 alkoxy, or C1-C3 haloalkoxy;
   Rₓ and R_{y} are each independently selected from C1-C6 alkyl; or Rₓ and R_{y} together with their attached atoms form 6-membered heterocycloalkyl optionally substituted by R_{z};
   R_{z} is halogen, oxo, -CN, -S(O)₁₋₂Rᵥ, -OH, C1-C6 alkoxy, -NRₙRₚ, -C(O)(C1-C3 alkyl), - (C0-3 alkyl) C(O)NRᵣRₛ, -S(O)₁₋₂ NRₙRₚ, -OP(O)(OC1-C3 alkyl)₂, C3-C10 cycloalkyl optionally substituted by -OH or halogen, 3-11 membered saturated or partially saturated heterocycloalkyl optionally substituted by oxo or C1-C3 alkyl, 5-6-membered monocyclic heteroaryl optionally substituted by halogen, oxo,-CN,-OH, C1-C4 alkyl,-NRₙRₚ, or C1-C4 alkyl optionally substituted by halogen or -OH, or C1-C4 alkyl substituted by 3-11-membered saturated or partially saturated heterocycloalkyl substituted by 5-6-membered monocyclic heteroaryl optionally substituted by halogen, oxo, -CN, -OH, -O(C1-C3 alkyl), - S(C1-C3 alkyl), -S(O)₂(C1-C3 alkyl), -NRₙRₚ, -C(O)NRₙRₚ, phenyl, C3-C10 cycloalkyl, optionally substituted by oxo, C1-C3 alkyl or optionally substituted by oxo, halogen or C1-C3 alkyl; or
   in some embodiments of the present invention, the PTM has the following structure: 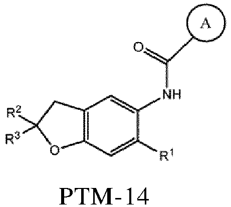
a stereoisomer or a pharmaceutically acceptable salt thereof; wherein, in PTM-14:
   R¹ is C1-C6 alkoxy, oxetanyl, -NR^{a}R^{b} or 6-membered heteroaryl optionally substituted by R^{c};
   R² is methyl, hydroxymethyl, or 2-hydroxypropyl-2-yl;
   R³ is methyl; or
   R² and R³ together with the attached carbon atom form a 6-membered heterocycloalkyl optionally substituted by a C1-C3 alkyl;
   ring A is a 5-membered or 6-membered heteroaryl, a 6-membered saturated or partially saturated heterocycloalkyl, and a 9-membered bicyclic heteroaryl containing at least two heteroatoms selected from N, O or S; the ring A is optionally substituted by R^{d}, provided that ring A does not contain an optionally substituted 9-membered bicyclic heteroaryl of the following structure: 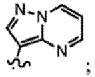
   R^{a} and R^{b} are each independently C1-C6 alkyl; or R^{a} and R^{b} together with the atoms to which they are attached form 6-membered heterocycloalkyl optionally substituted by R^{c};
   R^{c} is selected from halogen, oxo, -CN, -S(O)₁₋₂Rₙ, -OH, C1-C6 alkoxy, -NRₑR_{f}, C(O)(C1-C3 alkyl), -(C0-3 alkyl)C(O)NR_{g}R_{f}, -S(O)₁₋₂NRₑR_{f}, -OP(O)(OC1-C3 alkyl)₂, C3-C10 cycloalkyl optionally substituted by -OH or halogen, 3-11 membered saturated or partially saturated heterocycloalkyl optionally substituted by oxo or C1-C3 alkyl; 5-6 membered monocyclic heteroaryl substituted by oxygen, halogen,-CN, -OH, C1-C4 alkoxy, - NR^{e}R^{f}, or C1-C4 alkyl optionally substituted by halogen or -OH; 3-11 saturated or partially saturated 3-11 membered heterocycloalkyl optionally substituted by halogen, oxo, -CN, -OH, -O(C1-C3 alkyl), -S(C1-C3 alkyl), -S(O)₂(C1-C3 alkyl), -NR^{e}R^{f}, -C(O)NR^{e}R^{f}, phenyl, C3-C10 cycloalkyl, optionally substituted by oxo, C1-C3 alkyl or 5-6-membered monocyclic heteroaryl optionally substituted by oxo, halogen, C1-C3 alkyl;
   R^{d} is halogen, oxo, -CN, -OR¹¹, -S(O)₂Rⁿ, -OH, C1-C6 alkoxy, -NR^{e}R^{f}, C(O)(C1-C3 alkyl), -(C0-3 alkyl) C(O)NR^{g}R^{f}, -S(O)₁₋₂NR^{e}R^{f}, -OP(O)(OC1-C3 alkyl)₂, C3-C10 cycloalkyl optionally substituted by -OH or halogen, 3-11 membered saturated or partially saturated heterocycloalkyl optionally substituted by oxo or C1-C3 alkyl; 5-6 membered monocyclic heteroaryl substituted by oxo, halogen, -CN, -OH, C1-C4 alkoxy,-NR^{e}R^{f}, or C1-C4 alkyl optionally substituted by halogen or -OH; 3-11 saturated or partially saturated 3-11 membered heterocycloalkyl optionally substituted by halogen, oxo, -CN, -OH, -O(C1-C3 alkyl), -S(C1-C3 alkyl), -S(O)₂(C1-C3 alkyl), -NR^{e}R^{f}, -C(O)NR^{e}R^{f}, phenyl, C3-C10 cycloalkyl, optionally substituted by oxo or C1-C3 alkyl, or 5-6-membered monocyclic heteroaryl optionally substituted by oxo, halogen, or C1-C3 alkyl;
   R^{e}, R^{f}, R^{g} and R^{h} are each independently selected from hydrogen, deuterium, C1-C6 alkyl, C3-C6 cycloalkyl, -(C0-3 alkyl)-phenyl, 3-11-membered saturated heterocycloalkyl, 5-6-membered monocyclic heteroaryl, -C(O)Rⁿ, -C(O)ORⁿ, -C(O)NR^{k}R^{m}, -S(O)₁₋₂Rⁿ, or R^{g} and R^{h} together with their attached atoms to form 5-8-membered heterocycloalkyl; the alkyl, cycloalkyl, phenyl, heterocycloalkyl or heteroaryl is each independently optionally substituted by halogen, oxo,-CN, C1-C3 alkyl, C1-C3 haloalkyl, C1-C3 alkoxy, C1-C3 haloalkoxy,-ORn,-NRkRm, or 5-6-membered monocyclic heteroaryl;
   R^{k} and R^{m} are each independently selected from hydrogen, deuterium, C1-C3 alkyl or C3-C6 cycloalkyl; the alkyl or cycloalkyl are each independently optionally substituted by halogen, oxo, -CN, -OH, C1-C3 alkyl, C1-C3 haloalkyl, C1-C3 alkoxy or C1-C3 haloalkoxy;
   Rⁿ is selected from hydrogen, deuterium, C1-C6 alkyl, C3-C10 cycloalkyl, or 3-11 saturated heterocycloalkyl; the alkyl, cycloalkyl, or heterocycloalkyl is each independently optionally substituted by halogen, oxo, -CN, -OH, C1-C3 alkyl, C1-C3 haloalkyl, C1-C3 alkoxy, halogenated C1-C3 alkoxy,-OR^{p} or -NR^{g}R^{h};
   R^{p} is selected from hydrogen, deuterium, C1-C6 alkyl, or C3-C6 cycloalkyl; the alkyl or cycloalkyl are each independently optionally substituted by halogen, oxo, -CN, OH, C1-C3 alkyl, C1-C3 haloalkyl, C1-C3 alkoxy, C1-C3 haloalkoxy; or
   in some embodiments of the present invention, the PTM has the following structure: 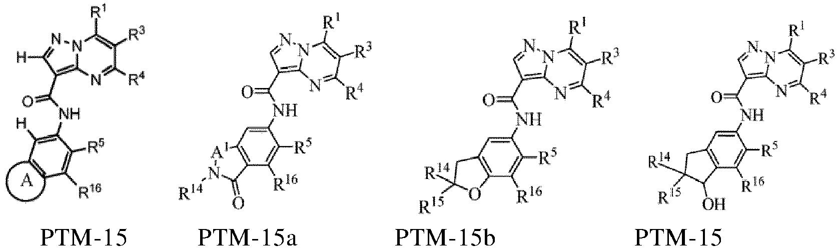 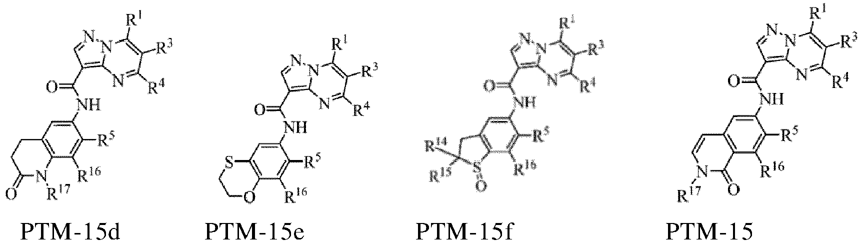 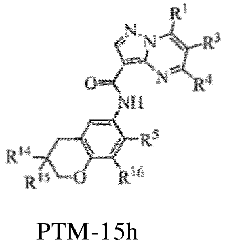
or a stereoisomer or a pharmaceutically acceptable salt thereof; wherein, in PTM-15, PTM-15a, PTM-15b, PTM-15c, PTM-15d, PTM-15e, PTM-15f, PTM-15g or PTM-15h:
   A¹ is CH₂ or NH;
   R¹ is hydrogen or halogen;
   R³ is hydrogen, deuterium, halogen, CN, OH, C1-C3 alkyl, C2-3 alkenyl, C3-7 cycloalkyl, C1-C3 alkyl acyl, -(C0-3 alkyl) C(O)NR⁶R⁷, -(C2-3 alkenyl) C(O)NR⁶R⁷, -S(O)₁₋₂NR⁶R⁷, -NR⁸R⁹, -OC1-C3 alkyl, 3-7-membered monocyclic saturated or partially saturated heterocycloalkyl, 5-6-membered monocyclic heteroaryl ring or 5-6-membered monocyclic aryl; the alkyl, alkylacyl or alkenyl is each independently optionally substituted by substituents selected from halogen, oxo, CN, OH, C1-C3 alkoxy or C1-C3 haloalkoxy; and the cycloalkyl, heterocycloalkyl, heteroaryl or aryl is each independently optionally substituted by substituents selected from halogen, oxo, CN, OH, C1-C3 alkyl or C1-C3 haloalkyl;
   R⁴ is hydrogen, deuterium, halogen, C1-C3 alkyl, C2-3 alkenyl, -(C0-3 alkyl) C(O)R¹³, - (C2-3 alkenyl) C(O)NR¹⁰R¹¹, -S(O)₁₋₂NR¹⁰R¹¹, 3-7-membered monocyclic saturated or partially saturated heterocycloalkyl, -C(O)NR⁸R⁹ or -NR⁸R⁹, the alkyl, alkenyl or heterocycloalkyl is each independently optionally substituted by substituents selected from halogen, oxo, CN, OH, C1-C3 alkoxy, C1-C3 haloalkoxy, or optionally oxo substituted 3-7-membered monocyclic saturated or partially saturated heterocycloalkyl;
   R⁵ is hydrogen, deuterium, -CN, C1-C6 alkyl, C1-C6 alkoxy, C3-C10 cycloalkyl, - NR⁸R⁹, -C(O)NR⁸R⁹, -O(C3-7 cycloalkyl), -O(C1-C3 alkyl)-3-8-membered cycloalkyl, - O(C0-3 alkyl)-3-8-membered saturated or partially saturated heterocycloalkyl, -O(C1-C3 alkyl)-phenyl, -O(C1-C3 alkyl)-5-6-membered heteroaryl ring, 3-11membered saturated or partially saturated heterocycloalkyl or 5-6-membered monocyclic heteroaryl ring, the alkyl and alkoxy are each independently optionally substituted by substituents selected from halogen, oxo, CN, OH, C1-C3 alkoxy, C1-C3 haloalkoxy or a 3-11 membered saturated or partially saturated heterocycloalkyl optionally substituted by the following substituents: (i) - C(O)(C1-C3 alkyl) optionally substituted by halogen, (ii) C1-C3 alkyl optionally substituted by halogen, and the cycloalkyl, heterocycloalkyl, phenyl or heteroaryl is optionally substituted by the followings: halogen, oxo, CN, OH, C1-C6 alkoxy, -NR⁸R⁹, -C(O)(C1-C3 alkyl), -(C0-3 alkyl) C(O)NR¹⁰R¹¹, -S(O)1-2NR⁸R⁹, -OP(O)(OC1-C3 alkyl)₂, C3-C10 cycloalkyl optionally substituted by OH or halogen, 3-11membered saturated or partially saturated heterocycloalkyl optionally substituted by oxo or C1-C3 alkyl, 5-6-membered monocyclic heteroaryl optionally substituted by halogen, oxo, CN, OH, C1-C3 alkyl or C1-C3 haloalkyl, or C1-C4 alkyl optionally substituted by the followings: halogen, oxo, CN, OH, -OC1-C3 alkyl, -SC1-C3 alkyl, -SO₂C1-C3 alkyl, -NR⁸R⁹, -C(O)NR⁸R⁹, phenyl, C3-C10 cycloalkyl, 3-11 membered saturated or partially saturated heterocycloalkyl optionally substituted by oxo or C1-C3 alkyl, or 5-6-membered monocyclic heteroaryl optionally substituted by oxo, halogen or C1-C3 alkyl;
   A is a 3-11-membered heterocycloalkyl optionally substituted by the following substituents: halogen, oxo, CN, OH, C1-C6 alkyl, -(C0-3 alkyl)-C3-C6cycloalkyl, -(C0-3 alkyl)-3-11membered heterocycloalkyl, -NR⁸R⁹, -NR¹²C(O)R¹³, -NR¹²S(O)₁₋₂R¹³, -C(O)(C1-C3 alkyl), -C(O)NR¹⁰R¹¹, -C(O)OR¹³, -S(O)₁₋₂NR¹⁰R¹¹ or -(C0-3 alkyl)-OP(O)(OC1-C3 alkyl)₂, wherein, the alkyl, cycloalkyl, or heterocycloalkyl are each independently optionally substituted by the following substituents: halogen, oxo, CN, OR¹³, C1-C3 haloalkoxy,-C(O)(C1-C3 alkyl),-SC1-C3 alkyl, or C1-C3 alkyl optionally substituted by the following substituents: OH, halogen, C1-C3 haloalkyl, C1-C3 alkoxy, C1-C3 haloalkoxy, or 3-8-membered heterocycloalkyl, and wherein, when A is a 5-membered nitrogen-containing heterocycloalkyl, the nitrogen atom is substituted;
   R⁶ and R⁷ are independently hydrogen, deuterium, C1-C3 alkyl, or C3-C6 cycloalkyl at each occurrence, wherein, the alkyl or cycloalkyl is each independently optionally substituted by the following substituents: halogen, oxo, CN, OH, C1-C3 alkyl, C1-C3 haloalkyl, C1-C3 alkoxy, or C1-C3 haloalkoxy;
   R⁸, R⁹, R¹⁰ and R¹¹ are independently hydrogen, deuterium, C1-C6 alkyl, C3-C6 cycloalkyl,-(C0-3 alkyl)-phenyl, 3-11 membered saturated heterocycloalkyl, 5-6-membered monocyclic heteroaryl, -C(O)R¹³, -C(O)OR¹³, -C(O)NR⁶R⁷ or -S(O)₁₋₂R¹³ at each occurrence; or R¹⁰ and R¹¹ together form a 5-8-membered heterocycloalkyl, wherein the alkyl, cycloalkyl, phenyl, heterocycloalkyl, or heteroaryl ring are each independently optionally substituted by the following substituents: halogen, oxo, CN, C1-C3 alkyl, C1-C3 haloalkyl, C1-C3 alkoxy, C1-C3 haloalkoxy, -OR¹³, -NR⁶R⁷, or 5-6-membered monocyclic heteroaryl;
   R¹² is independently hydrogen, deuterium, C1-C6 alkyl or C3-C6 cycloalkyl at each occurrence, wherein the alkyl or cycloalkyl is independently optionally substituted by the following substituents: halogen, oxo, CN, OH, C1-C3 alkyl, C1-C3 haloalkyl, C1-C3 alkoxy or C1-C3 haloalkoxy;
   R¹³ is independently hydrogen, deuterium, C1-C6 alkyl, C3-C10 cycloalkyl, or 3-11 membered saturated heterocycloalkyl at each occurrence, wherein, the alkyl, cycloalkyl, or heterocycloalkyl is each independently optionally substituted by the following substituents: halogen, oxo, CN, OH, C1-C3 alkyl, C1-C3 haloalkyl, C1-C3 alkoxy, C1-C3 haloalkoxy, - OR¹², or -NR⁶R⁷; and
   R¹⁶ is hydrogen, deuterium, halogen, CN or C1-C3 alkyl optionally substituted by -NH₂, halogen or CN; or
   in some embodiments of the present invention, the PTM has the following structure: 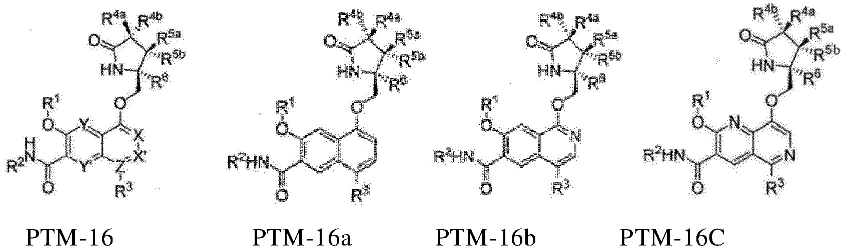 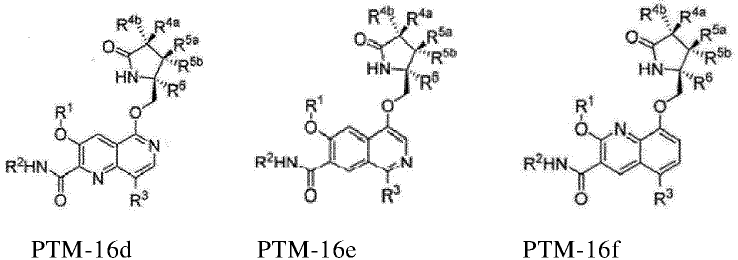
or a pharmaceutically acceptable salt thereof; wherein, in PTM-16, PTM-16a, PTM-16b, PTM-16c, PTM-16d, PTM-16e or PTM-16f:
   X, X', Y and Y' are each independently CH or N; Z is C or N; provided that: not more than 3 of X, X', Z, Y and Y' are N;
   R¹ is C1-C6 alkyl or -(C1-C6 alkyl)ₙ-(C1-C6 cycloalkyl), wherein, the alkyl or cycloalkyl is optionally substituted by deuterium, halogen, CN, OH or C1-C6 alkoxy;
   R² is hydrogen, methyl or optionally substituted cycloalkyl;
   R³ is hydrogen, deuterium, deuterium, halogen, nitrile, -(CH₂)ₜNR^{8a}R^{8b}, -(CH₂)ₜ(6-10-membered aryl) or -(CH2)t(5-10-membered heteroaryl) having 1 to 3 heteroatoms selected from N, O or S, wherein, the aryl or heteroaryl is optionally substituted by 1 to 3 of C1-C6 alkyl, deuterium, halogen, CN, OH, hydroxyl C1-C6 alkyl or C1-C6 alkoxy, wherein the alkyl is optionally substituted by hydroxyl, halogen, CN or C1-C3 alkoxy;
   R^{4a} and R^{4b} are each independently hydrogen, deuterium, fluorine, OH, C1-C3 alkoxy or CH₂OR⁷, wherein R⁷ together with R¹ are C1-C4 alkylene, and the C1-C4 alkylene is optionally substituted by halogen or alkyl;
   R^{5a} and R^{5b} are each independently hydrogen, deuterium, C1-C3 alkyl or C1-C3 alkoxy, wherein the alkyl or alkoxy is optionally substituted by 1 to 3 of deuterium, halogen, OH or CN; or, R^{5a} and R^{5b} together with the atoms to which they are bonded form C3-C7 cycloalkyl or C3-C7 heterocycloalkyl, wherein the cycloalkyl or heterocycloalkyl is optionally substituted by 1 to 3 of deuterium, halogen, OH, CN or C1-C3 alkyl;
   R⁶ is hydrogen or C1-C3 alkyl; or, R^{5b} and R⁶ together with the atoms to which they are attached form C3-C7 cycloalkyl or C3-C7 heterocycloalkyl, wherein, the cycloalkyl or heterocycloalkyl is optionally substituted by 1 to 3 of deuterium, halogen, OH, CN or C1-C3 alkyl;
   R^{8a} and R^{8b} are each independently hydrogen, deuterium, -S(O)₂R⁹ or -C(O)R⁹;
   R⁹ is C1-C6 alkyl, C1-C6 cycloalkyl, 6-10 membered aryl, or 5-10 membered heteroaryl having 1 to 3 heteroatoms, wherein, the alkyl, cycloalkyl, aryl or heteroaryl is optionally substituted by 1 to 3 of C1-C6 alkyl, halogen, CN, OH, C1-C6 alkoxy or C1-C6 hydroxyl;
   n is 0 or 1;
   t is 0, 1, 2 or 3; or
   in some embodiments of the present invention, the PTM has the following structure: 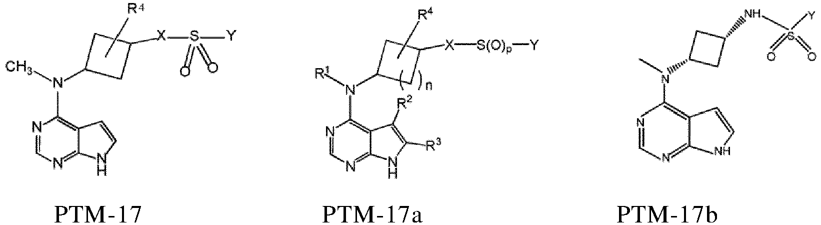 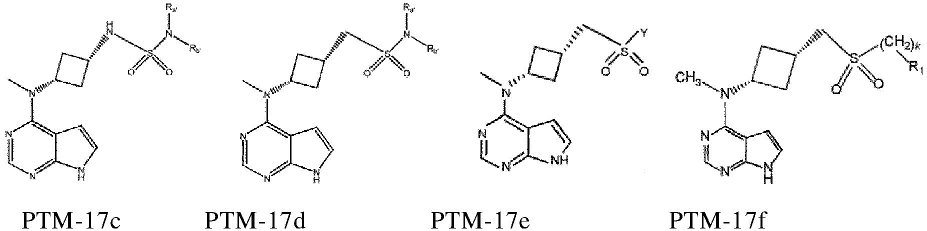
or pharmaceutically acceptable salt thereof; wherein, in PTM-17, PTM-17a, PTM-17b, PTM-17c, PTM-17d, PTM-17e or PTM-17f:
   R₁ is hydrogen or C1-C4 alkyl, wherein the alkyl is further optionally substituted by one or more substituents selected from halogen, hydroxyl, methoxy, amino, CF₃ or C3-C6 cycloalkyl;
   R² and R³ are each independently hydrogen, deuterium, deuterium, C1-C6 linear or branched alkyl, C3-C6 cycloalkyl, C1-C6 linear or branched perfluoroalkyl, C1-C6 linear or branched alkoxy, C1-C6 linear or branched perfluoroalkoxy, halogen, cyano, hydroxyl, amino, carboxyl, aminocarbonyl, aryl, heteroaryl, (aryl) C1-C6 linear or branched alkyl, (heteroaryl) C1-C6 linear or branched alkyl, (heterocycloalkyl) C1-C6 linear or branched alkyl, (C1-C6 linear or branched alkyl) aryl, (C1-C6 linear or branched alkyl) heteroaryl, (C1-C6 linear or branched alkyl) heterocycloalkyl, (C1-C6 linear or branched alkoxy) carbonyl, (C1-C6 linear or branched alkyl) aminocarbonyl amino, or (C1-C6 linear or branched alkyl) aminocarbonyl;
   R⁴ is selected from hydrogen, deuterium, deuterium, C1-C6 linear or branched alkyl, C1-C6 linear or branched perfluoroalkyl, aryl or alkaryl;
   X is selected from -NH- or -CRₐR_{b}-, wherein (a) Rₐ and R_{b} are independently hydrogen, deuterium, deuterium, C1-C6 linear or branched alkyl, C3-C6 cycloalkyl, aryl, (aryl) C1-C6 linear or branched alkyl, heteroaryl, (C1-C6 linear or branched alkyl) heteroaryl, (heteroaryl) C1-C6 linear or branched alkyl, (heterocyclyl) C1-C6 linear or branched alkyl, or (b) Rₐ and R_{b} together to form a chain containing -(CR_{c}R_{d})ⱼ-, wherein R_{c} and R_{d} are independently hydrogen, deuterium, deuterium, C1-C6 linear or branched alkyl, aryl, (C1-C6 linear or branched alkyl) aryl, heteroaryl, (C1-C6 linear or branched alkyl) heteroaryl, halogen, CN, CF₃, hydroxyl, CONH₂ or SO₂CH₃;
   Y is -A-R₅; wherein, A is bond, -(CH₂)ₖ- or -(CD₂)ₖ-; R₅ is C1-C6 linear or branched alkyl, C3-C6 cycloalkyl, aryl or-NR_{a'}R_{b '}, or unsaturated, saturated or partially saturated 4-11 membered monocyclyl or bicyclyl containing 1-4 heteroatoms selected from oxygen, nitrogen or sulfur; wherein, the alkyl, C3-C6 cycloalkyl, aryl or monocyclyl or bicyclyl is further optionally substituted by one or more substituents selected from the group consisting of deuterium, halogen, C1-C6 linear or branched alkyl, CN, hydroxyl, CF₃,-ORₑ,-NRₑR_{f},-S(O)ₚRₑ or C3-C6 cycloalkyl; wherein, the alkyl and cycloalkyl can be optionally substituted by one or more substituents selected from halogen, CN, hydroxyl, CONH₂or SO₂CH₃; wherein, (a) R_{a'} and R_{b'} are independently hydrogen, deuterium, deuterium, C1-C6 linear or branched alkyl, C3-C6 cycloalkyl, aryl, (C1-C6 linear or branched alkyl) aryl, heteroaryl or (C1-C6 linear or branched alkyl) heteroaryl, wherein, the alkyl and cycloalkyl can be optionally substituted by one or more R_{c'} or (b) R_{a'} and R_{b'} together form a chain comprising -(CR_{c'}R_{d'})ⱼ-, wherein, R_{c'} and R_{d'} are independently hydrogen, deuterium, deuterium, C1-C6 linear or branched alkyl, aryl, (C1-C6 linear or branched alkyl) aryl, heteroaryl, (C1-C6 linear or branched alkyl) heteroaryl, halogen, CN, hydroxyl, CF₃, CONH₂,-ORₑ or -NRₑR_{f}, or -S(O)ₚRₑ; wherein, and R_{f} are independently hydrogen, deuterium, deuterium, C1-C6 linear or branched alkyl, or C3-C6 cycloalkyl, wherein, the alkyl and cycloalkyl can optionally be substituted by one or more substituents selected from halogen, CN, hydroxyl, CF₃ and CONH₂;
   j is 2, 3, 4 or 5;
   k is 1, 2, 3 or 4;
   p is 0, 1 or 2;
   n is 1 or 2; or
   in some embodiments of the present invention, the PTM has the following structure: 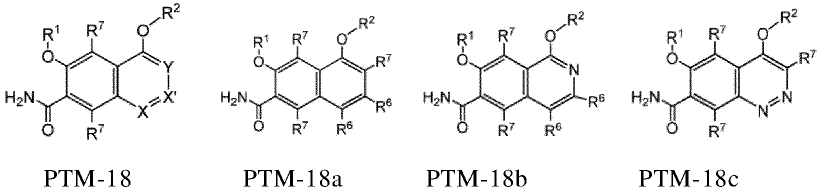 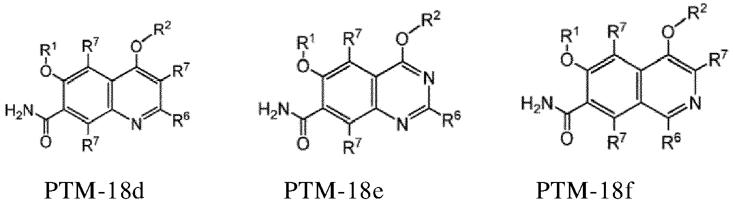
or pharmaceutically acceptable salt thereof; wherein, in PTM-18, PTM-18a, PTM-18b, PTM-18c, PTM-18d, PTM-18e or PTM-18f:
   X and X' are each independently CR⁶, N or -N⁺O⁻;
   Y is independently N, -N+O⁻ or CH; provided that at least one of X, X' or Y is neither N nor -N+O⁻, and not more than one of X, X' or Y is -N+O⁻;
   R¹ is C1-C6 alkyl or 3-7-membered cycloalkyl, wherein the alkyl or cycloalkyl is optionally substituted by 1 to 5 substituents selected from halogen, deuterium, -OR⁵, -SR⁵, - NR^{11a}R^{11b}, cyano, C1-C6 alkyl, C3-C6 cycloalkyl or C1-C6 alkoxy;
   R² is 3-10 membered cycloalkyl, 3-10 membered heterocycloalkyl with 1-3 heteroatoms, 5-10 membered heteroaryl with 1-3 heteroatoms, or C6-12 aryl, wherein, the cycloalkyl, heterocycloalkyl, heteroaryl or aryl is optionally substituted by 1 to 5 R³, and if the heteroatom on the heterocycloalkyl or heteroaryl is N, then the N is optionally substituted by R4;
   R³ is independently deuterium, halogen, C1-C6 alkyl, C3-C6 cycloalkyl, C1-C6 alkoxy, oxo, -SR⁵,-NR^{11a} R^{11b}, cyano or -OR⁵, wherein the alkyl, cycloalkyl or alkoxy is optionally and independently substituted by 1 to 5 groups selected from deuterium, halogen, -OR⁵, - SR⁵, -NR^{11a}R^{11b}, cyano, C1-C6 alkyl, C3-C6 cycloalkyl or C1-C6 alkoxy; or two R₃ together with the carbon atom to which they are attached form 3-6-membered cycloalkyl or 4-6-membered heterocycloalkyl, wherein, the cycloalkyl or heterocycloalkyl is optionally substituted by 1-3 substituents selected from halogen, deuterium,-OR⁵, -SR⁵, -NR^{11a}R^{11b}, cyano, C1-C6 alkyl or C1-C6 alkoxy, wherein, the alkyl or alkoxy is optionally substituted by a substituent selected from halogen, deuterium, -OR⁵, -SR⁵, -NR^{11a}R^{11b}, or cyano; wherein, if the heteroatom on the heterocycloalkyl is N, then the N is optionally substituted by R⁴;
   R⁴ is hydrogen, deuterium, C1-C6 alkyl, -C(O)R¹⁰ or -S(O)₂R⁸, wherein, the alkyl is optionally substituted by OH, halogen, deuterium, C1-C6 alkyl, C1-C6 alkoxy or cyano;
   R⁵ is hydrogen or C1-C6 alkyl, wherein, the alkyl is optionally substituted by halogen, deuterium, C1-C6 alkoxy, C1-C6 alkothiyl, -NR^{11a}R^{11b}, cyano, C1-C6 alkyl or C3-C6 cycloalkyl;
   R⁶ is hydrogen, deuterium, halogen, cyano, -OR⁵, -SR⁵, -NR^{11a}R^{11b}, C1-C6 alkyl, C3-C6 cycloalkyl, 3-7-membered heterocycloalkyl, 5-6-membered heteroaryl or aryl, wherein, the alkyl, cycloalkyl, heterocycloalkyl, heteroaryl or aryl is optionally substituted by 1-3 substituents selected from halogen,-OR⁵,-SR⁵,-NR^{11a}R^{11b}, cyano, C1-C3 alkyl, -C(O)R¹⁰ or oxo;
   R⁷ is independently hydrogen, deuterium, methyl, cyano, OCF₃, OMe, CF₃ or halogen;
   R⁸ is independently C1-6 alkyl, 3-6-membered cycloalkyl, 4-6-membered heterocycloalkyl, C6 -10 aryl, or 5-10-membered heteroaryl; wherein, the alkyl, cycloalkyl, heterocycloalkyl, aryl or heteroaryl is optionally substituted by 1-3 substituents selected from deuterium, halogen, OH, C1-C6 alkoxy, C1-C3 alkyl optionally substituted by -NR^{11a}R^{11b} or C1-C6 alkoxy, 3-6-membered cycloalkyl, -NR^{11a}R^{11b}, or cyano;
   R¹⁰ is C1-C6 alkyl, 3-6-membered cycloalkyl, 4-6-membered heterocycloalkyl, 6-10 membered aryl or 5-10-membered heteroaryl; wherein, the alkyl, cycloalkyl, heterocycloalkyl, aryl or heteroaryl is optionally substituted by one to three substituents selected from deuterium, halogen, OH, C1-C6 alkoxy, C1-C3 alkyl optionally substituted by -NR^{11a}R^{11b} or C1-C6 alkoxy, 3-6-membered cycloalkyl, -NR^{11a}R^{11b}, or cyano; and
   R^{11a} and R^{11b} are each independently hydrogen, deuterium, 3-6-membered cycloalkyl or C1-C6 alkyl, wherein, the cycloalkyl or alkyl is optionally substituted by a substituent selected from deuterium, C1-C6 alkoxy or cyano; if the alkyl is C2-6 alkyl, the alkyl is optionally substituted by deuterium, C1-C6 alkoxy, cyano, halogen or OH; or
   in some embodiments of the present invention, the PTM has the following structure: 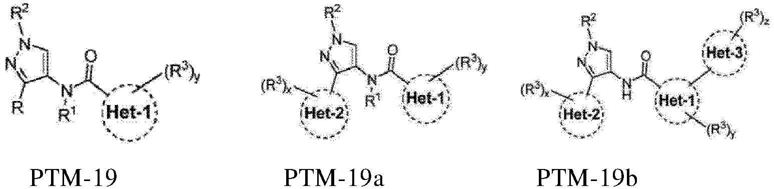 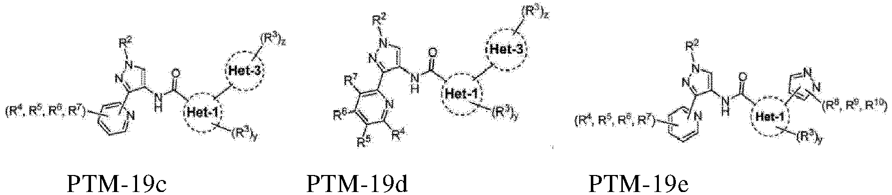 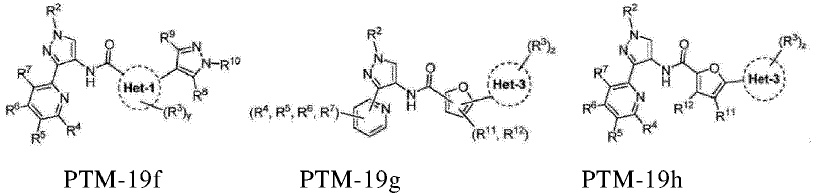 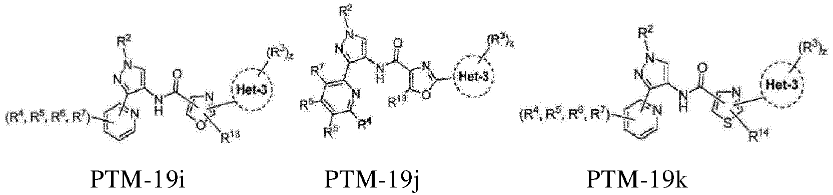 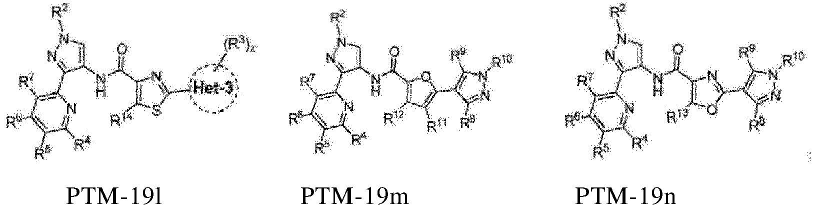 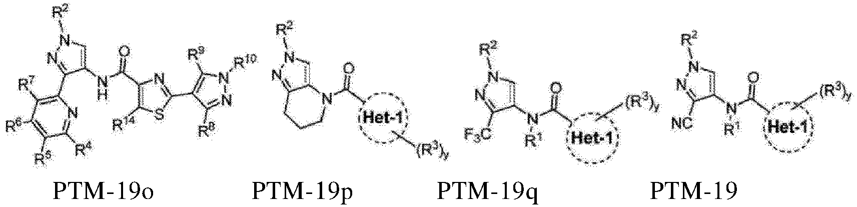 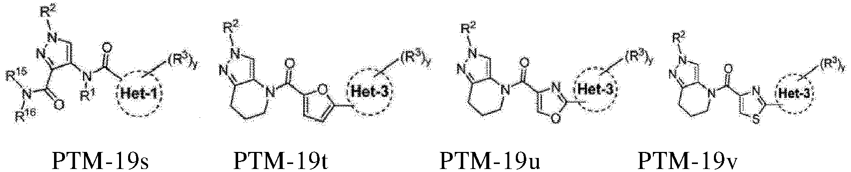 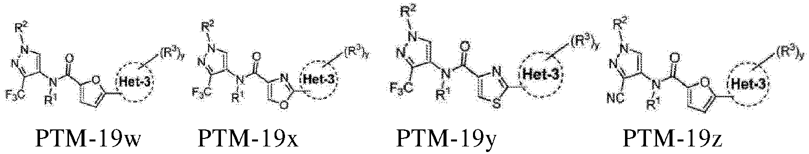 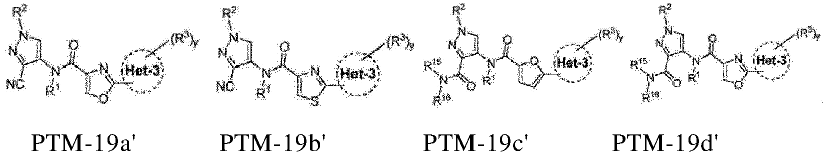
or a pharmaceutically acceptable salt thereof; wherein, in PTM-19, PTM-19a, PTM-19b, PTM-19c, PTM-19d, PTM-19e, PTM-19f, PTM-19g, PTM-19h, PTM-19i, PTM-19j, PTM-19k, PTM-191, PTM-19m, PTM-19n, PTM-19o, PTM-19p, PTM-19q, PTM-19r, PTM-19s, PTM-19t, PTM-19u, PTM-19v, PTM-19w, PTM-19x, PTM-19y, PTM-19z, PTM-19a', PTM-19b', PTM-19c', or PTM-19d':
   R is alkyl, heteroalkyl, heteroaryl, aryl, halogen, amide or CN;
   R¹ is H, alkyl, heteroalkyl; or R and R¹ together with the atom to which they are attached form a heterocycloalkyl;
   R² is H, aliphatic, alkyl, heteroalkyl, aryl, amide or heterocycloalkyl;
   R³ is independently H, alkyl, halogen, heteroalkyl,-O-alkyl, heterocycloalkyl, aryl,-O-heterocycloalkyl, hydroxyl, nitro, cyano, carboxylic acid, ester, acyl, amide, amino, sulfonyl, sulfonamide, alkyl-S-, sulfinyl, haloalkyl, alkyl phosphate, or alkyl phosphonate;
   R⁴, R⁵, R⁶, and R⁷ are each independently H, alkyl, heteroalkyl, alkoxy, heterocycloalkyl, aryl, O-heterocycloalkyl, hydroxyl, haloalkyl, halogen, nitro, cyano, carboxyl, ester, acyl, amide, amino, sulfonyl, sulfonamide, alkyl-S-, sulfinyl;
   R⁸ and R⁹ are each independently H, alkyl, heteroalkyl, aryl, heterocycloalkyl, sulfonyl, nitro, halogen, haloalkyl, ester, cyano or amino;
   R¹⁰ is H, alkyl, heteroalkyl, alkoxy, ester, aryl, nitro, cyano, hydroxyl, haloalkyl, alkyl phosphate, or alkyl phosphonate;
   R¹¹ , R¹² , R¹³ and R¹⁴ are each independently H, alkyl, heteroalkyl, aryl, heterocycloalkyl, sulfonyl, nitro, halogen, haloalkyl, ester, cyano or amino;
   R¹⁵ and R¹⁶ are each independently H, alkyl, heteroalkyl, aryl, heterocycloalkyl, or form heterocycloalkyl together with the N atom to which they are attached;
   x, y, and z are each independently integers from 1 to 6; and
   Het-1 and Het-2 are each independently heteroaryl;
   Het-3 is heterocycloalkyl; or
   in some embodiments of the present invention, the PTM has the following structure: 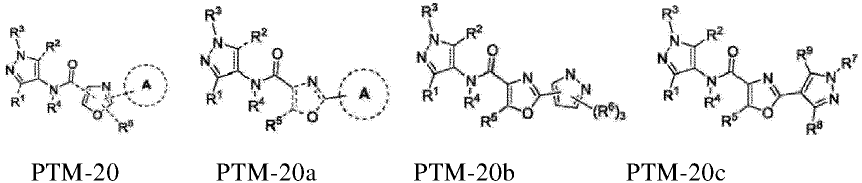
or pharmaceutically acceptable salt thereof, wherein, in PTM-20, PTM-20a, PTM-20b or PTM-20c:
   at least one of R¹ and R² is aromatic, and the rest of R¹ or R² is H, alkyl, haloalkyl, nitro, cyano, amide, amino, hydroxyl, carboxyl, carboxyl ester or acyl;
   R³ is H, alkyl, heteroalkyl, heterocycloalkyl, amide, aromatic or aromatic aliphatic;
   R⁴ is H, alkyl, heteroalkyl, or one of R¹ and R² forms heterocycloalkyl with R⁴ and the atoms to which they are attached;
   R⁵ is H or alkyl;
   R⁶, R⁷, R⁸, and R⁹ are H, alkyl, heteroalkyl, alkoxy, ester, sulfonyl, halogen, acyl, amino, aryl, heterocycloalkyl, nitro, cyano, hydroxyl, haloalkyl, alkyl phosphate, or alkyl phosphonate; and
   ring A is heterocycloalkyl; or
   in some embodiments of the present invention, the PTM has the following structure: 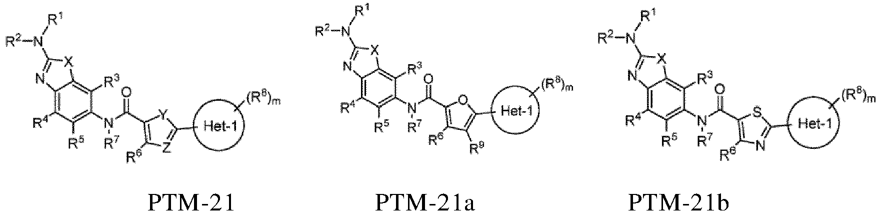
or pharmaceutically acceptable salt, solvate, hydrate, N-oxide or prodrug thereof; wherein, the PTM is PTM-21, PTM-21a or PTM-21b:
   X is O or S;
   Y is O or S;
   Z is N or CR⁹;
   Het-1 is heteroaryl;
   R¹ and R² are independently H, alkyl, cycloalkyl, heteroalkyl, heterocycloalkyl, aryl, or form heterocycloalkyl together with the nitrogen to which they are attached;
   R³, R⁴, R⁵, R⁶, and R⁹ are independently H, cycloalkyl, alkyl, halogen, heteroalkyl,-O-alkyl, heterocycloalkyl, aryl,-O-heterocycloalkyl, hydroxyl, nitro, cyano, carboxyl, ester, acyl, amide, amino, sulfonyl, sulfonamide, alkyl-S-, sulfinyl or haloalkyl;
   R⁷ is H, alkyl, heteroalkyl, heterocycloalkyl, or aryl;
   R⁸ is independently alkyl, halogen, heteroalkyl,-O-alkyl, heterocycloalkyl, aryl,-O-heterocycloalkyl, hydroxyl, nitro, cyano, carboxyl, ester, acyl, amide, amino, sulfonyl, sulfonamide, alkyl-S-, sulfinyl or haloalkyl; and
   m is from 0 to a possible number of substitution on Het-1; or
   in some embodiments of the present invention, the PTM has the following structure: 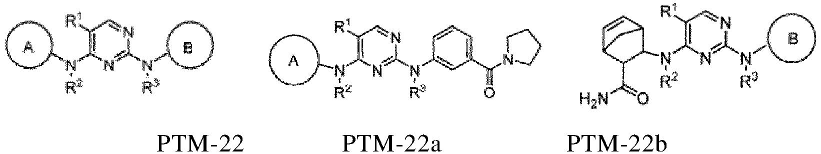 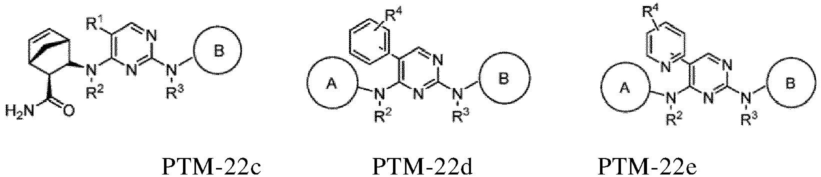 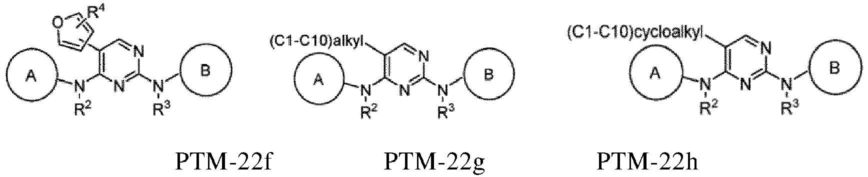
or pharmaceutically acceptable salt thereof, wherein, in PTM-22, PTM-22a, PTM-22b, PTM-22c, PTM-22d, PTM-22e, PTM-22g or PTM-22h:
   ring A is cycloalkyl;
   ring B is selected from aryl or heteroaryl;
   R¹ is selected from C1-10 alkyl, C3-C10 cycloalkyl, halogen, aryl or heteroaryl;
   R² and R³ are independently selected from hydrogen or C1-C6 alkyl;
   R⁴ is C1-C6 alkyl, cyano, halogen or hydrogen; or
   in some embodiments of the present invention, the PTM has the following structure: 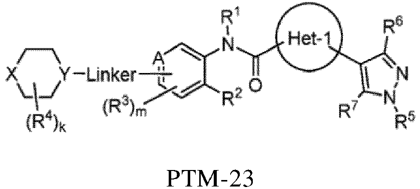
or pharmaceutically acceptable salt, solvate, hydrate, N-oxide or prodrug thereof; wherein, in PTM-23:
   Het-1 is heteroaryl;
   R¹ is hydrogen or alkyl;
   R² is alkoxy, or -N(R^{c})₂;
   R³ is C1-C6 alkyl, C1-C3 haloalkyl, or halogen;
   m is 0, 1 or 2;
   R⁴ is C1-C6 alkyl;
   k is 0, 1, 2, 3, 4, 5, 6, 7, 8 or 9;
   A is N or CR^{h};
   R^{h} is hydrogen, deuterium, R³, or 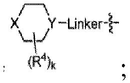
   R⁵ is hydrogen, deuterium, alkyl, phosphonoxyalkyl, phosphonoalkyl or acyl;
   R⁶ and R⁷ are each independently hydrogen, deuterium, alkyl or halogen;
   X is O or NR⁹;
   R⁹ is R^{a}, C(O)C1-C6 alkyl, C(O)N(R^{c})₂, C(O)OR^{a};
   Y is N or CH;
   linker is bond, -(C(R¹⁰)₂)ₙ-O-,-C(O)-(C(R¹⁰)₂)ₚ-, or (C(R¹⁰)₂)ₚ-N(R^{a})-;
   R¹⁰ is R^{a} or R^{b};
   n is 1, 2, 3, 4, 5 or 6;
   p is 0, 1, or 2;
   R^{a} is hydrogen, deuterium, D, C1-C6 alkyl, or C3-C6 cycloalkyl;
   R^{b} is hydroxyl, -OR^{a}, or halogen;
   R^{c} is R^{a}, or two R^{c} together with its attached nitrogen atoms to form a C3-7 heterocycloalkyl, and the heterocycloalkyl optionally additionally includes one or two heteroatoms selected from N, S or O; or
   in some embodiments of the present invention, the PTM has the following structure: 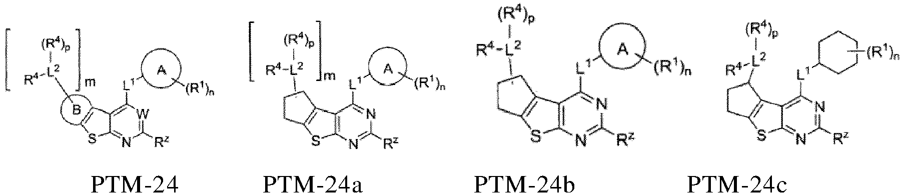 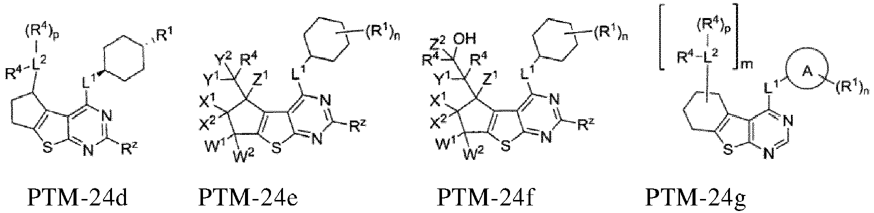 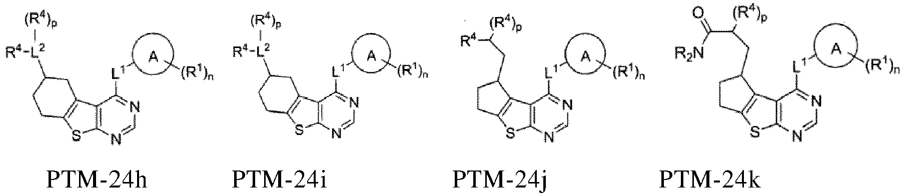 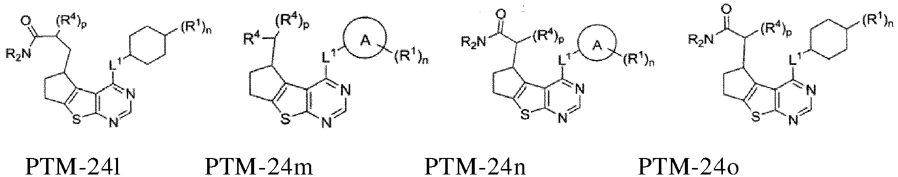 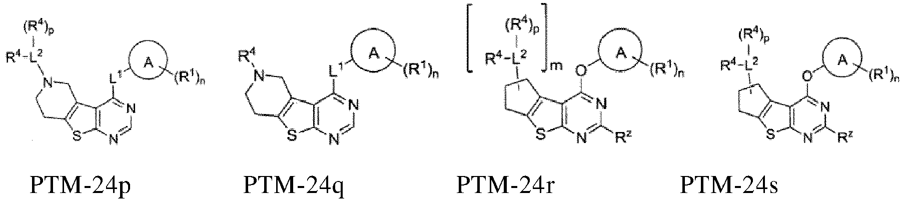 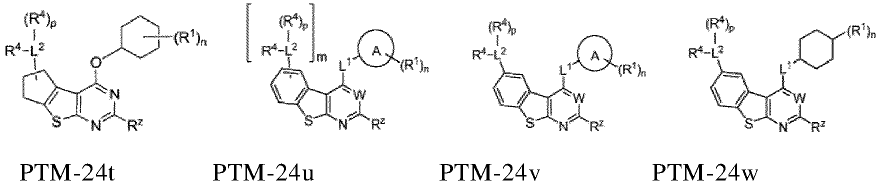 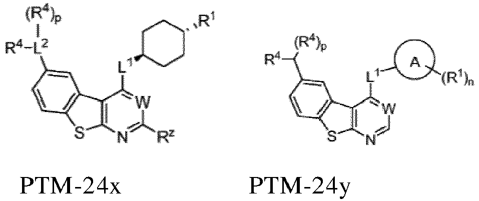
or a pharmaceutically acceptable salt thereof; wherein, in PTM-24, PTM-24a, PTM-24b, PTM-24c, PTM-24d, PTM-24e, PTM-24f, PTM-24g, PTM-24h, PTM-24i, PTM-24j, PTM-24k, PTM-241, PTM-24m, PTM-24n, PTM-24o, PTM-24p, PTM-24q, PTM-24r, PTM-24s, PTM-24t, PTM-24u, PTM-24v, PTM-24w, PTM-24x or PTM-24y:
   ring A is a 3-7-membered saturated or partially unsaturated carbocycle or a 4-7-membered saturated or partially unsaturated heterocycloalkyl containing 1-3 heteroatoms independently selected from nitrogen, oxygen or sulfur;
   n is an integer from 0 to 4;
   R¹ is independently -R, halogen, -CN, -NO₂, -OR, -CH₂OR, -SR, -N(R)₂, -SO₂R, - SO₂N(R)₂, -SOR, -C(O)R, -CO2R, -C(O)N(R)₂, -C(O)N(R)-OR, -NRC(O)OR, - NRC(O)N(R)₂, Cy or -NRSO₂R; or R¹ is selected from one of the following formulas: 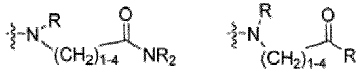
   or two R¹ together with the atoms to which they are attached form an optionally substituted 4-7 membered fused, spiro fused or bridged bicyclyl containing 0-2 heteroatoms independently selected from nitrogen, oxygen or sulfur;
   each Cy is selected from the following optionally substituted rings: 3 -7-membered saturated or partially unsaturated carbocycle or 4-7-membered saturated or partially unsaturated heterocyclyl containing 1-3 heteroatoms independently selected from nitrogen, oxygen or sulfur;
   each R is independently hydrogen or selected from the following optionally substituted groups: C1-C6 alkyl, phenyl, 4-7-membered saturated or partially unsaturated heterocycle containing 1-2 heteroatoms independently selected from nitrogen, oxygen or sulfur or 5-6-membered heteroaryl ring containing 1-4 heteroatoms independently selected from nitrogen, oxygen or sulfur, or two R together with the nitrogen atoms to which they are attached form a 4-7-membered saturated, partially unsaturated heterocyclic or heteroaryl ring containing 0-3 heteroatoms independently selected from nitrogen, oxygen or sulfur in addition to the nitrogen atom;
   ring B is a 4-8-membered partially unsaturated carbocyclic fused ring, a benzo fused ring, or a 4-7-membered partially unsaturated heterocyclic fused ring containing 1-2 heteroatoms selected from nitrogen, oxygen or sulfur, 5-6 membered heteroaromatic fused rings with 1-3 heteroatoms independently selected from nitrogen, oxygen or sulfur, wherein, the ring B can optionally be substituted by one or more selected from oxo, thiocarbonyl or imino;
   m is 1 to 4;
   p is 0 to 2;
   W is N or -C(R³)-;
   R^{z} is R, CN, NO₂, halogen, -C(O)N(R)₂, -C(O)OR, -C(O)R, -N(R)₂, -NH-[Ar], - N(R)C(O)OR, -NRC(O)N(R)₂, -OR or -SO₂N(R)₂;
   [Ar] is an optionally substituted phenyl or heteroaromatic ring;
   R³ is hydrogen, deuterium, halogen, -CN, C1-C4 aliphatic group, C1-C4 halogen aliphatic group, -OR,-C(O)R or -C(O)N(R)₂;
   L¹ is a bond or C1-C6 divalent hydrocarbon chain, wherein one or two methylene units of the chain are optionally and independently replaced by -NR-, -N(R)C(O)-, -C(O)N(R)-, - N(R)SO₂-, -SO₂N(R)-, -O-, -C(O)-, -OC(O)-, -C(O)-, -C(O) O-, -S-, -SO- or -SO2-;
   each L² is independently a bond or C1-C6 divalent hydrocarbon chain, wherein one or two methylene units of the chain are optionally and independently replaced by -NR-, - N(R)C(O)-, -C(O)N(R)-, -N(R)SO₂-, -SO₂N(R)-, -O-, -C(O)-, -OC(O)-, -C(O) O-, -S-, -SO- or -SO₂-;
   each R⁴ is independently halogen, -CN, -NO₂, -OR, -SR, -N(R)₂, -SO₂R, -SO₂N(R)₂, - SOR, -C(O)R, -CO₂R, -C(O)N(R)₂, -NRC(O)R, -NRC(O)N(R)₂, -C(O)N(R)OR, - N(R)C(O)OR, -N(R)S(O)₂N(R)₂, -NRSO₂R or selected from the following optionally substituted groups: C1-C6 aliphatic group, phenyl, 4-7-membered saturated or partially unsaturated heterocyclyl containing 1-2 heteroatoms independently selected from nitrogen, oxygen or sulfur, or 5-6-membered heteroaryl containing 1-4 heteroatoms independently selected from nitrogen, oxygen or sulfur, or two -L²(R⁴)p-R⁴groups together with the atoms to which they are attached form an optionally substituted 4-7 membered fused, spiro-fused or bridged bicyclyl containing 0-2 heteroatoms independently selected from nitrogen, oxygen or sulfur; or
   in some embodiments of the present invention, the PTM has the following structure: 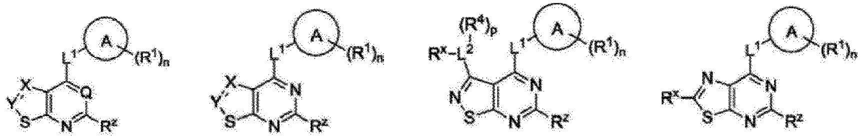 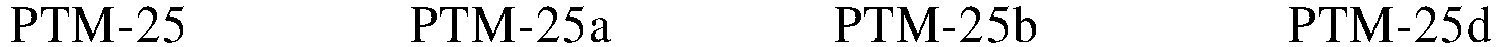 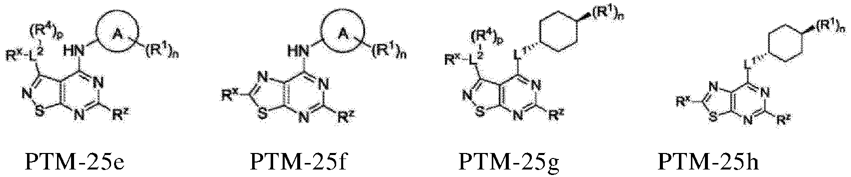
or pharmaceutically acceptable salt thereof, wherein, in PTM-25, PTM-25a, PTM-25b, PTM-25c, PTM-25d, PTM-25e, PTM-25f, PTM-25g or PTM-25h:
   Q is CH, C- CN or N;
   X is C- L²(R⁴)ₚR^{x} and Y is N; or X is N and Y is CR^{x};
   ring A is a 3-7-membered saturated or partially unsaturated carbocycle or a 4-7-membered saturated or partially unsaturated heterocycloalkyl containing 1-3 heteroatoms independently selected from nitrogen, oxygen or sulfur;
   each R¹ and R^{1'} are independently -R², halogen, -CN, -NO₂, -OR, -SR, -N(R)₂, -S(O)₂R, -S(O)₂N(R)₂, -S(O)R, -C(O)R, -C(O)OR, -C(O)N(R)₂, -C(O)N(R)OR, -N(R)C(O)OR, - N(R)C(O)N(R)₂, Cy or -N(R)S(O)₂R;
   or R¹ is selected from one of the following formulas: 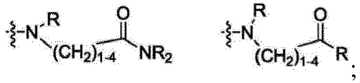 or
   two R¹ groups together with the atoms to which they are attached form an optionally substituted 4-7-membered fused, spiro-fused or bridged bicyclyl containing 0-2 heteroatoms independently selected from nitrogen, oxygen or sulfur;
   each Cy is an optionally substituted ring selected from a 3-7-membered saturated or partially unsaturated carbocycle or a 4-10-membered saturated or partially unsaturated heterocycle containing 1-3 heteroatoms independently selected from nitrogen, oxygen or sulfur;
   each R is independently hydrogen, or an optionally substituted group selected from the following: C1-C6 alkyl, phenyl, 4-7-membered saturated or partially unsaturated heterocycles containing 1-2 heteroatoms independently selected from nitrogen, oxygen or sulfur, or 5-6-membered heteroaromatic rings containing 1-4 heteroatoms independently selected from nitrogen, oxygen or sulfur, or two R together with the nitrogen atom to which they are attached form a 4-7-membered saturated, partially unsaturated heterocyclic or heteroaromatic ring containing 0-3 heteroatoms independently selected from nitrogen, oxygen or sulfur in addition to the nitrogen atom;
   each R² is independently an optionally substituted group selected from the following: C1-C6 alkyl, phenyl, 4-7-membered saturated or partially unsaturated heterocycle containing 1-2 heteroatoms independently selected from nitrogen, oxygen or sulfur, or 5-6-membered heteroaromatic ring containing 1-4 heteroatoms independently selected from nitrogen, oxygen or sulfur;
   each R⁴ is independently halogen, -CN, -NO₂, -OR, -SR, -N(R)₂, -S(O)₂R, -S(O)₂N(R)₂, -S(O)R, -C(O)R, -C(O)OR, -C(O)N(R)₂, -N(R)C(O)R, -N(R)C(O)N(R)₂, -C(O)N(R)OR, - N(R)C(O)OR, -N(R)S(O)₂N(R)₂, -N(R)S(O)₂R or an optionally substituted group selected from the following: C1-C6 alkyl, phenyl, 4-7-membered saturated or partially unsaturated heterocyclyl containing 1-2 heteroatoms independently selected from nitrogen, oxygen or sulfur, or 5-6-membered heteroaryl containing 1-4 heteroatoms independently selected from nitrogen, oxygen or sulfur;
   R^{x} is hydrogen, deuterium,-R², -CN, -NO₂, halogen, -C(O)N(R)₂, -C(O)OR, -C(O)R, - N(R)₂, -NH[Ar], -OR or -S(O)₂N(R)₂;
   R^{z} is hydrogen, deuterium,-R2, -CN, -NO₂, halogen, -C(O)N(R)₂, -C(O)OR, -C(O)R, - N(R)₂, -NH[Ar], -OR or -S(O)₂N(R)₂;
   [Ar] is a phenyl or heteroaromatic ring substituted by m R^{1'};
   L¹ is a covalent bond or C1-C6 divalent hydrocarbon chain, wherein one or two methylene units of the chain are optionally and independently replaced by -N(R)-, - N(R)C(O)-, -C(O)N(R)-, -N(R)S(O)₂-, -S(O)₂N(R)-, -O-, -C(O)-, -OC(O)-, -C(O)O-, -S-, - S(O)- or -S(O)₂-;
   L² is a covalent bond or C1-C6 divalent hydrocarbon chain, wherein one or two methylene units of the chain are optionally and independently replaced by -N(R)-, - N(R)C(O)-, -C(O)N(R)-, -N(R)S(O)₂-, -S(O)₂N(R)-, -O-, -C(O)-, -OC(O)-, -C(O)O-, -S-, - S(O)- or -S(O)₂-;
   m is an integer of 0-4;
   n is an integer of 0-4; and
   p is an integer of 0-2; or
   in some embodiments of the present invention, the PTM has the following structure: 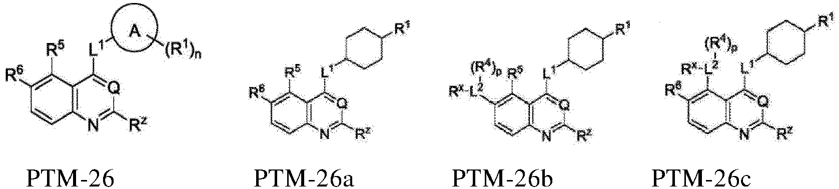 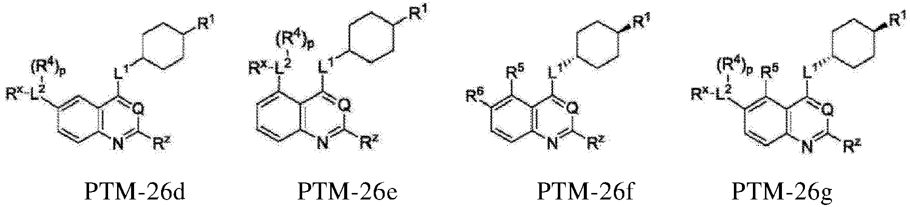 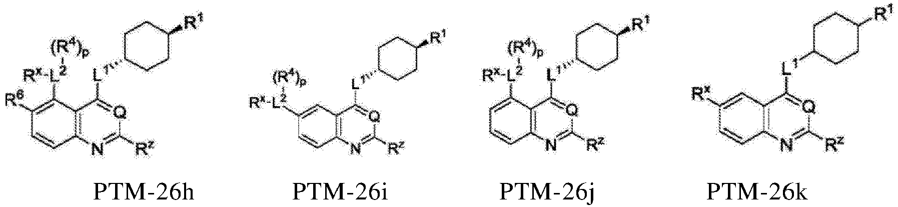 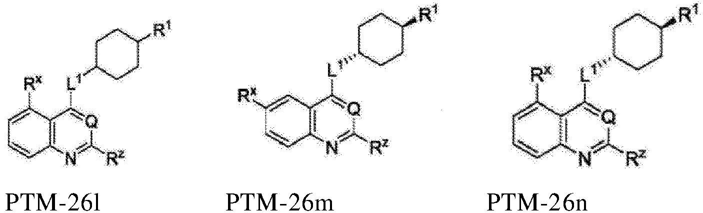
or pharmaceutically acceptable salt thereof; wherein, in PTM-26, PTM-26a, PTM-26b, PTM-26c, PTM-26d, PTM-26e, PTM-26f, PTM-26g, PTM-26h, PTM-26i, PTM-26g, PTM-26k, PTM-261, PTM-26m or PTM-26n:
   Q is CH, or N;
   ring A is a 3-7-membered saturated or partially unsaturated carbocycle or a 4-7-membered saturated or partially unsaturated heterocycloalkyl containing 1-3 heteroatoms independently selected from nitrogen, oxygen or sulfur;
   each R¹ is independently -R², halogen, -CN, -NO₂, -OR, -SR, -N(R)₂, -S(O)₂R, - S(O)₂N(R)₂, -S(O)R, -C(O)R, -C(O)OR, -C(O)N(R)₂, -C(O)N(R)OR, -N(R)C(O)OR, - N(R)C(O)N(R)₂, Cy or -N(R)S(O)₂R;
   or R1 is selected from one of the following formulas: 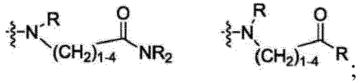 or
   two R¹ groups together with the atoms to which they are attached form an optionally substituted 4-7-membered fused, spiro-fused or bridged bicyclyl containing 0-2 heteroatoms independently selected from nitrogen, oxygen or sulfur;
   each Cy is an optionally substituted ring selected from a 3-7-membered saturated or partially unsaturated carbocycle or a 4-10-membered saturated or partially unsaturated heterocycle containing 1-3 heteroatoms independently selected from nitrogen, oxygen or sulfur;
   each R is independently hydrogen, or an optionally substituted group selected from the following: C1-C6 alkyl, phenyl,4-7-membered saturated or partially unsaturated heterocycles containing 1-2 heteroatoms independently selected from nitrogen, oxygen or sulfur, or 5-6-membered heteroaromatic rings containing 1-4 heteroatoms independently selected from nitrogen, oxygen or sulfur, or two R together with the nitrogen atom to which they are attached form a 4-7-membered saturated, partially unsaturated heterocyclic or heteroaromatic ring containing 0-3 heteroatoms independently selected from nitrogen, oxygen or sulfur in addition to the nitrogen atom;
   each R² is an optionally substituted group independently selected from the following: C1-C6 alkyl, phenyl, 4-7-membered saturated or partially unsaturated heterocycle containing 1-2 heteroatoms independently selected from nitrogen, oxygen or sulfur, or 5-6-membered heteroaromatic ring containing 1-4 heteroatoms independently selected from nitrogen, oxygen or sulfur;
   each of R⁵ and R⁶ is independently hydrogen or -L²(R⁴⁾ₚR^{x}; or
   R⁵ and R⁶ together with the attached atoms form a 4-7-membered partially unsaturated heterocyclic or aromatic ring containing 0-3 heteroatoms independently selected from nitrogen, oxygen or sulfur;
   each R⁴ is independently halogen, -CN, -NO₂, -OR, -SR, -N(R)₂, -S(O)₂R, -S(O)₂N(R)₂, -S(O)R, -C(O)R, -C(O)OR, -C(O)N(R)₂, -N(R)C(O)R, -N(R)C(O)N(R)₂, -C(O)N(R)OR, - N(R)C(O)OR, -N(R)S(O)₂N(R)₂, -N(R)S(O)₂R or an optionally substituted group selected from the following: C1-C6 aliphatic, phenyl, 4-7-membered saturated or partially unsaturated heterocycle containing 1-2 heteroatoms independently selected from nitrogen, oxygen or sulfur, or 5-6-membered heteroaryl ring containing 1-4 heteroatoms independently selected from nitrogen, oxygen or sulfur;
   R^{x} is hydrogen, deuterium, -R², -CN, -NO₂, halogen, -C(O)N(R)₂, -C(O)OR, -C(O)R, - N(R)₂, -NH[Ar], -OR or -S(O)₂N(R)₂;
   R^{z} is hydrogen, deuterium, -R², -CN, -NO₂, halogen, -C(O)N(R)₂, -C(O)OR, -C(O)R, - N(R)₂, -NH[Ar], -OR or -S(O)₂N(R)₂;
   [Ar] is an optionally substituted phenyl, or a 5-6-membered heteroaryl ring containing 1-4 heteroatoms independently selected from N, O, or S;
   L¹ is a covalent bond or C1-C6 divalent hydrocarbon chain, wherein one or two methylene units of the chain are optionally and independently replaced by -N(R)-, - N(R)C(O)-, -C(O)N(R)-, -N(R)S(O)2-, -S(O)2N(R)-, -O-, -C(O)-, -OC(O)-, -C(O)O-, -S-, - S(O)- or -S(O)₂-;
   L² is a covalent bond or C1-C6 divalent hydrocarbon chain, wherein one or two methylene units of the chain are optionally and independently replaced by -N(R)-, - N(R)C(O)-, -C(O)N(R)-, -N(R)S(O)₂-, -S(O)₂N(R)-, -O-, -C(O)-, -OC(O)-, -C(O)O-, -S-, - S(O)- or -S(O)₂-;
   m is an integer of 0-4;
   n is an integer of 0-4; and
   p is an integer of 0-2; or
   in some embodiments of the present invention, the PTM has the following structure: 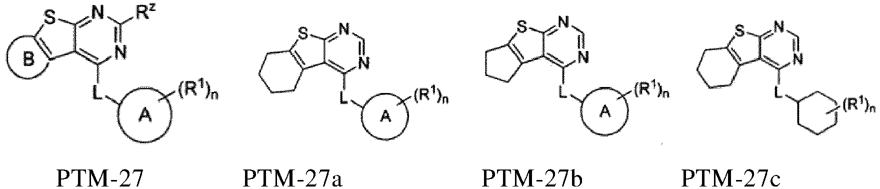 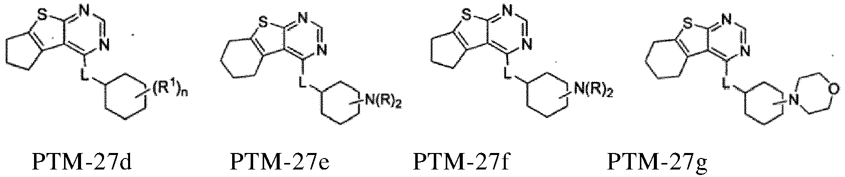 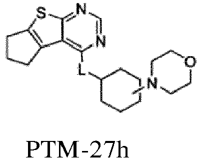
or a pharmaceutically acceptable salt thereof; wherein, in PTM-27, PTM-27a, PTM-27b, PTM-27c, PTM-27d, PTM-27e, PTM-27f, PTM-27g or PTM-27h:
   ring A is a 3-7-membered saturated or partially unsaturated carbocycle or a 4-7-membered saturated or partially unsaturated heterocycloalkyl containing 1-3 heteroatoms independently selected from nitrogen, oxygen or sulfur;
   each R¹ is independently -R, halogen, -CN, -NO₂, -OR, -SR, -N(R), -S(O)₂R, - S(O)₂N(R)₂, -S(O)R, -C(O)R, -C(O)OR, -C(O)N(R)₂, -C(O)N(R)OR, -N(R)C(O)OR, - N(R)C(O)N(R)₂, Cy or -N(R)S(O)₂R;
   or R¹ is selected from one of the following formulas: 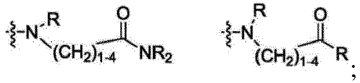 or
   two R¹ groups together with the atoms to which they are attached form an optionally substituted 4-7-membered fused, spiro-fused or bridged bicyclyl containing 0-2 heteroatoms independently selected from nitrogen, oxygen or sulfur;
   each Cy is an optionally substituted ring selected from a 3-7-membered saturated or partially unsaturated carbocycle or a 4-10-membered saturated or partially unsaturated heterocycle containing 1-3 heteroatoms independently selected from nitrogen, oxygen or sulfur;
   each R is independently hydrogen, or an optionally substituted group selected from the following: C1-C6 alkyl, phenyl, 4-7-membered saturated or partially unsaturated heterocycles containing 1-2 heteroatoms independently selected from nitrogen, oxygen or sulfur, or 5-6-membered heteroaromatic rings containing 1-4 heteroatoms independently selected from nitrogen, oxygen or sulfur, or two R together with the nitrogen atom to which they are attached form a 4-7-membered saturated, partially unsaturated heterocyclic or heteroaromatic ring containing 0-3 heteroatoms independently selected from nitrogen, oxygen or sulfur in addition to the nitrogen atom;
   R' is -R, -CN, -NO₂, halogen, -C(O)N(R)₂, -C(O)OR, -C(O)R, -N(R)₂, -OR or - S(O)₂N(R)₂;
   ring B is a 4-8-membered unsubstituted partially unsaturated carbocyclic fused ring;
   L is a covalent bond or C1-C6 divalent hydrocarbon chain, wherein one or two methylene units of the chain are optionally and independently replaced by -N(R)-, - N(R)C(O)-, -C(O)N(R)-, -N(R)S(O)₂-, -S(O)₂N(R)-, -O-, -C(O)-, -OC(O)-, -C(O)O-, -S-, - S(O)- or -S(O)₂-;
   n is an integer of 0-4; or
   in some embodiments of the present invention, the PTM has the following structure: 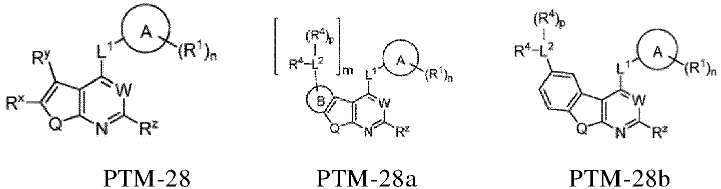 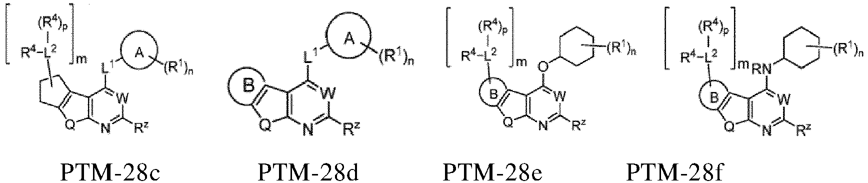
or an acceptable salt thereof; wherein, in PTM-28, PTM-28a, PTM-28b, PTM-28c, PTM-28d or PTM-28e:
   ring A is a 3-7-membered saturated or partially unsaturated carbocycle or a 4-7-membered saturated or partially unsaturated heterocycloalkyl containing 1-3 heteroatoms independently selected from nitrogen, oxygen or sulfur;
   n is 0 to 4;
   R¹ is independently -R, halogen, -CN, -NO₂, -OR, -CH₂OR, -SR, -N(R)₂, -SO₂R, - SO₂N(R)₂, -SOR, -C(O)R, -CO2R, -C(O)N(R)₂, -C(O)N(R)-OR, -NRC(O)OR, - NRC(O)N(R)₂, Cy or -NRSO₂R; or R¹ is selected from one of the following formulas: 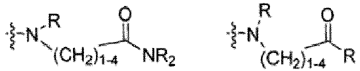
   or two R¹ together with the atoms to which they are attached form an optionally substituted 4-7 membered fused, spiro fused or bridged bicyclyl containing 0-2 heteroatoms independently selected from nitrogen, oxygen or sulfur;
   each Cy is selected from the following optionally substituted rings: 3 -7-membered saturated or partially unsaturated carbocycle or 4-7-membered saturated or partially unsaturated heterocyclyl containing 1-3 heteroatoms independently selected from nitrogen, oxygen or sulfur;
   each R is independently hydrogen or selected from the following optionally substituted groups: C1-C6 alkyl, phenyl, 4-7-membered saturated or partially unsaturated heterocycle containing 1-2 heteroatoms independently selected from nitrogen, oxygen or sulfur or 5-6-membered heteroaryl ring containing 1-4 heteroatoms independently selected from nitrogen, oxygen or sulfur, or two R together with the nitrogen atoms to which they are attached form a 4-7-membered saturated, partially unsaturated heterocyclic or heteroaryl ring containing 0-3 heteroatoms independently selected from nitrogen, oxygen or sulfur in addition to the nitrogen atom;
   R^{x} and R^{y} are each independently R, CN, NO₂, halogen, -SR, -OR, -N(R)₂, -SO₂R, - SO₂N(R)₂, -SOR, -C(O)R, -C(O)OR, -C(O)N(R)₂, -N(R)C(O)R, -NRC(O)N(R)₂, or - NRSO₂R;
   Rx and Ry together with the atoms to which they are attached form a ring B substituted by m 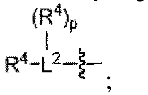
   the ring B is a 4-8-membered partially unsaturated carbocyclic fused ring, a benzo fused ring, or a 4-7-membered partially unsaturated heterocyclic fused ring containing 1-2 heteroatoms selected from nitrogen, oxygen or sulfur, 5-6 membered heteroaromatic fused rings containing 1-3 heteroatoms independently selected from nitrogen, oxygen or sulfur, wherein, the ring B can optionally be substituted by one or more selected from oxo, thiocarbonyl or imino;
   m is an integer from 0 to 4;
   p is an integer from 0 to 2;
   W is N or -C(R³)-;
   R^{z} is R, CN, NO₂, halogen, -C(O)N(R)₂, -C(O)OR, -C(O)R, -N(R)₂, -N(R)C(O)OR, - NRC(O)N(R)₂, -OR or -SO₂N(R)₂;
   R³ is hydrogen, deuterium, halogen, -CN, C1-C4 aliphatic group, C1-C4 halogen aliphatic group, -OR,-C(O)R or -C(O)N(R)₂;
   L¹ is bond or C1-C6 divalent hydrocarbon chain, wherein one or two methylene units of the chain are optionally and independently replaced by -NR-, -N(R)C(O)-, -C(O)N(R)-, - N(R)SO₂-, -SO₂N(R)-, -O-, -C(O)-, -OC(O)-, -C(O)-, -C(O) O-, -S-, -SO- or -SO2-;
   each L² is independently a covalent bond or C1-C6 divalent hydrocarbon chain, wherein one or two methylene units of the chain are optionally and independently replaced by -NR-, - N(R)C(O)-, -C(O)N(R)-, -N(R)SO₂-, -SO₂N(R)-, -O-, -C(O)-, -OC(O)-, -C(O) O-, -S-, -SO- or -SO₂-;
   each R⁴ is independently halogen, -CN, -NO₂, -OR, -SR, -N(R)₂, -SO₂R, -SO₂N(R)₂, - SOR, -C(O)R, -CO₂R, -C(O)N(R)₂, -NRC(O)R, -NRC(O)N(R)₂, -C(O)N(R)OR, - N(R)C(O)OR, -N(R)S(O)₂N(R)₂, -NRSO₂R or selected from the following optionally substituted groups: C1-C6 alkyl, phenyl, 4-7-membered saturated or partially unsaturated heterocyclyl containing 1-2 heteroatoms independently selected from nitrogen, oxygen or sulfur, or 5-6-membered heteroaryl containing 1-4 heteroatoms independently selected from nitrogen, oxygen or sulfur,
   or two -L²(R⁴)ₚR⁴ groups together with the atoms to which they are attached form an optionally substituted 4-7 membered fused, spiro fused or bridged bicyclyl containing 0-2 heteroatoms independently selected from nitrogen, oxygen or sulfur; or
   in some embodiments of the present invention, the PTM has the following structure: 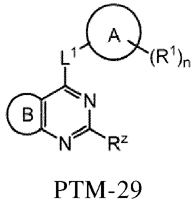
or pharmaceutically acceptable salt thereof; wherein, in PTM-29:
   ring A is a 3-7-membered saturated or partially unsaturated carbocycle or a 4-7-membered saturated or partially unsaturated heterocyclyl containing 1-3 heteroatoms independently selected from nitrogen, oxygen or sulfur;
   ring B is 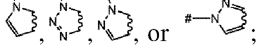
   wherein, 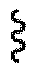 represents the ring portion fused to the pyrimidine ring, # is -L²(R⁴)_{P}-R^{X};
   R¹ and R^{1'} are independently -R², halogen, -CN, -NO₂, -OR, -CH₂OR, -SR, -N(R)₂, - SO₂R, -SO₂N(R)₂, -SOR, -C(O)R, -CO₂R, -C(O)N(R)₂, -C(O)N(R)-OR, -NRC(O)OR, - NRC(O)N(R)₂, Cy or -NRSO₂R; or R¹is selected from one of the following formulas: 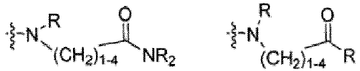
   or two R¹ together with the atoms to which they are attached form an optionally substituted 4-7 membered fused, spiro fused or bridged bicyclyl containing 0-2 heteroatoms independently selected from nitrogen, oxygen or sulfur;
   each Cy is selected from the following optionally substituted rings: 3 -7-membered saturated or partially unsaturated carbocycle or 4-10-membered saturated or partially unsaturated heterocyclyl containing 1-3 heteroatoms independently selected from nitrogen, oxygen or sulfur;
   each R is independently hydrogen or selected from the following optionally substituted groups: C1-C6 alkyl, phenyl, 4-7-membered saturated or partially unsaturated heterocycle containing 1-2 heteroatoms independently selected from nitrogen, oxygen or sulfur or 5-6-membered heteroaryl containing 1-4 heteroatoms independently selected from nitrogen, oxygen or sulfur, or
   two R together with the nitrogen atoms to which they are attached form a 4-7 membered saturated, partially unsaturated heterocyclic or heteroaryl ring containing 0-3 heteroatoms independently selected from nitrogen, oxygen or sulfur in addition to the nitrogen atom;
   each R² is an optionally substituted group independently selected from the following: C1-C6 alkyl, phenyl, 4-7-membered saturated or partially unsaturated heterocycle containing 1-2 heteroatoms independently selected from nitrogen, oxygen or sulfur, or 5-6-membered heteroaromatic ring containing 1-4 heteroatoms independently selected from nitrogen, oxygen or sulfur;
   each R⁴ is independently halogen, -CN, -NO₂, -OR, -SR, -N(R)₂, -S(O)₂R, -S(O)₂N(R)₂, -S(O)R, -C(O)R, -C(O)OR, -C(O)N(R)₂, -N(R)C(O)R, -N(R)C(O)N(R)₂, -C(O)N(R)OR, - N(R)C(O)OR, -N(R)S(O)₂N(R)₂, -N(R)S(O)₂R or an optionally substituted group selected from the following: C1-C6 aliphatic, phenyl, 4-7-membered saturated or partially unsaturated heterocycle containing 1-2 heteroatoms independently selected from nitrogen, oxygen or sulfur, or 5-6-membered heteroaryl ring containing 1-4 heteroatoms independently selected from nitrogen, oxygen or sulfur;
   R^{x} is hydrogen, deuterium,-R²,-CN,-NO₂, halogen,-C(O)N(R)₂,-C(O)OR,-C(O)R,-N(R)₂,-NH[Ar], -OR or -S(O)₂N(R)₂;
   R^{z} is hydrogen, deuterium,-R2,-CN,-NO2, halogen,-C(O)N(R)2,-C(O)OR,-C(O)R,-N(R)2,-NH[Ar], -OR or -S(O)₂N(R)₂;
   [Ar] is a phenyl substituted by m R1' or a heteroaromatic ring containing 1-4 heteroatoms selected from N, O or S;
   L¹ is a covalent bond or C1-C6 divalent hydrocarbon chain, wherein one or two methylene units of the chain are optionally and independently replaced by -N(R)-,-N(R)C(O)-,-C(O)N(R)-,-N(R)S(O)2-,-S(O)2N(R)-,-O-,-C(O)-,-OC(O)-,-C(O)O-,-S-,-S(O)- or -S(O)2-;
   L² is a covalent bond or C1-C6 divalent hydrocarbon chain, wherein one or two methylene units of the chain are optionally and independently replaced by -N(R)-,-N(R)C(O)-,-C(O)N(R)-,-N(R)S(O)₂-,-S(O)₂N(R)-,-O-,-C(O)-,-OC(O)-,-C(O)O-,-S-,-S(O)- or -S(O)₂-;
   m is an integer of 0-4;
   n is an integer of 0-4; and
   p is an integer of 0-2; or
   in some embodiments of the present invention, the PTM has the following structure: 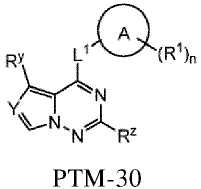
or a pharmaceutically acceptable salt thereof; wherein, in PTM-30:
   Y is N or CR^{x};
   ring A is a 3-7-membered saturated or partially unsaturated carbocycle or a 4-7-membered saturated or partially unsaturated heterocycloalkyl containing 1-3 heteroatoms independently selected from nitrogen, oxygen or sulfur;
   R¹ and R^{1'} are independently -R², halogen, -CN, -NO₂, -OR, -CH₂OR, -SR, -N(R)₂, - SO₂R, -SO₂N(R)₂, -SOR, -C(O)R, -CO₂R, -C(O)N(R)₂, -C(O)N(R)-OR, -NRC(O)OR, - NRC(O)N(R)₂, Cy or -NRSO₂R; or R¹ is selected from one of the following formulas: 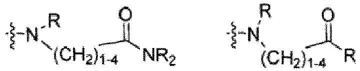
   or two R¹ together with the atoms to which they are attached form an optionally substituted 4-7 membered fused, spiro fused or bridged bicyclyl containing 0-2 heteroatoms independently selected from nitrogen, oxygen or sulfur;
   each Cy is selected from the following optionally substituted rings: 3 -7-membered saturated or partially unsaturated carbocycle or 4-10-membered saturated or partially unsaturated heterocyclyl containing 1-3 heteroatoms independently selected from nitrogen, oxygen or sulfur;
   each R is independently hydrogen or selected from the following optionally substituted groups: C1-C6 alkyl, phenyl, 4-7-membered saturated or partially unsaturated heterocycle containing 1-2 heteroatoms independently selected from nitrogen, oxygen or sulfur, or 5-6-membered heteroaryl ring containing 1-4 heteroatoms independently selected from nitrogen, oxygen or sulfur, or two R together with the nitrogen atoms to which they are attached form a 4-7-membered saturated, partially unsaturated heterocyclic or heteroaryl ring containing 0-3 heteroatoms independently selected from nitrogen, oxygen or sulfur in addition to the nitrogen atom;
   each R² is an optionally substituted group independently selected from the following: C1-C6 alkyl, phenyl, 4-7-membered saturated or partially unsaturated heterocycle containing 1-2 heteroatoms independently selected from nitrogen, oxygen or sulfur, or 5-6-membered heteroaromatic ring containing 1-4 heteroatoms independently selected from nitrogen, oxygen or sulfur;
   each R⁴ is independently halogen, -CN, -NO₂, -OR, -SR, -N(R)₂, -S(O)₂R, -S(O)₂N(R)₂, -S(O)R, -C(O)R, -C(O)OR, -C(O)N(R)₂, -N(R)C(O)R, -N(R)C(O)N(R)₂, -C(O)N(R)OR, - N(R)C(O)OR, -N(R)S(O)₂N(R)₂, -N(R)S(O)₂R or an optionally substituted group selected from the following: C1-C6 aliphatic, phenyl, 4-7-membered saturated or partially unsaturated heterocycle containing 1-2 heteroatoms independently selected from nitrogen, oxygen or sulfur, or 5-6-membered heteroaryl ring containing 1-4 heteroatoms independently selected from nitrogen, oxygen or sulfur;
   R^{x}, R^{y} and R^{z} are each independently hydrogen, deuterium,-R²,-CN,-NO₂, halogen,-C(O)N(R)₂,-C(O)OR,-C(O)R,-N(R)₂,-NH[Ar], -OR or -S(O)₂N(R)₂; or
   R^{x} and R^{y} together with the atoms to which they are attached form a 4-7-membered partially unsaturated carbocycle or a partially unsaturated heterocycloalkyl containing 1-3 heteroatoms selected from N, O or S;
   [Ar] is a phenyl substituted by m R^{1'} or a 5-6-membered heteroaryl containing 1-4 heteroatoms selected from N, O or S;
   L¹ is a covalent bond or C1-C6 divalent hydrocarbon chain, wherein one or two methylene units of the chain are optionally and independently replaced by -N(R)-,-N(R)C(O)-,-C(O)N(R)-,-N(R)S(O)₂-,-S(O)₂N(R)-,-O-,-C(O)-,-OC(O)-,-C(O)O-,-S-,-S(O)- or -S(O)₂-;
   m is an integer of 0-4;
   n is an integer of 0-4; or
   in some embodiments of the present invention, the PTM has the following structure: 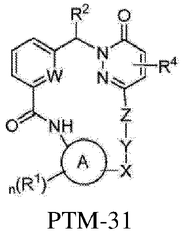
or a pharmaceutically acceptable salt thereof; in PTM-31:
   ring A is a 5-6-membered monocyclic heteroaromatic ring containing 1-4 heteroatoms independently selected from nitrogen, oxygen or sulfur; the heteroaromatic ring is optionally substituted;
   X is absent, or -O-, -S-, -SO₂-, -SO-, -C(O)-, -CO₂-, -C(O)N(R)-, -OC(O)N(R)-, - NRC(O)-, -NRC(O)N(R)-, -NRSO₂- or -N(R)-; or X is (CRR)ₘ-O-, -(CRR)ₘS-, - (CRR)ₘSO₂-, -(CRR)ₘSO-, -(CRR)ₘC(O)-, -(CRR)ₘCO₂-, -(CRR)ₘC(O)N(R)-, - (CRR)ₘOC(O)N(R)-, -(CRR)ₘNRC(O)-, -(CRR)ₘNRC(O)N(R)-, -(CRR)ₘNRSO₂- or - (CRR)ₘN(R)-;
   Y is an optionally substituted C1-6 alkyl;
   Z is absent, or is a divalent C₃₋₁₀ aryl, a divalent 3-8-membered saturated or partially unsaturated carbocycle, divalent 3-7-membered heterocyclyl containing 1-4 heteroatoms independently selected from nitrogen, oxygen or sulfur, or a divalent 5-6-membered monocyclic heteroaryl containing 1-4 heteroatoms independently selected from nitrogen, oxygen or sulfur; each of the above groups is optionally substituted; or Z is -O-, -S-, -SO₂-, - SO-, -C(O)-, -CO₂-, -C(O)N(R)-, -OC(O)N(R)-, -NRC(O)-, -NRC(O)N(R)-, -NRSO₂- or - N(R)-;
   W is CR or N;
   each R is independently hydrogen, deuterium, C1-6 alkyl, C3-C10 aryl, 3-8-membered saturated or partially unsaturated carbocycle, a 3-7-membered heterocyclyl containing 1-4 heteroatoms independently selected from nitrogen, oxygen or sulfur, or a 5-6-membered monocyclic heteroaromatic ring containing 1-4 heteroatoms independently selected from nitrogen, oxygen or sulfur; each of the above groups is optionally substituted; or two Rs on the same atom together with the atom to which they are attached form a C3-C10 aryl, a 3-8-membered saturated or partially unsaturated carbocycle, 3-7-membered heterocyclyl containing 1-4 heteroatoms independently selected from nitrogen, oxygen or sulfur, or a 5-6-membered monocyclic heteroaromatic ring containing 1-4 heteroatoms independently selected from nitrogen, oxygen or sulfur; each of the above groups is optionally substituted;
   each R¹ is independently -R, halogen, -OR, -SR, -CN, -NO₂, -SO₂R, -SOR, -C(O)R, - CO₂R, -C(O)N(R)₂, -NRC(O)R, -NRC(O)N(R)₂, -NRSO₂R or -N (R)₂;
   R² is -R, halogen, -OR, -SR, -CN, -NO₂, -SO₂R, -SOR, -C(O)R, -CO₂R, -C(O)N(R)₂, - NRC(O)R, -NRC(O)N(R)₂, -NRSO₂R or -N(R)₂;
   R⁴ is -R, halogen, -OR, -SR, -CN, -NO₂, -SO₂R, -SOR, -C(O)R, -CO₂R, -C(O)N(R)₂, - NRC(O)R, -NRC(O)N(R)₂, -NRSO₂R or -N(R)₂;
   each m is independently 1 or 2; and
   n is 0, 1, 2, 3, 4 or 5; or
   in some embodiments of the present invention, the PTM has the following structure: 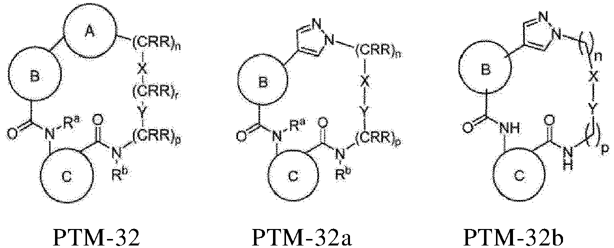
or a pharmaceutically acceptable salt thereof; in PTM-32, PTM-32a or PTM-32b:
   ring A is a 5-membered heterocyclyl containing 1-4 heteroatoms independently selected from nitrogen, oxygen or sulfur, or a 5-membered monocyclic heteroaromatic ring containing 1-4 heteroatoms independently selected from nitrogen, oxygen or sulfur; the heterocyclyl or heteroaromatic ring is optionally substituted;
   ring B is a 6-membered aryl, or a 6-membered monocyclic heteroaromatic ring containing 1-4 heteroatoms independently selected from nitrogen, oxygen or sulfur; the aryl or heteroaromatic ring is optionally substituted;
   ring C is a 5-membered heterocyclyl containing 1-4 heteroatoms independently selected from nitrogen, oxygen or sulfur, or a 5-membered monocyclic heteroaromatic ring containing 1-4 heteroatoms independently selected from nitrogen, oxygen or sulfur; the heterocyclyl or heteroaromatic ring is optionally substituted;
   X is absent, or -CH = CH-,-C≡C-, -O-, -S-, -SO₂-, -SO-, -C(O)-, -CO₂-, -C(O)N(R)-, - OC(O)N(R)-, -NRC(O)-, -NRC(O)N(R)-, -NRSO₂- or -N(R)-;
   Y is absent, or is a divalent C3-10 aryl, a divalent 3-8-membered saturated or partially unsaturated carbocycle, a divalent 3-7-membered heterocyclyl containing 1-4 heteroatoms independently selected from nitrogen, oxygen or sulfur, or a divalent 5 -6-membered monocyclic heteroaryl containing 1-4 heteroatoms independently selected from nitrogen, oxygen or sulfur; each of the above groups is optionally substituted;
   each R is independently hydrogen, C1-6 alkyl, C3-C10 aryl, 3-8-membered saturated or partially unsaturated carbocycle, 3-7-membered heterocyclyl containing 1-4 heteroatoms independently selected from nitrogen, oxygen or sulfur, or 5-6-membered monocyclic heteroaromatic ring containing 1-4 heteroatoms independently selected from nitrogen, oxygen or sulfur; each of the above groups is optionally substituted; or
   each R is independently -OR^{c}, -SR^{c}, -SO₂R^{c}, -SOR^{c}, -C(O)R^{c}, -CO₂R^{c}, -C(O)N(R)R^{c}, - OC(O)N(R)R^{c}, -NRC(O)R^{c}, -NRC(O)N(R)R^{c}, -NRSO₂R^{c}, or -N(R)R^{c};
   two R on the same atom together with the atom to which they are attached form a C3 - C10 aryl, a 3-8-membered saturated or partially unsaturated carbocycle, 3-7-membered heterocyclyl containing 1-4 heteroatoms independently selected from nitrogen, oxygen or sulfur, or 5-6-membered monocyclic heteroaromatic ring containing 1-4 heteroatoms independently selected from nitrogen, oxygen or sulfur; each of the above groups is optionally substituted;
   R^{a} is H or optionally substituted C1-6 alkyl;
   R^{b} is H or optionally substituted C1-6 alkyl;
   each R^{c} is independently H or optionally substituted C1-6 alkyl;
   n is 1, 2, 3, 4 or 5;
   p is 0, 1, 2, 3 or 4; and
   r is 0, 1, or 2; or
   in some embodiments of the present invention, the PTM has the following structure: 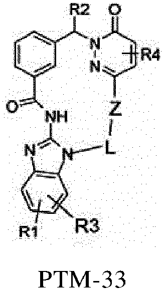
or pharmaceutically acceptable tautomers, solvates, salts and stereoisomers thereof, including mixtures thereof at all ratios; wherein, in PTM-33:
   R1 and R3 each independently refers to H, (CH₂)ₚCON(R5)₂, OA, Hal, COOH, COOA, (CH₂)ₚNHCOA, (CH₂)ₚHet1, (CH₂)ₚNR2R5 or OH;
   R2 refers to H or a linear or branched alkyl containing 1, 2 or 3 C atoms, wherein one or two H atoms of the alkyl can be substituted by OR6, NR5R6, NHCOR5, CONR5R6;
   R4 refers to H or A;
   R5 refers to H or a linear or branched alkyl containing 1, 2 or 3 C atoms;
   R6 refers to H or a linear or branched alkyl containing 1, 2 or 3 C atoms;
   Z is absent or divalent Ar or divalent Het;
   L refers to (CH₂)ₙ, wherein, one or two CH₂ groups can be replaced by O or CH=CH- , and/or one or two H atoms can be substituted by OR2, NR2R5 or Het1;
   divalent Ar refers to 1,2-, 1,3- or 1, 4-phenylene optionally substituted by 1 to 5 groups independently selected from Hal, CN,-CF₃,-OCF₃, OH, O-A, SO₂-A, COOH, COOA,-CO-A, O-phenyl, SO₂-phenyl, SO₂-CF₃, Het2 or A;
   divalent Het refers to an unsaturated, saturated or 5- or 6-membered heteroaryl containing 1 to 2 N, O or S atoms, it can be unsubstituted or monosubstituted, disubstituted or trisubstituted by Hal, CN,-CF₃,-OCF₃, O-A, SO₂-A, COOH, COOA,-CO-A, O-phenyl, SO₂-phenyl, SO₂-CF₃, Het2 or A;
   A refers to a linear or branched alkyl containing 1 to 10 C atoms, wherein 1 to 5 H atoms can be substituted by F or one or two non-adjacent CH₂ groups can be replaced by O;
   Het1 refers to morpholinyl, piperidinyl or pyrrolidinyl;
   Het2 refers to morpholinyl, piperidinyl or pyrrolidinyl;
   Hal refers to F, Cl, Br, I;
   n means 1, 2, 3, 4, 5 or 6;
   p means 0, 1 or 2; or
   in some embodiments of the present invention, the PTM has the following structure: 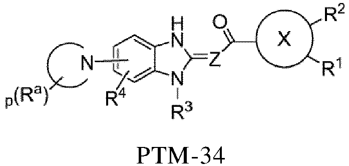
or pharmaceutically acceptable salt thereof; in PTM-34: 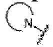
   is a 3-7-membered nitrogen-containing heterocycloalkyl or heteroaryl containing 0-2 additional heteroatoms independently selected from nitrogen, oxygen or sulfur;
   each R^{a} is independently -R, halogen, -haloalkyl, -OR, -SR, -CN, -NO₂, -SO₂R, -SOR, - C(O)R, -CO₂R, -C(O)N(R)₂, -NRC(O)R, -NRC(O)N(R)₂, -NRSO₂R or -N(R)₂;
   ring X is a C3-C10 aryl; 5-6-membered monocyclic heteroaryl containing 1-4 heteroatoms independently selected from nitrogen, oxygen or sulfur; fused C3-C10 aryl; fused 5-10-membered saturated or partially unsaturated cycloalkyl; fused 5-10-membered heterocycloalkyl containing 1-4 heteroatoms independently selected from nitrogen, oxygen or sulfur; or a fused 5-10-membered monocyclic heteroaryl containing 1-4 heteroatoms independently selected from nitrogen, oxygen or sulfur; each of the above groups is optionally substituted;
   R¹ is -R, halogen, -haloalkyl, -OR, -SR, -CN, -NO₂, -SO₂R, -SOR, -C(O)R, -CO₂R, - C(O)N(R)₂, -NRC(O)R, -NRC(O)N(R)₂, -NRSO₂R, or -N(R)₂;
   R² is -R, halogen, -haloalkyl, -OR, -SR, -CN, -NO₂, -SO₂R, -SOR, -C(O)R, -CO₂R, - C(O)N(R)₂, -NRC(O)R, -NRC(O)N(R)₂, -NRSO₂R or -N(R)₂;
   R³ is -R or -haloalkyl;
   R⁴ is -R, halogen, -haloalkyl, -OR, -SR, -CN, -NO₂, -SO₂R, -SOR, -C(O)R, -CO₂R, - C(O)N(R)₂, -NRC(O)R, -NRC(O)N(R)₂, -NRSO₂R, or -N(R)₂;
   Z is N or CR;
   each R is independently hydrogen, deuterium, C1-6 alkyl, C3-C10 aryl, 3-8-membered saturated or partially unsaturated carbocycle, a 3-7-membered heterocyclyl containing 1-4 heteroatoms independently selected from nitrogen, oxygen or sulfur, or a 5-6-membered monocyclic heteroaromatic ring containing 1-4 heteroatoms independently selected from nitrogen, oxygen or sulfur; each of the above groups is optionally substituted; or two Rs on the same atom together with the atom to which they are attached form a C3-C10 aryl, a 3-8-membered saturated or partially unsaturated carbocycle, 3-7-membered heterocyclyl containing 1-4 heteroatoms independently selected from nitrogen, oxygen or sulfur, or a 5-6-membered monocyclic heteroaromatic ring containing 1-4 heteroatoms independently selected from nitrogen, oxygen or sulfur; each of the above groups is optionally substituted; and
   p is 0, 1, 2, 3, 4 or 5;
   when ring X is phenyl, Z is N,R¹ is 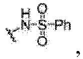 R² is H, R³ is n-propyl, and R⁴ is H, then 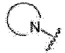 is not 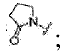 or
   in some embodiments of the present invention, the PTM has the following structure: 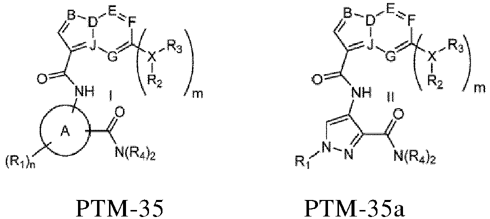
or pharmaceutically acceptable salt thereof, wherein, in PTM-35 or PTM-35a:
   B is CH, N or S; D is CH or N; E is CH or N; F is CH or N; G is CH or N; and J is C or N, wherein, when B is S, D is CH, E is N, F is CH, G is N, J is C;
   X is O, S, CH₂ or N;
   M is 0 or 1; n is 0, 1 or 2;
   ring A is pyridyl, pyrazolyl, thienyl, furanyl or phenyl,
   R₁ is independently selected from (C1-4) alkyl, pyrimidinyl, piperidinyl or phenyl, each of which is optionally substituted by (C1-4) alkyl, OH, halogen, O(C1-4) alkyl, methyl-piperidinyl, S(O)₂R_{c} substituted, C(O)N(Rb)₂, or C(O)O(C1-C4) alkyl;
   R₂ is absent or is H;
   R₃ is independently selected from: (C1-C4) alkyl, pyranyl, cyclopentyl, cyclohexyl, cycloheptyl, thiopyranyl, pyrazolyl, piperidinyl, morpholinyl, piperazinyl, each of which is optionally substituted by one or more independently selected from halogen, OH, oxo, N(Rb)₂, oxopyrrolidyl or morpholinyl;
   or R₂ and R₃ together with the nitrogen to which they are attached form piperazinyl or morpholinyl, each of which is optionally substituted by oxo;
   R₄ is independently H or methyl;
   R_{b} is independently selected from H and (C1-4) alkyl; and
   R_{c} is methyl; or
   in some embodiments of the present invention, the PTM has the following structure: 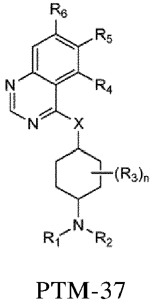
or a pharmaceutically acceptable salt or a stereoisomer thereof; wherein, in PTM-37:
   X is NH or O;
   b is 0 or 1;
   n is 0, 1, 2, 3 or 4;
   R₁ and R₂ are independently H or C1-C4 alkyl; or R₁ and R₂ together with the nitrogen to which they are attached form a monocyclic or bicyclic heterocyclyl containing 3-8 carbon atoms, in addition to the above nitrogen, the heterocyclyl optionally also contains one or two heteroatoms selected from N, O or S, the alkyl and heterocyclyl are optionally substituted by one or more substituents selected from Rₐ;
   R₃ is C1-C4 alkyl, in which, two adjacent alkyls can be connected together to form a bridge ring of 3-6 carbon atoms;
   R₄ is absent, halogen or O(C1-C4) alkyl;
   R₅ is halogen, CN, C1-C4 alkyl, O(C1-C4) alkyl, C2-4 alkenyl, aryl, heteroaryl or non-aromatic ring, the alkyl, alkenyl, aryl, heteroaryl and non-aromatic ring is optionally substituted by one or more R_{b};
   R₆ is absent, halogen, O(C1-C4) alkyl;
   Rₐ is independently selected from halogen, oxo, hydroxyl, CF₃, O(C1-C4) alkyl, SO₂(C1-C4) alkyl, C(O)O(C1-C4) alkyl, C(O) heterocycloalkyl, or heterocycloalkyl, wherein the alkyls can be aggregated together to form a bridging ring with another alkyl, and wherein the alkyl and heterocycloalkyl are optionally substituted by R_{b}; and
   R_{b} is independently selected from OH, halogen, CHF₂, (C1-C4) alkyl, CF₃, COOH, SO₂(C1-C4) alkyl, C(O)O(C1-C4) alkyl, O(C1-C4) alkyl, aryl, heterocycloalkyl, CN,C(O)N(Rc)₂, N(Rc)₂; Rc and alkyl are optionally substituted by OH, O(C1-C4) alkyl, alkyl and heterocycloalkyl; and
   Rc is independently H, SO₂(C1-C4) alkyl, or (C1-C4) alkyl; or
   in some embodiments of the present invention, the PTM has the following structure: 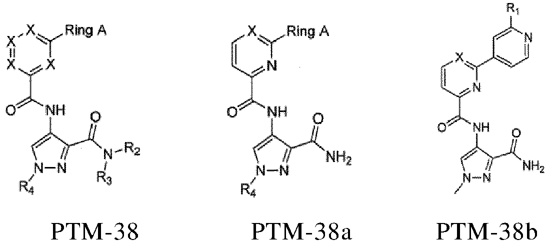
or a pharmaceutically acceptable salt or a stereoisomer thereof; wherein, in PTM-38, PTM-38a or PTM-38b:
   X is CH or N;
   a is 0 or 1; b is 0 or 1; m is 0, 1 or 2;
   ring A is C3-C8 cycloalkyl, C3-C8 cycloalkenyl, aryl or heterocyclic, optionally substituted by 1-3 substituents independently selected from R₁;
   R₁ selected from: H, oxo, (C=O)ₐO_{b}(C1-10) alkyl, (C = O)ₐO_{b}-aryl, (C = O)ₐO_{b}(C2-10) alkenyl, (C = O)ₐO_{b}(C2-10) alkynyl, CO₂H, halogen, OH, O_{b}(C1-6) fluoroalkyl, (C = O)ₐ NR₅R₆, CN, (C = O)ₐO_{b}(C3-C8) cycloalkyl, S(O)ₘNR₅R₆, SH, S(O)ₘ-(C1-10) alkyl or (C = O)ₐO_{b}-heterocycloalkyl, the alkyl, aryl, alkenyl, alkynyl, cycloalkyl and heterocycloalkyl are optionally substituted by one or more substituents selected from Rₐ;
   R₂ and R₃ are independently selected from: H, (C = O)ₐO_{b}(C1-10) alkyl, (C = O)ₐO_{b}-aryl, C2-10 alkenyl, C2-10 alkynyl, (C = O)ₐO_{b} heterocycloalkyl, CO₂H, CN, O_{b}(C1-6) fluoroalkyl, Oₐ(C = O)_{b}NR₅R₆, CHO, (N = O)R₅R₆, S(O)ₘNR₅R₆, SH, S(O)ₘ(C1-C10) alkyl, (C = O)ₐO_{b}-C3-C8 cycloalkyl, optionally substituted by one or more substituents selected from R₁; or
   R₂ and R₃ together with the nitrogen to which they are attached can form monocyclic or bicyclic heterocyclyl, each ring has 3-7 members, and in addition to the nitrogen, the ring optionally further contains one or two heteroatoms selected from N, O or S; the monocyclic or bicyclic heterocyclyl is optionally substituted by one or more substituents selected from R₁;
   R4is selected from: C1-C6 alkyl or C3-C6 cycloalkyl, and optionally substituted by Rₐ;
   R₅ and R₆ are independently selected from: H, oxo, (C = O)ₐO_{b}-(C1-10) alkyl, (C = O)ₐO_{b}-aryl, (C = O)ₐO_{b}(C2 -10) alkenyl, (C = O)ₐO_{b}-(C2-C10) alkynyl, CO₂H, O_{b}(C1-C6) fluoroalkyl, (C = O)ₐN(Rₐ)₂, CN, (C = O)ₐO_{b}-(C3-C8)) cycloalkyl, S(O)ₘN(Rₐ)₂, SH, S(O)ₘ(C1-10) alkyl and (C = O)ₐO_{b}-heterocycloalkyl; the alkyl, aryl, alkenyl, alkynyl, cycloalkyl and heterocycloalkyl are optionally substituted by one or more substituents selected from Ra;
   Rₐ is independently selected from R_{b}, OH, C1-C6 alkoxy, halogen, cyclopropyl, CO₂H, CN, Oₐ(= O)_{b}(C1-C6) alkyl, oxo or N(R_{b})₂;
   R_{b} is independently selected from H or C1-C6 alkyl; or
   in some embodiments of the present invention, the PTM has the following structure: 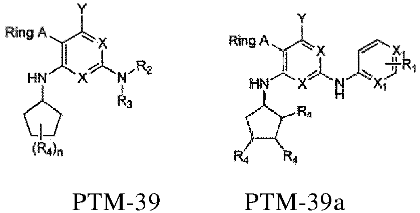
or a pharmaceutically acceptable salt or a stereoisomer thereof; wherein, in PTM-39 or PTM-39a:
   X is independently CH or N;
   Y is H or methyl;
   a is 0 or 1; b is 0 or 1; m is 0,1 or 2; n is 0,1,2,3 or 4;
   ring A is C3-8 cycloalkenyl, aryl or heterocyclic, optionally substituted by 1-3 substituents independently selected from R₁;
   R1 selected from: H, oxo, (C=O)ₐO_{b}(Cl-10) alkyl, (C = O)ₐO_{b} -aryl, (C = O)ₐO_{b} (C2-10) alkenyl, (C = O)ₐO_{b} (C2-10) alkynyl, CO2H, halogen, OH, O_{b}(C1-6) fluoroalkyl, (C = O)ₐ NR₅R₆, CN, (C = O)ₐO_{b} (C3-C8) cycloalkyl, S(O)ₘNR₅R₆, SH, S(O)m-(C1-10) alkyl or (C = O)ₐO_{b} -heterocycloalkyl, the alkyl, aryl, alkenyl, alkynyl, cycloalkyl and heterocycloalkyl are optionally substituted by one or more substituents selected from Rₐ;
   R₂ and R₃ are independently selected from: H, (C = O)ₐO_{b} (Cl-10) alkyl, (C = O)ₐO_{b} - aryl, C2-10 alkenyl, C2-10 alkynyl, (C = O)ₐO_{b} heterocycloalkyl, CO₂H, CN, O_{b}(C1-6) fluoroalkyl, Oₐ(C = O)_{b}NR₅R₆, CHO, (N = O)R₅R₆, S(O)ₘNR₅R₆, SH, S(O)m(C1-C10) alkyl, (C = O)ₐO_{b} -C3-C8 cycloalkyl, optionally substituted by one or more substituents selected from R1; or
   R₂ and R₃ can form monocyclic or bicyclic heterocyclic rings together with the nitrogen to which they are attached, each ring has 3-7 members, and in addition to the nitrogen, the ring optionally further contains one or two heteroatoms selected from N, O or S; the monocyclic or bicyclic heterocyclic ring is optionally substituted by one or more substituents selected from R₁;
   R₄ is selected from: C1-C6 alkyl, hydroxyl, methoxy, CF₃, or F, the alkyl is optionally substituted by hydroxyl;
   R5 and R6 are independently selected from: H, oxo, (C = O)aOb-(C1-10) alkyl, (C = O)aOb-aryl, (C = O)aOb(C2 -10) alkenyl, (C = O)aOb-(C2-C10) alkynyl, CO2H, Ob(C1-C6) fluoroalkyl, (C = O)aN(Ra)2, CN, (C = O)aOb-(C3-C8)) cycloalkyl, S(O)mN(Ra)2, SH, S(O)m(C1-10) alkyl and (C = O)aOb-heterocycloalkyl; the alkyl, aryl, alkenyl, alkynyl, cycloalkyl and heterocycloalkyl are optionally substituted by one or more substituents selected from Ra;
   Ra is independently selected from Rb, OH, C1-C6 alkoxy, halogen, cyclopropyl, CO2H, CN, Oa(= O)b(C1-C6) alkyl, oxo or N(Rb)2;
   R_{b} is independently selected from H or C1-C6 alkyl; or
   in some embodiments of the present invention, the PTM has the following structure: 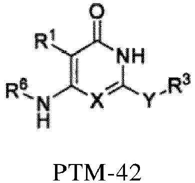
or a pharmaceutically acceptable salt thereof; wherein, in PTM-42:
   X is N or CH;
   Y is selected from -NR²-,-CH₂-,-CHR- or -O-, when Y is -CHR-, R and R³ together with the carbon to which they are attached optionally form 4 to 6-membered cycloalkyl, cycloalkenyl or heterocycloalkyl, wherein the 4 to 6-membered cycloalkyl, cycloalkenyl or heterocycloalkyl is optionally substituted by 1-3 substituents, the substituent is independently selected from C1-C4 alkyl, C3-C6 cycloalkyl, phenyl, CF₃, heterocycloalkyl, halogen,-COOR⁸,-NHR⁸,-SR⁸,-OR⁸,-SO₂R⁸,-COR⁸,-NHCOR⁸ or -CONHR⁸; or when Y is - NR², R² and R³ together with the nitrogen to which they are attached form 4 to 6-membered heterocyclyl, wherein the 4-6-membered heterocycloalkyl is optionally substituted by 1-3 substituents independently selected from C1-C3 alkyl, C3-6-cycloalkyl, phenyl, CF₃, heterocycloalkyl, halogen,-COOR⁸,-NHR⁸,-SR⁸,-OR⁸,-SO₂ R⁸,-COR⁸,-NHCOR⁸or - CONHR⁸;
   R¹ is selected from hydrogen, deuterium, C1-10 alkyl, C3-C6 cycloalkyl, aryl, heterocycloalkyl, halogen,-COOR⁷,-NHR⁷,-SR⁷,-OR⁷,-SO2R⁷,-COR⁷,-NHCOR⁷ or - CONHR⁷; wherein, the alkyl, cycloalkyl, aryl and heterocycloalkyl are optionally substituted by 1-3 substituents independently selected from C1-C4 alkyl, C3-C6 cycloalkyl, CN, phenyl, CF₃, heterocycloalkyl, halogen,-COOR⁸,-NHR⁸,-SR⁸,-OR⁸,-SO₂ R⁸,-COR⁸,-NHCOR⁸ or - CONHR⁸, wherein -NHR⁸, optionally substituted by -N(C1-C4 alkyl) NH₂ or -N(C3-C6 cycloalkyl) NH₂;
   R² is selected from hydrogen, deuterium, C1-10 alkyl or C3-C8 cycloalkyl;
   R³ is selected from hydrogen, deuterium, C1-10 alkyl, C3-C8 cycloalkyl, aryl, heterocycloalkyl or -COOR⁷; wherein the alkyl, cycloalkyl, aryl and heterocycloalkyl are optionally substituted by 1-3 substituents independently selected from C1-C4 alkyl, C3-6 cycloalkyl, phenyl, CF₃, heterocycloalkyl, halogen,-COOR⁸,-NHR⁸,-SR⁸,-OR⁸,-SO₂R⁸,-COR⁸,-NHCOR⁸ or -CONHR⁸;
   R⁶ is selected from C1-10 alkyl, C3-C8 cycloalkyl, aryl, heterocycloalkyl, -COOR⁷,-SO₂R⁷,-COR⁷; wherein the alkyl, cycloalkyl, aryl and heterocycloalkyl are optionally substituted by 1-3 substituents independently selected from C1-C4 alkyl, C3-C6 cycloalkyl, phenyl, CF₃, heterocycloalkyl, halogen,-COOR⁸,-NHR⁸,-SR⁸,-OR⁸,-SO₂R⁸,-COR⁸,-NHCOR⁸ or -CONHR⁸;
   R⁷ is selected from hydrogen, deuterium, C1-10 alkyl, C3-C8 cycloalkyl, aryl or heteroaryl; wherein the alkyl, cycloalkyl, aryl and heterocycloalkyl are optionally substituted by 1-3 substituents independently selected from C1-C4 alkyl, C3-C6 cycloalkyl, phenyl, CF₃, heterocycloalkyl, halogen, -COOR⁸, -NHR⁸, -SR⁸, -OR⁸,-SO₂R⁸,-COR⁸,-NHCOR⁸ or - CONHR⁸;
   R⁸ is selected from hydrogen, deuterium, C1-C6 alkyl or C3-C6 cycloalkyl; or
   in some embodiments of the present invention, the PTM has the following structure: 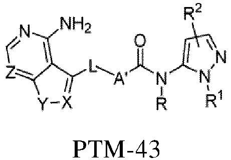
or a pharmaceutically acceptable salt thereof; wherein, in PTM-43:
   A' is C = O, C(R)₂ or NR;
   L is selected from C3-C10 aryl, 3-8-membered saturated or partially unsaturated carbocycle, divalent 3-7-membered heterocyclyl containing 1-4 heteroatoms independently selected from nitrogen, oxygen or sulfur, or 5-6-membered monocyclic heteroaryl containing 1-4 heteroatoms independently selected from nitrogen, oxygen or sulfur, each of which is optionally substituted;
   X is CR;
   Y is NR or S;
   Z is CR or N;
   R¹ is C3-C10 aryl, 3-8-membered saturated or partially unsaturated carbocycle, 3-7-membered heterocyclyl containing 1-4 heteroatoms independently selected from nitrogen, oxygen or sulfur, or 5-6-membered monocyclic heteroaryl ring containing 1-4 heteroatoms independently selected from nitrogen, oxygen or sulfur, and each of which is optionally substituted;
   R² is -R, halogen, haloalkyl, -OR, -SR, -CN, -NO2, -SO₂R, -SOR, -C(O)R, -CO₂R, - C(O)N(R)₂, -NRC(O)R, -NRC(O)N(R)₂, -NRSO₂R or -N(R)₂ ;
   each R is independently hydrogen, deuterium, C1-C6 alkyl, C3-C10 aryl, 3-8-membered saturated or partially unsaturated carbocycle, 3-7-membered heterocyclyl having 1-4 heteroatoms independently selected from nitrogen, oxygen or sulfur, or 5-6-membered monocyclic heteroaryl ring having 1-4 heteroatoms independently selected from nitrogen, oxygen or sulfur, each of which is optionally substituted; or two Rs on the same atom together with the atom connected to them form C3-C10 aryl, 3-8-membered saturated or partially unsaturated cycloalkyl, 3-7-membered heterocycloalkyl containing 1-4 heteroatoms independently selected from nitrogen, oxygen or sulfur, or 5-6-membered monocyclic heteroaryl containing 1-4 heteroatoms independently selected from nitrogen, oxygen, or sulfur, each of which is optionally substituted; or
   in some embodiments of the present invention, the PTM has the following structure: 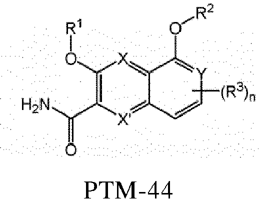
or pharmaceutically acceptable salt thereof, wherein, in PTM-44:
   X is CR or N;
   X' is CR or N; wherein at least one of X or X' is N;
   Y is CR or N;
   R¹ is C1-C6 alkyl, C3-C10 aryl, 3-8-membered saturated or partially unsaturated cycloalkyl, 3-7-membered heterocycloalkyl containing 1-4 heteroatoms independently selected from nitrogen, oxygen or sulfur, or 5-6-membered monocyclic heteroaryl containing 1-4 heteroatoms independently selected from nitrogen, oxygen or sulfur, each of which is optionally substituted;
   R² is C1-C6 alkyl, C3-C10 aryl, 3-8-membered saturated or partially unsaturated cycloalkyl, 3-7-membered heterocycloalkyl containing 1-4 heteroatoms independently selected from nitrogen and oxygen, or 5-6-membered monocyclic heteroaryl containing 1-4 heteroatoms independently selected from nitrogen, oxygen or sulfur, each of which is optionally substituted; or
   R² is (CR₂)ₘ-C3-C10 aryl, (CR₂)ₘ-3-8-membered saturated or partially unsaturated cycloalkyl, (CR₂)ₘ-3-7-membered heterocycloalkyl, the heterocycloalkyl has 1-4 heteroatoms independently selected from nitrogen, oxygen or sulfur, or (CR₂)ₘ-5-6-membered monocyclic heteroaryl, the heteroaryl has 1-4 heteroatoms independently selected from nitrogen, oxygen or sulfur, each of which is optionally substituted;
   each R³ is independently -R, halogen, -haloalkyl, -OR, -SR, -CN, -NO₂, -SO₂R, -SOR, - C(O)R, -CO₂R, -C(O)N(R)₂, NRC(O)R, -NRC(O)N(R)₂, -NR SO₂R or -N(R)₂;
   each R is independently hydrogen, deuterium, C1-C6 alkyl, C3-C10 aryl, 3-8-membered saturated or partially unsaturated cycloalkyl, 3-7-membered heterocycloalkyl containing 1-4 heteroatoms independently selected from nitrogen, oxygen or sulfur, or 5-6-membered monocyclic heteroaryl containing 1-4 heteroatoms independently selected from nitrogen, oxygen or sulfur, each of which is optionally substituted; or two Rs on the same atom together with the atom connected to them form C3-C10 aryl, 3-8-membered saturated or partially unsaturated cycloalkyl, 3-7-membered heterocyclyl containing 1-4 heteroatoms independently selected from nitrogen, oxygen or sulfur, or 5-6-membered monocyclic heteroaryl containing 1-4 heteroatoms independently selected from nitrogen, oxygen or sulfur, each of which is optionally substituted;
   m is 1 or 2;
   n is 0, 1, 2 or 3; or
   in some embodiments of the present invention, the PTM has the following structure: 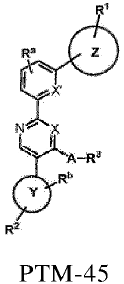
or a pharmaceutically acceptable salt thereof; in PTM-45:
   X and X' are each independently CR or N;
   A is O, S, SO₂, SO,-NRC(O),-NRSO₂, or -N(R), or A is absent;
   R³ is -R, halogen, -haloalkyl, -OR, -SR, -CN, -NO2, -SO₂R, -SOR, -C(O)R, -CO₂R, - C(O)N(R)₂, -NRC(O)R, -NRC(O)N(R)₂, -NRSO₂R, or -N(R)₂; or
   when A is -NRC(O),-NRSO₂ or -N(R); then R and R³ together with the atoms to which they are each connected form a 3-7-membered heterocycloalkyl containing 1-4 heteroatoms independently selected from nitrogen, oxygen or sulfur, or a 5-6-membered monocyclic heteroaromatic ring containing 1-4 heteroatoms independently selected from nitrogen, oxygen or sulfur, each of which is optionally substituted;
   ring Z is a 3-7-membered heterocycloalkyl containing 1-4 heteroatoms independently selected from nitrogen, oxygen or sulfur, or a 5-6-membered monocyclic heteroaryl containing 1-4 heteroatoms independently selected from nitrogen, oxygen or sulfur, each of which is optionally substituted;
   R¹ is -R, halogen, -haloalkyl, -OR, -SR, -CN, -NO₂, -SO₂R, -SOR, -C(O)R, -CO₂R, - C(O)N(R)₂, -NRC(O)R, -NRC(O)N(R)₂, -NRSO₂R, or -N(R)₂;
   R^{a} is absent or -R, halogen, haloalkyl, -OR, -SR, -CN, -NO2, -SO₂R, -SOR, -C(O)R, - CO₂R, -C(O)N(R)₂, -NRC(O)R, -NRC(O)N(R)₂, -NRSO₂R, or -N(R)₂;
   ring Y is an optionally substituted 5-6-membered monocyclic heteroaryl containing 2-4 heteroatoms independently selected from nitrogen, oxygen or sulfur;
   R² is -R, halogen, haloalkyl, -OR, -SR, -CN, -NO₂, -SO₂R, -SOR, -C(O)R, -CO₂R, - C(O)N(R)₂, -NRC(O)R, -NRC(O)N(R)₂, -NRSO₂R, or -N(R)₂;
   R^{b} is absent or -R, halogen, haloalkyl, -OR, -SR, -CN, -NO₂, -SO₂R, -SOR, -C(O)R, - CO₂R, -C(O)N(R)₂, -NRC(O)R, -NRC(O)N(R)₂, -NRSO₂R, or -N(R)₂;
   each R is independently hydrogen, deuterium, C1-C6 alkyl, C3-C10 aryl, 3-8-membered saturated or partially unsaturated cycloalkyl, 3-7-membered heterocyclyl containing 1-4 heteroatoms independently selected from nitrogen, oxygen or sulfur, or 5-6-membered monocyclic heteroaryl containing 1-4 heteroatoms independently selected from nitrogen, oxygen or sulfur, each of which is optionally substituted; or two Rs on the same atom together with the atom to which they are attached form C3-C10 aryl, 3-8-membered saturated or partially unsaturated cycloalkyl, 3-7-membered heterocycloalkyl containing 1-4 heteroatoms independently selected from nitrogen, oxygen or sulfur, or 5-6-membered monocyclic heteroaryl containing 1-4 heteroatoms independently selected from nitrogen, oxygen or sulfur; each of which is optionally substituted;
   wherein, when X is N and A is absent, then R3 is not H; or
   in some embodiments of the present invention, the PTM has the following structure: 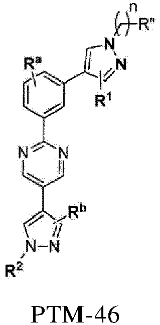
or pharmaceutically acceptable derivatives, solvates, tautomers, salts, hydrates, and stereoisomers thereof, including mixtures thereof at all ratios; in PTM-46:
   R" is H, C1-C6 alkyl, C3-C10 aryl, 3-8-membered saturated or partially unsaturated cycloalkyl, 3-7-membered heterocycloalkyl containing 1-4 heteroatoms independently selected from nitrogen, oxygen or sulfur, or 5-6-membered monocyclic heteroaryl containing 1-4 heteroatoms independently selected from nitrogen, oxygen or sulfur, each of which is optionally substituted;
   R¹ is absent or A or Q-R;
   R^{a} is absent or OR³, CF₃, Hal, NO₂;
   R^{b} is absent or A or COR;
   R² is independently C1-6 alkyl, C3-C10 aryl, 3-8-membered saturated or partially unsaturated cycloalkyl, 3-7-membered heterocycloalkyl containing 1-4 heteroatoms independently selected from nitrogen, oxygen or sulfur, or 5-6-membered monocyclic heteroaryl containing 1-4 heteroatoms independently selected from nitrogen, oxygen or sulfur, each of which is optionally substituted;
   each Q is independently a linear or branched alkylene containing 1-6 carbon atoms, wherein 1-5 hydrogen atoms can be independently substituted by groups selected from OR³, Hal, N(R³)₂, and wherein 1 or 2 -CH2- groups can be independently replaced by CO, SO, SO₂ or NR³, or Q represents a 4-8-membered saturated, unsaturated heterocycloalkyl or heteroaryl containing 1-3 heteroatoms independently selected from nitrogen, oxygen or sulfur;
   each A is independently a linear or branched alkyl containing 1-10 carbon atoms, wherein 1-7 hydrogen atoms can be independently substituted by groups selected from -OR³, Hal, NHSO₂A, SO₂A, SOA, N(R³)₂ , and wherein 1, 2 or 3 non-adjacent -CH₂- groups can be independently replaced by -CO-, NR³ or -O-,
   each Hal is F, Cl, Br or I,
   each R is independently hydrogen, C1-C6 alkyl, C3-C10 aryl, 3-8-membered saturated or partially unsaturated cycloalkyl, 3-7-membered heterocycloalkyl containing 1-4 heteroatoms independently selected from nitrogen, oxygen or sulfur, or 5-6-membered monocyclic heteroaryl containing 1-4 heteroatoms independently selected from nitrogen, oxygen or sulfur, each of which is optionally substituted;
   each R³ is H or C1-C6 alkyl, wherein one hydrogen atom can be substituted by a group selected from OH, O-(C1-C6 alkyl) or Hal;
   n is 0 or 1; or
   in some embodiments of the present invention, the PTM has the following structure: 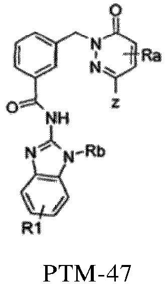
or pharmaceutical salt thereof; wherein, in PTM-47:
   Z is 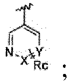
   X is CH or N;
   Y is CH or N;
   Ra, Rc and R1 each independently refer to H, Hal or A1;
   Rb is H or C1-12 alkyl;
   A1 is C1-12 branched or linear alkyl, wherein, 1-7 H atoms are optionally substituted by Hal, ORb, COORb, CN or N(Rb)₂, and wherein, 1-5 -CH₂- groups are optionally replaced by O, CO, NRb or S, SO, SO₂, 1,2-, 1,3- or 1,4-phenylene, -CH = CH- or -C= C-; and
   Hal refers to F, Cl, Br, I; or
   in some embodiments of the present invention, the PTM has the following structure: 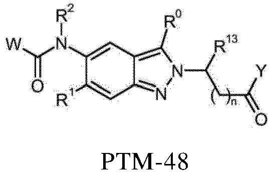
or a pharmaceutically acceptable salt thereof; wherein, in PTM-48:
   R⁰ is hydrogen or C1-C4 alkyl, wherein C1-C4 alkyl is optionally monosubstituted or polysubstituted by the same or different groups selected from hydroxyl and halogen;
   R¹ is hydrogen, deuterium, halogen, cyano, C(O)OH, C(O)OR^{a}, C(O)NH₂, C(O)N(H)R^{a}, C(O)N(R^{a})R^{b}, C(O)R^{d}, hydroxyl or C1-C6 alkyl, the C1-C6 alkyl is optionally monosubstituted or polysubstituted by the same or different groups selected from the following: hydroxy, halogen, cyano, C(O)OH, C(O)OR^{a}, S(O)₂C1-C6 alkyl, NH₂, NHR^{a}, N(R^{a})R^{b}, C1-C6 alkoxy optionally monosubstituted or polysubstituted by the same or different groups selected from halogen, C3-C8 cycloalkyl-O- optionally monosubstituted or polysubstituted by the same or different groups selected from halogen, heterocycloalkyl optionally monosubstituted or polysubstituted by the same or different groups selected from R^{c}, or is C1-C6 alkoxy, wherein the C1-C6-alkoxy is optionally monosubstituted or polysubstituted by the same or different groups selected from the following: hydroxy, halogen, cyano, C(O)OH, C(O)OR^{a}, S(O)₂C1-C6 alkyl, NH₂, NHR^{a}, N(R^{a})R^{b}, C3-C8 cycloalkyl optionally monosubstituted or polysubstituted by the same or different groups selected from halogen, C1-C6 alkoxy optionally monosubstituted or polysubstituted by the same or different groups selected from halogen, C3-C8 cycloalkyl-O- optionally monosubstituted or polysubstituted by the same or different groups selected from halogen, heterocycloalkyl optionally monosubstituted or polysubstituted by the same or different groups selected from R^{c}, aryl optionally monosubstituted or polysubstituted by the same or different groups selected from R^{c}, 5- or 6-membered heteroaryl optionally monosubstituted or polysubstituted by the same or different groups selected from R^{c}, or is C3-C8 cycloalkyl-O- or heterocycloalkyl-O- that is optionally monosubstituted or polysubstituted by the same or different groups selected from the following: hydroxyl, halogen, cyano or C1-C6 alkyl, or represents aryl-O- or 5-membered or 6-membered heteroaryl-O-, wherein aryl-O- and 5-membered or 6-membered heteroaryl-O- can optionally be monosubstituted or polysubstituted by the same or different groups selected from the following: hydroxyl, halogen, cyano, C(O)OH, C(O)OR^{a}, C1-C6 alkyl or C1-C6 alkoxy, or represents C3-C8-cycloalkyl or heterocycloalkyl that can optionally be monosubstituted or polysubstituted by the same or different groups selected from the following: hydroxyl, halogen, cyano and C1-C6 alkyl, or represents C2-6 alkenyl or C2-6 alkynyl, or represents aryl, 5-to 10-membered heteroaryl, aryl-C1-C4 alkyl, or 5-membered or 6-membered heteroaryl-C1-C4 alkyl, wherein the aryl and the heteroaryl can optionally be monosubstituted or polysubstituted by the same or different groups selected from the following: halogen, hydroxyl, cyano, C(O)OH, C(O)OR^{a}, C1-C6 alkyl, C3-C8 cycloalkyl or C1-C6 alkoxy;
   R^{a} represents C1-C6-alkyl, C3-C10-cycloalkyl, heterocycloalkyl, aryl or heteroaryl, wherein alkyl, cycloalkyl, heterocycloalkyl, aryl and heteroaryl are optionally monosubstituted or polysubstituted by the same or different groups selected from the following: halogen, hydroxyl, cyano, C1-C3 alkyl, C1-C3 alkoxy, heterocycloalkyl,-C(O)OC1-C6 alkyl or S(O)₂C1-C6 alkyl;
   R^{b} represents C1-C6 alkyl or C3-C10 cycloalkyl;
   or R^{a} and R^{b} together with the nitrogen atom to form a 5- or 6-membered heterocyclyl, the 5- or 6-membered heterocyclyl may optionally be monosubstituted or polysubstituted by the same or different groups selected from the following: hydroxyl, halogen, cyano or C1 - C6-alkyl;
   R^{c} represents hydroxyl, halogen, cyano, C1-C3-alkyl or C1-C3 alkoxy;
   R^{d} represents hydrogen, deuterium, C1-C6-alkyl or C3-C10 cycloalkyl;
   R² represents hydrogen, deuterium, C1-C6 alkyl or C3-C6 cycloalkyl;
   R¹³ represents hydrogen or C1-C6 alkyl;
   W represents 5-membered heteroaryl, which contains one to three heteroatoms selected from N, O, or S, and can be optionally mono-substituted by R³ and can be optionally mono-substituted or polysubstituted by the same or different R⁴, or W represents pyridyl, pyrazinyl, pyridazinyl, 1,2, 4-triazinyl or 1,3, 5-triazinyl, each of which can be optionally mono-substituted by R³ and optionally mono- or multi-substituted by the same or different R⁴;
   R³ represents hydrogen, deuterium, halogen, cyano, C(O)R^{a}, NH₂, NHR^{a}, N(R^{a})R^{b}, N(H)C(O)R^{a} or C1-C6-alkyl, wherein C1-C6-alkyl can be optionally monosubstituted or polysubstituted by the same or different groups selected from the following: hydroxyl, halogen, cyano, C(O)R^{a}, C(O)OH, C(O)OR^{a}, S(O)₂-C1-C6 alkyl, NH₂, NHR^{a}, N(R^{a})R^{b}, C1-C6 alkoxy, C3-C8-cycloalkyl-O-, wherein C1-C6-alkoxy and C3-C8-cycloalkyl-O- can be optionally monosubstituted or polysubstituted by the same or different halogens; or C1-C6-alkyl is optionally monosubstituted or polysubstituted by the same or different groups selected from the following: C3-C6-cycloalkyl and heterocycloalkyl, wherein C3-C6-cycloalkyl and heterocycloalkyl can optionally be mono-substituted, disubstituted or trisubstituted by the same or different groups selected from the following: halogen, cyano, C1-C3-alkyl and C1-C3-alkoxy, or C1-C6-alkyl are optionally monosubstituted or polysubstituted by the same or different groups selected from the following: aryl and 5-membered or 6-membered heteroaryl, the aryl and the 5-membered or 6-membered heteroaryl can optionally be monosubstituted, disubstituted or trisubstituted by the same or different groups selected from the following: halogen, cyano, C1-C3-alkyl and C1-C3-alkoxy, or
   R³ represents C1-C6 alkoxy, wherein the C1-C6-alkoxy may optionally be monosubstituted or polysubstituted by the same or different groups selected from the following: hydroxyl, halogen, cyano, C(O)OR^{a}, S(O)₂-C1-C6 alkyl, N(R^{a})R^{b}, C3-C8-cycloalkyl, C1-C4 alkoxy, C3-C8 cycloalkyl-O-, or represents C3-C6-cycloalkyl, heterocycloalkyl, or C5-C11 spiro alkyl, wherein cycloalkyl, heterocycloalkyl, and spiro alkyl can optionally be monosubstituted or polysubstituted by the same or different groups selected from the following: hydroxyl, halogen, cyano, C(O)R^{a}, C(O)OH, C(O)OR^{a}, C1-C6-alkyl and C1-C4-alkoxy; or represents aryl or 5- to 10-membered heteroaryl, wherein aryl and heteroaryl can optionally be monosubstituted or polysubstituted by the same or different groups selected from the following: halogen, hydroxyl, cyano, C(O)OR^{a}, S(O)₂-C1-C6 alkyl, NO₂, NH₂, NHR^{a}, N(R^{a})R^{b}, N(H)C(= O)R^{a}, C3-C8 cycloalkyl, C1-C3 alkoxy and C1-C3 alkyl, wherein C1-C3 alkyl can be optionally monosubstituted or polysubstituted by the same or different halogens;
   R⁴ represents halogen, hydroxyl, cyano or C1-C6-alkyl, wherein C1-C6-alkyl can optionally be monosubstituted or polysubstituted by the same or different groups selected from halogen; C1-C6-alkoxy, wherein C1-C6-alkoxy can optionally be monosubstituted or polysubstituted by the same or different groups selected from halogen; C2-6-alkenyl; C2-6-alkynyl; C3-C10-cycloalkyl; 3- to 10-membered heterocycloalkyl and aryl, wherein the aryl can be optionally mono- or multi-substituted by the same or different R, or
   R⁴ represents aryl or heteroaryl that can be optionally monosubstituted or polysubstituted by the same or different R, or
   R⁴ represents C(O)R^{a}, C(O)NH₂, C(O)N(H)R^{a}, C(O)N(R^{a})R^{b}, C(O)OR^{a}, NH₂, NHR^{a}, N(R^{a})R^{b}, N(H)C(O)R^{a}, N(R^{a})C(O)R^{a}, N(H)C(O)NH2, N(H)C(O)NHR^{a}, N(H)C(O)N(R^{a})R^{b}, N(R^{a}C(O)) NH₂, N(R^{a})C(O)NHR^{a}, N(R^{a})C(O)N(R^{a})R^{b}, N(H)C(O)OR^{a}, N(R^{a})C(O)OR^{a}, NO₂, N(H)S(O)R^{a}, N(R^{a})S(= O)R^{a}, N(H)S(O)₂R^{a}, N(R^{a})S(O)₂R^{a}, N = S(O)(R^{a})R^{b}, OC(O)R^{a}, OC(O)NH2, OC(O)NHR^{a}, OC(O)N(R^{a})R^{b}, SH, SR^{a}, S(O)R^{a}, S(O)₂R^{a}, S(O)2NH2, S(O)₂NHR^{a}, S(O)₂N(R^{a})R^{b} or S(O)(= N-R^{a})R^{b}
   R represents halogen, cyano, C1-C6-alkyl, C2-6-alkenyl, C2-6-alkynyl, C3-C10-cycloalkyl, 3- to 10-membered heterocycloalkyl, aryl, heteroaryl, C(O)R^{a}, C(O)NH₂, C(O)N(H)R^{a}, C(O)N(R^{a})R^{b}, C(O)OR^{a}, NH₂, NHR^{a}, N(R^{a})R^{b}, N(H)C(O)R^{a}, N(R^{a})C(O)R^{a}, N(H)C(O)NH₂, N(H)C(O)NHR^{a}, N(H)C(O)N(R^{a})R^{b}, N(R^{a})C(O)NH₂, N(R^{a})C(O)NHR^{a}, N(R^{a})C(O)N(R^{a})R^{b}, N(H)C(O)OR^{a}, N(R^{a})C(O)OR^{a}, NO₂, N(H)S(O)R^{a}, N(R^{a})S(O)R^{a}, N(H)S(O)₂R^{a}, N(R^{a})S(O)₂R^{a}, N = S(O)(R^{a})R^{b}, OH, C1-C6-alkoxy, OC(O)R^{a}, OC(O)NH₂, OC(O)NHR^{a}, OC(O)N(R^{a})R^{b}, SH, SR^{a}, S (O)R^{a}, S(O)₂R^{a}, S(O)₂NH₂, S(O)₂NHR^{a}, S(O)₂N(R^{a})R^{b} or S(O)(= N-R^{a})R^{b};
   n represents 0 or 1;
   Y represents a group selected from 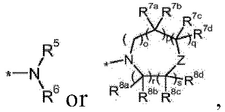
   wherein * represents the point where the group is attached to the rest of the molecule;
   R⁵ represents hydrogen, deuterium, C1-C6-alkyl or C3-C10-cycloalkyl, wherein C1-C6-alkyl can optionally be monosubstituted or polysubstituted by the same or different groups selected from the following: hydroxyl, halogen, cyano, C(O)OH, C(O)OR^{a}, S(O)₂-C1-C6 alkyl, N(R^{a})R^{b}, C1-C4 alkoxy or C3-C8-cycloalkyl;
   R⁶ represents hydrogen or C1-C6-alkyl, wherein C1-C6-alkyl can optionally be monosubstituted or polysubstituted by the same or different groups selected from the following: hydroxyl, halogen, cyano, C3-C10-cycloalkyl, C(O)R^{a}, C(O)OH, C(O)OR^{a}, S(O)₂-C1-C6 alkyl, N(R^{a})R^{b}, C1-C4 alkoxy or C3-C8 cycloalkyl-O-, or represents C3-C10-cycloalkyl, wherein the C3-C10-cycloalkyl can optionally be monosubstituted or polysubstituted by the same or different groups selected from the following: hydroxyl, halogen, cyano or C1-C6-alkyl, wherein the C1-C6-alkyl can optionally be substituted by a hydroxyl, or represents a heterocycloalkyl, wherein the heterocycloalkyl can optionally be monosubstituted or polysubstituted by the same or different groups selected from the following: halogen, cyano, C1-C3-alkyl or C1-C3-alkoxy, or represents aryl or 5-membered or 6-membered heteroaryl, wherein aryl and 5-membered or 6-membered heteroaryl can optionally be monosubstituted or polysubstituted by the same or different groups selected from the following: halogen, cyano, C1-C3-alkyl, C1-C3-alkoxy, S(O)₂NH₂, S(O)₂NHR^{a} and S(O)₂N(R^{a})R^{b};
   R^{7a} represents hydrogen, deuterium, halogen, N(R^{a})R^{b}, C1-C6-alkyl or C3-C10 cycloalkyl, wherein C1-C6-alkyl can optionally be monosubstituted or polysubstituted by the same or different groups selected from the following: hydroxyl, halogen, cyano, C(O)OH, C(O)OR^{a}, S(O)2-C1-C6 alkyl, N(R^{a})R^{b}, C1-C4-alkoxy, C3-C8-cycloalkyl and heterocycloalkyl;
   R^{7b} represents hydrogen, deuterium, halogen or C1-C6-alkyl, wherein C1-C6-alkyl can optionally be monosubstituted or polysubstituted by the same or different groups selected from the following: hydroxyl, halogen, cyano, C(O)OH, C(O)OR^{a}, S(O)₂-C1-C6 alkyl, N(R^{a})R^{b}, C1-C4-alkoxy, C3-C8-cycloalkyl or heterocycloalkyl; or
   R^{7a} and R^{7b} together with the carbon atom form a C3-C6-cycloalkyl, the C3-C6-cycloalkyl can optionally be monosubstituted or polysubstituted by the same or different groups selected from the following: hydroxyl, halogen, cyano and C1-C6-alkyl, or
   R^{7a} and R^{7b} together represent an oxo;
   R^{7c} represents hydrogen, deuterium, halogen, N(R^{a})R^{b}, C1-C6-alkyl or C3-C10-cycloalkyl, wherein C1-C6-alkyl can optionally be monosubstituted or polysubstituted by the same or different groups selected from the following: hydroxyl, halogen, cyano, C(O)OH, C(O)OR^{a}, S(O)₂-C1-C6-alkyl, N(R^{a})R^{b}, C1-C4 alkoxy, C3-C8-cycloalkyl or heterocycloalkyl;
   R^{7d} represents hydrogen, deuterium, halogen or C1-C6-alkyl, wherein C1-C6-alkyl can optionally be monosubstituted or polysubstituted by the same or different groups selected from the following: hydroxyl, halogen, cyano, C(O)OH, C(O)OR^{a}, S(O)₂-C1-C6 alkyl, N(R^{a})R^{b}, C1-C4 alkoxy, C3-C8 cycloalkyl or heterocycloalkyl; or
   R^{7c} and R^{7d} together with the carbon atom form a C3-C6-cycloalkyl, and the C3-C6-cycloalkyl may optionally be monosubstituted or polysubstituted by the same or different groups selected from the following: hydroxyl, halogen, cyano or C1-C6-alkyl, or
   R^{7c} and R^{7d} together represent an oxo;
   R^{8a} represents hydrogen, deuterium, halogen, N(R^{a})R^{b}, C1-C6 alkyl or C3-C10 cycloalkyl, wherein C1-C6-alkyl can optionally be monosubstituted or polysubstituted by the same or different groups selected from the following: hydroxyl, halogen, cyano, C(O)OH, C(O)OR^{a}, S(O)₂C1-C6 alkyl, N(R^{a})R^{b}, C1-C4 alkoxy, C3-C8 cycloalkyl or heterocycloalkyl;
   R^{8b} represents hydrogen, deuterium, halogen or C1-C6-alkyl, wherein C1-C6-alkyl can optionally be monosubstituted or polysubstituted by the same or different groups selected from the following: hydroxyl, halogen, cyano, C(O)OH, C(O)OR^{a}, S(O)₂C1-C6 alkyl, N(R^{a})R^{b}, C1-C4 alkoxy, C3-C8 cycloalkyl or heterocycloalkyl; or
   R^{8a} and R^{8b} together with the carbon atom form a C3-C6-cycloalkyl, and the C3-C6-cycloalkyl may optionally be monosubstituted or polysubstituted by the same or different groups selected from the following: hydroxyl, halogen, cyano or C1-C6-alkyl;
   R^{8c} represents hydrogen, deuterium, halogen, N(R^{a})R^{b}, C1-C6-alkyl or C3-C10 cycloalkyl, wherein C1-C6-alkyl can optionally be monosubstituted or polysubstituted by the same or different groups selected from the following: hydroxyl, halogen, cyano, C(O)OH, C(O)OR^{a}, S(O)₂C1-C6 alkyl, N(R^{a})R^{b}, C1-C4-alkoxy, C3-C8-cycloalkyl or heterocycloalkyl;
   R^{8d} represents hydrogen, deuterium, halogen or C1-C6-alkyl, wherein C1-C6-alkyl can optionally be monosubstituted or polysubstituted by the same or different groups selected from the following: hydroxyl, halogen, cyano, C(O)OH, C(O)OR^{a}, S(O)₂-C1-C6 alkyl, N(R^{a})R^{b}, C1-C4 alkoxy, C3-C8 cycloalkyl or heterocycloalkyl; or
   R^{8c} and R^{8d} together with the carbon atom form a C3-C6-cycloalkyl, and the C3-C6-cycloalkyl may optionally be monosubstituted or polysubstituted by the same or different groups selected from the following: hydroxyl, halogen, cyano or C1-C6-alkyl, or
   R^{8c} and R^{8d} together represent an oxo;
   o represents 0, 1 or 2,
   p represents 0, 1 or 2,
   q represents 0, 1 or 2,
   r represents 0, 1 or 2,
   s represents 0, 1, or 2,
   wherein, o, p, q, r and s do not represent 0 at the same time;
   Z is selected from C(= O), CR⁹R¹⁰, NR¹¹, O, S, S(O) or S(O)₂;
   R⁹ represents hydrogen or C1-C6-alkyl,
   R¹⁰ represents hydrogen, deuterium, halogen, cyano, C(O)R^{a}, C(O)OH, C(O)OR^{a}, C(O)NH₂, C(O)N(H)R^{a}, C(O)N(R^{a})R^{b}, N(H)C(O)R^{a}, N(R^{b})C(O)R^{a}, S(O)₂R^{a}, hydroxyl, N(R^{a})R^{b} or C1-C6 alkyl, the C1-C6 alkyl can optionally be monosubstituted or polysubstituted by the same or different groups selected from the following: hydroxyl, halogen, cyano, C(O)R^{a}, C(O)OH, C(O)OR^{a}, S(O)₂C1-C6 alkyl, N(R^{a})R^{b}, C1-C4 alkoxy and C3-C8 cycloalkyl-O-, or represents C1-C6-alkoxy, wherein, the C1-C6-alkoxy can be optionally monosubstituted or polysubstituted by the same or different groups selected from the following: hydroxyl, halogen, cyano, C(O)OH, C(O)OR^{a}, S(O)₂C1-C6 alkyl, N(R^{a})R^{b}, C3-C8-cycloalkyl, C1-C4-alkoxy, C3-C8-cycloalkyl-O-, heterocycloalkyl, aryl or 5-membered or 6-membered heteroaryl, wherein the aryl and 5-membered or 6-membered heteroaryl can be optionally monosubstituted or polysubstituted by the same or different groups selected from the following: halogen, cyano, C1-C3 alkyl and C1-C3 alkoxy, or represents aryl-O- or 5-membered or 6-membered heteroaryl-O-, wherein, the aryl-O- and 5-membered or 6-membered heteroaryl-O- can be optionally monosubstituted or polysubstituted by the same or different groups selected from the following: hydroxyl, halogen, cyano, C(O)OH, C(O)ORa, C1-C3 alkyl or C1-C3 alkoxy, or represents C3-C8 cycloalkyl, C3-C8 cycloalkyl-C1-C4 alkyl, heterocycloalkyl or heterocycloalkyl-C1-C4 alkyl, which can be optionally monosubstituted or polysubstituted by the same or different groups selected from the following: hydroxyl, halogen, cyano, C(O)R^{a}, C(O)OH, C(O)OR^{a}, C1-C6-alkyl or C1-C6-alkoxy, wherein C1-C6-alkoxy can optionally be monosubstituted or polysubstituted by the same or different halogen or oxo; or represents C2-6 alkenyl or C2-6 alkynyl, or represents aryl, 5-10-membered heteroaryl, aryl-C1-C4 alkyl, or 5- or 6-membered heteroaryl-C1-C4 alkyl, wherein the aryl and heteroaryl can optionally be monosubstituted or polysubstituted by the same or different groups selected from the following: halogen, hydroxyl, cyano, C(O)OH, C(O)OR^{a}, NHR^{a}, N(R^{a})R^{b}, C1-C3-alkyl, C3-C8-cycloalkyl or C1-C3-alkoxy;
   or R⁹ and R¹⁰ together with the carbon atom form C3-C8 cycloalkyl or 4-6-membered heterocycloalkyl, wherein C3-C8 cycloalkyl or 4-6-membered heterocycloalkyl can optionally be monosubstituted or polysubstituted by the same or different groups selected from the following: hydroxyl, halogen, cyano, C1-C6-alkyl, C(O)R^{a} or oxo;
   R¹¹ represents hydrogen, deuterium, C(O)R^{a}, C(O)OR^{a}, C(O)NH₂, C(O)N(H)R^{a}, C(O)N(R^{a})R^{b}, S(O)₂R^{a}, S(O)₂N(R^{a})R^{b} or C1-C6-alkyl, the C1-C6-alkyl can optionally be monosubstituted or polysubstituted by the same or different groups selected from the following: hydroxyl, halogen, cyano, C(O)R^{a}, C(O)OR^{a}, C(O)NH₂, C(O)N(H)R^{a}, C(O)N(R^{a})R^{b}, S(O)₂C1-C6 alkyl, N(R^{a})R^{b}, C3-C8 cycloalkyl, C1-C4 alkoxy or C3-C8 cycloalkyl-O-, wherein the C3-C8 cycloalkyl, C1-C4 alkoxy and C3-C8-cycloalkyl-O- can optionally be mono- or polysubstituted by the same or different groups selected from hydroxyl and halogen; or represents C3-C8-cycloalkyl, heterocycloalkyl or heterocycloalkyl-C1-C4 alkyl, which can optionally be mono- or polysubstituted by the same or different groups selected from the following: hydroxy, halogen, cyano, C1-C6-alkyl, C1-C6-alkoxy, wherein the alkyl and alkoxy can optionally be monosubstituted or polysubstituted by the same or different groups selected from halogen and oxo, or represents C2-6 alkenyl or C2-6 alkynyl, or represents aryl, 5-10-membered heteroaryl, aryl-C1-C4 alkyl, or 5-membered or 6-membered heteroaryl-C1-C4 alkyl, wherein the aryl and heteroaryl can optionally be monosubstituted or polysubstituted by the same or different groups selected from the following: halogen, hydroxyl, cyano, C(O)OH, C(O)OR^{a}, C1-C3 alkyl, C3-C8 cycloalkyl or C1-C3-alkoxy; or
   in some embodiments of the present invention, the PTM has the following structure: 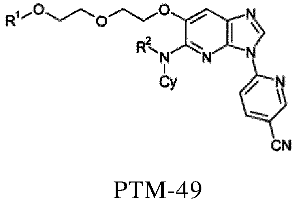
or a pharmaceutically acceptable salt or a solvate or a solvate salt thereof; wherein, in PTM-49:
   Cy is a monocyclic C3-7 cycloalkyl, which is optionally substituted by one or more independently selected R³, or 4 to 7 membered monocyclic heterocycloalkyl containing one or two heteroatoms independently selected from N, S or O, and optionally substituted by one or more groups independently selected from R³;
   R¹ is H, SO₃H, P(O)(OH)₂, C1-C4 alkyl, C(O)-(4 to 7 membered monocyclic heterocycloalkyl containing one or two heteroatoms independently selected from N, S and O), or C(O)C1-C6 alkyl, the C1-C6 alkyl is optionally substituted by one or more independently selected R⁴ group;
   R² is H or C1-C4 alkyl;
   each R³ is independently selected from: OH, oxo, halogen or C1-C4 alkyl;
   each R⁴ is independently selected from: -NR^{5a}R^{5b},-C(O)OH, 4 to 7 membered monocyclic heterocycloalkyl containing one or two heteroatoms independently selected from N, S and O, and optionally substituted by one or more independently selected C1-C4 alkyl, or -NHC(O)-C1-C4 alkyl-NH₂; and
   R^{5a} and R^{5b} are independently H or C1-C4 alkyl; or
   in some embodiments of the present invention, the PTM has the following structure: 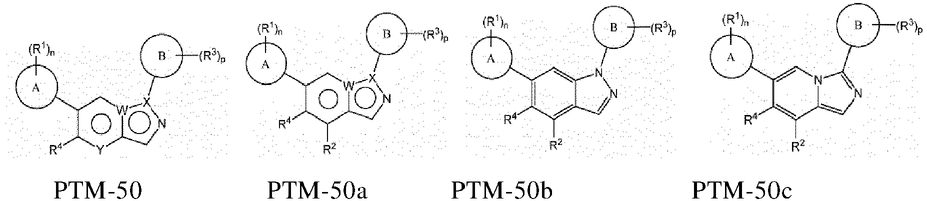
or a pharmaceutically acceptable salt thereof, wherein, in PTM-50, PTM-50, PTM-50 or PTM-50:
   ring A is selected from phenyl or 5- or 6-membered heteroaryl;
   ring B is selected from phenyl or 5- or 6-membered heteroaryl;
   n is 0, 1 or 2;
   p is 0, 1 or 2;
   one of W and X is N, and the other of W and X is C;
   Y is N or CR²;
   R¹ is selected from C1-C6 alkyl, C2-6 alkenyl, C2-6 alkynyl, C3-C6 cycloalkyl, 3-6-membered saturated heterocycloalkyl, halogen,-CN,-C(R^{1a})= NR(OR^{1a}),-C(R^{1a})= N(R^{1a}),-C(O)R^{1a},-C(O)₂R^{1a},-C(O)NR^{1a},-NO₂,-N(R^{1a})₂,-N(R^{1a})C(O)R^{1a},-NR^{1a}C(O)₂R^{1a},-N(R^{1a})C(O) N(R) N(R^{1a})₂,-N(R^{1a})S(O)₂R^{1a},-OR^{1a},-OC(O)R^{1a},-OC(O) N(R^{1a})₂,-SR^{1a},-S(O)R^{1a},-S(O)₂R^{1a},-S(O) N(R^{1a})₂, or -S(O)₂N(R^{1a})₂, wherein the C1-C6 alkyl, C2-6 alkenyl, C2-6 alkynyl, C3-C6 cycloalkyl, 3-6-membered saturated heterocycloalkyl is optionally substituted by one or more R¹⁰; or two R¹substituents together with the atoms to which they are attached form a C5-7 cycloalkyl or a saturated 5-7-membered heterocyclyl, wherein the C5-7 cycloalkyl or saturated 5-7-membered heterocyclyl is optionally substituted by one or more R¹⁵;
   R^{1a} is independently selected from H, C1-C6 alkyl, C2-6 alkenyl, C2-6 alkynyl, 3 to 6 membered monocyclic cycloalkyl, or 3 to 6 membered monocyclic heterocycloalkyl at each occurrence, wherein each of the C1-C6 alkyl, C2-6 alkenyl, C2-6 alkynyl, 3- to 6-membered monocyclic cycloalkyl, and 3- to 6-membered monocyclic heterocycloalkyl are independently optionally substituted by one or more R¹⁰;
   R¹⁰ is independently selected from C1-C6 alkyl, C2-6 alkenyl, C2-6 alkynyl, 3- to 6-membered cycloalkyl, 3-6-membered heterocycloalkyl, halogen,-CN,-C(R^{10a})= NR(OR^{10a}),-C(R^{10a})= N(R^{10a}),-C(O) R^{10a},-C(O)₂ R^{10a},-C(O) N(R^{10a})₂,-NO₂,-N(R^{10a})₂,-N(R^{10a}) C(O) R^{10a},-N(R^{10a}) C(O)₂ R^{10a},-N(R^{10a}) C(O) N(R^{10a})₂,-N(R^{10a}) S(O)₂R^{10a},-OR^{10a},-OC(O) R^{10a},-OC(O) N(R^{10a})₂,-SR^{10a},-S(O) R^{10a},-S(O)₂R^{10a},-S(O) N(R^{10a})₂ and -S(O)₂N(R^{10a})₂ at each occurrence;
   R^{10a} is independently selected from H and C1-C6 alkyl at each occurrence, wherein the C1-C6 alkyl is optionally substituted by one or more halogens;
   R¹⁵ is independently selected from C1-C6 alkyl, C2-6 alkenyl, C2-6 alkynyl, 3- to 6-membered cycloalkyl, 3- to 6-membered heterocycloalkyl, halogen,-CN,-C(R^{15a})= NR(OR^{15a}),-C(R^{15a})= N(R^{15a}),-C (O)(R^{15a}),-C (O)₂(R^{15a}),-C (O) N(R^{15a})₂,-NO₂,-N(R^{15a})₂,-N(R^{15a})C (O)(R^{15a}),-N(R^{15a})C (O)₂(R^{15a}), N(R^{15a})C (O) N(R^{15a})₂, N(R^{15a})S(O)₂(R^{15a}),-OR^{15a},-OC(O)R^{15a},-OC(O) N(R^{15a})₂,-SR^{15a},-S(O)R^{15a},-S(O)₂(R^{15a}),-S(O) N(R^{15a})₂ and - S(O)₂N(R^{15a})₂ at each occurrence;
   R^{15a} is independently selected from H and C1-C6 alkyl at each occurrence, wherein the C1-C6 alkyl is optionally substituted by one or more halogens;
   R is selected from H, C1-C8 alkyl, C2-6 alkenyl, C2-6 alkynyl, 3 to 7-membered cycloalkyl, 3-7-membered heterocycloalkyl, halogen,-CN,-C(R^{2a})= NR(OR^{2a}), -C(R^{2a})= N(R^{2a}),-C (O)(R^{2a}),-C (O)₂(R^{2a}),-C (O) N(R^{2a})₂,-NO₂,-N(R^{2a})2,-N(R^{2a})C (O)(R^{2a}),-N(R^{2a})C (O)₂(R^{2a}), N(R^{2a})C (O) N(R^{2a})₂, N(R^{2a})S(O)₂(R^{2a}),-OR^{2a},-OC(O)R^{2a},-OC(O) N(R^{2a})₂,-SR^{2a},-S(O)R^{2a},-S(O)₂(R^{2a}),-S(O) N(R^{2a})₂, and -S(O)₂N(R^{2a})₂, wherein the C1-C6 alkyl, C2-6 alkenyl, C2-6 alkynyl, 3-7-membered cycloalkyl, and 3-7-membered heterocycloalkyl are optionally substituted by one or more R;
   R^{2a} is independently selected from H and C1-C6 alkyl at each occurrence, wherein the C1-C6 alkyl for each occurrence is optionally and independently substituted by one or more R²⁰;
   R²⁰ is independently selected from C1-C6 alkyl, C2-6 alkenyl, C2-6 alkynyl, C3-7 cycloalkyl, 3-7-membered saturated heterocycloalkyl, halogen,-CN,-C(R^{20a})= NR(OR^{20a}),-C(R^{20a})= N(R^{20a}),-C (O)(R^{20a}),-C (O)₂(R^{20a}),-C (O) N(R^{20a})₂,-NO₂,-N(R^{20a})₂,-N(R^{20a})C (O)(R^{20a}),-N(R^{20a})C (O)₂(R^{20a}), N(R^{20a})C (O) N(R^{20a})₂, N(R^{20a})S(O)₂(R^{20a}),-OR^{20a},-OC(O)R^{20a},-OC(O) N(R^{20a}) ₂,-SR^{20a},-S(O)R^{20a},-S(O)₂(R^{20a}),-S(O) N(R^{20a}) ₂and -S(O)₂N(R^{20a}) ₂ at each occurrence, wherein the C1-C6 alkyl, C2-6 alkenyl, C2-6 alkynyl, C3-7 cycloalkyl and 3-7-membered saturated heterocycloalkyl are optionally and independently substituted by one or more R at each occurrence;
   R^{20a} is independently selected from H and C1-C4 alkyl at each occurrence, wherein the C1-C4 alkyl is optionally substituted by R;
   R²⁵ is selected from halogen and -OR^{25a};
   R^{25a} is selected from H and C1-C6 alkyl;
   R is selected from C1-C6 alkyl, C2-6 alkenyl, C2-6 alkynyl, C3-C6 cycloalkyl, 3-6-membered saturated heterocycloalkyl, halogen,-CN,-C(R^{3a})= NR(OR^{3a}),-C(R^{3a})= N(R^{3a}),-C (O)(R^{3a}),-C (O)₂(R^{3a}),-C (O) N(R^{3a})₂,-NO₂,-N(R^{3a})₂,-N(R^{3a})C (O)(R^{3a}),-N(R^{3a})C (O)₂(R^{3a}), N(R^{3a})C (O) N(R^{3a}) ₂, N(R^{3a})S(O)₂(R^{3a}),-OR^{3a},-OC(O)R^{3a},-OC(O) N(R^{3a}) ₂,-SR^{3a},-S(O)R^{3a},-S(O)₂(R^{3a}),-S(O) N(R^{3a}) ₂ and -S(O)₂N(R^{3a})_{2,}wherein the C1-C6 alkyl, C2-6 alkenyl, C2-6 alkynyl, C3-C6 cycloalkyl and 3-6-membered saturated heterocycloalkyl are optionally substituted by one or more R;
   R^{3a} is independently selected from H, C1-C6 alkyl, 3-6-membered cycloalkyl and 3-6-membered heterocycloalkyl at each occurrence, wherein the C1-C6 alkyl, 3-6-membered cycloalkyl, and 3-6-membered heterocycloalkyl are optionally and independently substituted by one or more R for each occurrence;
   R is independently selected from C1-C6 alkyl, C2-6 alkenyl, C2-6 alkynyl, 3-6-membered cycloalkyl, 3-6-membered heterocycloalkyl, halogen,-CN,-C(R^{30a})= NR(OR^{30a}),-C(R^{30a})= N(R^{30a}),-C (O)(R^{30a}),-C (O)₂(R^{30a}),-C (O) N(R^{30a})₂,-NO₂,-N(R^{30a})₂,-N(R^{30a})C (O)(R^{30a}),-N(R^{30a})C (O)₂(R^{30a}), N(R^{30a})C (O) N(R^{30a}) ₂, N(R^{30a})S(O)₂(R^{30a}),-OR^{30a},-OC(O)R^{30a},-OC(O) N(R^{30a}) ₂,-SR^{30a},-S(O)R^{30a},-S(O)₂(R^{30a}),-S(O) N(R^{30a}) ₂ and - S(O)₂N(R^{30a})₂ at each occurrence, wherein the C1-C6 alkyl, C2-6 alkenyl, C2-6 alkynyl, 3-6-membered cycloalkyl, 3-to 6-membered heterocycloalkyl are optionally and independently substituted by one or more R for each occurrence;
   R^{30a} is independently selected from H and C1-C4 alkyl at each occurrence, wherein C1-C4 alkyl is optionally substituted by one or more R;
   R³⁵ is independently selected from halogen and -OR^{35a} at each occurrence;
   R^{35a} is independently selected from H and C1-C6 alkyl at each occurrence;
   R⁴ is selected from H, halogen, C1-C4 alkyl, N(R^{4a})₂ and -OR^{4a}; and
   R^{4a} is independently selected from H and C1-C6 alkyl at each occurrence; or
   in some embodiments of the present invention, the PTM has the following structure: 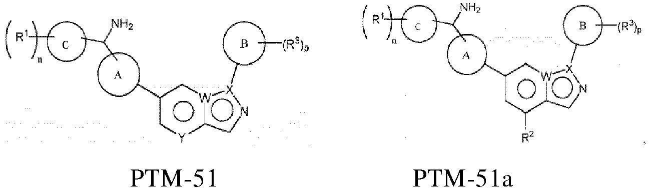 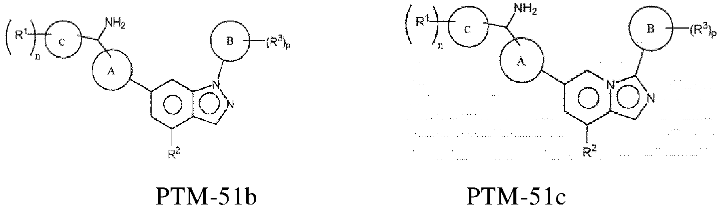
or a pharmaceutically acceptable salt thereof, wherein, in PTM-51, PTM-51a, PTM-51b or PTM-51c:
   ring A is selected from phenyl and 5- or 6-membered heteroaryl;
   ring B is selected from phenyl and 5- or 6-membered heteroaryl;
   ring C is 3- to 6-membered cycloalkyl,
   N is 1, 2 or 3;
   P is 0, 1 or 2;
   one of W and X is N, and the other of W and X is C;
   Y is N or CR²;
   R¹ is selected from C1-C6 alkyl, C2-6 alkenyl, C2-6 alkynyl, halogen,-CN,-C(R^{1a})= NR(OR^{1a}),-C(R^{1a})= N(R^{1a}),-C (O)(R^{1a}),-C (O)₂(R^{1a}),-C (O) N(R^{1a})₂,-NO₂,-N(R^{1a})₂,-N(R^{1a})C (O)(R^{1a}),-N(R^{1a})C (O)₂(R^{1a}), N(R^{1a})C (O) N(R^{1a})₂, N(R^{1a})S(O)₂(R^{1a}₎,-OR^{1a},-OC(O)R^{1a},-OC(O) N(R^{1a})₂,-SR^{1a},-S(O)R^{1a},-S(O)₂(R^{1a}),-S(O) N(R^{1a})₂ or -S(O)₂N(R^{1a})₂, wherein the C1-C6 alkyl, C2-6 alkenyl and C2-6 alkynyl are optionally substituted by one or more R¹⁰;
   R^{1a} is independently selected from H or C1-C6 alkyl at each occurrence, wherein the C1-C6 alkyl is optionally and independently substituted by one or more R¹⁰ at each occurrence;
   R¹⁰ is independently selected from halogen,-CN, -C(R^{10a})= NR(OR^{10a}), -C(R^{10a})= N(R^{10a}), -C (O)(R^{10a}), -C (O)₂(R^{10a}), -C (O) N(R^{10a})₂, -NO₂, -N(R^{10a})₂, -N(R^{10a})C (O)(R^{10a}), - N(R^{10a})C (O)₂(R^{10a}), N(R^{10a})C (O) N(R^{10a})₂, N(R^{10a})S(O)₂(R^{10a}), -OR^{10a}, -OC(O)R^{10a}, - OC(O)N(R^{10a}) ₂, -SR^{10a}, -S(O)R^{10a}, -S(O)₂(R^{10a}), -S(O) N(R^{10a}) ₂ and -S(O)₂N(R^{10a})₂ at each occurrence;
   R^{10a} is independently selected from H or C1-C6 alkyl at each occurrence, wherein the C1-C6 alkyl is optionally substituted by one or more halogens;
   R² is selected from H, C1-C6 alkyl, C2-6 alkenyl, C2-6 alkynyl, 3-7-membered cycloalkyl, 3-7-membered heterocycloalkyl, halogen, -CN, -C(R^{2a})= NR(OR^{2a}), -C(R^{2a})= N(R^{2a}), -C (O)(R^{2a}), -C (O)2(R^{2a}), -C (O)N(R^{2a})₂, -NO₂, -N(R^{2a})₂, -N(R^{2a})C (O)(R^{2a}), - N(R^{2a})C (O)₂(R^{2a}), N(R^{2a})C (O)N(R^{2a}) ₂, N(R^{2a})S(O)₂(R^{2a}), -OR^{2a}, -OC(O)R^{2a}, -OC(O) N(R^{2a}) ₂, -SR^{2a}, -S(O)R^{2a}, -S(O)₂(R^{2a}), -S(O)N(R^{2a}) ₂ and -S(O)₂N(R^{2a})₂, wherein the C1-C6 alkyl, C2-6 alkenyl, C2-6 alkynyl, 3-7-membered cycloalkyl and 3-7-membered heterocycloalkyl are optionally substituted by one or more R²⁰;
   R^{2a} is independently selected from H or C1-C6 alkyl at each occurrence, wherein the C1-C6 alkyl is optionally substituted by one or more R²⁰ at each occurrence;
   R²⁰ is independently selected from C1-C6 alkyl, C2-6 alkenyl, C2-6 alkynyl, C3-7 cycloalkyl, 3-7-membered saturated heterocycloalkyl, halogen,-CN,-C(R^{20a})= NR(OR^{20a}), - C(R^{20a})= N(R^{20a}), -C (O)(R^{20a}), -C (O)₂(R^{20a}), -C (O)N(R^{20a})₂, -NO₂, -N(R^{20a})₂, -N(R^{20a})C (O)(R^{20a}), -N(R^{20a})C (O)₂(R^{20a}), N(R^{20a})C (O) N(R^{20a}) ₂, N(R^{20a})S(O)₂(R^{20a}), -OR^{20a}, - OC(O)R^{20a}, -OC(O) N(R^{20a}) ₂, -SR^{20a}, -S(O) R^{20a}, -S(O)₂(R^{20a}), -S(O) N(R^{20a}) ₂ and - S(O)₂N(R^{20a})₂ at each occurrence, wherein the C1-6 alkyl, C2-6 alkenyl, C2-6 alkynyl, C3-7 cycloalkyl and 3-7-membered saturated heterocycloalkyl are optionally substituted by one or more R²⁵ at each occurrence;
   R^{20a}is independently selected from H and C1-C6 alkyl at each occurrence, wherein the C1-C6 alkyl is optionally substituted by R²⁵;
   R²⁵ is selected from halogen and -OR^{25a};
   R^{25a} is selected from H and C1-C6 alkyl;
   R is selected from C1-C6 alkyl, C2-6 alkenyl, C2-6 alkynyl, C3-C6 cycloalkyl, 3-6-membered saturated heterocycloalkyl, halogen, -CN, -C(R^{3a})= NR(OR^{3a}), -C(R^{3a})= N(R^{3a}), - C(O)(R^{3a}), -C (O)₂(R^{3a}), -C (O)N(R^{3a})₂, -NO₂, -N(R^{3a})₂, -N(R^{3a})C (O)(R^{3a}), -N(R^{3a})C (O)₂(R^{3a}), N(R^{3a})C (O)N(R^{3a}) ₂, N(R^{3a})S(O)₂(R^{3a}), -OR^{3a}, -OC(O)R^{3a}, -OC(O)N(R^{3a}) ₂, - SR^{3a}, -S(O)R^{3a}, -S(O)₂(R^{3a}), -S(O)N(R^{3a}) ₂ and -S(O)₂N(R^{3a})₂, wherein the C1-C6 alkyl, C2-6 alkenyl, C2-6 alkynyl, C3-C6 cycloalkyl and 3-6-membered saturated heterocycloalkyl are optionally substituted by one or more R³⁰;
   R^{3a} is independently selected from H, C1-C6 alkyl, 3-6-membered cycloalkyl and 3-6-membered heterocycloalkyl at each occurrence, wherein the C1-C6 alkyl, 3-6-membered cycloalkyl and 3-6-membered heterocycloalkyl are optionally substituted by one or more R³⁰ at each occurrence;
   R³⁰ is independently selected from C1-C6 alkyl, C2-6 alkenyl, C2-6 alkynyl, 3-6-membered cycloalkyl, 3-6-membered heterocycloalkyl, halogen, -CN, -C(R^{30a})= NR(OR^{30a}), -C(R^{30a})= N(R^{30a}), -C (O)(R^{30a}), -C (O)₂(R^{30a}), -C (O)N(R^{30a})₂, -NO₂, -N(R^{30a})₂, -N(R^{30a})C (O)(R^{30a}), -N(R^{30a})C (O)₂(R^{30a}), N(R^{30a})C (O) N(R^{30a}) ₂, N(R^{30a})S(O)2(R^{30a}), -OR^{30a}, - OC(O)R^{30a}, -OC(O) N(R^{30a}) ₂, -SR^{30a}, -S(O)R^{30a}, -S(O)₂(R^{30a}), -S(O) N(R^{30a}) ₂ and - S(O)₂N(R^{30a})₂ at each occurrence, wherein the C1-C6 alkyl, C2-6 alkenyl, C2-6 alkynyl, 3-6-membered cycloalkyl, 3-6-membered heterocycloalkyl are optionally substituted by one or more R³⁵;
   R^{30a} is independently selected from H or C1-C4 alkyl at each occurrence, wherein C1-C4 alkyl is optionally substituted by one or more R³⁵;
   R³⁵ is independently selected from halogen or -OR^{35a} at each occurrence;
   R^{35a} is independently selected from H or C1-C6 alkyl at each occurrence; or
   in some embodiments of the present invention, the PTM has the following structure: 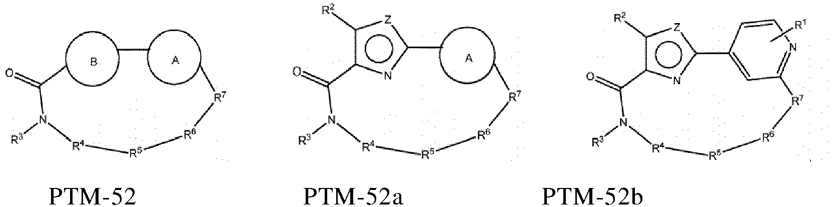 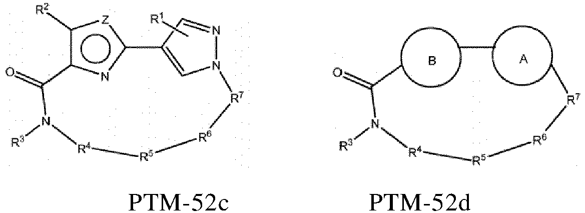
or a pharmaceutically acceptable salt thereof, wherein, in PTM-52, PTM-52a, PTM-52b, PTM-52c, PTM-52d, PTM-52e or PTM-52f:
   ring A is phenyl or 5-6-membered heteroaryl containing 1-3 heteroatoms selected from O, S or N, wherein ring A is optionally substituted by further optionally substituted alkyl;
   ring B is phenyl, 5-6-membered heterocycloalkyl containing 1-3 heteroatoms selected from O, S and N, or 5-6-membered heteroaryl containing 1-3 heteroatoms selected from O, S and N, wherein ring B is optionally substituted by further optionally substituted alkyl,
   R³ is selected from hydrogen, deuterium, alkyl optionally substituted by alkoxy, amino, N-(alkyl) amino, N,N-(dialkyl) amino, phenyl, heterocycloalkyl, heteroaryl, wherein the phenyl, heterocycloalkyl and heteroaryl are each independently optionally substituted by one or two groups selected from alkyl, and the alkoxy is optionally substituted by tri (alkyl) silyl;
   R⁴ is selected from heteroaryl or aryl, each of which is independently optionally substituted, or R⁴ and R³ together with the nitrogen to which they are bound form optionally substituted 3-7-membered heterocycloalkyl , or R⁴ is an alkylene chain containing 1-3 carbon atoms, which is optionally substituted by one or two groups independently selected from alkyl or cycloalkyl, wherein each is optionally substituted by hydroxyl or alkoxy, or R⁴ is absent;
   R⁵ is selected from C(O)NR⁵¹, NR⁵² or O or R⁵is absent, provided that if R⁴ is absent, R⁵ is absent;
   R⁶ is an alkylene or alkenylene chain containing one or two double bonds, wherein the alkylene or alkenylene chain has 2-10 carbon atoms, wherein the alkylene or alkenylene chain is optionally substituted by one or two groups independently selected from alkyl or cycloalkyl, wherein each is optionally substituted by hydroxyl or alkoxy, and wherein one or two carbon atoms in the alkylene chain are optionally substituted by O, S, SO, SO₂ or NR⁶¹, wherein the two carbon atoms in the alkylene chain are optionally connected by an alkylene chain of two or three carbon atoms to form a 5-7-membered ring;
   R⁷ selected from NR⁷¹ or O, or R⁷ is absent;
   R⁵¹ is selected from hydrogen or alkyl;
   R⁵² is selected from hydrogen, deuterium, alkyl and -C(O)OR⁸¹;
   R⁶¹ is selected from hydrogen, deuterium, alkyl and -C(O)OR⁸¹;
   R⁷¹ is selected from hydrogen, deuterium, alkyl and -C(O)OR⁸¹;
   R⁸¹ is alkyl; or
   in some embodiments of the present invention, the PTM has the following structure: 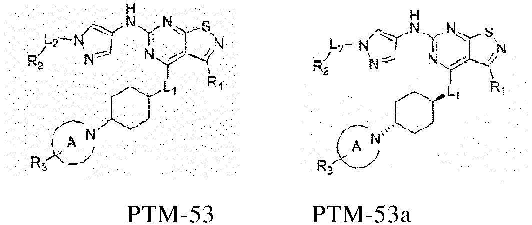
wherein, in PTM-53 or PTM-53a:
   R₁ is selected from CN, C1-C6 alkyl or 3-6-membered heterocycloalkyl, wherein the C1-C6 alkyl and 3-6-membered heterocycloalkyl are optionally substituted by 1, 2 or 3 Rₐ;
   R₂ is selected from C1-C6 alkyl and 3-6-membered heterocycloalkyl, wherein the C1-C6 alkyl and 3-6-membered heterocycloalkyl are optionally substituted by 1, 2 or 3 R_{b};
   R³ is selected from H, F, Cl, Br, I, OH, NH₂, CN, C1-C6 alkyl,-C(O)OC1-C6 alkyl,-C(O)C1-C6 alkyl, C3-C6 cycloalkyl, wherein, the C1-C6 alkyl,-C(O)OC1-C6 alkyl,-C(O)C1-C6 alkyl, C3-C6 cycloalkyl are optionally substituted by 1, 2 or 3 R_{c};
   ring A is selected from 3-10-membered heterocycloalkyl, and the 3-10-membered heterocycloalkyl is optionally substituted by 1, 2 or 3 R_{d};
   L₁ is selected from O and N(R₄);
   L₂ is selected from a single bond, CH₂ and CH₂CH₂;
   R₄ is selected from H and Me;
   each Rₐ is independently selected from F, Cl, Br, I, OH, NH₂, CN and COOH;
   each R_{b} is independently selected from F, Cl, Br, I, OH, NH₂, COOH and Me;
   each R_{c} is independently selected from F, Cl, Br, I, OH, NH₂ or CN;
   each R_{d} is independently selected from F, Cl, Br, I, OH, NH₂ or CN;
   the 3-6-membered heterocycloalkyl comprises 1, 2 or 3 heteroatoms or heteroatom groups independently selected from -O-,-S-,-NH-, N;
   the 3-10-membered heterocycloalkyl comprises 1, 2 or 3 heteroatoms or heteroatom groups independently selected from-O-,-S-,-NH-, N,-C(O)NH-; or
   in some embodiments of the present invention, the PTM has the following structure: 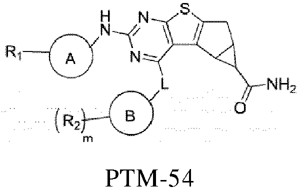
or pharmaceutically acceptable salt thereof, wherein, in PTM-54:
   m is 0, 1, 2 or 3;
   ring A is selected from 5-6-membered heteroaryl;
   ring B is selected from 3-7-membered cycloalkyl, 4-6-membered heterocycloalkyl;
   L is selected from O or NH;
   R₁ is selected from H, or selected from C1-C3 alkyl, or C1-C3 heteroalkyl, which are optionally substituted by 1, 2 or 3 R;
   R₂ is selected from OH, NH₂, CN, halogen, or selected from C1-C3 alkyl, C1-C3 heteroalkyl, or 4-6-membered heterocycloalkyl, which are optionally substituted by 1, 2 or 3 R;
   R is selected from F, Cl, Br, I, OH, NH₂, CN, methyl, ethyl, CF₃;
   the "hetero" of the C1-C3 heteroalkyl, 4-6-membered heterocycloalkyl or 5-6-membered heterocycloalkenyl are independently selected from: N, O, S, NH,-C(O)NH-; the number of the above-mentioned heteroatoms or heteroatom groups is independently selected from 1, 2, 3 or 4 respectively; or
   in some embodiments of the present invention, the PTM has the following structure: 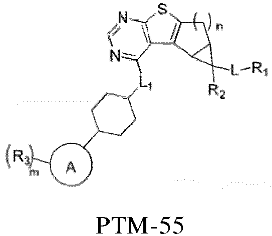
or a pharmaceutically acceptable salt thereof, wherein, in PTM-55:
   n is selected from 1 or 2;
   m is selected from 0, 1, 2 or 3;
   R₁ is selected from H, CN, OH, or selected from 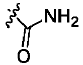 optionally substituted by R;
   R₂ is selected from: H, F, Cl, Br, or I;
   R₃ is selected from OH, NH₂, CN, halogen, or selected from C1-C3 alkyl, or C1-C3 heteroalkyl, which are optionally substituted by 1, 2 or 3 R; or,
   two R³ together with the atom to which they are attached form a 3-6-membered ring;
   L is selected from a single bond,-CH₂-, or -CH₂CH₂-;
   Li is selected from O or NH;
   ring A is selected from 4-6-membered heterocycloalkyl;
   R is selected from F, Cl, Br, I, OH, NH₂, CN, or selected from C1-C3 alkyl or C1-C3 heteroalkyl, which are optionally substituted by 1, 2 or 3 R'; and R' is selected from F, Cl, Br, I, OH, NH₂, CN, or CF₃; the "hetero" of the C1-C3 heteroalkyl and 4-6-membered heterocycloalkyl are respectively independently selected from N, O, S, NH;
   the number of the above heteroatoms or heteroatom groups is each independently selected from 1, 2, 3 or 4; or
   in some embodiments of the present invention, the PTM has the following structure: 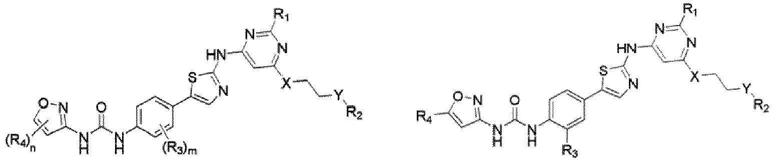 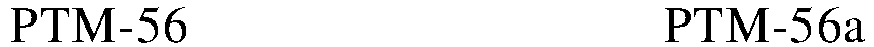
or pharmaceutically acceptable salt thereof, wherein, in PTM-56 or PTM-56a:
   R₁ is H;
   X is O or NRₐ, wherein Rₐ is H or C1-C6 alkyl;
   Y is CR_{b}R_{c} or NR_{d}, wherein R_{b} and R_{c} are each independently H, halogen, C1-C6 alkyl, C1-C6 alkoxy or amino, and R_{d} is H or C1-C6 alkyl; or R_{b} and Rₐ, carbon atom bonded to R_{b} and nitrogen atom bonded to Ra together to form C3-C10 heterocycloalkyl; or R_{d} and Rₐ together with nitrogen atoms to which they are bonded form C3-C10 heterocycloalkyl ;
   R₂ is -CH₂CH₂Rₑ or NR_{f}R_{g}, wherein Rₑ is H, halogen, C1-C6 alkyl or ORₕ, R_{f} and R_{g} are each independently C1-C6 alkyl or C3-C8 cycloalkyl, and Rₕ is H or C1-C6 alkyl, or Rₕ, R_{d}, oxygen atom bonded to Rₕ and nitrogen atom bonded to R_{d} together form C3-C10 heterocycloalkyl;
   R₃ and R₄ are independently H, halogen, nitro, cyano, amino, C1-C6 alkyl, C1-C6 alkoxy, C3-C10 cycloalkyl, C3-C10 heterocycloalkyl, aryl or heteroaryl;
   m is 1, 2, 3 or 4; and
   n is 1 or 2; or
   in some embodiments of the present invention, the PTM has the following structure: 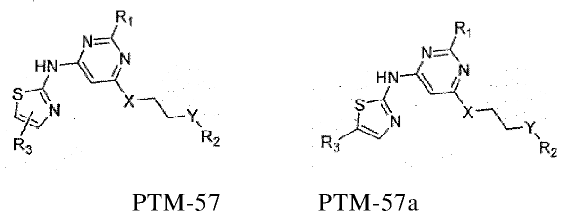
or pharmaceutically acceptable salt thereof; wherein, in PTM-57 or PTM-57a:
   R¹ is C1-C6 alkyl or thioalkyl;
   X is O or NRₐ, wherein Rₐ is H or C1-C6 alkyl;
   Y is CR_{b}R_{c} or NR_{d}, wherein R_{b} and R_{c} are each independently H, halogen, C1-C6 alkyl, C1-C6 alkoxy, amino, or R_{d} is H or C1-C6 alkyl; when X is NRₐ and Y is CR_{b}R_{c}, R_{b} together with Rₐ, the carbon atom bonded to Rₐ and the nitrogen atom bonded to Rₐ form an unsubstituted C3-C10 heterocycloalkyl; or when X is NRₐ and Y is NR_{d}, R_{d} together with Rₐ and the nitrogen atoms to which they are bonded from an unsubstituted C3-C10 heterocycloalkyl;
   R₂ is -CH₂CH₂Rₑ or NR_{f}R_{g}, wherein Rₑ is H, halogen, or ORₕ, and R_{f} and R_{g} are each independently unsubstituted C1-C6 alkyl, and Rₕ is H or unsubstituted C1-C6 alkyl; when Y is NR_{d}, R₂ is -CH₂CH₂Rₑ and Rₑ is ORₕ, Rₕ together with R_{d}, oxygen atom bonded to Rₕ and nitrogen atom bonded to R_{d} from unsubstituted C3-C10 heterocycloalkyl; and
   R₃ is an unsubstituted 6-membered heteroaryl containing one or two nitrogen atoms; or
   in some embodiments of the present invention, the PTM has the following structure: 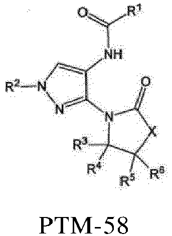
or pharmaceutically acceptable salt thereof; wherein, in PTM-58:
   R¹ is 5-6-membered monocyclic aromatic heterocycloalkyl, 8-14-membered fused polycyclic aromatic heterocycloalkyl, or C6-14 aryl, each is optionally substituted by 1 to 3 substituents selected from the following:
      (1) halogen atom;
      (2) C1-C6 alkyl optionally substituted by 1 to 3 groups selected from halogen or hydroxyl;
      (3) C6-14 aryl optionally substituted by 1 to 3 halogens;
      (4) 5-6-membered monocyclic aromatic heterocycloalkyl, or 8-14 membered fused polycyclic aromatic heterocycloalkyl, each is optionally substituted by 1 to 3 substituents selected from the following:
         (i) amino optionally monosubstituted or bisubstituted by C1-C6 alkyl, wherein, the C1-C6 alkyl is optionally substituted by 1 to 3 substituents selected from: (a) halogen atom, or (b) C3-C10 cycloalkyl;
         (ii) halogen atom;
         (iii) C1-C6 alkoxy;
         (iv) cyano;
         (v) C1-C6 alkyl optionally substituted by 1 to 3 substituents selected from: (a) azido, (b) amino optionally monosubstituted or bisubstituted by C1-C6 alkyl, wherein C1-C6 alkyl is optionally substituted by 1 to 3 substituents selected from halogen atom or C3-C10 cycloalkyl, (c) hydroxyl, (d) halogen atom;
         (vi) formyl;
         (vii) carboxyl;
         (viii) carbamoyl;
         (ix) C3-C10 cycloalkyl; or
         (x) 3-8 membered monocyclic non-aromatic heterocycloalkyl;
      (5) 3-8 membered monocyclic non-aromatic heterocycloalkyl, 9-14 membered fused polycyclic non-aromatic heterocycloalkyl, or 7-14 membered spiro heterocycloalkyl, each is optionally substituted by 1 to 3 substituents selected from the following:
         (i) C1-C6 alkyl optionally substituted by 1 to 3 substituents selected from: (a) hydroxyl, (b) amino optionally monosubstituted or bisubstituted by C1-C6 alkyl, (c) cyano, or (d) C6-14 aryl;
         (ii) oxo;
         (iii) hydroxyl;
         (iv) carbamoyl; or
         (v) thio group;
      (6) C3-C10 cycloalkylsulfonyl;
      (7) C1-C6 alkyl-carbonyl;
      (8) 5-6 membered monocyclic aromatic heterocyclic sulfonyl; or
      (9) halogenated thio group;
   R² is
      (1) C1-C6 alkyl optionally substituted by 1 to 3 substituents selected from the following:
         (i) C1-C6 alkoxy-carbonyl, (ii) C1-C6 alkylsulfonyl, (iii) carbamoyl, (iv) cyano, (v) 3-8 membered monocyclic non-aromatic heterocycloalkyl optionally substituted by 1 to 3 oxos, or (vi) halogen atom;
      (2) C3-C10 cycloalkyl optionally substituted by 1 to 3 hydroxyls; or
      (3) 3-8 membered monocyclic non-aromatic heterocycloalkyl;
   R₃ and R₄ independently are
      (1) hydrogen atom; or
      (2) C1-C6 alkyl optionally substituted by 1 to 3 substituents selected from amino, wherein the amino is optionally monosubstituted or bisubstituted by C1-C6 alkyl;
   R₅ and R₆ independently are
      (1) hydrogen atom;
      (2) hydroxyl;
      (3) C1-C6 alkyl optionally substituted by 1 to 3 substituents selected from: (i) hydroxyl; (ii) amino optionally monosubstituted or bisubstituted by substituents selected from: (a) C1-C6 alkyl optionally substituted by 1 to 3 halogen atoms; (b) C3-C10 cycloalkyl optionally substituted by 1 to 3 halogen atoms; (c) 3-8 membered monocyclic non-aromatic heterocycloalkyl; (d) C1-C6 alkyl sulfonyl; (e) C1-C6 alkyl-carbonyl; or (f) C3-C10 cycloalkyl-carbonyl; (iii) halogen atoms; (iv) C1-C6 alkyl thio; (v) C1-C6 alkyl sulfinyl; or (vi) C1-C6 alkyl sulfonyl;
      (4) C1-C6 alkoxy;
      (5) amino optionally monosubstituted or bisubstituted by substituents selected from the following: (i) C1-C6 alkyl, (ii) C1-C6 alkyl, (iii) C1-C6 alkyl sulfonyl;
      (6) 3-8 membered monocyclic non-aromatic heterocycloalkyl;
      (7) carboxyl; or
      (8) carbamoyl optionally monosubstituted or bisubstituted by C1-C6 alkyl; or
   R⁵ and R⁶ are combined to optionally form: (1) 3-8 membered monocyclic non-aromatic heterocyclyl; or (2) C3-C10 cycloalkyl;
   X is CR⁷R⁸, NR⁹, O or S;
   R⁷ and R⁸ are independently: (1) hydrogen atom; (2) cyano; (3) C1-C6 alkyl optionally substituted by 1 to 3 hydroxyls; or (4) hydroxyl;
   R⁷ and R⁸ are combined to optionally form:
      (1) C3-C10 cycloalkyl optionally substituted by 1 to 3 substituents selected from: (i) oxo; or (ii) hydroxyl;
      (2) 3-8 membered monocyclic non-aromatic heterocyclyl optionally substituted by 1 to 3 C7-16 (aryl) alkyl-;
   R⁹ is:
      (1) hydrogen atom;
      (2) C1-C6 alkyl optionally substituted by 1 to 3 substituents selected from: (i) hydroxyl; (ii) C1-C6 alkoxy optionally substituted by 1 to 3 C6-14 aryls; or (iii) amino optionally monosubstituted or bisubstituted by C1-C6 alkyl;
      (3) C2-6 alkenyl; or
      (4) C7-16 (aryl) alkyl- optionally substituted by 1 to 3 C1-C6 alkoxys; or
   in some embodiments of the present invention, the PTM has the following structure: 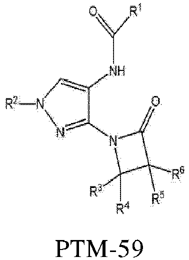
or a pharmaceutically acceptable salt thereof; in PTM-59:
   R¹ is optionally substituted heteroaryl or C6-14 aryl;
   R², R³, R⁴, R⁵, and R⁶ are independently hydrogen or substituents; or
   in some embodiments of the present invention, the PTM has the following structure: 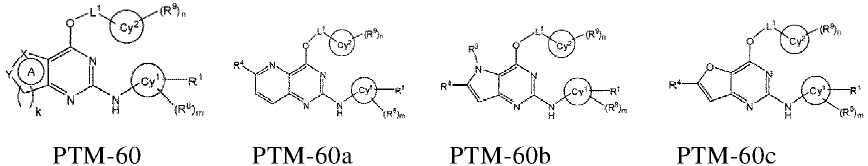 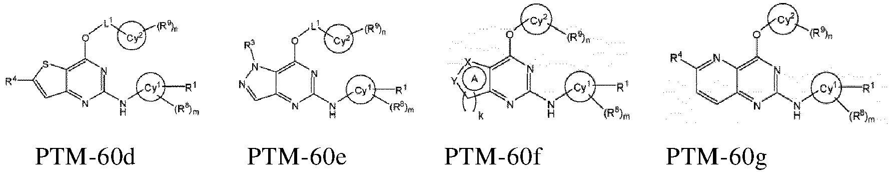 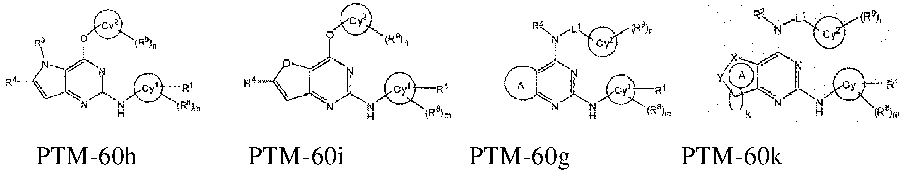 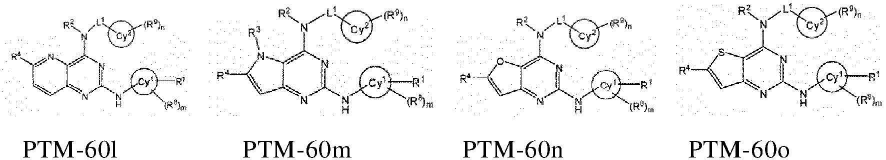 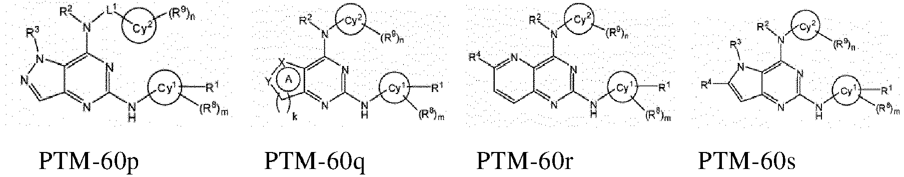 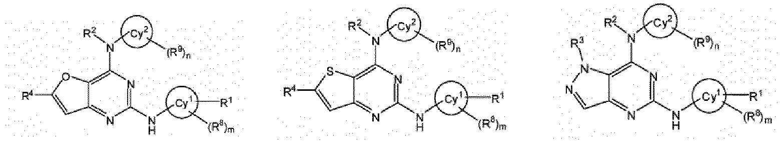 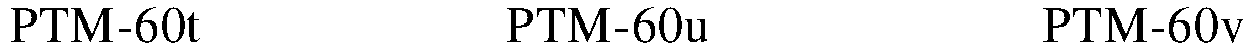
or a pharmaceutically acceptable oxide, a pharmaceutically acceptable solvate and/or a pharmaceutically acceptable salt thereof, wherein, in PTM-60, PTM-60a, PTM-60b, PTM-60c, PTM-60d, PTM-60e, PTM-60f, PTM-60g, PTM-60h, PTM-60i, PTM-60j, PTM-60k, PTM-601, PTM-60m, PTM-60n, PTM-60o, PTM-60p, PTM-60q, PTM-60r, PTM-60s, PTM-60t, PTM-60u or PTM-60v:
   ring A is substituted or unsubstituted, saturated, unsaturated or partially unsaturated 5-6-membered heterocycloalkyl, provided that the heterocycloalkyl is not thiazolyl;
   Cy¹ is 5-membered heteroaryl;
   Cy² is a C3-12 cycloalkyl optionally fused with 5- or 6-membered heteroaryl or 5- to 7-membered heteroaryl;
   L¹ is a bond or C1-C3 alkylene, and one of the CH₂ groups is optionally replaced by O or NH;
   X is N, NR³, O or S;
   Y is CR⁴ or N;
   when X is N, then k is 2 and ring A is 6-membered heteroaryl; when X is NR³, S or O, then k is 1 and ring A is 5-membered heteroaryl;
   R¹ is selected from H, substituted or unsubstituted C1-C3 alkyl, substituted or unsubstituted C1-C4 alkoxy, substituted or unsubstituted C3-C6 cycloalkyl, substituted or unsubstituted 3-7-membered heterocycloalkyl, substituted or unsubstituted C6-12 aryl, substituted or unsubstituted 5-7-membered heteroaryl, halogen, CN, -NO₂, -OR¹⁰, -SR¹⁰, - NR¹¹R¹² , -C(O)R¹⁴, -C(O) NR¹¹R¹², -NR¹³C(O)R¹⁴, -OC(O)R¹⁴, -C(O)₂R¹¹, -NR¹³ C(O) NR¹¹R¹², -NR¹³S(O)₂R¹⁴, -S(O)₂ NR¹¹R¹², -S(O)R¹⁴, -S(O)₂ R¹⁴; provided that R¹ is not - NH₂;
   R² is selected from H, or substituted or unsubstituted C1-C3 alkyl;
   R³ is selected from H, substituted or unsubstituted C1-C4 alkyl, substituted or unsubstituted C2-4 alkenyl, substituted or unsubstituted C2-4 alkynyl, substituted or unsubstituted C3-C6 cycloalkyl, substituted or unsubstituted C3-C6 cycloalkenyl, substituted or unsubstituted 3-7-membered heterocycloalkyl, substituted or unsubstituted C5-12 aryl, substituted or unsubstituted 5-7-membered-heteroaryl,-CN,-C(O)R⁶ and -C(O)NR^{7a}R^{7b};
   R⁴ is selected from H, substituted or unsubstituted C1-4 alkyl, substituted or unsubstituted C2-4 alkenyl, substituted or unsubstituted C2-4 alkynyl, substituted or unsubstituted C1-C4 alkoxy, substituted or unsubstituted C3-C6 cycloalkyl, substituted or unsubstituted C3-C6 cycloalkenyl, substituted or unsubstituted 3-7-membered heterocycloalkyl, substituted or unsubstituted C5-12 aryl, substituted or unsubstituted 5-7-membered heteroaryl, -OH, -OR⁶, -CN, -C(O)R⁶, -C(O)OR⁶, -C(O)NR^{7a}R^{7b}, COOH or halogen;
   R⁶ is selected from substituted or unsubstituted C1-C4 alkyl, substituted or unsubstituted C3-C6 cycloalkyl, substituted or unsubstituted 3-7-membered heterocycloalkyl, substituted or unsubstituted C6-12 aryl, substituted or unsubstituted 5-7-membered heteroaryl;
   R^{7a} and R^{7b} are each independently selected from H, or substituted or unsubstituted C1-C3 alkyl; or R^{7a} and R^{7b} together with the N to which they are attached form substituted or unsubstituted 3-7-membered heterocycloalkyl;
   each R⁸ is independently selected from C1-C4 alkyl, C1-C4 haloalkyl, halogen, CN or - NO₂;
   each R⁹ is independently selected from C1-C4 alkyl, C1-C4 haloalkyl, C3-C8 cycloalkyl, 3-7-membered heterocycloalkyl, phenyl, 5-7-membered heteroaryl, halogen, CN, -NO₂, -OR¹⁰, -SR¹⁰, -NR¹¹R¹², -C(O)R¹⁴, -C(O)NR¹¹R¹², -NR¹³C(O)R¹⁴, -OC(O)R¹⁴, - C(O)₂R¹¹, -NR¹³C(O) NR¹¹R¹², -NR¹³S(O)₂R¹⁴, -S(O)₂ NR¹¹R¹², -S(O)R¹⁴, -S(O)₂R¹⁴;
   R¹⁰ is selected from H, C1-C4 alkyl, C2-4 alkenyl, C2-4 alkynyl, C3-C8 cycloalkyl, 3-7-membered heterocycloalkyl, phenyl and 5-7-membered heteroaryl;
   R¹¹ and R¹² are independently H, C1-C4 alkyl, C3-C8 cycloalkyl, 3-7-membered heterocycloalkyl, phenyl, and 5-7-membered heteroaryl; or R¹¹ and R¹² together with the nitrogen to which they are attached form 3-7-membered heterocycloalkyl ;
   R¹³ is selected from H or C1-C4 alkyl;
   R¹⁴ is selected from C1-C4 alkyl, C3-C8 cycloalkyl, 3-7-membered heterocycloalkyl, phenyl or 5-7-membered heteroaryl;
   m is 0, 1 or 2; and
   n is 0, 1, or 2; or
   in some embodiments of the present invention, the PTM has the following structure: 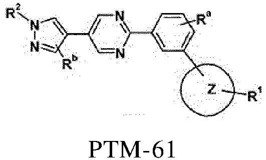
and a pharmaceutically acceptable tautomer, salt and stereoisomer thereof, including mixtures thereof at all ratios; wherein, in PTM-61:
   R¹ is absent or is A or Q-Het,
   Z is 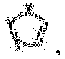 wherein X is O, S or N, Y is C or N, T is C or N, or Z represents pyridinyl or pyridazinyl;
   R^{a} is absent or is OR³, CF₃, Hal, NO₂;
   R^{b} is absent or is selected from A or COHet;
   R² is H, Het, Q-Het, Cyc, A or OA,
   Het represents a 4-9 membered monocyclic or fused, spiro or bridged bicyclyl, which is saturated, unsaturated or aromatic, and contains 1-3 groups independently selected from N, O, S, CO, SO or SO₂, and wherein one or two H atoms can be substituted by A, OA, COA, CN, Hal, NO₂, OR³, SOA or SO₂A;
   Cyc represents a 4-8-membered saturated carbocycle, which optionally contains SO, SO₂, CO and is optionally monosubstituted or bisubstituted by groups selected from CO(NR³)₂and COHet, OR³, Het1, A, CH₂Het1, NH₂, NHCOA, OCH₂Cyc1, SO₂A or - SA(=NH)(=O),
   Q represents a linear or branched alkylene containing 1-6 carbon atoms, wherein 1-5 H atoms can be substituted by groups independently selected from OR³, Hal, N(R³)₂, and wherein 1 or 2 CH₂ groups can be substituted by groups independently selected from CO, SO, SO₂ and NR³, or Q represents a 4-8-membered divalent heterocyclyl, which is saturated, unsaturated or aromatic, and contains 1-3 heteroatoms independently selected from N, O and S;
   A represents a linear or branched alkyl containing 1-10 carbon atoms, wherein 1-7 H atoms can be substituted by groups independently selected from -OR³, Hal, NHSO₂A, SO₂A, SOA, N(R³)₂, and wherein 1, 2 or 3 non-adjacent CH₂-groups can be replaced by groups independently selected from -CO-, NR³and/or -O-;
   Hal is F, Cl, Br or I,
   R³ represents H or C1-C6 alkyl, wherein one H atom can be substituted by a group selected from OH, O-C1-C6 alkyl or Hal,
   Het1 represents a 5- or 6-membered saturated monocyclic heterocyclyl, which contains 1-3 N- and/or O- atoms, which is optionally monosubstituted by A;
   Cyc1 represents a cycloalkyl containing 3-7 atoms; or
   in some embodiments of the present invention, the PTM has the following structure: 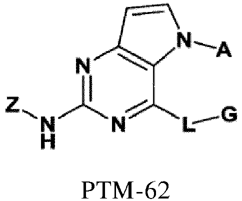
or a pharmaceutically acceptable salt thereof; wherein, in PTM-62:
   A is phenyl or 5-10 membered heteroaryl, optionally substituted by one or more R¹ groups;
   L is selected from O or NR²;
   G is C1-C6 alkyl, C3-C6 cycloalkyl, 4-6-membered heterocycloalkyl or phenyl, optionally substituted by one or more R³ groups;
   Z is phenyl or 5-10 membered heteroaryl, optionally substituted by one or more R⁴ groups;
   each R¹ is independently selected from C1-C6 alkyl, 4-6-membered heterocycloalkyl, hydroxyl, halogen, C1-C6 alkoxy C1-C6 alkyl, C1-C6 alkoxy, amino, (C1-C6 alkyl)₂amino, carboxyl or C1-C6 alkyl sulfonyl;
   R² is selected from hydrogen or C1-C6 alkyl;
   each R³ is independently selected from hydroxyl, oxo, C1-C6 alkyl, 4-6-membered heterocycloalkyl, 5-10-membered heteroaryl, hydroxyl C1-C6 alkyl, C1-C6 alkoxy, C1-C6 alkyl acyl amino, C1-C6 alkyl carbonyl or C1-C6 alkyl amino acyl;
   each R⁴ is independently selected from the group consisting of C1-C6 alkyl, cyano, C3-C6 cycloalkyl, 4-6-membered heterocycloalkyl, phenyl, 5-10-membered heteroaryl, oxo, halogenated C1-C6 alkyl, hydroxyl C1-C6 alkyl, 4-6-membered heterocycloalkyl carbonyl or R^{4a}R^{4b}NC(O)-;
   each R^{4a} and R^{4b} are independently selected from hydrogen, deuterium, C1-C6 alkyl, halogenated C1-C6 alkyl, C3-C6 cycloalkyl, C1-C6 alkoxy C1-C6 alkyl, 4-6-membered heterocycloalkyl C1-C6 alkyl, 5-10-membered heteroaryl C1-C6 alkyl, or 4-6-membered heterocycloalkyl;
   wherein in R¹, R³, R⁴, R^{4a}, and R^{4b}, the cycloalkyl, heterocycloalkyl, aryl, and heteroaryl, as an independent group or as part of a group, are optionally substituted by one or more substituents independently selected from C1-C6 alkyl or oxo; wherein the heterocycloalkyl and the heteroaryl contain one or more heteroatoms selected from nitrogen, oxygen and sulfur; or
   in some embodiments of the present invention, the PTM has the following structure: 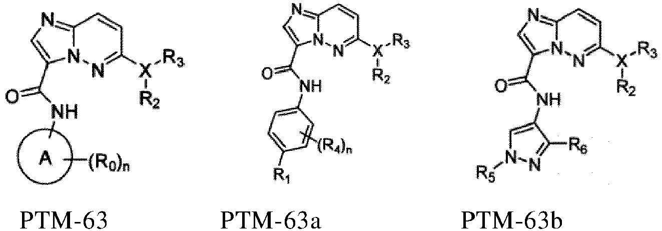
or a pharmaceutically acceptable salt thereof, wherein, in PTM-63, PTM-63a or PTM-63b:
   A ring is an aromatic ring or an aromatic heterocyclyl;
   n is an integer of 0-4;
   X is selected from N or O;
   R₀ is selected from C1-C4 alkyl, heterocyclyl, -OCH₃, -CF₃, -CHF₂, -CN, -CONH₂, halogen, -(CH₂)ₘ-Rₐ, -O-(CH₂)ₘ-Rₐ;
   m is an integer of 1-4;
   Rₐ is a nitrogen-containing heterocyclyl, wherein the nitrogen-containing heterocyclyl may optionally be substituted by one or more substituents selected from halogen, -OH, -CH₃, -OCH₃;
   R₂ and R₃ are independently selected from hydrogen atoms, heterocyclyl, -CH₂CH₂-N(R_{b})₂, R₂ and R₃ can be connected by nitrogen atoms to form a heterocyclyl, wherein the above heterocyclyl can be further substituted by one or more R_{c};
   R_{b} is selected from hydrogen atom, C1-4 alkyl or acetyl;
   R_{c} is selected from -CF₃,-CHF₂,-OH,-NH₂ or formyl; or
   in some embodiments of the present invention, the PTM has the following structure: 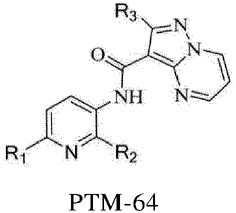
or a pharmaceutically acceptable salt thereof; wherein, in PTM-64:
   R₁ represents hydrogen, deuterium, halogen, hydroxyl, nitro, trifluoromethyl, cyano, NH(CO)R₄, C1-C8 alkyl, C1-C8 alkoxy or C3-C6 cycloalkyl;
   R₂ represents hydrogen, deuterium, halogen or NR₅R₆;
   R₃ represents hydrogen, deuterium, halogen or amino;
   R₄ represents C1-C8 alkyl, C3-C6 cycloalkyl, C5-C10 aryl, or C5-C10 arylheterocycloalkyl; wherein the arylheterocycloalkyl can optionally contain one or more other heteroatoms selected from O, S, or N; wherein the aryl or arylheterocycloalkyl can optionally be monosubstituted to pentasubstituted by the following the same or different substituents, and the substituents are selected from: halogen, trifluoromethyl, cyano, nitro or hydroxyl;
   R₅ and R₆ may be the same or different, and represent hydrogen, deuterium, C1-C8 alkyl, or R⁵ and R⁶ together with the nitrogen to which they are attached form a 5-7-membered heterocycloalkyl which may optionally contain one or more other heteroatoms selected from O, S or N, and the heterocycloalkyl may optionally be monosubstituted to pentasubstituted by the following same or different substituents, the substituents are selected from: formamide, halogen, hydroxyl, nitro, cyano or amino; or
   in some embodiments of the present invention, the PTM has the following structure: 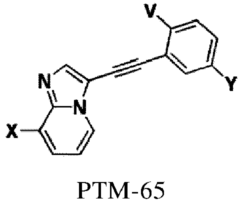
or a pharmaceutically acceptable salt thereof; wherein, in PTM-65:
   V is hydrogen, deuterium, halogen or substituted or non-substituted C1-5 linear or branched alkyl, wherein the substituted alkyl may be substituted by one or more substituents selected from hydroxyl, halogen, nitro or -CN;
   X is -NHR¹;
   R¹ is a unsubstituted or substituted 6-10-membered aryl or a unsubstituted or substituted 5-10-membered heteroaryl containing one or more heteroatoms selected from N, O or S, wherein substituted aryl or substituted heteroaryl can be substituted by the following s: unsubstituted C1-10 linear or branched alkyl, or C1-10 linear or branched alkyl substituted by one or more substituents selected from halogen, methoxy and dimethylamine, or halogen, or amino, or 5-10 membered heterocycloalkyl containing one or more heteroatoms selected from N, O or S, or substituted aryl or substituted heteroaryl is fused with a C3-C10 ring or a 5-10 membered ring containing one or more heteroatoms selected from N, O or S;
   Y is -(C=O)NHR²,-NH(C=O)R², or -NH(C=O)NHR²;
   R² is a non-substituted or substituted 6-10-membered aryl, or a unsubstituted or substituted 5-10-membered heteroaryl containing one or more heteroatoms selected from N, O, and S, wherein, the substituted aryl or substituted heteroaryl can be substituted by one or more substituents selected from the followings: halogen, -CH₂-R³, C₁₋₁₀ linear or branched alkyl substituted or unsubstituted by halogen, C1-2 alkoxy unsubstituted or substituted by halogen, C6-C10 cycloalkyl unsubstituted or substituted by halogen, substituted or unsubstituted 5-10 membered heterocycloalkyl containing one or more heteroatoms selected from N, O and S, unsubstituted or substituted 5-10 membered heteroaryl containing one or more heteroatoms selected from N, O and S and unsubstituted or substituted amino, wherein, the substituted heteroaryl, the substituted heterocycloalkyl and the substituted amino can be substituted by unsubstituted or substituted C1-C3 linear or branched alkyl, wherein the substituted C1-C3 linear or branched alkyl can be substituted by dimethylamino;
   R³ is a 5-10 membered heterocycloalkyl containing one or more heteroatoms selected from N, O and S, which can be substituted by one or more substituents selected from methyl, ethyl, dimethylamino and halogen or is unsubstituted; or
   in some embodiments of the present invention, the PTM has the following structure: 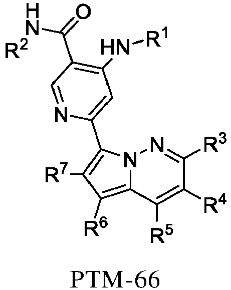
or a pharmaceutically acceptable salt thereof; wherein, in PTM-66:
   R¹ and R² are each independently selected from: a) C1-10 alkyl, which is optionally substituted by Z¹; b) C3-C10 cycloalkyl, which is optionally substituted by Z¹; c) 5-10-membered heteroaryl, which is optionally substituted by Z¹; d) 6-10-membered aryl, which is optionally substituted by Z¹; e) 4-7-membered monocyclic heterocycloalkyl, which is optionally substituted by Z¹; f) 6-12-membered bicyclic heterocycloalkyl, which is optionally substituted by Z¹; or g) -N(R¹²)(R¹²),-S(O)₂R¹²,-S(O)₂N(R¹²)(^{R12}), or -H;
   R³ and R⁴ are each independently selected from: a) H, halogen, -NO₂, -CN, -O-R¹², - C(O)-R¹², -C(O)-N(R¹²)(R¹²), -N(R¹²)(R¹²), -N(R¹²)C(O)-R¹², -N(R¹²)C(O)O-R¹², - N(R¹²)S(O)₂(R¹²), -N(R¹²)C(O)-N(R¹²)(R¹²), -S(O)₂R¹² or -S(O)₂N(R¹²)(R¹²);
   b) C1-9 alkyl, which is optionally substituted by Z¹;
   c) C2-9 alkynyl, which is optionally substituted by Z¹;
   d) C2-9 alkenyl, which is optionally substituted by Z¹;
   e) 5-10 membered heteroaryl, which is optionally substituted by Z¹;
   f) 6-10 membered aryl, which is optionally substituted by Z¹;
   g) 4-12-membered heterocycloalkyl, which is optionally substituted by Z¹; or
   h) C3-C10 cycloalkyl, which is optionally substituted by Z¹;
   R⁵, R⁶ and R⁷ are each independently selected from: a) H, halogen, -NO₂, -CN, -O-R¹², - C(O)-R¹², -C(O)-N(R¹²)(R¹²), -N(R¹²)(R¹²), -N(R¹²)C(O)-R¹², -N(R¹²)C(O)O-R¹², or - N(R¹²)S(O)₂(R¹²);
   b) C1-5 alkyl, which is optionally substituted by Z¹; or
   c) cyclopropyl, oxetanyl or azetanyl, which is optionally substituted by Z¹;
   Z¹ is independently oxo, halogen,-NO₂,-N₃,-CN, C1-9 alkyl, C2-6 alkenyl, C2-6 alkynyl, C3-15 cycloalkyl, C1-C8 haloalkyl, aryl, heteroaryl, heterocycloalkyl, -O-R¹², - C(O)-R¹², -C(O)O-R¹², -C(O)-N(R¹²)(R¹²), -N(R¹²)(R¹²), -N(R¹²)₂(R¹²)⁺, -N(R¹²)C(O)-R¹², - N(R¹²)C(O)O-R¹², -N(R¹²)C(O)N(R¹²)(R¹²), -N(R¹²)S(O)₂(R¹²), -NR¹²S(O)₂N(R¹²)(R¹²), - NR¹²S(O)₂O(R¹²), -OC(O)R¹², -OC(O)OR¹², -OC(O)-N(R¹²)(R¹²), -Si(R¹²)₃, -S-R¹², - S(O)R¹², -S(O)(NH)R¹², -S(O)₂R¹² or -S(O)₂N(R¹²)(R¹²);
   wherein, any alkyl, alkenyl, alkynyl, cycloalkyl, haloalkyl, aryl, heteroaryl or heterocycloalkyl is optionally substituted by Z^{1a}; each Z^{1a} is independently oxo, halogen,-NO₂,-CN,-N₃, C1-9 alkyl, C2-6 alkenyl, C2-6 alkynyl, C3-15 cycloalkyl, C1-C8 haloalkyl, aryl, heteroaryl, heterocycloalkyl, -O-R¹², -C(O)R¹², -C(O)O-R¹², -C(O)N(R¹²)(R¹²), - N(R¹²)(R¹²), -N(R¹²)₂(R¹²)+, -N(R¹²)-C(O)R¹², -N(R¹²)C(O)O(R¹²), -N(R¹²)C(O)N(R¹²)(R¹²), -N(R¹²)S(O)₂(R¹²), -N(R¹²)S(O)₂-N(R¹²)(R¹²), -N(R¹²)S(O)₂O(R¹²), -OC(O)R¹², - OC(O)OR¹², -OC(O)-N(R¹²)(R¹²), -Si(R¹²)₃, -S-R¹², -S(O)R¹², -S(O)(NH)R¹², -S(O)₂R¹² or - S(O)₂N(R¹²)(R¹²);
   wherein, any alkyl, alkenyl, alkynyl, cycloalkyl, haloalkyl, aryl, heteroaryl or heterocycloalkyl is optionally substituted by Z^{1b};
   each R¹² is independently H, C1-9 alkyl, C2-6 alkenyl, C2-6 alkynyl, C3-15 cycloalkyl, aryl, heteroaryl or heterocycloalkyl;
   wherein, any alkyl, alkenyl, alkynyl, cycloalkyl, aryl, heteroaryl or heterocycloalkyl is optionally substituted by Z^{1a}; each Z^{1b} is independently oxo, hydroxyl, halogen,-NO₂,-N₃,-CN, C1-9 alkyl, C2-6 alkenyl, C2-6 alkynyl, C3-15 cycloalkyl, C1-C8 haloalkyl, aryl, heteroaryl, heterocycloalkyl,-O(C1-9 alkyl),-O(C2-6 alkenyl),-O(C2-6 alkynyl),-O(C3-15 cycloalkyl) ,-O(C1-C8 haloalkyl), -O(aryl), -O(heteroaryl), -O (heterocycloalkyl), -NH₂, - NH(C1-9 alkyl), -NH(C2-6 alkenyl), -NH(C2-6 alkynyl), -NH(C3-15 cycloalkyl), -NH(C1-C8 haloalkyl), -NH (aryl), -NH (heteroaryl), -NH (heterocycloalkyl), -N(C1-9 alkyl)₂, - N(C3-15 cycloalkyl)₂, -N(C2-6 alkenyl)₂, -N(C2-6 alkynyl)₂, -N(C3-15 cycloalkyl)₂, -N(C1-C8 haloalkyl)₂, -N (aryl)₂, -N (heteroaryl)₂, -N (heterocycloalkyl)2, -N(C1-9 alkyl)(C3-15 cycloalkyl), -N(C1-9 alkyl)(C2-6 alkenyl), -N(C1-9 alkyl)(C2-6 alkynyl), -N(C1-9 alkyl)(C3-15 cycloalkyl), -N(C1-9 alkyl)(C1-C8 haloalkyl), -N(C1-9 alkyl)(aryl), -N (C1-9 alkyl)(heteroaryl), -N(C1-9 alkyl)(heterocycloalkyl), -C(O)(C1-9 alkyl), -C(O)(C2-6 alkenyl), -C(O)(C2-6 alkynyl), -C(O)(C3-15 cycloalkyl), -C(O)(C1-C8 haloalkyl), - C(O)(aryl), -C(O)(heteroaryl), -C(O)(heterocycloalkyl), -C(O)O(C1-9 alkyl), -C(O)O(C2-6 alkenyl), -C(O)O(C2-6 alkynyl), -C(O)O(C3-15 cycloalkyl), -C(O)O(C1-C8 haloalkyl), - C(O)O (aryl), -C(O)O (heteroaryl), -C(O)O (heterocycloalkyl), -C(O)NH₂, -C(O)NH(C1-9 alkyl), -C(O)NH(C2-6 alkenyl), -C(O)NH(C2-6 alkynyl), -C(O)NH (C3-15 cycloalkyl), - C(O)NH(C1-C8 haloalkyl), -C(O)NH (aryl), -C(O)NH (heteroaryl), -C(O)NH (heterocycloalkyl), -C(O)N(C1-9 alkyl)₂, -C(O)N(C3-15 cycloalkyl)₂, -C(O)N(C2-6 alkenyl)₂, -C(O)N(C2-6 alkynyl)₂, -C(O)N(C3-15 cycloalkyl)₂, -C(O)N(C1-C8 haloalkyl)₂, - C(O)N (aryl)₂, -C(O)N (heteroaryl)₂, -C(O)N (heterocycloalkyl)₂, -NHC(O)(C1-9 alkyl), - NHC(O)(C2-6 alkenyl), -NHC(O)(C2-6 alkynyl), -NHC(O)(C3-15 cycloalkyl), -NHC(O)(C1-C8 haloalkyl), -NHC(O)(aryl), -NHC(O)(heteroaryl), -NHC(O) (heterocycloalkyl), - NHC(O)O(C1-9 alkyl), -NHC(O)O(C2-6 alkenyl), -NHC(O)O(C2-6 alkynyl), - NHC(O)O(C3-15 cycloalkyl), -NHC(O)O(C1-C8 haloalkyl), -NHC(O)O (aryl), -NHC(O)O (heteroaryl), -NHC(O)O (heterocycloalkyl), -NHC(O)NH(C1-9 alkyl), -NHC(O)NH(C2-6 alkenyl), -NHC(O)NH(C2-6 alkynyl), -NHC(O)NH(C3-15 cycloalkyl), -NHC(O)NH(C1-C8 haloalkyl), -NHC(O)NH (aryl), -NHC(O)NH (heteroaryl), -NHC(O)NH (heterocycloalkyl), - SH, -S(C1-9 alkyl), -S(C2-6 alkenyl), -S(C2-6 alkynyl), -S(C3-15 cycloalkyl), -S(C1-C8 haloalkyl), -S (aryl), -S (heteroaryl), -S (heterocycloalkyl), -NHS(O)(C1-9 alkyl), -N(C1-9 alkyl)(S(O)(C1-9 alkyl), -S(O)N(C1-9 alkyl)₂, -S(O)(C1-9 alkyl), -S(O)(NH)(C1-9 alkyl), - S(O)(C2-6 alkyl), -S(O)(C2-6 alkynyl), -S(O)(C3-15 cycloalkyl), -S(O)(C1-C8 haloalkyl), - S(O)(aryl), -S(O)(heteroaryl), -S(O)(heterocycloalkyl), -S(O)₂(C1-9 alkyl), -S(O)₂(C2-6 alkenyl), -S(O)₂(C2-6 alkynyl), -S(O)₂(C3-15 cycloalkyl), -S(O)₂(C1-C8 haloalkyl), - S(O)₂(aryl), -S(O)₂(heteroaryl), -S(O)₂(heterocycloalkyl), -S(O)₂NH(C1-9 alkyl), or - S(O)₂N(C1-9 alkyl)₂;
   wherein, any alkyl, cycloalkyl, aryl, heteroaryl or heterocycloalkyl is optionally substituted by one or more halogens, C1-9 alkyl, C1-C8 haloalkyl, -OH, -NH₂, -NH(C1-9 alkyl), -NH(C3-15 cycloalkyl), -NH(C1-C8 haloalkyl), -NH (aryl), -NH (heteroaryl), -NH (heterocycloalkyl), -N(C1-9 alkyl)₂, -N(C3-15 cycloalkyl)₂, -NHC(O)(C3-15 cycloalkyl), - NHC(O)(C1-C8 haloalkyl), -NHC(O)(aryl), -NHC(O)(heteroaryl), - NHC(O)(heterocycloalkyl), -NHC(O)O(C1-9 alkyl), -NHC(O)O(C2-6 alkynyl), - NHC(O)O(C3-15 cycloalkyl), -NHC(O)O(C1-C8 haloalkyl), -NHC(O)O (aryl), -NHC(O)O (heteroaryl), -NHC(O)O (heterocycloalkyl), -NHC(O)NH(C1-9 alkyl), -S(O)(NH)(C1-9 alkyl), S(O)₂(C1-9 alkyl), -S(O)₂(C3-15 cycloalkyl), -S(O)₂(C1-C8 haloalkyl), - S(O)₂(aryl), -S(O)₂(heteroaryl), -S(O)₂(heterocycloalkyl), -S(O)₂NH(C1-9 alkyl), - S(O)₂N(C1-9 alkyl)₂, -O(C3-15 cycloalkyl), -O(C1-C8 haloalkyl), -O (aryl), -O (heteroaryl), -O (heterocycloalkyl), or -O(C1-9 alkyl); provided that: when R¹ is C3 alkyl, R² is F or hydroxyl substituted C5 alkyl, R³, R⁵, R⁶, R⁷ are H, and when R⁴ is CN, R¹ is substituted by Z¹; or
   in some embodiments of the present invention, the PTM has the following structure: 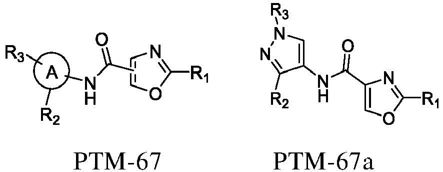 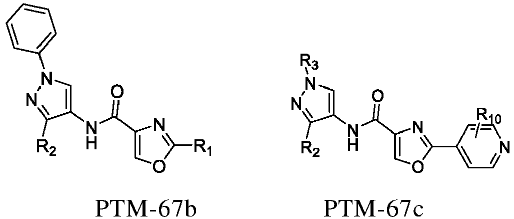
wherein, in PTM-67, PTM-67a, PTM-67b or PTM-67c:
   ring A is monocyclic heteroaryl;
   R₁ is hydrogen or monocyclic or bicyclic heteroaryl optionally substituted by 1-3 R₁₀;
   R₂ is hydrogen, deuterium, R₀, cyano, 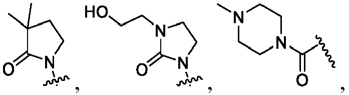 haloalkyl, - C(O)NH₂, -C(O)NHR₀, -C(O)NH-R₀-OH, -C(O)NH-R₀-N (alkyl)₁₋₂, -C(O)NH-R₀₀-OR₀, - C(O)N(R₀)₂, -C(O)NH-cycloalkyl, -C(O)NH-heterocycloalkyl, -C(O)NH-(pyrazolyl optionally substituted by R₀), -C(O)-R₀, -C(O)-cycloalkyl, -S(O)₂NH₂, -S(O)₂NH-R₀, - S(O)₂NH-cycloalkyl, -R₀-NH₂, -R₀₀-OH, -R₀₀-OR₀, -R₀₀-(morpholin-4-yl) phenyl, phenyl, oxadiazolyl or tetrazolyl optionally substituted by R₀, the oxadiazolyl is optionally substituted by R₀, R₀₀-OH or R₀-OR₀;
   R₃ is hydrogen, deuterium, R₀, haloalkyl, cycloalkyl, heterocycloalkyl, phenyl, pyridyl, pyrimidinyl, pyrazinyl, -C(O)N(R₀)₂, -R₀₀-cycloalkyl, -R₀₀-heterocycloalkyl, -R₀₀-phenyl, - R₀₀-OH, -R₀₀-OR₀, the cycloalkyl, heterocycloalkyl, phenyl and pyridyl are optionally substituted by R0, halogen,-C(O)-OR₀, -C(O)-R₀, -OH, -OR₀, -S(O)₂-R, -O-haloalkyl, -OR₀₀-(morpholin-4-yl), -R₀-OH, -R₀₀-OR, morpholin-4-yl or -R₀₀-(morpholin-4-yl);
   R₁₀ is hydrogen, deuterium, R₀, halogen, cyano, haloalkyl, cycloalkyl,-OR₀, optionally substituted amino, -O-haloalkyl, -R₀-OH, -R₀₀-OR₀ or -R₀₀ optionally substituted by amino;
   R₀ is alkyl;
   R₀₀ is alkyl or alkylene; or
   in some embodiments of the present invention, the PTM has the following structure: 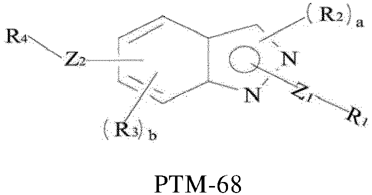
wherein, in PTM-68:
   Z₁ is absent, substituted or unsubstituted C1-C6 alkylene, substituted or unsubstituted C3-C6 cycloalkylene, substituted or unsubstituted C2-C6 alkenylene, or substituted or unsubstituted C2-C6 alkynylene;
   Z₂ is carbonyl, 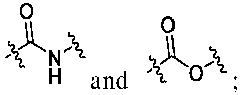
   R₁ is 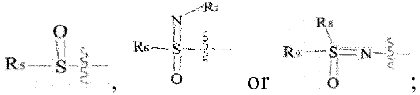
   each R₂ and each R₃ are independently absent, substituted or unsubstituted C1-C10 alkyl, substituted C3-C10 cycloalkyl, halogen, substituted or unsubstituted C1-C10 haloalkyl, substituted or unsubstituted C1-C12 hydroxyalkyl, substituted or unsubstituted C₁-C₁₂ sulfydrylalkyl, substituted or unsubstituted C3-C12 hydroxycycloalkyl, substituted or unsubstituted C3-C12 sulfydrylcycloalkyl, cyano, nitro, substituted or unsubstituted 3-12-membered heterocycloalkyl, -A-R₁₀, or -N(R₁₁)R₁₂;
   R₄ is substituted or unsubstituted C6-C20 aryl, or substituted or unsubstituted 5-20-membered heteroaryl;
   R₅ is substituted or unsubstituted C1-C10 alkyl, or substituted or unsubstituted C3-C10 cycloalkyl;
   R₆ is substituted or unsubstituted C1-C10 alkyl, or substituted or unsubstituted C3-C10 cycloalkyl;
   R₇ is hydrogen, deuterium, substituted or unsubstituted C1-C10 alkyl, substituted or unsubstituted C3-C10 cycloalkyl, or R₁₃-C(O)-;
   R₈ and R₉ are each independently substituted or unsubstituted C1-C10 alkyl, or R₈ and R₉ together with S atom to which they are attached form a 3-12 membered heterocycloalkyl;
   R₁₀ is hydrogen, deuterium, substituted or unsubstituted C1-C10 alkyl, substituted or unsubstituted C3-C8 cycloalkyl, substituted or unsubstituted 3-12-membered heterocycloalkyl, or -R₁₄-R₁₅;
   R₁₁ and R₁₂ are each independently hydrogen, deuterium, substituted or unsubstituted C1-C10 alkyl, or substituted or unsubstituted C3-C8 cycloalkyl;
   R₁₃ is substituted or unsubstituted C1-C10 alkyl, or substituted or unsubstituted C3-C10 cycloalkyl;
   R₁₄ is substituted or unsubstituted C1-C6 alkylene;
   R₁₅ is substituted or unsubstituted C3-C12 cycloalkyl, or substituted or unsubstituted C3-C12 heterocycloalkyl;
   wherein, any "substituted" refers to one or more (preferably 1, 2, 3 or 4) hydrogen atoms on the group is substituted by a substituent selected from the group consisting of: C2-C8 acyl, C3-C8 cycloalkyl, C1-C6 alkyl, C1-C6 alkoxy, C1-C6 alkylthio, hydroxyl, sulfydryl, amino, nitro, halogen, 3-12 membered heterocycloalkyl, cyano, C1-C10 haloalkyl, C3-C8 halocycloalkyl, C2-C4 ester, C2-C4 amide, C1-C4 carboxyl, C2-C6 alkenyl, C2-C6 alkynyl, C6-C12 aryl, 5-12 membered heteroaryl, -N(R₁₆)R₁₇;
   R₁₆ and R₁₇ are each independently hydrogen, deuterium, C1-C6 alkyl, C3-C8 cycloalkyl, or 3-10 membered heterocycloalkyl;
   the heterocycloalkyl, heteroaryl and heteroalkyl ring are each independently have 1-3 (preferably 1, 2 or 3) heteroatoms selected from N, O and S;
   A is S or O;
   a is 0 or 1,
   b is 0, 1, 2 or 3; or
   In some embodiments of the present invention, the PTM has the following structure: 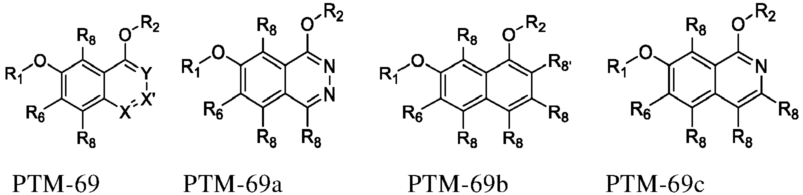 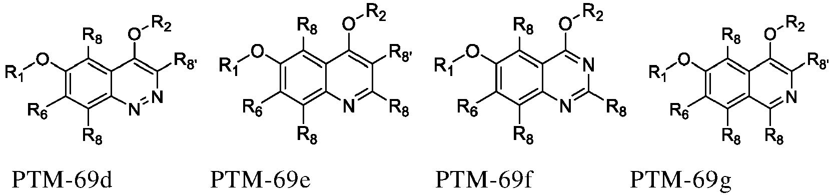 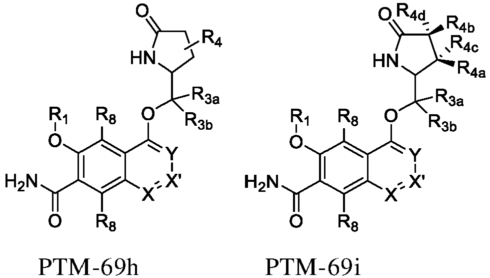
or a pharmaceutically acceptable salt thereof; wherein, in PTM-69, PTM-69a, PTM-69b, PTM-69c, PTM-69d, PTM-69e, PTM-69f, PTM-69g, PTM-69h, PTM-69i:
   X and X' are each independently CRs, N or -N⁺-O⁻; Y is independently N, -N⁺-O⁻ or CR₈ ; provided that at least one of X, X' or Y is neither N nor -N⁺-O⁻, and at most one of X, X' or Y is -N⁺-O⁻;
   R₁ is C1-C6 alkyl, C2-6 alkenyl, C2-6 alkynyl,-(CR₃ₐR_{3b})ₘ-(3-7 membered cycloalkyl),-(CR₃ₐR_{3b})ₘ- (3-7 membered heterocycloalkyl) containing 1-3 heteroatoms, -(CR₃ₐR_{3b})ₘ-(5-10 membered heteroaryl) containing 1-3 heteroatoms, or -(CR₃ₐR_{3b})ₘ-C6-C12 aryl, wherein, the alkyl, alkenyl, alkynyl, cycloalkyl, heterocycloalkyl, heteroaryl or aryl is optionally substituted by one to five substituents selected from halogen, deuterium, -OR₅,-SR₅,-NR₁₁ₐR_{11b}, cyano, C1-C6 alkyl, C3-C6 cycloalkyl or -C1-C6 alkoxy;
   R₂ is -(CR₃ₐR_{3b})ₘ-(3-10 membered cycloalkyl), -(CR₃ₐR_{3b})ₘ- (3-10 membered heterocycloalkyl) containing 1-3 heteroatoms, -(CR₃ₐR_{3b})ₘ- (5-10-membered heteroaryl) containing 1-3 heteroatoms, or -(CR₃ₐR_{3b})ₘ-C6-C12 aryl; wherein the cycloalkyl, heterocycloalkyl, heteroaryl, or aryl are optionally substituted by 1-5 R₄; wherein, if the heteroatom on the heterocycloalkyl and heteroaryl is N, the N is optionally substituted by R₄ ; or R₂ is C1-C6 alkyl, wherein the alkyl is optionally substituted by NH₂, OH or cyano;
   R₃ₐ and R_{3b} are independently hydrogen or C1-C3 alkyl at each occurrence;
   R₄ is independently bond, deuterium, halogen, cyano, C1-C6 alkyl, C2-6 alkenyl, oxo,-OR₅,-SR₅,-S(O)R₉,-S(O)₂R⁹,-NR₁₁ₐR_{11b},-C(O)R₁₀,-(CR₃ₐR_{3b})ₙ-(3-7-membered cycloalkyl),-(CR₃ₐR_{3b})ₙ-(4-10-membered heterocycloalkyl) containing 1-3 heteroatoms, -(CR₃ₐR_{3b})ₙ- (5-10-membered heteroaryl) containing 1-3 heteroatoms or -(CR₃ₐR_{3b})ₙ-C6-C12 aryl at each occurrence; wherein, the alkyl, cycloalkyl, heterocycloalkyl, heteroaryl or aryl are each optionally and independently substituted by 1 to 5 substituents selected from deuterium, halogen, OR₅,-SR₅,-NR₁₁ₐR_{11b}, cyano, C1-C6 alkyl, C3-C6 cycloalkyl or -C1-C6 alkoxy; or two R₄ together with the carbon atoms to which they are attached form a 3-6 membered cycloalkyl or a 4-6 membered heterocycloalkyl, wherein the cycloalkyl or heterocycloalkyl is optionally substituted by 1 to 3 substituents selected from halogen, deuterium, OR₅,-SR₅,-NR₁₁ₐR_{11b}, cyano, C1-C6 alkyl or C1-C6 alkoxy; wherein the alkyl or alkoxy is optionally substituted by a substituent selected from halogen, deuterium, OR₅,-SR₅,-NR₁₁ₐR_{11b}, or cyano; wherein, if the heteroatom on the heterocycloalkyl is N, the N is optionally substituted by R₄;
   R₄ is independently C1-C6 alkyl, C2-6 alkenyl,-C(O)R₁₀,-S(O)₂R₉,-(CR₃ₐR_{3b})ₙ- (3-7 membered cycloalkyl),-(CR₃ₐR_{3b})ₙ-(4-10 membered heterocycloalkyl) or C(O)(CH₂)ₜCN; wherein, the alkyl, alkenyl, cycloalkyl or heterocycloalkyl are each optionally and independently substituted by 1-5 substituents selected from deuterium, halogen, OH, cyano or C1-C6 alkoxy; or R₄ and R_{4'} together with the corresponding atoms to which they are attached form 3-6 membered cycloalkyl or 4-6 membered heterocycloalkyl, wherein, the cycloalkyl or heterocycloalkyl is optionally substituted by 1-3 substituents selected from halogen, deuterium, OR₅,-SR₅,-NR₁₁ₐR_{11b}, cyano, C1-C6 alkyl or C1-C6 alkoxy, wherein the alkyl or alkoxy is optionally substituted by halogen, deuterium, OR₅,-SR₅,-NR₁₁ₐR_{11b}, or cyano;
   R₄ₐ and R_{4b} are each independently hydrogen, deuterium, deuterium, F, OH,-ORs, methyl, ethyl, vinyl, cyclopropyl or propyl, optionally substituted by 1-5 deuterium, fluorine, methoxy or OH;
   R_{4c} and R_{4d} are independently halogen, OH, deuterium, C1-C6 alkyl, C2-6 alkenyl,-OR₅,-(CR₃ₐR_{3b})ₙ-(3-6 membered cycloalkyl),-(CR₃ₐR_{3b})ₙ-(4-6 membered heterocycloalkyl), NH₂ at each occurrence, wherein, the alkyl, cycloalkyl, and heterocycloalkyl are each optionally and independently substituted by 1-5 substituents selected from deuterium, halogen, OH, cyano or C1-C6 alkoxy; or R_{4c} and R_{4d} together with the carbon atoms to which they are attached form 4-7-membered heterocycloalkyl or 3-7-membered cycloalkyl, wherein, the heterocycloalkyl and cycloalkyl are independently optionally substituted by 1-3 fluorines, C1-C3 alkyls or C1-C3 fluoroalkyls; or
   R_{4c} and R₄ₐ together with the atoms to which they are attached form 4-7-membered heterocycloalkyl or 3-7-membered cycloalkyl, wherein the heterocycloalkyl and cycloalkyl are each independently optionally substituted by 1-3 fluorines, C1-C3 alkyls or C1-C3 fluoroalkyls;
   R₅ is independently hydrogen or C1-C6 alkyl, wherein the alkyl is optionally substituted by halogen, deuterium, C1-C6 alkoxy, C1-C6 alkylthio, -NR₁₁ₐR_{11b}, cyano, C1-C6 alkyl or C3-C6 cycloalkyl; or two R₅ together with the oxygen atoms to which they are attached form 5 or 6 membered heterocycloalkyl;
   R₆ is hydrogen, deuterium, -C(O)NHR₇, CO₂R₇ or cyano;
   R₇ is hydrogen or C1-C6 alkyl;
   R₈ is each independently hydrogen, deuterium, halogen, cyano, OR₅, -SR₅, -NR₁₁ₐR_{11b}, C1-C6 alkyl, C2-6 alkenyl, C2-6 alkynyl, C3-C6 cycloalkyl, 3-10 membered heterocycloalkyl, or 5-6 membered heteroaryl or aryl, wherein, the alkyl, alkenyl, alkynyl, cycloalkyl, heterocycloalkyl, heteroaryl or aryl are optionally substituted by 1 to 3 halogens, C3-C6 cycloalkyls, C3-C6 heterocycloalkyls containing 1 or 2 N heteroatoms, OR₅, -SR₅, - NR₁₁ₐR_{11b}, cyano, C1-C3 alkyl,-C(O)R₁₀ or oxo;
   R₈ is hydrogen, deuterium, deuterium, halogen, cyano, OR₅,-SR₅, or -NR₁₁ₐR_{11b};
   R₉ is -(CR₃ₐR_{3b})ₚ-(C1-C3 alkyl),-(CR₃ₐR_{3b})ₚ-(4 -6 membered cycloalkyl),-(CR₃ₐR_{3b})ₚ-(4-6 membered heterocycloalkyl) or -(CR₃ₐR_{3b})ₚ-(C5-C9 aryl), wherein, the alkyl, cycloalkyl, heterocycloalkyl or aryl are each optionally substituted by fluorine or C1-C3 alkyl;
   R₁₀ is C1-C6 alkyl, wherein the alkyl is optionally substituted by deuterium, halogen, OH, C1-C6 alkoxy or cyano;
   R₁₁ₐ and R_{11b} are each independently hydrogen or C1-C6 alkyl, wherein the alkyl is optionally substituted by deuterium, C1-C6 alkoxy or cyano; if it is C2-6 alkyl, the alkyl is optionally substituted by deuterium, C1-C6 alkoxy, cyano, halogen or OH;
   m is independently 0, 1, 2 or 3;
   n is independently 0, 1, 2 or 3;
   p is independently 0 or 1; and
   t is 1, 2 or 3; or
   in some embodiments of the present invention, the PTM has the following structure: 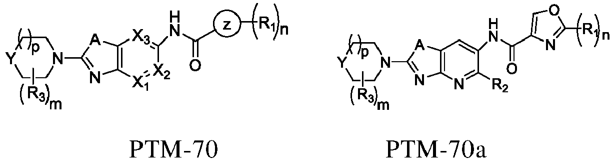 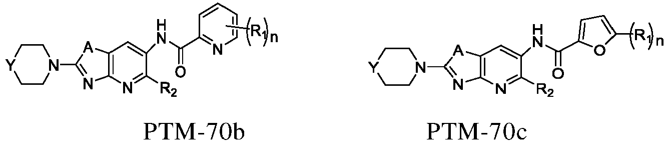
or a pharmaceutically acceptable salt thereof; wherein, in PTM-70, PTM-70a, PTM-70b or PTM-70c:
   X₁ and X₃ are each independently CH or N; X₂ is CR₂ or N; provided that one of X₁, X₂ or X₃and no more than one is N;
   A is O, N or S;
   Y is N, CH₂ or O;
   ring Z is aryl, heteroaryl or heterocycloalkyl;
   R₁ is independently halogen, optionally substituted heterocycloalkyl or optionally substituted heteroaryl at each occurrence; the substituent is alkyl, alkoxy, aminoalkyl, halogen, hydroxyl, hydroxyalkyl or -NRₐR_{b};
   R₂ is hydrogen, deuterium, optionally substituted cycloalkyl, optionally substituted aryl, optionally substituted heterocycloalkyl, optionally substituted heteroaryl or -NRₐR_{b}; wherein the substituent is alkyl, amino, halogen or hydroxyl;
   R₃ is alkyl or hydroxyl at each occurrence;
   Rₐ and R_{b} are independently hydrogen, deuterium, alkyl, acyl, heteroaryl or heterocycloalkyl;
   m and n are each independently 0, 1 or 2;
   p is 0 or 1; or
   in some embodiments of the present invention, the PTM has the following structure: 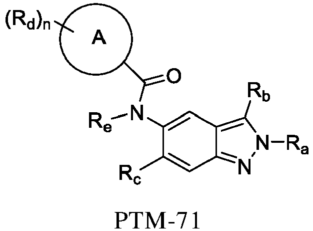
wherein, in PTM-71:
   ring A is 6-10 membered aryl or 5-10 membered heteroaryl;
   R_{d} is each independently hydrogen, deuterium, halogen, cyano, C 1-C6 alkyl, C3-C6 cycloalkyl, or 5-10-membered heteroaryl; and the alkyl, cycloalkyl, and heteroaryl are optionally substituted by one or more groups selected from halogen, hydroxyl, or amino;
   n is 1, 2, 3 or 4;
   Rₑ is hydrogen or C1-C6 alkyl;
   R_{c} is hydrogen, deuterium,-O-(C1-C6 alkyl),-O-(C3-C8 cycloalkyl), -O-(3-8-membered heterocycloalkyl),-O-(6-10-membered aryl),-O-(5-10-membered heteroaryl),-N(C1-C6 alkyl) ₂,-NH(C3-C8 cycloalkyl),-NH(C3-C8 heterocycloalkyl), -NH(6-10 membered aryl),-NH(5-10 membered heteroaryl), C1-C6 alkyl, C2-C6 alkenyl, C2-C6 alkynyl, C3-C8 cycloalkyl, C3-C8 heterocycloalkyl, C6-C10 aryl, or 5-10 membered heteroaryl, the alkyl, cycloalkyl, heterocycloalkyl, aryl and heteroaryl are optionally substituted by one or more groups independently selected from hydroxyl, amino, halogen,-O-(C1-C6 alkyl) and cyano;
   R_{b} is hydrogen, deuterium,-O-(C1-C6 alkyl),-O-(C3-C8 cycloalkyl),-O-(C3-C8 heterocycloalkyl),-O-(6-10 membered aryl),-O-(5-10 membered heteroaryl),-N(C1-C6 alkyl)₁₋₂,-NH (C3-C8 cycloalkyl),-NH (C3-C8 heterocycloalkyl)),-NH(6-10 membered aryl),-NH(5-10 membered heteroaryl), C1-C6 alkyl, C2-C6 alkenyl, C2-C6 alkynyl, C3-C8 cycloalkyl, C3-C8 heterocycloalkyl, C6-C10 aryl, or 5-10 membered heteroaryl, the alkyl, cycloalkyl, heterocycloalkyl, aryl, and heteroaryl are optionally substituted by one or more groups independently selected from hydroxyl, amino, halogen, and cyano;
   Rₐ is hydrogen, deuterium, 3-8-membered heterocycloalkyl, C3-C8 cycloalkyl, 6-10 membered aryl, 5-10 membered heteroaryl, C1-C6 alkyl, 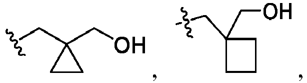 or 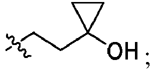 the alkyl is optionally substituted by one or more substituents selected from halogen, hydroxyl, C1-C6 alkoxy, halogenated C1-C6 alkoxy, S(O)1-2(C1-C6 alkyl), S(O)1-2(C3-C6 cycloalkyl), unsubstituted or mono- or polyhalogen-substituted C3-C6 cycloalkyl, unsubstituted or mono- or di-methyl substituted monocyclic saturated heterocycloalkyl having 4-6 ring atoms and containing heteroatoms or heterogroups selected from O, S, SO or SO2; and the cycloalkyl, heterocycloalkyl, aryl, and heteroaryl are optionally substituted by one or more groups selected from hydroxyl, halogen, amino, cyano, C1-C6 alkyl, -O(C1-C6 alkyl), halogenated C1-C6 alkyl, hydroxyl C1-C6 alkyl, and amino C1-C6 alkyl; wherein * represents the bonding site where the group is attached to the rest of the molecule; or 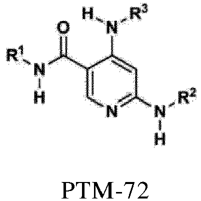
or a stereoisomer or a pharmaceutically acceptable salt thereof; wherein, in PTM-72:
   R¹ is: (a) C2-C3 hydroxylalkyl substituted by 0-4 R^{1a}, wherein R^{1a} is selected from F, Cl,-OH,-CHF₂,-CN,-CF₃,-OCH₃ and cyclopropyl;
   (b) C1-C3 alkyl substituted by -O(C1-C3) alkyl and 0-4 R^{1a}, wherein R^{1a} is selected from F, Cl,-OH,-CHF₂,-CN,-CF₃ and cyclopropyl;
   (c) C4-8 alkyl substituted by 0-7 R^{1a}, wherein R^{1a} is selected from F, Cl,-OH,-CHF₂,-CN,-CF₃,-OCH₃, cyclopropyl or -OP(O)(OH)₂;
   (d) -(CH₂)₂₋₄NHC(O)(C1-C6 alkyl), -(CH₂)₂CH(CH₃)NHC(O)(C1-C6 alkyl), - (CH₂)₂CH(CH₃)NHC(O) O(C1-C6 alkyl), -(CH₂)₂CH(CH₃)NHC(O)(CH₂)₀₋₁NH(C1-C6 alkyl) or -(CH₂)₂CH(CH₃)NHC(O)(CH₂)₀₋₁N(C1-C6 alkyl)₂, -(CH₂)₂CH(CH₃)NHC(O)(CH₂)₀₋₁(C3-C6 cycloalkyl), -(CH₂)₂CH(CH₃)NHC(O)(CH₂)₀₋₁(C3-C6 fluorocycloalkyl), - (CH₂)₂CH(CH₃)NHC(O)(C1-C6 hydroxyalkyl), -(CH₂)₂CH(CH₃)NHC(O)N(C1-C6 alkyl) (phenyl) or -(CH₂)₂CH(CH₃)NHC(O)(CH₂)₀₋₁R, wherein R is phenyl, morpholinyl, pyrrolidinyl, triazolyl, or tetrahydropyran;
   (e) cyclohexyl, cyclopentyl, cyclopropyl or cyclobutyl, which are substituted by 0-2 substituents independently selected from the following: -OH, -OCH₃, oxo, -NH₂, nitro, C1-C6 alkyl, C1-C6 hydroxyalkyl, -C(O)NH₂, -C(O)NH(C1-C3 alkyl), -C(O)NH(C1-C6 hydroxyalkyl), -C(O)NH(C3-C6 cycloalkyl), -C(O)NH(C3-C6 fluorocycloalkyl), - C(O)NH(C3-C6 cyanocycloalkyl), -NHC(O)(C1-C3 alkyl), -NHC(O)(pyridyl), - NHC(O)(morpholinyl), -NHC(O)(hydroxyl bicyclo [2.2.2] heptyl), -NHC(O)NH(C1-C4 alkyl), -NHC(O)O(C1-C3 alkyl), -NHS(O)₂CH₃, -NHS(O)₂NH₂, -NHS(O)₂(C1-C3 alkyl), - S(C1-C3 alkyl), thiazolyl, methylpyrazolyl, or C1-C3 alkyl substituted by -OH or cyclopropyl;
   (f) -(CH₂)₂phenyl, wherein the phenyl is substituted by -C(O)NH₂,-C(O)NH(C1-C3 alkyl) or -S(O)₂NH₂;
   (g) piperidinyl, azetidinyl, azacyclopentane, piperazinyl, optionally, which are optionally substituted by -C(O)(C1-C3 alkyl); or 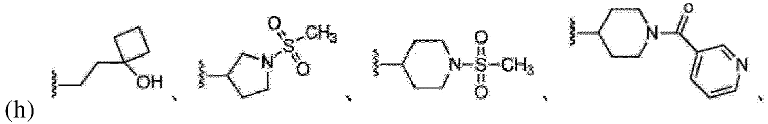 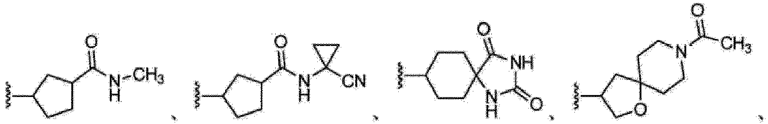 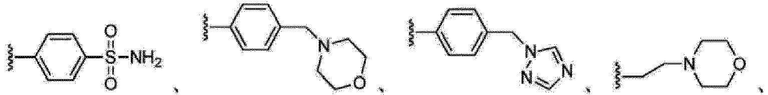 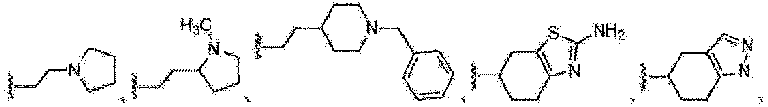 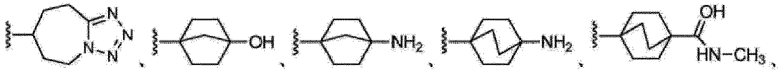 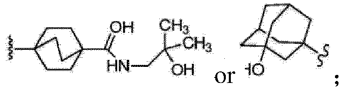
   R² is phenyl, pyridyl, pyridazinyl, pyrimidinyl, pyrazolyl, thiazolyl, triazolyl, benzoxazolyl, pyrazolopyridyl, pyrrolopyridyl, benzothiazolyl, thiazolopyridyl, pyrazolopyrimidinyl, benzoxadiazolyl, benzothiadiazolyl, quinolyl, cinnolinyl, quinazolinyl, quinoxalyl, 1, 5-diazanaphthyl, 1, 6-diazanaphthyl, pyridopyrazinyl or pyridopyrimidinyl, and each is independently substituted by 0-2 substituents independently selected from the following: F, Cl,-OH,-CN, C1-C3 alkyl, -CH₂C(O)OCH₃,-O(C1-C3 alkyl),-NH₂,-NH(C1-C3 alkyl),-NH (cyclopropyl), -C(O)NH₂,-NHC(O)(C1-C3 alkyl),-NH (tetrahydropyranyl), hydroxypyrrolidyl, oxo,-O(piperidinyl) or pyridyl;
   R3 is:(a) C1-C6 alkyl substituted by 0-4 substituents independently selected from the following: F,-CH₃,-CF₃, phenyl, tetrahydrofuranyl, morpholinyl or C3-C6 cycloalkyl;
   (b) C1-C6 hydroxyalkyl substituted by 0-3 groups selected from F, phenyl, fluorophenyl, difluorophenyl or dichlorophenyl;
   (c) -(CH₂)₀₋₂(C3-C7 cycloalkyl) substituted by 0-2 substituents independently selected from the following: F, Cl,-OH, C1-C3 hydroxyalkyl, C1-C3 alkyl,-O(C1-C3 alkyl),-OCF₃,-C(O)NH₂,-C(O)NH(C1-C3 alkyl),-C(O)NH(C1-C3 fluoroalkyl),-C(O)NH(C1-C3 hydroxyalkyl),-C(O)NH(C3-C5 cycloalkyl),-C(O)N(C1-C3 alkyl)₂,-S(O)₂CH₃,-CF₂H,-NH₂,-C(O)CH₂CH₃ or pyrazolyl;
   (d) oxetanyl, tetrahydropyranyl or fluorotetrahydropyranyl;
   (e) thiazolyl substituted by 0-2 groups selected from C1-C3 hydroxyalkyl;
   (f) phenyl substituted by 0-2 substituents independently selected from the following: - OH,-CN,-O(C1-C3 alkyl), C1-C3 hydroxyalkyl, -C(O)NH₂, -S(O)₂NH₂, -NHS(O)₂(C1-C3 alkyl), pyrazolyl, imidazolyl, or methyltetrazolyl; or 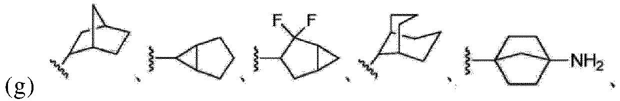 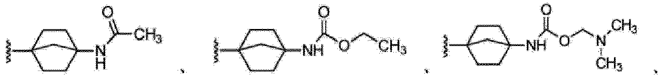 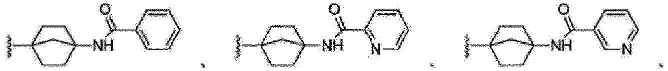 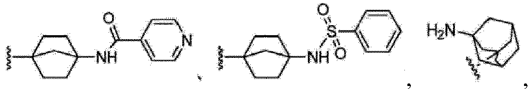 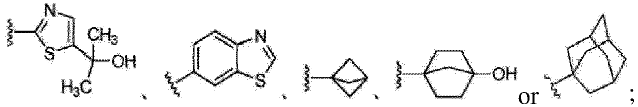 or
   in some embodiments of the present invention, the PTM has the following structure: 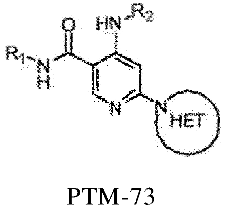
or a pharmaceutically acceptable salt thereof; wherein, in PTM-73:
   HET is a heteroaryl selected from the following: pyrazolyl, indolyl, pyrrolo [2,3 -b] pyridyl, pyrrolo [2,3-b] pyrimidyl, pyrazolo [3,4-b] pyridyl, pyrazolo [3,4-b] pyrimidyl, 2, 3-dihydro-1H-pyrrolo [2,3-b] pyridyl, imidazolo [4,5-b] pyridyl or purinyl, wherein the heteroaryl is optionally substituted by Rₐ or R_{b};
   Rₐ is hydrogen, deuterium, F, Cl, Br,-CN,-OH, C1-C4 alkyl, C1-C4 hydroxyalkyl, C1-C4 fluoroalkyl, C1-C4 alkoxy,-NH₂,-NH(C1-C4 alkyl),-N(C1-C4 alkyl)₂,-NH(C1-C4 hydroxyalkyl),-NH(C1-C4 fluoroalkyl),-NH(C1-C6 hydroxyl-fluoroalkyl),-C(O)NH₂,-CH₂NHC(O)(C1-C6 alkyl),-CH₂NHC(O)(C1-C6 hydroxyalkyl),-CH₂NHC(O)NH(C1-C6 alkyl),-CH₂NHC(O) NHCH₂(phenyl), -CH₂NHC(O)N(C1-C4 alkyl)₂,-CH₂NHC(O)O(C1-C4 alkyl),-CH₂NHC(O)(C3-C6 cycloalkyl), -CH₂NHC(O)(tetrahydrofuranyl),-CH2NHC(O)CH₂(C3-C6 cycloalkyl),-CH₂NHC(O)CH₂(tetrahydropyranyl),-CH₂NHC(O)CH₂(phenyl),-NHC(O)(C1-C4 alkyl), pyrrolidinyl, hydroxypyrrolyl or pyridazinyl;
   R_{b} is hydrogen or -NH₂;
   R₁ is: (i) C1-C6 alkyl, C1-C6 fluoroalkyl, C1-C6 hydroxyl alkyl, C1-C8 hydroxylfluoroalkyl,-(Cl-C6 alkylene) O(C1-C4 alkyl),-(C1-C6 alkylene) O(C1-C4 fluoroalkyl),-(C1-C6 fluoroalkylene) O(C1-C4 alkyl),-(C1-C6 fluoroalkylene) O(C1-C4 deuterated alkyl),-(C1-C6 fluoroalkylene)) O(C1-C4 fluoroalkyl),-(Cl-C6 fluoroalkylene) C(C3-C6 cycloalkyl) 2(OH),-(C1-C4 alkylene) NHC(O)(C1-C4 alkylene) OC(O)(C1-C3 alkyl),-(C1-C6 alkylene) NHS(O)₂(C1-C4 alkyl),-(C1-C6 alkylene) P(O)(C1-C4 alkoxy)₂,-(C1-C6 fluoroalkylene) NH(C1-C4 alkyl),-(C1-C6 alkylene) C(O)NH(C1-C4 alkyl),-(C1-C6 fluoroalkylene) C(O)NH(C1-C4 alkyl),-(Cl-C6 fluoroalkylene) C(O)NH(C1-C4 hydroxyalkyl) or -(C1-C6 fluoroalkylene) OP(O)(OH)₂;
   (ii) -(C1-C3 alkylene) Rₓ,-(C1-C3 fluoroalkylene) Rₓ,-(C1-C3 alkylene) C(O)Rₓ, -(C1-C3 alkylene) C(O)NHRₓ,-(C1-C3 fluoroalkylene) C(O)NHRₓ, or -CH₂CF = (tetrahydropyranyl), wherein, Rₓ is selected from the following cyclyls: C3-C6 cycloalkyl, tetrazolyl, 1,1-dioxidotetrahydrothiophenyl, 1,1- dioxidothiomorpholino, oxadiazolyl, piperidyl, piperazinyl, pyrrolidyl, oxetanyl, tetrahydrofuranyl, tetrahydropyranyl, pyridyl, imidazolyl, morpholinyl, phenyl and triazinyl, wherein each cyclyl is optionally substituted by 0-3 substituents independently selected from the following: F,-OH,-CH₃,-(CH₂)₂OH,-OCH₃,-C(O)CH₂CN,-S(O)₂CH₃,-S(O)₂NH₂,-NHC(O)CH₃,-N(S(O)₂CH₃)₂,-CH₂CH₂(acetaminophenyl),-CH₂CH₂(methoxyphenyl),-CH₂CH₂(aminosulfonyl phenyl), oxetanyl, phenyl or morpholinyl;
   (iii) C3-C6 cycloalkyl or C4-C6 cycloalkenyl, each is substituted by 0-3 substituents independently selected from the following: F,-OH,-CN, C1-C3 alkyl, C1-C3 alkoxy, S(C1-C3 alkyl), nitro, S(O)₂(C1-C3 alkyl), C1-C4 hydroxyalkyl,-C(C1-C3 alkyl)(OH)(C3-C6 cycloalkyl),-CH₂C(O)NH(C1-C3alkyl),-NHC(O)(C1-C3alkyl),-NHC(O)(C1-C4 hydroxyalkyl),-C(O)NH(C1-C3 alkyl),-C(O)NH(C1-C3 deuterated alkyl),-C(O)NH(C3-C6 cycloalkyl), -NHC(O)O(C1-C3 alkyl), -NHS(O)₂(C1-C3 alkyl), pyridyl, imidazolyl, pyrazolyl, methylimidazolyl, methylpyrazolyl or thiazolyl;
   (iv) tetrahydrofuranyl, piperidyl, pyrazolyl, phenyl, pyridyl or pyrimidyl, each is substituted by 0-1 substituents selected from the following: -OH, C1-C3 alkyl, C1-C3 fluoroalkyl, C1-C4 hydroxyalkyl, C1-C3 alkoxy,-C(O)(C1-C4 alkyl),-S(O)₂(C1-C4 alkyl),-S(O)₂NH(C1-C4 alkyl),-NH(C1-C3 alkyl),-O(C1-C3 alkylene) N(C1-C3 alkyl)₂,-CH₂(morpholinyl), azetidinyl, tetrahydropyranyl, morpholinyl, oxetanyl, piperazinyl, piperidinyl, methylpiperazinyl, methoxypiperidinyl, pyridyl, pyrimidyl, methylsulfonyl azetidine, or -C(O)(methylsulfonyl azetidine); or
   (v) pyrrolo [2,3-c] pyridyl, bicyclic [2.2.1] hept-1-ol, tetrahydrobenzo [d] thiazol-2-amine or 1, 3-diazaspiro [4.5] dec-2, 4-dione; and
   R₂ is: (i) C1-C7 alkyl or C2-6 alkenyl, each of which is independently substituted by 0-3 substituents independently selected from F,-OH or -CN; -(C1-C4 alkylene) O(C1-C4 alkyl), - (C1-C4 alkylene) O(C1-C4 fluoroalkyl), -(C1-C6 alkylene)NH₂, -(C1-C6 alkylene) S(O)₂(C1-C3 alkyl),-(C1-C6 fluoroalkylene) NH(C1-C3 alkyl), or -(C1-C6 alkylene) NHC(O)(C1-C4 fluoroalkyl);
   (ii) -(C1-C4 alkylene) R_{y}, wherein R_{y} is C3-C6 alkyl, azetidine, oxetane, oxazolyl, pyridyl, tetrahydropyranyl, each is independently substituted by 0-3 substituents selected from the following: F,-OH, C1-C3 alkyl, C1-C3 hydroxyalkyl,-C(O) (C1-C3 alkyl),-C(O) (C1-C3 fluoroalkyl),-C(O) (C1-C3 cyanoalkyl),-C(O)O(C1-C3 alkyl),-C(O)NH₂,-C(O)NH(C1-C3 alkyl),-C(O) (difluorophenyl),-NH₂,-NH(C1-C3 alkyl),-NH(C1-C3 fluoroalkyl),-NH (oxetane),-NHC(O) (C1-C3 alkyl),-NHC(O) (C1-C3 fluoroalkyl),-NHC(O) (C3-C6 cycloalkyl),-NHC(O) (fluorophenyl),-S(O))₂(C1-C3 alkyl), imidazolyl, phenyl, pyrimidyl, fluoropyrimidyl, chloropyrimidyl, or methoxypyrimidyl;
   (iv) adamantyl, hydroxyadamantyl, benzo [d] imidazolyl, benzo [d] oxazolyl, benzo [d] triazolyl, benzo [d] thiazolyl, bicyclo [1.1.1] pentyl or hydroxy-bico [2.2.1] heptyl; or
   (v) phenyl, pyrazolyl, thiazolyl, thiadiazolyl or indazolyl, each is independently substituted by 0-2 substituents selected from the following: F,-C1,-OH,-CN, C1-C4 alkyl, C1-C4 hydroxyalkyl, C1-C4 fluoroalkyl, C1-cyanoalkyl, C1-C3 alkoxy, C3-C6 cycloalkyl,-(C1-C3 alkylene) O (C1-C3 alkyl),-(C1-C3 alkylene) O (C1-C3 fluoroalkyl),-C(O)NH₂,-C(O)NH(C1-C3 alkyl),-NHC(O)(C1-C3 alkyl),-NHC(O) S(O)₂(C1-C3 alkyl),-S(O)₂NH₂,-S(O)₂(C1-C3 alkyl), pyrazolyl, methylpyrazolyl, imidazolyl, triazolyl, methyltetrazolyl, ethyltetrazolyl, phenyl, pyrimidyl, fluoropyrimidyl, or tetrahydropyranyl; or
   in some embodiments of the present invention, the PTM has the following structure: 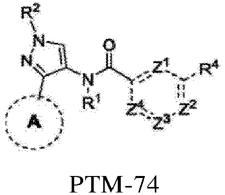
or a pharmaceutically acceptable salt thereof; wherein, PTM-74 is:
   ring A is aryl or heterocycloalkyl;
   R¹ is H, alkyl, or heteroalkyl;
   R² is H, alkyl, heteroalkyl, or heterocycloalkyl;
   each Z¹, Z², Z³, and Z⁴ are independently N or CR³, wherein at least one of Z¹, Z², Z³, and Z⁴ is N;
   R⁴ is halogen, heterocycloalkyl, aryl, -O-aryl, or -NH-aryl; or
   in some embodiments of the present invention, the PTM has the following structure: 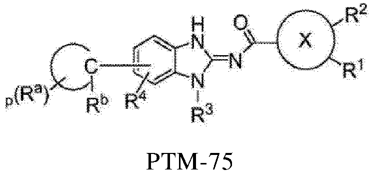
   or a pharmaceutically acceptable salt thereof, wherein, in PTM-75: 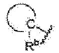 is C₃₋₁₀ aryl, 3-8-membered saturated or partially unsaturated carbocycle, 3-7-membered heterocyclyl containing 1-4 heteroatoms independently selected from nitrogen, oxygen or sulfur, or 5-6-membered monocyclic heteroaromatic ring containing 1-4 heteroatoms independently selected from nitrogen, oxygen or sulfur;
   each R^{a} is independently -R, halogen, -haloalkyl, -OR, -SR, -CN, -NO₂, -SO₂R, -SOR, - C(O)R, -CO₂R, -C(O)N(R)₂, -NRC(O)R, -NRC(O)N(R)₂, -NRSO₂R or -N(R)₂;
   R^{b} is -R, halogen, -haloalkyl, -OR, -SR, -CN, -NO₂, -SO₂R, -SOR, -C(O)R, -CO₂R, - C(O)N(R)₂, -NRC(O)R, -NRC(O)N(R)₂, -NRSO₂R or -N(R)₂; or R^{b} is absent;
   ring X is C3-10 aryl; 5-6-membered monocyclic heteroaromatic ring containing 1-4 heteroatoms independently selected from nitrogen, oxygen or sulfur; fused C3-10 aryl; fused 5-10-membered saturated or partially unsaturated carbocycle; fused 5-10-membered heterocyclyl containing 1-4 heteroatoms independently selected from nitrogen, oxygen or sulfur; or fused 5-10-membered monocyclic heteroaromatic ring containing 1-4 heteroatoms independently selected from nitrogen, oxygen or sulfur; each of the aboves is optionally substituted;
   R¹ is -R, halogen, -haloalkyl, -OR, -SR, -CN, -NO₂, -SO₂R, -SOR, -C(O)R, -CO₂R, - C(O)N(R)₂, -NRC(O)R, -NRC(O)N(R)₂, -NRSO₂R, or -N(R)₂;
   R² is -R, halogen, -haloalkyl, -OR, -SR, -CN, -NO₂, -SO₂R, -SOR, -C(O)R, -CO₂R, - C(O)N(R)₂, -NRC(O)R, -NRC(O)N(R)₂, -NRSO₂R, or -N(R)₂; R³ is -R or -haloalkyl;
   R⁴ is -R, halogen, -haloalkyl, -OR, -SR, -CN, -NO₂, -SO₂R, -SOR, -C(O)R, -CO₂R, - C(O)N(R)₂, -NRC(O)R, -NRC(O)N(R)₂, -NRSO₂R, or -N(R)₂;
   each R is independently hydrogen, deuterium, C₁₋₆ aliphatic group, C3-C10 aryl, 3-8-membered saturated or partially unsaturated carbocycle, 3-7-membered heterocyclyl containing 1-4 heteroatoms independently selected from nitrogen, oxygen or sulfur, or 5-6-membered monocyclic hetero-aromatic ring containing 1-4 heteroatoms independently selected from nitrogen, oxygen or sulfur; each of the aboves is optionally substituted; or
   two Rs on the same atom together with the atom to which they are attached form C3-C10 aryl, 3-8-membered saturated or partially unsaturated carbocycle, 3-7-membered heterocyclyl containing 1-4 heteroatoms independently selected from nitrogen, oxygen or sulfur, or 5-6-membered monocyclic heteroaromatic ring containing 1-4 heteroatoms independently selected from nitrogen, oxygen or sulfur; each of the above is optionally substituted; and
   p is 0, 1, 2, 3, 4 or 5; or
   in some embodiments of the present invention, the PTM has the following structure: 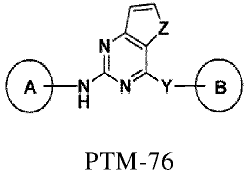
or a stereoisomer, a tautomer, a solvate or a pharmaceutically acceptable salt thereof, wherein, in PTM-76:
   Y is selected from O, S, SO, SO₂ or NR¹;
   Z is selected from S, O or NR²;
   A is substituted or unsubstituted aryl or heteroaryl;
   B is cycloalkyl, and the cycloalkyl is optionally substituted by one or more R³ groups;
   R¹ is selected from hydrogen or alkyl;
   R² is selected from hydrogen, deuterium, alkyl, hydroxyalkyl, cyanoalkyl, alkoxyalkyl, alkoxycarbonyl alkyl, aminoacyl alkyl, alkylaminoacyl alkyl, dialkylaminoacyl alkyl, (heterocycloalkyl) alkyl-, (aryl) alkyl- or (heteroaryl) alkyl-;
   each R³ is independently selected from alkyl, heterocycloalkyl, halogen, hydroxyl, R^{3a}R^{3b}N-, carboxyl, haloalkyl, hydroxyalkyl, alkoxy, heterocycloalkoxyl, (heterocycloalkyl) alkyl-oxy, hydroxyalkoxyl or R^{3a}R^{3b}NC(O)-, wherein the heterocycloalkyl is optionally substituted by one or more substituents selected from halogen, alkyl, haloalkyl, hydroxyl, alkoxy, amino, alkyl amino and dialkylamino at each occurrence;
   each R^{3a} and R^{3b} are independently selected from hydrogen, deuterium, alkyl, cycloalkyl, hydroxyalkyl, heterocycloalkyl, aryl or heteroaryl, wherein the cycloalkyl, heterocycloalkyl, aryl or heteroaryl is optionally substituted by one or more substituents selected from halogen, alkyl, haloalkyl, hydroxyl, alkoxy, amino, alkylamino and dialkylamino; or
   in some embodiments of the present invention, the PTM has the following structure: 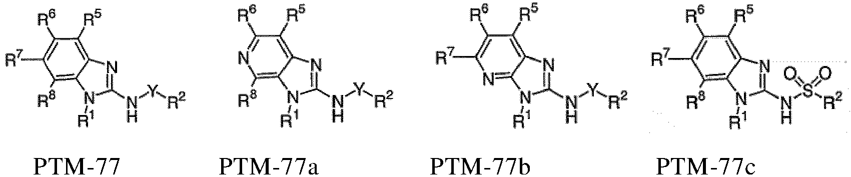 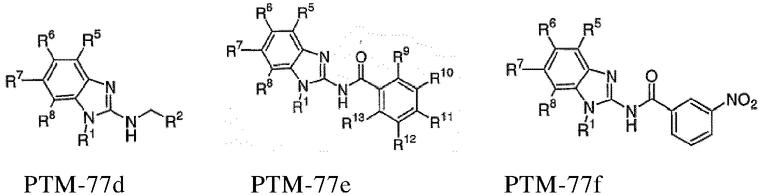 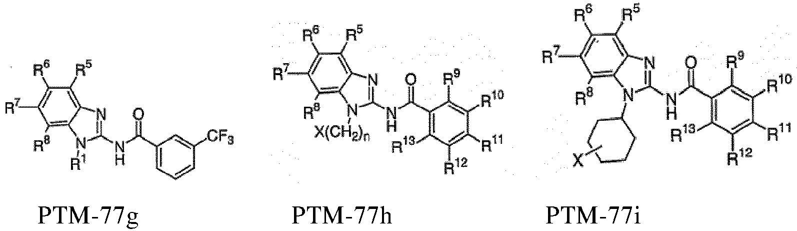
or stereoisomers, tautomers, solvates or pharmaceutically acceptable salts thereof, wherein, in PTM-77, PTM-77a, PTM-77b, PTM-77c, PTM-77d, PTM-77e, PTM-77f, PTM-77g, PTM-77h or PTM-77i:
   R¹ is selected from H, (C1-C8) alkyl, C1-C4 haloalkyl, C1-C4 hydroxyalkyl, C1-C4 aminoalkyl,-C1-C4 alkyl-O-C1-C4 alkyl,-O-C1-C4 alkyl, C3-C8 cycloalkyl, C3-C8 cycloalkyl (C1-C8) alkyl, heterocycloalkyl (C1-C8) alkyl, 6-10 membered aryl, aryl(C1-C8) alkyl, heteroaryl (C1-C8) alkyl or heteroaryl, the alkyl, cycloalkyl, aryl, and heteroaryl are optionally substituted;
   R² is selected from (C1-C8) alkyl, C1-C4 haloalkyl, C1-C4 hydroxyalkyl, C1-C4 aminoalkyl, -C1-C4 alkyl-O-C1-C4 alkyl, -O-C1-C4 alkyl, 6-10-membered aryl, or heteroaryl, the alkyl, cycloalkyl, aryl, heteroaryl are optionally substituted;
   R⁵, R⁶, R⁷ and R⁸ are each independently selected from H, halogen, C1-C4 alkyl, C1-C4 haloalkyl, C1-C4 hydroxyalkyl, C1-C4 aminoalkyl,-C1-C4 alkyl-O-C1-C4 alkyl,-O-C1-C4 alkyl, aryl, aryl (C1-C4) alkyl, heteroaryl, CN, CO₂R', CONR'R", NR'R", NO₂, OR', SR', C(O)R ', OC(O)R', N(R")C(O)R', N(R")CO₂R', N(R")C(O)NR'R", S(O)ₘNR'R", S(O)ₘR and N(R")S(O)ₘR', the alkyl, cycloalkyl, aryl, and heteroaryl are optionally substituted, wherein the subscript m is an integer of 1 or 2;
   R⁹, R¹⁰, R¹¹, R¹², and R¹³ are each independently selected from H, halogen, C1-C4 alkyl, C1-C4 haloalkyl, C1-C4 hydroxyalkyl, C1-C4 aminoalkyl,-C1-C4 alkyl-O-C1-C4 alkyl,-O-C1-C4 alkyl, aryl, aryl (C1-C4) alkyl, heteroaryl, CN, CO₂R', CONR'R", NR'R", NO₂, OR', SR', C(O)R', OC(O)R', N(R")C(O)R', N(R")CO₂R', N(R")C(O)NR'R", S(O)ₘNR'R", S(O)ₘR and N(R")S(O)ₘR ', the alkyl, cycloalkyl, aryl, and heteroaryl are optionally substituted; wherein the subscript m is an integer of 1 or 2;
   X is selected from H, OR', NR'R", OC(O)R', CO₂R', CONR'R", OCONR'R", N(R")C(O)R', C(O)R', N(R")CO₂R', or N(R")C(O)NR'R ";
   Y is C(O), S(O), S(O)₂, or -CH2-;
   R' and R" are each independently selected from H, C1-C4 alkyl, C1-C4 haloalkyl, C1-C4 hydroxyalkyl, C1-C4 aminoalkyl,-C1-C4 alkyl-O-C1-C4 alkyl,-O-C1-C4 alkyl, aryl, aryl (C1-C4) alkyl, the alkyl, cycloalkyl, aryl, and heteroaryl are optionally substituted; or
   R' and R" together with the N atoms connected to them form a 5-, 6-, or 7-membered ring.

Preferably, in some embodiments of the present invention, the PTM has the following structure: 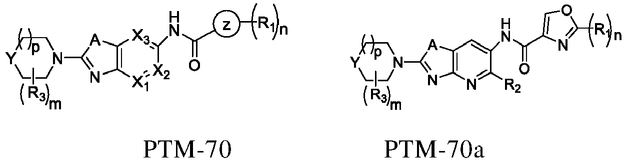 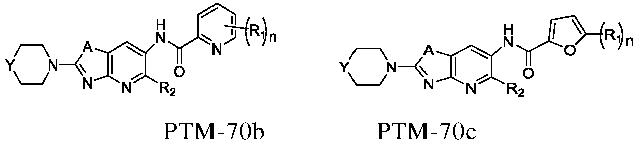 or a pharmaceutically acceptable salt thereof; wherein, in PTM-70, PTM-70a, PTM-70b or PTM-70c:
X₁ and X₃ are each independently CH or N; X₂ is CR₂ or N; provided that one of X₁, X₂ or X₃and no more than one is N;
A is O, or S;
Y is N, CH₂ or O;
ring Z is 6-10 membered aryl, 5-6 membered heteroaryl or 3-8 membered heterocycloalkyl;
R₁ is independently halogen, optionally substituted pyridyl, optionally substituted pyrazolyl, optionally substituted pyrrolidyl or optionally substituted piperidinyl at each occurrence; the substituent is alkyl, alkoxy, aminoalkyl, halogen, hydroxyl, hydroxyalkyl or - NRₐR_{b};
R₂ is hydrogen, deuterium, optionally substituted piperidinyl, optionally substituted pyrrolidyl, optionally substituted morpholinyl, optionally substituted piperazinyl, optionally substituted azetidinyl, optionally substituted pyrazolyl, optionally substituted furanyl, optionally substituted azabicyclo [3.2.1] octane or cyclopropyl; wherein the substituent is alkyl, amino, halogen or hydroxyl;
R₃ is alkyl or hydroxyl at each occurrence;
Rₐ and R_{b} are independently hydrogen, deuterium, alkyl, acyl, heteroaryl or heterocycloalkyl;
m and n are each independently 0, 1 or 2;
p is 0 or 1.

Preferably, in some embodiments of the present invention, the PTM is selected from

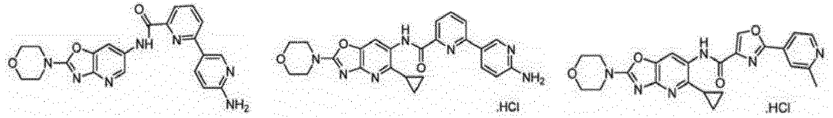

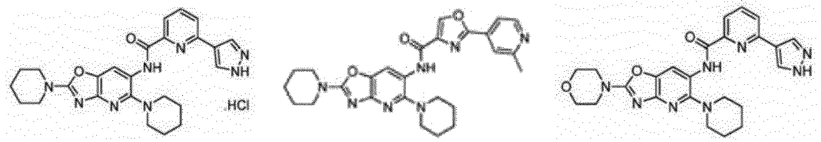

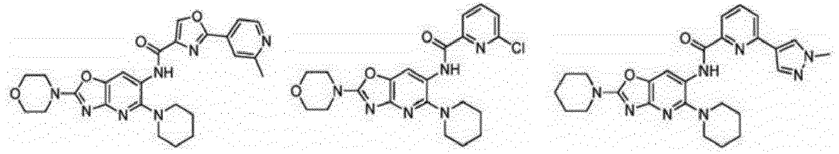

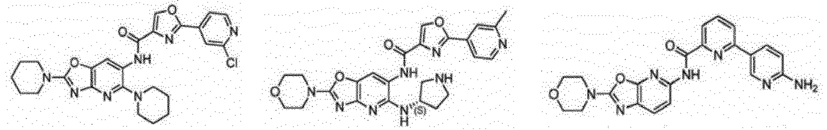

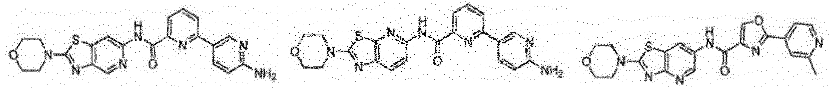

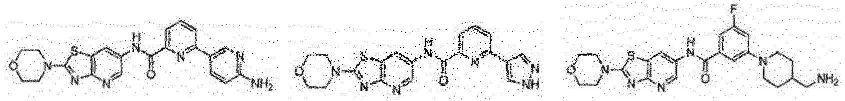

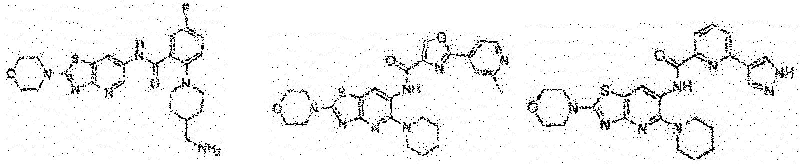

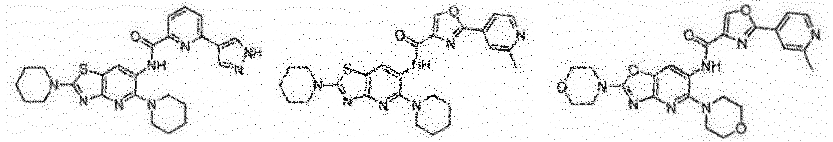

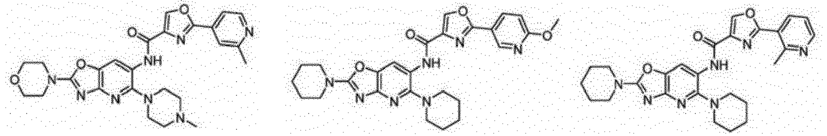

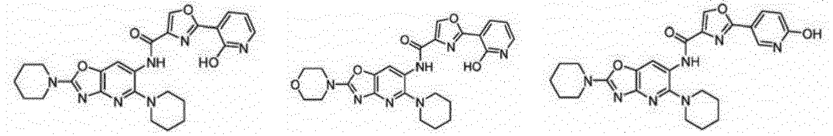

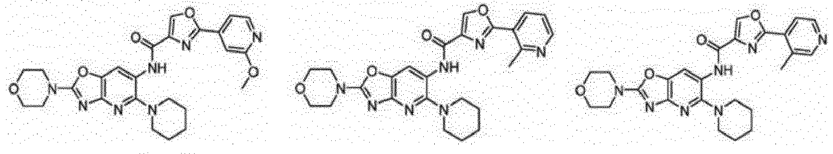

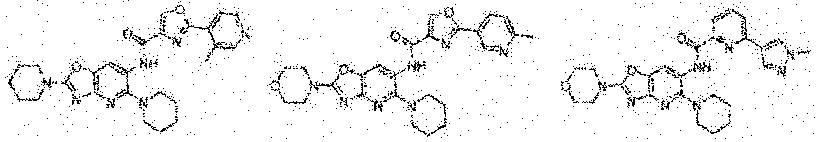

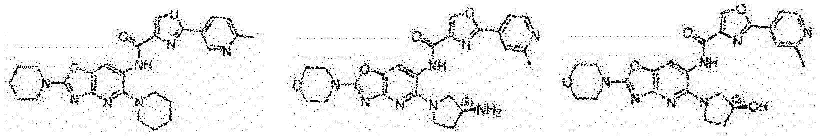

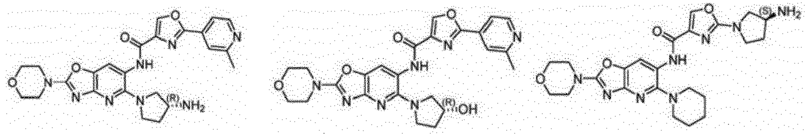

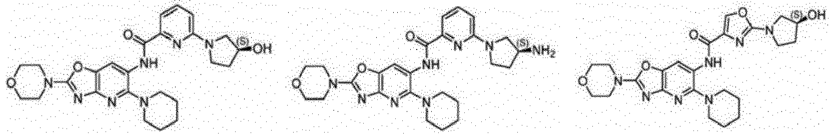

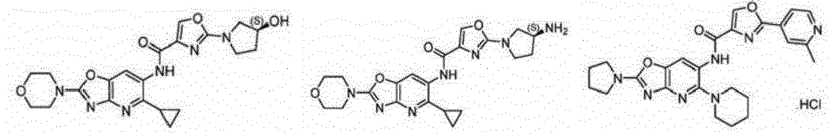

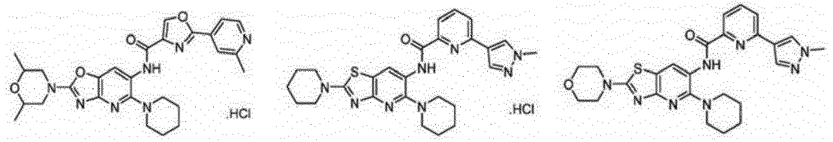

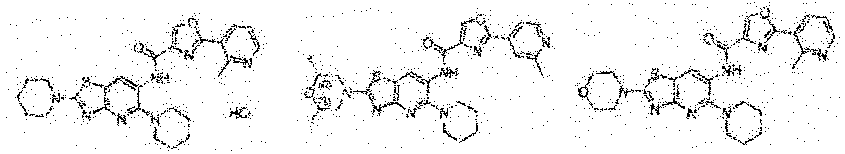

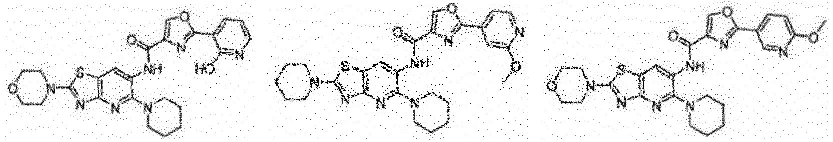

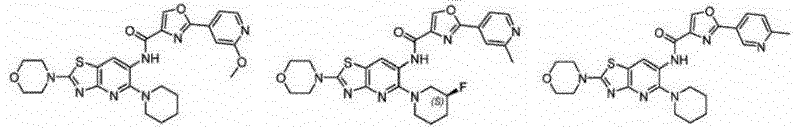

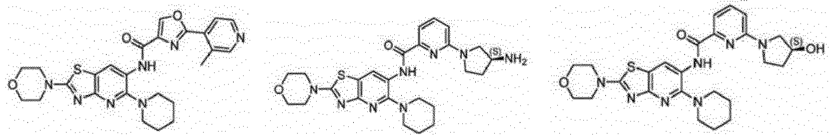

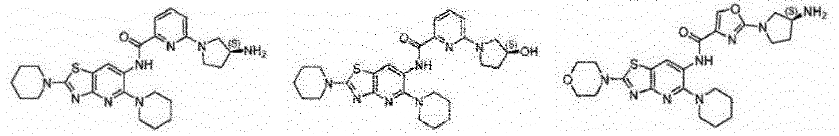

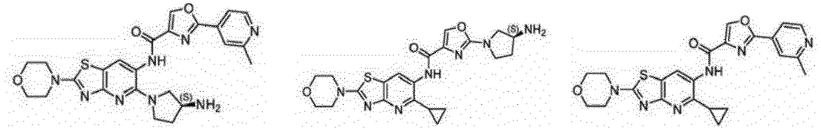

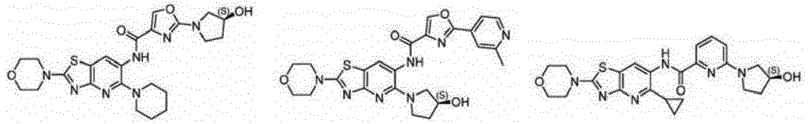

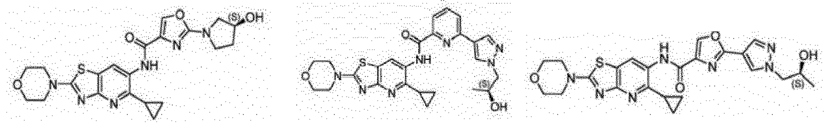

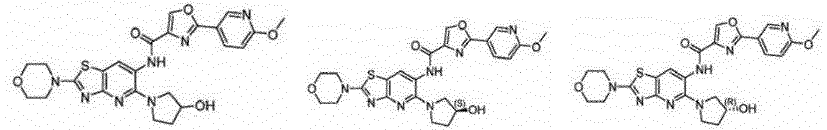

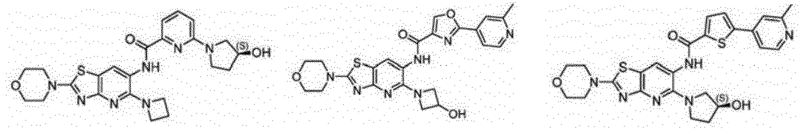

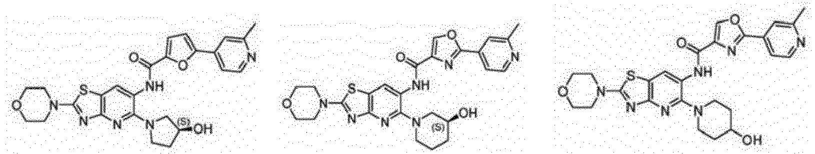

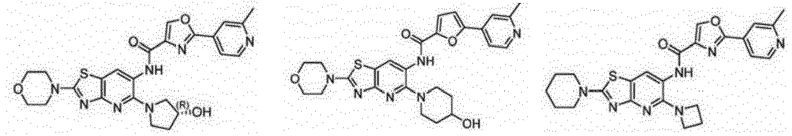

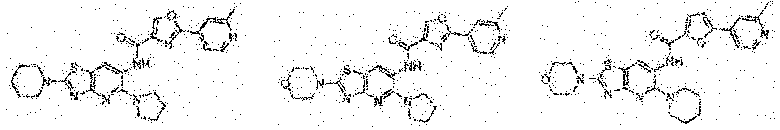

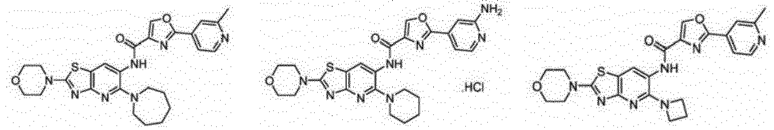

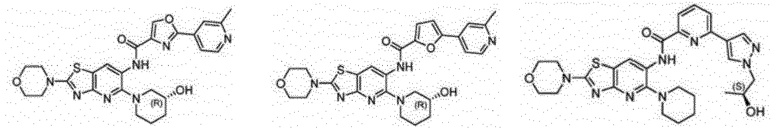

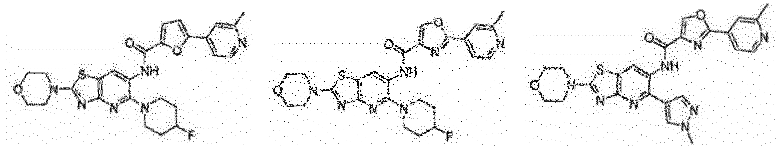

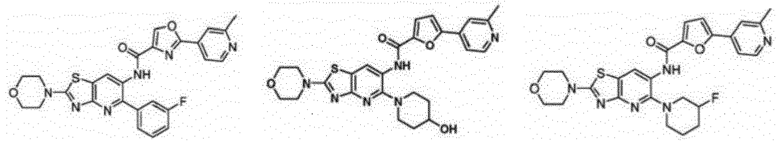

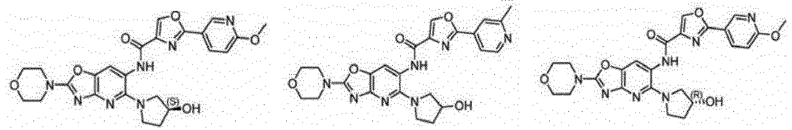

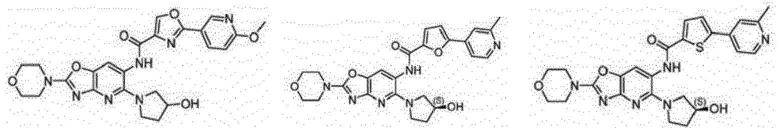

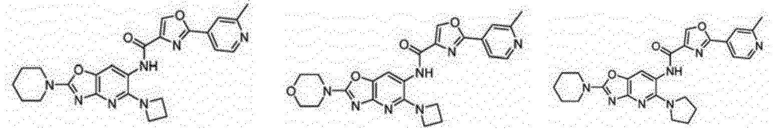

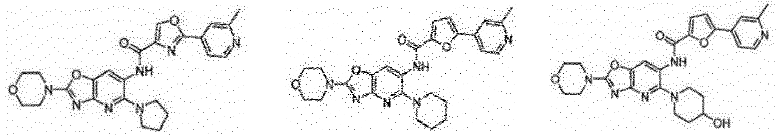

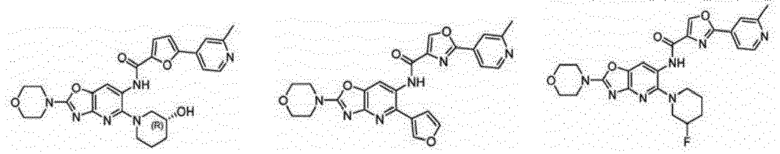

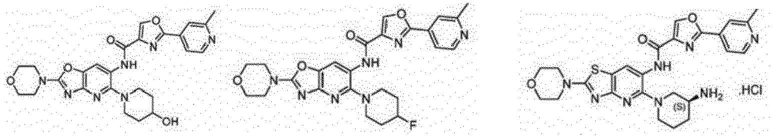

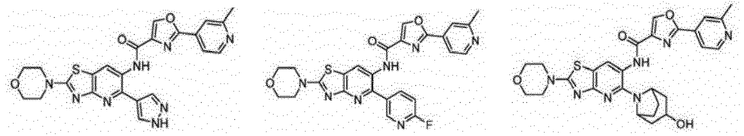

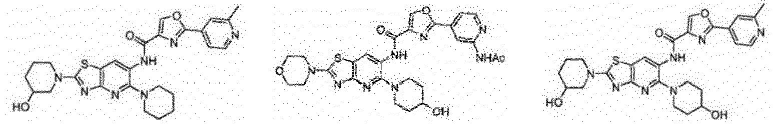

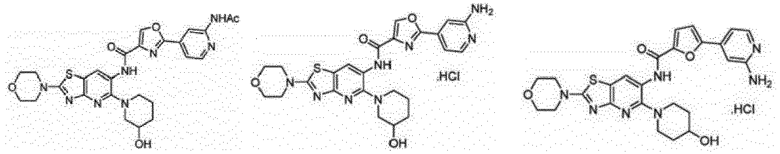

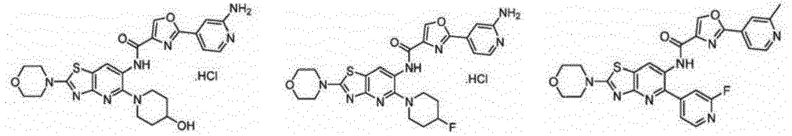

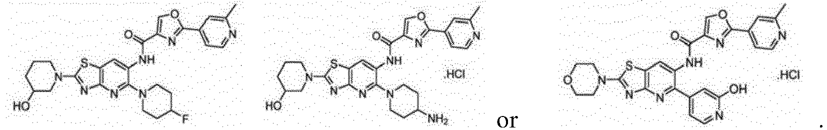

Preferably, in some embodiments of the present invention, the PTM has the following structure:

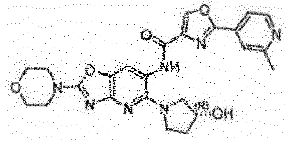

Preferably, in some embodiments of the present invention, the compound of formula I, and/or stereoisomer, enantiomer, diastereomer, deuterate, metabolite, hydrate, solvate, prodrug thereof and/or pharmaceutically acceptable salt thereof is a compound of formula IA, and/or stereoisomer, enantiomer, diastereomer, deuterate, metabolite, hydrate, solvate, prodrug thereof and/or pharmaceutically acceptable salt thereof, 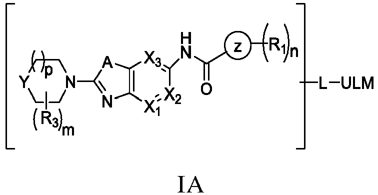 wherein, X₁, X₂, X₃, A, Y, p, R₁, R₃, m, Z, n are as defined in PTM-70, and L, ULM are as defined herein.

Preferably, in some embodiments of the present invention, the compound of formula I, and/or a stereoisomer, enantiomer, diastereomer, deuterate, metabolite, hydrate, solvate, prodrug thereof and/or its pharmaceutically acceptable salt thereof is a compound of formula IA-1, and/or stereoisomer, enantiomer, diastereomer, deuterate, metabolite, hydrate, solvate, prodrug thereof and/or pharmaceutically acceptable salt thereof, 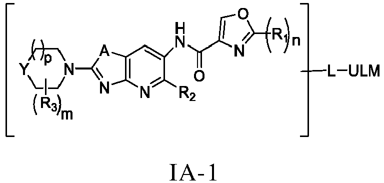 wherein, A, Y, p, R₁, R₂, R₃, m, n are as defined in PTM-70a, and L, ULM are as defined herein.

Preferably, in some embodiments of the present invention, the compound of formula I, and/or stereoisomer, enantiomer, diastereomer, deuterate, metabolite, hydrate, solvate, prodrug thereof and/or pharmaceutically acceptable salt thereof is a compound of formula IA-2, and/or stereoisomer, enantiomer, diastereomer, deuterate, metabolite, hydrate, solvate, prodrug thereof and/or pharmaceutically acceptable salt thereof, 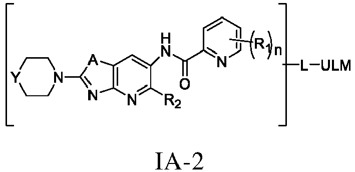 wherein, A, Y, R₁, R₂, n are as defined in PTM-70b, and L, ULM are as defined herein.

Preferably, in some embodiments of the present invention, the compound of formula I, and/or stereoisomer, enantiomer, diastereomer, deuterate, metabolite, hydrate, solvate, prodrug thereof and/or pharmaceutically acceptable salt thereof is a compound of formula IA-3, and/or its stereoisomer, enantiomer, diastereomer, deuterate, metabolite, hydrate, solvate, prodrug thereof and/or pharmaceutically acceptable salt thereof, 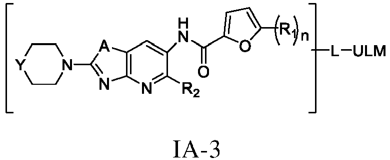 wherein: A, Y, R₁, R₂, n are as defined in PTM-70c, and L, ULM are as defined herein.

Preferably, in some embodiments of the present invention, the PTM has the following structure: 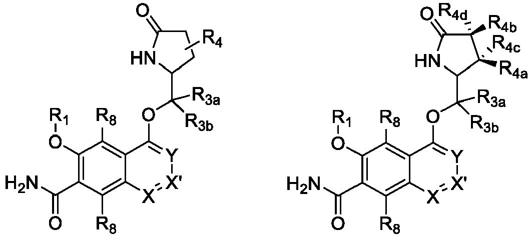 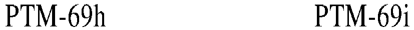 or a pharmaceutically acceptable salt thereof; wherein, in PTM-69h or PTM-69i:
X and X' are each independently CRs or N; Y is independently N or CR₈; provided that at least one of X, X' or Y is not N;
R₁ is C1-C6 alkyl or C3-C6 cycloalkyl, wherein the alkyl and cycloalkyl are optionally substituted by a substituent selected from halogen, deuterium, -OH, cyano, C1-C3 alkyl, C3-C6 cycloalkyl or C1-C6 alkylthio;
R₃ₐ and R_{3b} are independently hydrogen or C1-C3 alkyl at each occurrence;
R₄ is independently halogen, C1-C6 alkyl, C2-6 alkenyl,-OR₅,-(CR₃ₐR_{3b})ₙ-(3-6 membered cycloalkyl), -(CR₃ₐR_{3b})ₙ-(4-6 membered heterocycloalkyl), -NR₁₁ₐR_{11b} at each occurrence, wherein, the alkyl, cycloalkyl, heterocycloalkyl are each optionally and independently substituted by 1-5 substituents selected from halogen, OH, cyano, C(O)(CH₂)ₜCN or C1-C6 alkoxyl; or two R₄ together with their attached carbon atoms form cyclopropyl, cyclopentyl, or cyclobutyl, wherein the cyclopropyl, cyclopentyl and cyclobutyl are optionally substituted by 1-3 substituents selected from F, Cl, OH, methyl, ethyl, propyl, C1-C3 fluoroalkyl, C1-C3 difluoroalkyl, C1-C3 hydroxyalkyl, methoxy and ethoxy;
R₄ₐ and R_{4b} are each independently hydrogen, deuterium, deuterium, F, OH,-ORs, methyl, ethyl, vinyl, cyclopropyl or propyl, optionally substituted by 1-5 deuterium, fluorine, methoxy or OH;
R_{4c} and R_{4d} are independently halogen, OH, deuterium, C1-C6 alkyl, C2-6 alkenyl, - OR₅, -(CR₃ₐR_{3b})ₙ-(3-6 membered cycloalkyl), -(CR₃ₐR_{3b})ₙ-(4-6 membered heterocycloalkyl), NH₂ at each occurrence, wherein, the alkyl, cycloalkyl, heterocycloalkyl are each optionally and independently substituted by 1-5 substituents selected from deuterium, halogen, OH, cyano or C1-C6 alkoxy; or R_{4c} and R_{4d} together with the carbon atoms to which they are attached form 4-7-membered heterocycloalkyl or 3-7-membered cycloalkyl, wherein, the heterocycloalkyl and cycloalkyl are independently optionally substituted by 1-3 fluorines, C1-C3 alkyls or C1-C3 fluoroalkyls; or
R_{4c} and R₄ₐ together with the atoms to which they are attached form 4-7-membered heterocycloalkyl or 3-7-membered cycloalkyl, wherein the heterocycloalkyl and cycloalkyl are each independently optionally substituted by 1-3 fluorines, C1-C3 alkyls or C1-C3 fluoroalkyls;
R₅ is independently hydrogen or C1-C6 alkyl, wherein the alkyl is optionally substituted by F;
R₈ is each independently hydrogen, deuterium, halogen, cyano, C1-C6 alkyl, C2-6 alkenyl, C2-6 alkynyl, 5-6-membered heteroaryl or aryl, wherein, the alkyl, alkenyl, alkynyl, heteroaryl or aryl is optionally substituted by one, two or three halogens, -NR₁₁ₐR_{11b}, C3-C6 cycloalkyl, C3-C6 heterocycloalkyl containing one or two N heteroatoms, C1-C3 alkyl or oxo;
R_{8'} is hydrogen, deuterium, deuterium, halogen or cyano;
R₁₀ is C1-C6 alkyl, wherein the alkyl is optionally substituted by deuterium, F or cyano;
R₁₁ₐ and R_{11b} are each independently hydrogen or C1-C6 alkyl, wherein the alkyl is optionally substituted by OH;
n is independently 0, or 1;
p is independently 0 or 1; and
t is 1, 2 or 3.

In some embodiments of the present invention, in PTM 69h or PTM-69i, R¹ is C1-C6 alkyl optionally substituted by 1-3 deuteriums, fluorines, chlorines or C1-C3 alkoxyls; preferably R₁ is methyl, ethyl, propyl or isopropyl, wherein each R₁ is optionally substituted by deuterium, fluorine or methoxy.

In some embodiments of the present invention, in PTM 69h or PTM-69i, R₃ₐ and R_{3b} are independently hydrogen or methyl at each occurrence.

In some embodiments of the present invention, in PTM69h or PTM-69i, R₄ is independently fluorine, chlorine, hydroxyl, or C1-C3 alkyl optionally substituted by 1-5 deuterated, fluorine, chlorine, hydroxyl or C1-C3 alkoxy at each occurrence, or two R₄ together with the carbon atom to which they are attached form cyclopropyl, cyclobutyl or cyclopentyl, the cyclopropyl, cyclobutyl and cyclopentyl are optionally substituted by 1-3 fluorine, chlorine, hydroxyl, methyl, ethyl, propyl, C1-C3 hydroxylalkyl, C1-C3 fluoroalkyl, C1-C3 difluoroalkyl, C1-C3 trifluoroalkyl, methoxy or ethoxy; preferably R₄ is independently fluorine, hydroxyl, methyl, ethyl, propyl at each occurrence, wherein the methyl, ethyl or propyl is optionally substituted by 1, 2 or 3 fluorine, hydroxyl or methoxy, or two R₄ together with the carbon atoms to which they are attached form cyclopropyl, cyclobutyl or cyclopentyl, the cyclopropyl, cyclobutyl and cyclopentyl are optionally substituted by 1 to 3 fluoro, chlorine, hydroxyl, methyl, fluoromethyl, difluoromethyl, trifluoromethyl, ethyl, propyl, C1-C3 hydroxyalkyl, methoxy or ethoxy.

In some embodiments of the present invention, in PTM 69h or PTM-69i, R₈ is each independently hydrogen, deuterium, halogen or C1-C6 alkyl, wherein the alkyl is optionally substituted by fluorine; preferably R₈ is each independently hydrogen, deuterium, fluorine or methyl.

In some embodiments of the present invention, in PTM 69h or PTM-69i, R₈ is each independently C2-6 alkenyl or C2-6 alkynyl, wherein the alkenyl, alkynyl are optionally substituted by C3-C6 cycloalkyl, C3-C6 heterocycloalkyl containing 1 or 2 N heteroatoms; preferably R₈ is each independently vinyl, ethynyl, and optionally substituted by cyclohexanyl, piperidinyl or piperazinyl.

In some embodiments of the present invention, in PTM-69i, R₄ₐ is hydrogen, deuterium, methyl, ethyl or propyl optionally substituted by fluorine, deuterium or methoxy.

In some embodiments of the present invention, in PTM-69i, R_{4b} is hydrogen or fluorine.

In some embodiments of the present invention, in PTM-69i, R_{4c} is hydroxyl.

In some embodiments of the present invention, in PTM-69i, R_{4d} is fluorine, methoxy or hydroxyl, or methyl optionally substituted by one, two or three fluoros, or ethyl optionally substituted by one, two or three fluoros.

In some embodiments of the present invention, in PTM-69i, R_{4c} and R₄ₐ or R_{4c} and R_{4d} together with the carbon atoms to which they are attached form cyclopropyl optionally substituted by 1-3 fluorine, C1-C3 alkyl or C1-C3 fluoroalkyl.

Preferably, in some embodiments of the present invention, the PTM is selected from

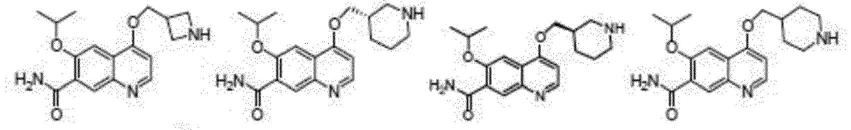

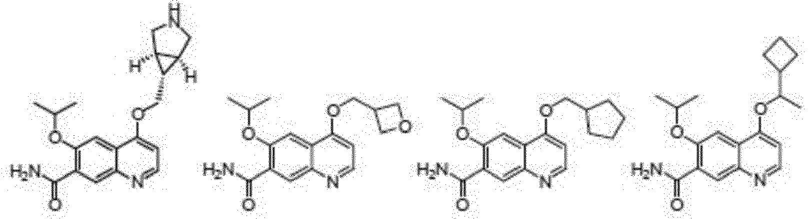

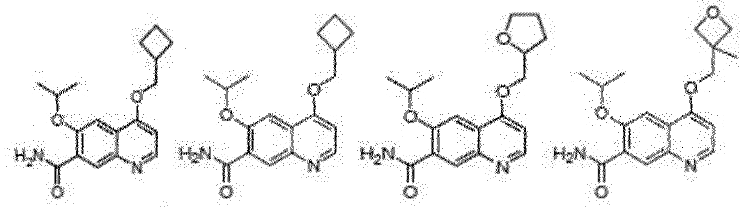

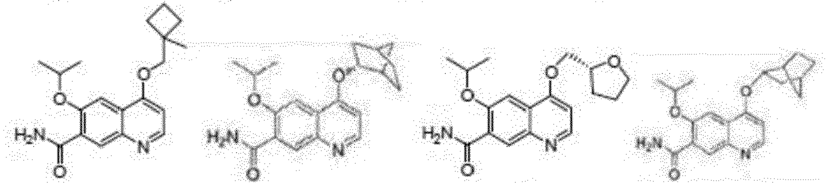

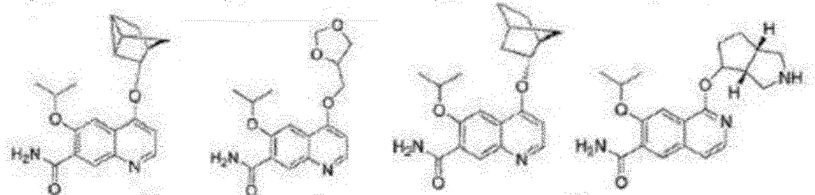

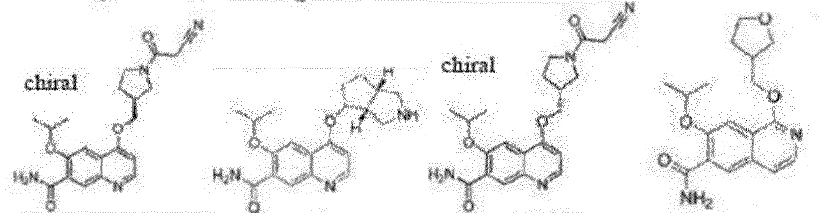

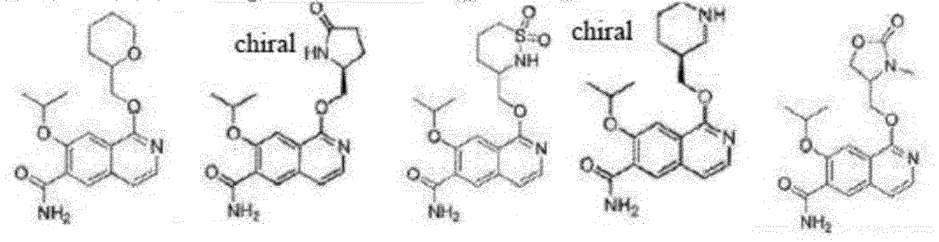

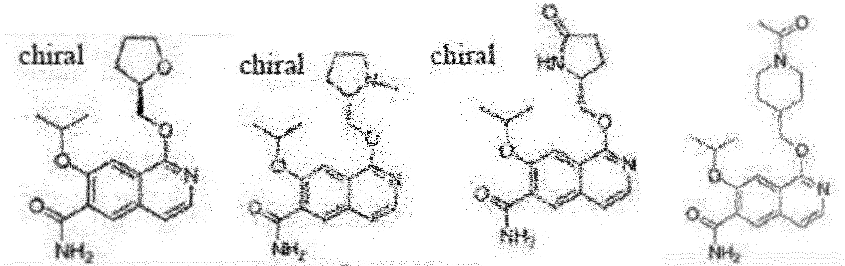

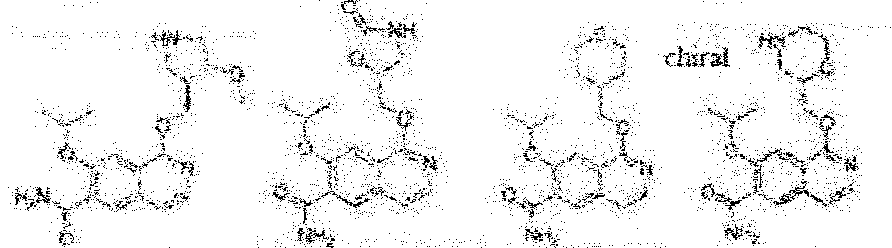

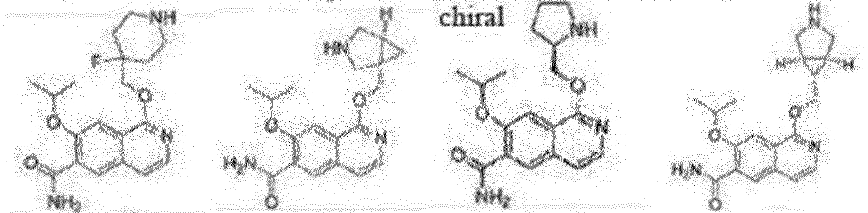

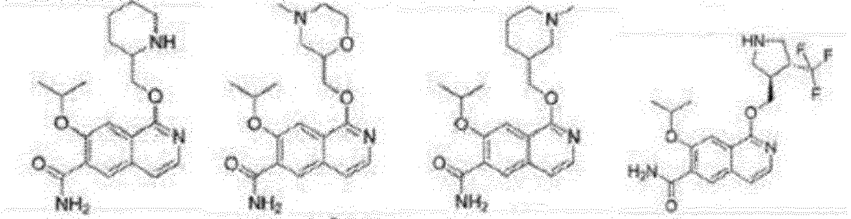

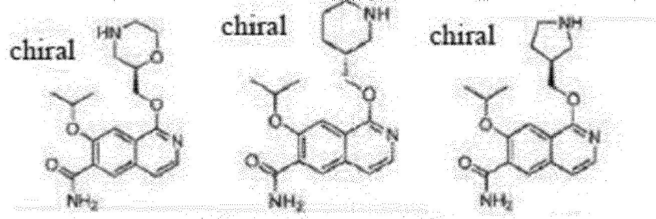

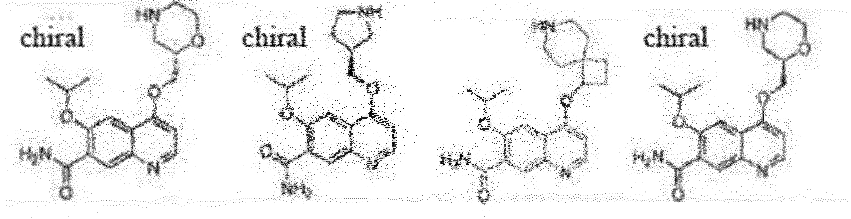

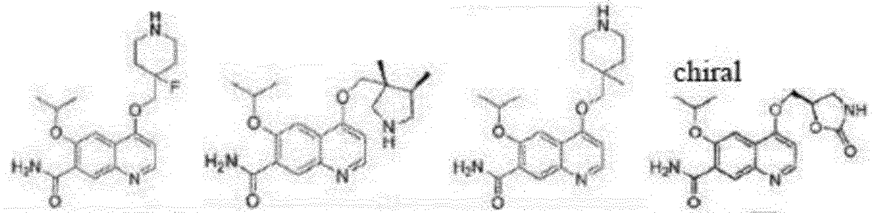

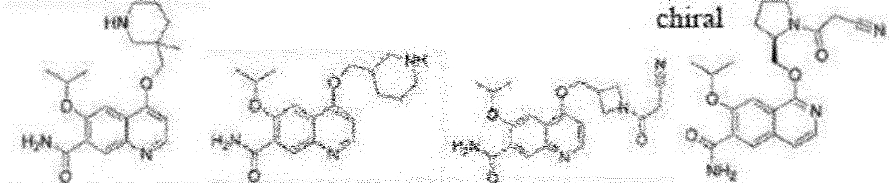

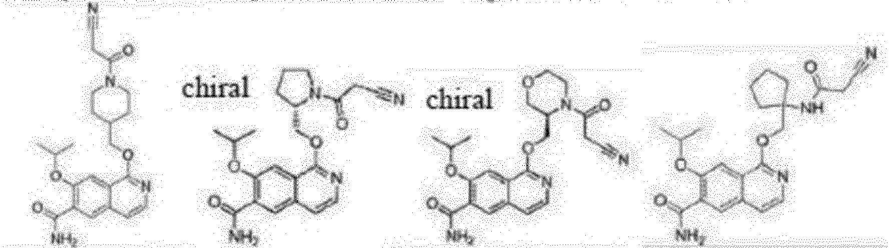

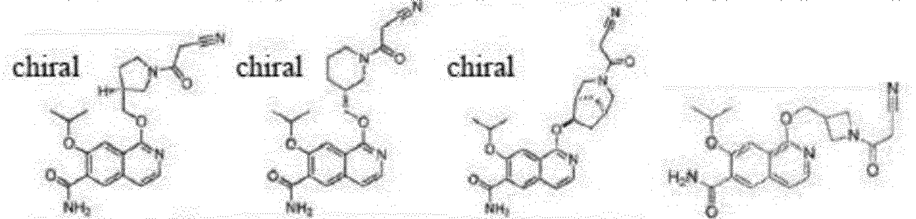

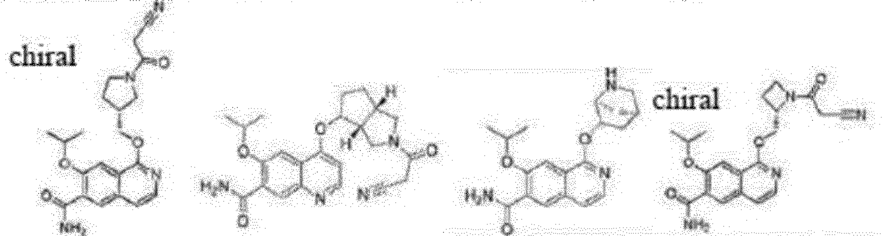

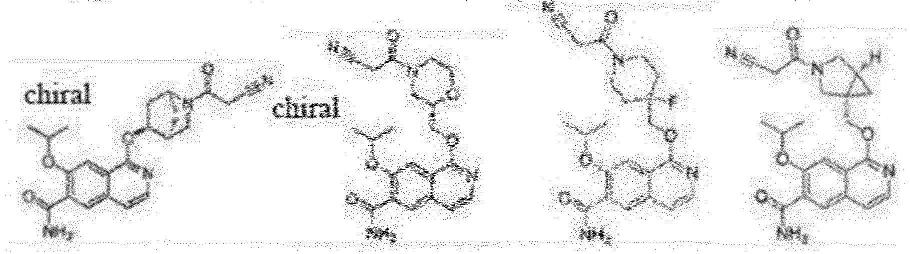

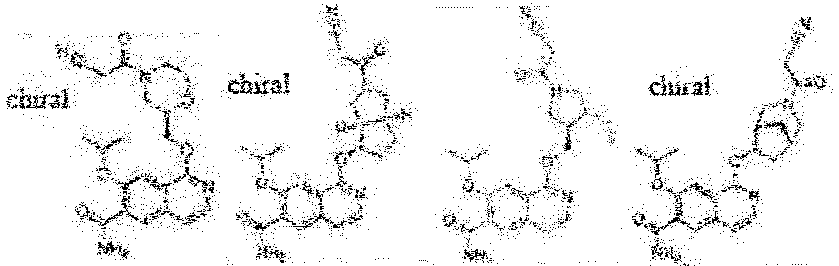

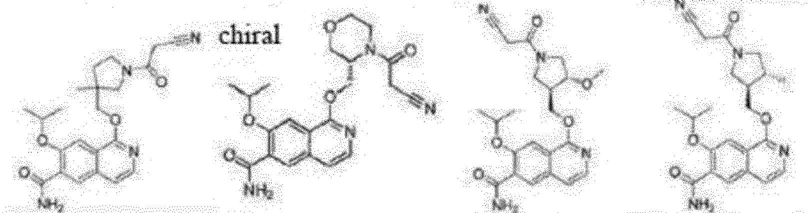

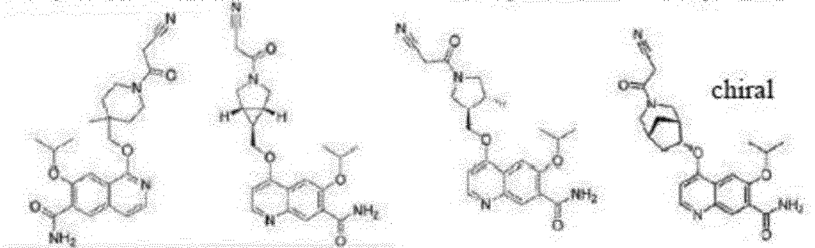

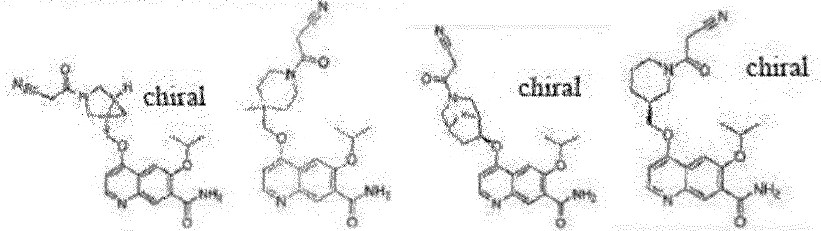

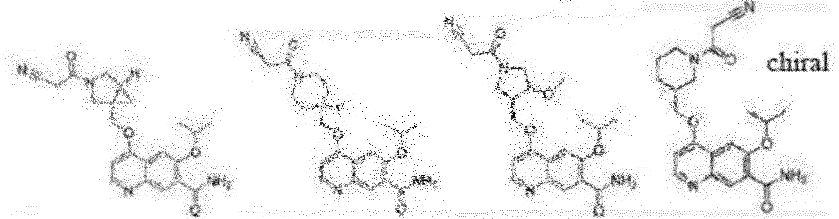

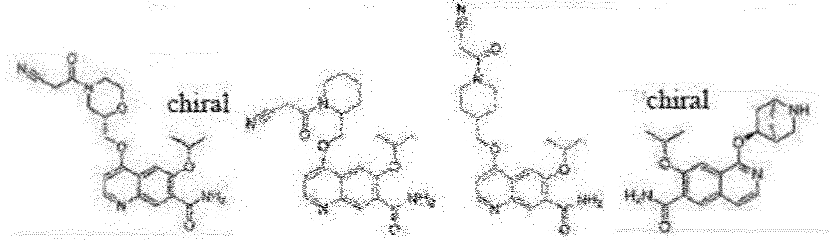

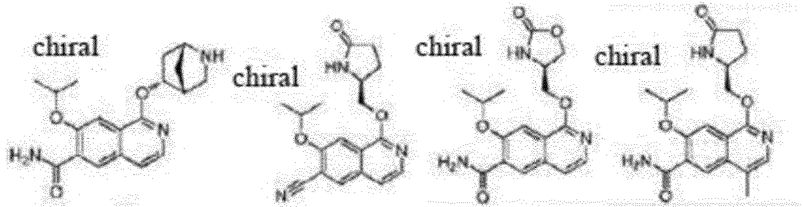

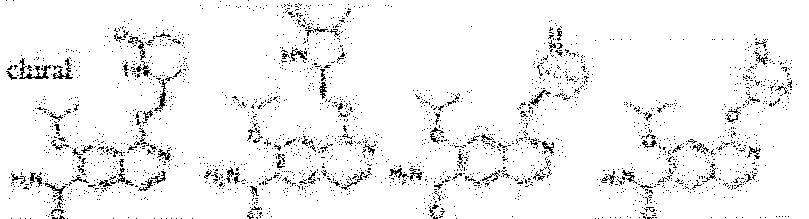

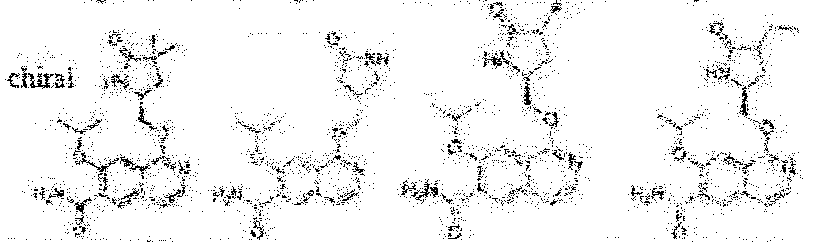

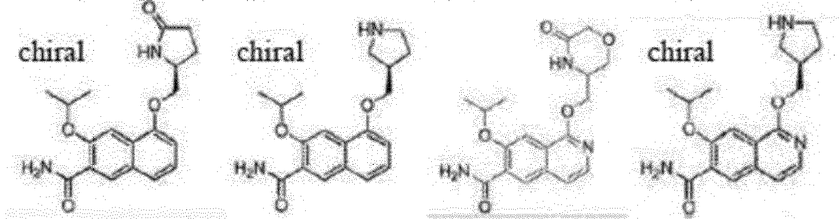

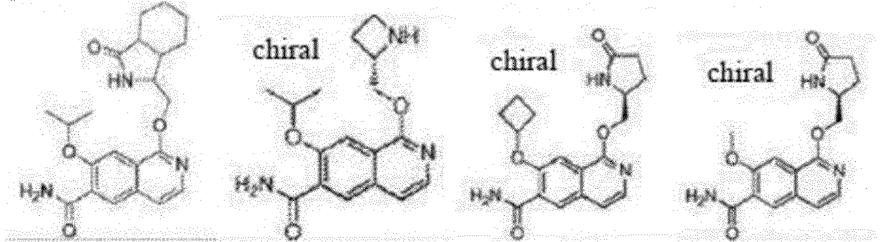

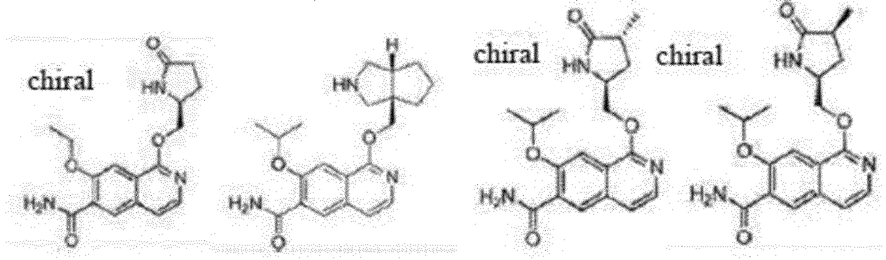

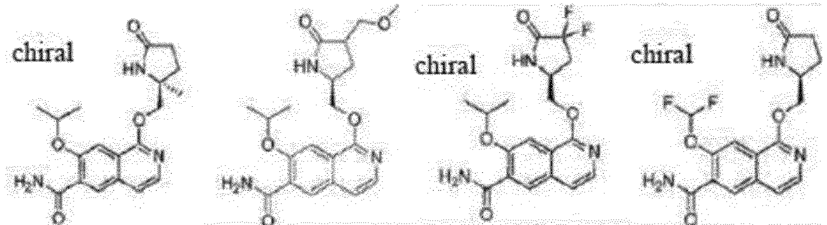

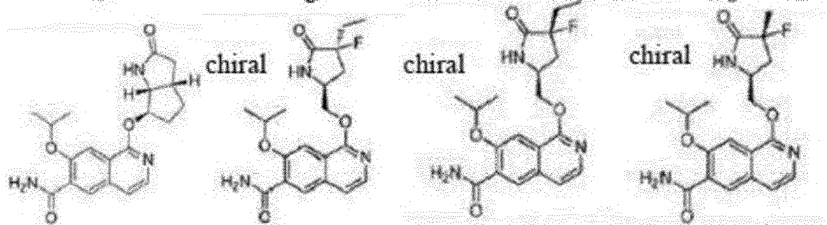

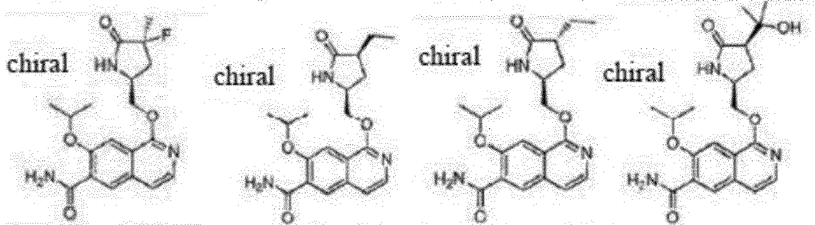

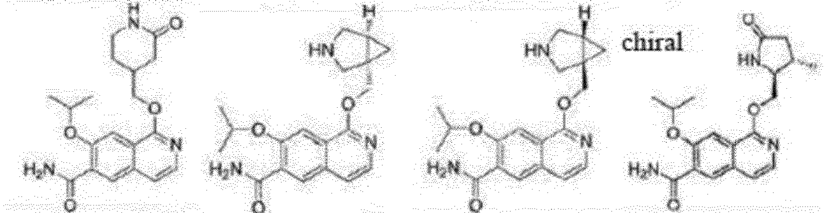

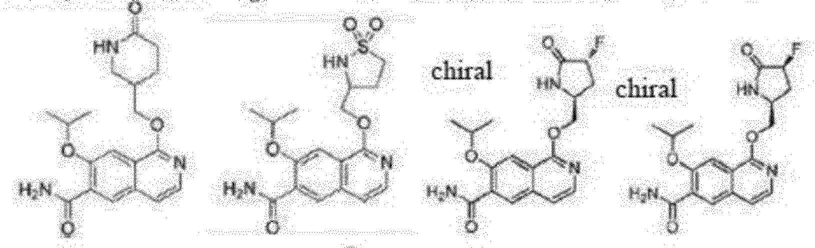

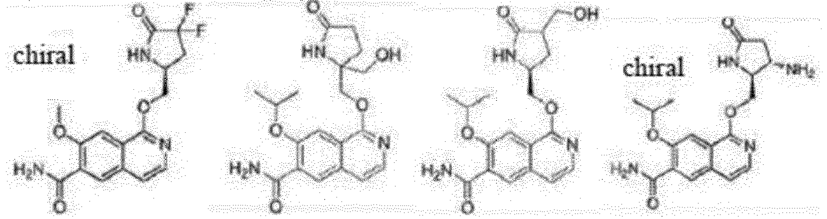

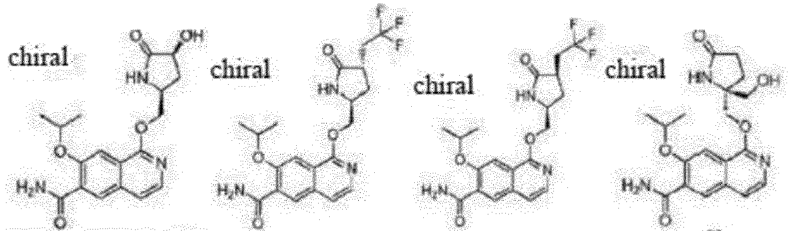

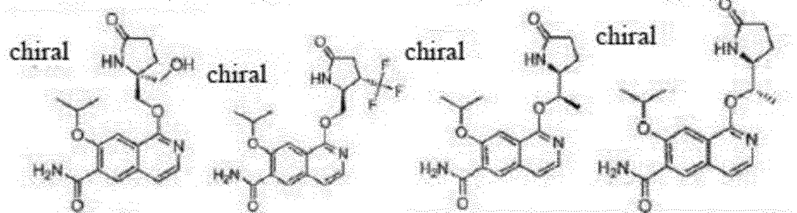

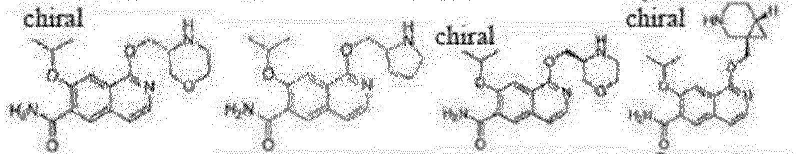

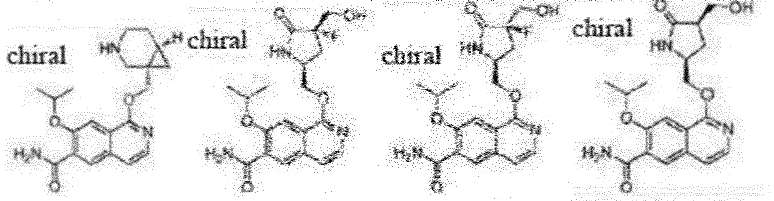

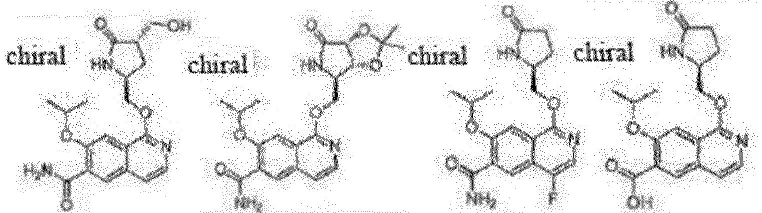

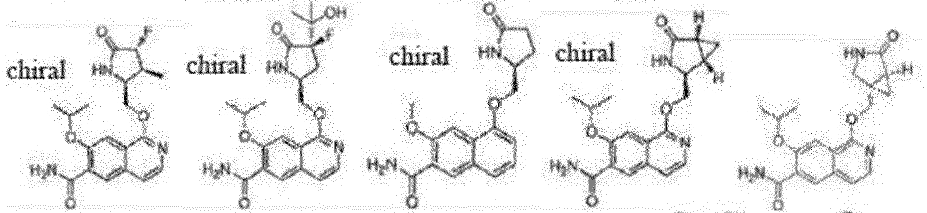

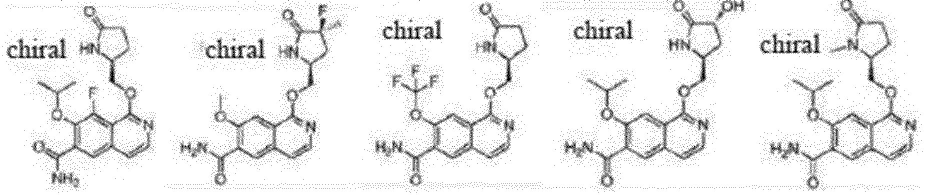

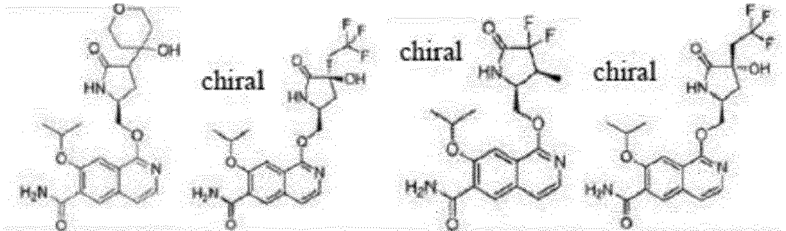

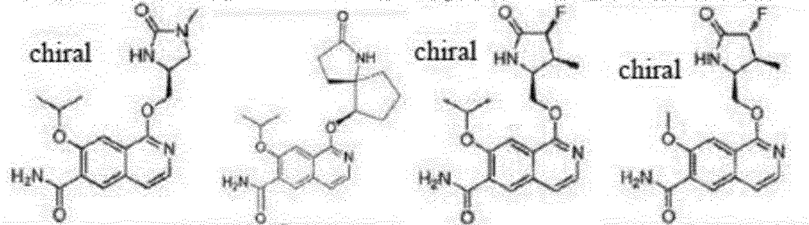

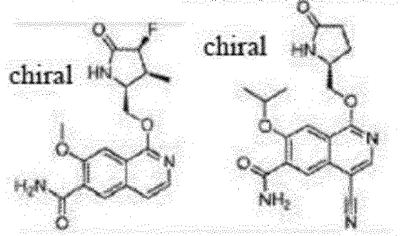

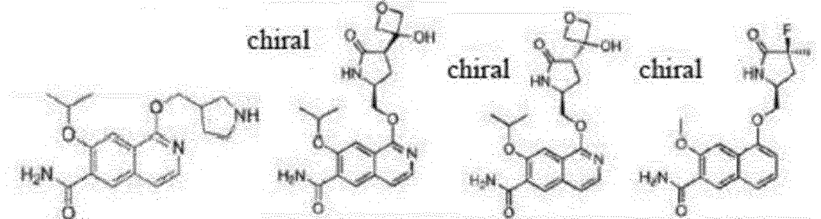

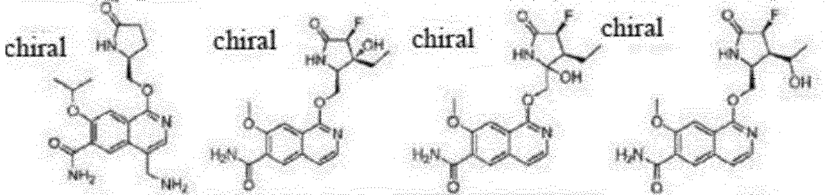

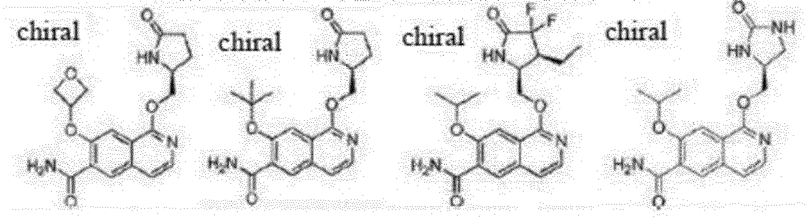

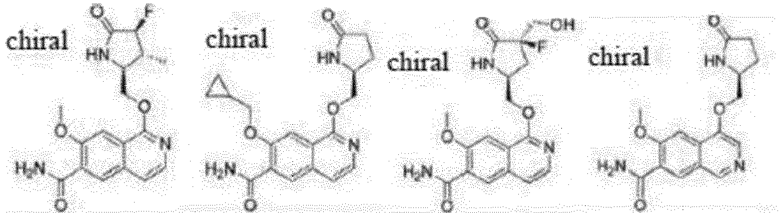

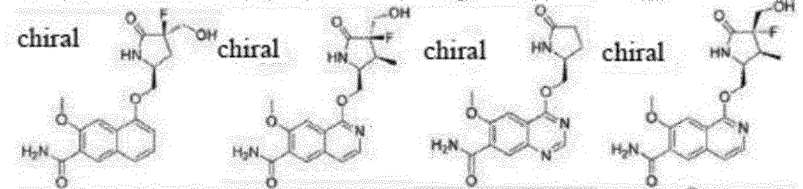

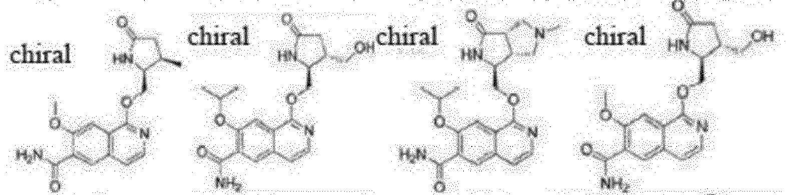

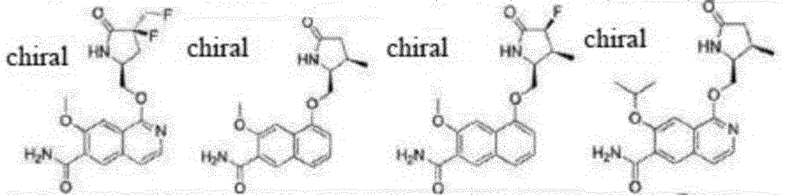

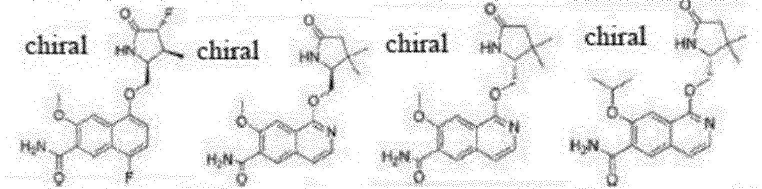

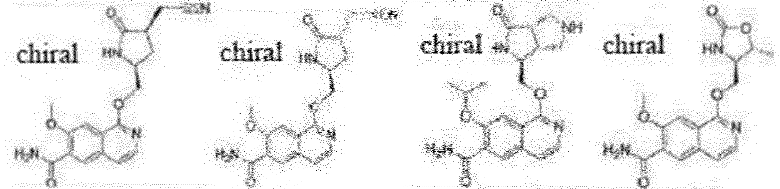

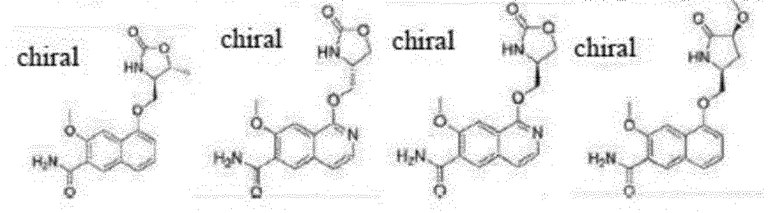

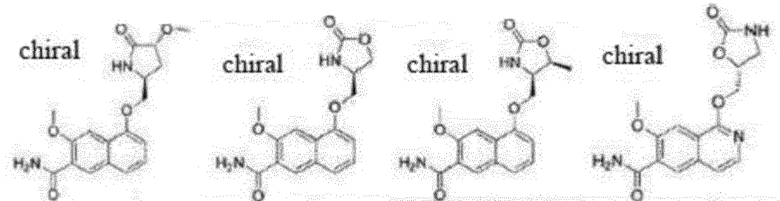

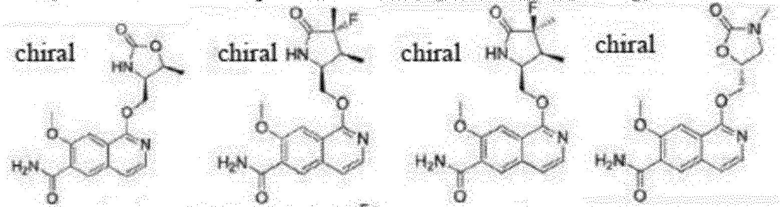

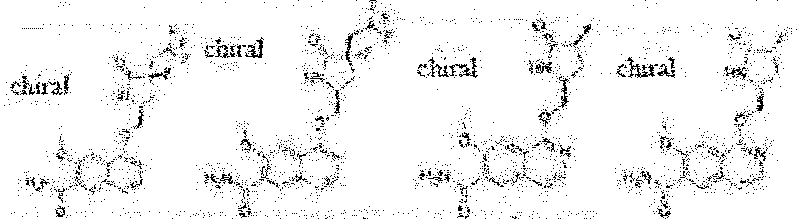

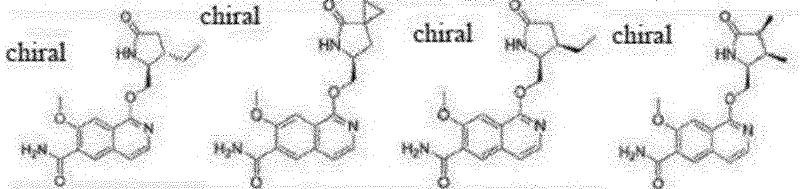

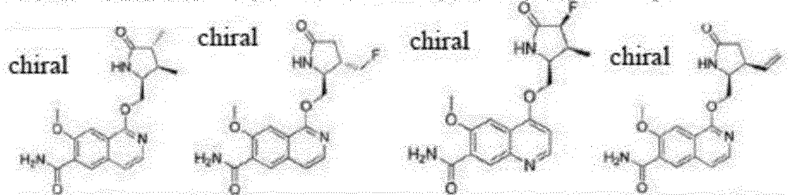

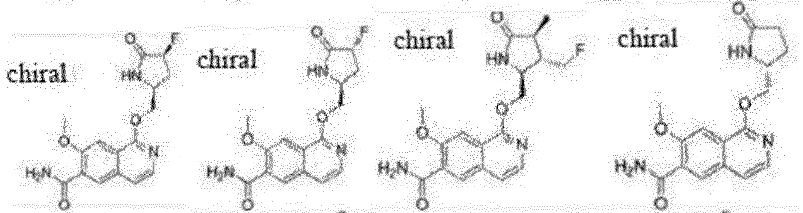

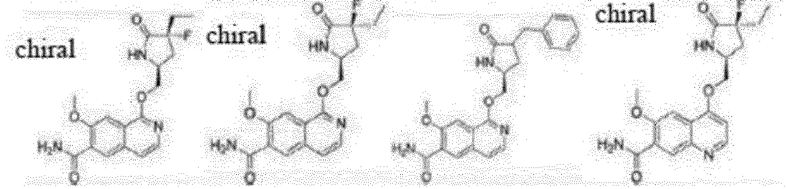

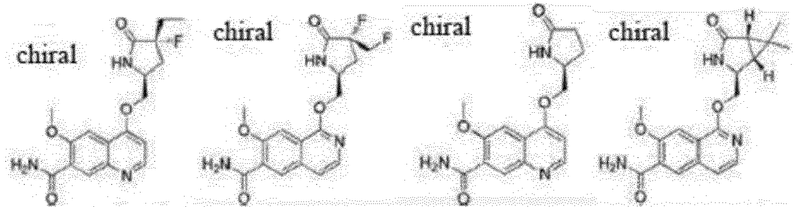

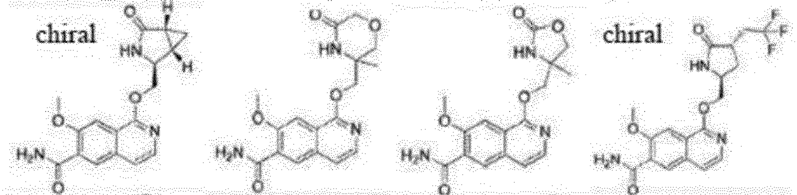

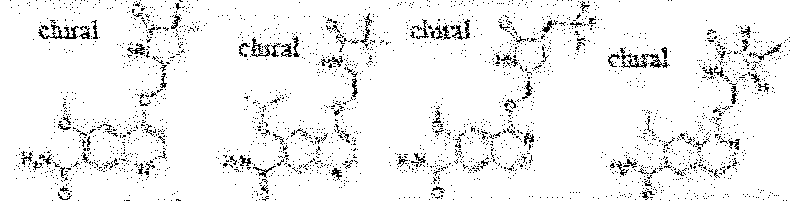

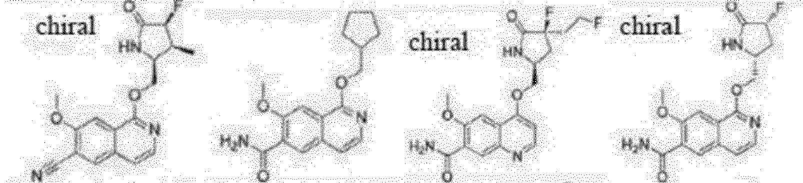

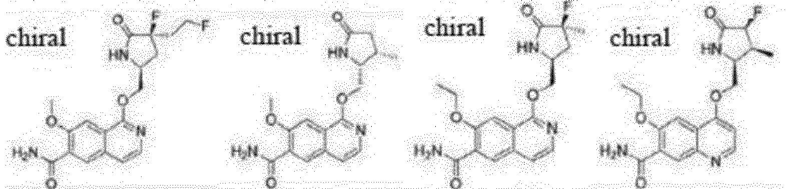

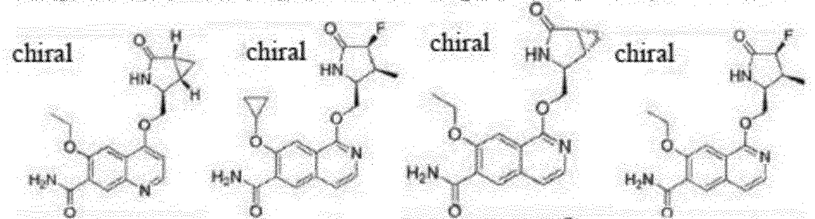

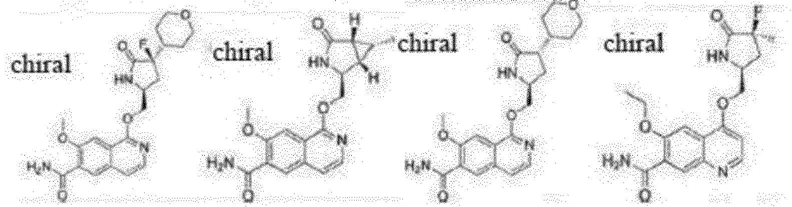

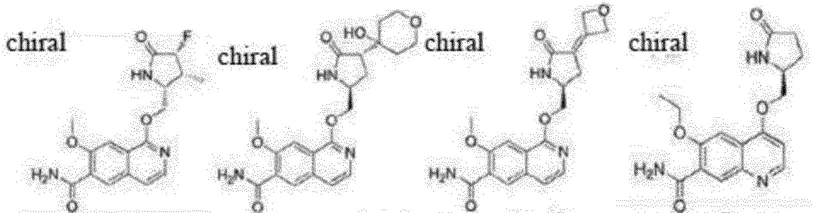

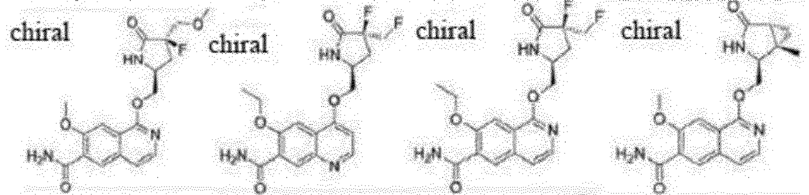

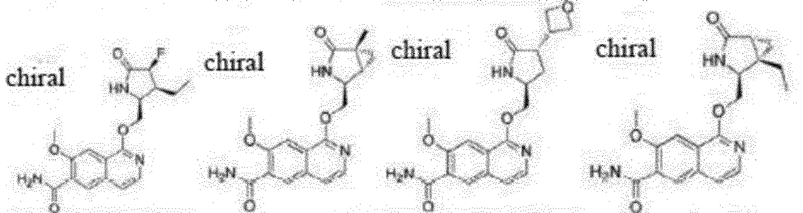

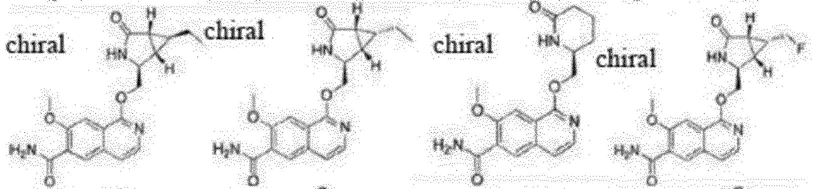

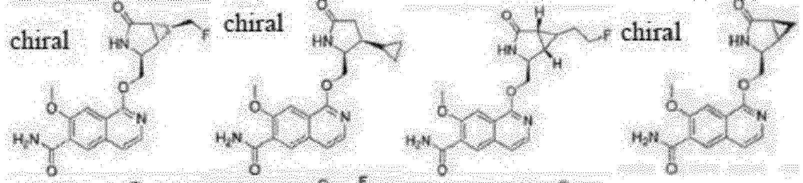

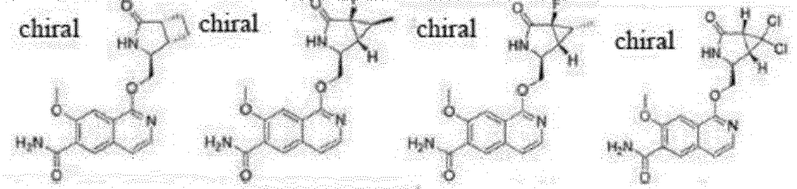

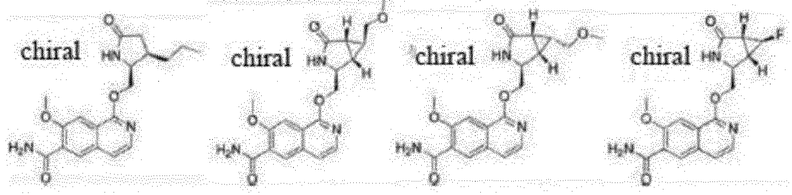

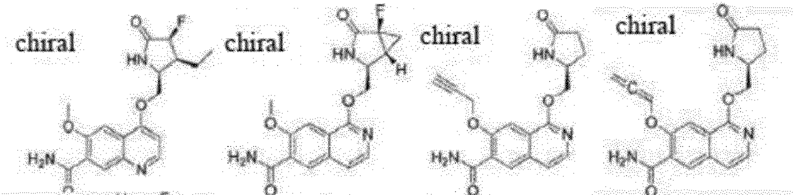

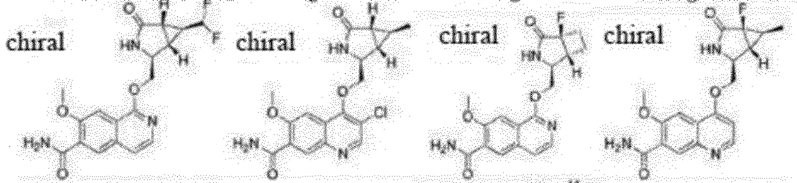

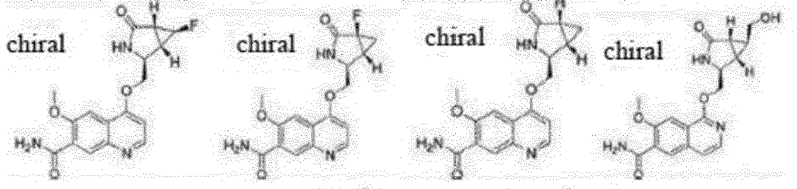

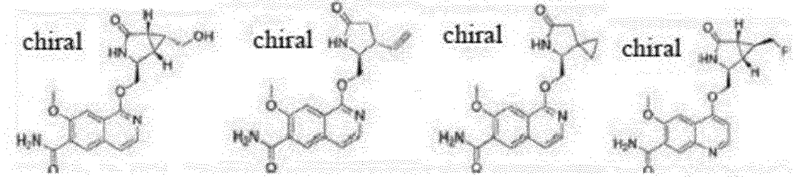

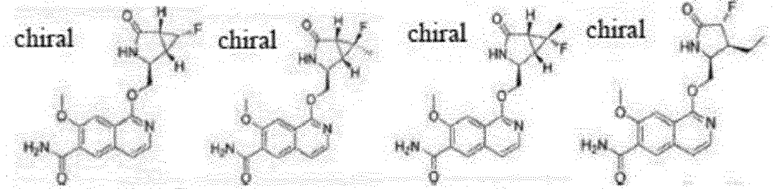

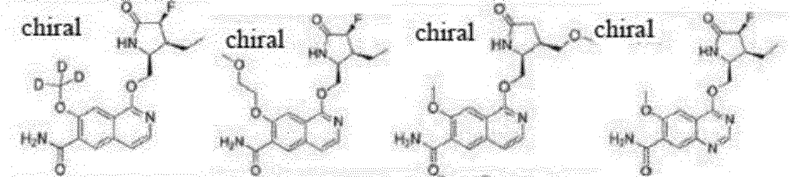

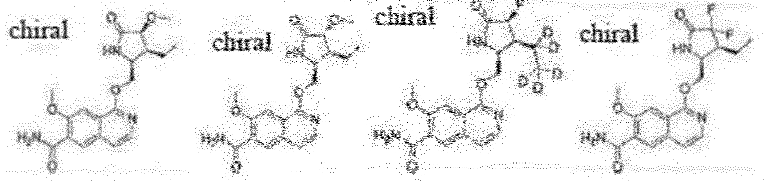

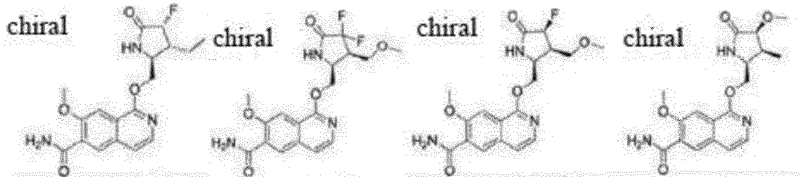

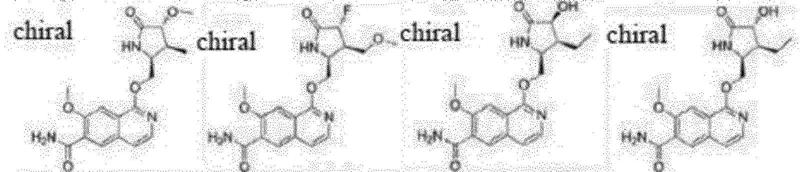

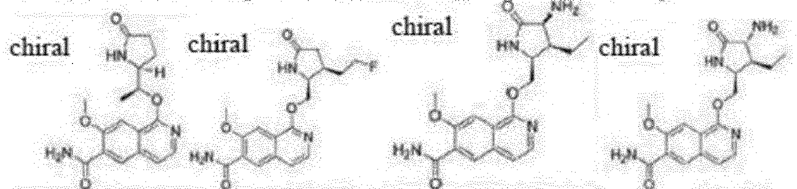

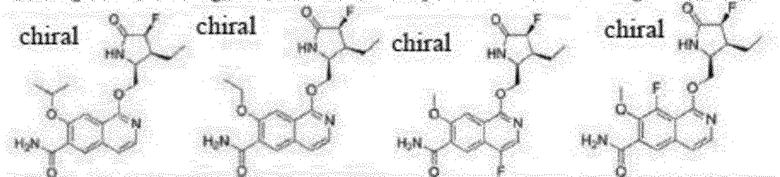

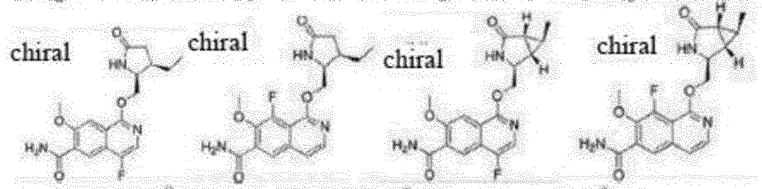

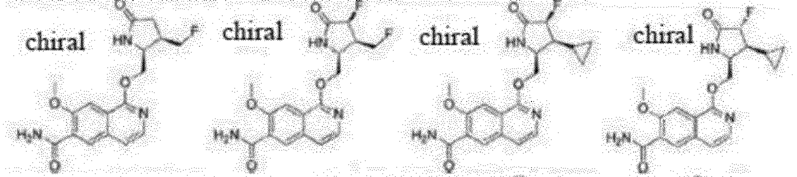

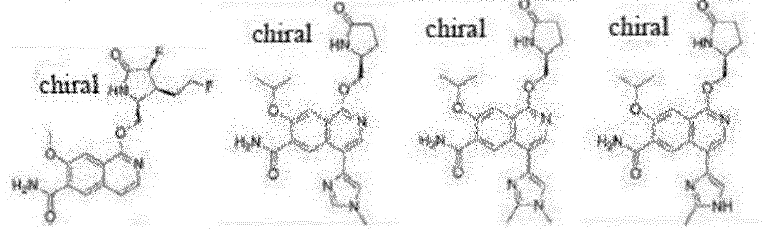

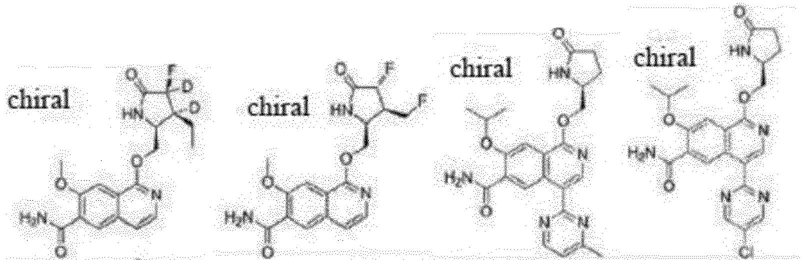

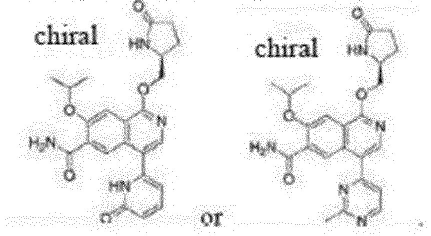

Preferably, in some embodiments of the present invention, the PTM is selected from

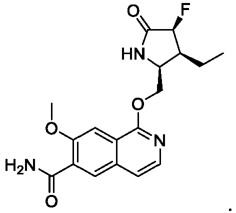

More preferably, in some embodiments of the present invention, the PTM is selected from 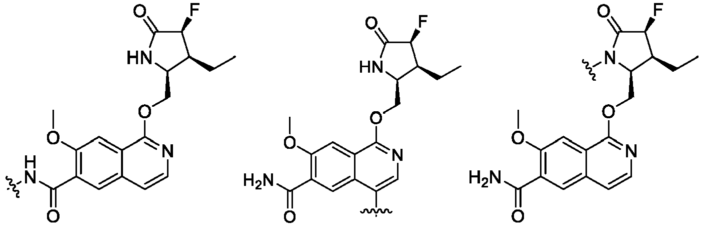 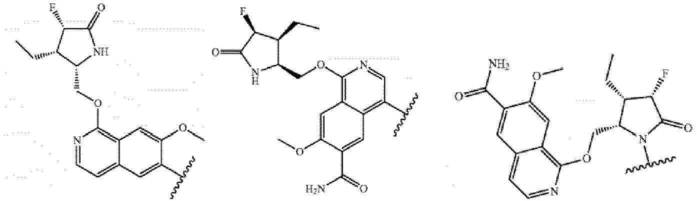 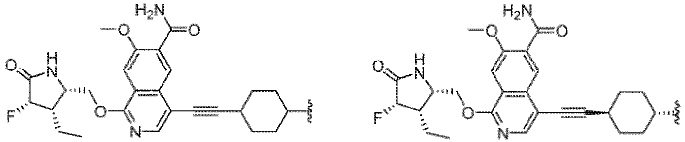 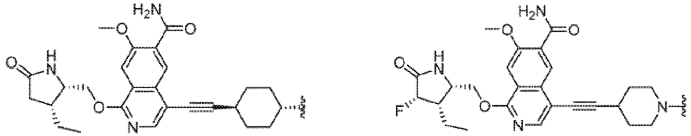 or

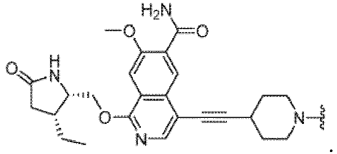

Preferably, in some embodiments of the present invention, the compound of formula I, and/or a stereoisomer, enantiomer, diastereomer, deuterate, metabolite, hydrate, solvate, prodrug thereof and/or a pharmaceutically acceptable salt thereof is a compound of formula IB, and/or a stereoisomer, enantiomer, diastereomer, deuterate, metabolite, hydrate, solvate, prodrug thereof and/or a pharmaceutically acceptable salt thereof, 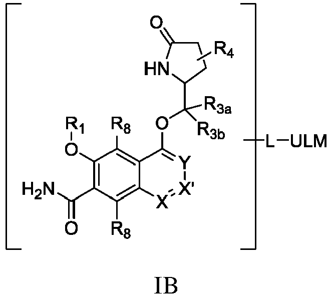 wherein: X, X', Y, R₁, R₄, R₃ₐ, R_{3b}, R₈, n are as defined in PTM-69h, and L, ULM are as defined herein.

Preferably, in some embodiments of the present invention, the compound of formula I, and/or a stereoisomer, enantiomer, diastereomer, deuterate, metabolite, hydrate, solvate, prodrug thereof and/or pharmaceutically acceptable salt thereof is a compound of formula IB-1, and/or a stereoisomer, enantiomer, diastereomer, deuterate, metabolite, hydrate, solvate, prodrug thereof and/or a pharmaceutically acceptable salt thereof, 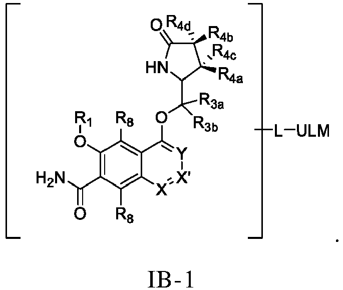 wherein: X, X', Y, R₁, R₄ₐ, R_{4b}, R_{4c}, R_{4d}, R₃ₐ, R_{3b}, R₈, n are as defined in PTM-69i, and L, ULM are as defined herein.

Preferably, in some embodiments of the present invention, the PTM has the following structure: 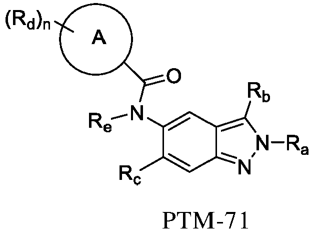 wherein, in PTM-71:
ring A is 6-10 membered aryl or 5-10 membered heteroaryl;
R_{d} is each independently hydrogen, deuterium, halogen, cyano, C1-C6 alkyl, C3-C6 cycloalkyl, or 5-10-membered heteroaryl, the alkyl, cycloalkyl, and heteroaryl are optionally substituted by one or more groups selected from halogen, hydroxyl, or amino;
n is 1, 2, 3 or 4;
Rₑ is hydrogen or C1-C6 alkyl;
R_{c} is hydrogen, deuterium,-O-(C 1-C6 alkyl),-O-(C3-C8 cycloalkyl), -O-(3-8-membered heterocycloalkyl), -O-aryl,-O-heteroaryl,-N(C1-C6 alkyl)₁₋₂, -NH(C3-C8 cycloalkyl), -NH (3-8-membered heterocycloalkyl), -NH-aryl, -NH-heteroaryl, C1-C6 alkyl, C2-C6 alkenyl, C2-C6 alkynyl, C3-C8 cycloalkyl, 3-8membered heterocycloalkyl, 6-10 membered aryl, 5-10 membered heteroaryl, the alkyl, cycloalkyl, heterocycloalkyl, aryl and heteroaryl are optionally substituted by one or more groups independently selected from hydroxyl, amino, halogen, cyano or -O-(C1-C6 alkyl);
R_{b} is hydrogen, deuterium,-O-(C1-C6 alkyl),-O-(C3-C8 cycloalkyl), -O-(3-8-membered heterocycloalkyl), -O-aryl, -O-heteroaryl, -N(C1-C6 alkyl)₁₋₂, -NH(C3-C8 cycloalkyl), -NH (3-8-membered heterocycloalkyl), -NH-aryl, -NH-heteroaryl, C1-C6 alkyl, C2-C6 alkenyl, C2-C6 alkynyl, C3-C8 cycloalkyl, 3-8membered heterocycloalkyl, 6-10 membered aryl, 5-10 membered heteroaryl, the alkyl, cycloalkyl, heterocycloalkyl, aryl, and heteroaryl are optionally substituted by one or more groups independently selected from hydroxyl, amino, halogen, and cyano;
Rₐ is hydrogen, deuterium, 3-8-membered heterocycloalkyl, C3-C8 cycloalkyl, 6-10 membered aryl, 5-10 membered heteroaryl, C1-C6 alkyl, 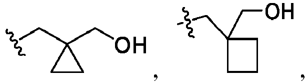 or 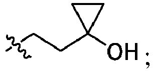 the alkyl is optionally substituted by one or more substituents selected from halogen, hydroxyl, C1-C6 alkoxy, halogenated C1-C6 alkoxy, S(O)₁₋₂(C1-C6 alkyl), S(O)₁₋₂(C3-C6 cycloalkyl), unsubstituted or mono- or polyhalogen-substituted C3-C6 cycloalkyl, unsubstituted or mono- or dimethyl substituted monocyclic saturated heterocycloalkyl having 4-6 ring atoms and containing heteroatoms or heterogroups selected from O, S, SO or SO₂; the cycloalkyl, heterocycloalkyl, aryl, heteroaryl are optionally substituted by one or more groups selected from hydroxyl, halogen, amino, cyano, C1-C6 alkyl, -O(C1-C6 alkyl), halogenated C1-C6 alkyl, hydroxyl C1-C6 alkyl, amino C1-C6 alkyl.

More preferably, in some embodiments of the present invention, the PTM has the following structure 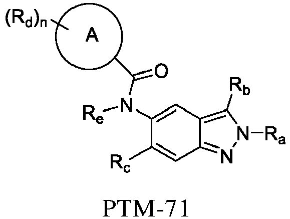 wherein, in PTM-71:
ring A is phenyl or pyridyl;
R_{d} is each independently hydrogen, deuterium, halogen, cyano, C1-C6 alkyl, or C3-C6 cycloalkyl, the alkyl and the cycloalkyl are optionally substituted by one or more groups selected from halogen, hydroxyl or amino;
n is 1 or 2;
Rₑ is hydrogen;
R_{c} is hydrogen, deuterium,-O(C1-C6 alkyl),-N(C1-C6 alkyl)₁₋₂, C1-C6 alkyl,-O(C3-C6 cycloalkyl),-N(C3-C6 cycloalkyl), -O (3-6-membered heterocycloalkyl),-N (3-6-membered heterocycloalkyl), the alkyl, cycloalkyl and heterocycloalkyl are optionally substituted by one or more groups independently selected from hydroxyl, amino, halogen, cyano or -O-(C1-C6 alkyl);
R_{b} is hydrogen or C1-C6 alkyl, and the alkyl is optionally substituted by one or more groups independently selected from hydroxyl, amino, halogen or cyano;
Ra is hydrogen, deuterium, 3-8-membered heterocycloalkyl, C3-C8 cycloalkyl, C1-C6 alkyl, 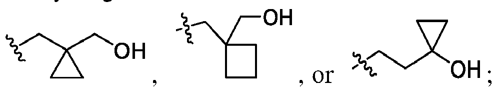 the alkyl is optionally substituted by one or more substituents selected from halogen, hydroxyl, C1-C6 alkoxy, halogenated C1-C6 alkoxy, S(O)1-2(C1-C6 alkyl), S(O)1-2(C3-C6 cycloalkyl), unsubstituted or mono- or polyhalogen-substituted C3-C6 cycloalkyl, unsubstituted or mono- or di-methyl substituted monocyclic saturated heterocycloalkyl having 4-6 ring atoms and containing heteroatoms or heterogroups selected from O, S, SO or SO2; the cycloalkyl and the heterocycloalkyl, aryl and heteroaryl are optionally substituted by one or more groups selected from hydroxyl, halogen, amino, cyano, C1-C6 alkyl, -O(C1-C6 alkyl), halogenated C1-C6 alkyl, hydroxyl C1-C6 alkyl, or amino C1-C6 alkyl.

In some embodiments of the present invention, in PTM-71, ring A is pyridyl.

In some embodiments of the present invention, in PTM-71, R_{d} is each independently hydrogen, deuterium, halogen or C1-C6 alkyl, optionally substituted by one or more groups selected from halogen or hydroxyl; preferably R_{d} is hydrogen, deuterium, F, methyl, difluoromethyl, trifluoromethyl or 2-hydroxypropyl.

In some embodiments of the present invention, in PTM-71, Rₑ is hydrogen.

In some embodiments of the present invention, in PTM-71, R_{c} is C1-C6 alkyl, the alkyl is optionally substituted by one or more groups independently selected from hydroxyl and halogen; preferably R_{c} is difluoromethyl or 2-hydroxypropyl.

In some embodiments of the present invention, in PTM-71, R_{c} is -O(C1-C6 alkyl), the alkyl is optionally substituted by one or more groups independently selected from hydroxyl, halogen or -OCH₃; preferably R_{c} is methoxy, ethoxy, isopropoxy, -OCH₂CH₂OCH₃, difluoromethoxy or trifluoromethoxy.

In some embodiments of the present invention, in PTM-71, R_{c} is -O-(3-6-membered heterocycloalkyl containing 1 or 2 heteroatoms selected from N or O); preferably

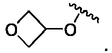

In some embodiments of the present invention, in PTM-71, R_{b} is hydrogen or methyl.

In some embodiments of the present invention, in PTM-71, Rₐ is hydrogen.

In some embodiments of the present invention, in PTM-71, Rₐ is 3-8-membered heterocycloalkyl containing 1 or 2 heteroatoms selected from N, O or S, the heterocycloalkyl is optionally substituted by one or more groups selected from hydroxyl, halogen, amino, cyano, C1-C6 alkyl,-O(Cl-C6 alkyl), halogenated C1-C6 alkyl, hydroxyl C1-C6 alkyl, amino C1-C6 alkyl; preferably Rₐ is 3-8-membered heterocycloalkyl containing 1 or 2 heteroatoms selected from N or O, the heterocycloalkyl is optionally substituted by one or more groups selected from hydroxyl, halogen, amino, cyano, C1-C6 alkyl,-O(C1-C6 alkyl), halogenated C1-C6 alkyl, hydroxyl C1-C6 alkyl, amino C1-C6 alkyl; more preferably Rₐ is piperidinyl or piperazinyl, the piperidinyl and piperazinyl are optionally substituted by one or more groups selected from hydroxyl, halogen, amino, cyano, C1-C6 alkyl,-O(C1-C6 alkyl), halogenated C1-C6 alkyl, hydroxyl C1-C6 alkyl, amino C1-C6 alkyl; most preferably is piperidinyl optionally substituted by one or more groups selected from hydroxyl, amino, methyl, methoxy, hydroxymethyl, trifluoromethyl.

In some embodiments of the present invention, in PTM-71, Rₐ is cyclohexane, and the cyclohexane is optionally substituted by one or more groups selected from hydroxyl, halogen, amino, cyano, C1-C6 alkyl,-O(Cl-C6 alkyl), halogenated C1-C6 alkyl, hydroxyl C1-C6 alkyl, amino C1-C6 alkyl, preferably optionally substituted by one or more groups selected from hydroxyl, amino, methyl, methoxy, hydroxymethyl, trifluoromethyl.

In some embodiments of the present invention, in PTM-71, Rₐ is hydrogen or C1-C6 alkyl, the C1-C6 alkyl is optionally substituted by one or more groups selected from F, hydroxyl, methoxy, fluoromethoxy,-S(O)₂CH₃,-S(O)₂-cyclopropyl, cyclopropyl, and oxetanyl.

In some embodiments of the present invention, PTM has the following structure: 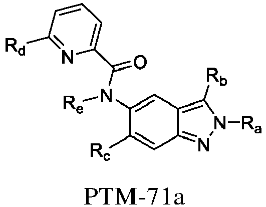 wherein, Rₐ, R_{b}, R_{c}, R_{d}, and Rₑ are as defined in the above PTM-71.

In some embodiments of the present invention, PTM has the following structure: 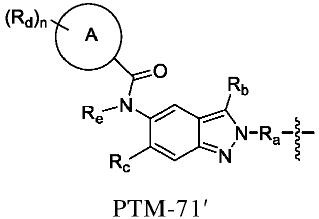 wherein, Rₐ, R_{b}, R_{c}, R_{d}, Rₑ, and n are as defined in the above PTM-71.

Preferably, in some embodiments of the present invention, PTM has the following structure: 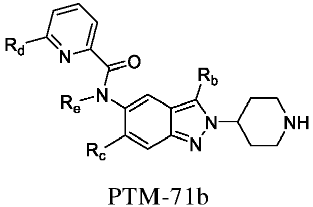 wherein, R_{b}, R_{c}, R_{d}, and Rₑ are as defined in the above PTM-71.

Preferably, in some embodiments of the present invention, PTM has the following structure: 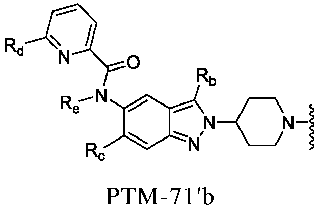 wherein, R_{b}, R_{c}, R_{d}, and Rₑ are as defined in the above PTM-71.

Preferably, in some embodiments of the present invention, PTM has the following structure: 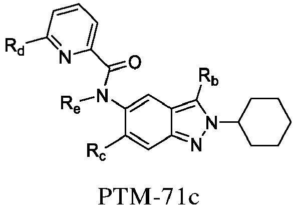 wherein, R_{b}, R_{c}, R_{d}, and Rₑ are as defined in the above PTM-71.

Preferably, in some embodiments of the present invention, PTM has the following structure: 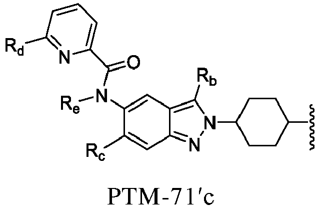 wherein, R_{b}, R_{c}, R_{d}, and Rₑ are as defined in the above PTM-71.

Preferably, in some embodiments of the present invention, the PTM is selected from 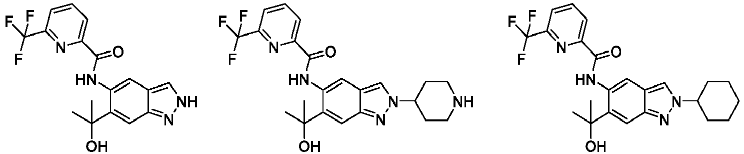 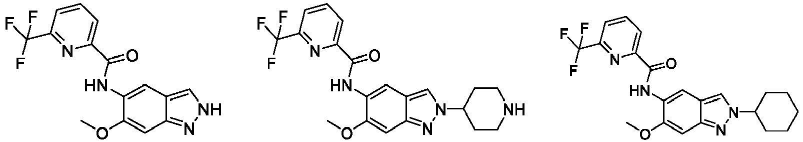 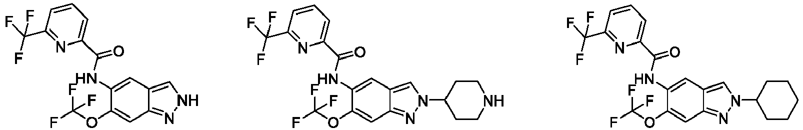 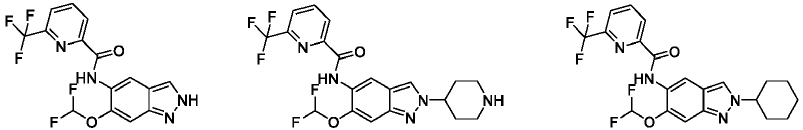 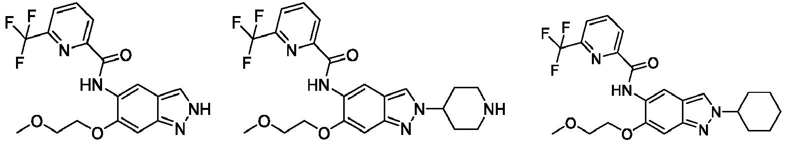 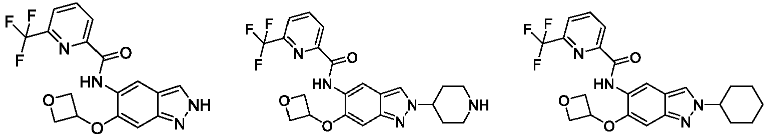 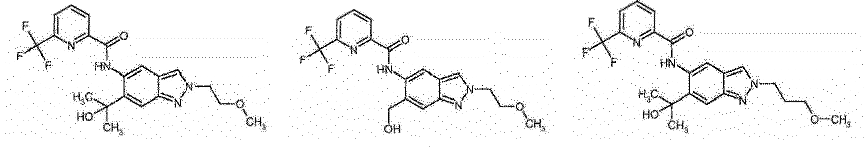 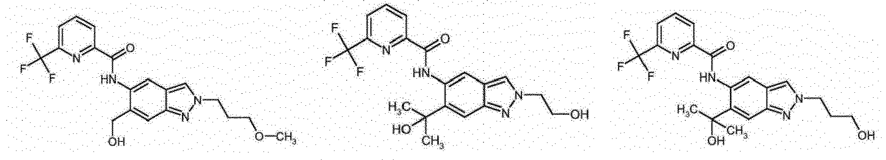 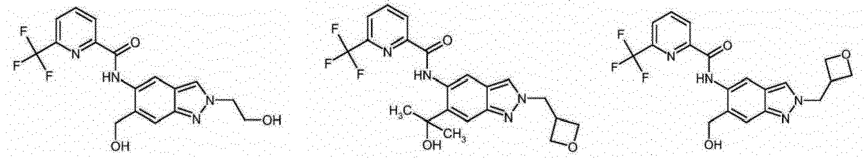 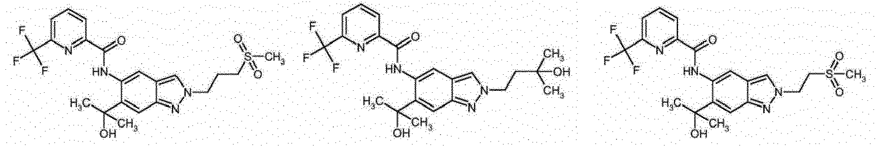 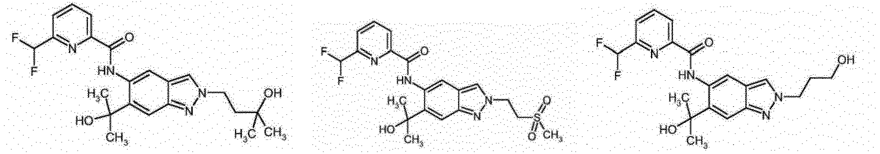 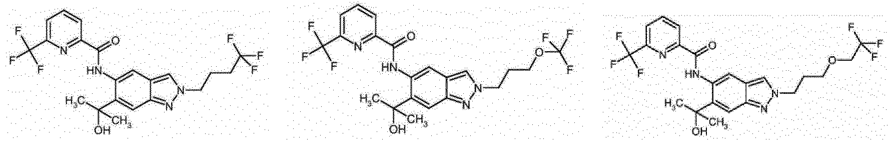 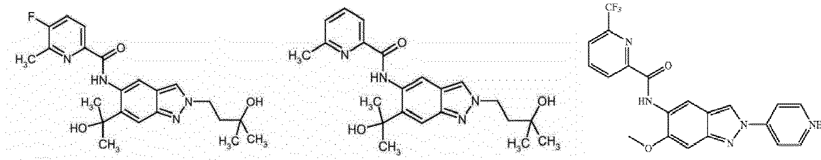 or

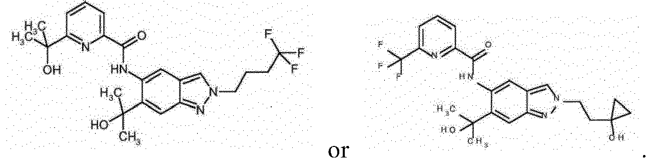

More preferably, in some embodiments of the present invention, the PTM is selected from

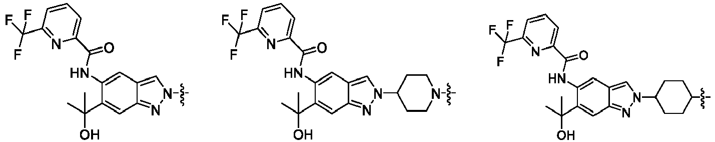

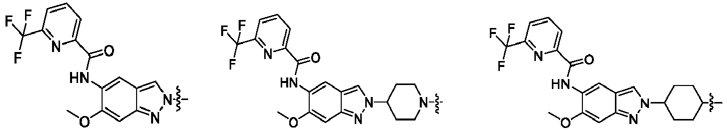

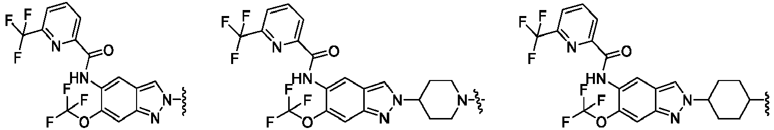

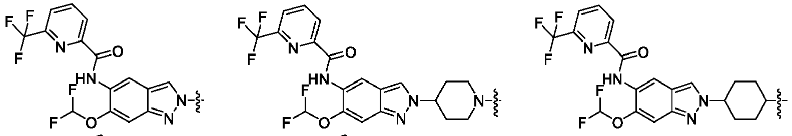

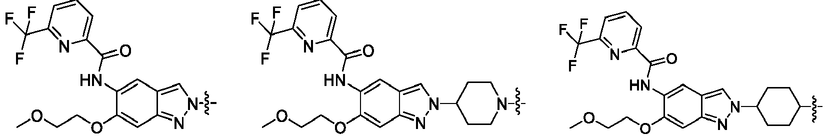

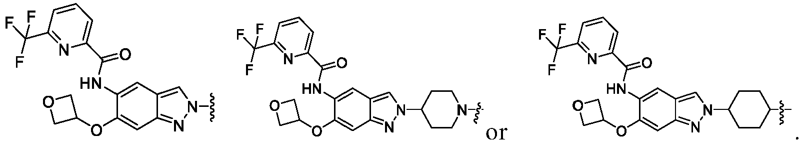

Preferably, in some embodiments of the present invention, the PTM is selected from

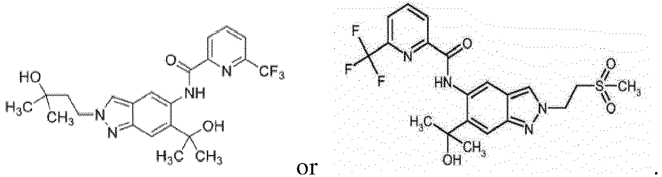

Preferably, in some embodiments of the present invention, the compound of formula I, and/or a stereoisomer, enantiomer, diastereomer, deuterate, metabolite, hydrate, solvate, prodrug thereof and/or pharmaceutically acceptable salt thereof is a compound of formula IC, and/or a stereoisomer, enantiomer, diastereomer, deuterate, metabolite, hydrate, solvate, prodrug thereof and/or pharmaceutically acceptable salt thereof, 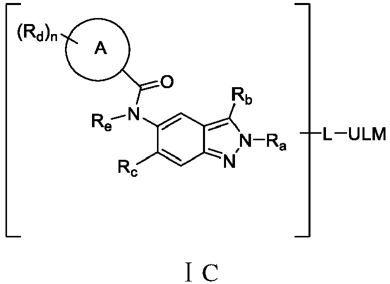 wherein, ring A, Rₐ, R_{b}, R_{c}, R_{d}, Rₑ, and n are as defined in PTM-71, and L, ULM are as defined herein.

Preferably, in some embodiments of the present invention, the compound of formula I, and/or a stereoisomer, enantiomer, diastereomer, deuterate, metabolite, hydrate, solvate, prodrug thereof and/or a pharmaceutically acceptable salt thereof is a compound of formula IC-1, and/or a stereoisomer, enantiomer, diastereomer, deuterate, metabolite, hydrate, solvate, prodrug thereof and/or a pharmaceutically acceptable salt thereof, 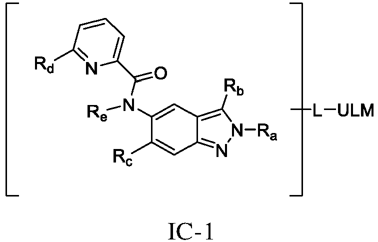 wherein, Rₐ, R_{b}, R_{c}, R_{d}, and Rₑ are as defined in PTM-71, and L, ULM are as defined herein.

Preferably, in some embodiments of the present invention, the compound of formula I, and/or a stereoisomer, enantiomer, diastereomer, deuterate, metabolite, hydrate, solvate, prodrug thereof and/or a pharmaceutically acceptable salt thereof is a compound of formula IC-2, and/or a stereoisomer, enantiomer, diastereomer, deuterate, metabolite, hydrate, solvate, prodrug thereof and/or a pharmaceutically acceptable salt thereof, 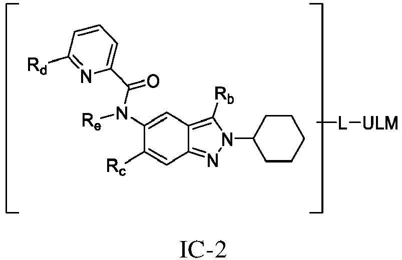 wherein, R_{b}, R_{c}, R_{d}, Rₑ are as defined in PTM-71, and L, ULM are as defined herein.

Preferably, in some embodiments of the present invention, the compound of formula I, and/or a stereoisomer, enantiomer, diastereomer, deuterate, metabolite, hydrate, solvate, prodrug thereof and/or a pharmaceutically acceptable salt thereof is a compound of formula IC-3, and/or a stereoisomer, enantiomer, diastereomer, deuterate, metabolite, hydrate, solvate, prodrug thereof and/or a pharmaceutically acceptable salt thereof, 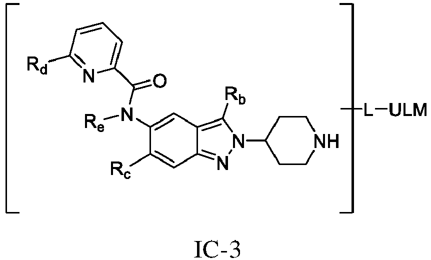 wherein, R_{b}, R_{c}, R_{d}, Rₑ are as defined in PTM-71, and L, ULM are as defined herein.

In some embodiments of the present invention, L is bond.

In some embodiments of the present invention, L is -(CH₂)ⱼ-, one or more methylenes in -(CH₂)ⱼ- are optionally replaced by groups selected from -NR^{3 '}-,-O-,-S-,-S(O)-,-S(O)NR^{3'}-,-NR^{3 '}S(O)-,-S(O)₂-,-S(O)₂NR^{3'}-,-NR^{3 '}S(O)₂-,-NR^{4'}S(O)₂NR^{3 '}-,-CR^{1'}R^{2'}-,-C(O)-,-C(O)O-,-OC(O)-,-NR^{3'}C(O)O-,-OC(O)NR^{3 '}-,-C(O)NR^{3 '}-,-NR^{3 '}C(O)-,-NR^{4'}C(O)NR^{3 '}-,-P(O)-,-P(O)O-,-OP(O)-,-OP(O)O-, vinylidene, ethynylene, C3-C12 cycloalkylene, 3-12 membered heterocycloalkylene containing 1 or more heteroatoms selected from N, O or S, 6-10 membered arylene or 5-10 membered heteroarylene, wherein the vinylidene, cycloalkylene, heterocycloalkylene, arylene, heteroarylene are each independently optionally substituted by one or more substituents selected from halogen,-OR^{3'},-NR^{3'}R^{4'}, oxo, nitro, cyano, C1-C6 alkyl,-S(C1-C6 alkyl), C3-C10 cycloalkyl, 3-10 membered heterocycloalkyl, 6-10 membered aryl, 5-10 membered heteroaryl,-C(O)R^{1'},-C(O)OR^{3'},-OC(O)R^{1'},-C(O)NR^{3'},-NR^{3'}C(O)R^{1'},-S(O)R^{1 '},-S(O)NR^{3'},-S(O)₂R^{1 '},-S(O)₂NR^{3'},-NR^{3 '}S(O)₂R^{1'},-NR^{4 '}S(O)₂NR^{3'},-OC(O)NR^{3 '},and - NR^{4'}C(O)NR^{3 '}, and the alkyl, cycloalkyl, heterocycloalkyl, aryl, and heteroaryl are each independently optionally substituted by one or more substituents selected from halogen, - OH,-NR³R^{4'}, oxo, nitro, cyano, C1-C6 alkyl, C3-C10 cycloalkyl, 3-10 membered heterocycloalkyl, 6-10 membered aryl, 5-10 membered heteroaryl; R^{1'} and R^{2'} are each independently halogen,-OH,-NR^{3 '}R^{4'}, C1-C6 alkyl, chlorinated C1-C6 alkyl, hydroxyl C1-C6 alkyl,-O(C1-C6 alkyl),-NH(C1-C6 alkyl),-NH(C1-C6 alkyl)₂, C3-C10 cycloalkyl,-O(C3-C10 cycloalkyl),-NH(C3-C10 cycloalkyl), 3-10-membered heterocycloalkyl,-O(3-10-membered heterocycloalkyl),-NH(3-10-membered heterocycloalkyl), 6-10-membered aryl,-O (6-10 membered aryl),-NH(6-10 membered aryl), 5-10 membered heteroaryl,-O(5-10 membered heteroaryl),-NH(5-10 membered heteroaryl), R^{3 '}, R^{4'} are each independently hydrogen, deuterium, C1-C6 alkyl, C3-C10 cycloalkyl, 3-10 membered heterocycloalkyl, 6-10 membered aryl, 5-10 membered heteroaryl; j is 1, 2, 3, 4, 5, 6, 7, 8, 9, 10, 11, 12, 13, 14, 15, 16, 17, 18, 19, 20, 21, 22, 23, 24 or 25.

Preferably, in some embodiments of the present invention, L is -(CH₂)ⱼ-, one or more methylenes in -(CH₂)ⱼ- are optionally replaced by groups selected from -NR^{3 '}-,-O-,-CR^{1'}R^{2 '}-,-C(O)-,-S(O)-,-S(O)₂-,-C(O)O-,-OC(O)-,-C(O)NR^{3'}-,-NR^{3 '}C(O)-,-S(O)₂NR^{3'}-,-NR^{3 '}S(O)₂-, vinylidene, ethynylene, phenyl, 8-10 membered bicycloarylene, 3-7 membered saturated or partially unsaturated cycloalkylene, 4-11 membered saturated or partially unsaturated spiro cycloalkylene, 4-11-membered saturated or partially unsaturated fused cycloalkylene, 8-10-membered bicyclic saturated or partially unsaturated cycloalkylene, 4-7-membered saturated or partially unsaturated heterocycloalkylene containing 1-2 heteroatoms independently selected from nitrogen, oxygen or sulfur, 4-11-membered saturated or partially unsaturated spiro heterocycloalkylene containing 1-2 heteroatoms independently selected from nitrogen, oxygen or sulfur, 4-11-membered saturated or partially unsaturated fused heterocycloalkylene containing 1-2 heteroatoms independently selected from nitrogen, oxygen or sulfur, 8-10-membered bicyclic saturated or partially unsaturated heterocycloalkylene containing 1-2 heteroatoms independently selected from nitrogen, oxygen or sulfur, 5-6-membered heteroarylene containing 1-4 heteroatoms independently selected from nitrogen, oxygen or sulfur, or 8-10 membered bicyclic heteroaryl containing 1-5 heteroatoms selected from nitrogen, oxygen or sulfur, wherein the vinylidene, ethynylene, cycloalkylene, heterocycloalkylene, phenyl, spiro-heterocycloalkylene, fused heterocycloalkylene, spiro-cycloalkylene, fused cycloalkylene, and heteroarylene are each independently optionally substituted by one or more substituents selected from halogen, oxo,-NR^{3'}R⁴,-OR^{3 '}, nitro,-CN, C1-C6 alkyl, C3-C10 cycloalkyl, and 3-10 membered heterocycloalkyl, the alkyl, cycloalkyl, heterocycloalkyl is optionally substituted by one or more substituents selected from halogen,-OH,-NH₂,-CN, C1-C4 alkyl, C3-C6 cycloalkyl, R^{1'} and R^{2'} are each independently halogen,-OH,-NH₂, C1-C4 alkyl, C1-C4 chloroalkyl, C1-C4 hydroxyalkyl,-O(C1-C4 alkyl),-NH(C1-C4 alkyl),-NH(C1-C4 alkyl), C3-C6 cycloalkyl,-O(C3-C6 cycloalkyl),-NH(C3-C6 cycloalkyl), 3-6-membered heterocycloalkyl,-O (3-6-membered heterocycloalkyl),-NH(C3-C6 cycloalkyl); R^{3'} and R^{4'} are each independently hydrogen, deuterium, C1-C4 alkyl, C3-C6 cycloalkyl, 3-6-membered heterocycloalkyl, and j is 2, 3, 4, 5, 6, 7, 8, 9, 10, 11 or 12.

Preferably, in some embodiments of the present invention, L is -(CH₂)ⱼ-, one or more methylenes in -(CH₂)ⱼ- are optionally replaced by groups selected from -NR³ '-,-O-,-CR^{1'}R^{2 '}-,-C(O)-,-S(O)-,-S(O)₂-,-C(O)O-,-OC(O)-,-C(O)NR^{3'} -,-NR^{3 '}C(O)-,-S(O)₂NR^{3'}-,-NR^{3 '}S(O)₂-, vinylidene, ethynylene, phenyl, 8-10 membered bicyclic arylene, 3-7 membered saturated or partially unsaturated monocyclic cycloalkylene, 4-11 membered saturated or partially unsaturated spiro cycloalkylene, 4-11-membered saturated or partially unsaturated fused cycloalkylene, 8-10-membered bicyclic saturated or partially unsaturated cycloalkylene, 4-7-membered saturated or partially unsaturated heterocycloalkylene containing 1-2 heteroatoms independently selected from nitrogen or oxygen, 4-11-membered saturated or partially unsaturated spiro heterocycloalkylene containing 1-2 heteroatoms independently selected from nitrogen or oxygen, 4-11-membered saturated or partially unsaturated fused heterocycloalkylene containing 1-2 heteroatoms independently selected from nitrogen or oxygen, 8-10-membered bicyclic saturated or partially unsaturated heterocycloalkylene containing 1-2 heteroatoms independently selected from nitrogen or oxygen, 5-6-membered heteroarylene containing 1-4 heteroatoms independently selected from nitrogen or oxygen, or 8-10 membered bicyclic heteroaryl containing 1-5 heteroatoms selected from nitrogen or oxygen, wherein the vinylidene, ethynylene, cycloalkylene, heterocycloalkylene, phenyl, spiro-heterocycloalkylene, fused heterocycloalkylene, fused cycloalkylene, spiro-cycloalkylene, and heteroarylene are each independently optionally substituted by one or more substituents selected from halogen, oxo,-NR^{3'}R^{4'},-OR^{3 '}, nitro,-CN, C1-C6 alkyl, C3-6 cycloalkyl, 3-6-membered heterocycloalkyl, the alkyl, cycloalkyl, or heterocycloalkyl is optionally substituted by one or more substituents selected from halogen,-OH,-NH2,-CN, C1-C4 alkyl, C3-C6 cycloalkyl, R^{1'} and R^{2'} are each independently halogen,-OH,-NH₂, C1-C4 alkyl, C1-C4 chloroalkyl, C1-C4 hydroxyalkyl,-O(C1-C4 alkyl),-NH(C1-C4 alkyl),-NH(C1-C4 alkyl), C3-C6 cycloalkyl,-O(C3-C6 cycloalkyl),-NH(C3-C6 cycloalkyl), 3-6-membered heterocycloalkyl,-O (3-6-membered heterocycloalkyl),-NH(C3-C6 cycloalkyl); R^{3'} and R^{4'} are each independently hydrogen, deuterium, C1-C4 alkyl, C3-C6 cycloalkyl, 3-6-membered heterocycloalkyl, and j is 2, 3, 4, 5, 6, 7, 8, 9 or 10.

Preferably, in some embodiments of the present invention, L is -(CH2)j, 1, 2, 3 or 4 methylenes in -(CH2)j- are optionally replaced by a group selected from -NH-, -NCH3-, - NCH2CH3-, -O-, -C(CH3)2-, -CHF-, -CHCF3-, -C(O)-, -C(O)O-, -OC(O)-, -C(O)NH-, - C(O)NCH3-, -NHC(O)-, -NCH3C(O)-, vinylidene, ethynylene, cyclopropylene, cyclobutylene, cyclopentylene, cyclohexylidene, oxiranylene, oxetanylene, oxolanylene, oxanilene, azridinylene, azetidinylene, azacyclopentylene, piperidinylidene, piperazinylidene, morpholinylidene, perhomomorpholinylidene, phenylene, pyrrolylidene, thienylidene, furanylidene, imidazolylidene, pyrazolylidene, triazolylidene, tetrazolylidene, oxazolylidene, isoxazolylidene, thiazolylidene, isothiazolylidene, pyridylidene, pyrimidinylidene, pyridazinylidene, pyrazinylidene, 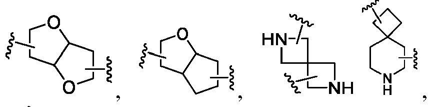 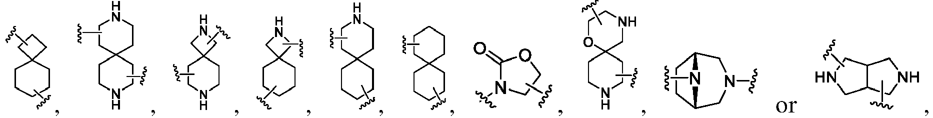 and the replacing group is optionally substituted by one or more substituents selected from halogen, oxo, -NR3 'R4', -OR3 ', or C1-C4 alkyl, and the alkyl is optionally substituted by one or more substituents selected from halogen, -OH, or -NH₂; R3' and R4' are each independently hydrogen, deuterium, or C1-C4 alkyl, and j is 2, 3, 4, 5, 6, 7 or 8.

Preferably, in some embodiments of the present invention, L is -(CH2)j, 1, 2 or 3 methylenes in -(CH2)j- are optionally replaced by a group selected from -NH-, -NCH3-, - NCH2CH3-, -O-, -C(CH3)2-, -CHF-, -CHCF3-, -C(O)-, -C(O)O-, -OC(O)-, -C(O)NH-, - C(O)NCH3-, -NHC(O)-, -NCH3C(O)-, cyclopropylene, cyclobutylene, cyclopentylene, cyclohexylidene, oxiranylene, oxetanylene, oxolanylene, oxanilene, azridinylene, azetidinylene, azacyclopentylene, piperidinylidene, piperazinylidene, morpholinylidene, perhomomorpholinylidene, 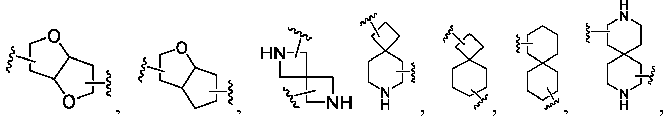 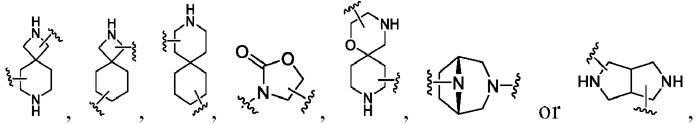 and the replacing group is optionally substituted by one or more substituents selected from F, Cl, oxo, -NR3 'R4', -OR3 ', or C1-C4 alkyl, the alkyl is optionally substituted by one or more substituents selected from halogen, -OH, or -NH2, R^{3'} and R^{4'} are each independently hydrogen, deuterium, methyl, ethyl, propyl, and j is 2, 3, 4, 5, 6, 7 or 8.

Preferably, in some embodiments of the present invention, L is -(CH2)j-, 1, 2 or 3 methylenes in -(CH2)j- are optionally replaced by a group selected from -O-, -NH-, -NCH3-, -NCH2CH3-, -C(O)-, -C(O)NH-, -NHC(O)-, -NCH3C(O)-, -C(O)NCH3-, azridinylene, azetidinylene, azacyclopentylene, piperidinylidene, piperazinylidene, 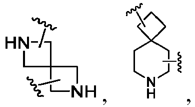 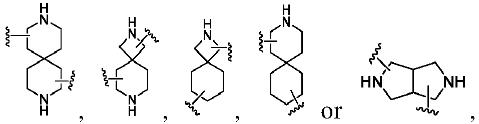 j is 2, 3, 4, 5, 6, 7, or 8.

Preferably, in some embodiments of the present invention, L is -(CH₂)ⱼ₋₁-C(O)-, the methylene in -(CH₂)ⱼ₋₁-C(O)- is as defined in above L, optionally substituted by one or more groups, the j is as defined in above L.

Preferably, in some embodiments of the present invention, L is selected from

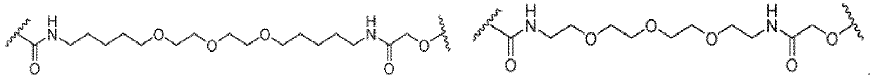

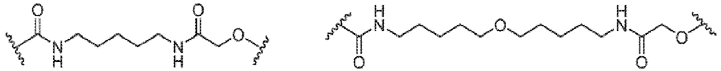

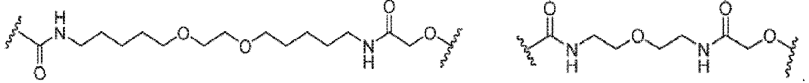

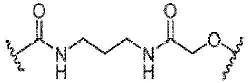

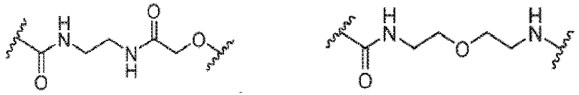

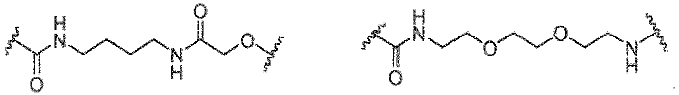

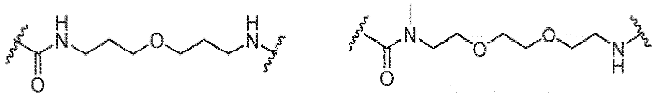

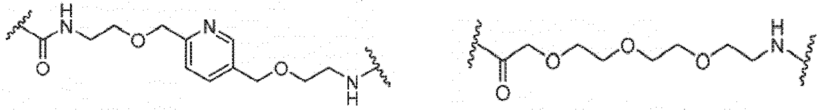

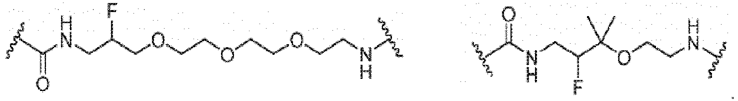

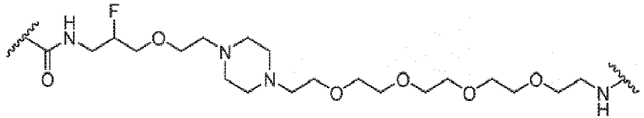

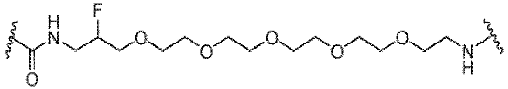

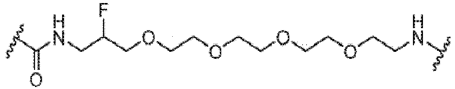

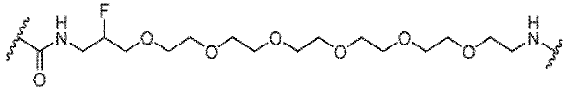

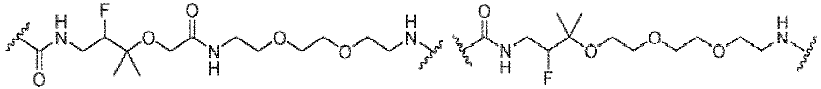

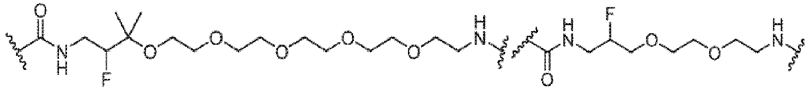

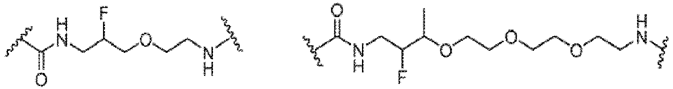

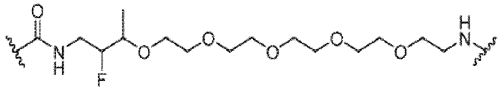

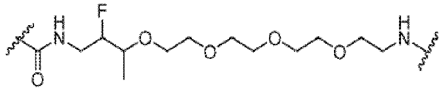

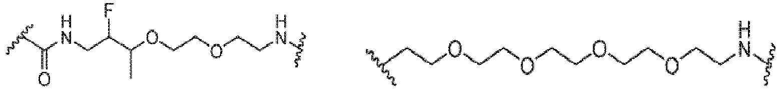

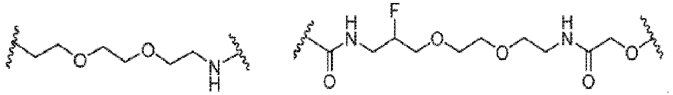

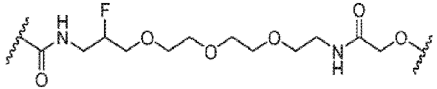

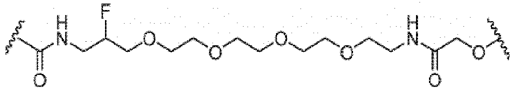

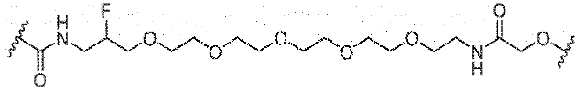

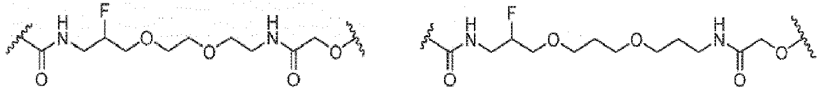

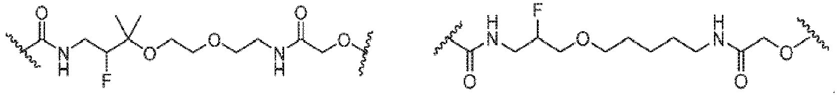

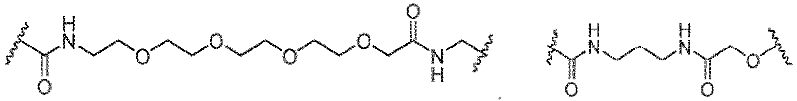

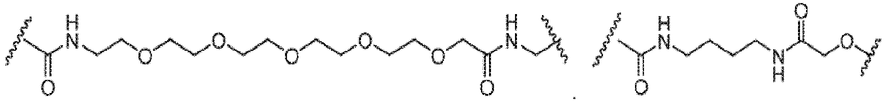

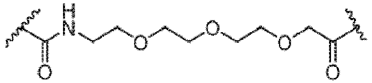

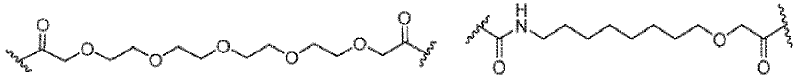

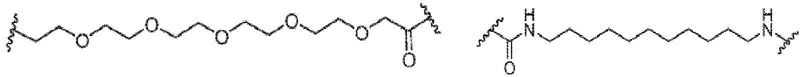

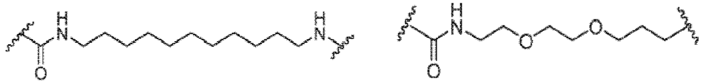

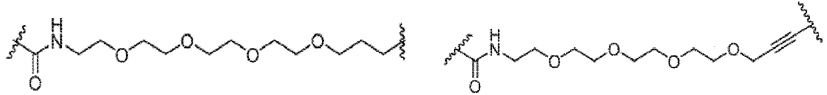

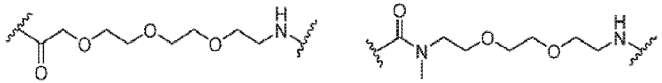

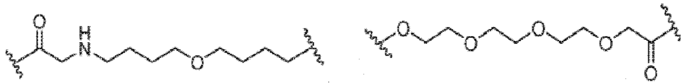

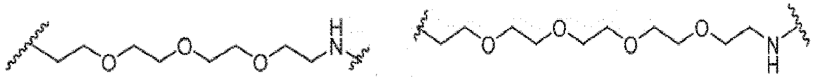

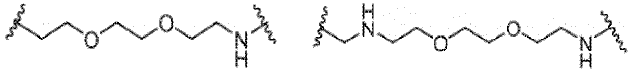

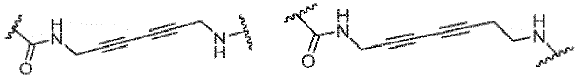

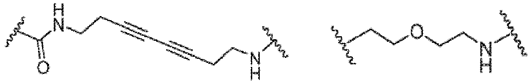

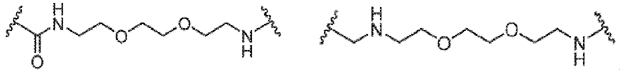

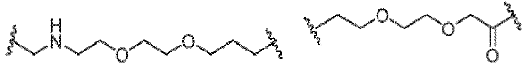

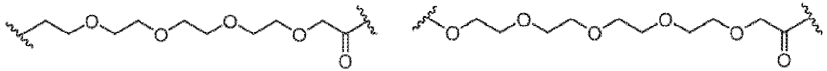

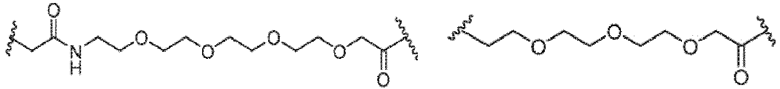

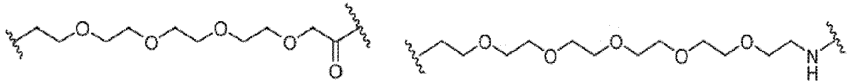

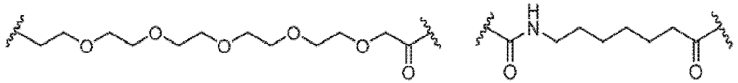

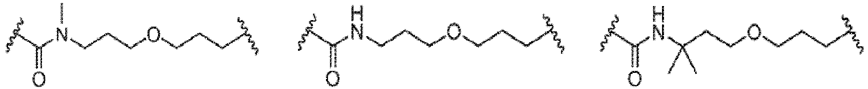

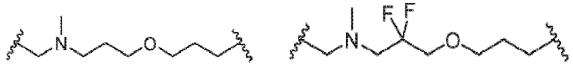

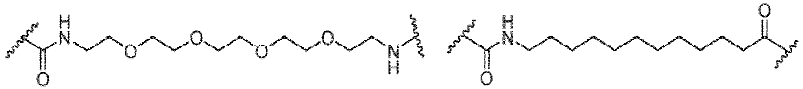

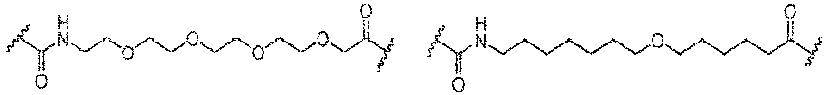

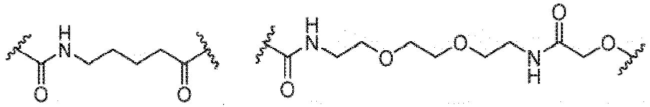

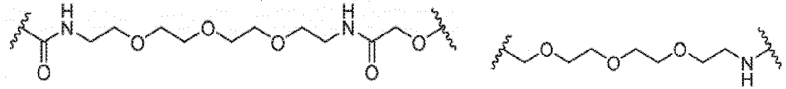

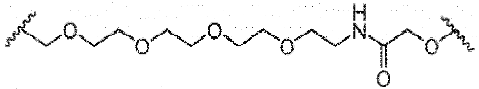

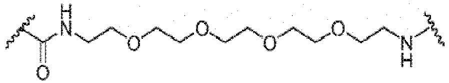

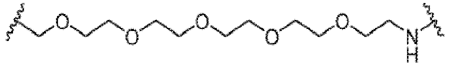

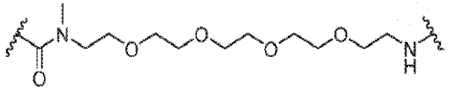

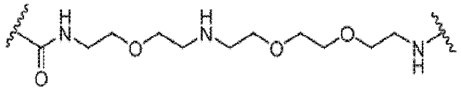

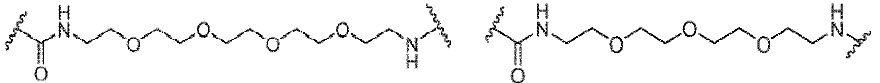

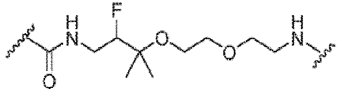

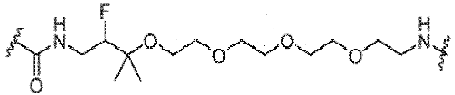

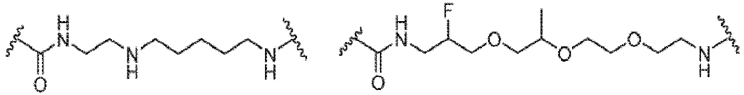

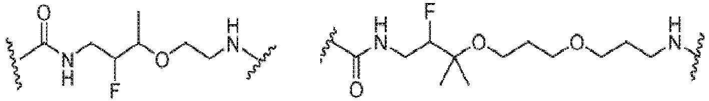

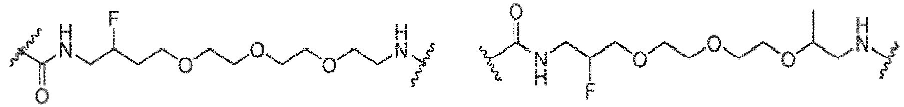

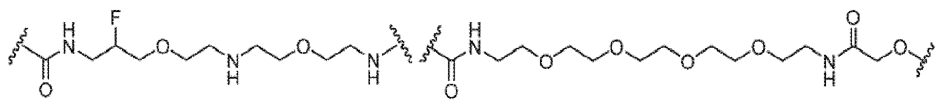

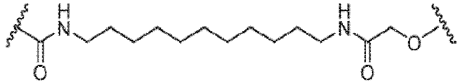

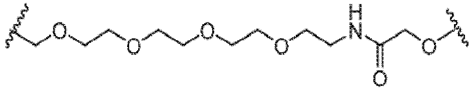

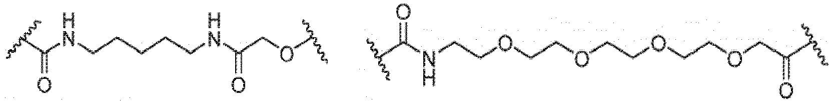

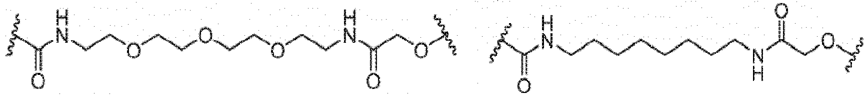

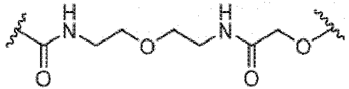

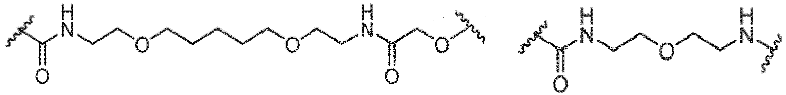

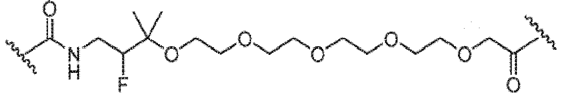

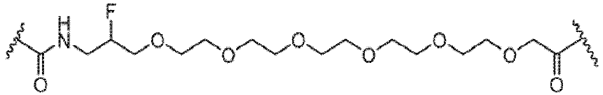

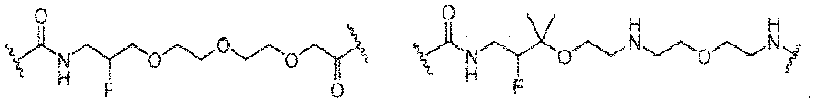

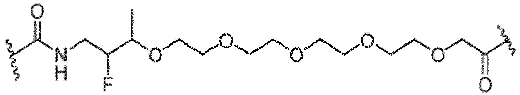

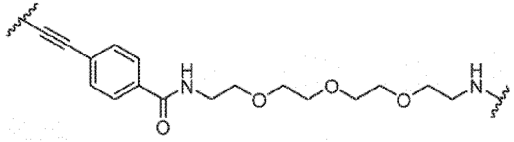

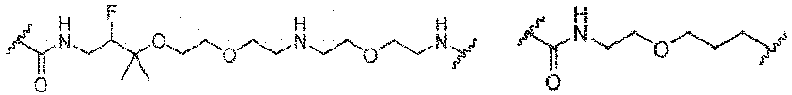

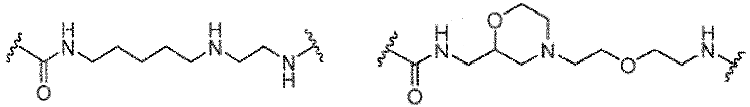

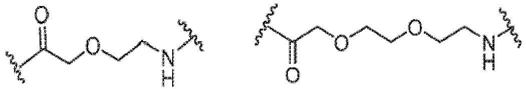

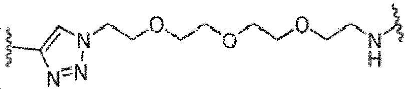

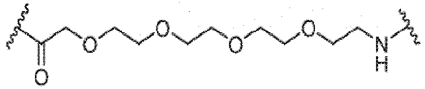

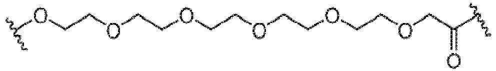

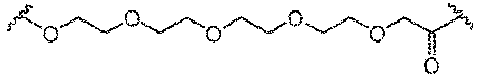

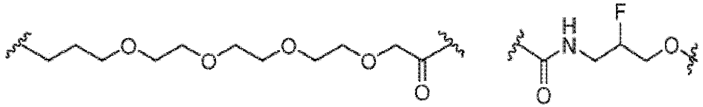

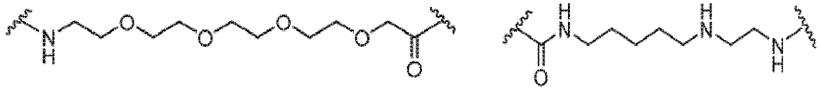

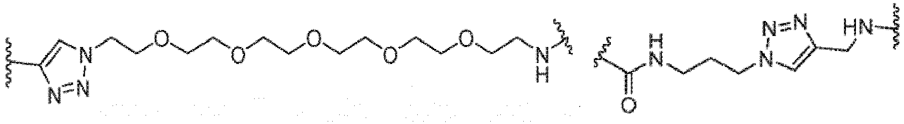

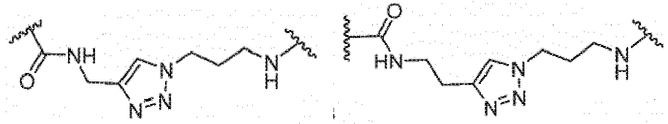

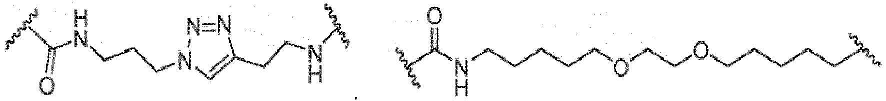

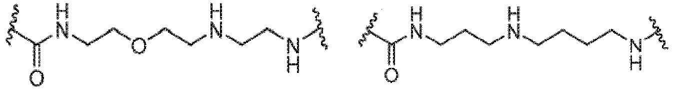

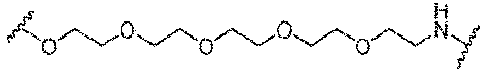

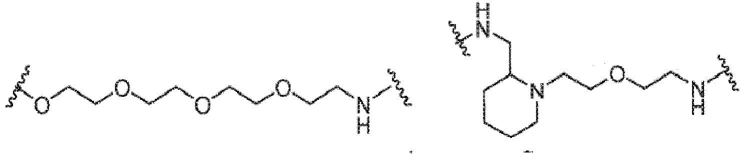

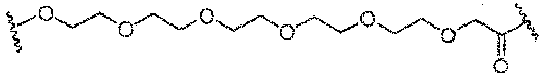

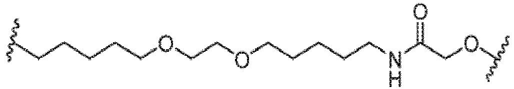

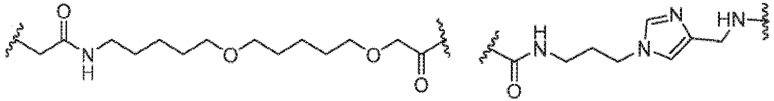

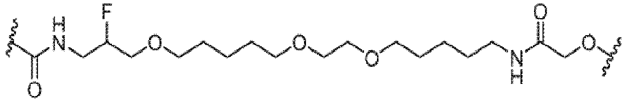

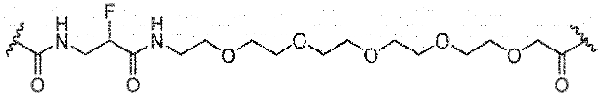

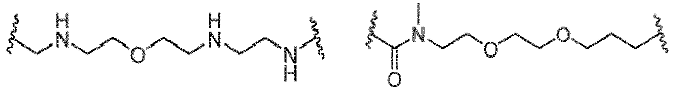

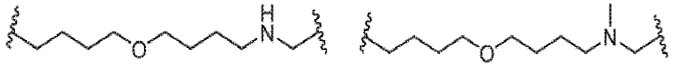

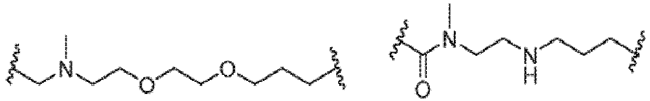

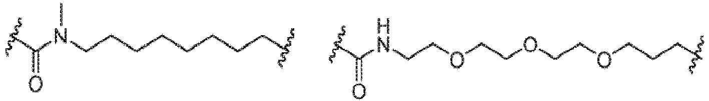

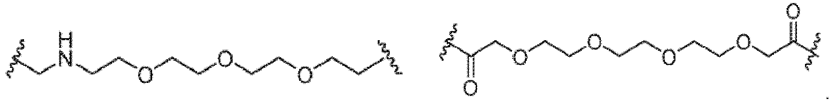

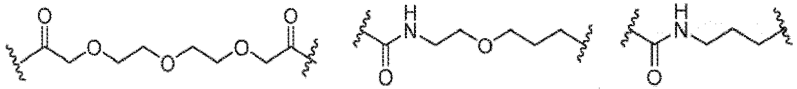

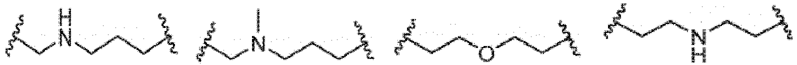

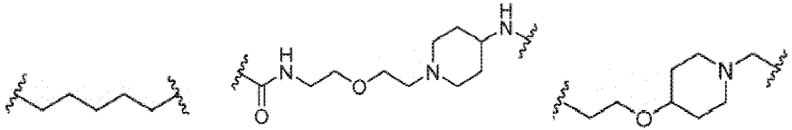

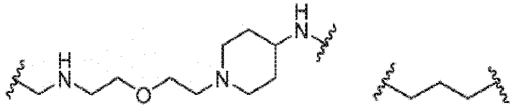

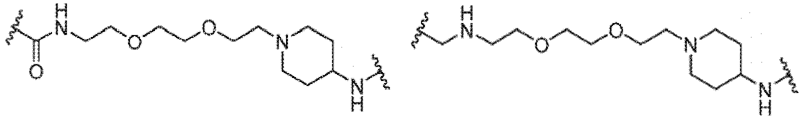

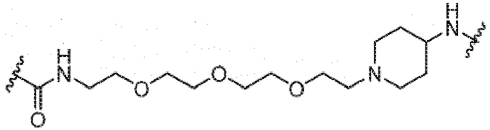

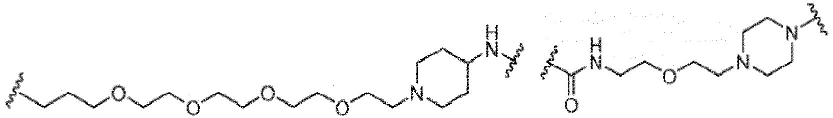

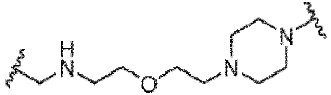

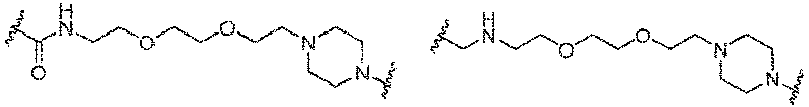

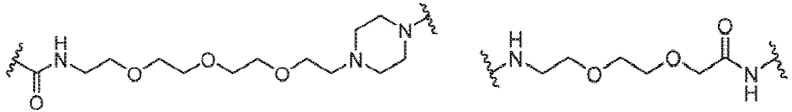

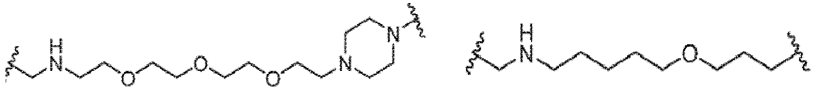

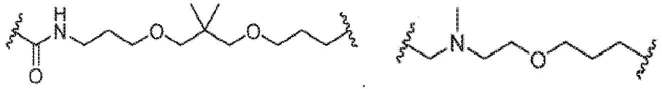

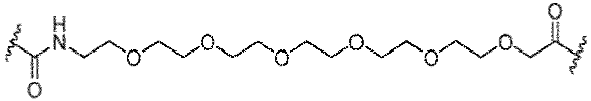

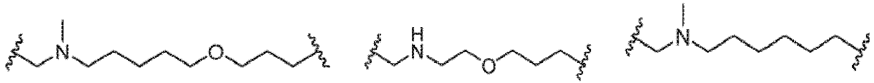

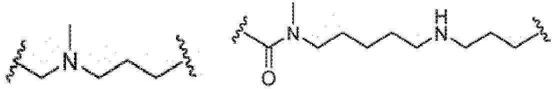

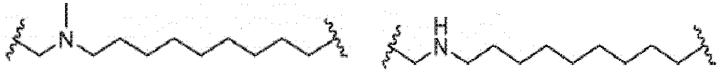

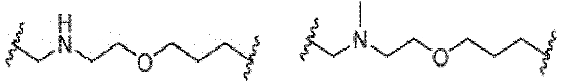

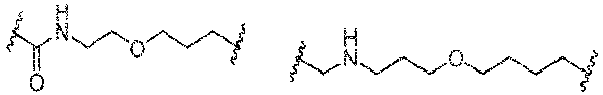

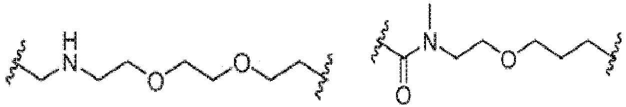

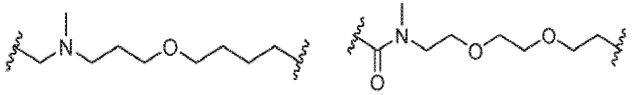

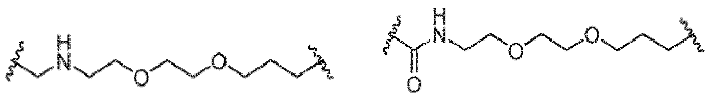

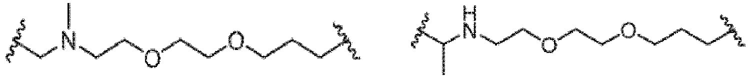

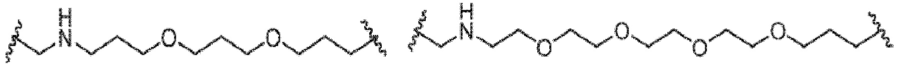

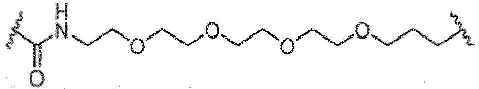

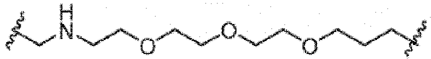

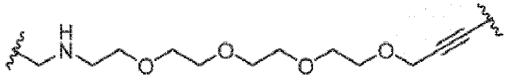

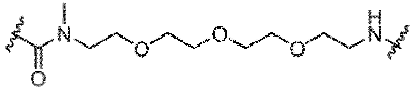

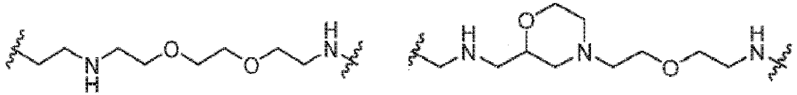

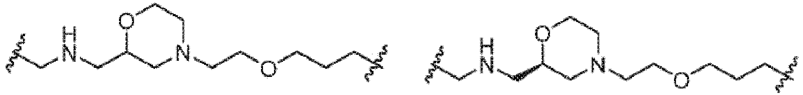

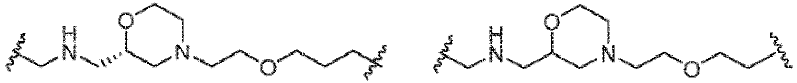

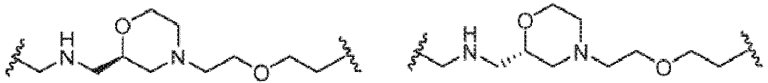

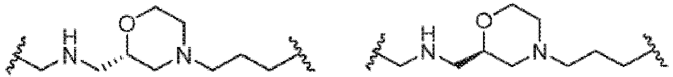

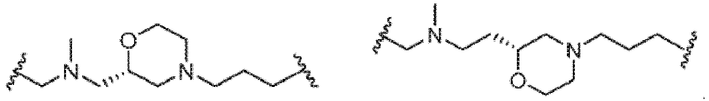

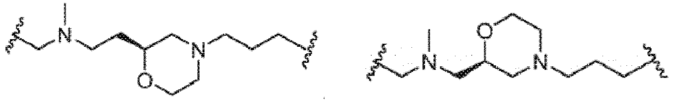

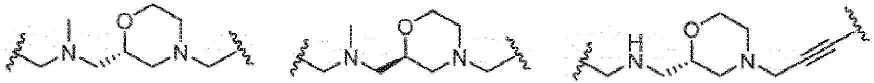

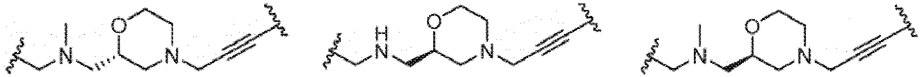

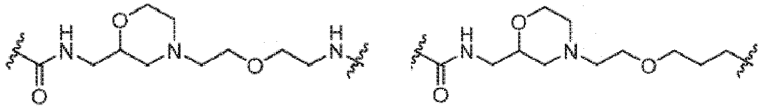

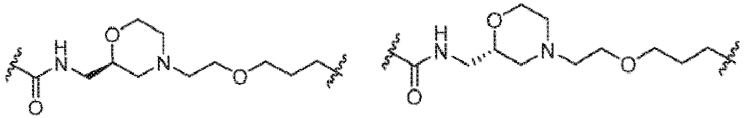

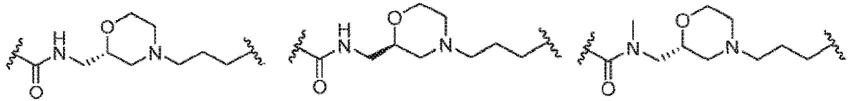

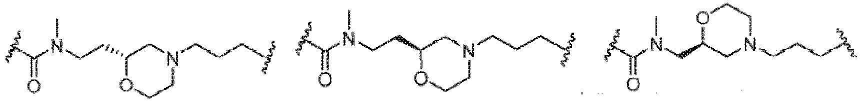

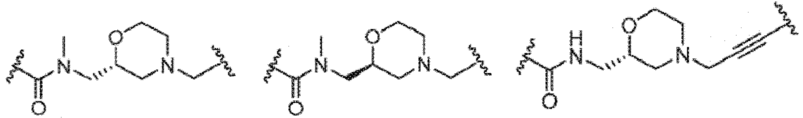

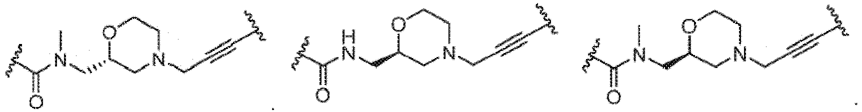

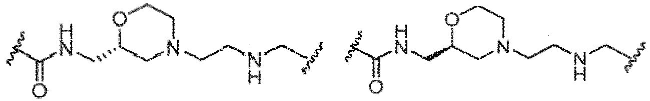

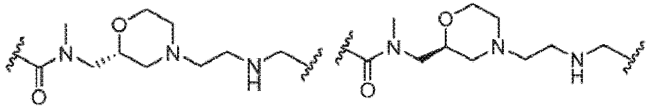

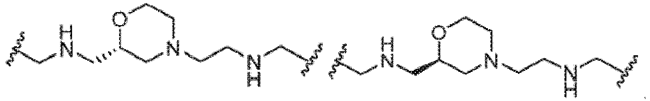

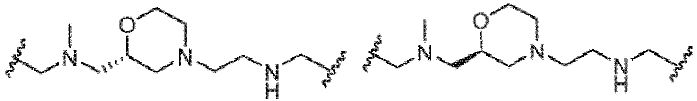

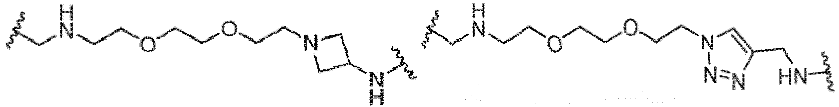

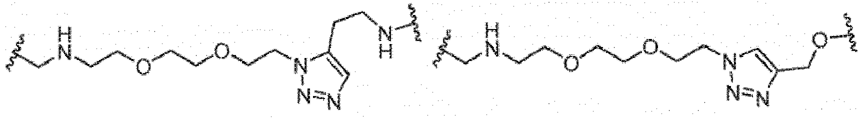

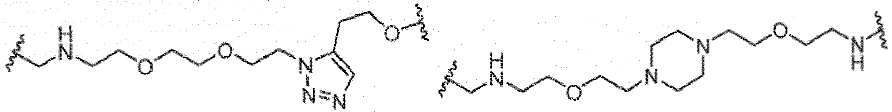

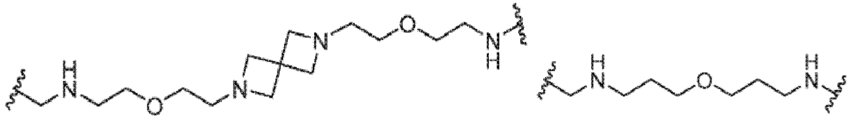

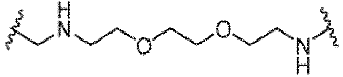

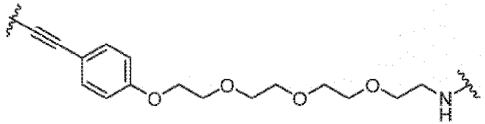

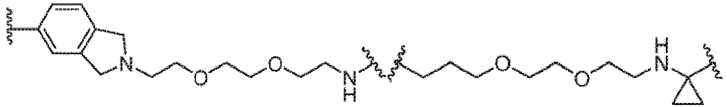

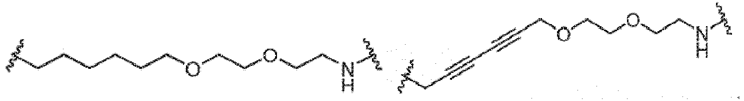

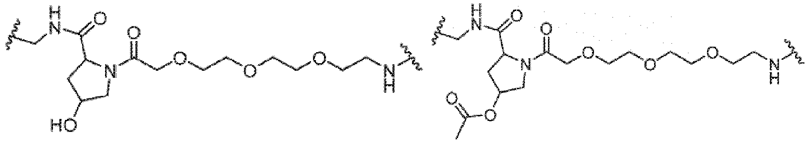

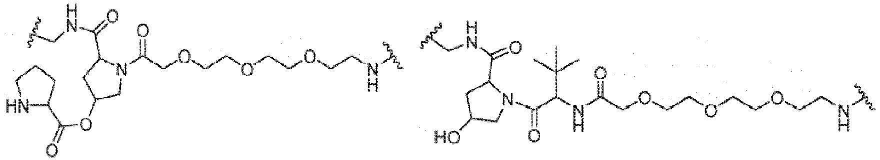

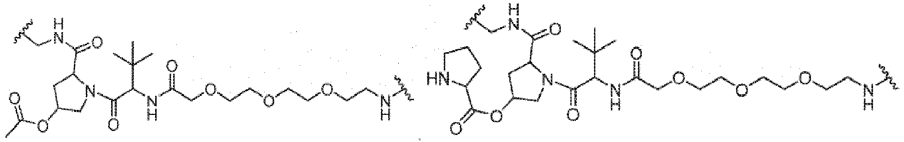

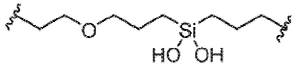

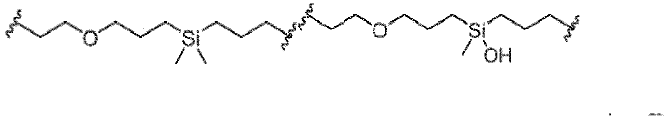

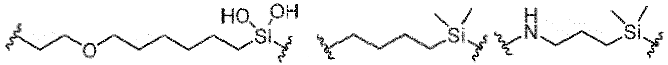

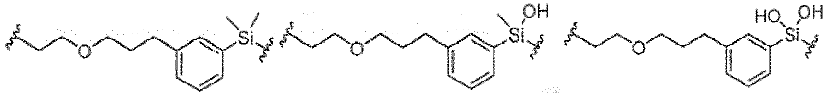

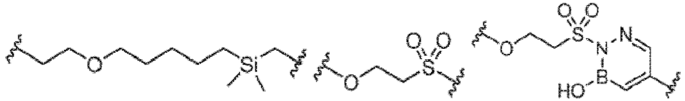

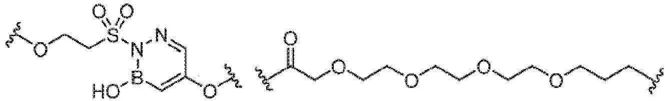

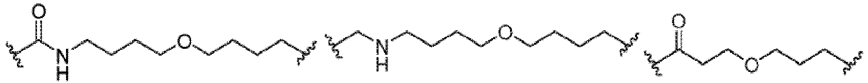

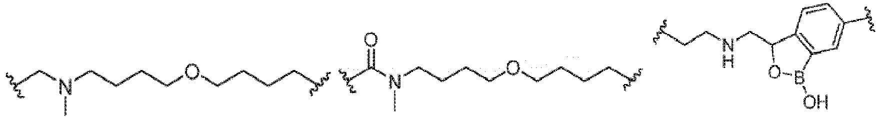

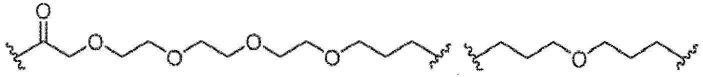

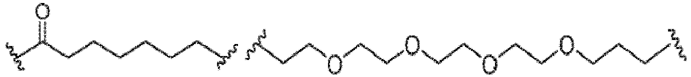

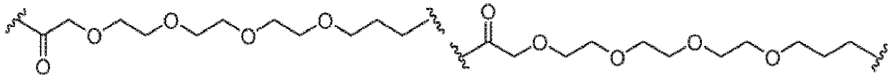

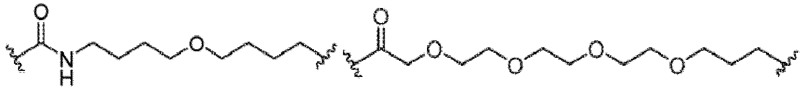

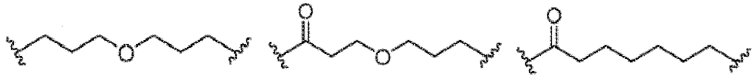

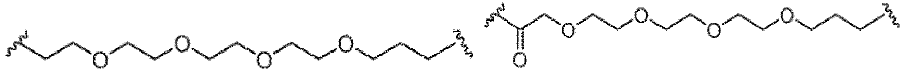

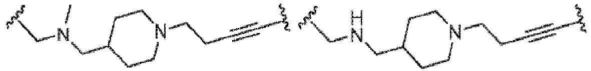

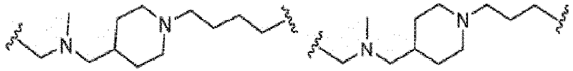

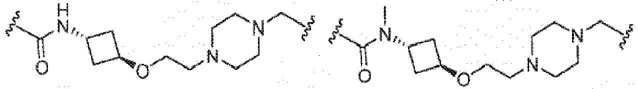

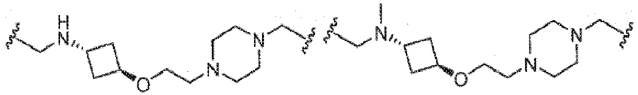

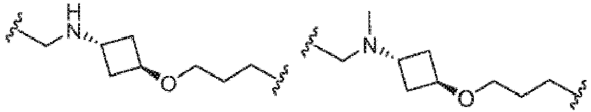

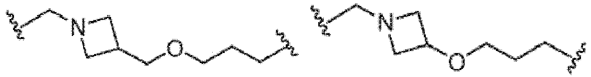

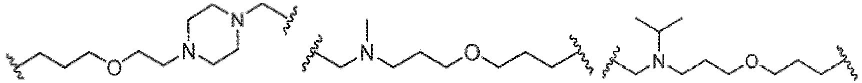

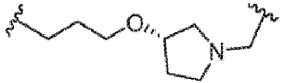

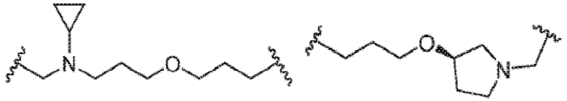

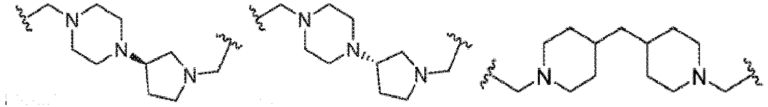

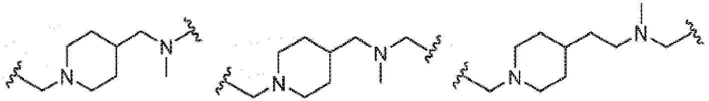

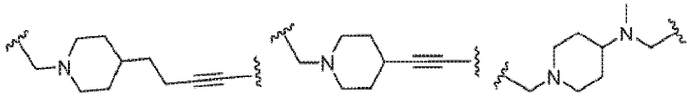

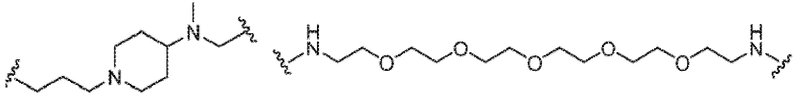

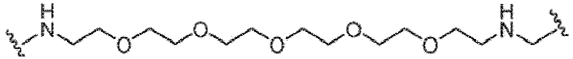

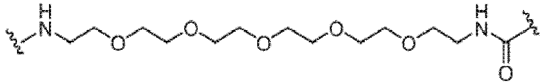

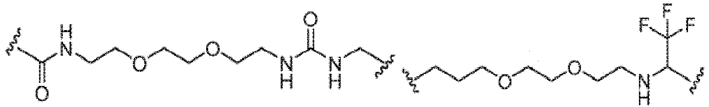

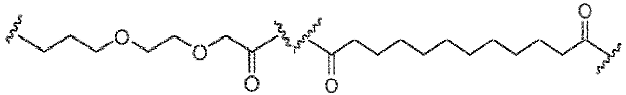

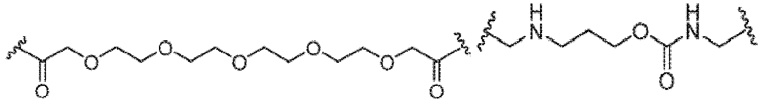

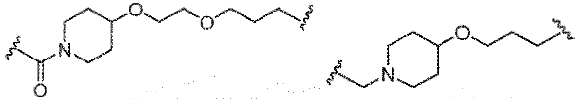

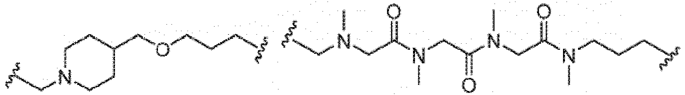

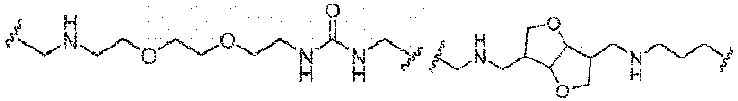

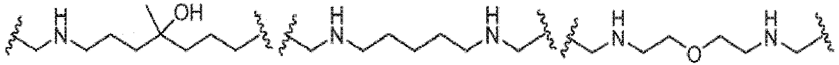

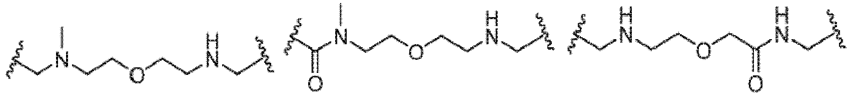

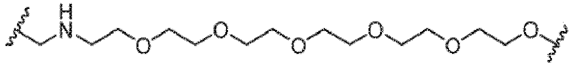

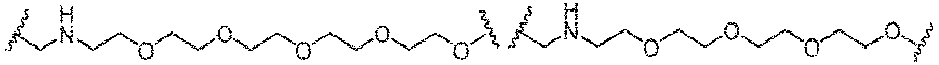

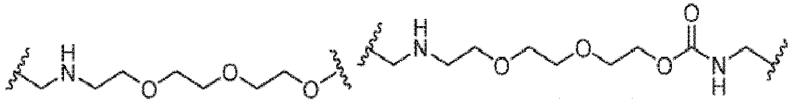

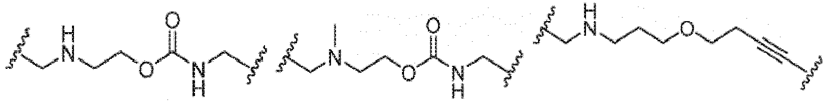

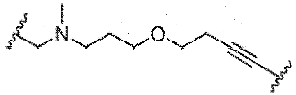

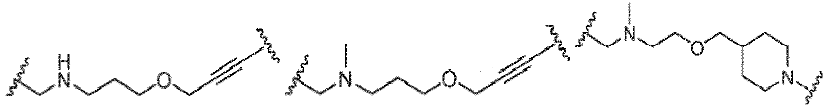

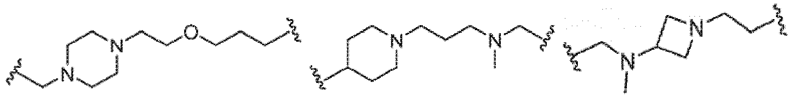

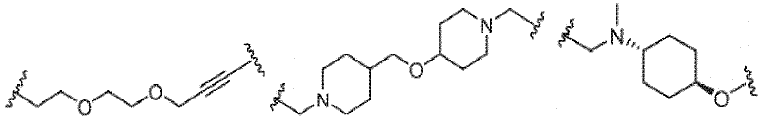

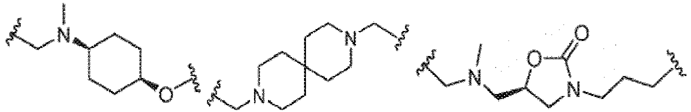

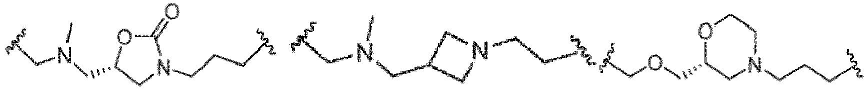

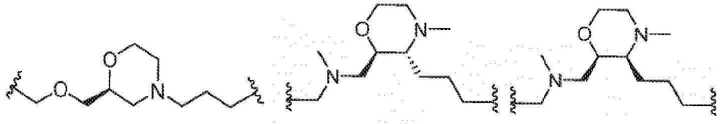

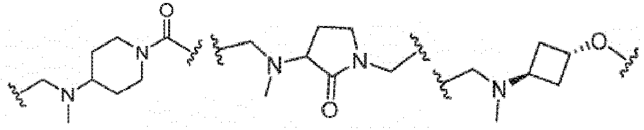

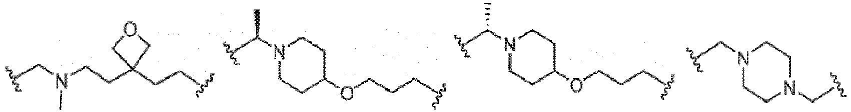

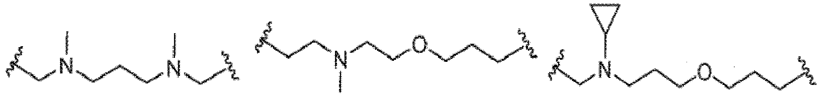

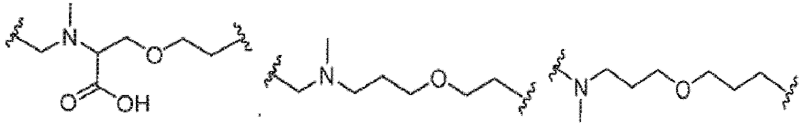

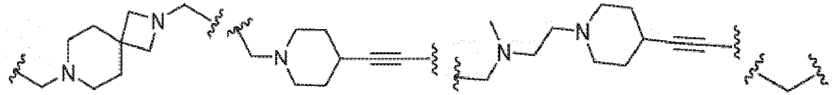

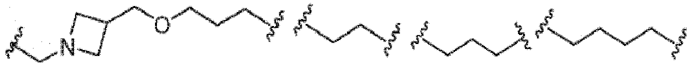

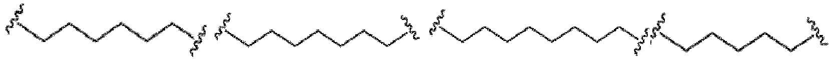

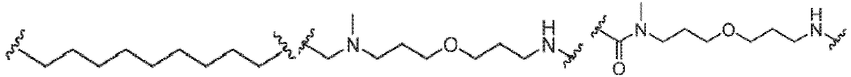

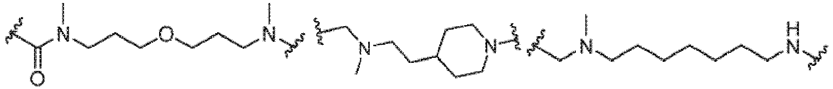

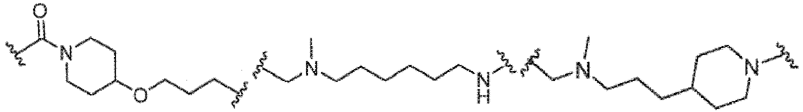

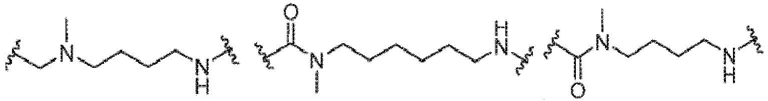

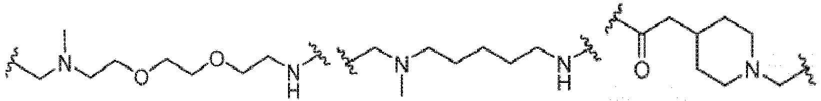

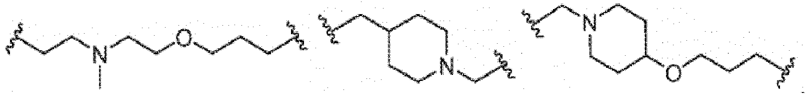

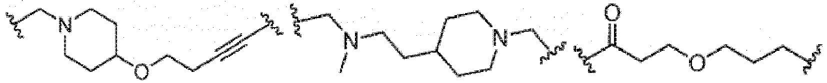

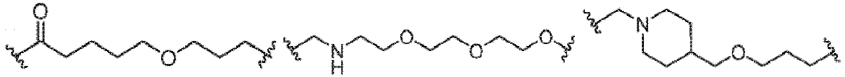

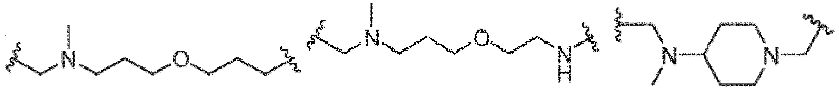

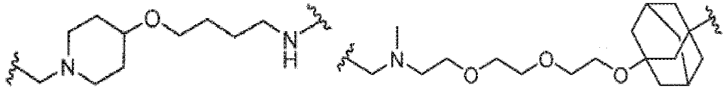

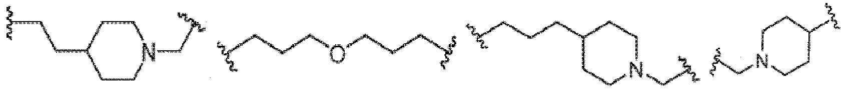

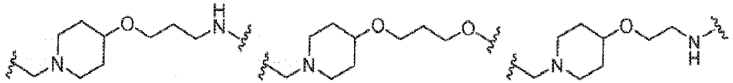

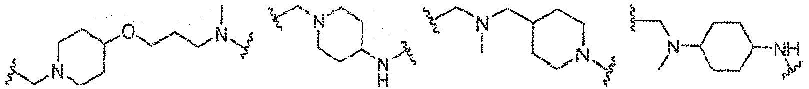

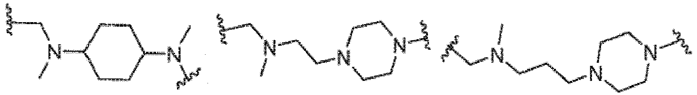

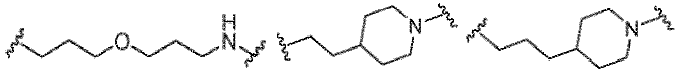

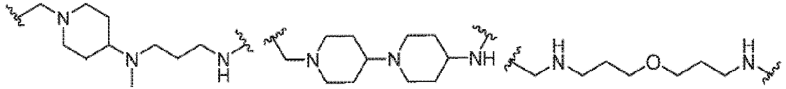

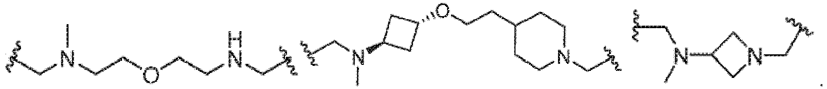

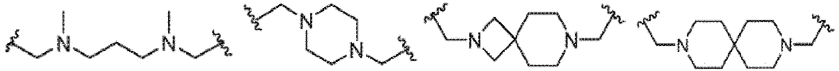

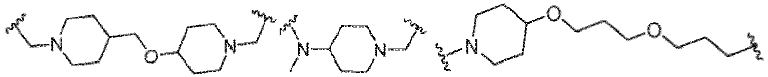

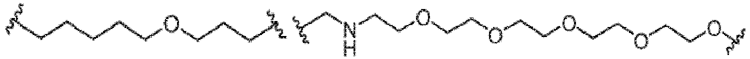

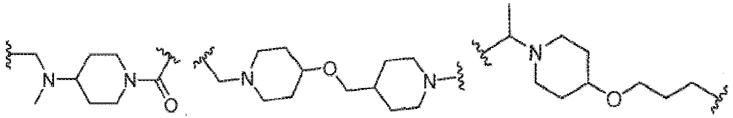

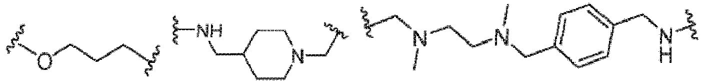

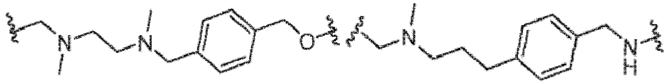

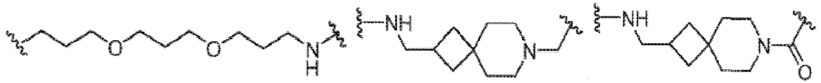

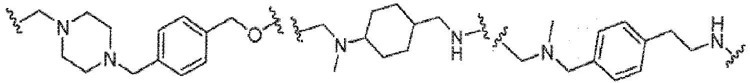

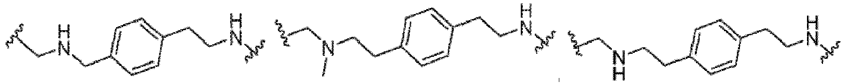

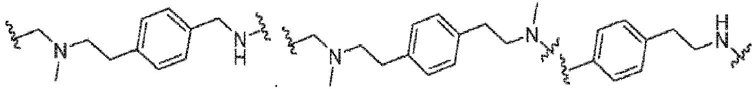

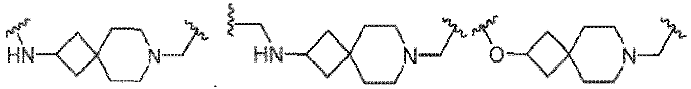

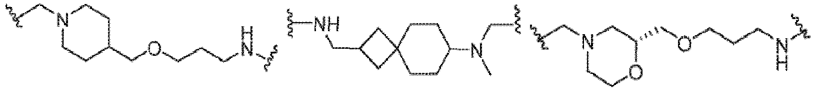

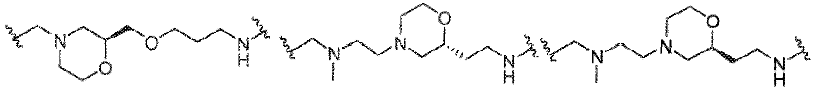

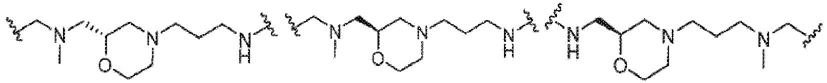

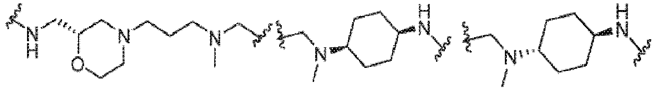

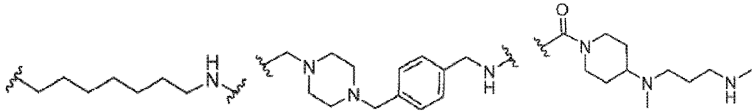

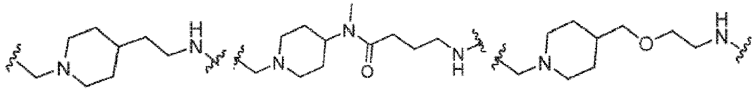

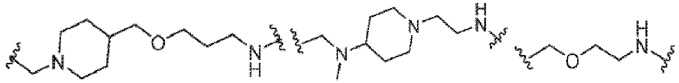

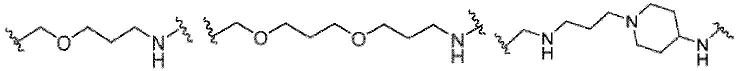

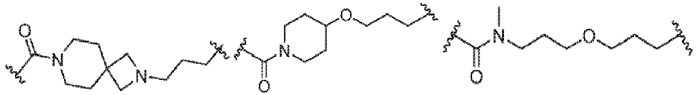

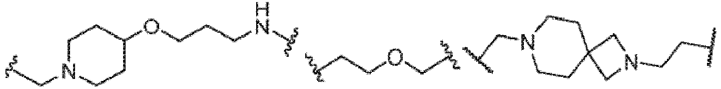

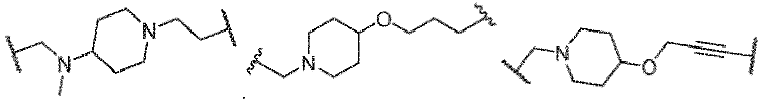

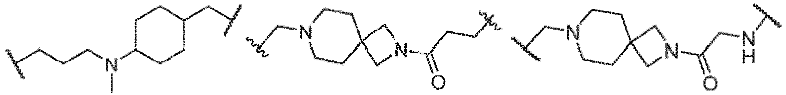

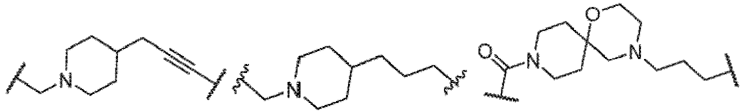

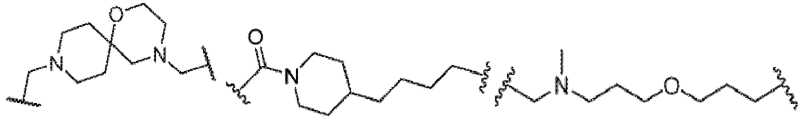

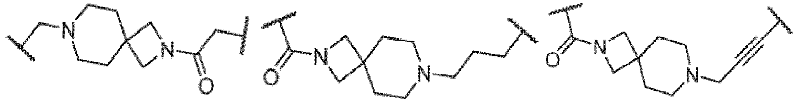

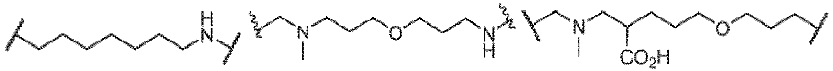

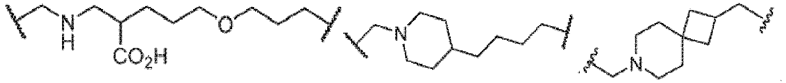

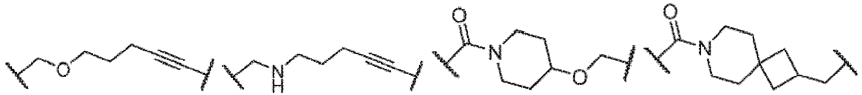

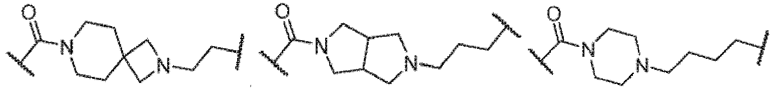

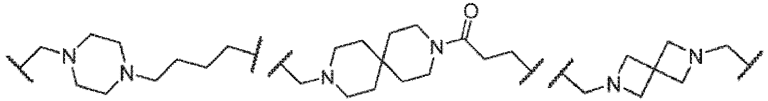

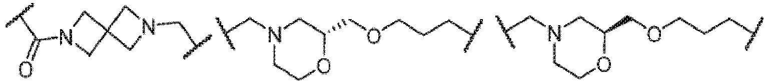

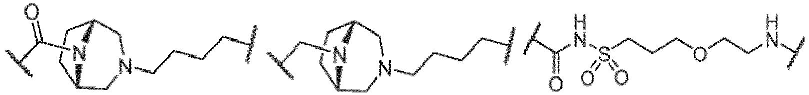

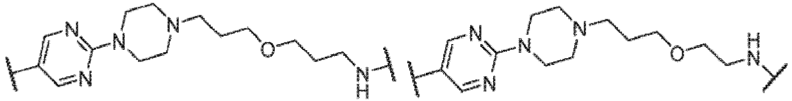

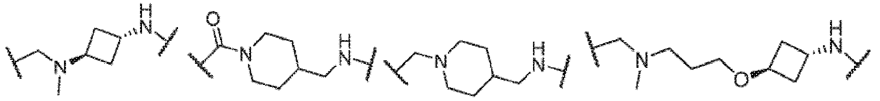

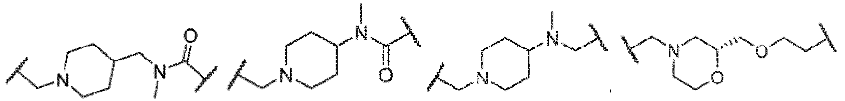

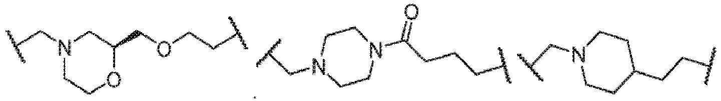

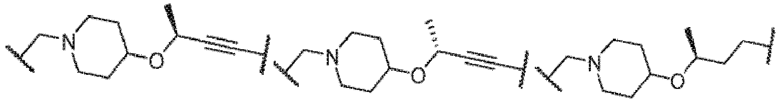

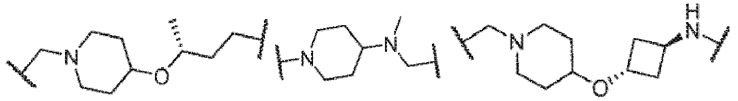

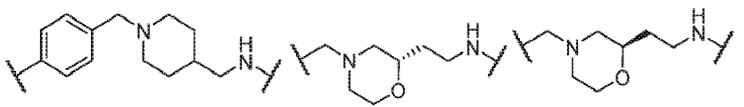

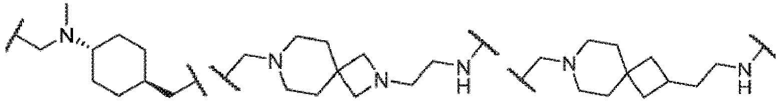

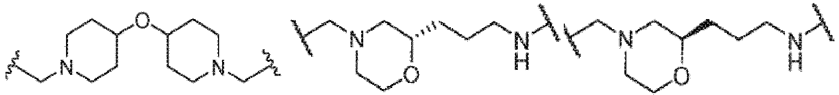

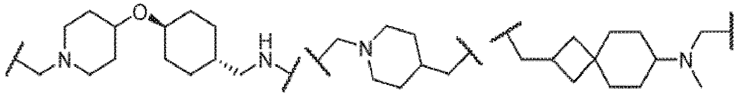

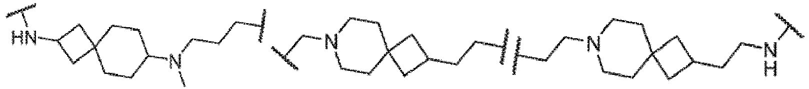

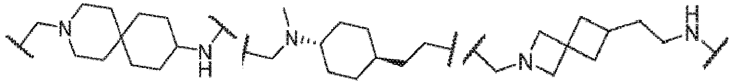

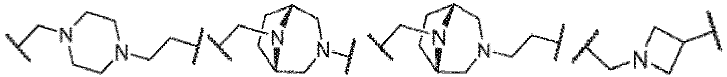

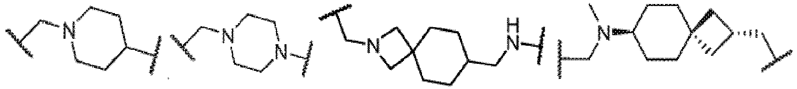

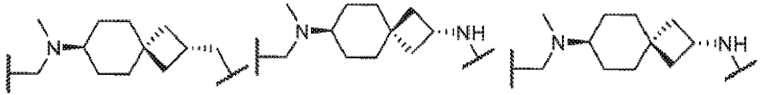

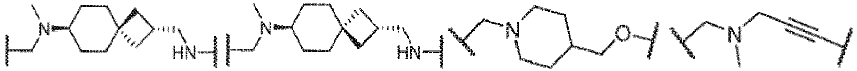

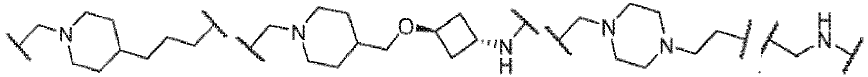

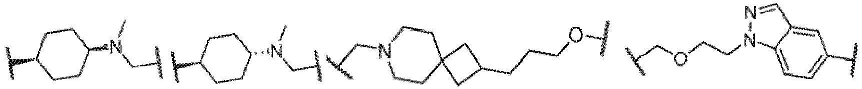

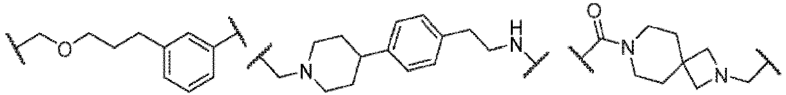

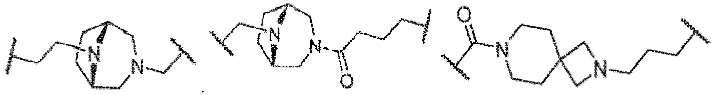

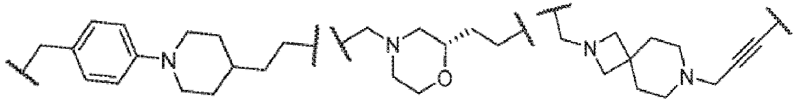

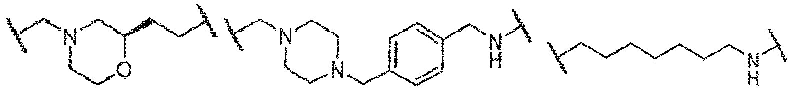

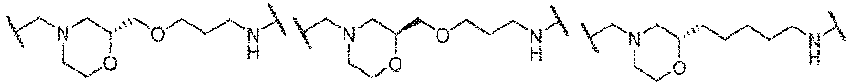

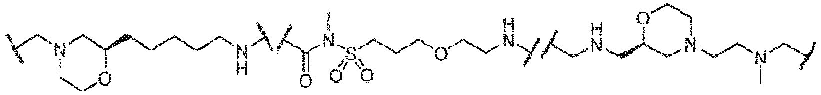

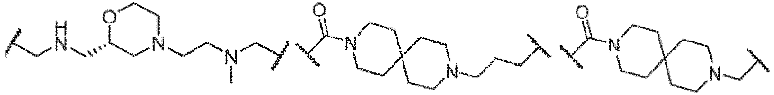

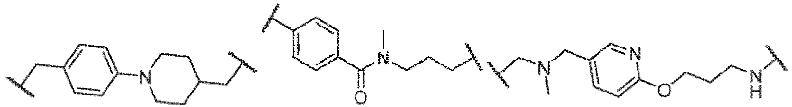

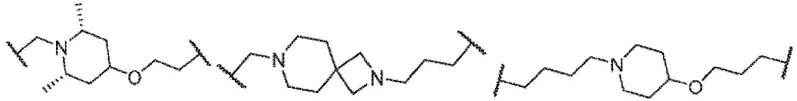

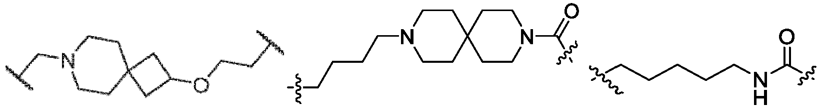

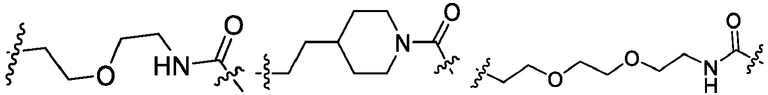

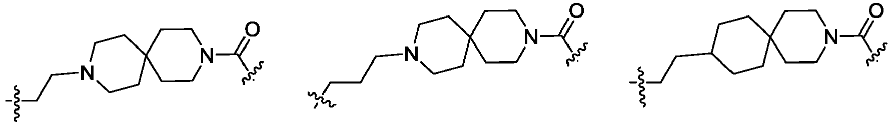

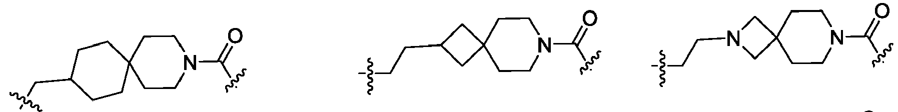

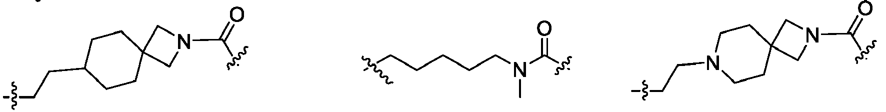

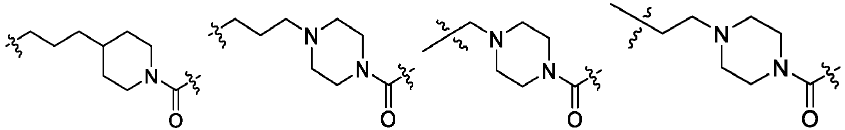

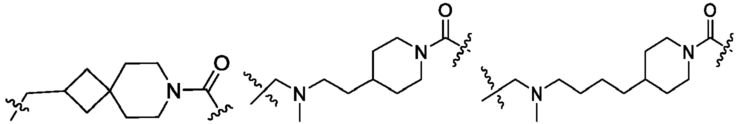

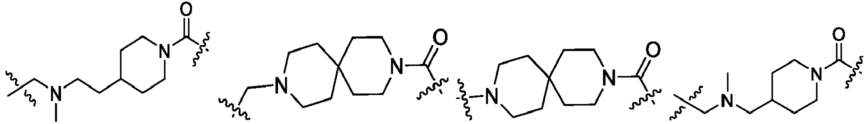

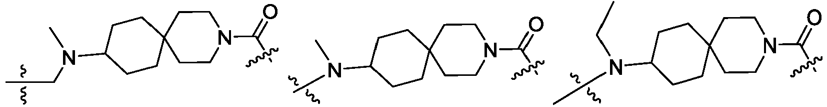

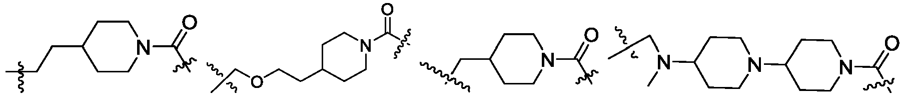

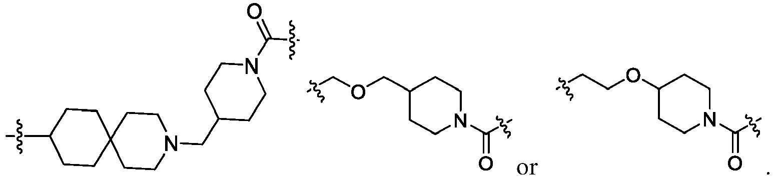

Preferably, in some embodiments of the present invention, the L is LA, 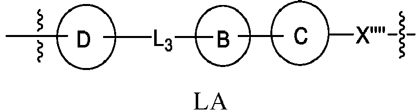 wherein, in LA,
ring D is absent or is C3-C12 cycloalkylene or 3-12 membered heterocycloalkylene containing 1-2 heteroatoms selected from N, O or S, the cycloalkylene and the heterocycloalkylene are optionally substituted by a substituent selected from halogen, oxo, cyano, amino, hydroxyl, C1-C6 alkyl, C1-C6 haloalkyl, C1-C6 hydroxyalkyl or -O-(C1-C6 alkyl);
ring B is absent or is C3-C12 cycloalkylene or 3-12 membered heterocycloalkylene containing 1-2 heteroatoms selected from N, O or S, the cycloalkylene and the heterocycloalkylene are optionally substituted by a substituent selected from halogen, oxo, cyano, amino, hydroxyl, C1-C6 alkyl, C1-C6 haloalkyl, C1-C6 hydroxyalkyl or -O-(C1-C6 alkyl);
ring C is absent, C3-C12 cycloalkylene or 3-12 membered heterocycloalkylene containing 1-2 heteroatoms selected from N, O or S, the cycloalkylene and the heterocycloalkylene are optionally substituted by a substituent selected from halogen, oxo, cyano, amino, hydroxyl, C1-C6 alkyl, C1-C6 haloalkyl, C1-C6 hydroxyalkyl or -O-(C1-C6 alkyl);
Xʺʺ is a bond, -NH-, -NCH₃-, -O-, -C(CH₃)₂-, -S-, -C=C- , -C=C-, -CHF-, -CHCF₃-, - C(O)-, -S(O)-, -S(O)₂-, -C(O)O-, -OC(O)-, -C(O)NH-, -C(O)NCH₃-, -NHC(O)- or - NCH₃C(O)-;
L₃ is -(CH₂)ₖ, one or two methylenes in L₃ are optionally replaced by a substituent selected from -O-, -NH-, -N(C1-C6 alkyl)-, -N(C1-C6 haloalkyl)-, -N(C1-C6 hydroxyalkyl)- or -N(C3-C8 cycloalkyl)-, k is 0, 1, 2, 3, 4, 5, 6, 7, 8, 9 or 10.

Preferably, in some embodiments of the present invention, the ring D is absent in the LA.

Preferably, in some embodiments of the present invention, in the LA, ring D is a 4-7-membered saturated monocyclic heterocycloalkylene containing 1 or 2 nitrogen heteroatoms, or a 7-11 membered spiro heterocycloalkylene or fused heterocycloalkylene containing 1 or 2 nitrogen heteroatoms.

Preferably, in some embodiments of the present invention, in the LA, the ring D is a 7-11 membered spiro heterocycloalkylene containing one or two nitrogen atoms.

Preferably, in some embodiments of the present invention, in the LA, the ring D is

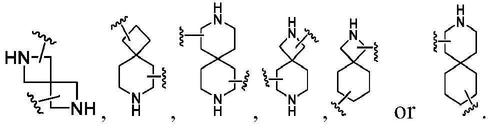

Preferably, in some embodiments of the present invention, ring B is absent in the LA.

Preferably, in some embodiments of the present invention, in the LA, ring B is a 4-7-membered saturated monocyclic heterocycloalkylene containing 1 or 2 nitrogen heteroatoms, or a 7-11 membered spiro heterocycloalkylene or fused heterocycloalkylene containing 1 or 2 nitrogen heteroatoms.

Preferably, in some embodiments of the present invention, in the LA, ring B is a 4-7-membered saturated monocyclic heterocycloalkylene containing one or two nitrogen heteroatoms.

Preferably, in some embodiments of the present invention, in the LA, ring B is piperidylidene, piperazinylidene, 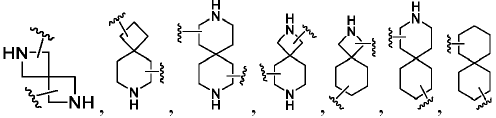 or

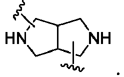

Preferably, in some embodiments of the present invention, in the LA, ring C is 3-7-membered saturated or partially unsaturated cycloalkylene, 4-11membered saturated or partially unsaturated spirocycloalkylene, 4-11 membered saturated or partially unsaturated fused cycloalkylene, 8-10 membered bicyclic saturated or partially unsaturated cycloalkylene, 4-7 membered saturated or partially unsaturated heterocycloalkylene containing 1-2 heteroatoms independently selected from nitrogen, oxygen or sulfur, 4-11 membered saturated or partially unsaturated spiro heterocycloalkylene containing 1-2 heteroatoms independently selected from nitrogen, oxygen or sulfur, 4-11 membered saturated or partially unsaturated fused heterocycloalkylene containing 1-2 heteroatoms independently selected from nitrogen, oxygen or sulfur, 8-10 membered bicyclic saturated or partially unsaturated heterocycloalkylene containing 1-2 heteroatoms independently selected from nitrogen, oxygen or sulfur.

Preferably, in some embodiments of the present invention, in the LA, ring C is a 4-7-membered saturated monocyclic heterocycloalkylene containing 1 or 2 nitrogen heteroatoms, a 7-11 membered spiro heterocycloalkylene or fused heterocycloalkylene containing 1 or 2 nitrogen heteroatoms.

Preferably, in some embodiments of the present invention, in the LA, ring C is a 4-7-membered saturated monocyclic heterocycloalkylene containing 1 or 2 nitrogen heteroatoms, a 7-11 membered spiro heterocycloalkylene containing 1 or 2 nitrogen heteroatoms.

Preferably, in some embodiments of the present invention, in the LA, the ring C is piperidylidene, piperazinylidene, 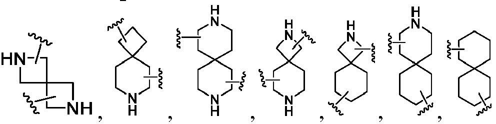 or

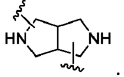

Preferably, in some embodiments of the present invention, in the LA, the ring C is piperidylidene, piperazinylidene,

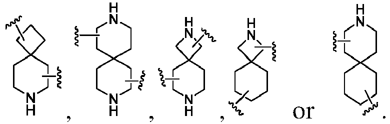

Preferably, in some embodiments of the present invention, in the LA, X‴′ is a bond or - C(O)-.

Preferably, in some embodiments of the present invention, in the LA, X‴′ is -C(O)-.

Preferably, in some embodiments of the present invention, in the LA, L₃ is -(CH₂)ₖ, and one methylene in the L₃ is optionally replaced by a group selected from -O-,-NH-,-NCH₃- or -NCH₂CH₃-; k is 1, 2, 3 or 4.

Preferably, in some embodiments of the present invention, in the LA, ring D and ring B are absent, ring C is 4-7-membered saturated monocyclic heterocycloalkylene containing 1 or 2 nitrogen heteroatoms, or 7-11-membered spiro heterocycloalkylene containing 1 or 2 nitrogen heteroatoms; Xʺʺ is -C(O)-; L₃ is -(CH₂)ₖ, one methylene in L3 is optionally replaced by a group selected from -O-, -NH-, -NCH3- or -NCH2CH3-; k is 2, 3 or 4.

Preferably, in some embodiments of the present invention, formula L is selected from

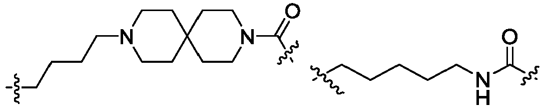

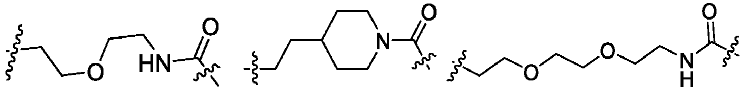

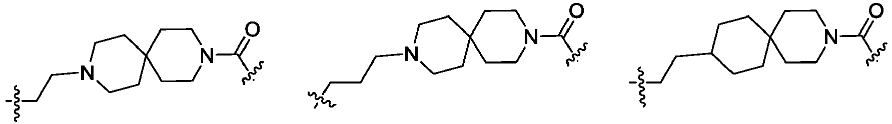

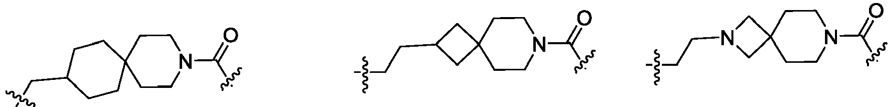

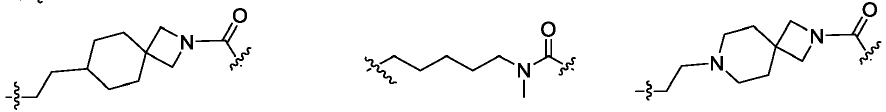

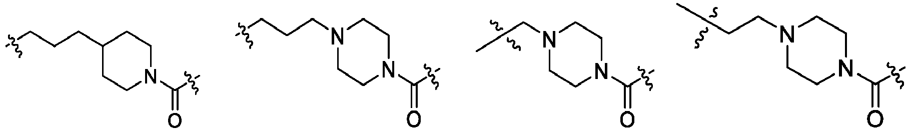

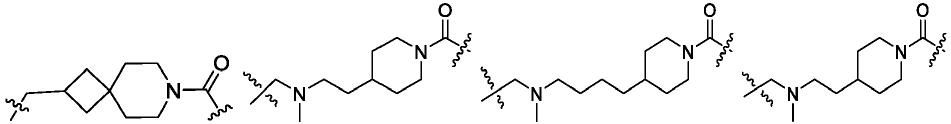

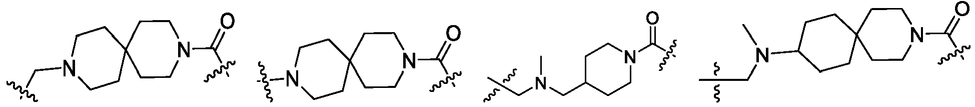

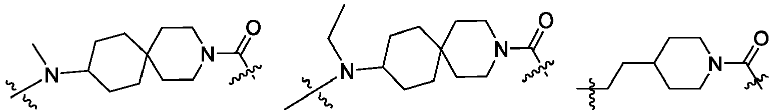

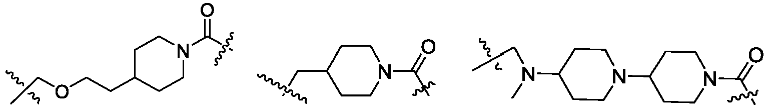

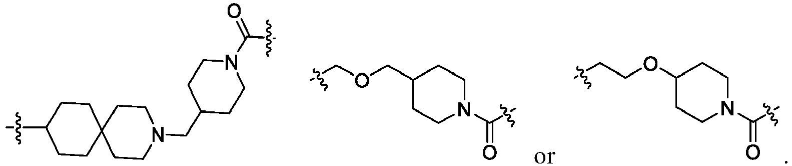

Preferably, in some embodiments of the present invention, the compound of formula I, and/or a stereoisomer, enantiomer, diastereomer, deuterate, metabolite, hydrate, solvate, prodrug thereof and/or a pharmaceutically acceptable salt thereof is a compound of formula IIa, and/or a stereoisomer, enantiomer, diastereomer, deuterate, metabolite, hydrate, solvate, prodrug thereof and/or a pharmaceutically acceptable salt thereof, 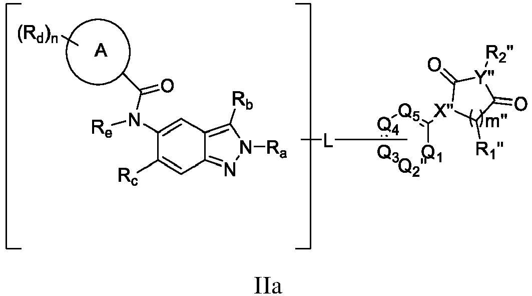 wherein the ring A, Rₐ, R_{b}, R_{c}, R_{d}, Rₑ, n are as defined in PTM-71, and the Q₁, Q₂, Q₃, Q₄, Q₅, X", Y", m", R₁", R₂" are as defined in ULM-1 herein; the L is as defined herein.

Preferably, in some embodiments of the present invention, the compound of formula I, and/or a stereoisomer, enantiomer, diastereomer, deuterate, metabolite, hydrate, solvate, prodrug thereof and/or a pharmaceutically acceptable salt thereof is a compound of formula IIa-1, and/or a stereoisomer, enantiomer, diastereomer, deuterate, metabolite, hydrate, solvate, prodrug thereof and/or a pharmaceutically acceptable salt thereof, 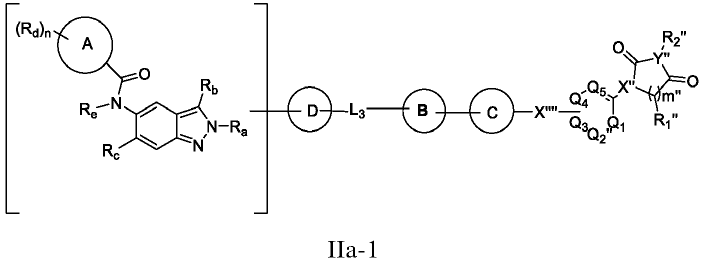 wherein the ring A, Rₐ, R_{b}, R_{c}, R_{d}, Rₑ, and n are as defined in PTM-71, the Q₁, Q₂, Q₃, Q₄, Q₅, X", Y", m", R₁",and R₂" are as defined in ULM-1 herein; the L3, ring D, ring B, ring C, and X‴ are as defined herein.

Preferably, in some embodiments of the present invention, the compound of formula I has the following structure:

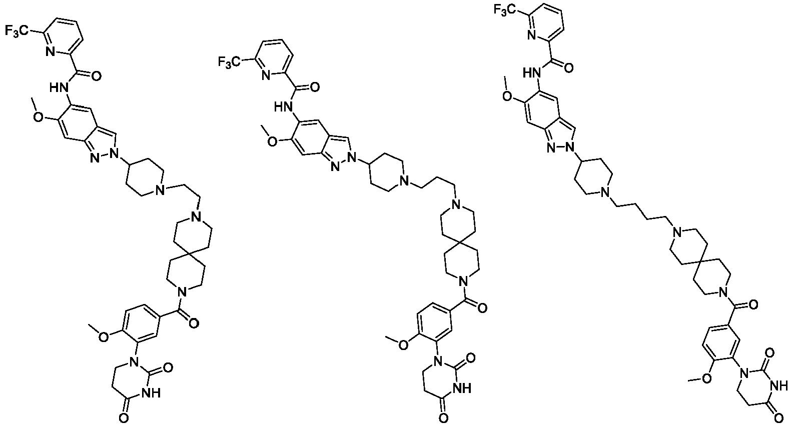

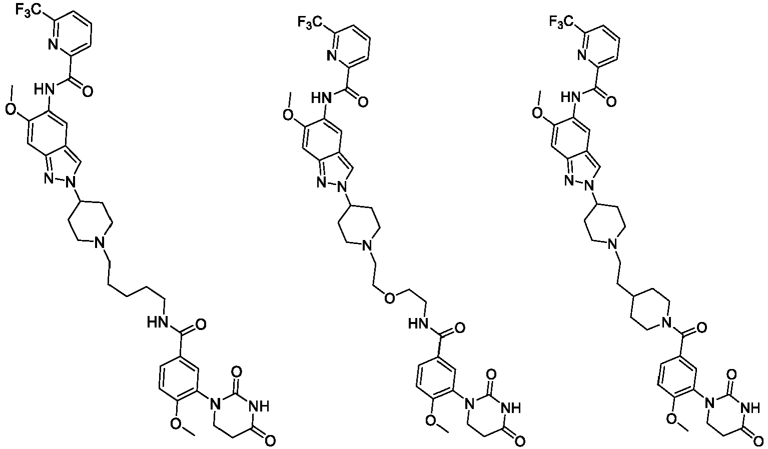

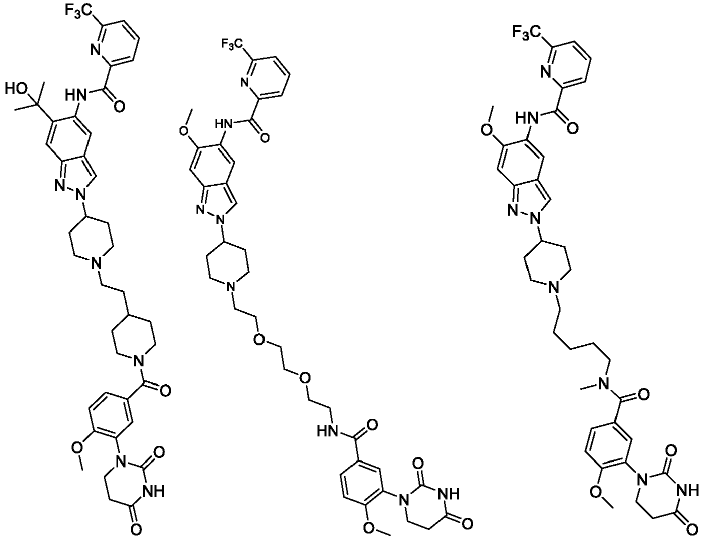

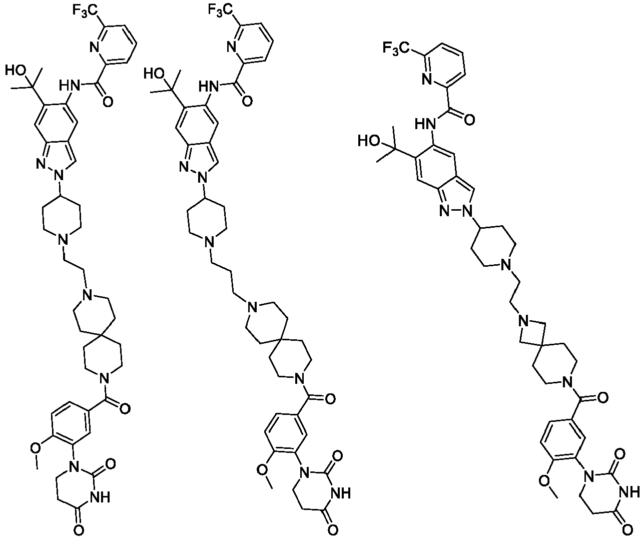

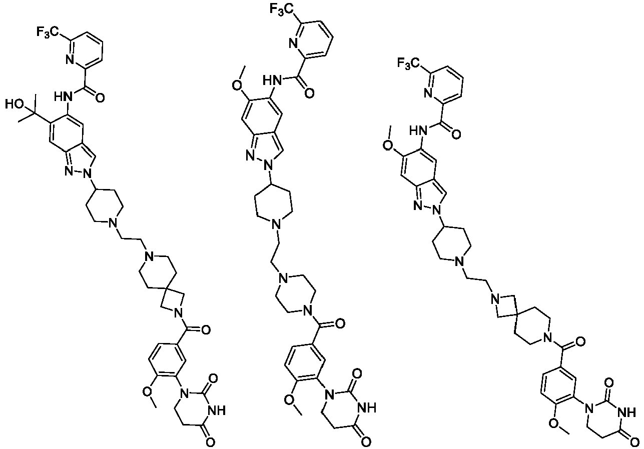

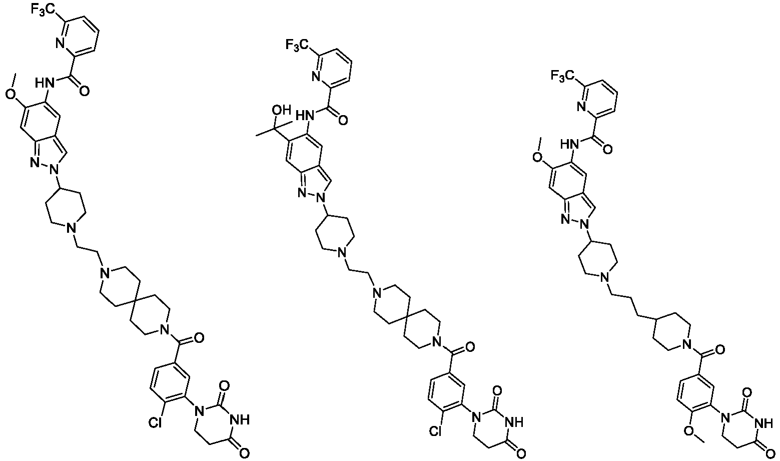

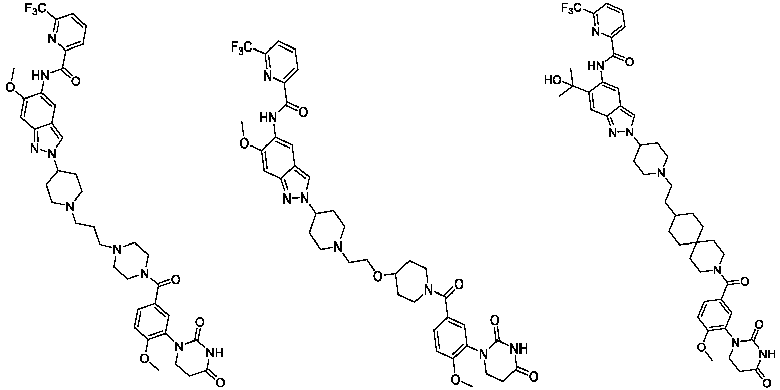

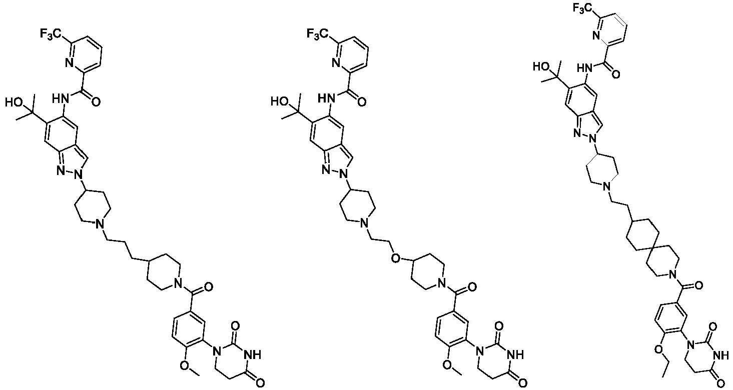

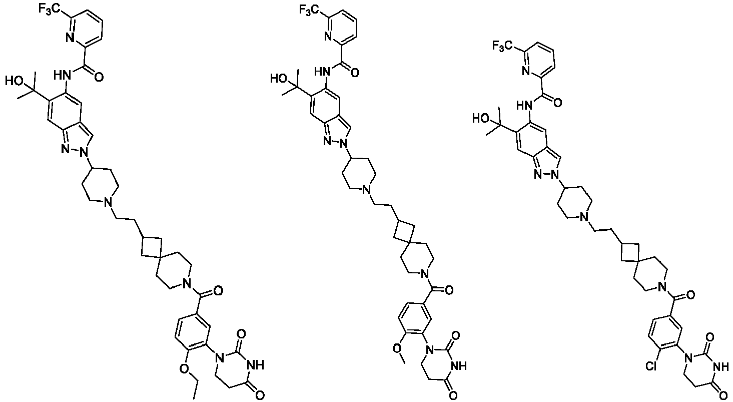

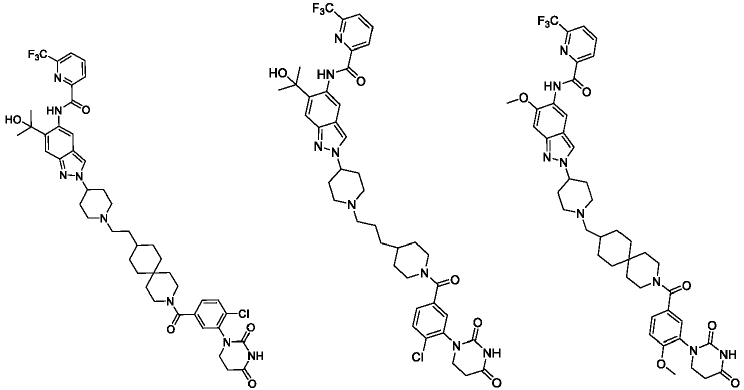

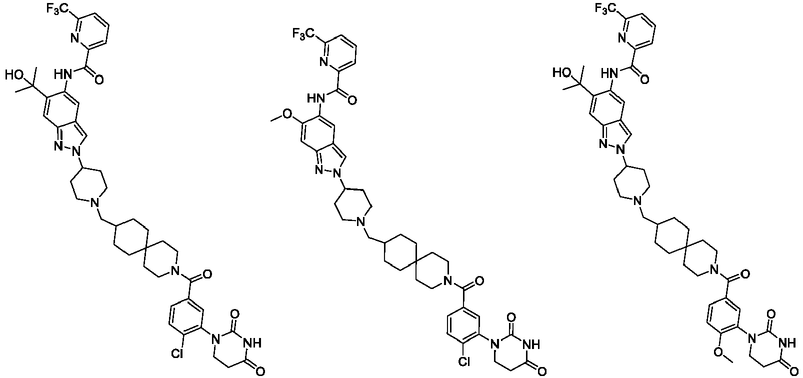

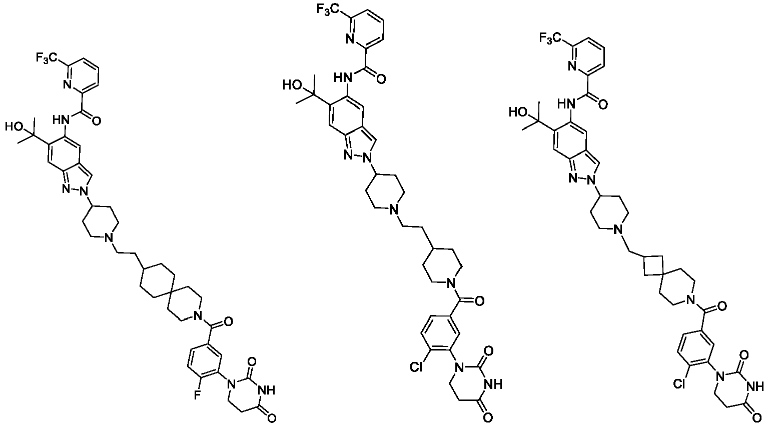

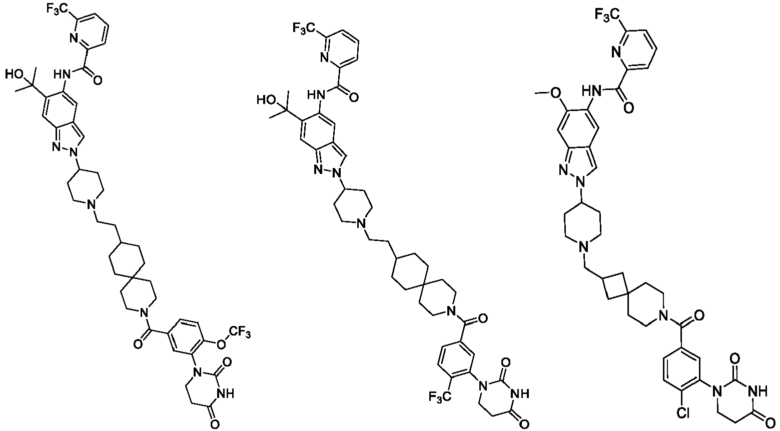

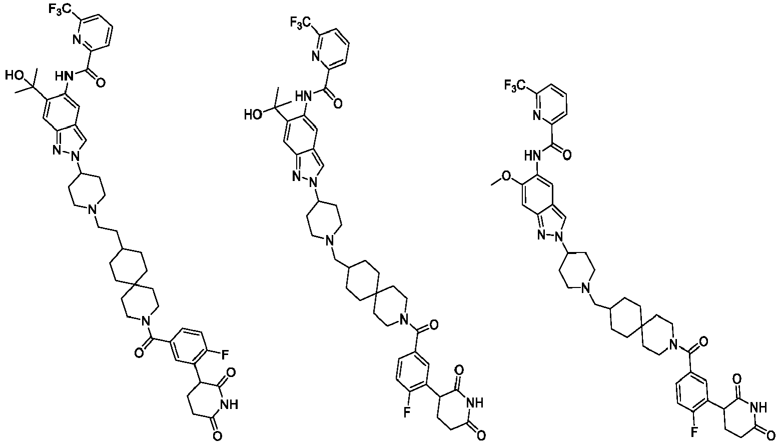

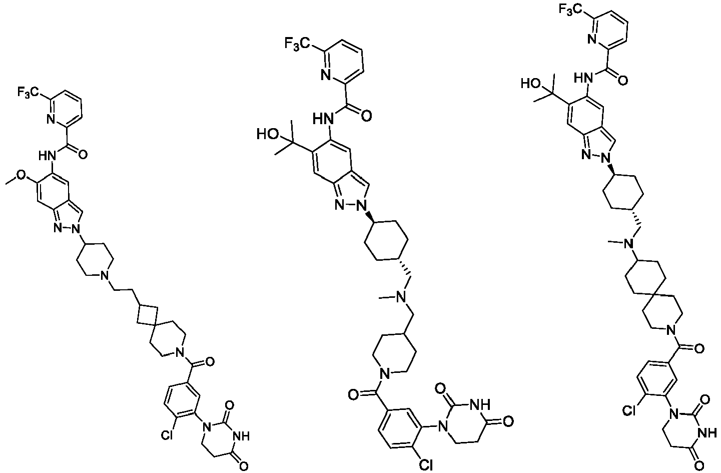

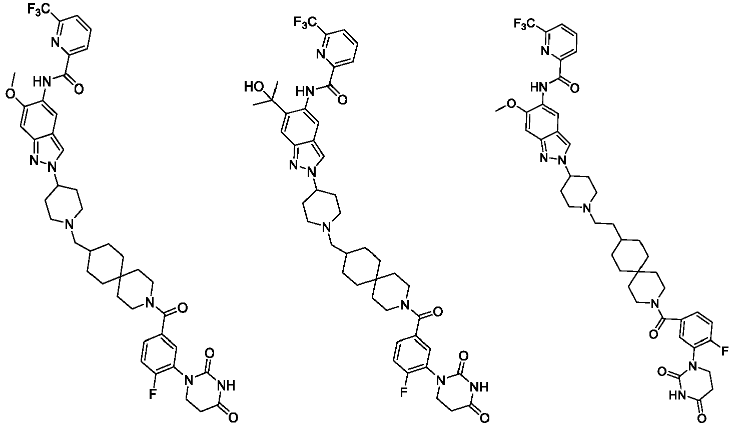

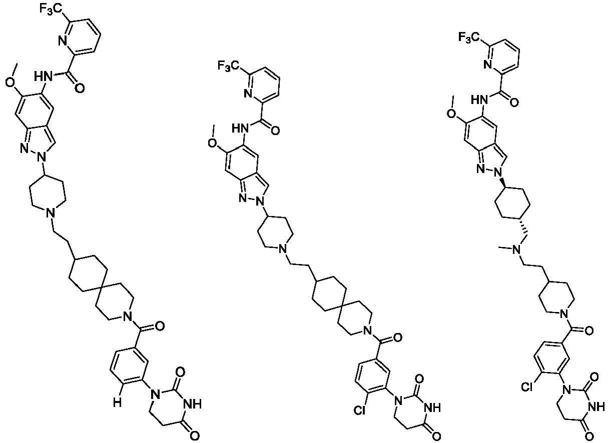

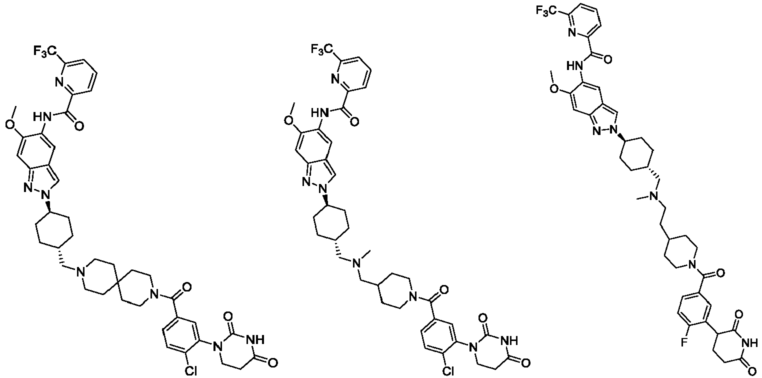

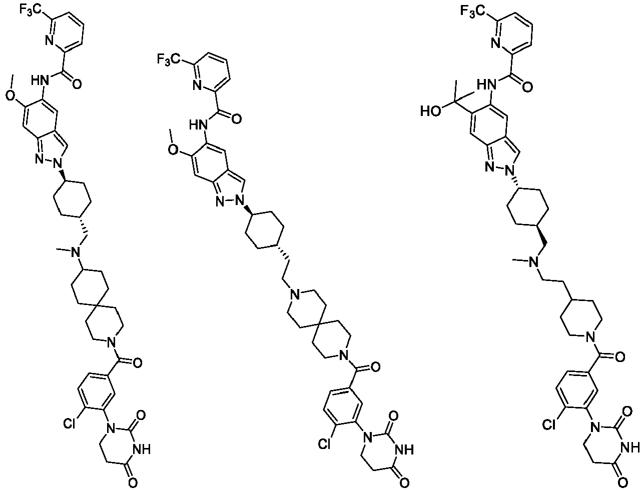

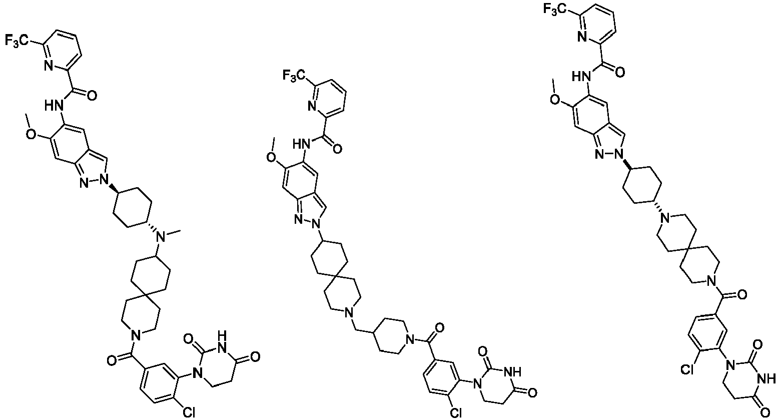

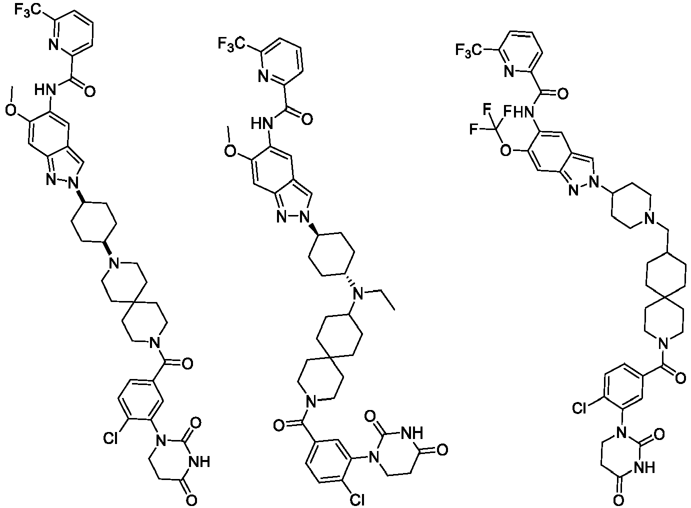

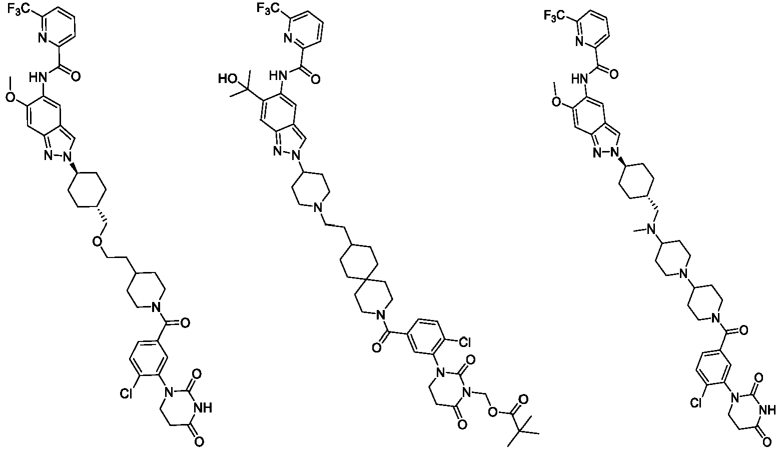

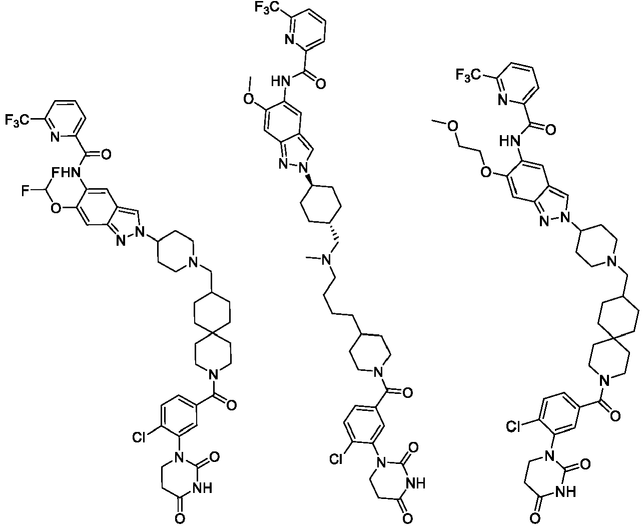

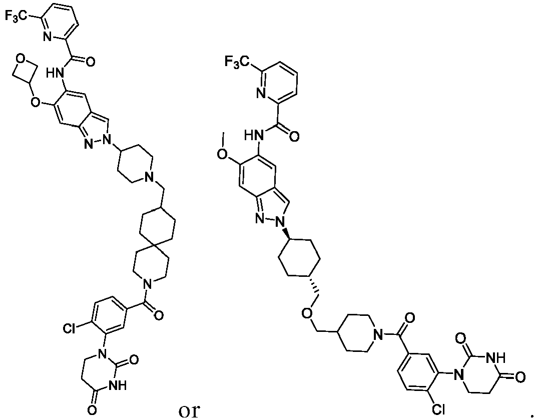

The invention provides a method for preparing the compound of formula I, formula Ia, formula IA, formula IA-1, formula IA-2, formula IA- 3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt.

The invention provides a pharmaceutical composition comprising a therapeutically effective amount of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, and a pharmaceutically acceptable carrier, diluent or excipient.

The invention provides a method for degrading IRAK4 protein, including a step of contacting the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition with IRAK4 protein.

The compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition is used as a drug for the treatment or prevention of IRAK4-mediated diseases or conditions.

The compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition is used as a drug for the treatment or prevention of diseases or conditions mediated by TLR (other than TLR3R), IL-1α or IL-1β receptor family (including IL-1R, IL-18R, IL-33R, IL-36R).

The compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition is used as a drug for the treatment or prevention of IRAK4-mediated diseases or conditions, which are diseases or conditions driven by MyD88.

The invention provides a use of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition in the preparation of a medicament for the treatment or prevention of IRAK4-mediated diseases or conditions.

The invention provides a use of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition in the preparation of a medicament for the treatment or prevention of diseases or conditions mediated by TLR (other than TLR3R), IL-1α, or IL-1β receptor family (including IL-1R, IL-18R, IL-33R, IL-36R).

The invention provides a use of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition in the preparation of a medicament for the treatment or prevention of a disease or condition regulated by IRAK4, and the IRAK4-mediated disease or condition is a disease or condition driven by MyD88.

The invention provides a use of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition in the preparation of a medicament for the treatment or prevention of cancer, neurodegenerative diseases, viral diseases, autoimmune diseases, inflammatory diseases, hereditary diseases, hormone-related diseases, metabolic diseases, organ transplantation-related diseases, immunodeficiency diseases, destructive bone diseases, proliferative disorders, infectious diseases, conditions related to cell death, thrombin-induced platelet aggregation, liver diseases, pathological immune conditions involving T cell activation, cardiovascular diseases or CNS diseases.

The invention provides a use of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition in the preparation of a medicament for the treatment or prevention of cancer or proliferative disease, and the cancer or proliferative disease is brain cancer, kidney cancer, liver cancer, adrenal cancer, bladder cancer, breast cancer, gastric cancer, ovarian cancer, colon cancer, rectal cancer, prostate cancer, pancreatic cancer, lung cancer, vaginal cancer, cervical cancer, testicular cancer, urogenital cancer, esophageal cancer, laryngeal cancer, skin cancer, bone cancer, thyroid cancer, sarcoma, neuroglioblastoma, neuroblastoma, multiple myeloma, gastrointestinal cancer, neck or head tumor, epidermal hyperproliferation, psoriasis, prostatic hyperplasia, adenoma, adenocarcinoma, keratoacanthoma, epidermoid cancer, large cell carcinoma, non-small cell lung cancer, lymphoma, Hodgkin's and non-Hodgkin's, breast cancer, follicular cancer, undifferentiated tumor, papillary tumor, seminoma, melanoma, ABC DLBCL, Hodgkin's lymphoma, primary cutaneous T-cell lymphoma, chronic lymphocytic leukemia, smoldering indolent multiple myeloma, leukemia, diffuse large B- cell lymphoma (DLBCL), chronic lymphocytic leukemia (CLL), chronic lymphocytic lymphoma, primary exudative lymphoma, Burkitt lymphoma/leukemia, acute lymphocytic leukemia, B-cell prolymphocytic leukemia, lymphoplasmacytic lymphoma, Waldenstroms's macroglobulinemia (WM), splenic marginal zone lymphoma, multiple myeloma, or plasmacytoma or intravascular large B -cell lymphoma.

The invention provides a use of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition in the preparation of a medicament for the treatment or prevention of neurodegenerative disease, and the neurodegenerative disease is neurodegenerative disease caused by Alzheimer's disease, Parkinson's disease, amyotrophic lateral sclerosis, Huntington's disease, cerebral ischemia or traumatic injury, glutamate neurotoxicity, hypoxia, epilepsy, diabetes treatment, metabolic syndrome, obesity, organ transplantation or graft-versus-host disease.

The invention provides a use of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition in the preparation of a medicament for the treatment or prevention of inflammatory disease, and the inflammatory disease is eye disease, such as eye allergy, conjunctivitis, dry eye or spring conjunctivitis, diseases affecting the nose, including allergic rhinitis; autoimmune hematological diseases, such as hemolytic anemia, aplastic anemia, pure red blood cell anemia and idiopathic thrombocytopenia, systemic lupus erythematosus, rheumatoid arthritis, polychondritis, scleroderma, dermatomyositis, chronic active hepatitis, myasthenia gravis, Stephen-Johnson syndrome, idiopathic stomatitis diarrhea, autoimmune inflammatory bowel disease, bowel syndrome, celiac disease, root periostitis, lung hyaline membrane disease, nephropathy, glomerular disease, Alcoholic liver disease, multiple sclerosis, endocrine ophthalmopathy, Grave's disease, Sarcomatosis, dry eye, spring conjunctival keratitis, interstitial pulmonary fibrosis, psoriatic arthritis, systemic juvenile idiopathic arthritis, nephritis, vasculitis, interstitial cystitis, diverticulitis, Glomerulonephritis, chronic granulomatous disease, endometriosis, leptospirosis nephropathy, glaucoma, retinal disease, aging, headache, pain, complex regional pain syndrome, cardiac hypertrophy, muscle atrophy, catabolism, obesity, slow fetal growth, hypercholesterolemia, heart disease, chronic heart failure, mesothelioma, anhidromic ectodermal dysplasia, Behcet's disease, pigment incontinence, Paget's disease, pancreatitis, hereditary periodic fever syndrome, asthma, acute lung injury, acute respiratory distress syndrome, eosinophilia, allergic reaction, systemic allergic reaction, sinusitis, eye allergy, silica-induced diseases, COPD, lung disease, cystic fibrosis, liver fibrosis, renal fibrosis, alcoholic fatty liver, non-alcoholic fatty liver, heart fibrosis, psoriasis, Crohn's disease, inflammatory bowel disease, acid-induced lung injury, pulmonary hypertension, polyneuropathy, Cataract, muscle inflammation combined with systemic sclerosis, inclusion body myositis, myasthenia gravis, thyroiditis, Addison's disease, lichen planus, type 1 diabetes, type 2 diabetes, appendicitis, atopic dermatitis, asthma, allergies, bronchiolitis, bronchitis, bursitis, cervicitis, cholangitis, cholecystitis, chronic transplant rejection, colitis, conjunctivitis, cystitis, lacrimal gland inflammation, dermatitis, dermatomyositis, encephalitis, endocarditis, Endometritis, enteritis, enterocolitis, upper ankle inflammation, epididymitis, fasciitis, fibrous tissue inflammation, gastritis, gastroenteritis, Henoch-Schonlein purpura, hepatitis, suppurative sweat Inflammation, immunoglobulin A nephropathy, interstitial lung disease, laryngitis, mastitis, meningitis, myelitis, myocarditis, myositis, nephritis, oophoritis, orchitis, osteitis, otitis, pancreatitis, mumps, pericarditis, peritonitis, pharyngitis, pleurisy, phlebitis, local pneumonia, pneumonia, polymyositis, proctitis, prostatitis, pyelonephritis, rhinitis, salpingitis, sinusitis, stomatitis, articular inflammation, tendinitis, tonsillitis, ulcerative colitis, uveitis, vaginitis, vasculitis, vulvitis, alopecia areata, erythema multiforme, dermatitis herpetiformis, sclerosis, vitiligo, hypersensitivity vasculitis, urticaria, bullous pemphigoid, pemphigus vulgaris, deciduous pemphigus, paraneoplastic pemphigus, acquired bullous epidermal laxity, acute and chronic gout, chronic gouty arthritis, psoriasis, psoriasis arthritis, rheumatoid arthritis, juvenile rheumatoid arthritis, cryopyrin-associated periodic syndrome or osteoarthritis.

The invention provides a method for treating or preventing IRAK4-mediated diseases or conditions, including the administraton of a therapeutically effective amount of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition to a subject in need thereof.

The invention provides a method for treating or preventing IRAK4-mediated diseases or conditions, including the administration of a therapeutically effective amount of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition to a subject in need thereof, and the IRAK4-mediated disease or condition is a disease or condition driven by MyD88.

The invention provides a method for the treatment or prevention of diseases or conditions mediated by TLR (other than TLR3R) or IL-1 receptor family (including IL-1R, IL-18R, IL-33R, IL-36R), including the administration of a therapeutically effective amount of the compound of formula I, formula Ia, formula IA, formula IA-1, formula IA-2, formula IA-3, formula IB, formula IB-1, formula IC, formula IC-1, formula IC-2, formula IC-3, formula IIa, formula IIa-1, and/or its stereoisomer, enantiomer, diastereomer, deuterate, hydrate, solvate, metabolite, prodrug and/or pharmaceutically acceptable salt, or its pharmaceutical composition to a subject in need thereof.

The invention provides a preparation method for the compound of formula I, and/or its stereoisomer, enantiomer, diastereomer, deuterate, hydrate, solvate, metabolite, prodrug and/or pharmaceutically acceptable salt,
method 1: 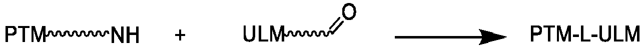 wherein the 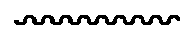 is the moiety of the linker L; the NH can be a primary amine or a secondary amine; and when the 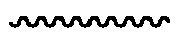 is a bond, the PTM 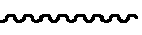 NH refers to PTM containing an amino group; the NH undergoes a reductive amination reaction with an aldehyde to prepare the compound of formula I, the PTM is as defined above, preferably PTM is 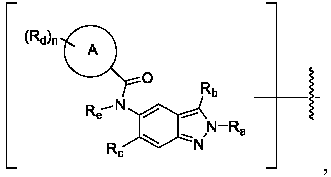 wherein the ring A, n, Rₐ, R_{b}, R_{c}, R_{d} and Rₑ are as defined in PTM-71; the ULM is as defined above; and the reducing reagents for reductive amination include, but are not limited to, Pd/C, sodium borohydride, sodium cyanoborohydride, borane, sodium triacetyl borohydride.
method 2: 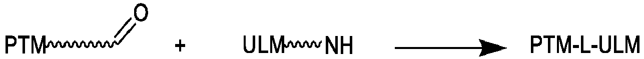
the 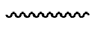 is the moiety of the linker L; the NH can be a primary amine or a secondary amine; the NH undergoes a reductive amination reaction with an aldehyde to prepare the compound of formula I, the PTM is as defined above, preferably PTM is 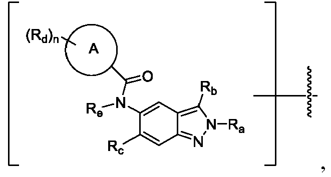 wherein the ring A, n, Rₐ, R_{b}, R_{c}, R_{d}, and Rₑ are as defined in PTM-71; the ULM is as defined above; and the reducing reagents for reductive amination include, but are not limited to, Pd/C, sodium borohydride, sodiumcyanoborohydride, borane, sodium triacetyl borohydride.
method 3: 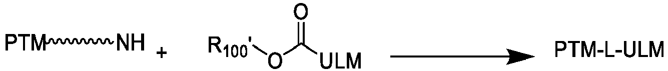 wherein the 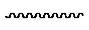 is the moiety of the linker L; the NH can be a primary amine or a secondary amine; the NH undergoes a substitution reaction with the ester in the 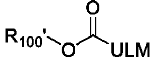 under basic conditions to prepare the compound of formula I, and the R_{100'} is pentafluorophenyl or p-nitrophenyl; the PTM is as defined above, preferably PTM is 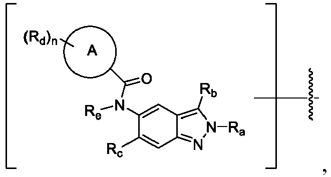 wherein the ring A, n, Rₐ, R_{b}, R_{c}, R_{d} and Rₑ are as defined in PTM-71; the ULM is as defined above; the bases in the substitution reaction include, but are not limited to, triethylamine, N,N-diisopropylethylamine, potassium carbonate, sodium carbonate, sodium bicarbonate.

DETAILED DESCRIPTION: Unless stated to the contrary, the following terms used in the specification and claims have the following meanings.

"Alkyl" refers to saturated aliphatic hydrocarbon groups, including linear or branched alkyls; C1-C8 alkyl refers to alkyls containing 1-8 carbon atoms, for example, methyl, ethyl, n-propyl, isopropyl, n-butyl, isobutyl, tert-butyl, sec-butyl, n-amyl, 1, 1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1, 1, 2-trimethylpropyl, 1,1-dimethylbutyl, 1,2-dimethylbutyl, 2,2-dimethylbutyl, 1, 3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or its various branched isomers; preferably C1-C6 alkyl; more preferably C1-C4 alkyl. The alkyl may be substituted or unsubstituted.

"Cycloalkyl" means saturated or partially unsaturated monocyclic or polycyclic hydrocarbon substituents; "C3-C12 cycloalkyl" means cycloalkyl comprising 3 to 12 carbon atoms; "C3-C8 cycloalkyl" means cycloalkyl comprising 3 to 8 carbon atoms; "C5-C10 cycloalkyl" means cycloalkyl comprising 5 to 10 carbon atoms;
Non-limiting examples of monocycloalkyl comprise cyclopropyl, cyclobutyl, cyclopentyl, cyclopentenyl, cyclohexyl, cyclohexenyl, cyclohexadienyl, cycloheptyl, cyclohepttrienyl, cyclooctyl, etc., preferably cyclopropyl, cyclobutyl, cyclopentyl, cyclohexyl; preferably C3-C8 membered cycloalkyl; more preferably C3-C6 cycloalkyl.

Polycyclic cycloalkyls include spiro, fused and bridged cycloalkyls. "Spiro cycloalkyl" refers to a polycyclic group that shares a carbon atom (called a spiro atom) between monocyclyls. They can contain one or more double bonds, but none of the rings have a fully conjugated π electronic system. According to the number of spiro atoms shared between the rings, the spiro alkyl is divided into single spiro cycloalkyl, double spiro cycloalkyl or polyspiro cycloalkyl, preferably double spiro cycloalkyl. Non-limiting examples of spiro cycloalkyl include:

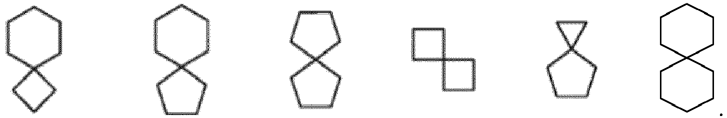

"Fused cycloalkyl" refers to a fully carbon polycyclic group in which each ring in the system shares an adjacent pair of carbon atoms with the other rings in the system, wherein one or more rings may contain one or more double bonds, but none of them has a fully conjugated π-electron system. According to the number of constituent rings, it can be divided into bicyclic, tricyclic, tetracyclic or polycyclic fused cycloalkyl, preferably bicyclic fused cycloalkyl. Non-limiting examples of fused cycloalkyl include:

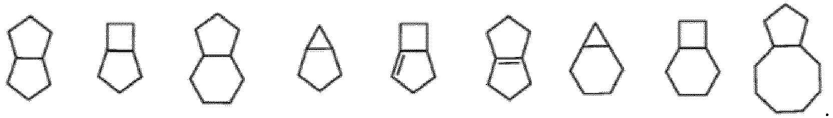

"Bridged cycloalkyl" refers to an all-carbon polycyclic group in which any two rings share two carbon atoms that are not directly connected, they can contain one or more double bonds, but no ring has a fully conjugated π electron system. According to the number of constituent rings, it can be divided into bicyclic, tricyclic, tetracyclic or polycyclic bridged cycloalkyl. Non-limiting examples of bridged cycloalkyl include:

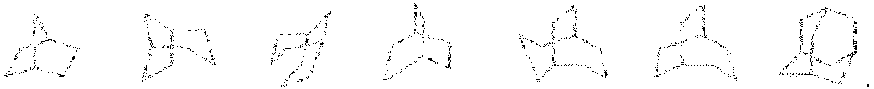

The cycloalkyl ring may be fused to an aryl, heteroaryl or heterocycloalkyl ring, wherein the ring connected to the parent structure is a cycloalkyl, and non-limiting examples include indanyl, tetrahydronaphthyl, benzocycloheptyl, etc. The cycloalkyl may be optionally substituted or unsubstituted.

"Heterocycloalkyl" refers to a saturated or partially unsaturated monocyclic or polycyclic hydrocarbon substituent, wherein one or more ring atoms are selected from nitrogen, oxygen, or S(O)r (wherein r is an integer of 0, 1 or 2), but do not contain the ring moieties of -O-O-, -O-S- or -S-S-, and the remaining ring atoms are carbon. "3-12-membered heterocycloalkyl" refers to a cyclyl containing 3 to 12 ring atoms, "5-10-membered heterocycloalkyl" refers to a cyclyl containing 5 to 10 ring atoms, and "3-8-membered heterocycloalkyl" refers to a cyclyl containing 3 to 8 ring atoms, preferably "3-12-membered heterocycloalkyl" containing 1-3 heteroatoms selected from N, O or S, more preferably a 3-12-membered heterocycloalkyl containing 1 or 2 N atoms.

Non-limiting examples of monocyclic heterocycloalkyl include pyrrolidyl, piperidinyl, piperazinyl, morpholinyl, thiomorpholinyl, homopiperazinyl, etc., preferably piperidinyl and piperazinyl.

Polycyclic heterocycloalkyls include spiro, fused and bridged heterocycloalkyls. "Spiro heterocycloalkyl" refers to a polycyclic heterocycloalkyl that shares one atom (called a spiro atom) between single rings, wherein one or more ring atoms are selected from nitrogen, oxygen or S(O)r (wherein r is an integer of 0, 1, 2), and the remaining ring atoms are carbon. They can contain one or more double bonds, but none of the rings have a fully conjugated π-electron system. According to the number of spiro atoms shared between the rings, the spiro cycloalkyl is divided into mono-spiro heterocycloalkyl, bis-spiro heterocycloalkyl or polyspiro heterocycloalkyl, preferably saturated "3-12 membered bis-spiro heterocycloalkyl" containing 1-3 heteroatoms selected from N, O or S; more preferably saturated "3-12 membered bis-spiro heterocycloalkyl" containing 1 or 2 N atoms. Non-limiting examples of spiro heterocycloalkyl include:

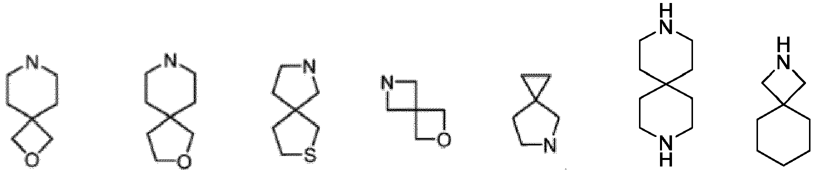

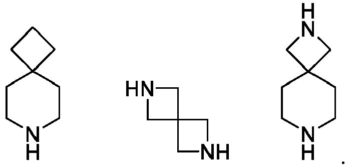

"Fused heterocycloalkyl" refers to a polycyclic heterocycloalkyl in which each ring in the system shares an adjacent pair of atoms with other rings in the system, wherein one or more rings may contain one or more double bonds, but none of them has a fully conjugated π-electron system, wherein one or more ring atoms are selected from nitrogen, oxygen, or S(O)r (wherein r is an integer of 0, 1, 2), the remaining ring atoms are carbon. According to the number of constituent rings, it can be divided into bicyclic, tricyclic, tetracyclic or polycyclic fused heterocycloalkyl, preferably "3-12-membered bicyclic fused heterocycloalkyl" containing 1 to 3 heteroatoms selected from N, O or S; more preferably saturated "3-12-membered bicyclic fused heterocycloalkyl" containing 1 or 2 N atoms. Non-limiting examples of fused heterocycloalkyl include:

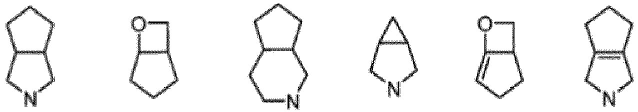

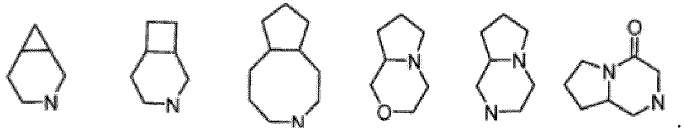

"Bridged heterocycloalkyl" refers to a polycyclic heterocycloalkyl in which any two rings share two atoms that are not directly connected. They can contain one or more double bonds, but no ring has a fully conjugated π electron system, wherein one or more ring atoms are selected from nitrogen, oxygen or S(O)r (wherein r is an integer of 0, 1, 2), and the remaining ring atoms are carbon. According to the number of constituent rings, it can be divided into bicyclic, tricyclic, tetracyclic or polycyclic bridged cycloalkyl. The non-limiting examples of bridged heterocycloalkyl include:

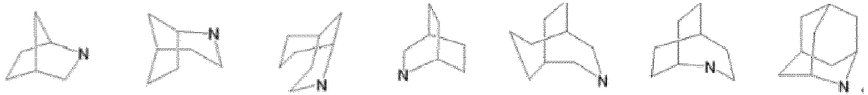

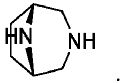

The heterocycloalkyl ring can be fused to the aryl, heteroaryl or cycloalkyl, wherein the ring connected with the parent structure is heterocycloalkyl, non-limiting examples include: 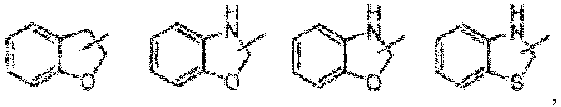 the heterocycloalkyl may be optionally substituted or unsubstituted.

"Aryl" refers to all-carbon monocyclyls or fused polycyclyls (that is, rings sharing adjacent pairs of carbon atoms) and polycyclyls having conjugated π-electron systems, and "6-10-membered aryl" refers to all-carbon aryls containing 6-10 carbons, such as phenyl and naphthyl; preferably phenyl. The aryl ring may be fused to the heteroaryl, heterocycloalkyl or cycloalkyl, wherein the ring connected with the parent structure is an aryl ring, and the non-limiting examples include: 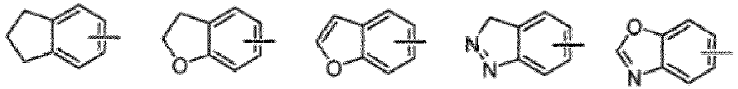 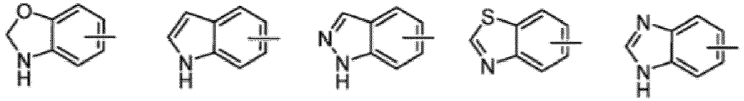 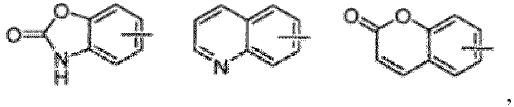 the aryl may be optionally substituted or unsubstituted.

"Heteroaryl" refers to a heteroaromatic system comprising 1 to 4 heteroatoms, the heteroatoms include nitrogen, oxygen or S(O)r (wherein r is an integer of 0, 1, 2), 5-6-membered heteroaryl refers to a heteroaromatic system containing 5 to 6 ring atoms, and 5-10-membered heteroaryl refers to a heteroaromatic system containing 5 to 10 ring atoms, preferably 5-6-membered heteroaryl; more preferably a 5-6-membered heteroaryl containing 1 or 2 N atoms; non-limiting examples include furyl, thienyl, pyridyl, pyrrolyl, N-alkylpyrrolyl, pyrimidyl, pyrazinyl, pyrazolyl, imidazolyl, triazolyl, tetrazolyl, etc.; preferably pyridyl. The heteroaryl ring may be fused to the aryl, heterocycloalkyl or cycloalkyl, wherein the ring connected with the parent structure is a heteroaryl, and the non-limiting examples include: 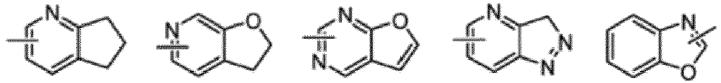 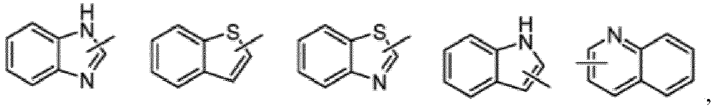 the heteroaryl may be optionally substituted or unsubstituted.

"Alkenyl" means an alkyl as defined above consisting of at least two carbon atoms and at least one carbon-carbon double bond, and "C2-8 alkenyl" means a linear or branched alkenyl containing 2-8 carbons, including but not limited to vinyl, 1-propenyl, 2-propenyl, 1-, 2- or 3-butenyl, etc., preferably "C2-6 alkenyl", more preferably "C2-4 alkenyl". The alkenyl may be substituted or unsubstituted.

"Alkynyl" refers to an alkyl as defined above consisting of at least two carbon atoms and at least one carbon-carbon triple bond, and "C2-8 alkynyl" refers to a linear or branched alkynyl containing 2-8 carbons, including but not limited to ethynyl, 1-propynyl, 2-propynyl, 1-, 2- or 3-butynyl, preferably "C2-6 alkynyl", more preferably "C2-4 alkynyl". The alkynyl may be substituted or unsubstituted.

"Subgroup" refers to a divalent, such as alkylene refers to divalent alkyl, alkenylene refers to divalent alkenyl, alkynylene refers to divalent alkynyl, cycloalkylene to divalent cycloalkyl, heterocycloalkylene to divalent heterocycloalkyl, arylene to divalent aryl, heteroarylene to divalent heteroaryl, and the alkyl, alkenyl, alkynyl, cycloalkyl, heterocycloalkyl, aryl, heteroaryl as defined above, the subgroups may be optionally substituted or unsubstituted.

"Alkoxy" refers to -O-(alkyl), wherein the alkyl is as defined above. "C1-C8 alkoxy" refers to an alkyloxy containing 1 to 8 carbons. Non-limiting examples include methoxy, ethoxy, propoxy, butoxy, etc. The alkoxy may be optionally substituted or unsubstituted.

"Cycloalkoxy" refers to -O-(unsubstituted cycloalkyl), wherein the cycloalkyl is as defined above. C3-C8 cycloalkoxy refers to cycloalkyloxys containing 3 to 8 carbons. Non-limiting examples include cyclopropoxy, cyclobutoxy, cyclopentoxy, cyclohexoxy, etc. The cycloalkoxy may be optionally substituted or unsubstituted.

"Haloalkyl" refers to an alkyl substituted by one or more fluorine, chlorine, bromine or iodine atoms, wherein the alkyl is as defined above, and non-limiting examples include difluoromethyl, dichloromethyl, dibromomethyl, trifluoromethyl, trichloromethyl, tribromomethyl, etc.

"Haloalkoxy" refers to a group in which hydrogen on an alkyl is substituted by one or more fluorine, chlorine, bromine or iodine atoms, wherein the alkoxy is as defined above. For example, difluoromethoxy, dichloromethoxy, dibromomethoxy, trifluoromethoxy, trichloromethoxy, tribromomethoxy, etc.

"Hydroxyalkyl" refers to an alkyl optionally substituted by one or more -OH, wherein the alkyl is as defined above, and non-limiting examples include hydroxymethyl, hydroxyethyl, hydroxypropyl, hydroxyisopropyl.

"Aminoalkyl" refers to an alkyl optionally substituted by one or more -NH₂, wherein the alkyl is as defined above, and non-limiting examples include aminomethyl, aminoethyl, aminopropyl, aminoisopropyl.

"Amino" and "amine" are recognized in the art and refer to substituted or unsubstituted ammonia.

"Carboxyl" or "carboxylic acid" refers to -COOH.

"Oxo" refers to =O.

"Halogen" refers to fluorine, chlorine, bromine or iodine.

"Cyano" refers to -CN.

"Hydroxyl" refers to -OH.

"Raney-Ni" refers to Raney nickel (hydrogen active catalytic reducing agent).

"Dess-martin" refers to 1,1,1-triacetoxy-1,1-dihydro-1,2-benziodoxol-3-(1H)-one.

"PdCl₂(dppf)" refers to [1,1-bis (diphenylphosphorus) ferrocene] palladium dichloride.

"T3P" refers to 2,4, 6-tripropyl-1,3,5,2,4,6-trioxytriphosphate-2,4,6-trioxide.

"HATU" refers to 2-(7-azabenzotriazole)-N,N,N',N'-tetramethylurea hexafluorophosphate.

"DPBS" refers to Dulbecco's phosphate buffer saline.

"PBS" refers to phosphate buffer saline.

"SDS-PAGE" refers to sodium dodecyl sulfate-polyacrylamide gel electrophoresis.

"PVDF" refers to polyvinylidene fluoride.

"More" independently refers to 1, 2, 3, 4, 5, 6, 7, 8, 9, or 10.

"Optional" means that a subsequently described event or environment may, but does not have to, occur, and that description includes the place where the event or environment occurs or does not occur. For example, "a heterocycloalkyl optionally substituted by an alkyl" means that an alkyl may, but does not have to be present, and the description includes the case where the heterocycloalkyl is substituted by an alkyl and the case where the heterocycloalkyl is not substituted by an alkyl.

"Substituted" means that one or more hydrogen atoms, preferably at most 5, more preferably 1 to 3 hydrogen atoms are substituted independently of each other by a corresponding number of substituents. It goes without saying that the substituents are only in their possible chemical positions, and those skilled in the art can determine (experimentally or theoretically) possible or impossible substitutions without too much effort. For example, amino or hydroxyls with free hydrogen may be unstable when combined with carbon atoms with unsaturated (e. g. olefinic) bonds.

"Pharmaceutical composition" means a mixture comprising one or more compounds described herein or their physiologically/pharmaceutically acceptable salts or prodrugs with other chemical components, as well as other components such as physiologically/pharmaceutically acceptable carriers and excipients. The purpose of the pharmaceutical composition is to promote the administration of organisms, facilitate the absorption of active ingredients and thus exert biological activity.

The present invention also provides pharmaceutically acceptable salts of compounds of formula (I). The term "pharmaceutically acceptable salt" refers to an acid addition salt or a base addition salt of a compound of the present invention that is relatively non-toxic. The acid addition salts are salts of the compounds of formula (I) of the present invention and suitable inorganic or organic acids, these salts can be prepared during the final separation and purification of the compounds, or can be prepared by reacting the purified compounds of formula (I) in their free base form with suitable organic or inorganic acids. Representative acid addition salts include hydrobromate, hydrochloride, sulfate, bisulfate, sulfite, acetate, oxalate, valerate, oleate, palmitic acid, stearate, lauroleate, borate, benzoate, lactate, phosphate, hydrophosphate, carbonate, bicarbonate, toluate, citrate, maleate, fumarate, succinate, tartrate, benzoate, mesylate, p-toluene sulfonate, gluconate, lactobionate and lauryl sulfonate, etc. The base addition salt is a salt formed by a compound of formula (I) and a suitable inorganic or organic base, including, for example, a salt formed with alkali metals, alkaline earth metals, and quaternary ammonium cations, such as sodium salt, lithium salt, potassium salt, calcium salt, magnesium salt, tetramethylquaternary ammonium salt, tetraethyl quaternary ammonium salt, etc.; amine salt includes salts formed with ammonia (NH₃), primary amine, secondary amine or tertiary amine, such as methylamine salt, dimethylamine salt, trimethylamine salt, triethylamine salt, ethylamine salt, etc.

The compounds of the invention, or pharmaceutically acceptable salts thereof, can be administered to mammals, including humans, orally, rectally, parenterally (intravenous, intramuscular or subcutaneous), topically (powder, ointment or drops), or intratumorally.

The dosage of the compound of the present invention may be about 0.05-300mg/kg body weight/day, preferably 10-300mg/kg body weight/day, more preferably 10-150mg/kg body weight/day.

The compounds of the present invention, or pharmaceutically acceptable salts thereof, may be formulated as solid dosage forms for oral administration, including, but not limited to, capsules, tablets, pills, powders, granules, and the like. In these solid dosage forms, the active ingredient, i.e. the compound of formula (I) of the present invention, is mixed with at least one conventional inert excipient (or carrier), such as sodium citrate or dicalcium phosphate, or mixed with any of the following components: (1) fillers or compatibilizer, for example, starch, lactose, sucrose, glucose, mannitol and silicic acid, etc.; (2) binders, for example, hydroxymethyl cellulose, alginate, gelatin, polyvinylpyrrolidone, sucrose and arabic gum, etc.; (3) humectants, for example, glycerol, etc; (4) disintegrating agents, for example, agar, calcium carbonate, potato starch or tapioca starch, alginic acid, certain composite silicates, and sodium carbonate, etc; (5) dissolution-retarding agents, for example, paraffin, etc; (6) absorption accelerators, for example, quaternary ammonium compounds, etc; (7) wetting agents, for example, cetyl alcohol and glyceryl monostearate; (8) adsorbents, for example, kaolin, etc; and (9) lubricants, for example, talc, calcium stearate, magnesium stearate, solid polyethylene glycol, sodium lauryl sulfate, or the mixture thereof. Buffering agents may also be included in capsules, tablets and pills.

The solid dosage forms such as tablets, sugar pills, capsules, pills and granules can be coated or microencapsulated with coating and shell materials such as enteric coatings and other materials known in the art. They may contain opaque agents, and the release of the active ingredient in such a composition may be released in a delayed manner in a part of the digestive tract. Examples of embedding components that can be employed are polymeric materials and waxy materials. If necessary, the active ingredient may also form a microcapsule form with one or more of the excipients described above.

The compounds of the present invention, or pharmaceutically acceptable salts thereof, may be formulated as liquid dosage forms for oral administration, including, but not limited to, pharmaceutically acceptable emulsions, solutions, suspensions, syrups, tinctures, and the like. In addition to the compounds of formula (I) or pharmaceutically acceptable salts thereof as active ingredients, liquid dosage forms may contain inert diluents such as water and other solvents, solubilizers and emulsifiers, such as ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil or a mixture of these substances as conventionally used in the art. In addition to these inert diluents, the liquid dosage forms of the present invention may also contain conventional auxiliaries such as wetting agents, emulsifiers and suspending agents, sweeteners, flavoring agents and spices.

The suspending agent includes, for example, ethoxylated isooctadecanol, polyoxyethylene sorbitol and dehydrated sorbitol esters, microcrystalline cellulose, aluminum methanol and agar, or a mixture of these substances.

The compounds of the present invention, or pharmaceutically acceptable salts thereof, may be formulated as dosage forms for parenteral injection including, but not limited to, physiologically acceptable sterile aqueous or anhydrous solutions, dispersions, suspensions or emulsions, and sterile powders for re-dissolution into sterile injectable solutions or dispersions. Suitable carriers, diluents, solvents or excipients include water, ethanol, polyols and suitable mixtures thereof.

The compounds of the present invention, or pharmaceutically acceptable salts thereof, may also be formulated as dosage forms for topical administration, including, for example, ointments, powders, suppositories, drops, propellants, inhalants, and the like. The compounds of formula (I) of the invention, or pharmaceutically acceptable salts thereof, as active ingredient, are mixed with a physiologically acceptable carrier and optionally a preservative, a buffer, or, if necessary, a propellant as may be required under sterile conditions.

The present invention also provides a pharmaceutical composition comprising a compound of formula (I) of the present invention or a pharmaceutically acceptable salt thereof as active ingredient, and a pharmaceutically acceptable carrier, excipient or diluent. In the preparation of pharmaceutical compositions, a compound of formula (I) or a pharmaceutically acceptable salt thereof is usually mixed with a pharmaceutically acceptable carrier, excipient or diluent.

The composition of the present invention can be formulated as a conventional pharmaceutical preparation according to the conventional preparation method. For example, tablets, pills, capsules, powders, granules, emulsions, suspensions, dispersions, solutions, syrups, elixirs, ointments, drops, suppositories, inhalants, propellants, etc.

The compound of the present invention or its pharmaceutically acceptable salt may be administered alone, or (if necessary) in combination with other pharmaceutically acceptable therapeutic agents, such as in combination with other anti-tumor drugs, anti-inflammatory drugs or autoimmune drugs. The ingredients to be combined may be administered simultaneously or sequentially, in the form of a single formulation or in the form of a different formulation. The combination may include not only a combination of a compound of the present invention and one other active agent, but also a combination of a compound of the present invention and two or more other active agents.

The present invention proves that the compound of formula I of the present invention can effectively bind to the IRAK4 target protein or produce an inhibitory effect through the IRAK4 kinase activity test experiment, and the compound of formula I of the present invention can effectively and specifically degrade the IRAK4 protein in THP-1 cells by Western-Blot. It is proved by AlphaLISA detection method that the compound of formula I of the present invention can effectively inhibit the production of IL-6 by immune cells, and the compound of the present invention has good degradation selectivity, and the compound of formula I of the present invention, and/or its stereoisomers, enantiomers, diastereomers, deuterates, hydrates, solvates, metabolites, prodrugs and/or pharmaceutically acceptable salts can effectively and selectively degrade IRAK4 protein, so as to achieve the effect of preventing or treating diseases or conditions related to IRAK4.

### Examples

Hereinafter, the present invention will be described in further detail and completely with reference to the examples, but the present invention is not in any way limited to the contents of the examples. The starting materials in the examples of the present invention are known and can be commercially available or can be synthesized using or in accordance with methods known in the art. In the absence of special instructions, in the present examples, the experimental method that does not specify the specific conditions, usually in accordance with the conventional conditions, or in accordance with the conditions suggested by raw material or commodity manufacturer.

### I Examples for compounds preparation

### Intermediate 1: 2-Fluoro-4-methoxy-5-nitrobenzaldehyde

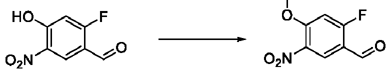

Cesium carbonate (3.5g,10.8mmol) and potassium iodide (1.85g,13.0mmol) were added to a solution of 2-fluoro-4-hydroxy-5-nitrobenzaldehyde (2g,10.8mmol) in N,N-dimethylformamide (20ml). The reaction solution was stirred for 10 hours at room temperature, water (30ml) was added, extracted with ethyl acetate for three times, the organic phase was collected, washed with saturated saline, dried over anhydrous sodium sulfate, filtered, concentrated under reduced pressure, and the concentrate was purified by column to obtain 1.6g of the target product 2-fluoro-4 methoxy-5-nitrobenzaldehyde.

LC-MS: (ES, *m*/*z*): [M+H] ⁺= 200.1

### Intermediate 2: 2-Fluoro-5-nitro-4-(trifluoromethoxy) benzaldehyde

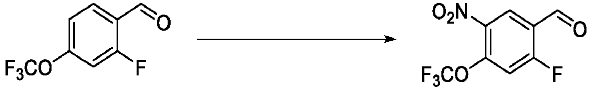

Nitric acid (1.0mL,65%) was added dropwise to a solution of 2-fluoro-4-(trifluoromethoxy) benzaldehyde (1.0g,4.8mmol) in sulfuric acid (5ml) while keeping the temperature below -10°C. After dripping, the solution was poured into ice water (20mL) and stirred for 10 minutes. Filtered, solid was washed with water (10mL × 3), and dried under reduced pressure to obtain 0.9g of crude target product. The crude product can be used for the next step without further purification.

LC-MS: (ES, *m*/*z*): [M+H] ⁺= 254.0

### Intermediate 3: N-(6-methoxy-2-(piperidin-4-yl)-2H-indazol-5-yl)-6-(trifluoromethyl) pyridinecarboxamide

### Step 1: Preparation of 2-azido-4-methoxy-5-nitrobenzaldehyde

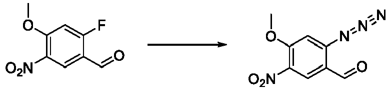

Sodium azide (1.06g,16.1mmol) was added to a solution of 2-fluoro-4 methoxy-5-nitrobenzaldehyde (1.6g,8.04mmol) in dimethyl sulfoxide (20ml), stirred at room temperature for 2 hours, then the reaction solution was poured into ice water, extracted with ethyl acetate, the organic phase was collected, washed with saturated saline, dried over anhydrous sodium sulfate, and 1.7g crude product was obtained by concentration, which was directly used in the next reaction without purification.

### Step 2: Preparation of tert-butyl 4-(6-methoxy-5-nitro-2H-indazol-2-yl) piperidine-1-carboxylate

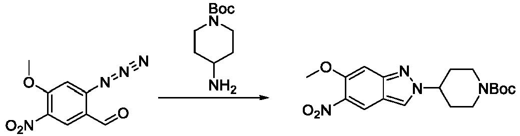

The solution of 2-azido-4-methoxy-5-nitrobenzaldehyde (1.7g,7.65mmol) and tert-butyl 4-aminopiperidine-1-carboxylate (1.53g,7.65mmol) in toluene (20ml) was stirred and reacted at 100 °C for 2 hours, cooled to room temperature, the reaction solution was concentrated, 50ml of water was added, extracted with ethyl acetate, the organic phase was collected, washed with saturated saline, dried over anhydrous sodium sulfate, the organic phase was concentrated, and the concentrate was purified by column to obtain 1.72g of tert-butyl 4-(6-methoxy-5-nitro-2H-indazol-2-yl) piperidin-1-carboxylate.

LC-MS: (ES, *m*/*z*): [M+H] ⁺= 377.2

### Step 3: Preparation of tert-butyl 4-(5-amino-6-methoxy-2H-indazol-2-yl) piperidine-1-carboxylate

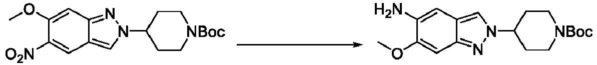

Iron powder (2.85g,50.9mmol) and ammonium chloride (0.13g,2.3mmol) were added to a solution of tert-butyl 4-(6-methoxy-5-nitro-2H-indazol-2-yl) piperidin-1-carboxylate (1.7g,4.5mmol) in ethanol (20ml), and the reaction solution was reacted at 90 °C for 2 hours. After the reaction solution was cooled to room temperature, it was filtered and concentrated to obtain 2.01g of crude target product, which was directly used for the next reaction.

LC-MS: (ES, *m*/*z*): [M+H] ⁺= 347.3

### Step 4: Preparation of tert-butyl 4-(6-methoxy-5-(6-(trifluoromethyl) pyridine-2-amido)-2H-indazol-2-yl) piperidine-1-carboxylate

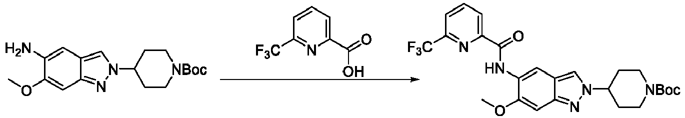

6-(Trifluoromethyl) pyridine-2-carboxylic acid (940mg,4.9mmol) and N,N-diisopropylethylamine (1.9g,14.7mmol) were added to a solution of tert-butyl 4-(5-amino-6-methoxy-2H-indazol-2-yl) piperidine -1-carboxylate (1.7g,4.9mmol) in tetrahydrofuran, then T₃P (1.5g) was added, and the reaction solution was stirred for 2 hours at room temperature. After concentrating the reaction solution, water was added, extracted with ethyl acetate for three times, and the organic phase was collected, washed with saturated saline, dried over anhydrous sodium sulfate and concentrated. The concentrate was purified by column to obtain 1.78g of the target product tert-butyl 4-(6-methoxy-5-(6-(trifluoromethyl) pyridine-2-amide)-2H-indazol-2-yl) piperidine-1-carboxylate.

LC-MS: (ES, *m*/*z*): [M+H] ⁺= 520.2

### Step 5: Preparation of N-(6-methoxy-2-(piperidin-4-yl)-2H-indazol-5-yl)-6-(trifluoromethyl) pyridinecarboxamide

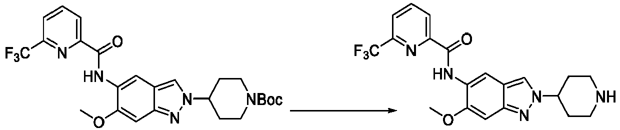

Trifluoroacetic acid (1ml) was added to a solution of tert-butyl 4-(6-methoxy-5-(6-(trifluoromethyl) pyridine-2-amido)-2H-indazol-2-yl) piperidine-1-carboxylate (435 mg,0.795 mmol) in dichloromethane. The reaction solution was stirred at room temperature for 1h. The solvent was removed by concentration under reduced pressure to obtain the target crude product (500mg). The crude product was used directly for the next step.

LC-MS: (ES, *m*/*z*): [M+H] ⁺= 420.2

The following intermediates were prepared with reference to the method of intermediate 3.

| Interme diate | Structure | Name | LC-MS |
|---|---|---|---|
| Interme diate 4 | 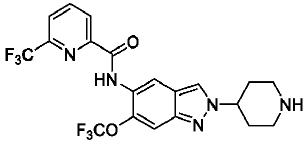 | N-(2-(piperidin-4-yl)-6-(trifluoromethoxy)-2H-indazol-5-yl)-6-(trifluoromethyl) pyridinecarboxamide | (ESI,*m*/*z*): [M+H]⁺= 474.1. |

### Intermediate 5:N-(6-(2-hydroxyprop-2-yl)-2-(piperidin-4-yl)-2H-indazol-5-yl)-6-(trifluoromethyl) pyridinecarboxamide

### Step 1: Preparation of methyl 5-nitro-1H-indazole-6-carboxylate

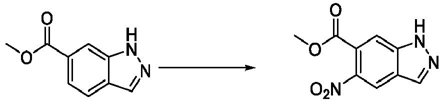

At -10°C, concentrated nitric acid (15mL) was slowly added to a solution of methyl 1H-indazole-6-carboxylate (9.2 g,0.052mol) in concentrated sulfuric acid (20mL). The reaction solution was stirred at -10°C for 2h. The reaction solution was poured into ice water, and the solid was filtered to obtain 10.8g of the target crude product as a white solid. The crude product was used directly for the next step.

LC-MS: (ES, *m*/*z*): [M+H] ⁺= 222.1

### Step 2: Preparation of methyl 2-(1-(tert-butoxycarbonyl) piperidin-4-yl)-5-nitro-2H-indazole-6-carboxylate

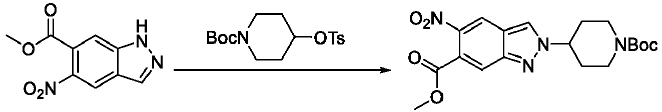

Potassium carbonate (11.1g,81.3mmol) was added to a solution of methyl 5-nitro-1H-indazol-6-carboxylate (6g,27.12 mmol) and tert-butyl 4-(p-toluenesuloyloxy) piperidine-1-carboxylate (15.3g,43.5 mmol) in N,N-dimethylformamide (100mL). The reaction solution was stirred at 100 °C for 10h. The reaction solution was cooled to room temperature, water (50ml) and ethyl acetate (100ml) were added, the water layer was separated and extracted with ethyl acetate (100ml × 2). The organic layers were combined, washed with saline (100mL × 2), dried over anhydrous sodium sulfate, filtered and concentrated under reduced pressure. The concentrate was purified by column chromatography to obtain 2.01g of target product as a yellow solid.

LC-MS: (ES, *m*/*z*): [M+H]⁺=405.2

### Step 3: Preparation of methyl 5-amino-2-(1-(tert-butoxycarbonyl) piperidin-4-yl)-2H-indazole-6-carboxylate

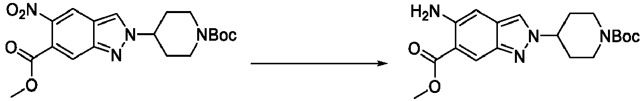

Iron powder (2.85g,50.9mmol) and ammonium chloride (0.13g,2.3mmol) were added to a solution of methyl 2-(1-(tert-butoxycarbonyl) piperidin-4-yl)-5-nitro-2H-indazole-6-carboxylate (1.7g,4.5 mmol) in ethanol (20 ml). The reaction solution was stirred at 90°C for 2h. The reaction solution was cooled to room temperature, filtered, and the filtrate was concentrated under reduced pressure to obtain 2.01g of the crude target product as an oil. The crude product was used directly for the next step.

LC-MS: (ES, *m*/*z*): [M+H]⁺=375.3

### Step 4: methyl 2-(1-(tert-butoxycarbonyl) piperidin-4-yl)-5-(6-(trifluoromethyl) pyridine carboxamide)-2H-indazole-6-carboxylate

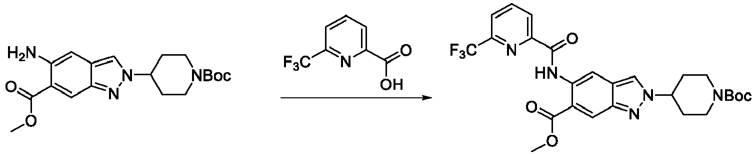

T₃P(650 mg) was added to a mixture of methyl 5-amino -2-(1-(tert-butoxycarbonyl) piperidin-4-yl)-2H-indazole-6-carboxylate (170 mg,0.45 mmol), 6-(trifluoromethyl) pyridine-2-carboxylic acid (86.8mg,0.45 mmol) and N,N-diisopropylethylamine(88 mg, 0.68 mmol) in tetrahydrofuran. The reaction solution was stirred at room temperature for 2 hours. The solvent was removed by concentration under reduced pressure. Water (20mL) and ethyl acetate (20mL) were added. The water layer was separated and extracted with ethyl acetate (20ml × 2). The organic layers were combined, washed with saline (20mL × 2), dried over anhydrous sodium sulfate, filtered and concentrated under reduced pressure. The concentrate was purified by column chromatography to obtain 150mg of target product as a yellow solid.

LC-MS: (ES, *m*/*z*): [M+H]⁺=548.3

### Step 5: Preparation of tert-butyl 4-(6-(2-hydroxyprop-2-yl)-5-(6-(trifluoromethyl) pyridinecarboxamido)-2H-indazol-2-yl) piperidine-1-carboxylate

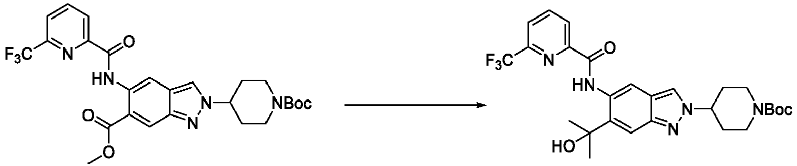

Lithium chloride (192mg,4.57mmol) was added to a solution of methyl 2-(1-(tert-butoxycarbonyl) piperidin-4-yl)-5-(6-(trifluoromethyl) pyridinecarboxamido)-2H-indazole-6-carboxylate (500 mg,0.91 mmol) in tetrahydrofuran. The reaction solution was cooled to 0°C, and then tetrahydrofuran methylmagnesium bromide (3.04ml,1M) was added. The reaction solution was stirred at room temperature for 12h, quenched with ammonium chloride aqueous solution, and water (30mL) and ethyl acetate (50mL) were added. The water layers were separated and extracted with ethyl acetate (50ml × 2), the organic layers were combined, washed with saline (50ml × 2), dried over anhydrous sodium sulfate, filtered and concentrated under reduced pressure, and the concentrate was purified by column chromatography to obtain 435 mg of target product as a white solid.

LC-MS: (ES, *m*/*z*): [M+H]⁺=548.3

### Step 6: Preparation of N-(6-(2-hydroxyprop-2-yl)-2-(piperidin-4-yl)-2H-indazol-5-yl)-6-(trifluoromethyl) pyridinecarboxamide

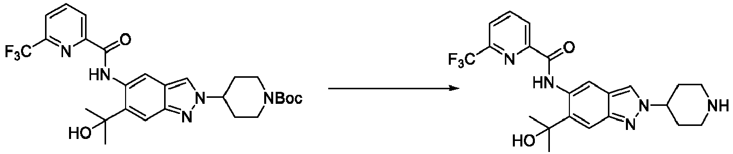

A mixture of tert-butyl 4-(6-(2-hydroxyprop-2-yl)-5-(6-(trifluoromethyl) pyridineformamido)-2H-indazol-2-yl) piperidine-1-carboxylate (435 mg, 0.79mmol) in dichloromethane (3mL) and trifluoroacetic acid (1mL) was stirred and reacted at room temperature for 1h, and the solvent was removed by concentration under reduced pressure, 500mg of target crude product was obtained as a yellow oil. The crude product was used directly for the next step.

LC-MS: (ES, *m*/*z*): [M+H]⁺=448.2

### Intermediate 6: N-(6-(2-hydroxyprop-2-yl)-1H-indazol-5-yl)-6-(trifluoromethyl) pyridinecarboxamide

### Step 1:methyl 5-amino-1H-indazole-6-carboxylate

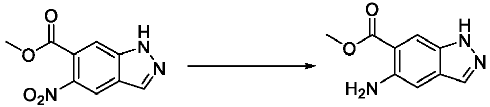

Palladium (800 mg, 10% w.t) was added to a solution of methyl 5-nitro-1 H-indazole-6-carboxylate (5.37g, 0.024 mol) in ethyl acetate (500 mL). The reaction mixture was stirred for 16 hours at room temperature in a hydrogen atmosphere. The solids were filtered and washed with ethyl acetate (100mL × 2). The filtrate was concentrated under reduced pressure to obtain 4.45g of crude target product as a brown solid. The crude product was used directly for the next reaction.

LC-MS: (ES, *m*/*z*): [M+H]⁺= 192.2.

### Step 2:methyl 5-(6-(trifluoromethyl) pyridine carboxamide)-1H-indazole-6-carboxylate

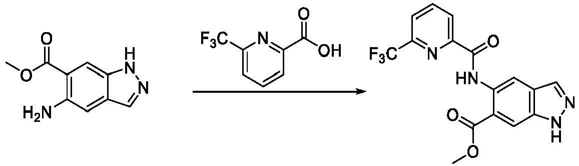

N,N-diisopropylethylamine(4.5g, 0.0345 mol) was added to a solution of methyl 5-amino-1H-indazole-6-carboxylate (4.4g,0.023 mol) and 6-(trifluoromethyl) picolinic acid (4.4g,0.023 mol) in tetrahydrofuran (50 ml), the mixture was cooled to 0 °C, and then T3P(18.0g, 0.0345 mol) was added in batches, and the reaction mixture was stirred at room temperature for 2 hours. The solvent was removed by concentration under reduced pressure, water (50ml) and ethyl acetate (100ml) were added, and the water layer was separated and extracted with ethyl acetate (100ml × 2). The organic layer was collected, washed with saturated saline (100mL × 2), dried over anhydrous sodium sulfate, filtered and concentrated under reduced pressure. The concentrate was purified by column chromatography to obtain 7.87g of target product as a yellow solid.

LC-MS: (ES, *m*/*z*): [M+H]⁺=365.1.

### Step 3: N-(6-(2-hydroxyprop-2-yl)-1H-indazol-5-yl)-6-(trifluoromethyl) pyridine carboxamide

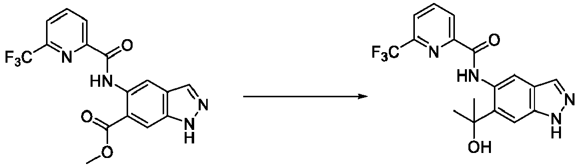

The solution of methyl 5-(6-(trifluoromethyl) pyridinecarboxamido)-1H-indazole-6-carboxylate (1g,0.002mol) and lithium chloride (576mg,0.013mol) in tetrahydrofuran (30mL) was cooled to -10°C, and then methylmagnesium bromide (9.1mL,0.027mol) was added under nitrogen protection. After the reaction mixture was stirred at room temperature for 8 hours, it was cooled to 0°C, and saturated ammonium chloride aqueous solution (50mL), water (50ml) and ethyl acetate (100ml) were added to quench the solution. The water layer was separated and extracted with ethyl acetate (100ml × 2). The organic layer was collected, washed with saturated salt water (100mL × 2), dried over anhydrous sodium sulfate, filtered and concentrated under reduced pressure. The concentrate was purified by column chromatography to obtain 532mg of target product as a yellow solid.

LC-MS: (ES, *m*/*z*): [M+H]⁺=365.1.

### Intermediate 7: 3-(2, 4-dioxotetrahydropyrimidin-1(2H)-yl)-4-methoxybenzoic acid

### Step 1: Preparation of 3-(2,4-dioxotetrahydropyrimidin-1 (2H)-yl)-4-methoxybenzoic acid

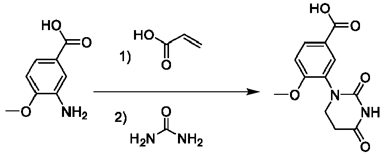

3-Amino-4-methoxybenzoic acid (5.0g,2.93mmol) was suspended in acrylic acid (8.05mL,117mmol) and stirred at 100 °C for 3 h, then the reaction solution was stirred and cooled to room temperature. Acetic acid (33ml) was added, the stirred suspension was heated at 100 °C for 10 minutes, urea (11.00g, 183mmol) was added, and the reaction solution was stirred at 120 °C overnight. The reaction solution was added to a mixture of ice water and concentrated hydrochloric acid (37%), stirred, and the obtained suspension was stored overnight in a refrigerator at 5°C, then filtered, and the solid was washed with water and dried to obtain the solid. The solid was ground in hydrochloric acid solution (0.05M), filtered, washed with methyl tert-butyl ether, and dried under reduced pressure at 40 °C to obtain 6.29g of target product.

¹H NMR (400 MHz, DMSO) δ 12.70 (s, 1H), 10.34 (s, 1H), 7.92 (dd, J = 8.6, 2.2 Hz, 1H), 7.83 (d, J = 2.2 Hz, 1H), 7.21 (d, J = 8.8 Hz, 1H), 3.94 - 3.82 (m, 3H), 3.60 (t, J = 6.7 Hz, 2H), 2.69 (s, 2H).

The following intermediates were prepared with reference to the method of intermediate 7:

| Intermediate | Structure | Name | LC-MS |
|---|---|---|---|
| Intermediate 8 | 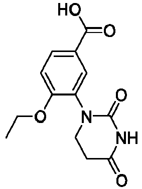 | 3-(2, 4-dioxotetrahydropyrimidi n-1(2*H*)-yl)-4-ethoxybenzoic acid | (ES,*m*/*z*):[M+H]⁺=279.1 |
| Intermediate 9 | 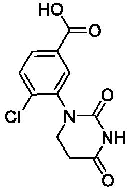 | 4-Chloro-3-(2, 4-dioxotetrahydropyrimidi n-1 (2H)-yl) benzoic acid | (ES,*m*/*z*):[M+H]⁺=269.0 |
| Intermediate 10 | 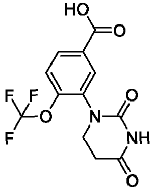 | 3-(2, 4-dioxotetrahydropyrimidi n-1 (2H)-yl)-4-(trifluoromethoxy) benzoic acid | (ES,*m*/*z*):[M+H]⁺=319.1 |
| Intermediate 11 | 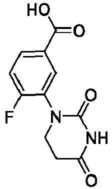 | 3-(2, 4-dioxotetrahydropyrimidi n-1 (2H)-yl)-4-fluorobenzoic acid | (ES,*m*/*z*):[M+H]⁺=253.0 |
| Intermediate 12 | 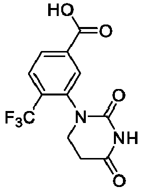 | 3-(2, 4-dioxotetrahydropyrimidi n-1 (2H)-yl)-4-(trifluoromethyl) benzoic acid | (ES,*m*/*z*):[M+H]⁺=303.0 |
| Intermediate 13 | 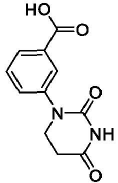 | 3-(2, 4-dioxotetrahydropyrimidi n-1 (2H)-yl) benzoic acid | (ES,*m*/*z*):[M+H]⁺=235.1 |

### Intermediate 14: 3-(2, 6-dioxopiperidin-3-yl)-4-fluorobenzoic acid

### Step 1: Preparation of 2, 6-bis (benzyloxy) pyridine

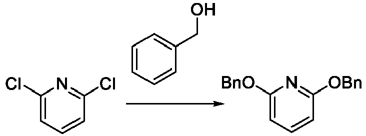

Benzyl alcohol (109.6g,1.01mol) was added to a solution of sodium hydride (60% mineral oil mixture, 54.0g,1.35mol) in N,N-dimethylformamide (600mL) at 0 °C. After stirring for 30 minutes, 2,6-dichloropyridine (50.0g,0.338mol) was added. The reaction mixture was heated to 80°C and stirred overnight, and after cooling to room temperature, the reaction mixture was quenched with ice water (1000mL). The solid was filtered and dried to obtain 75.9g of target product.

¹H NMR (DMSO-*d*₆, 400 MHz): *δ* 7.68 (t, *J =* 8.4 Hz, 1H), 7.48-7.36 (m, 10H), 6.45 (d, *J* = 8.4 Hz, 2H), 5.38 (s, 4H).

LC-MS: (ES, *m*/*z*): [M+H]⁺=292.2.

### Step 2: Preparation of 2,6-bis (benzyloxy)-3-bromopyridine

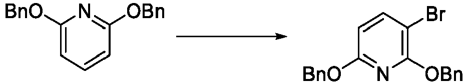

N-bromosuccinimide(41.7g,0.23mol) was added to a solution of 2,6-bis (benzyloxy) pyridine (75.9g,0.26mol) in acetonitrile (600mL). The reaction mixture was stirred at 80°C for 2 hours. After the reaction was completed, the reaction mixture was concentrated under reduced pressure. The concentrate was diluted with ethyl acetate (500mL) and washed with water and saturated saline. The organic layer was collected and dried over anhydrous sodium sulfate, filtered and concentrated. The concentrate was purified by column chromatography to obtain 75.0g of target product.

¹H NMR (DMSO-*d*₆, 400 MHz): *δ* 7.88 (d, *J* = 8.4 Hz, 1H), 7.45-7.37 (m, 10H), 6.44 (d, *J* = 8.4 Hz, 1H), 5.42 (s, 2H), 5.33 (s, 2H).

LC-MS: (ES, *m*/*z*): [M+H]⁺=370.1.

### Step 3: Preparation of 3-(2, 6-bis (benzyloxy) pyridin-3-yl)-4-fluorobenzoic acid

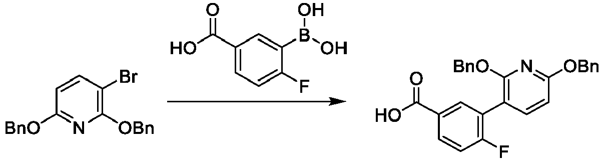

PdCl₂(dppf)-chloroform complex (612 mg,0.84 mmol) and sodium carbonate (1.77g,16.74 mmol) were added to a mixture of 2, 6-bis (benzyloxy)-3-bromopyridine (3.1g,8.37 mmol) and 3-boro-4-fluorobenzoic acid (1.85g,10.05 mmol) in dioxane (31 mL) and water (7.5 mL). The reaction mixture was heated to 90°C under nitrogen protection and the reaction was maintained for 16 hours. After the reaction was completed, 15% hydrochloric acid solution (20mL) was added, extracted with ethyl acetate (100mL × 3), washed with saturated saline (50mL), dried over anhydrous sodium sulfate, concentrated under reduced pressure, and the concentrate was purified by column chromatography to obtain 1.50g of target product.

LC-MS: (ES, *m*/*z*): [M+H]⁺= 430.2

### step 4: Preparation of 3-(2, 6-dioxopiperidin-3-yl)-4-fluorobenzoic acid

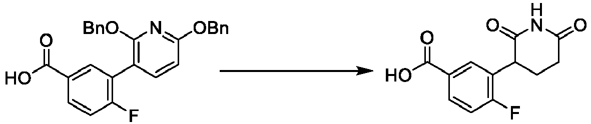

Palladium/carbon (10%,120 mg) was added to a solution of 3-(2,6-bis (benzyloxy) pyridin-3-yl)-4-fluorobenzoic acid (1.50g,3.49 mmol) in methanol (10 mL). At room temperature, the reaction mixture was stirred in a hydrogen atmosphere for 48 hours. After the reaction was completed, the reaction mixture was filtered through diatomite, and the filtrate was concentrated under reduced pressure. The concentrate was purified to obtain 600mg of target product.

LC-MS: (ES, *m*/*z*): [M+H]⁺= 252.1

¹H NMR (400 MHz, DMSO-d₆) δ 13.04 (s, 1H), 10.92 (s, 1H), 7.91 -7.95 (m, 2H), 7.30-7.34 (m, 1H), 4.15-4.19 (m, 1H), 2.70-2.79 (m, 1H), 2.53-2.57 (m, 1H), 2.23-2.27 (m, 1H), 2.01-2.07 (m, 1H).

### Intermediate 15: pentafluorophenyl 3-(2, 4-dioxotetrahydropyrimidin -1(2H)-yl)-4-methoxybenzoate

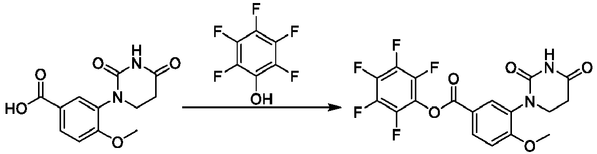

A mixture of 3-(2, 4-dioxotetrahydropyrimidin-1(2H)-yl)-4-methoxybenzoic acid (2.0g,7.58 mmol), 2,3,4,5, 6-pentafluorophenol (1.67g,9.09 mmol) and N,N'-dicyclohexylcarbimide(1.87g,9.09 mmol) in N,N-dimethylformamide (20mL) was stirred for 3h at room temperature. The reaction solution was poured into water (200mL) and stirred for 0.5h. The solution was extracted with ethyl acetate (3 × 200mL). The organic phase was collected, washed with water (2 × 500mL) and saturated saline (300mL), dried over anhydrous sodium sulfate, filtered and concentrated. The concentrate was purified by column chromatography to obtain 3.0g of target product as a brown solid.

LC-MS: (ESI, *m*/*z*): [M+H] ⁺ =431.1

The following intermediates were prepared with reference to the method of intermediate 15:

| Intermediate | Structure | Name | LC-MS |
|---|---|---|---|
| Intermediate 16 | 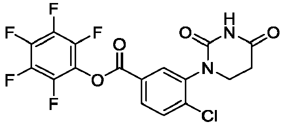 | pentafluorophenyl 4-chloro-3-(2, 4-dioxotetrahydropyrimid in-1 (2H)-yl) benzoate | (ES,*m*/*z*):[M+H]⁺=4 35.0 |
| Intermediate 17 | 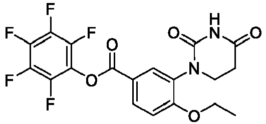 | pentafluorophenyl 3-(2, 4-dioxotetrahydropyrimid in-1 (2H)-yl)-4-ethoxy benzoate | (ES,*m*/*z*):[M+H]⁺=4 45.1 |
| Intermediate 18 | 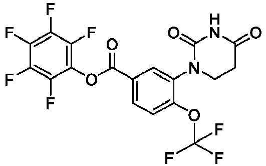 | pentafluorophenyl 3-(2, 4-dioxotetrahydropyrimid in-1 (2H)-yl)-4-(trifluoromethoxy) benzoate | (ES,*m*/*z*):[M+H]⁺=4 85.0 |
| Intermediate 19 | 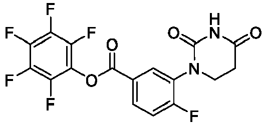 | pentafluorophenyl 3-(2, 4-dioxotetrahydropyrimid in-1 (2H)-yl)-4-fluorobenzoate | (ES,*m*/*z*):[M+H]⁺=4 19.0 |
| Intermediate 20 | 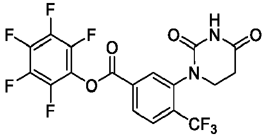 | pentafluorophenyl 3-(2, 4-dioxotetrahydropyrimid in-1 (2H)-yl)-4-(trifluoromethyl) benzoate | (ES,*m*/*z*):[M+H]⁺=4 69.0 |
| Intermediate 21 | 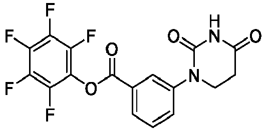 | pentafluorophenyl 3-(2, 4-dioxotetrahydropyrimid in-1 (2H)-yl) benzoate | (ES,*m*/*z*):[M+H]⁺=4 01.0 |
| Intermediate 22 | 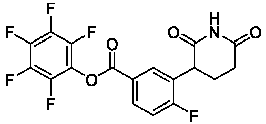 | pentafluorophenyl 3-(2, 6-dioxopiperidin-3 -yl)-4-fluorobenzoate | (ES,*m*/*z*):[M+H]⁺=4 18.0 |

### Intermediate 23: 1-(2-methoxy-5-(3, 9-diazaspiro[5.5] undecyl-3-carbonyl) phenyl) dihydropyrimidine-2, 4(1H,3H)-dione

### Step 1: Preparation of tert-butyl 9-(3-(2, 4-dioxotetrahydropyrimidin-1 (2H)-yl)-4-methoxybenzoyl)-3, 9-diazaspiro[5.5] undecyl-3-carboxylate

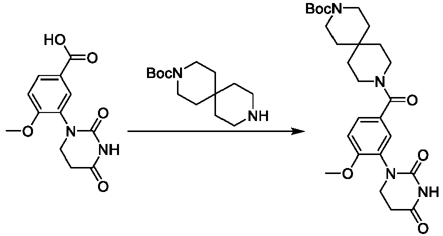

At room temperature, under the protection of nitrogen, HATU(1.66g,4.37 mmol), tert-butyl 3, 9-diazaspiro[5.5] undecyl-3-carboxylate (0.96g,3.78 mmol) and N-methylmorpholine(0.8g,7.92 mmol) were added to a solution of 3-(2, 4-dioxotetrahydropyrimidin-1(2H)-yl)-4-methoxybenzoic acid (1.0g,3.78 mmol) in N,N-dimethylformamide (10 mL). After stirring for 2h, the reaction mixture was quenched with water (50mL) and extracted with ethyl acetate (3 × 50mL). The organic layer was collected and washed with saturated saline (3 × 50mL), dried over anhydrous sodium sulfate, filtered and concentrated. The concentrate was purified by column chromatography to obtain 1.55g of target product as a white solid.

LC-MS: (ES, *m*/*z*): [M+H] ⁺= 501.2

### Step 2: Preparation of 1-(2-methoxy-5-(3, 9-diazaspiro [5.5] undecyl-3-carbonyl) phenyl) dihydropyrimidine-2,4 (1H,3H)-dione

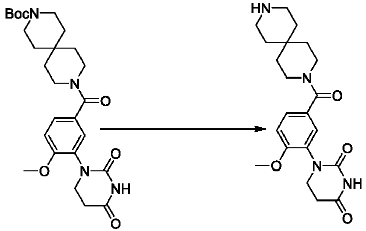

The reaction solution of tert-butyl 9-(3-(2, 4-dioxotetrahydropyrimidin -1(2H)-yl)-4-methoxybenzoyl)-3, 9-diazaspiro[5.5] undecyl-3-carboxylate (1.55g,3.1 mmol) in trifluoroacetic acid/dichloromethane (2.5mL / 5mL) was stirred at room temperature for 4h. The reaction solution was concentrated under reduced pressure to obtain 2.7g of crude target compound as a yellow oil. The crude product was used directly for the next reaction.

LC-MS: (ESI, *m*/*z*): [M+H] ⁺ = 401.1

The following intermediates were prepared according to the method of intermediate 23:

| Intermediate | Structure | Name | LC-MS |
|---|---|---|---|
| Intermediate 24 | 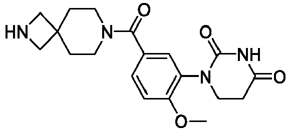 | 1-(2-methoxy-5-(2, 7-diazaspiro[3.5] nonyl-7-carbonyl) phenyl) dihydropyrimidine-2, 4(1H,3H)-dione | (ES,*m*/*z*):[M+H]⁺=37 3.2 |
| Intermediate 25 | 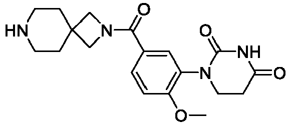 | 1-(2-methoxy-5-(2, 7-diazaspiro[3.5] nonyl-2-carbonyl) phenyl) dihydropyrimidine-2, 4(1H,3H)-dione | (ES,*m*/*z*):[M+H]⁺=37 3.2 |
| Intermediate 26 | 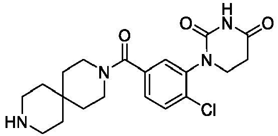 | 1-(2-chloro-5-(3, 9-diazaspiro[5.5]undec yl-3-carbonyl) phenyl) dihydropyrimidine-2,4 (1H,3H)-dione | (ES,*m*/*z*):[M+H]⁺=40 5.1 |

### Intermediate 27: 2-(9-(3-(2, 4-dioxotetrahydropyrimidin-1 (2H)-yl)-4-methoxybenzoyl)-3, 9-diazaspiro[5.5] undec-3-yl) acetaldehyde

### Step 1: Preparation of 1-(5-(9-(2, 2-dimethoxyethyl)-3, 9-diazaspiro[5.5]undecyl-3-carbonyl)-2-methoxyphenyl) dihydropyrimidine -2,4(1H,3H)-dione

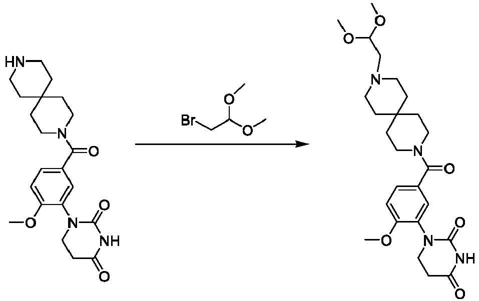

2-Bromo-1, 1-dimethoxyethane (46mg,0.3mmol) was added to a mixture of 1-(2-methoxy-5-(3, 9-diazaspiro [5.5] undecyl-3-carbonyl) phenyl) dihydropyrimidine-2, 4(1H,3H)-dione (200 mg,0.25 mmol), potassium carbonate (155mg,1.25mmol) and potassium iodide (19mg,0.11mmol) in acetone (4mL). The reaction mixture was stirred at 80°C for 3h and then concentrated under reduced pressure. The concentrate was purified by column chromatography to obtain 120mg of target compound as a white solid.

LC-MS: (ESI, *m*/*z*): [M+H] ⁺ = 489.3

### Step 2: Preparation of 2-(9-(3-(2, 4-dioxotetrahydropyrimidin-1 (2H)-yl)-4-methoxybenzoyl)-3, 9-diazaspiro [5.5] undec-3-yl) acetaldehyde

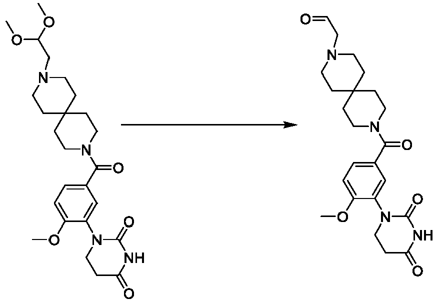

A mixture of 1-(5-(9-(2, 2-dimethoxyethyl)-3, 9-diazaspiro [5.5] undecyl-3-carbonyl)-2-methoxyphenyl) dihydropyrimidine-2, 4(1H,3H)-dione (120mg,0.246mmol) in trifluoroacetic acid/dichloromethane (1mL / 2mL) was stirred and reacted at room temperature for 4 hours. The reaction solution was concentrated under reduced pressure. The concentrate was purified by column chromatography to obtain 100mg of target compound as a white solid.

LC-MS: (ESI, *m*/*z*): [M+H] ⁺=443.1

The following intermediates were prepared with reference to intermediate 27:

| Intermediate | Structure | Name | LC-MS |
|---|---|---|---|
| Intermediate 28 | 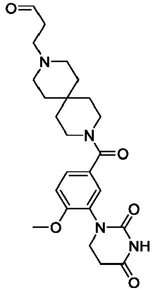 | 3-(9-(3-(2, 4-dioxotetrahydropyrimidin-1 (2H)-yl)-4-methoxybenzoyl)-3, 9-diazaspiro[5.5] undec-3-yl) propionaldehyde | (ES,*m*/*z*):[M+H]⁺=457.3 |
| Intermediate 29 | 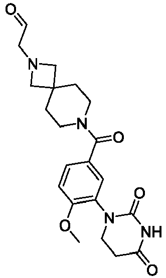 | 2-(7-(3-(2, 4-dioxotetrahydropyrimidin-1 (2H)-yl)-4-methoxybenzoyl)-2, 7-diazaspiro[3.5] non-2-yl) acetaldehyde | (ESI,*m*/*z*):[M+H]⁺=415.1 |
| Intermediate 30 | 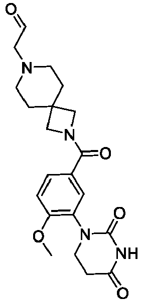 | 2-(2-(3-(2, 4-dioxotetrahydropyrimidin-1 (2H)-yl)-4-methoxybenzoyl)-2, 7-diazaspiro[3.5] non-7-yl) acetaldehyde | (ESI,*m*/*z*):[M+H]⁺=415.1 |
| Intermediate 31 | 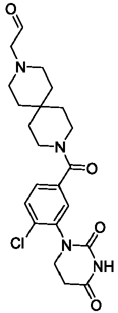 | 2-(9-(4-chloro-3-(2, 4-dioxotetrahydropyrimidin-1 (2H)-yl) benzoyl)-3, 9-diazaspiro[5.5]undec-3-yl) acetaldehyde | (ES,*m*/*z*):[M+H]⁺=447.1 |

### Intermediate 32:4-(9-(3-(2, 4-dioxotetrahydropyrimidin-1 (2H)-yl)-4-methoxybenzoyl)-3, 9-diazaspiro[5.5] undec-3-yl) butyraldehyde

### Step 1: Preparation of 1-(5-(9-(3-(1, 3-dioxan-2-yl) propyl)-3, 9-diazaspiro[5.5] undecyl-3-carbonyl)-2-methoxyphenyl) dihydropyrimidine-2, 4( 1H,3H)-dione

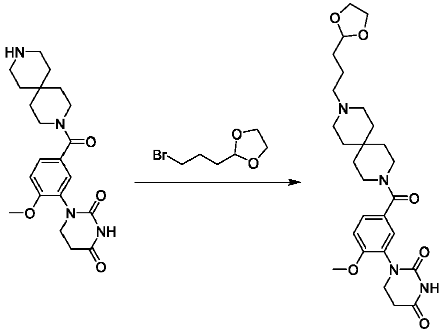

2-(3-Bromopropyl)-1, 3-dioxane (58mg,0.3mmol) was added to a mixture of 1-(2-methoxy-5-(3, 9-diazaspiro [5.5] undecyl-3-carbonyl) phenyl) dihydropyrimidine-2, 4(1H,3H)-dione (200 mg,0.25 mmol), potassium carbonate (160mg,1.25mmol) and potassium iodide (19mg,0.125mmol) in acetone (10mL). The reaction solution was stirred at 80°C for 3h, and the resulting mixture was concentrated under reduced pressure. The concentrate was purified by column chromatography to obtain 70mg of target compound as a white solid.

LC-MS: (ESI, *m*/*z*): [M+H] ⁺ = 515.1

### Step 2: Preparation of 4-(9-(3-(2, 4-dioxotetrahydropyrimidin-1 (2H)-yl)-4-methoxybenzoyl)-3, 9-diazaspiro [5.5] undec-3-yl) butyraldehyde

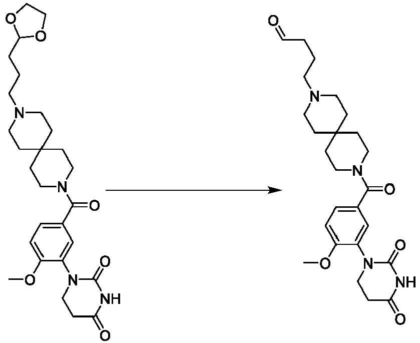

A mixture of 1-(5-(9-(3-(1, 3-dioxacyclopentan-2-yl) propyl)-3, 9-diazaspiro[5.5] undecyl-3-carbonyl)-2-methoxyphenyl) dihydropyrimidine-2, 4( 1H,3H)-dione (70mg,0.136mmol) in trifluoroacetic acid/dichloromethane (1mL / 2mL) was stirred for 4h at room temperature. The reaction solution was concentrated under reduced pressure. The concentrate was purified by column chromatography to obtain 60mg of target compound as a white solid.

LC-MS: (ESI, *m*/*z*): [M+H] ⁺=471.1

### Intermediate 33:3-(2, 4-dioxotetrahydropyrimidin-1 (2H)-yl)-4-methoxy-N-methyl-N-(5-oxopentyl) benzamide

### Step 1:Preparation of tert-butyl (5-((tetrahydro-2H-pyran-2-yl) oxy) pentyl) carbamate

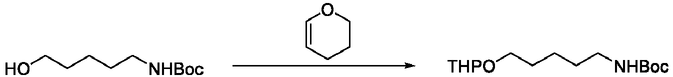

Tert-butyl (5-hydroxypentyl) carbamate (1.0g,4.92 mmol), dichloromethane (10.0 mL), p-toluenesulfonic acid(99.47 mg,0.49 mmol) were added to a 50 mL round bottom flask, followed by 3, 4-dihydro-2H-pyran (496.0mg,5.90mmol). The reaction mixture was stirred at room temperature overnight. 30mL of water was added to quench the reaction, extracted with dichloromethane (2 × 30mL), organic layers were combined, and washed with saturated saline (30mL). The organic layer was dried over anhydrous sodium sulfate, concentrated under reduced pressure, and the concentrate was purified by column chromatography to obtain 900mg of target product.

LC-MS: (ES, *m*/*z*): [M+H]⁺=288.2

### Step 2: Preparation of tert-butyl methyl (5-((tetrahydro-2H-pyran-2-yl) oxy) pentyl) carbamate

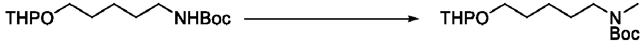

Tert-butyl (5-((tetrahydro-2H-pyran-2-yl) oxy) pentyl) carbamate (900.0 mg,3.13 mmol), N,N-dimethylformamide (5.0 mL), and tetrahydrofuran (5.0 mL) were added to a 50mL round-bottomed flask and placed in an ice-water bath, followed by sodium hydride (60% mineral oil mixture, 137.72 mg,3.44 mmol). The reaction mixture was stirred at 0 °C for 0.5h. Potassium iodide (533.35mg,3.75mmol) was then added and stirred overnight at room temperature. 30mL of water was added to quench the reaction, extracted with ethyl acetate (2 × 30mL), organic layers were combined, and washed with 30mL of water and 30mL of saturated saline. The organic layer was dried over anhydrous sodium sulfate and then concentrated under reduced pressure. The concentrate was purified by column chromatography to obtain 400mg of target product.

LC-MS: [M+Na] ⁺ = 324.2

### Step 3: Preparation of 5-(methylamino) pentyl-1-ol

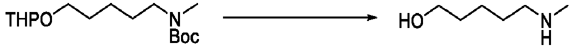

Tert-butyl methyl (5-((tetrahydro-2H-pyran-2-yl) oxy) pentyl) carbamate (400 mg,1.33 mmol), and dichloromethane (5.00 mL) were added to a 25 mL round bottom flask, followed by trifluoroacetic acid (3.0mL), and the reaction mixture was stirred at room temperature for 2h. The reaction solution was concentrated under reduced pressure to obtain 500mg of target product. The target product was directly used in the next reaction.

### Step 4: Preparation of 3-(2,4-dioxotetrahydropyrimidin-1 (2H)-yl)-N-(5-hydroxypentyl)-4-methoxy-N-methylbenzamide

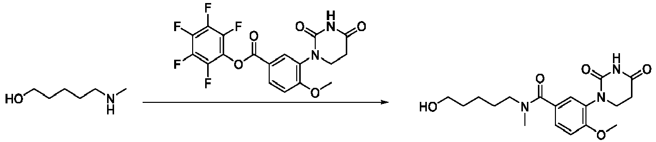

5-(Methylamino) pentyl-1-ol (500 mg crude product, 1.33 mmol), dimethyl sulfoxide (5.0 mL), and N,N-diisopropylethylamine(686.28 mg,5.32 mmol) were added to a 50 mL round bottom flask, followed by pentafluorophenyl 3-(2, 4-dioxotetrahydropyrimidin-1 (2H)-yl)-4-methoxybenzoate (430.0mg,1.0mmol), the mixed reaction solution was stirred at room temperature overnight. 10mL of water was added to quench the reaction. The mixture was extracted with ethyl acetate (2 × 20mL), organic layers were combined, and washed with 10mL of water and 20mL of saturated brine. The mixture was dried over anhydrous sodium sulfate and concentrated under reduced pressure. The concentrate was purified by column chromatography to obtain 100 mg of the target product.

### Step 5: Preparation of 3-(2,4-dioxotetrahydropyrimidin-1 (2H)-yl)-4-methoxy-N-methyl-N-(5-oxopentyl) benzamide

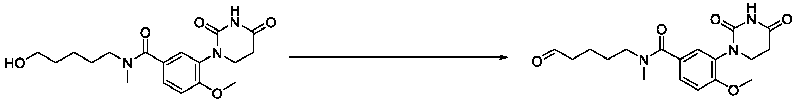

3-(2, 4-Dioxotetrahydropyrimidin-1(2H)-yl)-N-(5-hydroxypentyl)-4-methoxy-N-methylbenzamide (100 mg,0.27 mmol) and dichloromethane (5.0mL) were added to a 25 mL round-bottomed flask and placed in an ice-water bath, followed by the addition of a Dess-Martin oxidant (137.37mg,0.32mmol), and the reaction mixture was stirred at room temperature for 2 hours. 5mL of saturated sodium thiosulfate and 5mL of saturated sodium bicarbonate were added to quench the reaction, and then the mixture was continued to be stirred for 20 minutes. The mixture was extracted with dichloromethane (2 × 20mL), organic layers were combined and washed with 20mL saturated saline. The organic layer was collected and dried over anhydrous sodium sulfate, then concentrated under reduced pressure to obtain 110.0mg of the target crude product.

LC-MS: (ES, *m*/*z*): [M+H]⁺=362.2

### Intermediate 34:2-(1-(3-(2, 4-dioxotetrahydropyrimidin-1 (2H)-yl)-4-methoxybenzoyl) piperidin-4-yl) acetaldehyde

### Step 1: Preparation of 2-(piperidin-4-yl) acetaldehyde

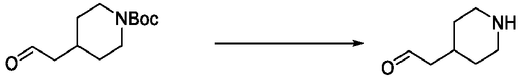

Trifluoroacetic acid (1.5mL) was added to a solution of tert-butyl 4-(2-oxoethyl) piperidin-1-carboxylate (580mg,2.72mol) in dichloromethane (10mL). The reaction solution was stirred at room temperature for 2 hours. The reactants were concentrated under reduced pressure to obtain 320mg of oil, which was directly used in the next reaction.

LC-MS: (ES, *m*/*z*): [M+H]⁺=128.2

### Step 2: Preparation of 2-(1-(3-(2, 4-dioxotetrahydropyrimidin-1 (2H)-yl)-4-methoxybenzoyl) piperidin-4-yl) acetaldehyde

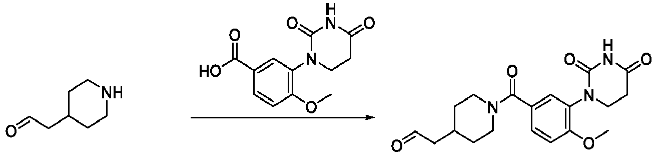

Triethylamine (406mg,3.98mmol) and HATU(605mg,1.59mmol) were added to a mixture of 2-(piperidin-4-yl) acetaldehyde (320 mg,1.33 mmol) and 3-(2, 4-dioxotetrahydropyrimidin-1 (2H)-yl)-4-methoxybenzoic acid (150 mg,1.33 mmol) in N,N-dimethylformamide (5mL). The reaction solution was stirred at room temperature for 12 hours. The reaction solution was poured into water (50mL), extracted with ethyl acetate (50mL × 2), and the organic layer was collected, washed with saline (50mL × 2), dried over anhydrous sodium sulfate, filtered and concentrated under reduced pressure. The concentrate was purified by column to obtain 120 mg of target product as a yellow solid.

LC-MS: (ES, *m*/*z*): [M+H]⁺=374.2

### Intermediate 35:2-(4-(3-(2, 4-dioxotetrahydropyrimidin-1 (2H)-yl)-4-methoxybenzoyl) piperazin-1-yl) acetaldehyde

### Step 1: Preparation of tert-butyl 4-(3-(2, 4-dioxotetrahydropyrimidin-1 (2H)-yl)-4-methoxybenzoyl) piperazine-1-carboxylate

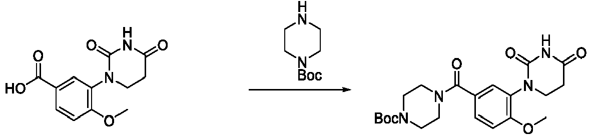

N,N-diisopropylethylamine(440mg,9.3mmol) and HATU(520mg, 1.36mmol) were added to a mixture of 3-(2, 4-dioxotetrahydropyrimidin-1 (2H)-yl)-4-methoxybenzoic acid (300 mg,1.14 mmol) and tert-butyl piperazine-1-carboxylate (212 mg,1.14 mmol) in N,N-dimethylformamide (10mL). The reaction solution was stirred at room temperature for 2h, then the reaction solution was poured into water (20mL) and extracted with ethyl acetate (20mL × 2). The organic layers were combined, washed with saline (20mL × 2), dried over anhydrous sodium sulfate, filtered and concentrated under reduced pressure. The concentrate was purified by column to obtain 400mg of target product as a yellow solid.

LC-MS: (ES, *m*/*z*): [M+H]⁺=433.2

### Step 2: Preparation of 1-(2-methoxy-5-(piperazin-1-carbonyl) phenyl) dihydropyrimidine-2,4(1H,3H)-dione

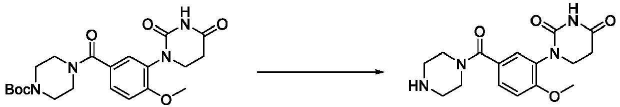

Trifluoroacetic acid (1.5mL) was added to a solution of tert-butyl 4-(3-(2, 4-dioxotetrahydropyrimidin-1 (2H)-yl)-4-methoxybenzoyl) piperazine-1-carboxylate (400 mg,0.93 mmol) in dichloromethane (10 mL). The reaction solution was stirred at room temperature for 2 hours. The solution was concentrated under reduced pressure to obtain 300mg of crude target product as an oil. The crude product was used directly for the next step.

LC-MS: (ES, *m*/*z*): [M+H]⁺=333.2

### Step 3: Preparation of 1-(5-(4-(2, 2-dimethoxyethyl) piperazin-1-carbonyl)-2-methoxyphenyl) dihydropyrimidine-2,4 (1H,3H)-dione

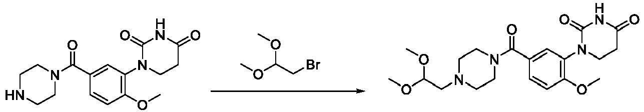

Potassium carbonate (371 mg, 2.71 mmol) and potassium iodide (15 mg,0.01 mmol) were added to a mixture of 1-(2-methoxy-5-(piperazin-1-carbonyl) phenyl) dihydropyrimidine-2,4(1H,3H)-dione (300 mg,0.9 mmol) and 2-bromo-1, 1-dimethoxyethane (305 mg,1.81 mmol) in acetone (20 mL). The reaction solution was stirred at 80°C for 12h. The reaction solution was concentrated under reduced pressure to remove the solvent, and water (10mL) and ethyl acetate (10mL) were added. The water layers were separated and extracted with ethyl acetate (10mL × 2), and the organic layers were combined, washed with saline (10mL × 2), dried over anhydrous sodium sulfate, filtered and concentrated under reduced pressure. The concentrate was purified by column to obtain 120 mg of target product as an oil.

LC-MS: (ES, *m*/*z*): [M+H]⁺=421.2

### Step 4: Preparation of 2-(4-(3-(2, 4-dioxotetrahydropyrimidin-1 (2H)-yl)-4-methoxybenzoyl) piperazin-1-yl) acetaldehyde

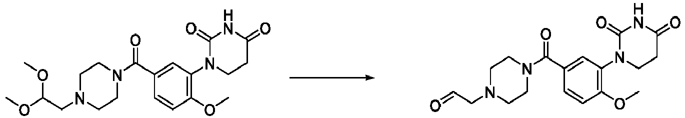

Trifluoroacetic acid (6mL) was added to a solution of 1-(5-(4-(2, 2-dimethoxyethyl) piperazin-1-carbonyl)-2-methoxyphenyl) dihydropyrimidine-2,4 (1H,3H)-dione (120 mg,0.27 mmol) in dichloromethane (10mL). The reaction solution was stirred at room temperature for 12h. The solvent was removed by concentration under reduced pressure to obtain 53mg of the crude target product. The crude product was used directly for the next step.

LC-MS: (ES, *m*/*z*): [M+H]⁺=375.1

### Intermediate 36:3-(2, 4-dioxotetrahydropyrimidin-1 (2H)-yl)-4-methoxy-N-(2-(2-(2-oxoethoxy) ethoxy) ethyl) benzamide

### Step 1: Preparation of 3-(2, 4-dioxotetrahydropyrimidin-1 (2H)-yl)-N-(2-(2-(2-(2-hydroxyethoxy) ethoxy) ethyl)-4-methoxybenzamide

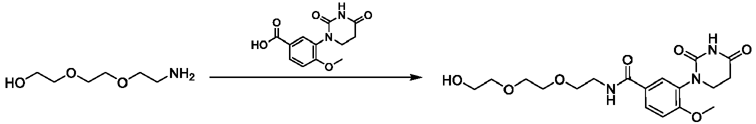

A mixture of 2-(2-(2-aminoethoxy) ethoxy) ethan-1-ol (200mg,0.75 mmol), 3-(2, 4-dioxotetrahydropyrimidin-1 (2H)-yl)-4-methoxybenzoic acid (135mg ,0.91mmol), HATU(432mg,1.14mmol), and N,N-diisopropylethylamine (293mg,2.27mmol) in N,N-dimethylformamide (5mL) was stirred for 16h at room temperature. The reactants were poured into water (30mL) and extracted with ethyl acetate (3 × 30mL), the organic phase was collected, dried over anhydrous sodium sulfate, filtered and concentrated under reduced pressure. The concentrate was purified by column chromatography to give 190mg of product as yellow solid.

LC-MS-3: (ES, *m*/*z*): [M-H]⁻=394.1

### Step 2: Preparation of 3-(2, 4-dioxotetrahydropyrimidin-1 (2H)-yl)-4-methoxy-N-(2-(2-(2-oxoethoxy) ethoxy) ethyl) benzamide

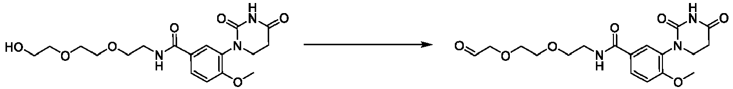

Dess-Martin(408mg,0.96mmol) was added to a mixture of 3-(2,4-dioxotetrahydropyrimidin-1(2H)-yl)-N-(2-(2-(2-(2-hydroxyethoxy)ethoxy)ethyl)-4-methoxybenzamide (190 mg, 0.48 mmol) in dichloromethane (5 mL) at 0 °C. The reaction solution was stirred for 2h at room temperature, filtered and concentrated under reduced pressure. The concentrate was purified by column chromatography to obtain 100mg of target product as a yellow solid.

LC-MS-4: (ES, *m*/*z*): [M-H]⁻=394.1

### Intermediate 37: 5-((tert-butoxycarbonyl) amino) pentyl-4-methylbenzenesulfonate

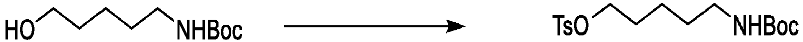

### A mixture of tert-butyl (5-hydroxypentyl) aminocarboxylate (500 mg,2.463 mmol), p-toluenesulfonyl chloride(706 mg,3.695 mmol) and 4-dimethylaminopyridine (154 mg,1.232 mmol) in pyridine (5 mL) was stirred for 3 h at room temperature. The obtained reaction solution was diluted with water (50mL) and extracted with ethyl acetate (3 × 50mL), and the organic phase was collected. The organic phase was dried over anhydrous sodium sulfate, filtered and concentrated under reduced pressure to obtain 400mg of crude target product as a light yellow oil. The crude product was used directly for the next step.

LC-MS: (ESI, *m*/*z*): [M+H]⁺=358.2

### Intermediate 38: tert-butyl 9-(2-hydroxyethyl)-3-azaspiro[5.5] undecyl-3-carboxylate

### Step 1:tert-butyl 9-(2-ethoxy-2-oxoethylene)-3-azaspiro[5.5] undecyl-3-carboxylate

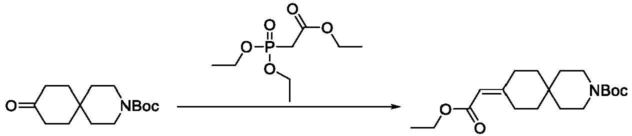

At 0 °C, sodium hydride (60% mineral oil mixture, 224mg,5.6mmol) was added to a solution of 2-(diethoxyphosphoryl) ethyl acetate (1.26g,5.63mmol) in N,N-dimethylformamide (15mL), and stirred at 0 °C for 0.5 h, then tert-butyl 9-oxo-3-azaspiro [5.5] undecyl-3-carboxylate (1.0g,3.75mmol) was added. The reaction mixture was stirred at 0 °C for 3h. The reaction mixture was diluted with water (100mL) and extracted with ethyl acetate (3 × 30mL). The organic layer was collected and washed with water (2 × 20mL) and saturated saline (20mL), dried over anhydrous sodium sulfate, filtered and concentrated to obtain 0.7g of the crude target product. The crude product was used directly for the next step.

### Step 2:tert-butyl 9-(2-ethoxy-2-oxoethyl)-3-azaspiro[5.5] undecyl-3-carboxylate

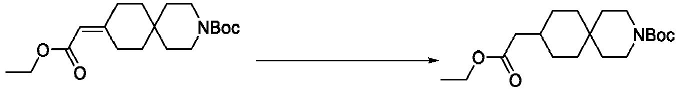

The reaction mixture of tert-butyl 9-(2-ethoxy-2-oxoethylene)-3-azaspiro[5.5] undecyl-3-carboxylate (0.7g,2.08 mmol) and palladium hydroxide (0.2g,1.43 mmol) in ethanol (10mL) was stirred at room temperature for 16h under hydrogen atmosphere. The reaction mixture was filtered and concentrated under reduced pressure to obtain 0.5g of the crude target product. The crude product was used directly for the next step.

¹H NMR (400 MHz, CDCl₃) δ 4.12 (q, *J* = 7.1 Hz, 2H), 3.35 (ddd, *J* = 7.6, 6.8, 5.0 Hz, 4H), 2.20 (d, *J* = 7.1 Hz, 2H), 1.70 - 1.63 (m, 2H), 1.58 (dd, *J* = 12.7, 7.1 Hz, 2H), 1.45 (s, 9H), 1.27 (dt, *J =* 14.3, 6.5 Hz, 6H), 1.18 - 1.10 (m, 4H), 0.86 (dd, *J =* 13.4, 6.3 Hz, 2H).

### Step 3:tert-butyl 9-(2-hydroxyethyl)-3-azaspiro[5.5] undecyl-3-carboxylate

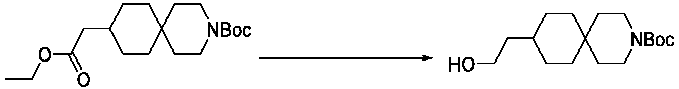

The reaction mixture of tert-butyl 9-(2-ethoxy-2-oxoethyl)-3-azaspiro[5.5] undecyl-3-carboxylate (0.5g,1.47 mmol) and lithium borohydride(0.13g,5.9 mmol) in tetrahydrofuran (5 mL) was refluxed overnight. The reaction mixture was diluted with water (50mL) and extracted with ethyl acetate (3 × 30mL). The organic layer was collected and washed with water (2 × 20mL) and saturated saline (20mL), dried over anhydrous sodium sulfate, filtered and concentrated to obtain 0.4g of the crude target product.

LC-MS: (ESI, m/z): [M+H] ⁺ = 298.2

¹H NMR (400 MHz, MeOD) δ 3.58 (t, *J* = 6.6 Hz, 2H), 3.43 - 3.28 (m, 5H), 1.77 - 1.66 (m, 2H), 1.62 - 1.52 (m, 2H), 1.52- 1.41 (m, 13H), 1.29 (t, *J =* 5.6 Hz, 3H), 1.12 (dd, *J* = 19.2, 11.5 Hz, 4H).

The following intermediates were prepared with reference to the method of intermediate 38:

| Intermediate | Structure | Name | LC-MS |
|---|---|---|---|
| Intermediate 39 | 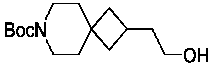 | tert-butyl 2-(2-hydroxyethyl)-7-azaspiro[3.5] nonyl-7-carboxylate | (ESI, m/z): [M+H] ⁺ = 270.3 |

### Intermediate 40:N-(2-(1-(5-aminopentyl) piperidin-4-yl)-6-methoxy-2H-indazol-5-yl)-6-(trifluoromethyl) pyridine carboxamide

### Step 1:Preparation of tert-butyl (5-(4-(6-methoxy-5-(6-(trifluoromethyl) pyridinecarboxamido)-2H-indazol-2-yl) piperidin-1-yl) pentyl) carbamate

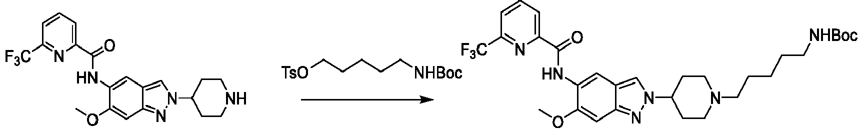

A mixture of N-(6-methoxy-2-(piperidin-4-yl)-2H-indazol-5-yl)-6-(trifluoromethyl) pyridinecarboxamide (387 mg,0.924 mmol), 5-((tert-butoxycarbonyl) amino) pentyl-4-methylbenzenesulfonate (330 mg,0.924 mmol) and potassium carbonate (638 mg,4.622 mmol) in N,N-dimethylformamide (10 mL) was stirred at 75°C for 3 hours, and the reaction solution was poured into water (100mL), extracted with ethyl acetate (3 × 100mL), the organic phase was collected, and washed with water (2 × 200mL) and saline (200mL), dried over anhydrous sodium sulfate, filtered and concentrated. The concentrate was purified by column chromatography to obtain yellow oily product (60 mg,Y = 11%).

LC-MS: (ESI, *m*/*z*): [M+H]⁺= 605.3

### Step 2: Preparation of N-(2-(1-(5-aminopentyl) piperidin-4-yl)-6-methoxy-2H-indazol-5-yl)-6-(trifluoromethyl) pyridinecarboxamide

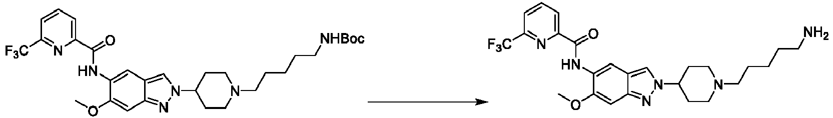

A mixture of tert-butyl (5-(4-(6-methoxy-5-(6-(trifluoromethyl) pyridinecarboxamido)-2H-indazol-2-yl) piperidin-1-yl) pentyl) carbamate (60 mg,0.0993 mmol) in trifluoroacetic acid/dichloromethane (1mL,1:1) was stirred and reacted overnight at room temperature, and the reactants were concentrated under reduced pressure to obtain 160mg of crude target product as a yellow oil, and the crude product was directly used for the next step.

LC-MS: (ESI, *m*/*z*): [M+H]⁺=505.2

### Intermediate 41:N-(2-(1-(2-(2-aminoethoxy) ethyl) piperidin-4-yl)-6-methoxy-2H-indazol-5-yl)-6-(trifluoromethyl) pyridinecarboxamide

### Step 1:Preparation of tert-butyl (2-(2-(4-(6-methoxy-5-(6-(trifluoromethyl) pyridinecarboxamido)-2H-indazol-2-yl) piperidin-1-yl) ethoxy) ethyl) carbamate

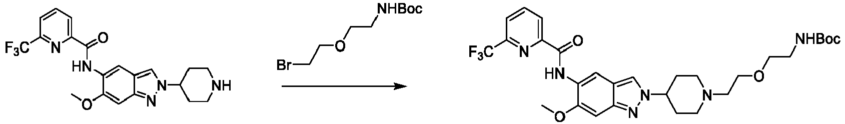

A solution of N-(6-methoxy-2-(piperidin-4-yl)-2H-indazol-5-yl)-6-(trifluoromethyl) pyridinecarboxamide (500 mg,1.193 mmol), tert-butyl (2-(2-bromoethoxy) ethyl) carbamate (610 mg,2.864 mmol), potassium carbonate (823 mg,5.967 mmol) and potassium iodide (99 mg,0.597 mmol) in acetonitrile (10 mL) was stirred at 80°C and reacted for 5 hours. The resulting reaction solution was diluted with ethyl acetate (50mL), the organic phase was collected, washed with water (2 × 50mL) and brine (50mL), dried over anhydrous sodium sulfate and concentrated under reduced pressure. The concentrate was purified by column chromatography to obtain 330 mg of yellow oily product.

LC-MS: (ESI, *m*/*z*): [M+H] ⁺ = 607.3

### Step 2: Preparation of N-(2-(1-(2-(2-aminoethoxy) ethyl) piperidin-4-yl)-6-methoxy-2H-indazol-5-yl)-6-(trifluoromethyl) pyridinecarboxamide

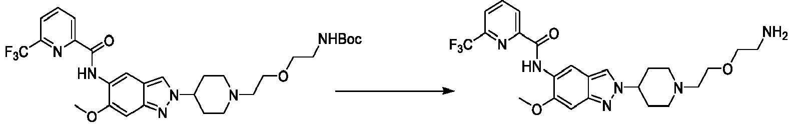

A mixture of tert-butyl (2-(2-(4-(6-methoxy-5-(6-(trifluoromethyl) pyridinecarboxamido)-2H-indazol-2-yl) piperidin-1-yl) ethoxy) ethyl) carbamate(140mg,0.231mmol) in trifluoroacetic acid/dichloromethane (3mL, 1 : 1) was stirred and reacted overnight at room temperature. The reactants were concentrated under reduced pressure to obtain 270mg of crude product as a yellow oil. The crude product was used directly for the next step.

LC-MS: (ESI, *m*/*z*): [M+H]⁺=507.3

### Intermediate 42:N-(6-methoxy-2-(1-(2-(piperidin-4-yl) ethyl) piperidin-4-yl)-2H-indazol-5-yl)-6-(trifluoromethyl) pyridinecarboxamide

### Step 1: Preparation of tert-butyl 4-(2-(4-(6-methoxy-5-(6-(trifluoromethyl) pyridinecarboxamido)-2H-indazol-2-yl) piperidin-1-yl) ethyl) piperidine-1-formate

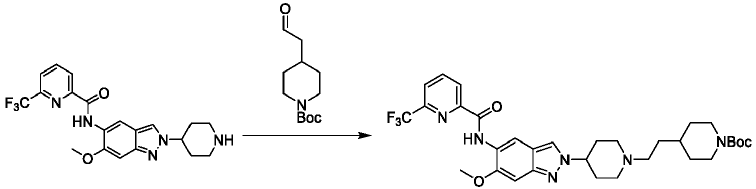

Sodium triacetoxyborohydride(1.518g,7.160mmol) was added to a mixture of N-(6-methoxy-2-(piperidin-4-yl)-2H-indazol-5-yl)-6-(trifluoromethyl) pyridinecarboxamide (1.0g,2.386 mmol) and tert-butyl 4-(2-oxoethyl) piperidine-1-carboxylate(0.813g,3.581mmol) in tetrahydrofuran (20mL). The reaction solution was stirred for reaction overnight at room temperature. The reaction solution was diluted with ethyl acetate (50mL), washed with water (2 × 50mL) and saturated saline (50mL), and the organic phase was collected, dried over anhydrous sodium sulfate, and concentrated under reduced pressure. The concentrate was purified by column chromatography to obtain 700 mg of target product as a yellow solid.

LC-MS: (ESI, *m*/*z*): [M+H]⁺ = 631.3

### Step 2: Preparation of N-(6-methoxy-2-(1-(2-(piperidin-4-yl) ethyl) piperidin-4-yl)-2H-indazol-5-yl)-6-(trifluoromethyl) pyridinecarboxamide

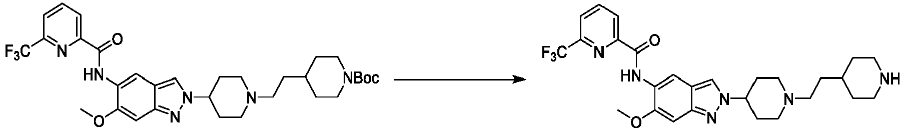

A mixture of tert-butyl 4-(2-(4-(6-methoxy-5-(6-(trifluoromethyl) pyridinecarboxamido)-2H-indazol-2-yl) piperidin-1-yl) ethyl) piperidine-1-formate(700mg,1.11mmol) in hydrochloric acid/ethyl acetate (1M,20mL) was stirred and reacted overnight at room temperature. The reactants were concentrated under reduced pressure to obtain 650mg of crude target product a yellow oil. The crude product was used directly for the next step.

LC-MS: (ESI, *m*/*z*): [M+H]⁺=531.3

### Intermediate 43:N-(6-methoxy-2-(1-(2-oxoethyl) piperidin-4-yl)-2H-indazol-5-yl)-6-(trifluoromethyl) pyridinecarboxamide

### Step 1: Preparation of N-(2-(1-(2, 2-dimethoxyethyl) piperidin-4-yl)-6-methoxy-2H-indazol-5-yl)-6-(trifluoromethyl) pyridinecarboxamide

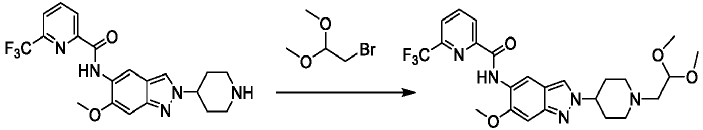

Potassium carbonate (1.235g, 8.95 mmol) and potassium iodide (149mg,0.89 mmol) were added to a mixture of N-(6-methoxy-2-(piperidin-4-yl)-2H-indazol-5-yl)-6-(trifluoromethyl) pyridinecarboxamide (750 mg,1.79 mmol) and 2-bromo-1,1-dimethoxyethane (46mg,0.3 mmol) in acetonitrile (20 mL). The reaction solution was stirred at 80°C for reaction overnight. The reaction solution was concentrated under reduced pressure to remove the solvent, water (20mL) was added, extracted with ethyl acetate (20mL × 3), and the organic layers were combined, washed with saturated saline (50mL), dried over anhydrous sodium sulfate, filtered and concentrated under reduced pressure. The concentrate was purified by column to obtain 500 mg of target product as an oil.

LC-MS: (ESI, *m*/*z*): [M+H] ⁺ = 508.1

### Step 2: Preparation of N-(6-methoxy-2-(1-(2-oxoethyl) piperidin-4-yl)-2H-indazol-5-yl)-6-(trifluoromethyl) pyridinecarboxamide

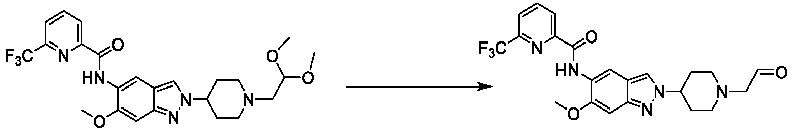

A mixture of N-(2-(1-(2, 2-dimethoxyethyl) piperidin-4-yl)-6-methoxy-2H-indazol-5-yl)-6-(trifluoromethyl) pyridinecarboxamide (500mg,0.99 mmol) in hydrochloric acid (4mL)/dioxane (5mL) was stirred and reacted overnight at 50°C. The reaction solution was concentrated under reduced pressure to remove the solvent, water (20mL) was added, extracted with ethyl acetate (20mL × 3), and the organic layers were combined, washed with saturated saline (50mL), dried over anhydrous sodium sulfate, filtered and concentrated under reduced pressure. The concentrate was purified by column to obtain 300 mg of target product as an oil.

LC-MS: (ESI, *m*/*z*): [M+H] ⁺ = 462.1

The following intermediates were prepared with reference to the method of intermediate 43:

| Intermediate | Structure | Name | LC-MS |
|---|---|---|---|
| Intermediate 44 | 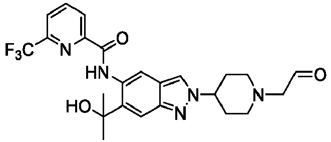 | N-(6-(2-hydroxyprop -2-yl)-2-(1-(2-oxoethyl) piperidin-4-yl)-2H-indazol-5-yl)-6-(trifluoromethyl) pyridinecarboxamide | (ES,*m*/*z*): [M+H]⁺=49 0.2 |

### Intermediate 45:N-(6-methoxy-2-(1-(3-piperidin-4-yl) propyl) piperidin-4-yl)-2H-indazol-5-yl)-6-(trifluoromethyl) pyridinecarboxamide

### step 1: tert-butyl 4-(3-(p-toluenesulfonyloxy) propyl) piperidine-1-formate

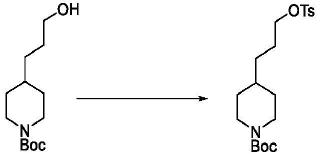

Triethylamine (2.1g,20.6 mmol) and p-toluenesulfonyl chloride(1.2g,6.1 mmol) were added to a stirred solution of tert-butyl 4-(3-hydroxypropyl) piperidine-1-formate (1.0g,4.1 mmol) in dichloromethane (30 mL). After the reaction mixture was stirred at 25°C overnight, the reaction was quenched with saturated ammonium chloride (50mL) and extracted with dichloromethane (2 × 50mL). The organic layer was collected and concentrated under reduced pressure. The concentrate was purified by column chromatography to obtain 1.1g of target product.

LC-MS: (ESI, *m*/*z*): [M+H] ⁺ = 398.2.

### Step 2:tert-butyl 4-(3-(4-(6-methoxy-5-(6-(trifluoromethyl) pyridinecarboxamido))-2H-indazol-2-yl) piperidin-1-yl) propyl) piperidine-1-formate

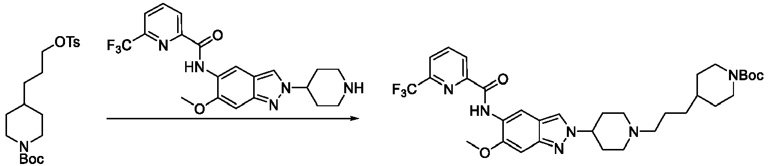

N-(6-methoxy-2-(piperidin-4-yl)-2H-indazol-5-yl)-6-(trifluoromethyl) pyridinecarboxamide (1.2g,2.9 mmol) and potassium carbonate (2.0g,14.5 mmol) were added to a stirred solution of tert-butyl 4-(3-(p-toluenesulfonyloxy) propyl) piperidine-1-formate (1.1g,2.9 mmol) in N,N-dimethylformamide(15mL). The reaction mixture was stirred at 75°C for 3 hours. The reaction was quenched with water (100mL) and extracted with ethyl acetate (3 × 50mL). The organic layer was collected and washed with saturated saline (100mL) and concentrated under reduced pressure. The concentrate was purified by column chromatography to obtain 1.2 g of target product.

LC-MS: (ESI, *m*/*z*): [M+H] ⁺ = 645.3

### Step 3:N-(6-methoxy-2-(1-(3-piperidin-4-yl) propyl) piperidin-4-yl)-2H-indazol-5-yl)-6-(trifluoromethyl) pyridinecarboxamide

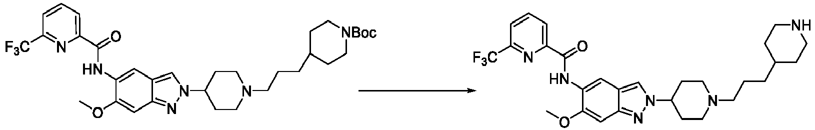

Trifluoroacetic acid (4.0mL) was added to a stirred solution of tert-butyl 4-(3-(4-(6-methoxy-5-(6-(trifluoromethyl) pyridinecarboxamido))-2H-indazol-2-yl) piperidin-1-yl) propyl) piperidine-1-formate (1.4g, 2.1 mmol) in dichloromethane (20.0mL). The reaction mixture was stirred at 25 °C for 2 hours, then concentrated under reduced pressure to obtain 1.1g of crude target product. The crude product was used directly for the next step.

LC-MS: (ESI, *m*/*z*): [M+H] ⁺ = 545.2.

The following intermediates were prepared with reference to the method of intermediate 45:

| Intermediate | Structure | Name | LC-MS |
|---|---|---|---|
| Intermediate 46 | 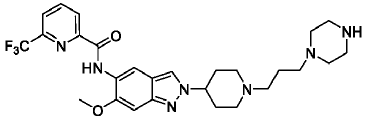 | N-(6-methoxy-2-(1-(3-piperazin-1-yl) propyl) piperidin-4-yl)-2H-indazol-5-yl)-6-(trifluoromethyl) pyridinecarboxamide | (ESI,*m*/*z* ): [M+H] ⁺ = 546.3 |
| Intermediate 47 | 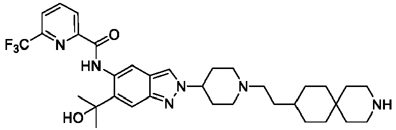 | N-(2-(1-(2-(3-azaspiro[5.5] undec-9-yl) ethyl) piperidin-4-yl) 6-(2-hydroxyprop-2-yl)-2H-indazol-5-yl)-6-(trifluoromethyl) pyridinecarboxamide | (ES,*m*/*z*) [M+H]⁺ =627.2 |
| Intermediate 48 | 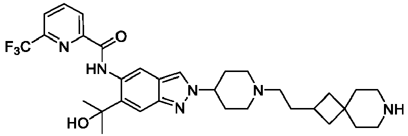 | N-(2-(1-(2-(7-azaspiro[3.5] non-2-yl) ethyl) piperidin-4-yl)-6-(2-hydroxyprop-2-yl)-2H-indazol-5-yl)-6-(trifluoromethyl) pyridinecarboxamide | (ESI,*m*/*z* ): [M+H] ⁺ = 599.3 |
| Intermediate 49 | 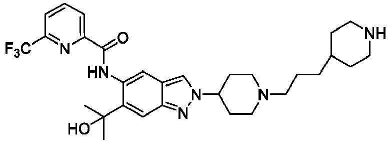 | N-(6-(2-hydroxyprop -2-yl)-2-(1-(3-(piperidin-4-yl) propyl) piperidin-4-yl)-2H-indazol-5-yl)-6-(trifluoromethyl) pyridinecarboxamide | (ESI,*m*/*z* ): [M+H] ⁺ = 573.2 |
| Intermediate 50 | 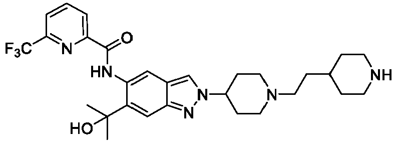 | N-(6-(2-hydroxyprop -2-yl)-2-(1-(2-(piperidin-4-yl) ethyl) piperidin-4-yl)-2H-indazol-5-yl)-6-(trifluoromethyl) pyridinecarboxamide | (ESI,*m*/*z* ): [M+H] ⁺ = 559.3 |
| Intermediate 51 | 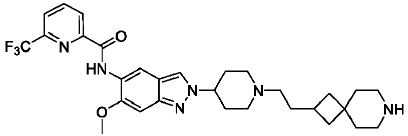 | N-(2-(1-(2-(7-azaspiro[3.5] non-2-yl) ethyl) piperidin-4-yl)-6-methoxy-2H-indazol-5-yl)-6-(trifluoromethyl) pyridinecarboxamide | (ESI,*m*/*z* ): [M+H]⁺ =571.2 |
| Intermediate 52 | 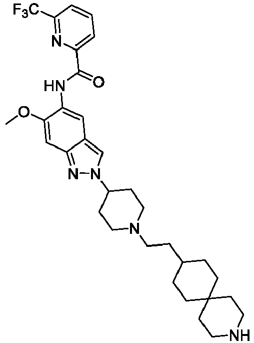 | N-(2-(1-(2-(3-azaspiro[5.5] undec-9-yl) ethyl] piperidin-4-yl)-6-methoxy-2H-indazol-5-yl)-6-(trifluoromethyl) pyridinecarboxamide | (ESI,*m*/*z* ): [M+H] ⁺ = 599.1. |

### Intermediate 53:N-(6-methoxy-2-(1-(2-(piperidin-4-yloxy) ethyl) piperidin-4-yl)-2H-indazol-5-yl)-6-(trifluoromethyl) pyridinecarboxamide

### Step 1:tert-butyl 4-(allyloxy) piperidine-1-carboxylate

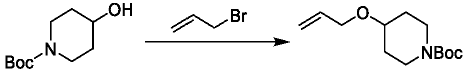

Sodium hydride (60% mineral oil mixture, 360mg,12mmol) was added to a stirred solution of tert-butyl 4-hydroxypiperidine-1-formate (2.0g,10mmol) in tetrahydrofuran (50mL) at 0 °C, and then stirred for 1h at 0 °C. Then 3-bromoprop-1-ene (1.44g,12mmol) was added. After the stirring reaction was continued for 4h, the reaction was quenched with water (300mL) and extracted with ethyl acetate (3 × 200mL). The organic phase was collected and concentrated under reduced pressure. The concentrate was purified on a silica gel column to obtain 2.2g of the target product.

¹H NMR (400 MHz, CDCl3) δ 6.02 -5.81 (m, 1H), 5.23 (dddd, J = 42.1, 10.4, 3.1, 1.5 Hz, 2H), 4.02 (dt, J = 5.5, 1.4 Hz, 2H), 3.86 - 3.71 (m, 2H), 3.49 (dt, J = 12.0, 4.1 Hz, 1H), 3.15 -2.97 (m, 2H), 1.91 -1.74 (m, 2H), 1.60 - 1.36 (m, 11H).

### Step 2:tert-butyl 4-(2-oxoethoxy) piperidine-1-carboxylate

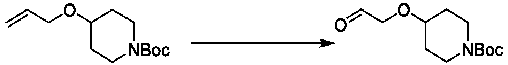

Under ozone atmosphere, tert-butyl 4-(allyloxy) piperidine-1-carboxylate (1g,4.15mmol) in dichloromethane (30 mL) was reacted at -78 °C for 30 minutes and then quenched with dimethyl sulfide (10mL), concentrated under reduced pressure, and the concentrate was purified by column chromatography to obtain 300mg of the target product.

¹H NMR (400 MHz, CDCl₃) δ 9.74 (t, J = 0.8 Hz, 1H), 4.68 (m, 1H), 4.51 -4.43 (m, 1H), 3.56 - 3.52 (m, 2H), 3.29 (m, 2H), 3.21 (m, 1H), 2.31 - 2.19 (m, 2H), 1.82 -1.68 (m, 2H), 1.46 (s, 9H).

### Step 3:tert-butyl 4-(2-(4-(6-methoxy-5-(6-(trifluoromethyl) pyridinecarboxamido))-2H-indazol-2-yl) piperidin-1-yl) ethoxy) piperidine-1-carboxylate

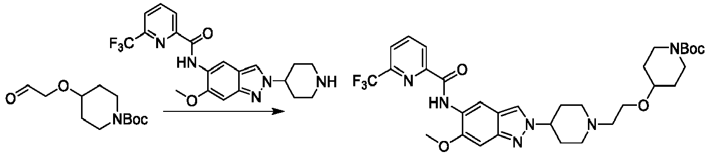

Sodium triacetoxyborohydride(303mg,1.40mmol) and three drops of acetic acid were added to a stirred solution of N-(6-methoxy-2-(piperidin-4-yl)-2H-indazol-5-yl)-6-(trifluoromethyl) pyridinecarboxamide (200 mg,0.477 mmol) and tert-butyl 4-(2-oxoethoxy) piperidine-1-carboxylate (174mg, 0.716mmol) in tetrahydrofuran (15mL), then the reaction mixture was stirred at 25 °C for 2 hours. The reaction mixture was diluted with ethyl acetate (60mL) and washed with water (2 × 60mL) and saturated saline (60mL). The organic phase was collected and dried over anhydrous sodium sulfate, filtered, and concentrated under reduced pressure. The concentrate was purified on a silica gel column to obtain 260mg of the target product.

LC-MS: (ESI, *m*/*z*): [M+H] ⁺ = 647.1.

### Step 4:N-(6-methoxy-2-(1-(2-(piperidin-4-yloxy) ethyl) piperidin-4-yl)-2H-indazol-5-yl)-6-(trifluoromethyl) pyridinecarboxamide

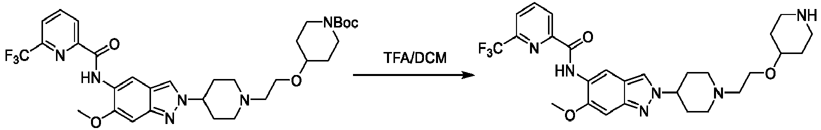

The reaction mixture of tert-butyl 4-(2-(4-(6-methoxy-5-(6-(trifluoromethyl) pyridinecarboxamido))-2H-indazol-2-yl) piperidin-1-yl) ethoxy) piperidine-1-formate (260mg,0.402mmol) in trifluoroacetic acid/dichloromethane (4mL,1:1) was stirred at room temperature overnight. The reaction mixture was concentrated under reduced pressure to obtain 500mg of the crude target product. The crude product was used directly for the next step.

LC-MS: (ESI, *m*/*z*): [M+H] ⁺ = 547.3.

The following intermediates were prepared with reference to the method of intermediate 53:

| Intermediate | Structure | Name | LC-MS |
|---|---|---|---|
| Intermediate 54 | 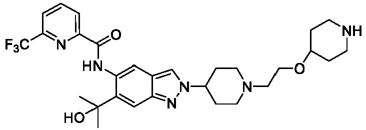 | N-(6-(2-hydroxyprop-2-yl)-2-(1-(2-(piperidin-4-yloxy) ethyl) piperidin-4-yl)-2H-indazol-5-yl)-6-(trifluoromethyl) pyridinecarboxamide | (ESI,*m*/ *z*): [M+H] ⁺ = 575.2 |

### Intermediate 55: N-(2-(1-((3-azaspiro[5.5] undec-9-yl) methyl) piperidin-4-yl)-6-(2-hydroxyprop-2-yl)-2H-indazol-5-yl)-6-(trifluoromethyl) pyridinecarboxamide

### Step 1: tert-butyl 9-((4-(6-(2-hydroxyprop -2-yl)-5-(6-(trifluoromethyl) pyridinecarboxamido)-2H-indazol-2-yl) piperidin-1-yl) methyl)-3-azaspiro[5.5] undecyl-3-carboxylate

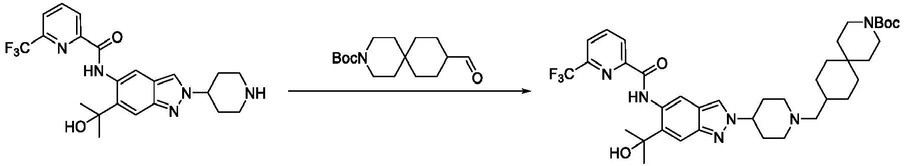

At room temperature, sodium triacetylborohydride (212 mg, 1 mmol) was added to a mixture of N-(6-(2-hydroxyprop-2-yl)-2-(piperidin-4-yl)-2H-indazol-5-yl)-6-(trifluoromethyl) pyridinecarboxamide (223 mg,0.5 mmol) and tert-butyl 9-formyl-3-azaspiro[5.5] undecyl-3-carboxylate (140 mg,0.5 mmol) in tetrahydrofuran (5 mL), and then stirred and reacted for 2 hours. The solution was diluted with water (20mL) and extracted with dichloromethane (10mL X 3). The organic layer was collected, concentrated under reduced pressure, and the concentrate was purified by column chromatography to obtain 260mg of the target product.

LC-MS: (ESI, *m*/*z*): [M+H] ⁺ = 713.1.

¹H NMR (400 MHz, DMSO) δ 12.37 (s, 1H), 8.72 (s, 1H), 8.45 (d, J = 7.7 Hz, 1H), 8.37 (dd, J = 14.8, 7.0 Hz, 2H), 8.16 (dd, J = 7.8, 0.8 Hz, 1H), 7.57 (s, 1H), 5.95 (s, 1H), 4.44 (s, 1H), 3.28 (s, 4H), 2.96 (s, 2H), 2.08 (d, J = 10.9 Hz, 7H), 1.70 - 1.44 (m, 12H), 1.39 (s, 11H), 1.22 (d, J = 6.0 Hz, 2H), 1.07 (d, J = 9.7 Hz, 4H).

### Step 2:N-(2-(1-((3-azaspiro[5.5] undec-9-yl) methyl) piperidin-4-yl)-6-(2-hydroxyprop-2-yl)-2H-indazol-5-yl)-6-(trifluoromethyl) pyridinecarboxamide

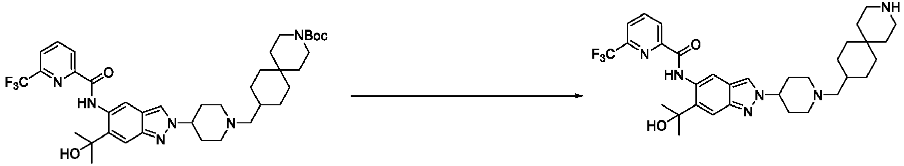

Tert-butyl 9-((4-(6-(2-hydroxyprop-2-yl)-5-(6-(trifluoromethyl) pyridinecarboxamido)-2H-indazol-2-yl) piperidin-1-yl) methyl)-3-azaspiro[5.5] undecyl-3-carboxylate (260mg,0.365mmol) was dissolved in dichloromethane (5mL) and trifluoroacetic acid (1mL) was added at 0 °C. The reaction mixture was stirred and reacted at 0 °C for 2h. N,N-diisopropylethylamine was slowly added at 0-5 °C until the pH of the reaction solution was > 9, the solution was concentrated under reduced pressure at room temperature, and the crude product was directly used for the next step without further purification.

LC-MS: (ESI, *m*/*z*): [M+H] ⁺ = 613.1.

The following intermediates were prepared with reference to the method of intermediate 55:

| Intermediate | Structure | Name | LC-MS |
|---|---|---|---|
| Intermediate 56 | 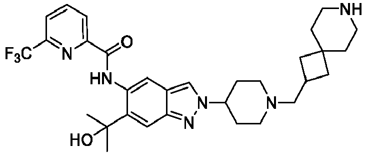 | N-(2-(1-(((7-azaspiro[3.5] non-2-yl)methyl) piperidin-4-yl)-6-(2-hydroxyprop-2-yl)-2H-indazol-5-yl)-6-(trifluoromethyl) pyridinecarboxamide | (ESI,*m*/*z*):[ M+H] ⁺ = 585.3 |
| Intermediate 57 | 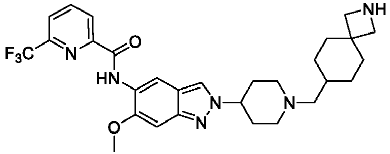 | N-(2-(1-(((2-azaspiro[3.5] non-7-yl)methyl) piperidin-4-yl)-6-methoxy-2H-indazol-5-yl)-6-(trifluoromethyl) pyridinecarboxamide | (ESI,*m*/*z*):[ M+H] ⁺ = 557.3 |
| Intermediate 58 | 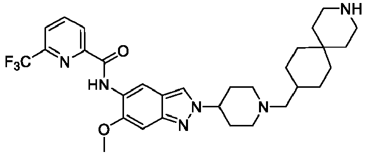 | N-(2-(1-((3-azaspiro[5.5] undec-9-yl)methyl) piperidin-4-yl)-6-methoxy-2H-indazol-5-yl)-6-(trifluoromethyl) pyridinecarboxamide | (ESI,*m*/*z*):[ M+H] ⁺ = 585.3 |
| Intermediate 59 | 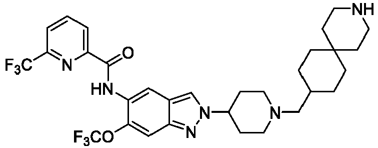 | N-(2-(1-((3-azaspiro[5.5] undec-9-yl)methyl) piperidin-4-yl)-6-(trifluoromethoxy)-2H-indazol-5-yl)-6-(trifluoromethyl)) pyridinecarboxamide | (ESI,*m*/*z*):[ M+H]⁺ = 639.2 |

### Intermediate 60:N-(6-methoxy-2-(1-(2-(piperazin-1-yl) ethyl) piperidin-4-yl)-2H-indazol-5-yl)-6-(trifluoromethyl) pyridinecarboxamide

### Step 1: Preparation of tert-butyl 4-(2-(4-(6-methoxy-5-(6-(trifluoromethyl) pyridinecarboxamido))-2H-indazol-2-yl) piperidin-1-yl) ethyl) piperazine-1-carboxylate

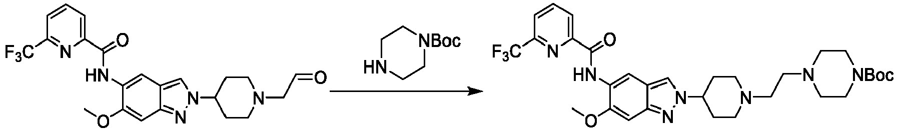

Sodium triacetoxyborohydride(110mg,0.521mmol) was added to a mixture of N-(6-methoxy-2-(1-(2-oxoethyl) piperidin-4-yl)-2H-indazol-5-yl)-6-(trifluoromethyl) pyridinecarboxamide (80 mg,0.174 mmol) and tert-butyl piperazine-1-formate (32mg,0.174 mmol) in tetrahydrofuran (2mL). The reaction solution was stirred and reacted overnight at room temperature, the resulting reaction solution was diluted with ethyl acetate (20mL), washed with water (2 × 20mL) and saturated saline (20mL), and the organic phase was collected. The organic phase was dried over anhydrous sodium sulfate and concentrated under reduced pressure. The concentrate was purified by column chromatography to obtain 80 mg of target product as a yellow oil.

LC-MS: (ESI, *m*/*z*): [M+H]⁺ = 632.1

### Step 2:N-(6-methoxy-2-(1-(2-(piperazin-1-yl) ethyl) piperidin-4-yl)-2H-indazol-5-yl)-6-(trifluoromethyl) pyridinecarboxamide

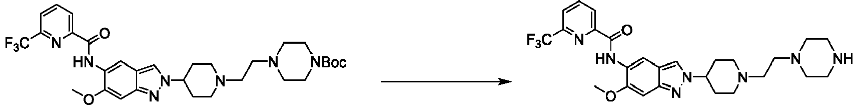

A mixture of tert-butyl 4-(2-(4-(6-methoxy-5-(6-(trifluoromethyl) pyridinecarboxamido))-2H-indazol-2-yl) piperidin-1-yl) ethyl) piperazine-1-carboxylate (80 mg,0.127 mmol) in trifluoroacetic acid/dichloromethane (2 ml,1:1) was stirred and reacted overnight at room temperature. The reaction solution was concentrated under reduced pressure to obtain 120mg of crude target product as a yellow oil. The crude product was used directly for the next step.

LC-MS: (ESI, *m*/*z*): [M+H]⁺=532.3

The following intermediates were prepared with reference to the method of intermediate 60:

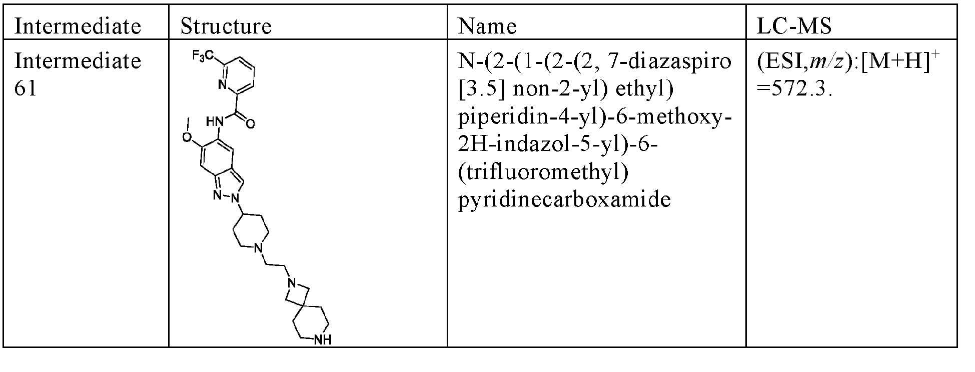

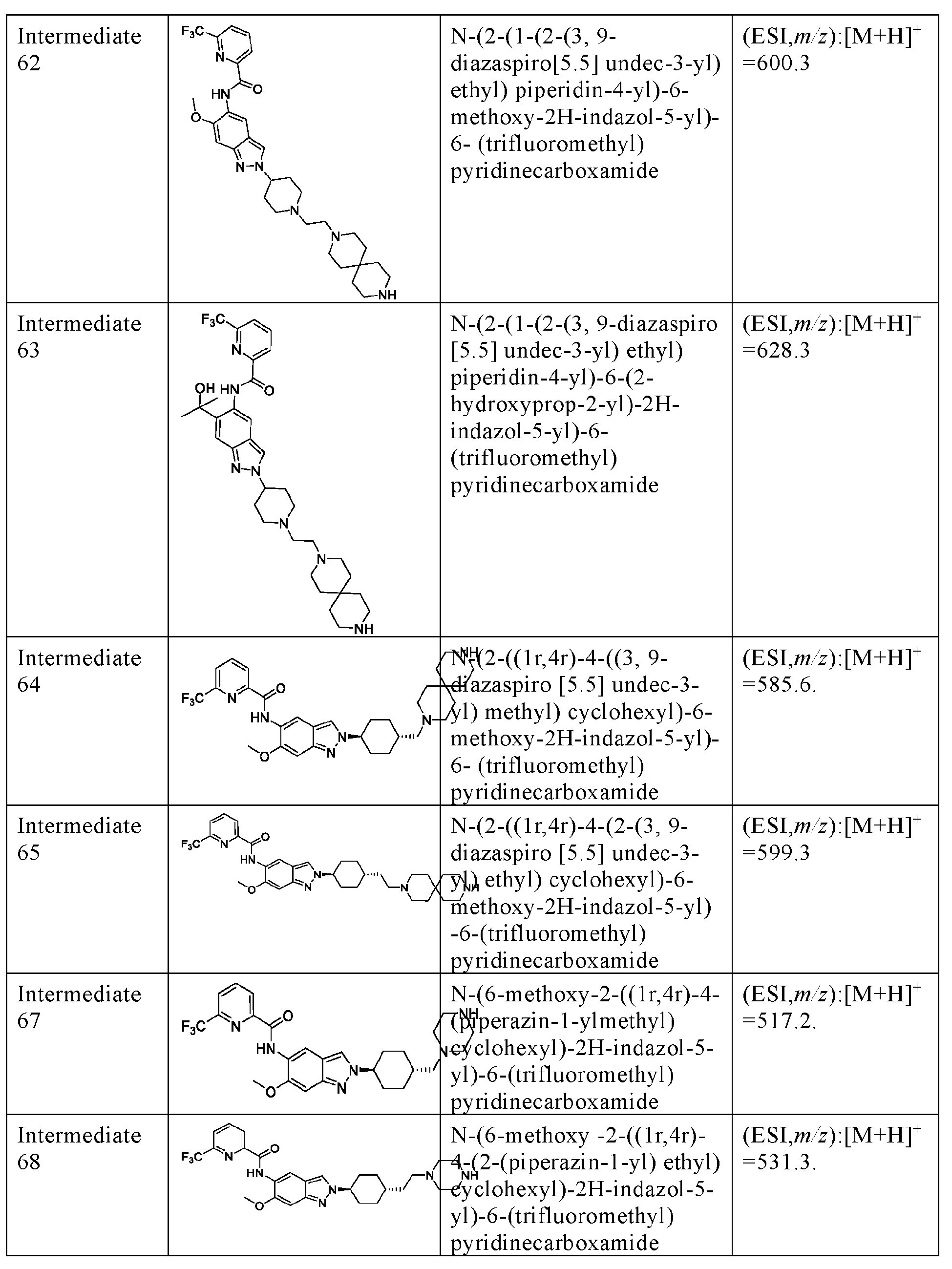

### Intermediate 69:N-(6-methoxy-2-(1-(2-(piperidin-4-yl) methyl) piperidin-4-yl)-2H-indazol-5-yl)-6-(trifluoromethyl) pyridinecarboxamide

### Step 1: Preparation of tert-butyl 4-(2-(4-(6-methoxy-5-(6-(trifluoromethyl) pyridinecarboxamido)-2H-indazol-2-yl) piperidin-1-yl) methyl) piperidine-1-formate

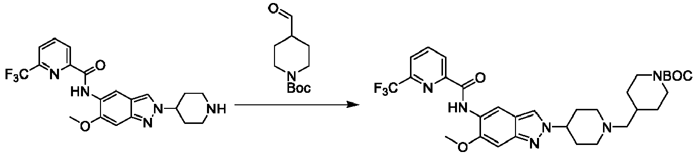

Sodium triacetoxyborohydride(1.518g, 7.160mmol) was added to a mixture of N-(6-methoxy-2-(piperidin-4-yl)-2H-indazol-5-yl)-6-(trifluoromethyl) pyridinecarboxamide (1.0g,2.386 mmol) and tert-butyl 4-formylpiperidine-1-carboxylate (0.763g,3.581mmol) in tetrahydrofuran (20mL). The reaction solution was stirred for reaction overnight at room temperature. The reaction solution was diluted with ethyl acetate (50mL), washed with water (2 × 50mL) and saturated saline (50mL), the organic phase was collected, dried over anhydrous sodium sulfate, and concentrated under reduced pressure. The concentrate was purified by column chromatography to obtain 550 mg of target product as a yellow solid.

LC-MS: (ESI, *m*/*z*): [M+H]⁺ = 617.3

### Step 2: Preparation of N-(6-methoxy-2-(1-(2-(piperidin-4-yl) methyl) piperidin-4-yl)-2H-indazol-5-yl)-6-(trifluoromethyl) pyridinecarboxamide

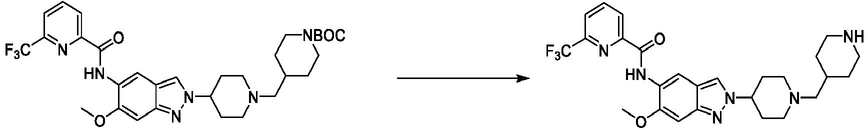

A mixture of tert-butyl 4-(2-(4-(6-methoxy-5-(6-(trifluoromethyl) pyridinecarboxamido)-2H-indazol-2-yl) piperidin-1-yl) methyl) piperidine-1-formate (573mg,1.11mmol) in hydrochloric acid/ethyl acetate (1M,20mL) was stirred and reacted overnight at room temperature. The reactants were concentrated under reduced pressure to obtain 400mg of crude target product as a yellow oil. The crude product was used directly for the next step.

LC-MS: (ESI, *m*/*z*): [M+H]⁺=517.2

### Intermediate 70:N-(6-methoxy-2-(1-(2-(piperidin-4-yl) methyl) piperidin-4-yl)-2H-indazol-5-yl)-6-(trifluoromethyl) pyridinecarboxamide

### Step 1: Preparation of tert-butyl 4-(2-(4-(6-methoxy-5-(6-(trifluoromethyl) pyridinecarboxamido)-2H-indazol-2-yl) piperidin-1-yl) methyl) piperidine-1-formate

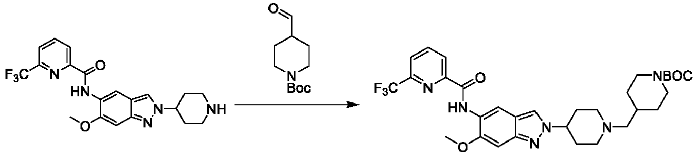

Sodium triacetoxyborohydride(1.518g, 7.160mmol) was added to a mixture of N-(6-methoxy-2-(piperidin-4-yl)-2H-indazol-5-yl)-6-(trifluoromethyl) pyridinecarboxamide (1.0g,2.386 mmol) and tert-butyl 4-formylpiperidine-1-carboxylate (0.763g,3.581mmol) in tetrahydrofuran (20mL). The reaction solution was stirred for reaction overnight at room temperature. The reaction solution was diluted with ethyl acetate (50mL), washed with water (2 × 50mL) and saturated saline (50mL), and the organic phase was collected, dried over anhydrous sodium sulfate, and concentrated under reduced pressure. The concentrate was purified by column chromatography to obtain 550 mg of target product as a yellow solid.

LC-MS: (ESI, *m*/*z*): [M+H]⁺ = 617.3

### Step 2: Preparation of N-(6-methoxy-2-(1-(2-(piperidin-4-yl) methyl) piperidin-4-yl)-2H-indazol-5-yl)-6-(trifluoromethyl) pyridinecarboxamide

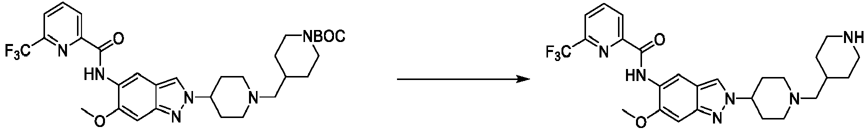

A mixture of tert-butyl 4-(2-(4-(6-methoxy-5-(6-(trifluoromethyl) pyridinecarboxamido)-2H-indazol-2-yl) piperidin-1-yl) methyl) piperidine-1-formate (573mg,1.11mmol) in hydrochloric acid/ethyl acetate (1M,20mL) was stirred and reacted overnight at room temperature. The reactants were concentrated under reduced pressure to obtain 400mg of crude target product as a yellow oil. The crude product was used directly for the next step.

LC-MS: (ESI, *m*/*z*): [M+H]⁺=517.2

### Intermediate 71:N-(2-((1R,4R)-4-formylcyclohexyl)-6-methoxy-2H-indazol-5-yl)-6-(trifluoromethyl) pyridine carboxamide

### Step 1:Preparation of ((1R,4R)-4-(6-methoxy-5-nitro-2H-indazol-2-yl) cyclohexyl) methanol

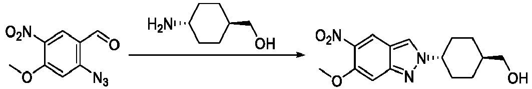

A mixture of 2-azido-4-methoxy-5-nitrobenzaldehyde (1.33g,6 mmol) and ((1r,4r)-4-aminocyclohexyl) methanol (774 mg,6 mmol) in toluene (50 mL) was heated to 100°C and stirred for reaction overnight. The reaction mixture was concentrated under reduced pressure, and the concentrate was purified by column chromatography to obtain 1.5g of target product as a brown solid.

¹H NMR (400 MHz, DMSO) δ 8.60 (s, 1H), 8.39 (s, 1H), 7.27 (s, 1H), 4.54 - 4.40 (m, 2H), 3.91 (s, 3H), 3.29 (t, *J* = 5.8 Hz, 2H), 2.20 - 2.09 (m, 2H), 1.96 - 1.82 (m, 4H), 1.47 (dtd, *J* = 11.9, 6.0, 3.0 Hz, 1H), 1.22 - 1.04 (m, 2H).

### Step 2:((1R,4R)-4-(5-amino-6-methoxy-2H-indazol-2-yl) cyclohexyl) methanol

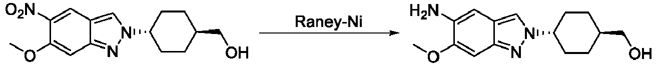

Hydrazine hydrate (2mL) and Raney-Ni (150mg) were added to a solution of ((1R,4R)-4-(6-methoxy-5-nitro-2H-indazol-2-yl) cyclohexyl) methanol (1.5g,5.0mmol) in ethanol (50mL). The reaction mixture was stirred at room temperature for 2 hours. Filtered, solid was washed with ethanol (10mL × 1). The filtrate was collected and concentrated under reduced pressure to obtain 1.4g of crude product as a brown gel.

LC-MS: (ESI, *m*/*z*): [M+H] ⁺ = 276.3.

### Step 3:N-(2-((1R,4R)-4-(hydroxymethyl) cyclohexyl)-6-methoxy-2H-indazol-5-yl)-6-(trifluoromethyl) pyridinecarboxamide

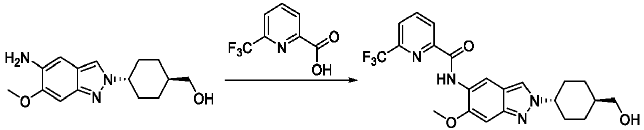

6-(Trifluoromethyl) picolinic acid (0.97g,5.07 mmol), N,N-diisopropylethylamine(1.96g,15.21 mmol) and HATU(2.5g,6.59 mmol) were added to a solution of ((1R,4R)-4-(5-amino-6-methoxy-2H-indazol-2-yl) cyclohexyl) methanol (1.4g,5.07 mmol) in N,N-dimethylformamide(20 mL). After stirring the reaction mixture at room temperature for 2h, the reaction was quenched with water and extracted with ethyl acetate to collect the organic layer. The organic layer was washed with saturated saline, dried over anhydrous sodium sulfate, filtered and concentrated under reduced pressure. The concentrate was purified by column chromatography to obtain 1.2g of target product as a brown solid.

LC-MS: (ESI, *m*/*z*): [M+H] ⁺ = 449.2.

### Step 4:N-(2-((1R,4R)-4-formylcyclohexyl)-6-methoxy-2H-indazol-5-yl)-6-(trifluoromethyl) pyridinecarboxamide

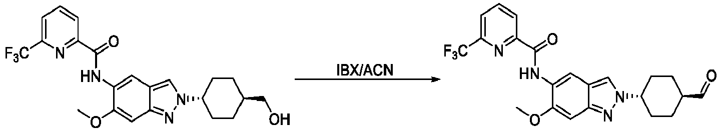

2-Iodoacyl benzoic acid(404mg,1.0mmol) was added to a mixture of N-(2-((1R,4R)-4-(hydroxymethyl) cyclohexyl)-6-methoxy-2H-indazol-5-yl)-6-(trifluoromethyl) pyridinecarboxamide (224 mg,0.5 mmol) in acetonitrile (10mL), the reaction mixture was heated to 80°C and stirred for 2h. The reaction solution was concentrated under reduced pressure, and the concentrate was purified by column chromatography to obtain 180mg of target product as a brown solid.

LC-MS: (ESI, *m*/*z*): [M+H] ⁺ =447.1

The following intermediates were prepared with reference to the method of intermediate 71:

| Intermediate | Structure | Name | LC-MS |
|---|---|---|---|
| Intermediate 72 | 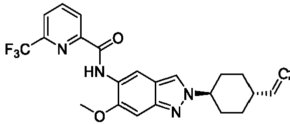 | N-(2-((1r,4r)-4-formylcyclohexyl)-6-methoxy-2H-indazol-5-yl)-6-(trifluoromethyl) pyridinecarboxamid e | LC-MS:(ESI,*m*/*z*): [M+H]⁺=447.1. |
| | | | ¹H NMR (400 MHz, DMSO) δ 10.50 (s, 1H), 9.65 (s, 1H), 8.69 (s, 1H), 8.47 (d, *J* = 7.5 Hz, 1H), 8.41 (t, *J* = 7.8 Hz, 1H), 8.34 (s, 1H), 8.22 (dd, *J* = 7.6, 0.8 Hz, 1H), 7.16 (s, 1H), 4.40 (ddd, *J* = 11.7, 8.0, 3.9 Hz, 1H), 3.98 (s, 3H), 2.43 (t, *J* = 12.3 Hz, 1H), 2.16 (dd, *J* = 37.9, 10.4 Hz, 4H), 1.99 - 1.90 (m, 2H), 1.46 (tt, *J* = 13.1, 6.6 Hz, 2H). |
| Intermediate 73 | 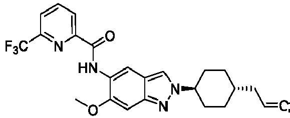 | N-(6-methoxy-2-((1r, 4r)-4-(2-oxoethyl) cyclohexyl)-2H-indazol-5-yl)-6-(trifluoromethyl) pyridinecarboxamid e | (ESI, *m*/*z*): [M+H]⁺=461.2 |

### Intermediates 74:N-(2-((1r,4r)-4-formylcyclohexanyl)-6-(2-hydroxyprop -2-yl)-2H-indazol-5-yl)-6-(trifluoromethyl) pyridinecarboxamide

### Step 1:ethyl (1s, 4s)-4-hydroxycyclohexan-1-carboxylate

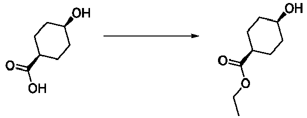

Sulfuric acid (0.25 mL) was added to a solution of (1s, 4s)-4-hydroxycyclohexan-1-carboxylic acid (10g,69.4 mmol) in ethanol (50 mL), and the reaction mixture was stirred at 70 °C for 2 hours. The reaction mixture was concentrated under reduced pressure to obtain 11.6g of crude target product. The crude product was used directly for the next step.

### Step 2:ethyl (1s, 4s)-4-(toluenesulfonyloxy) cyclohexane-1-carboxylate

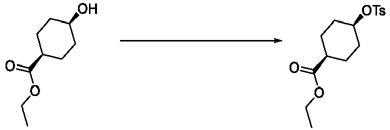

p-Toluenesulfonyl chloride (12g, 62 mmol), TEA (16g, 156 mmol) and 4-dimethylaminopyridine(61mg,0.5 mmol) were added to a mixture of ethyl (1s, 4s)-4-hydroxycyclohexane-1-carboxylate (9.0g, 12 mmol) in dichloromethane (150 mL). The reaction mixture was stirred at room temperature for 16 hours. The reaction mixture was concentrated under reduced pressure, and the concentrate was purified by column chromatography to obtain 11g of the target compound as a yellow solid.

LC-MS: (ES, *m*/*z*): [M+ NH₄] ⁺ =344.2.

### Step 3:(1s, 4s)-4-(hydroxymethyl) cyclohexyl 4-methylbenzenesulfonate

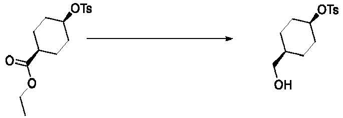

At 0 °C, lithium aluminum hydride (24mL) was added to a solution of ethyl (1s, 4s)-4-(toluenesulfonyloxy) cyclohexane-1-carboxylate (5.0g,15.3mmol) in tetrahydrofuran (50mL). The reaction mixture was stirred at 0°Cfor 2 hours. Sodium sulfate decahydrate was added, filtered and the filtrate was concentrated to obtain 3.6g of target compound as a colorless solid .

LC-MS: (ES, *m*/*z*): [M+ HN₄] ⁺ =302.1.

### Step 4:N-(2-((1r,4r)-4-(hydroxymethyl) cyclohexyl)-6-(2-hydroxyprop-2-yl)-2H-indazol-5-yl)-6-(trifluoromethyl) pyridinecarboxamide

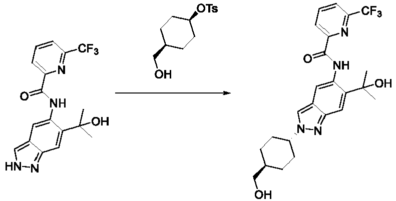

(1s, 4s)-4-(hydroxymethyl) cyclohexyl-4-methylbenzenesulfonate (2.65g, 9 mmol) and cesium carbonate (8.8g, 27 mmol) were added to a mixture of N-(6-(2-hydroxyprop-2-yl)-2H-indazol-5-yl)-6-(trifluoromethyl)pyridinecarboxamide (3.4 g, 9 mmol) in N,N-dimethylformamide (50 mL). The reaction mixture was stirred at 80°C for 16 hours. The reaction mixture was quenched with water (100mL) and the solution was extracted with ethyl acetate (3 × 30mL). The organic layer was collected, washed with water (2 × 20mL) and saturated saline (10mL), dried over anhydrous sodium sulfate, filtered and concentrated. The concentrate was purified by column chromatography to obtain 500mg of target compound as a yellow solid.

LC-MS: (ES, *m*/*z*): [M+ H] ⁺ = 477.1

### Step 5:N-(2-((1r,4r)-4-formylcyclohexanyl)-6-(2-hydroxyprop-2-yl)-2H-indazol-5-yl)-6-(trifluoromethyl) pyridinecarboxamide

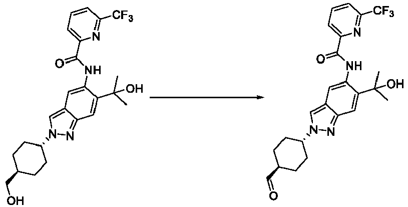

Dess-Martin (213mg,0.50mmol) was added to a mixture of N-(2-(1r,4r)-4-(hydroxymethyl) cyclohexyl)-6-(2-hydroxyprop-2-yl)-2H-indazol-5-yl)-6-(trifluoromethyl) pyridinecarboxamide (200mg,0.42mmol) in 5mL of dichloromethane. After the reaction mixture was stirred at room temperature for 16 hours, the reaction mixture was quenched with water (10mL) and extracted with dichloromethane (3 × 10mL). The organic layer was collected, washed with water (2 × 2mL) and saturated saline (10mL), dried over anhydrous sodium sulfate, filtered and concentrated. The concentrate was purified by column chromatography to obtain 50mg of target compound as a yellow solid.

LC-MS: (ES, *m*/*z*): [M+ H] ⁺ =475.0.

### Intermediate 75: N-(6-methoxy-2-((1R,4R)-4-((methyl (2-(piperidin-4-yl) ethyl) amino) methyl) cyclohexyl)-2H-indazol-5-yl)-6-(trifluoromethyl) pyridinecarboxamide

### Step 1:tert-butyl 4-(2-((((1R,4R)-4-(6-methoxy-5-(6-(trifluoromethyl) pyridinecarboxamido)-2H-indazol-2-yl) cyclohexyl) methyl) amino) ethyl) piperidine-1-formate

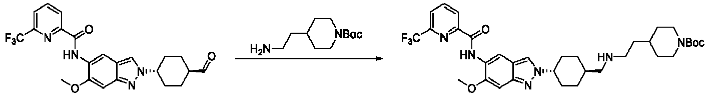

Sodium triacetoxyborohydride (424mg,2mmol) was added to a mixture of N-(2-((1R,4R)-4-formylcyclohexyl)-6-methoxy-2H-indazol-5-yl)-6-(trifluoromethyl) pyridinecarboxamide (180 mg,0.4 mmol) and butyl 4-(2-aminoethyl) piperidine-1-formate (91mg,0.4 mmol) in tetrahydrofuran (10mL), and then the reaction mixture was stirred overnight at room temperature. Water (20mL) was added, extracted with ethyl acetate (20mL × 3), the organic layer was collected, washed with saturated saline (20mL × 1), and the organic layer was collected, dried over anhydrous sodium sulfate, and concentrated under reduced pressure. The concentrate was purified by a chromatographic column to obtain 170mg of target product as a white solid.

LC-MS: (ESI, *m*/*z*): [M+H] ⁺=659.4.

### Step 2:tert-butyl 4-(2-((((1R,4R)-4-(6-methoxy-5-(6-(trifluoromethyl) pyridineformyl)-2H-indazol-2-yl) cyclohexyl) methyl) (methyl) amino) ethyl) piperidine-1-carboxylate

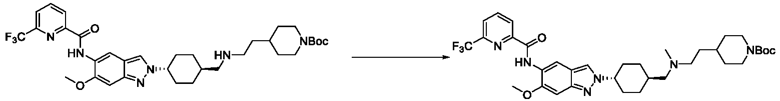

At room temperature, sodium triacetoxyborohydride (276 mg, 1.3 mmol) was added to a mixture of tert-butyl 4-(2-((((1R,4R)-4-(6-methoxy-5-(6-(trifluoromethyl) pyridinecarboxamido)-2H-indazol-2-yl) cyclohexyl) methyl) amino) ethyl) piperidine-1-formate (170 mg,0.26 mmol) and formaldehyde (37wt% aqueous solution, 162 mg,2 mmol) in tetrahydrofuran (10 mL), and the reaction mixture was stirred for reaction overnight. Water (20mL) was added to the reaction mixture, extracted with ethyl acetate (20mL × 3), the organic layer was collected, washed with saturated saline (20mL × 1), dried over anhydrous sodium sulfate, concentrated under reduced pressure, and the concentrate was purified by column chromatography to obtain 135mg of target product as a white solid.

LC-MS: (ESI, *m*/*z*): [M+H] ⁺=673.4.

### Step 3:N-(6-methoxy-2-((1R,4R)-4-((methyl (2-(piperidin-4-yl) ethyl) amino) methyl) cyclohexyl)-2H-indazol-5-yl)-6-(trifluoromethyl) pyridinecarboxamide

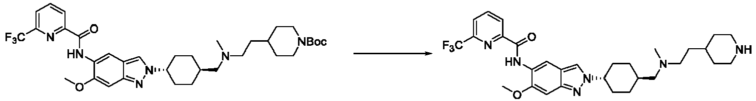

At room temperature, trifluoroacetic acid (4mL) was added to a mixture of tert-butyl 4-(2-((((1R,4R)-4-(6-methoxy-5-(6-(trifluoromethyl)pyridineformyl)-2H-indazol-2-yl) cyclohexyl) methyl) (methyl) amino) ethyl) piperidine-1-carboxylate (135mg,0.20mmol) in dichloromethane (10mL), and the mixture was stirred for 2h. The reaction mixture was concentrated under reduced pressure to obtain 130mg of crude product as a brown gel. The crude product can be used directly for the next step without further purification.

LC-MS: (ESI, *m*/*z*): [M+H] ⁺=573.4.

The following intermediates were prepared with reference to the method of intermediate 75:

| Intermediate | Structure | Name | LC-MS |
|---|---|---|---|
| Intermediate 76 | 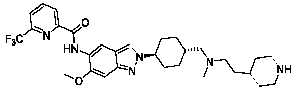 | N-(6-methoxy-2-((1r,4r)-4-((methyl(2-(piperidin-4-yl)ethyl) amino) methyl)cyclohexyl)-2H-indazol-5-yl)-6- (trifluoromethyl) pyridinecarboxamide | (ESI,*m*/*z*):[M+H]⁺ =573.3 |
| Intermediate 77 | 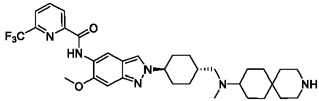 | N-(6-methoxy-2-((1r,4r)-4-((methyl(3-azaspiro[5.5]undec-9-yl)amino)methyl) cyclohexyl)-2H-indazol-5-yl)-6-(trifluoromethyl) pyridinecarboxamide | (ESI,*m*/*z*):[M+H]⁺ =613.3 |
| Intermediate 78 | 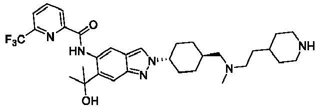 | N-(6-(2-hydroxyprop-2-yl)-2-((1r,4r)-4-((methyl (2-(piperidin-4-yl)ethyl) amino)methyl) cyclohexyl)-2H-indazol-5-yl)-6-(trifluoromethyl) pyridinecarboxamide | (ES,*m*/*z*):[M+H]⁺= 601.3. |
| Intermediate 79 | 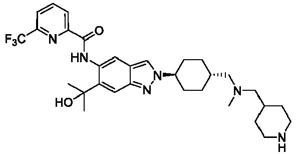 | N-(6-(2-hydroxyprop-2-yl)-2-((1r,4r)-4-((methyl (piperidin-4-ylmethyl) amino)methyl) cyclohexyl)-2H-indazol-5-yl)-6-(trifluoromethyl) pyridinecarboxamide | (ESI,*m*/*z*):[M+H]⁺ =587.6. |
| Intermediate 80 | 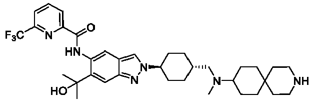 | N-(6-(2-hydroxyprop-2-yl)-2-((1r,4r)-4-((methyl (3-azaspiro[5.5]undec-9-yl)amino)methyl) cyclohexyl)-2H-indazol-5-yl)-6-(trifluoromethyl) pyridinecarboxamide | (ESI,*m*/*z*):[M+H]⁺ =641.5. |
| Intermediate 81 | 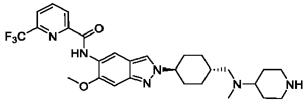 | N-(6-methoxy-2-((1r,4r)-4-((methyl (piperidin-4-yl)amino) methyl) cyclohexyl)-2H-indazol-5-yl)-6-(trifluoromethyl) pyridinecarboxamide | (ESI,*m*/*z*):[M+H]⁺ =545.2 |
| Intermediate 82 | 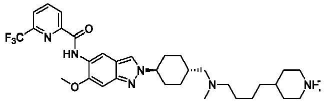 | N-(6-methoxy-2-((1r,4r)-4-((methyl(4-(piperidin-4-yl)butyl)amino) methyl)cyclohexyl)-2H-indazol-5-yl)-6-(trifluoromethyl) pyridinecarboxamide | (ESI,*m*/*z*):[M+H]⁺ =601.2. |

### Intermediate 83:N-(6-methoxy-2-((1r,4r)-4-((methyl (piperidin-4-ylmethyl) amino) methyl) cyclohexyl)-2H-indazol-5-yl)-6-(trifluoromethyl) pyridinecarboxamide

### Step Etert-butyl 4-(((((1r,4r)-4-(6-methoxy-5-(6-(trifluoromethyl) pyridinecarboxamido)-2H-indazol-2-yl) cyclohexyl) methyl) (methyl) amino) methyl) piperidine-1-formate

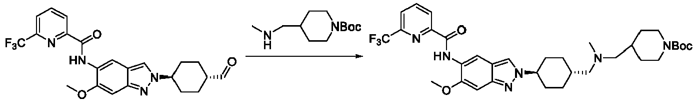

A mixture of N-(2-((1r,4r)-4-formylcyclohexyl)-6-methoxy-2H-indazol-5-yl)-6-(trifluoromethyl) pyridinecarboxamide (100 mg,0.22 mmol), tert-butyl 4-((methylamino) methyl) piperidine-1-formate (51mg,0.22 mmol) and sodium triaceoxyborohydride(142mg, 0.67mmol) in tetrahydrofuran (10mL) was stirred overnight at room temperature. The reaction mixture was quenched with water (50mL) and extracted with dichloromethane (3 × 20mL), the organic layer was collected, washed with saturated saline (60mL), dried over anhydrous sodium sulfate, filtered and concentrated. The concentrate was purified by column chromatography to obtain 120mg of target product as a light yellow solid.

LC-MS: (ESI, *m*/*z*): [M+H]⁺=659.4.

### Step 2:N-(6-methoxy-2-((1r,4r)-4-((methyl (piperidin-4-ylmethyl) amino) methyl) cyclohexyl)-2H-indazol-5-yl)-6-(trifluoromethyl) pyridinecarboxamide

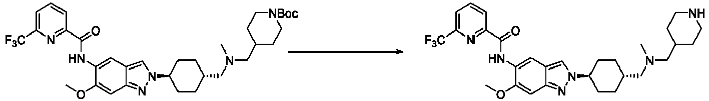

Trifluoroacetic acid (2mL) was added to a mixture of tert-butyl 4-((((1r,4r)-4-(6-methoxy-5-(6-(trifluoromethyl) pyridinecarboxamido)-2H-indazol-2-yl) cyclohexyl) methyl) (methyl) amino) methyl) piperidine-1-formate (120mg,0.18mmol) in dichloromethane (5mL). The reaction mixture was stirred at room temperature for 2h. The reaction mixture was concentrated, to obtain 200 mg of crude target product as a light yellow solid was obtained. The crude product was used directly for the next step.

LC-MS: (ESI, *m*/*z*): [M+H]⁺=559.2.

### Intermediate 84:tert-butyl 9-amino-3-azaspiro[5.5] undecyl-3-carboxylate

### Step 1:tert-butyl 9-(benzylamino)-3-azaspiro[5.5] undecyl-3-carboxylate

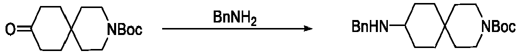

A mixture of tert-butyl 9-oxo-3-azaspiro[5.5] undecyl-3-carboxylate (267mg,1.0 mmol), benzylamine(321 mg,3.0 mmol) and sodium triacetoxyborohydride(2.10g,10.0mmol) in tetrahydrofuran (30 mL) was stirred for 2 hours at room temperature. The reaction mixture was quenched with water (100mL) and extracted with dichloromethane (3 × 50mL). The organic layer was collected and washed with saturated saline (150mL), dried over anhydrous sodium sulfate, filtered and concentrated, and the concentrate was purified by column chromatography to obtain 310mg of target compound as a colorless oil.

LC-MS: (ESI, *m*/*z*): [M+H]⁺=359.2

### Step 2: tert-butyl 9-amino-3-azaspiro[5.5] undecyl-3-carboxylate

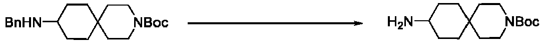

Palladium hydroxide/carbon (303mg) was added to a mixture of tert-butyl 9-(benzylamino)-3-azaspiro[5.5] undecyl-3-carboxylate (310mg,0.86mmol) in methanol (30mL). The reaction mixture was stirred overnight at room temperature in a hydrogen atmosphere. The reaction mixture was filtered through a diatomite mat and the filtrate was concentrated to obtain 200mg of target crude product as a colorless oil. The crude mixture was used directly for the next step.

LC-MS: (ESI, *m*/*z*): [M+H]⁺=269.3.

¹H NMR (400 MHz, CDCl₃) δ 3.49 (s, 1H), 3.35 (d, *J* = 3.3 Hz, 4H), 3.15 (s, 1H), 1.97 (d, *J* = 12.3 Hz, 2H), 1.83 - 1.65 (m, 5H), 1.45 (s, 9H), 1.36 - 1.12 (m, 6H).

### Intermediate 85:N-(6-methoxy-2-((1r,4r)-4-(methyl (3-azaspiro[5.5] undec-9-yl) amino) cyclohexyl)-2H-indazol-5-yl)-6-(trifluoromethyl) pyridinecarboxamide

### Step 1:tert-butyl ((1r,4r)-4-(6-methoxy-5-nitro-2H-indazol-2-yl) cyclohexyl) carbamate

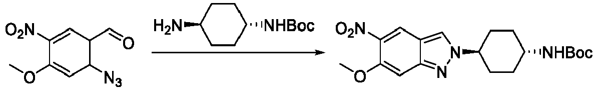

Tert-butyl ((1r,4r)-4-aminocyclohexyl) carbamate(1.03g, 4.8 mmol) was added to a solution of 2-azido-4-methoxy-5-nitrobenzaldehyde (1.07g, 4.8 mmol) in toluene (50 mL), and the mixture was heated to 110°C and stirred overnight. The reaction mixture was concentrated under reduced pressure, and the concentrate was purified by column chromatography to obtain 1.5g of target product as a yellow solid.

LC-MS: (ESI, *m*/*z*): [M+H]⁺=391.2.

### Step 2:tert-butyl ((1r,4r)-4-(5-amino-6-methoxy-2H-indazol-2-yl) cyclohexyl) carbamate

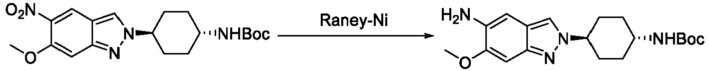

Raney-Ni (100 mg) and hydrazine hydrate (85%, 1.5 mL) were added to a mixture of tert-butyl ((1r,4r)-4-(6-methoxy-5-nitro-2H-indazol-2-yl) cyclohexyl) carbamate (1.5g,3.85mmol) in ethanol (20mL). The reaction mixture was stirred at room temperature for 2 hours, filtered, and the filtrate was concentrated under reduced pressure to obtain 1.15g of the crude target product as a yellow gel, which can be used for the next step without further purification.

LC-MS: (ESI, *m*/*z*): [M+H]⁺=361.2.

### Step 3:tert-butyl ((1r, 4r)-4-(6-methoxy-5-(6-(trifluoromethyl) pyridinecarboxamido)-2H-indazol-2-yl) cyclohexyl) carbamate

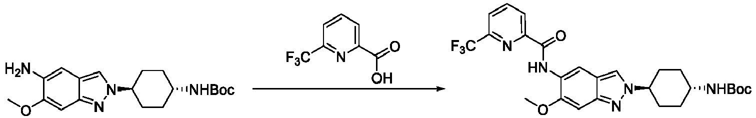

6-(Trifluoromethyl) picolinic acid (609mg,3.19 mmol), HATU(1.5g, 4.0mmol) and N,N-diisopropylethylamine(1.2g,9.3mmol) were added to a mixture of tert-butyl ((1r,4r)-4-(5-amino-6-methoxy-2H-indazol-2-yl) cyclohexyl) carbamate (1.15g, 3.19 mmol) in N,N-dimethylformamide (10mL). The reaction mixture was stirred at room temperature for 2 hours, and the reaction was quenched with water (100mL), and extracted with ethyl acetate (3 × 50mL). The organic layers were collected and washed with saturated saline (150mL), dried over anhydrous sodium sulfate, filtered and concentrated. The concentrate was purified by column chromatography to obtain 1.3g of crude target product as a yellow solid.

LC-MS: (ESI, *m*/*z*): [M+H]⁺=534.3.

### Step 4:N-(2-((1r,4r)-4-aminocyclohexyl)-6-methoxy-2H-indazol-5-yl)-6-(trifluoromethyl) pyridinecarboxamide

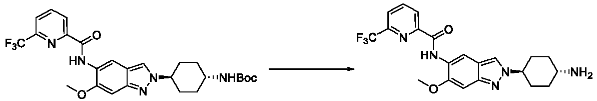

A mixture of tert-butyl ((1r, 4r)-4-(6-methoxy-5-(6-(trifluoromethyl) pyridinecarboxamido)-2H-indazol-2-yl) cyclohexyl) carbamate (400 mg, 0.75 mmol) in trifluoroacetic acid/dichloromethane (8mL,1:3) was stirred at room temperature for 1 hour. The reaction mixture was concentrated under reduced pressure to obtain 430mg of the crude target product as a brown oil. The crude product was used directly for the next reaction.

LC-MS: (ESI, *m*/*z*): [M+H] ⁺ =434.2.

### Step 5:tert-butyl 9-(((1r,4r)-4-(6-methoxy-5-(6-(trifluoromethyl) pyridinecarboxamido)-2H-indazol-2-yl) cyclohexyl) amino)-3-azaspiro[5.5] undecyl-3-carboxylate

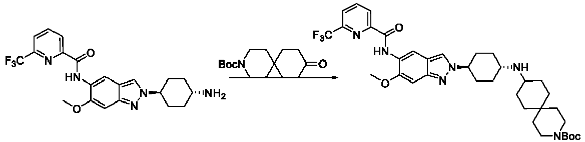

A mixture of N-(2-((1r,4r)-4-aminocyclohexyl)-6-methoxy-2H-indazol-5-yl)-6-(trifluoromethyl) pyridinecarboxamide (430mg, crude), tert-butyl 9-oxo-3-azaspiro[5.5] undecyl-3-carboxylate (210 mg, 0.79 mmol) and sodium triacetoxyborohydride (835 mg, 3.93 mmol) in tetrahydrofuran (10 mL) was stirred overnight at room temperature. The reaction mixture was diluted with water (30mL) and extracted with ethyl acetate (3 × 30mL). The organic layer was collected, washed with saturated saline (50mL), dried over anhydrous sodium sulfate, filtered and concentrated. The concentrate was purified by column chromatography to obtain 400mg of target product as a light yellow solid.

LC-MS: (ESI, *m*/*z*): [M+H] ⁺ = 685.4.

### Step 6: tert-butyl 9-(((1r,4r)-4-(6-methoxy-5-(6-(trifluoromethyl) pyridinecarboxamido)-2H-indazol-2-yl) cyclohexyl) (methyl) amino)-3-azaspiro[5.5] undecyl-3-carboxylate

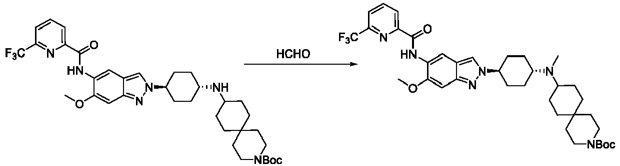

A mixture of tert-butyl 9-(((1r,4r)-4-(6-methoxy-5-(6-(trifluoromethyl) pyridinecarboxamido)-2H-indazol-2-yl) cyclohexyl) amino)-3-azaspiro[5.5] undecyl-3-carboxylate (400mg,0.58mmol), formaldehyde (0.5mL) and sodium triacetoxylborohydride(615mg, 2.9mmol) in tetrahydrofuran (10mL) was stirred overnight at room temperature. The reaction mixture was diluted with water (30mL) and extracted with ethyl acetate (3 × 30mL). The organic layer was collected, washed with saturated brine (50mL), dried over anhydrous sodium sulfate, filtered and concentrated. The concentrate was purified by column chromatography to obtain 300mg of the target product as a yellow oil.

LC-MS: (ESI, *m*/*z*): [M+H] ⁺=699.3.

### Step 7:N-(6-methoxy-2-((1r,4r)-4-(methyl (3-azaspiro[5.5] undec-9-yl) amino) cyclohexyl)-2H-indazol-5-yl)-6-(trifluoromethyl) pyridinecarboxamide

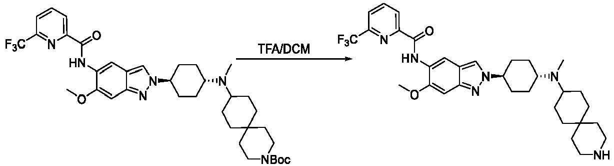

A mixture of tert-butyl 9-(((1r,4r)-4-(6-methoxy-5-(6-(trifluoromethyl) pyridinecarboxamido)-2H-indazol-2-yl) cyclohexyl) (methyl) amino)-3-azaspiro[5.5] undecyl-3-carboxylate (300 mg, 0.43 mmol) in trifluoroacetic acid/dichloromethane (6 mL, 1:2) was stirred and reacted at room temperature for 1 hour. The reaction mixture was concentrated under reduced pressure to obtain 330mg of the crude target product as a brown oil. The crude product was used directly for the next step.

LC-MS: (ESI, *m*/*z*): [M+H] ⁺ =599.4.

The following intermediates were prepared with reference to intermediate 85.

| Intermediate | Structure | Name | LC-MS |
|---|---|---|---|
| Intermediate 86 | 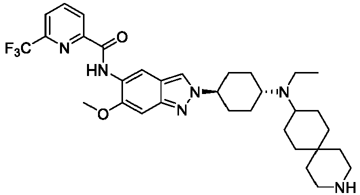 | N-(2-((1r,4r)-4-(ethyl (3-azaspiro[5.5] undec-9-yl)amino) cyclohexyl)-6-methoxy-2H-indazol-5-yl)-6-(trifluoromethyl) pyridinecarboxamide | LC-MS: (ESI, *m*/*z*): [M+H] ⁺ = 613.4. |

### Intermediate 87 :N-(6-methoxy-2-(3-(piperidin-4-ylmethyl)-3-azaspiro [5.5] undec-9-yl)-2H-indazol-5-yl)-6-(trifluoromethyl) pyridinecarboxamide

### Step 1:tert-butyl 9-(6-methoxy-5-nitro-2H-indazol-2-yl)-3-azaspiro[5.5] undecyl-3-carboxylate

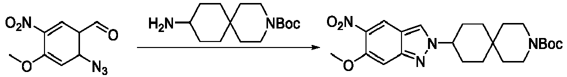

Tert-butyl 9-amino-3-azaspiro[5.5] undecyl-3-carboxylate (498mg, 1.85 mmol) was added to a solution of 2-azido-4-methoxy-5-nitrobenzaldehyde (416 mg, 1.85 mmol) in toluene (50 mL). The reaction mixture was heated to 110°C and stirred for reaction overnight. The reaction mixture was concentrated under reduced pressure, and the concentrate was purified by column chromatography to obtain 600mg of the target product as a yellow solid.

LC-MS: (ESI, *m*/*z*): [M+H]⁺=445.2.

### Step 2:tert-butyl 9-(5-amino-6-methoxy-2H-indazol-2-yl)-3-azaspiro[5.5] undecyl-3-carboxylate

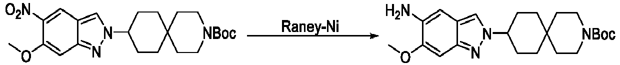

Raney-Ni (60 mg) and hydrazine hydrate (85%, 1 mL) were added to a mixture of tert-butyl 9-(6-methoxy-5-nitro-2H-indazol-2-yl)-3-azaspiro [5.5] undecyl -3-carboxylate (600 mg, 1.35 mmol) in ethanol (20 mL). The reaction mixture was stirred at room temperature for 2 hours, filtered, and concentrated under reduced pressure to obtain 450mg of crude target product as a yellow gel. The crude product can be used for the next step without further purification.

LC-MS: (ESI, *m*/*z*): [M+H]⁺=415.2.

### Step 3:tert-butyl 9-(6-methoxy-5-(6-(trifluoromethyl) pyridinecarboxamido)-2H-indazol-2-yl)-3-azaspiro[5.5] undecyl-3-carboxylate

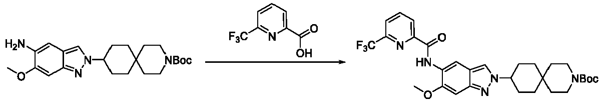

6-(Trifluoromethyl) picolinic acid (208mg, 1.09 mmol), HATU(538mg, 1.42mmol) and N,N-diisopropylethylamine(554mg,4.26mmol) were added to a mixture of tert-butyl 9-(5-amino-6-methoxy-2H-indazol-2-yl)-3-azaspiro[5.5] undecyl -3-carboxylate (450 mg, 1.09 mmol) in N,N-dimethylformamide (10 mL), and the reaction mixture was stirred at room temperature for 2 hours. The reaction was quenched with water (100mL) and extracted with ethyl acetate (3 × 50mL). The organic layer was collected and washed with saturated saline (150mL), dried over anhydrous sodium sulfate, filtered and concentrated. The concentrate was purified by chromatography to obtain 600mg of the target product as a light yellow solid.

LC-MS: (ESI, *m*/*z*): [M+H]⁺=588.2.

### Step 4:N-(6-methoxy-2-(3-azaspiro[5.5] undec-9-yl)-2H-indazol-5-yl)-6-(trifluoromethyl) pyridinecarboxamide

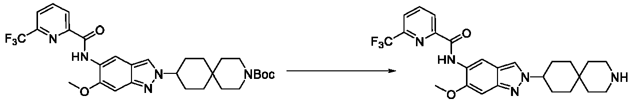

Trifluoroacetic acid (2 mL) was added to a mixture of tert-butyl 9-(6-methoxy-5-(6-(trifluoromethyl) pyridinecarboxamido)-2H-indazol-2-yl)-3-azaspiro[5.5] undecyl-3-carboxylate (200mg,0.34 mmol) in dichloromethane (5 mL). The reaction mixture was stirred at room temperature for 2 hours, and concentrated under reduced pressure to obtain 270mg of the desired crude target product as a light yellow solid, which was directly used for the next step.

LC-MS: (ESI, *m*/*z*): [M+H]⁺=488.3.

### Step 5: tert-butyl 4-((9-(6-methoxy-5-(6-(trifluoromethyl) pyridinecarboxamido)-2H-indazol-2-yl)-3-azaspiro[5.5] undec-3-yl) methyl) piperidine-1-carboxylate

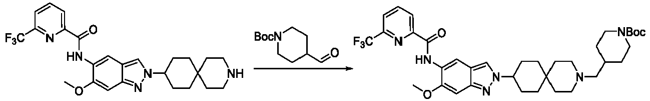

A reaction mixture of N-(6-methoxy-2-(3-azaspiro[5.5] undec-9-yl)-2H-indazol-5-yl)-6-(trifluoromethyl) pyridinecarboxamide (270mg, crude), tert-butyl 4-formylpiperidine-1-carboxylate (130 mg, 0.61 mmol) and sodium triacetoxylborohydride (643 mg, 3.05 mmol) in tetrahydrofuran (10 mL) was stirred and reacted overnight at 60°C. The reaction was quenched with water (50mL) and extracted with dichloromethane (3 × 20mL), and the organic layer was collected, washed with saturated saline (60mL), dried over anhydrous sodium sulfate, filtered and concentrated. The concentrate was purified by column chromatography to obtain 130mg of target product as a light yellow solid.

LC-MS: (ESI, *m*/*z*): [M+H]⁺=685.6.

### Step 6:N-(6-methoxy-2-(3-(piperidin-4-ylmethyl)-3-azaspiro [5.5] undec-9-yl)-2H-indazol-5-yl)-6-(trifluoromethyl) pyridinecarboxamide

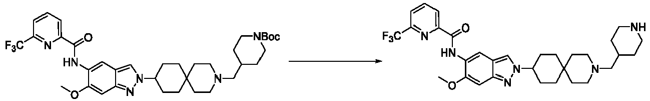

Trifluoroacetic acid (2mL) was added to a stirred mixture of tert-butyl 4-((9-(6-methoxy-5-(6-(trifluoromethyl) pyridinecarboxamido)-2H-indazol-2-yl)-3-azaspiro [5.5] undec-3-yl) methyl) piperidine-1-carboxylate (130mg,0.19mmol) in dichloromethane (5mL). The reaction mixture was stirred at room temperature for 2 hours. The reaction mixture was concentrated to obtain 220mg of crude target product as a light yellow solid, and the crude product was directly used for the next step.

LC-MS: (ESI, *m*/*z*): [M+H]⁺=585.3.

### Intermediate 88:tert-butyl 9-(4-(6-methoxy-5-nitro-2H-indazol-2-yl) cyclohexyl)-3, 9-diazaspiro [5.5] undecyl-3-carboxylate

### Step 1: 4-aminocyclohexyl-1-one

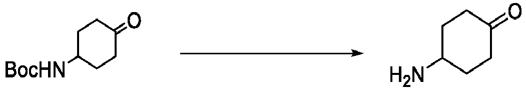

Trifluoroacetic acid (5mL) was added to a solution of tert-butyl (4-oxocyclohexyl) carbamate (2.13g,10mmol) in dichloromethane (20mL), and the reaction mixture was stirred at room temperature for 3 hours. The reaction mixture was concentrated under reduced pressure to obtain 2.1g of the crude target product, which can be used for the next step without further purification.

### Step 2:4-(6-methoxy-5-nitro-2H-indazol-2-yl) cyclohexyl-1-one

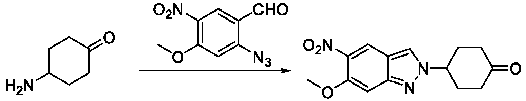

2-Azido-4-methoxy-5-nitrobenzaldehyde (2.22g, 10 mmol) was added to a reaction mixture of 4-aminocyclohexyl-1-one (crude, 2.1g) in toluene (50mL), and the reaction mixture was heated to 100°C and stirred for reaction overnight, and concentrated under reduced pressure. The concentrate was purified by column chromatography to obtain 1.5g of target compound as a yellow solid.

### LC-MS: (ESI, m/z): [M+H]⁺=290.1

### Step 3:tert-butyl 9-(4-(6-methoxy-5-nitro-2H-indazol-2-yl) cyclohexyl)-3, 9-diazaspiro [5.5] undecyl-3-carboxylate

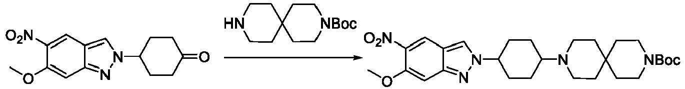

A reaction mixture of 4-(6-methoxy-5-nitro-2H-indazol-2-yl) cyclohexyl-1-one (500 mg, 1.73 mmol), tert-butyl 3,9-diazaspiro [5.5] undecyl-3-carboxylate (484 mg, 1.90 mmol), sodium triacetoxyborohydride (4.00g, 18.95 mmol) and acetic acid (12 mg, 0.19 mmol) in dichloroethane (100 mL) was refluxed overnight. The reaction mixture was quenched with water (100mL), and extracted with dichloromethane (3 × 50mL). The organic layer was collected, washed with saturated saline (200mL), dried over anhydrous sodium sulfate, filtered and concentrated. The concentrate was purified by column chromatography to obtain the target products P1:150mg and P2:160 mg as a light yellow solid.

### P1: tert-butyl 9-((1r,4r)-4-(6-methoxy-5-nitro-2H-indazol-2-yl) cyclohexyl)-3, 9-diazaspiro [5.5] undecyl-3-carboxylate

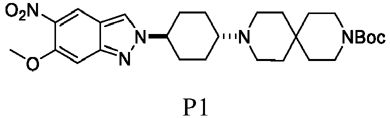

LC-MS-P1: (ESI, *m*/*z*): [M+H]⁺=528.3.

### P2: tert-butyl 9-((1s,4s)-4-(6-methoxy-5-nitro-2H-indazol-2-yl) cyclohexyl)-3, 9-diazaspiro [5.5] undecyl-3-carboxylate

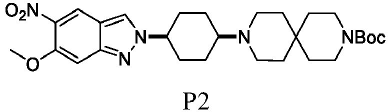

LC-MS-P2: (ESI, *m*/*z*): [M+H]⁺=528.3.

### Intermediate 89:N-(2-((1s, 4s)-4-(3, 9-diazaspiro [5.5] undec-3-yl) cyclohexyl)-6-methoxy-2H-indazol-5-yl)-6-(trifluoromethyl) pyridinecarboxamide

### Step 1:tert-butyl 9-((1s, 4s)-4-(5-amino-6-methoxy-2H-indazol-2-yl) cyclohexyl)-3, 9-diazaspiro [5.5] undecyl-3-carboxylate

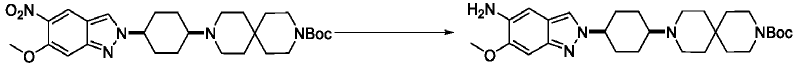

Palladium/carbon (100 mg) was added to a solution of tert-butyl 9-(4-(6-methoxy-5-nitro-2H-indazol-2-yl) cyclohexyl)-3, 9-diazaspiro[5.5] undecyl-3-carboxylate (P2, 160mg, 0.30 mmol) in methanol (10 mL). The resulting mixture was stirred for 4 hours at room temperature in a hydrogen atmosphere. The reaction mixture was filtered through diatomite and the filtrate was concentrated to obtain 140mg of crude target product as a light yellow solid. The crude mixture was used directly for the next step.

LC-MS: (ESI, *m*/*z*): [M+H]⁺=498.3.

### Step 2:tert-butyl 9-((1s, 4s)-4-(6-methoxy-5-(6-(trifluoromethyl) pyridinecarboxamido)-2H-indazol-2-yl) cyclohexyl)-3, 9-diazaspiro[5.5] undecyl-3-carboxylate

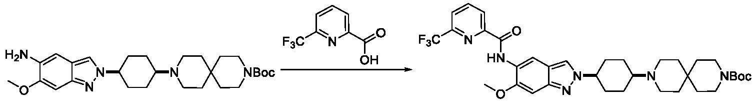

6-(Trifluoromethyl) picolinic acid (58mg,0.30mmol), HATU(149mg,0.39mmol) and N,N-diisopropylethylamine(117mg,0.90mmol) were added to a stirred solution of tert-butyl 9-((1s, 4s)-4-(5-amino-6-methoxy-2H-indazol-2-yl) cyclohexyl)-3, 9-diazaspiro [5.5] undecyl-3-carboxylate (140mg,0.23mmol) in N,N-dimethylformamide (3mL). The resulting reaction mixture was stirred for 2 hours at room temperature. The reaction mixture was quenched with water (50mL), and extracted with ethyl acetate (3 × 20mL). The organic layers were collected and washed with saturated saline (60mL), dried over anhydrous sodium sulfate, filtered and concentrated. The concentrate was purified by chromatographic column to obtain 90mg of target product as light yellow solid.

LC-MS: (ESI, *m*/*z*): [M+H]⁺=671.3.

### Step 3:N-(2-((1s, 4s)-4-(3, 9-diazaspiro [5.5] undec-3-yl) cyclohexyl)-6-methoxy-2H-indazol-5-yl)-6-(trifluoromethyl) pyridinecarboxamide

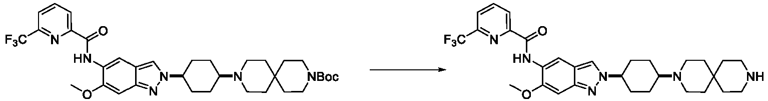

Trifluoroacetic acid (2mL) was added to a solution of tert-butyl 9-((1s, 4s)-4-(6-methoxy-5-(6-(trifluoromethyl) pyridinecarboxamido)-2H-indazol-2-yl) cyclohexyl)-3, 9-diazaspiro[5.5] undecyl-3-carboxylate (90mg,0.13mmol) in dichloromethane (5mL). The resulting reaction mixture was stirred for 2 hours at room temperature. The reaction mixture was concentrated to obtain 120mg of the crude target product as a light yellow solid.

LC-MS: (ESI, *m*/*z*): [M+H]⁺=571.3

The following compounds were prepared with reference to the method of intermediate 89.

| Intermediate | Structure | Name | LC-MS |
|---|---|---|---|
| Intermediate 90 | 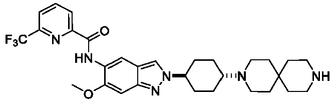 | N-(2-((1r,4r)-4-(3, 9-diazaspiro [5.5] undec-3-yl) cyclohexyl)-6-methoxy-2H-indazol-5-yl)-6-(trifluoromethyl) pyridinecarboxamide | (ESI,*m*/*z*): |
| | | | [M+H]⁺=571.2. |

### Intermediate 91: N-(6-methoxy-2-((1r,4r)-4-((2-(piperidin-4-yl) ethoxy) methyl) cyclohexyl)-2H-indazol-5-yl)-6-(trifluoromethyl) pyridinecarboxamide

### Step 1:benzyl 4-(2-hydroxyethyl) piperidine-1-carboxylate

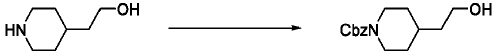

Benzyl carbonyl chloride (5.06g, 29.8 mmol) was added to a mixture of 2-(piperidin-4-yl) ethyl-1-ol (3.2g, 24.8 mmol) in saturated sodium bicarbonate aqueous solution (30 mL) and water (30 mL). The reaction mixture was stirred overnight at room temperature, and extracted with ethyl acetate (3 × 100mL). The organic layer was collected, washed with saturated saline (50mL), dried over anhydrous sodium sulfate, filtered and concentrated. The concentrate was purified by column chromatography to obtain 6.0g of the target product as a yellow oil.

LC-MS: (ESI, *m*/*z*): [M+H] ⁺ =264.3.

### Step 2:benzyl 4-(2-(toluenesulfonyloxy) ethyl) piperidine-1-carboxylate

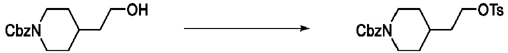

A mixture of benzyl 4-(2-hydroxyethyl) piperidine-1-carboxylate (6.0g, 22.8 mmol), p-toluenesulfonyl chloride(5.23g, 27.4 mmol), triethylamine (6.9g, 68.4 mmol) and 4-dimethylaminopyridine (0.278g, 2.28 mmol) in dichloromethane (100 mL) was stirred overnight at room temperature. The reaction mixture was diluted with water (100mL) and extracted with dichloromethane (3 × 100mL). The organic layers were collected and washed with saturated saline (200mL), dried over anhydrous sodium sulfate, filtered and concentrated. The concentrate was purified by column chromatography to obtain 8.0g of the target product as a white solid.

¹H NMR (400 MHz, DMSO) δ 7.85- 7.75 (m, 2H), 7.49 (d, J = 8.0 Hz, 2H), 7.43- 7.24 (m, 5H), 5.05 (s, 2H), 4.08 - 4.02 (m, 2H), 3.92 (d, J = 13.2 Hz, 2H), 2.68 (s, 2H), 2.42 (s, 3H), 1.55-1.38 (m, 5H), 1.01 - 0.85 (m, 2H).

### Step 3:benzyl 4-(2-(((1r, 4r)-4-((tert-butoxycarbonyl) amino) cyclohexyl) methoxy) ethyl) piperidine-1-carboxylate

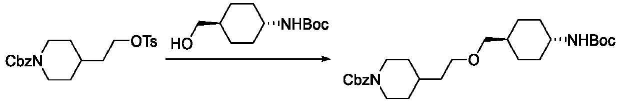

At 0 °C, sodium hydride (60%,210mg) was added to a solution of tert-butyl ((1r,4r)-4-(hydroxymethyl) cyclohexyl) carbamate (1.0g,4.37mmol) in anhydrous tetrahydrofuran (20 mL), and the reaction mixture was stirred at this temperature for 1 hour, benzyl 4-(2-(toluenesulfonyloxy) ethyl) piperidine-1-carboxylate (2.0g,4.80mmol) was added, the reaction mixture was heated to 60°C and refluxed overnight. Water (50mL) was slowly added, extracted with ethyl acetate (100mL * 3), the organic layer was collected, washed with saturated saline (50mL), concentrated under reduced pressure, and the concentrate was purified by column chromatography to obtain 1.2g of target product as a colorless oil.

LC-MS: (ESI, *m*/*z*): [M+H] ⁺ =475.3.

### Step 4: benzyl 4-(2-(((1r, 4r)-4-aminocyclohexyl) methoxy) ethyl) piperidine-1-carboxylate

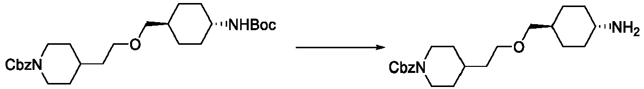

Trifluoroacetic acid (20 mL) was added to a solution of benzyl 4-(2-(((1r, 4r)-4-((tert-butoxycarbonyl) amino) cyclohexyl) methoxy) ethyl) piperidine-1-carboxylate (1.4g,2.95mmol) in dichloromethane (50 mL), and the reaction mixture was stirred at room temperature for 2 hours. The reaction mixture was concentrated under reduced pressure to obtain 1.4g of crude target product. It can be used for the next step without further purification.

LC-MS: (ESI, *m*/*z*): [M+H] ⁺ =375.3.

### Step 5:benzyl 4-(2-(((1r, 4r)-4-(6-methoxy-5-nitro-2H-indazol-2-yl) cyclohexyl) methoxy) ethyl) piperidine-1-carboxylate

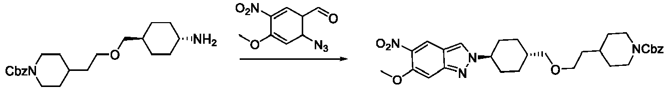

Benzyl 4-(2-(((1r, 4r)-4-aminocyclohexyl) methoxy)ethyl) piperidine-1-carboxylate (1.4 g, 2.95 mmol) was added to a solution of 2-azido -4-methoxy-5-nitrobenzaldehyde (661 mg, 2.95 mmol) in toluene (50 mL), the reaction mixture was heated to 110°C and stirred for reaction overnight, concentrated under reduced pressure, and the concentrate was purified by column chromatography to obtain 0.9g of target product as a yellow solid.

LC-MS: (ESI, *m*/*z*): [M+H] ⁺ =551.2.

### Step 6:benzyl 4-(2-(((1r, 4r)-4-(5-amino-6-methoxy-2H-indazol-2-yl) cyclohexyl) methoxy) ethyl) piperidine-1-carboxylate

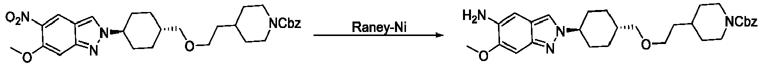

Raney-Ni (100 mg) and hydrazine hydrate (85%, 1 mL) were added to a mixture of benzyl 4-(2-(((1r, 4r)-4-(6-methoxy-5-nitro-2H-indazol-2-yl) cyclohexyl) methoxy) ethyl) piperidine-1-carboxylate (570,1.0mmol) in ethanol (20 mL), the reaction mixture was stirred for 2 hours at room temperature, filtered, and the filtrate was concentrated under reduced pressure to obtain 500mg of crude target product as a yellow gel. The crude product can be used for the next step without further purification.

LC-MS: (ESI, *m*/*z*): [M+H] ⁺ =521.4.

### Step 7:benzyl 4-(2-(((1r,4r)-4-(6-methoxy-5-(6-(trifluoromethyl) pyridinecarboxamido)-2H-indazol-2-yl) cyclohexyl) methoxy) ethyl) piperidine-1-carboxylate

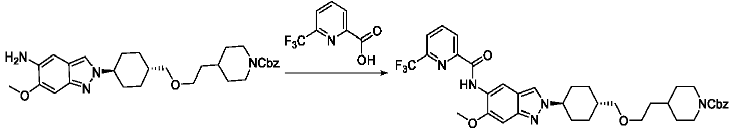

6-(Trifluoromethyl) picolinic acid (184mg, 0.96mmol), HATU(474mg,1.25mmol) and N,N-diisopropylethylamine(372mg,2.88mmol) were added to a mixture of benzyl 4-(2-(((1r, 4r)-4-(5-amino-6-methoxy-2H-indazol-2-yl) cyclohexyl) methoxy) ethyl) piperidine-1-carboxylate(500mg,0.96mmol) in N,N-dimethylformamide (10mL). The reaction mixture was stirred at room temperature for 2 hours. The reaction was quenched with water (20mL). The resulting solution was extracted with ethyl acetate (3 × 50mL). The organic layers were collected and washed with saturated saline (50mL), dried over anhydrous sodium sulfate, filtered and concentrated. The concentrate was purified by column chromatography to obtain 700mg of target product as a yellow solid, ¹H-NMR showed that the target product contained N,N-dimethylformamide.

LC-MS: (ESI, *m*/*z*): [M+H] ⁺ =694.2.

### Step 8:N-(6-methoxy-2-((1r,4r)-4-((2-(piperidin-4-yl) ethoxy) methyl) cyclohexyl)-2H-indazol-5-yl)-6-(trifluoromethyl) pyridinecarboxamide

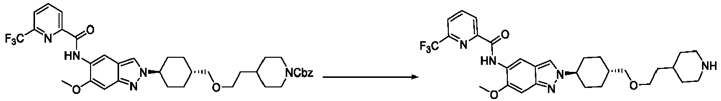

Palladium/carbon (10%, 70 mg) was added to a mixture of benzyl 4-(2-(((1r,4r)-4-(6-methoxy-5-(6-(trifluoromethyl) pyridinecarboxamido)-2H-indazol-2-yl) cyclohexyl) methoxy) ethyl) piperidine-1-carboxylate(700 mg, 0.96 mmol) in ethyl acetate (20 mL), the reaction mixture was stirred overnight at room temperature in a hydrogen atmosphere, filtered, the solid was washed with ethyl acetate (20mL), and the organic layer was collected, concentrated under reduced pressure to obtain 550mg of crude target product. The crude product can be used for the next step without further purification.

LC-MS: (ESI, *m*/*z*): [M+H] ⁺ =560.3.

### Intermediate 92:N-(2-((1r,4r)-4-(([1,4 '-bipiperidin]-4-yl (methyl) amino) methyl) cyclohexyl)-6-methoxy-2H-indazol-5-yl)-6-(trifluoromethyl) pyridinecarboxamide

### Step Ltert-butyl 4-(((1r,4r)-4-(6-methoxy-5-(6-(trifluoromethyl) pyridinecarboxamido)-2H-indazol-2-yl) cyclohexyl) methyl) (methyl) amino)- [1,4 '-bipiperidine]-1'-carboxylate

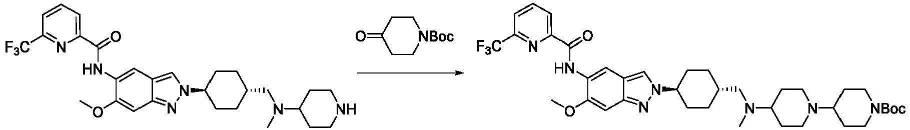

N-(6-methoxy-2-((1r,4r)-4-((methyl (piperidin-4-yl) amino) methyl) cyclohexyl)-2H-indazol-5-yl)-6-(trifluoromethyl) pyridinecarboxamide (280 mg, 0.40 mmol) and tert-butyl 4-oxopiperidine-1-carboxylate (88 mg, 0.44 mmol) were dissolved with 1,2-dichloroethane (20 mL), the temperature was increased to 70 °C, three drops of acetic acid were added, sodium triacetoxyborohydride (844 mg, 4.0 mmol) was divided into five parts, one part was added to the reaction solution every 1 hour, and stirred overnight at 70 °C. The reaction solution was cooled to room temperature, added with water (30 mL), extracted with dichloromethane (3x20 mL), and the organic phase was washed with saturated saline (50 mL). The organic phase was dried over anhydrous sodium sulfate, filtered, concentrated under reduced pressure, and the crude product was purified by column chromatography to obtain 70 mg of target product as a light yellow solid.

LC-MS: (ESI, *m*/*z*): [M+H] ⁺ =728.4.

### step 2: N-(2-((1r,4r)-4-(([1,4'-bipiperidin]-4-yl (methyl) amino) methyl) cyclohexyl)-6-methoxy-2H-indazol-5-yl)-6-(trifluoromethyl) pyridinecarboxamide

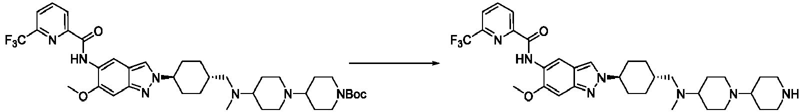

Tert-butyl 4-((((1r,4r)-4-(6-methoxy-5-(6-(trifluoromethyl) pyridinecarboxamido)-2H-indazol-2-yl) cyclohexyl) methyl) (methyl) amino)-[1,4 '-biperidine]-1'-carboxylate (70 mg, 0.096mmol) was dissolved with dichloromethane (5 mL), trifluoroacetic acid (2 mL) was added and stirred at room temperature for 2 hours, directly concentrated under reduced pressure to obtain 100 mg of crude target product as a yellow solid, which was directly used for the next reaction without purification.

LC-MS: (ESI, *m*/*z*): [M+H] ⁺ =628.3.

### Intermediate 93:N-(2-(1-((3-azaspiro [5.5] undec-9-yl) methyl) piperidin-4-yl)-6-(difluoromethoxy)-2H-indazol-5-yl)-6-(trifluoromethyl)) pyridinecarboxamide

### Step Ltert-butyl 4-(6-hydroxy-5-nitro-2H-indazol-2-yl) piperidine-1-carboxylate

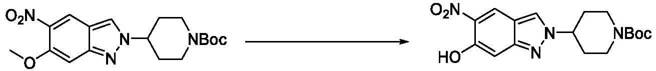

Tert-butyl 4-(6-methoxy-5-nitro-2H-indazol-2-yl) piperidine-1-carboxylate (5.0g, 13.29mmol) was dissolved in hydrogen bromide aqueous solution (48%,60 mL), heated to 110 °C, stirred for 48 hours, adjusted the pH value of the reaction solution to 9-10 with sodium hydroxide aqueous solution, and ditert-butyl dicarbonate (3.73g, 13.29mmiol) and 4-dimethylaminopyridine (324mg, 2.65mmol) were added, stirred overnight at room temperature, extracted with ethyl acetate (3 × 100 mL), the organic phases was washed with saturated saline (300 mL), dried over anhydrous sodium sulfate, filtered, concentrated under reduced pressure, and the crude product was purified by column chromatography to obtain 530 mg of target compound as an orange-red solid.

LC-MS: (ESI, *m*/*z*): [M+H] ⁺ =363.1.

¹H NMR (400 MHz, DMSO) δ 10.47 (s, 1H), 8.62 (s, 1H), 8.42 (s, 1H), 7.02 (s, 1H), 4.68 (tt, *J =* 11.4, 4.0 Hz, 1H), 4.18 - 3.98 (m, 2H), 2.94 (m, 2H), 2.10 (d, *J =* 10.1 Hz, 2H), 1.92 (qd, *J =* 12.3, 4.3 Hz, 2H), 1.43 (s, 9H).

### Step 2:tert-butyl 4-(6-(difluoromethoxy)-5-nitro-2H-indazol-2-yl) piperidine-1-carboxylate

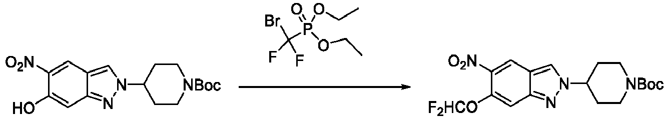

Sodium hydroxide (1.17g, 29.2 mmol) was dissolved in water (10 mL) and acetonitrile (10 mL), cooled to -20 °C, tert-butyl 4-(6-hydroxy-5-nitro-2H-indazol-2-yl) piperidine-1-carboxylate(530 mg,1.46 mmol) was added, diethyl (bromodifluoromethyl) phosphonate (782 mg,2.92 mmol) was slowly added, stirred at -20 °C for 3h, extracted with ethyl acetate (3 × 30 mL), the organic phase was washed with saturated saline (90 mL), dried over anhydrous sodium sulfate, filtered, concentrated under reduced pressure, and the crude product was purified to obtain 420 mg of the target product.

LC-MS: (ESI, *m*/*z*): [M-55] ⁺ =357.2.

¹H NMR (400 MHz, DMSO) δ 8.84 (s, 1H), 8.68 (s, 1H), 7.64 (s, 1H), 7.31 (t, J = 73.2 Hz, 1H), 4.89 - 4.75 (m, 1H), 4.11 (d, J = 12.0 Hz, 2H), 2.97 (s, 2H), 2.13 (d, J = 10.5 Hz, 2H), 2.02 - 1.89 (m, 3H), 1.44 (s, 9H).

### Step 3:tert-butyl 4-(5-amino-6-(difluoromethoxy)-2H-indazol-2-yl) piperidine-1-carboxylate

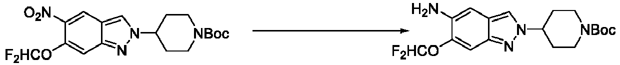

Tert-butyl 4-(6-(difluoromethoxy)-5-nitro-2H-indazol-2-yl) piperidine -1-carboxylate (420 mg,1.02 mmol) was added to ethanol (20 mL), Raney-Ni(0.5 mL) and hydrazine hydrate (0.5 mL) were added under stirring, stirred for 2 hours at room temperature, filtered, and the filter cake was washed twice with ethanol (5 mLX2), concentrated under reduced pressure to obtain 380mg of the target compound, which can be directly used in the next reaction without purification.

LC-MS: (ESI, *m*/*z*): [M+H]⁺=383.1

### Step 4:tert-butyl 4-(6-(difluoromethoxy)-5-(6-(trifluoromethyl) pyridinecarboxamido)-2H-indazol-2-yl) piperidine-1-carboxylate

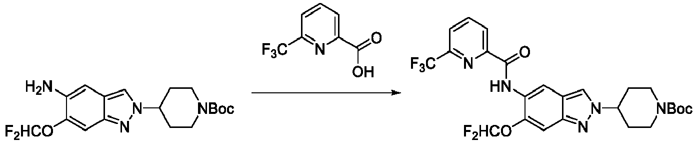

Tert-butyl 4-(5-amino-6-(difluoromethoxy)-2H-indazol-2-yl) piperidine-1-carboxylate (380 mg, 0.99 mmol) was dissolved in N,N-dimethylformamide (5mL), 6-(trifluoromethyl) picolinic acid (229 mg, 1.2 mmol), HATU(494 mg, 1.3 mmol) and N,N-diisopropylethylamine (387 mg, 3.0 mmol) were added, stirred at room temperature for 2 hours, water (20 mL) was added, filtered, the filter cake was washed with water (10mL × 2) twice to obtain crude product, and the crude product was purified by column chromatography to obtain 400 mg of target compound.

LC-MS: (ESI, *m*/*z*): [M-55] ⁺ =556.0.

¹H NMR (400 MHz, DMSO) δ 10.51 (s, 1H), 8.69 (s, 1H), 8.46 (d, *J* = 7.4 Hz, 1H), 8.41 (t, *J* = 7.8 Hz, 1H), 8.35 (s, 1H), 8.22 (dd, *J* = 7.7, 1.0 Hz, 1H), 7.15 (s, 1H), 4.39 (ddd, *J =* 11.7, 8.0, 3.8 Hz, 1H), 3.97 (d, *J =* 14.3 Hz, 5H), 3.04 (s, 1H), 2.76 (d, *J =* 6.1 Hz, 4H), 2.17 (d, *J =* 10.1 Hz, 2H), 2.00 (d, *J =* 8.7 Hz, 4H), 1.93 - 1.84 (m, 2H), 1.73 (s, 1H), 1.39 (d, *J* = 15.8 Hz, 11H), 1.21 (dt, *J =* 14.3, 9.8 Hz, 2H).

### Step 5:N-(6-(difluoromethoxy)-2-(piperidin-4-yl)-2H-indazol-5-yl)-6-(trifluoromethyl) pyridinecarboxamide

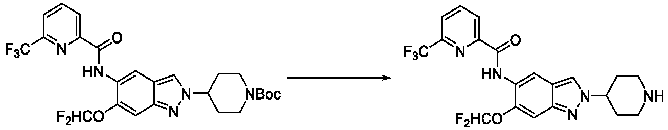

Tert-butyl 4-(6-(difluoromethoxy)-5-(6-(trifluoromethyl) pyridinecarboxamide)-2H-indazol-2-yl) piperidine-1-carboxylate(200 mg, 0.36mmol) was dissolved in dichloromethane (5 mL), trifluoroacetic acid (2 mL) was added and stirred at room temperature for 2 hours, directly concentrated under reduced pressure to obtain 220 mg of crude target product, which was directly used for the next reaction without purification.

LC-MS: (ESI, *m*/*z*): [M+H] ⁺ =456.1.

### Step 6: tert-butyl 9-((4-(6-(difluoromethoxy)-5-(6-(trifluoromethyl) pyridinecarboxamido)-2H-indazol-2-yl) piperidin-1-yl) methyl)-3-azaspiro[5.5] undecyl-3-carboxylate

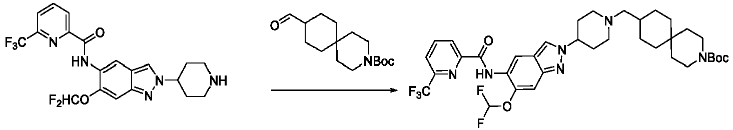

N-(6-(difluoromethoxy)-2-(piperidin-4-yl)-2H-indazol-5-yl)-6-(trifluoromethyl) pyridinecarboxamide (220 mg) and tert-butyl 9-formyl-3-azaspiro[5.5] undecyl-3-carboxylate (109 mg, 0.38 mmol) were dissolved in tetrahydrofuran (10 mL), stirred at room temperature, sodium triacetoxyborohydride (371 mg, 1.76 mmol) was added and stirred at room temperature for 2 hours, water (30 mL) was added, extracted with dichloromethane (3 × 30 mL), the organic phase was washed with saturated saline (100 mL), dried over anhydrous sodium sulfate, filtered, concentrated under reduced pressure, and the crude product was purified by column chromatography to obtain 100 mg of the target product.

LC-MS: (ESI, *m*/*z*): [M+H] ⁺ =721.7.

### Step 7:N-(2-(1-((3-azaspiro[5.5] undec-9-yl) methyl) piperidin-4-yl)-6-(difluoromethoxy)-2H-indazol-5-yl)-6-(trifluoromethyl)) pyridinecarboxamide

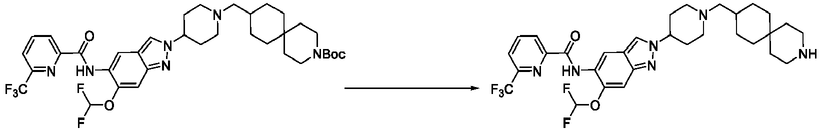

Tert-butyl 9-((4-(6-(difluoromethoxy)-5-(6-(trifluoromethyl) pyridinecarboxamido)-2H-indazol-2-yl) piperidin-1-yl) methyl)-3-azaspiro[5.5] undecyl-3-carboxylate (100 mg, 0.138mmol) was dissolved with dichloromethane (5 mL), trifluoroacetic acid (2 mL) was added and stirred at room temperature for 2 hours, and it was directly concentrated under reduced pressure to obtain 120 mg of target crude product, which was directly used for the next reaction without purification.

LC-MS: (ESI, *m*/*z*): [M+H] ⁺ =621.2.

### Intermediate 94:tert-butyl 4-(4-aminobutyl) piperidine-1-carboxylate

### Step 1:tert-butyl 4-(4-(1, 3-dioxoisoindolin-2-yl) butyl) piperidine-1-carboxylate

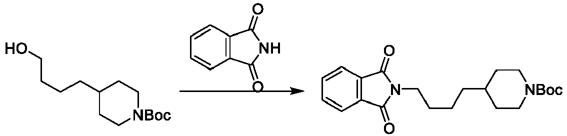

Tert-butyl 4-(4-hydroxybutyl) piperidine-1-carboxylate (1.2g, 4.73 mmol), phthalimide (0.82g, 5.52 mmol) and triphenylphosphine (1.45g, 5.52 mmol) were dissolved in anhydrous tetrahydrofuran (25 mL), the reaction solution was stirred at room temperature under nitrogen protection for 10 minutes, and then cooled to 0 °C, diethyl azodicarate(0.87 mL, 5.52 mmol) was slowly added dropwise to the reaction solution at 0 °C, the ice bath was removed, stirred at room temperature for 7 hours, the solvent was removed by concentration under reduced pressure, and the concentrate was purified by column chromatography (20% ethyl acetate/petroleum ether) to obtain 1.5g of target compound as a white solid.

LC-MS: (ESI, *m*/*z*): [M-99]⁺=387.2.

### Step 2:tert-butyl 4-(4-aminobutyl) piperidine-1-carboxylate

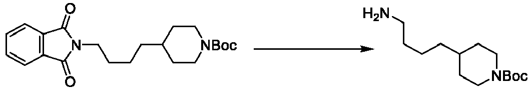

Tert-butyl 4-(4-hydroxybutyl) piperidine-1-carboxylate(1.5g,3.88 mmol) and hydrazine hydrate (5 mL) were added to ethanol (20 mL), heated and refluxed overnight, cooled to room temperature, filtered, the filter cake was washed twice with ethanol (5 mLX2), and concentrated under reduced pressure to obtain 1.0g of target compound as a colorless oil, which can be directly used for the next reaction without purification.

¹H NMR (400 MHz, DMSO) δ 3.95 (t, J = 27.6 Hz, 2H), 2.64 (s, 2H), 2.57 - 2.52 (m, 2H), 1.60 (d, J = 12.4 Hz, 2H), 1.48 - 1.21 (m, 14H), 1.17 (dd, J = 13.9, 6.3 Hz, 2H), 0.92 (qd, J = 12.5, 4.2 Hz, 2H).

### Intermediate 95:N-(2-(1-((3-azaspiro[5.5] undec-9-yl) methyl) piperidin-4-yl)-6-(2-methoxyethoxy)-2H-indazol-5-yl)-6-(trifluoromethyl) pyridinecarboxamide

### Step 1: 5-nitro-2-(piperidin-4-yl)-2H-indazol-6-ol

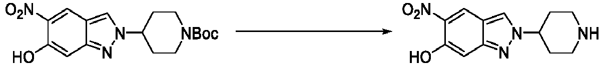

Tert-butyl 4-(6-hydroxy-5-nitro-2H-indazol-2-yl) piperidine-1-carboxylate (1.4g, crude product) was dissolved in 4M hydrochloric acid-1,4-dioxane(20 mL), stirred for 3 hours at room temperature, and concentrated directly under reduced pressure to obtain 800 mg of crude target product as a light yellow solid. The crude product was directly used for the next reaction without purification.

LC-MS: (ESI, *m*/*z*): [M+H] ⁺ =263.1.

### Step 2:tert-butyl 9-((4-(6-hydroxy-5-nitro-2H-indazol-2-yl) piperidin-1-yl) methyl)-3-azaspiro[5.5] undecyl-3-carboxylate

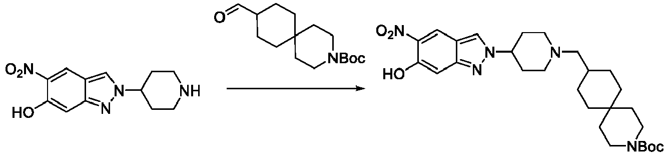

5-Nitro-2-(piperidin-4-yl)-2H-indazol-6-ol (500 mg) and tert-butyl 9-formyl-3-azaspiro [5.5] undecyl-3-carboxylate (536 mg, 1.9 mmol) were dissolved in tetrahydrofuran (20 mL), stirred at room temperature, sodium triacetoxyborohydride (1.2g, 5.7 mmol) was added and stirred at room temperature for 2 hours, water (50 mL) was added, extracted with dichloromethane (3 × 30 mL), the organic phase was washed with saturated saline (100 mL), dried over anhydrous sodium sulfate, filtered, concentrated under reduced pressure, and the crude product was purified by column chromatography to obtain 440 mg of target compound as a yellow solid.

LC-MS: (ESI, *m*/*z*): [M+H] ⁺ =528.2.

### Step 3:tert-butyl 9-((4-(6-(2-methoxyethoxy)-5-nitro-2H-indazol-2-yl) piperidin-1-yl) methyl)-3-azaspiro[5.5] undecyl-3-carboxylate

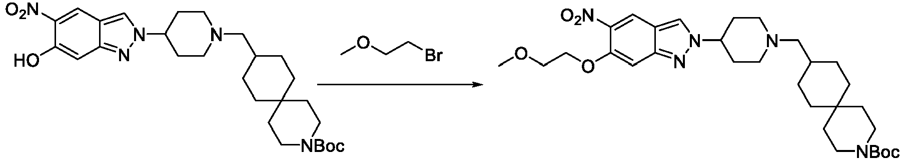

Tert-butyl 9-((4-(6-hydroxy-5-nitro-2H-indazol-2-yl) piperidin-1-yl) methyl)-3-azaspiro[5.5] undecyl-3-carboxylate (440 mg, 0.8 mmol) and 1-bromo-2-methoxyethane (1.15g, 8. 0 mmol) were dissolved with acetonitrile (20 mL), anhydrous potassium carbonate (331 mg, 2.4 mmol) was added and heated to 80 °C, stirred for 2 hours, water (50 mL) was added, extracted with ethyl acetate (3 × 30 mL), organic phase was washed with saturated saline (100 mL), dried over anhydrous sodium sulfate, filtered, concentrated under reduced pressure, and the crude product was purified by column chromatography to obtain 110 mg of target compound as a yellow solid.

LC-MS: (ESI, *m*/*z*): [M+H] ⁺ =586.5.

### Step 4:tert-butyl 9-((4-(5-amino-6-(2-methoxyethoxy)-2H-indazol-2-yl) piperidin-1-yl) methyl)-3-azaspiro[5.5] undecyl-3-carboxylate

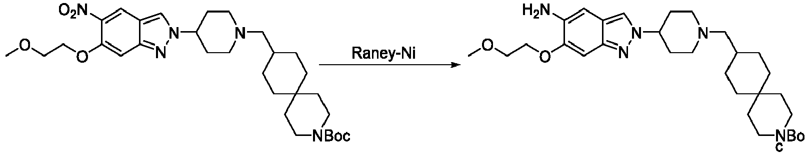

Tert-butyl 9-((4-(6-(2-methoxyethoxy)-5-nitro-2H-indazol-2-yl) piperidin-1-yl) methyl)-3-azaspiro[5.5] undecyl-3-carboxylate (110 mg,0.18 mmol) was added to ethanol (10 mL), Raney-Ni (0.5 mL) and hydrazine hydrate (0.5 mL) were added under stirring, stirred for 2 hours at room temperature, filtered, and the filter cake was washed twice with ethanol (5 mLX2), and concentrated under reduced pressure to obtain 100mg of target compound as a yellow solid, which can be directly used for the next reaction without purification.

LC-MS: (ESI, *m*/*z*): [M+H]⁺=556.5.

### Step 5: tert-butyl 9-((4-(6-(2-methoxyethoxy))-5-(6-(trifluoromethyl) pyridinecarboxamido)-2H-indazol-2-yl) piperidin-1-yl) methyl)-3-azaspiro[5.5] undecyl-3-carboxylate

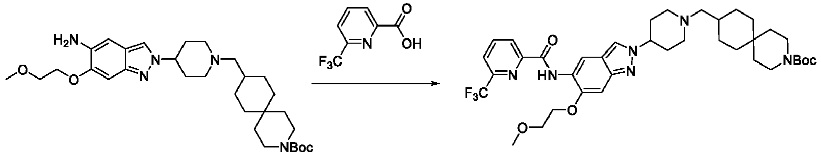

Tert-butyl 9-((4-(5-amino-6-(2-methoxyethoxy)-2H-indazol-2-yl) piperidin-1-yl) methyl)-3-azaspiro[5.5] undecyl-3-carboxylate (100 mg, 0.18 mmol) was dissolved in N,N-dimethylformamide (5mL), 6-(trifluoromethyl) picolinic acid (41 mg, 0.21 mmol), HATU(89 mg, 0.23 mmol) and N,N-diisopropylethylamine (70 mg, 0.54 mmol) were added, stirred for 2 hours at room temperature, water (20 mL) was added, extracted with ethyl acetate (3 × 20 mL), the organic phase was washed with saturated saline (60 mL), dried over anhydrous sodium sulfate, filtered, concentrated under reduced pressure, and the crude product was purified by column chromatography to obtain 80 mg of target product as a yellow solid.

LC-MS: (ESI, *m*/*z*): [M+H] ⁺=729.1.

¹H NMR (400 MHz, DMSO) δ 10.57 (s, 1H), 8.74 (s, 1H), 8.48 (d, *J* = 7.8 Hz, 1H), 8.41 (t, *J* = 7.8 Hz, 1H), 8.37 (s, 1H), 8.22 (d, *J* = 7.7 Hz, 1H), 7.17 (s, 1H), 4.37 (m, 1H), 4.32 - 4.26 (m, 2H), 3.86 - 3.77 (m, 2H), 3.27 (m, 5H), 2.94 (m, 2H), 2.17 (m, *J* = 5.6 Hz, 2H), 2.07 (m, *J* = 5.3 Hz, 6H), 1.64 (d, *J* = 7.7 Hz, 2H), 1.56 (d, *J* = 9.3 Hz, 2H), 1.53 - 1.32 (m, 14H), 1.22 (d, *J* = 7.0 Hz, 2H), 1.12 - 0.98 (m, 4H).

### Step 6:N-(2-(1-((3-azaspiro[5.5] undec-9-yl) methyl) piperidin-4-yl)-6-(2-methoxyethoxy)-2H-indazol-5-yl)-6-(trifluoromethyl) pyridinecarboxamide

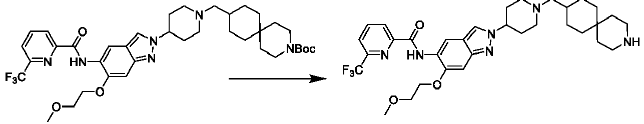

Tert-butyl 9-((4-(5-amino-6-(2-methoxyethoxy)-2H-indazol-2-yl) piperidin-1-yl) methyl)-3-azaspiro[5.5] undecyl-3-carboxylate (80 mg, 0.110mmol) was dissolved with dichloromethane (5 mL), trifluoroacetic acid (2 mL) was added and stirred at room temperature for 2 hours, directly concentrated under reduced pressure to obtain 105 mg of crude target compound, which was directly used for the next reaction without purification.

LC-MS: (ESI, *m*/*z*): [M+H] ⁺=629.1.

The following intermediates were prepared with reference to the method of intermediate 95.

| Intermediate | Structure | Name | LC-MS |
|---|---|---|---|
| Intermediate 96 | 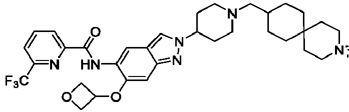 | N-(2-(1-((3-azaspiro[5.5] undec-9-yl) methyl) piperidin-4-yl)-6-(oxetan-3-yloxy)-2H-indazol-5-yl) -6-(trifluoromethyl) pyridinecarboxamide | (ESI,*m*/*z*): |
| | | | [M+H] ⁺ =627.3 |

### Intermediate 97:benzyl 4-((((1r,4r)-4-((tert-butoxycarbonyl) amino) cyclohexyl) methoxy) methyl) piperidine-1-carboxylate

### Step 1:tert-butyl 1(1r,4r)-4-((pyridin-4-ylmethoxy) methyl) cyclohexyl) carbamate

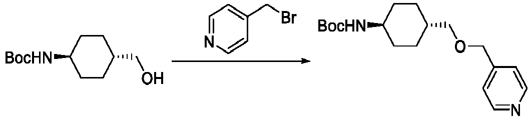

Sodium hydride (211 mg, 8.8 mmol) was added to tert-butyl ((1r,4r)-4-(hydroxymethyl) cyclohexyl) carbamate (1.0g, 4.4 mmol) in tetrahydrofuran (20 mL) at 0 °C, stirred at 0 °C for 30 minutes, continued stirring at room temperature for 30 minutes, 4-bromomethylpyridine (1.1g, 4.4 mmol) was added, and stirred at room temperature overnight, water (30 mL) was added, extracted with ethyl acetate (3 × 50 mL), the organic phase was washed with saturated saline (50 mL), dried over anhydrous sodium sulfate, filtered, concentrated under reduced pressure, and the crude product was purified by column chromatography (50% EA/PE) to obtain 1.0g of target product as a white solid.

LC-MS: (ESI, m/z): [M+H] ⁺=321.1.

### Step 2:tert-butyl ((1r,4r)-4-((piperidin-4-ylmethoxy) methyl) cyclohexyl) carbamate

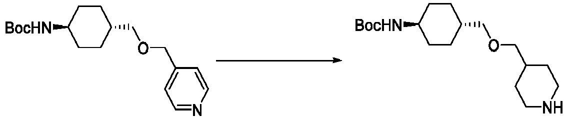

Tert-butyl ((1r,4r)-4-((pyridin-4-ylmethoxy) methyl) cyclohexyl) carbamate (1.0g, 3.1 mmol) was dissolved in isopropanol (30 mL) and water (35 mL), palladium/carbon (0.5g) was added, stirred at 75 °C for 72 hours in hydrogen environment, filtered, concentrated and dried to obtain 1.0g of crude target product as a white solid.

LC-MS: (ESI, *m*/*z*): [M+H] ⁺=327.2.

### Step 3:benzyl 4-((((1r,4r)-4-((tert-butoxycarbonyl) amino) cyclohexyl) methoxy) methyl) piperidine-1-carboxylate

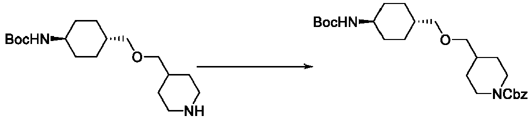

Saturated sodium bicarbonate solution (30 mL) and benzyl chloroformate(680 mg, 3.98 mmol) were added to tert-butyl ((1r,4r)-4-((piperidin-4-ylmethoxy) methyl) cyclohexyl) carbamate (1.0g, 3.06 mmol) in ethyl acetate (30 mL), stirred overnight at room temperature, water (30 mL) was added, extracted with ethyl acetate (3 × 50 mL), the organic phase was washed with saturated saline (50 mL), dried over anhydrous sodium sulfate, filtered, concentrated under reduced pressure, and the crude product was purified by column chromatography to obtain 400 mg of target product as a white solid.

LC-MS: (ESI, *m*/*z*): [M+H] ⁺=461.2.

The following intermediates were prepared with reference to the method of intermediate 91.

| Intermediate | Structure | Name | LC-MS |
|---|---|---|---|
| Intermediate 98 | 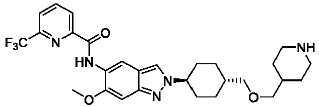 | N-(6-methoxy -2-((1r,4r)-4-((piperidin-4-ylmethoxy) methyl) cyclohexyl)-2H-indazol-5-yl)-6-(trifluoromethyl) pyridinecarboxamide | (ESI,*m*/*z*): |
| | | | [M+H] ⁺ =546.2 |

### Example 1:N-(2-(1-(2-(9-(3-(2, 4-dioxotetrahydropyrimidin-1 (2H)-yl)-4-methoxybenzoyl)-3, 9-diazaspiro[5.5] undec-3-yl) ethyl) piperidin-4-yl)-6-methoxy-2H-indazol-5-yl)-6-(trifluoromethyl)-2-pyridinecarboxamide

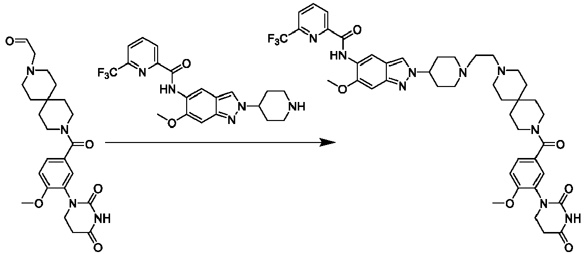

N-(6-methoxy-2-(piperidin-4-yl)-2H-indazol-5-yl)-6-(trifluoromethyl) pyridinecarboxamide (95 mg, 0.226 mmol) was added to a mixture of 2-(9-(3-(2, 4-dioxo tetrahydropyrimidin-1 (2H)-yl)-4-methoxybenzoyl)-3,9-diazaspiro[5.5] undec-3-yl) acetaldehyde (100 mg, 0.226 mmol) and sodium cyanoborohydride (28mg,0.452mmol) in methanol/acetic acid (4mL,10:1). The mixture was stirred at room temperature for 4h, concentrated under reduced pressure, and the concentrate was purified by preparative HPLC to obtain 11.23 mg of the target compound as a light yellow solid.

LC-MS: (ESI, *m*/*z*): [M+H]⁺=846.3

1HNMR (400 MHz, MeOD) δ 8.85 (s, 1H), 8.50 - 8.42 (m, 2H), 8.32 (t, J = 7.9 Hz, 1H), 8.07 (d, J = 7.8 Hz, 1H), 7.49 - 7.39 (m, 2H), 7.20 (d, J = 8.6 Hz, 1H), 7.13 (s, 1H), 4.94 (s, 1H), 4.09 (s, 3H), 3.97 (s, 1H), 3.93 (s, 3H), 3.82 - 3.36 (m, 16H), 3.26 - 3.18 (m, 1H), 2.81 (t, J = 6.7 Hz, 2H), 2.63 (m, J = 35.7, 11.9 Hz, 4H), 2.12 - 1.52 (m, 8H).

The following compounds were prepared with reference to the method of Example 1:

| Example | Structure | Name | LC-MS/NMR |
|---|---|---|---|
| Example 2 | 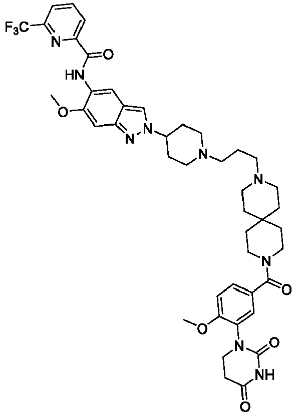 | N-(2-(1-(3-(9-(3-(2,4-dioxotetrahydropyrimidi n-1 (2H)-yl)-4-methoxybenzoyl)-3, 9-diazaspiro [5.5] undec-3-yl) propyl) piperidin-4-yl)-6-methoxy-2H-indazol-5-yl)-6-(trifluoromethyl)-2-pyridinecarboxamide | LC-MS:(ESI, *m*/*z*):[M+H]⁺=860.4. |
| | | | ¹HNMR(400MHz,DMS O) δ 7.98 (s, 1H), 7.69 (d, J = 7.9 Hz, 1H), 7.52 (t, J = 7.8 Hz, 1H), 7.42 (s, 1H), 7.26 (d, J = 7.8 Hz, 1H), 6.66 (m, J = 8.5, 2.0 Hz, 1H), 6.60 (d, J = 2.0 Hz, 1H), 6.40 (d, J = 8.6 Hz, 1H), 6.29 (s, 1H), 3.70 - 3.59 (m, 1H), 3.27 (s, 3H), 3.13 (s, 3H), 2.94 (m, 4H), 2.74 (s, 2H), 2.35 (d, J= 7.8 Hz, 2H), 2.02 (t, J = 6.7 Hz, 2H), 1.71 (m, J = 23.2, 16.2 Hz, 8H), 1.46 (t, J = 10.2 Hz, 6H), 1.01 (s, 2H), 0.87 (s, 4H), 0.81 - 0.66 (m, 4H). |
| Example 3 | 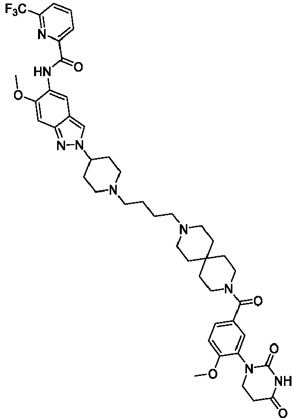 | N-(2-(1-(4-(9-) 3-(2, 4-dioxotetrahydropyrimidi n-1 (2H)-yl)-4-methoxybenzoyl)-3, 9-diazaspiro [5.5] undec-3-yl) butyl) piperidin-4-yl)-6-methoxy-2H-indazol-5-yl)-6-(trifluoromethyl)-2-pyridinecarboxamide | LC-MS: (ESI, *m*/*z):* [M+H] ⁺=874.2 |
| | | | ¹HNMR(400MHz,MeO D) δ 8.76 (s, 1H), 8.47 (d, J = 7.6 Hz, 1H), 8.30 (t, J = 7.7 Hz, 1H), 8.21 (s, 1H), 8.05 (d, J = 7.5 Hz, 1H), 7.47 - 7.37 (m, 2H), 7.18 (d, J = 8.5 Hz, 1H), 7.07 (s, 1H), 4.42 (s, 1H), 4.05 (s, 3H), 3.92 (s, 3H), 3.72 (s, 4H), 3.52 (s, 2H), 3.12 (s, 2H), 2.80 (t, J = 6.5 Hz, 2H), 2.50 (d, J = 23.1 Hz, 7H), 2.24 (s, 6H), 1.61 (d, J = 22.7 Hz, 13H). |

### Example 4:N-(2-(1-(5-(3-(2, 4-dioxotetrahydropyrimidin-1 (2H)-yl)-4-methoxybenzamido) pentyl) piperidin-4-yl)-6-methoxy-2H-indazol-5-yl)-6-(trifluoromethyl) pyridinecarboxamide

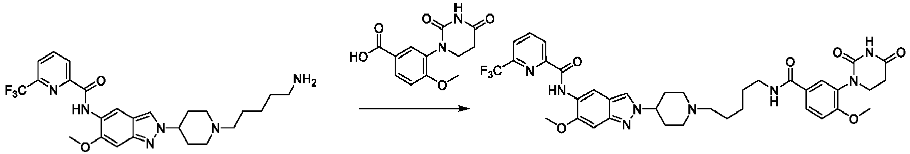

A mixture of N-(2-(1-(5-aminopentyl) piperidin-4-yl)-6-methoxy-2H-indazol-5-yl)-6-(trifluoromethyl) pyridinecarboxamide (160 mg,0.317 mmol), 3-(2, 4-dioxotetrahydropyrimidin-1(2H)-yl)-4-methoxybenzoic acid (26 mg, 0.0985 mmol), HATU(49 mg,0.129 mmol) and N,N-diisopropylethylamine(64 mg,0.496 mmol) in N,N-dimethylformamide (3mL) was stirred for 3 h at room temperature. The reaction solution was poured into water (20mL) and extracted with ethyl acetate (3 × 20mL), the organic phase was collected, washed with water (2 × 50mL) and saline (50mL), dried over anhydrous sodium sulfate, filtered and concentrated. The concentrate was purified by preparative HPLC to obtain 19.97mg of target product as a light yellow solid.

LC-MS: (ESI, *m*/*z*): [M+H] ⁺=751.2.

1H NMR (400 MHz, DMSO) δ 10.50 (s, 1H), 10.35 (s, 1H), 8.69 (s, 1H), 8.49 - 8.32 (m, 4H), 8.22 (d, J = 7.6 Hz, 1H), 7.86 (d, J = 8.8 Hz, 1H), 7.79 (d, J=6.2 Hz, 1H), 7.18 (d, J = 8.8 Hz, 2H), 4.45 - 4.30 (m, 1H), 3.98 (s, 3H), 3.85 (s, 3H), 3.62 - 3.55 (m, 2H), 3.29 - 3.23 (d, J = 6.2 Hz, 2H), 3.05 - 2.95 (m, 2H), 2.72 - 2.65 (m, 2H), 2.38 - 2.29 (m, 2H), 2.15 - 1.98 (m, 6H), 1.60 - 1.44 (m, 4H), 1.38 - 1.28 (m, 2H).

The following compounds were prepared with reference to the method of Example 4:

| Example | Structure | Name | LC-MS/NMR |
|---|---|---|---|
| Example 5 | 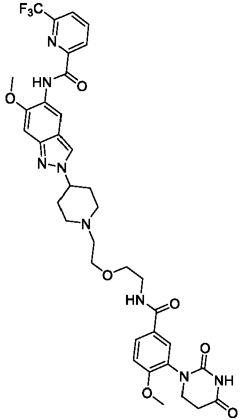 | N-(2-(1-(2-(2-(3-(2, 4-dioxotetrahydropyrimid in-1 (2H)-yl)-4-methoxybenzamido) ethoxy) ethyl) piperidin-4-yl)-6-methoxy-2H-indazol-5-yl)-6-(trifluoromethyl) pyridinecarboxamide | LC-MS: (ESI, *m*/*z*): [M+H] ⁺ =753.2 |
| | | | 1H NMR (400 MHz, DMSO) δ 10.50 (s, 1H), 10.36 (s, 1H), 8.68 (s, 1H), 8.50 - 8.30 (m, 4H), 8.22 (d, J = 7.6 Hz, 1H), 7.91 - 7.85 (m, 1H), 7.80 (d, J = 2.0 Hz, 1H), 7.17 (d, J = 9.8 Hz, 2H), 4.37 - 4.27 (m, 1H), 3.98 (s, 3H), 3.84 (s, 3H), 3.58 - 3.53 (m, 6H), 3.45 - 3.38 (m, 2H), 3.00 (d, J = 11.3 Hz, 2H), 2.71 - 2.62 (m, 2H), 2.58 - 2.51 (m, 2H), 2.25 2.13 (m, 2H), 2.11 - 1.95 (m, 4H). |
| Example 6 | 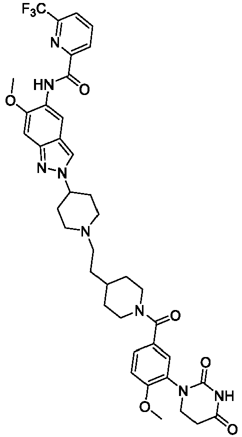 | N-(2-(1-(2-(1-(3-(2, 4-dioxotetrahydropyrimid in-1 (2H)-yl)-4-methoxybenzoyl) piperidin-4-yl) ethyl) piperidin-4-yl)-6-methoxy-2H-indazol-5-yl) -6-(trifluoromethyl) pyridinecarboxamide | LC-MS: (ESI, *m*/*z*): [M+H] ⁺ =777.3. |
| | | | ¹H NMR (400 MHz, CDC13) δ 10.71 (s, 1H), 8.82 (s, 1H), 8.50 (d, J = 7.8 Hz, 1H), 8.12 (t, J = 7.8 Hz, 1H), 7.95 - 7.82 (m, 2H), 7.59 (s, 1H), 7.45 - 7.34 (m, 2H), 7.10 - 6.96 (m, 2H), 4.62-4.50 (m, 1H), 4.04 (s, 3H), 3.90 (s, 3H), 3.76-3.68 (m, 2H), 3.45 - 3.34 (m, 2H), 2.87 -2.79 (m, 2H), 2.82 -2.73 (m, 4H), 2.48 -2.37 (m, 8H), 1.75 -1.69 (m, 5H), 1.30-1.10 (m, 2H). |

### Example 7:N-(2-(1-(2-(1-(3-(2, 4-dioxotetrahydropyrimidin-1 (2H)-yl)-4-methoxybenzoyl) piperidin-4-yl) ethyl) piperidin-4-yl) -6-(2-hydroxyprop-2-yl)-2H-indazol-5-yl)-6-(trifluoromethyl) pyridinecarboxamide

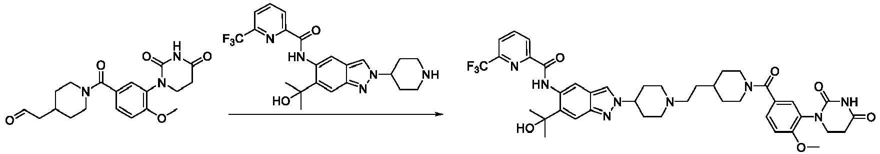

STAB(71mg,0.33mmol) was added to a mixture of 2-(1-(3-(2, 4-dioxotetrahydropyrimidin-1 (2H)-yl)-4-methoxybenzoyl) piperidin-4-yl) acetaldehyde (80 mg,0.22 mmol) and N-(6-(2-hydroxyprop -2-yl)-2-(piperidin-4-yl)-2H-indazol-5-yl)-6-(trifluoromethyl) pyridinecarboxamide (50mg,0.11mmol) in tetrahydrofuran (10mL). The reaction solution was stirred at room temperature for 2 hours. Water (10mL) and ethyl acetate (10mL) were added. The water layer was separated and extracted with ethyl acetate (10mL × 2). The organic layers were combined, washed with saline (10mL × 2), dried over anhydrous sodium sulfate, filtered and concentrated under reduced pressure. The concentrate was purified by preparative HPLC to obtain 9.8 mg of target product as a yellow solid. LC-MS: (ES, *m*/*z*): [M+H]⁺=805.3

¹H NMR (400 MHz, MeOD) δ 8.72 (s, 1H), 8.47 (d, *J* = 7.8 Hz, 2H), 8.32 - 8.24 (m, 2H), 8.02 (d, *J* = 7.8 Hz, 1H), 7.67 (s, 1H), 7.45 (dd, *J* = 8.5, 2.1 Hz, 1H), 7.39 (d, *J* = 2.0 Hz, 1H), 7.19 (d, *J =* 8.6 Hz, 1H), 5.02 - 4.96 (m, 1H), 4.59- 4.56 (m, 2H), 3.92 (s, 3H), 3.73 (s, 2H), 2.81 (t, *J* = 6.7 Hz, 3H), 2.73 - 2.69 (m, 2H), 2.53 - 2.49 (m, 2H), 2.33 -2.29 (m, 4H), 1.95 - 1.55 (m, 12H), 1.29 - 1.25 (m, 2H).

The following examples were prepared with reference to the method of example 7:

| Example | structure | Name | LC-MS/NMR |
|---|---|---|---|
| Example 8 | 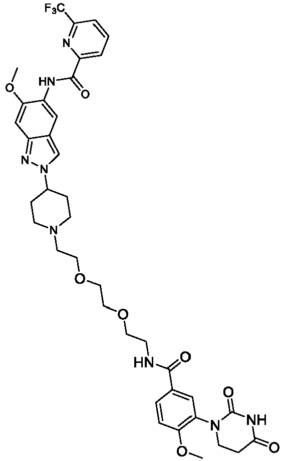 | N-(2-(1-(2-(2-(2-(3-(2, 4-dioxotetrahydropyrimidin-1 (2H)-yl)-4-methoxybenzamido) ethoxy) ethoxy) ethyl) piperidin-4-yl)-6-methoxy-2H-indazol-5-yl)-6-(trifluoromethyl) pyridin carboxamide | LC-MS:(ESI, *m*/*z):* [M+H]⁺= 797.2 |
| | | | ¹H-NMR: 1H NMR (400 MHz, ) δ 10.51 (s, 1H), 10.36 (s, 1H), 8.69 (s, 1H), 8.47 - 8.36 (m, 3H), 8.22 (d, J = 7.8 Hz, 2H), 7.87 (dd, J = 8.6, 2.2 Hz, 1H), 7.80 (d, J = 2.2 Hz, 1H), 7.17 (t, J = 4.2 Hz, 2H), 4.39 - 4.31 (m, 1H), 3.98 (s, 3H), 3.84 (s, 3H), 3.60 - 3.53 (m ,10H), 3.44 - 3.40 (m, 2H), 3.00 (d, J = 11.3 Hz, 2H), 2.68 (t, J = 6.0 Hz, 2H), 2.53 (d, J = 5.8 Hz, 2H), 2.18 (dd, J = 16.4, 9.2 Hz, 2H), 2.11 - 1.99 (m, 4H) |

### Example 9:N-(2-(1-(5-(3-(2, 4-dioxotetrahydropyrimidin-1 (2H)-yl)-4-methoxy-N-methylbenzamido) pentyl) piperidin-4-yl)-6-methoxy-2H-indazol-5-yl)-6-(trifluoromethyl) pyridinecarboxamide

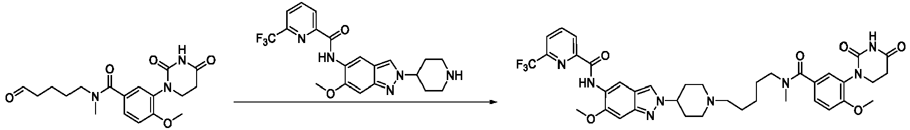

3-(2, 4-dioxotetrahydropyrimidin-1 (2H)-yl)-4-methoxy-N-methyl-N-(5-oxopentyl) benzamide (100 mg, 0.27 mmol), dichloroethane (2 mL), methanol (2 mL), N-(6-methoxy-2-(piperidin-4-yl)-2H-indazol-5-yl)-6-(trifluoromethyl) pyridinecarboxamide (113.13 mg, 0.27 mmol), acetic acid (1 drop) and sodium triacetate borohydride(114.48mg,0.54mmol) were added to a 25mL round bottom flask, and the reaction mixture was stirred at room temperature overnight. 10mL of water was added to quench the reaction, extracted with dichloromethane (2 × 20mL), and the organic layers were combined and washed with 20mL saturated saline, e dried over anhydrous sodium sulfate and concentrated under reduced pressure. The concentrate was purified by preparative HPLC to obtain 36.5 mg of target product as a white solid.

LC-MS: (ES, *m*/*z*): [M+H]⁺=765.4

¹H NMR (400 MHz, DMSO-d₆) δ 10.51 (s, 1H), 10.34 (s, 1H), 8.69 (s, 1H), 8.49 - 8.38 (m, 2H), 8.36 (s, 1H), 8.22 (d, *J* = 8.6 Hz, 2H), 7.44 - 7.27 (m, 2H), 7.16 (d, *J* = 8.4 Hz, 2H), 4.39 -4.35 (m, 1H), 3.98 (s, 3H), 3.84 (s, 3H), 3.59 (t, *J =* 6.6 Hz, 3H), 2.97 -2.90 (m, 6H), 2.68 (t, *J* = 6.1 Hz, 2H), 2.35 -2.31 (m, 2H), 2.11 -2.05 (m, 6H), 1.64 - 1.16 (m, 6H).

### Example 10:N-(2-(1-(2-(9-(3-(2, 4-dioxotetrahydropyrimidin-1 (2H)-yl)-4-methoxybenzoyl)-3, 9-diazaspiro[5.5] undec-3-yl) ethyl) piperidin-4-yl)-6-(2-hydroxyprop-2-yl)-2H-indazol-5-yl)-6-(trifluoromethyl) pyridinecarboxamide

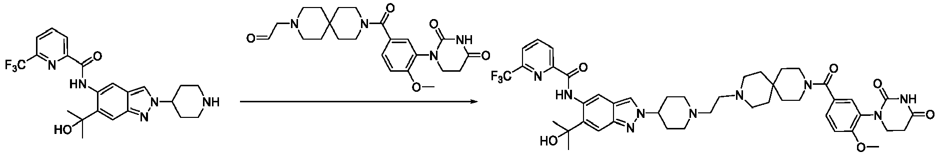

2-(9-(3-(2, 4-Dioxotetrahydropyrimidin-1 (2H)-yl)-4-methoxybenzoyl)-3, 9-diazaspiro[5.5] undec-3-yl) acetaldehyde (150mg,0.336 mmol) was added to a mixture of N-(6-(2-hydroxyprop-2-yl)-2-(piperidin-4-yl)-2H-indazol-5-yl)-6-(trifluoromethyl) pyridinecarboxamide (50 mg,0.112 mmol) and sodium cyanoborohydride(21 mg,0.336 mmol) in methanol/acetic acid (4 mL,10:1). The reaction mixture was stirred at room temperature for 4h. Then it was concentrated under reduced pressure, and the concentrate was purified by preparative HPLC to obtain 26.96mg of the crude target product.

LC-MS: (ESI, *m*/*z*): [M+H] ⁺= 874.4.

¹H NMR (400 MHz, DMSO) δ 12.37 (s, 1H), 10.34 (s, 1H), 8.71 (s, 1H), 8.46 - 8.34 (m, 3H), 8.16 (dd, J = 7.8, 0.8 Hz, 1H), 7.57 (s, 1H), 7.43 - 7.26 (m, 2H), 7.15 (d, J = 8.6 Hz, 1H), 5.95 (s, 1H), 4.44 - 4.42 (m, 1H), 3.84 (s, 3H), 3.61 - 3.35 (m, 6H), 3.01 (d, J = 10.5 Hz, 2H), 2.68 (t, J = 6.6 Hz, 2H), 2.49 - 2.31 (m, 8H), 2.21 - 2.03 (m, 6H), 1.62 (s, 6H), 1.48 - 1.27 (m, 8H).

The compounds in the following table were prepared with reference to the method of Example 10.

| | | | |
|---|---|---|---|
| Example 11 | 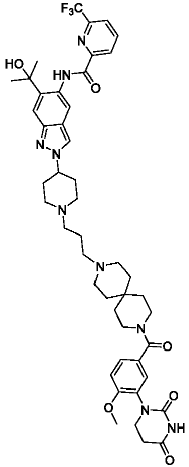 | N-(2-(1-(2-(9-(3-(2, 4-dioxotetrahydropyrimidin-1 (2H)-yl)-4-methoxybenzoyl)-3, 9-diazaspiro[5.5] undec-3-yl) propyl) piperidin-4-yl)-6-(2-hydroxyprop-2-yl)-2H-indazol-5-yl)-6-(trifluoromethyl) pyridinecarboxamide | LC-MS: (ES, m/z): [M+H]⁺=888.4 |
| | | | ¹_{H} NMR (400 MHz, DMSO-d₆) δ 12.37 (s, 1H), 10.33 (s, 1H), 8.71 (s, 1H), 8.45 (d, *J* = 7.7 Hz, 1H), 8.37 (dd, *J* = 14.9, 7.1 Hz, 2H), 8.28 (s, 2H), 8.16 (d, *J* = 7.7 Hz, 1H), 7.57 (s, 1H), 7.41 - 7.28 (m, 2H), 7.15 (d, *J =* 8.6 Hz, 1H), 5.94 (s, 1H), 4.47 -4. 43 (m, 1H), 3.84 (s, 3H), 3.60 (t, *J* = 6.6 Hz, 4H), 3. 02 - 2.97 (m, 2H), 2.68 (t, *J* = 6.3 Hz, 2H), 2.35 (d, *J* = 5.7 Hz, 8H), 2.09 (d, *J* = 5.7 Hz, 6H), 1.62 (s, 8H), 1.46 (d, *J* = 29.9 Hz, 8H). |
| Example 12 | 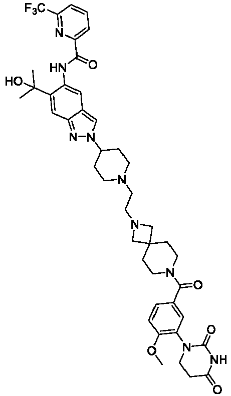 | N-(2-(1-(2-(7-(3-(2, 4-dioxotetrahydropyrimidin-1 (2H)-yl)-4-methoxybenzoyl)-2, 7-diazaspiro [3.5] non-2-yl) ethyl) piperidin-4-yl)-6-(2-hydroxyprop-2-yl)-2H-indazol-5-yl)-6-(trifluoromethyl) pyridinecarboxamide | LC-MS: (ESI, *m*/*z*): [M+H] ⁺ =846.3. |
| | | | ¹H NMR (400 MHz, DMSO-d₆) δ 12.37 (s, 1H), 10.34 (s, 1H), 8.71 (s, 1H), 8.45 (d, J = 7.8 Hz, 1H), 8.37 (dd, J = 13.7, 5.9 Hz, 2H), 8.16 (d, J = 7.8 Hz, 1H), 7.58 (s, 1H), 7.39 - 7.30 (m, 2H), 7.15 (d, J = 8.6 Hz, 1H), 5.95 (s, 1H), 4.44 .40 (m, 1H), 3.84 (s, 3H), 3.59 (t, J = 6.6 Hz, 2H), 3.5 .35 (m, 4H), 2.99 (d, J = 12.4 Hz, 6H), 2.68 (t, J = 6.6 Hz, 2H), 2.55 (s, 2H), 2.31 (t, J = 6.7 Hz, 2H), 2.19 - 2.03 (m, 6H), 1.72 - 1.58 (m, 10H). |
| Example 13 | 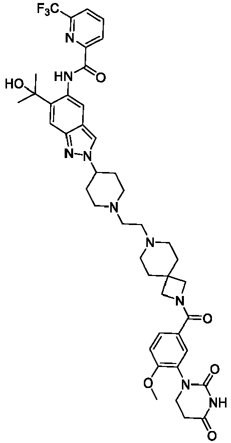 | N-(2-(1-(2-(2-(3-(2, 4-dioxotetrahydropyrimidin-1 (2H)-yl)-4-methoxybenzoyl)-2, 7-diazaspiro [3.5] non-7-yl) ethyl) piperidin-4-yl)-6-(2-hydroxyprop-2-yl)-2H-indazol-5-yl)-6-(trifluoromethyl) pyridinecarboxamide | LC-MS: (ESI, *m*/*z*): [M+H] ⁺ =846.3. |
| | | | ¹H NMR (400 MHz, DMSO-d₆) δ 12.37 (s, 1H), 10.34 (s, 1H), 8.71 (s, 1H), 8.45 (d, J = 7.8 Hz, 1H), 8.37 (dd, J = 14.3, 6.4 Hz, 2H), 8.16 (d, J = 7.8 Hz, 1H), 7.65 (dd, J = 8.6, 2.2 Hz, 1H), 7.60 - 7.56 (m, 2H), 7.16 (d, J = 8.8 Hz, 1H), 5.95 (s, 1H), 4.45 - 4.42 (m, 1H), 4.03 (s, 2H), 3.86 (s, 3H), 3.72 (s, 2H), 3.59 (t, J = 6.7 Hz, 2H), 3.01 (d, J = 10.1 Hz, 2H), 2.69 (t, J = 6.5 Hz, 2H), 2.49 - 2.22 (m, 8H), 2.16 - 2.07 (m, 6H), 1.75 - 1.68 (m, 4H), 1.62 (s, 6H). |

### Example 14:N-(2-(1-(2-(4-(3-(2, 4-dioxotetrahydropyrimidin-1 (2H)-yl)-4-methoxybenzoyl) piperazin-1-yl) ethyl) piperidin-4-yl)-6-methoxy-2H-indazol-5-yl) -6-(trifluoromethyl) pyridinecarboxamide

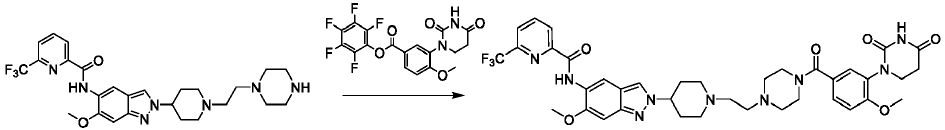

A mixture of N-(6-methoxy-2-(1-(2-(piperazin-1-yl) ethyl) piperidin-4-yl)-2H-indazol-5-yl)-6-(trifluoromethyl) pyridinecarboxamide (120 mg,0.226 mmol), pentafluorophenyl 3-(2, 4-dioxotetrahydropyrimidin-1 (2H)-yl)-4-methoxybenzoate (49 mg,0.113 mmol) and N,N-diisopropylethylamine(291 mg,2.26 mmol) in dimethyl sulfoxide (3 mL) was stirred for 2h at room temperature. The reaction solution was poured into water (50mL) and stirred for 0.5h, the solution was extracted with ethyl acetate (3 × 50mL), and the organic phase was collected, washed with water (2 × 100mL) and saturated saline (100mL), dried over anhydrous sodium sulfate, filtered and concentrated. The concentrate was purified by preparative HPLC to obtain 28.57mg of target product as a yellow solid.

LC-MS: (ESI, *m*/*z*): [M+H]⁺=778.2

¹H NMR (400 MHz, CDC13) δ 10.71 (s, 1H), 8.82 (s, 1H), 8.50 (d, J = 7.9 Hz, 1H), 8.12 (t, J = 7.8 Hz, 1H), 7.93 - 7.81 (m, 2H), 7.71 (s, 1H), 7.50 - 7.33 (m, 2H), 7.14 - 6.93 (m, 2H), 4.52-4.42 (m, 1H), 4.03 (s, 3H), 3.90 (s, 3H), 3.82-3.53 (m, 6H), 3.33 (d, J = 11.7 Hz, 2H), 2.82 (t, J = 6.6 Hz, 4H), 2.72 (t, J = 6.2 Hz, 2H), 2.55-2.43 (m, 6H), 2.35-2.28 (m, 4H).

The following examples were prepared with reference to the method of example 14:

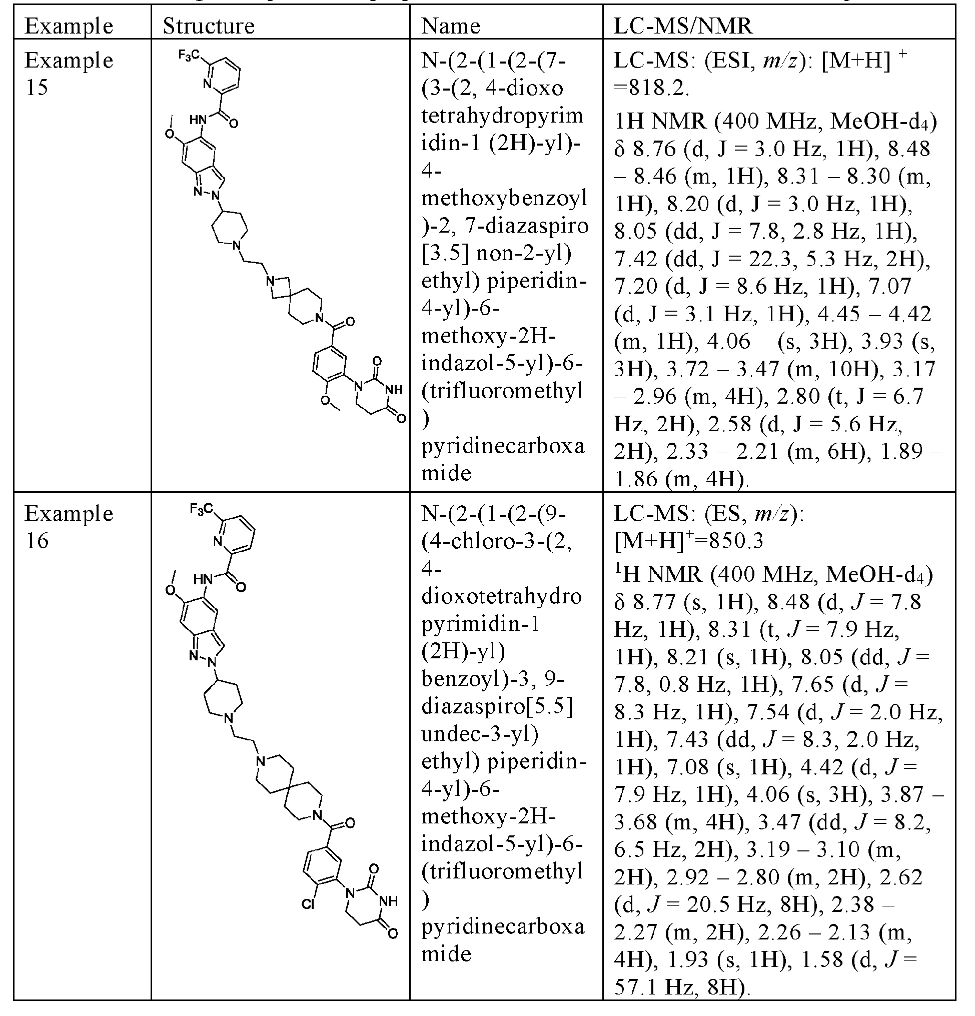

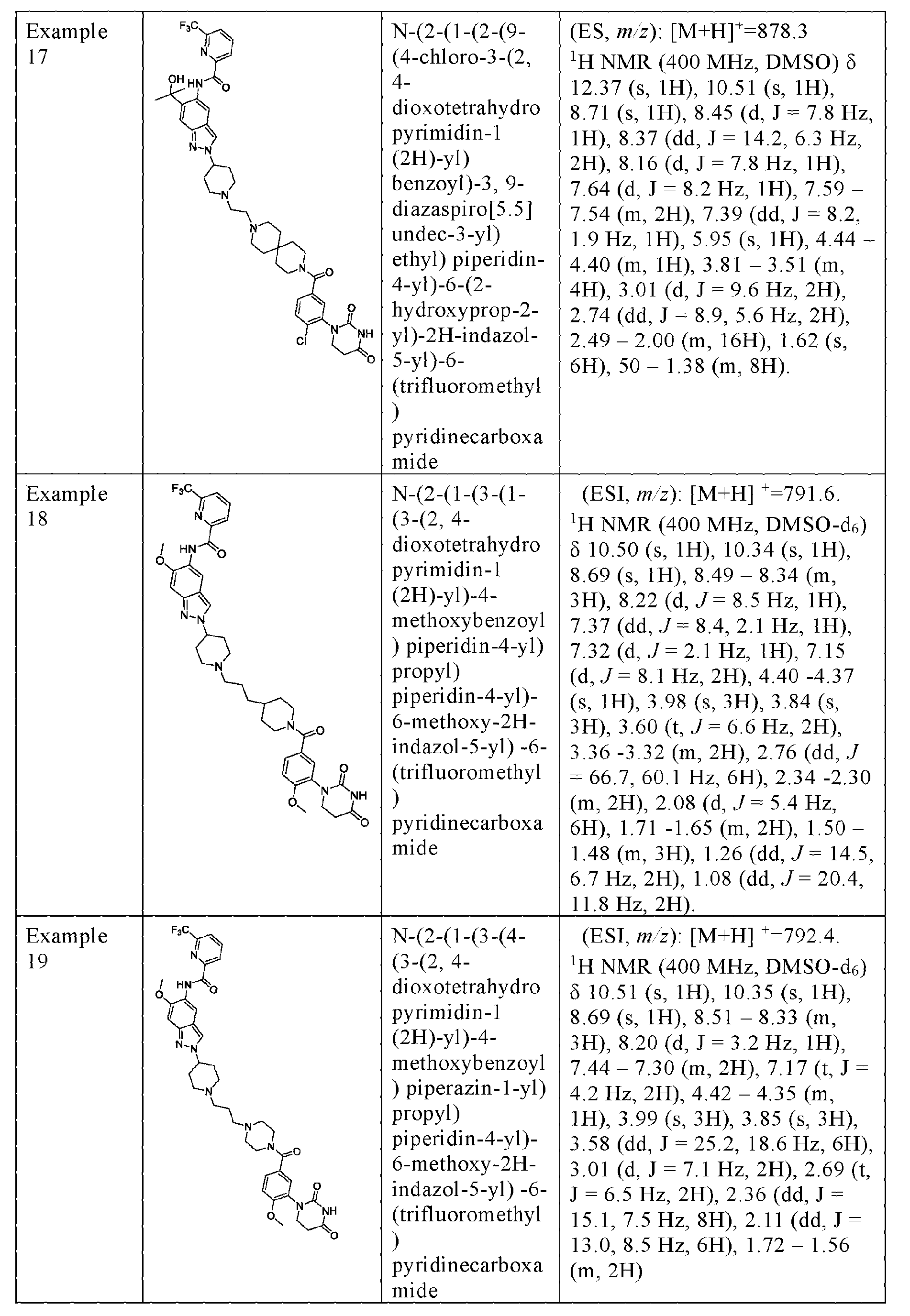

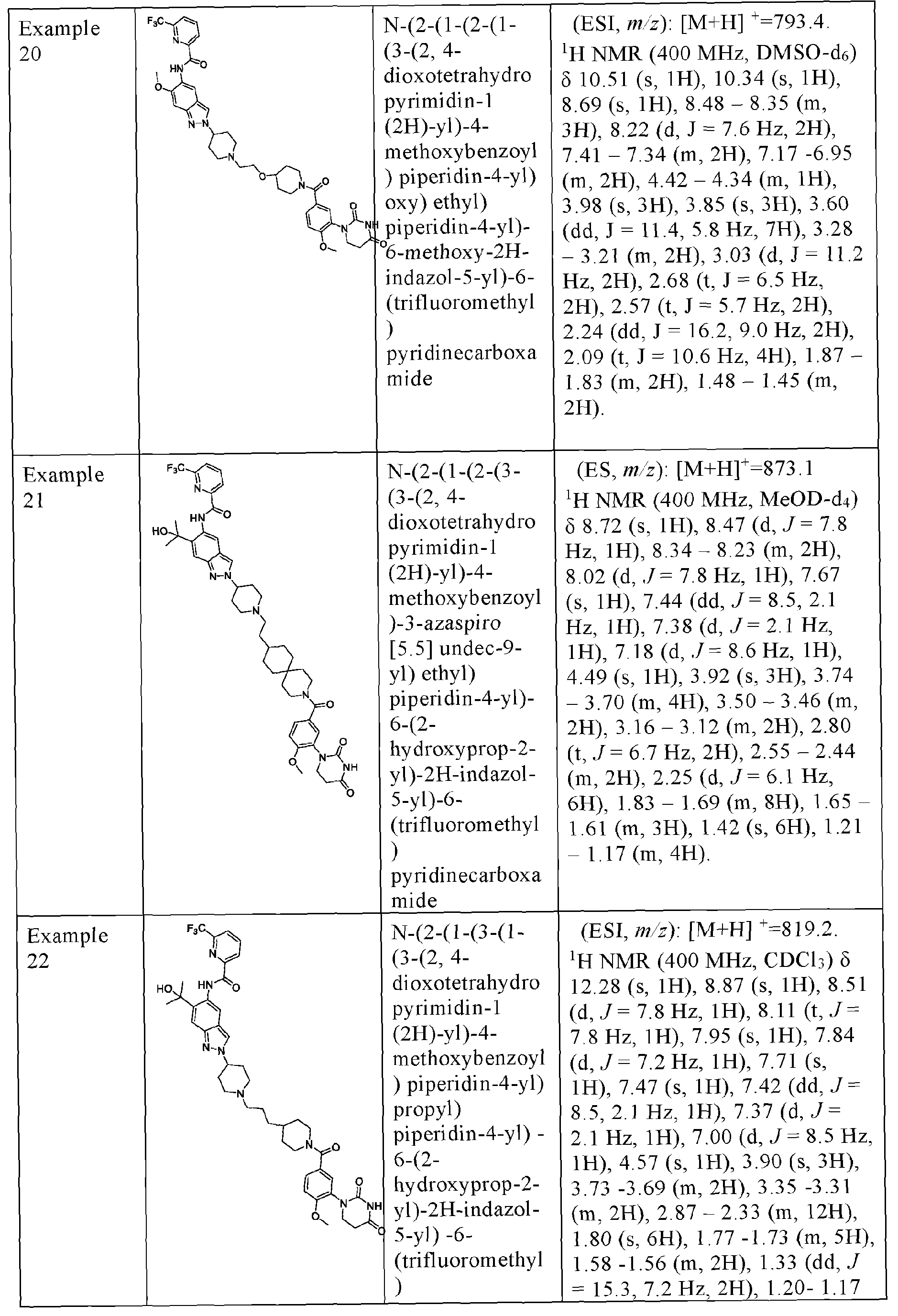

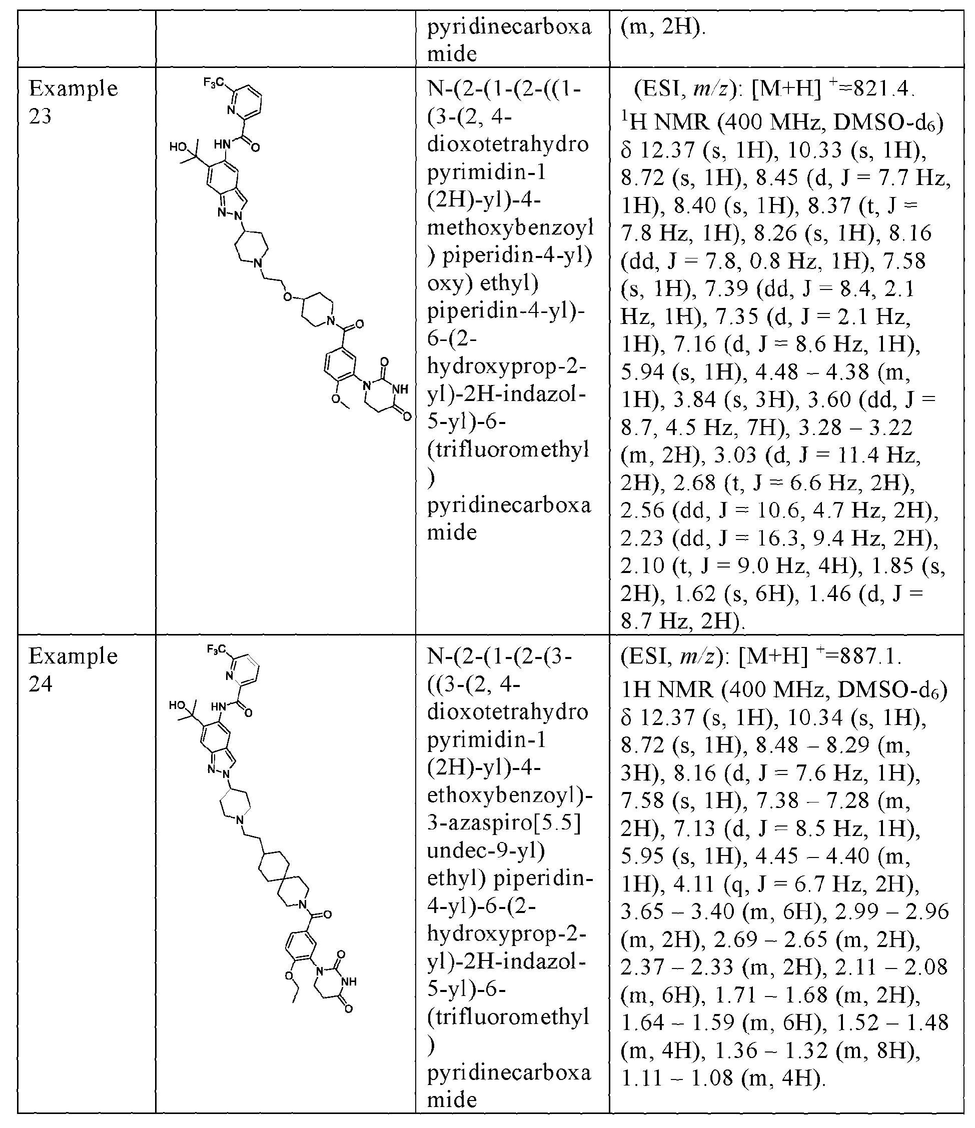

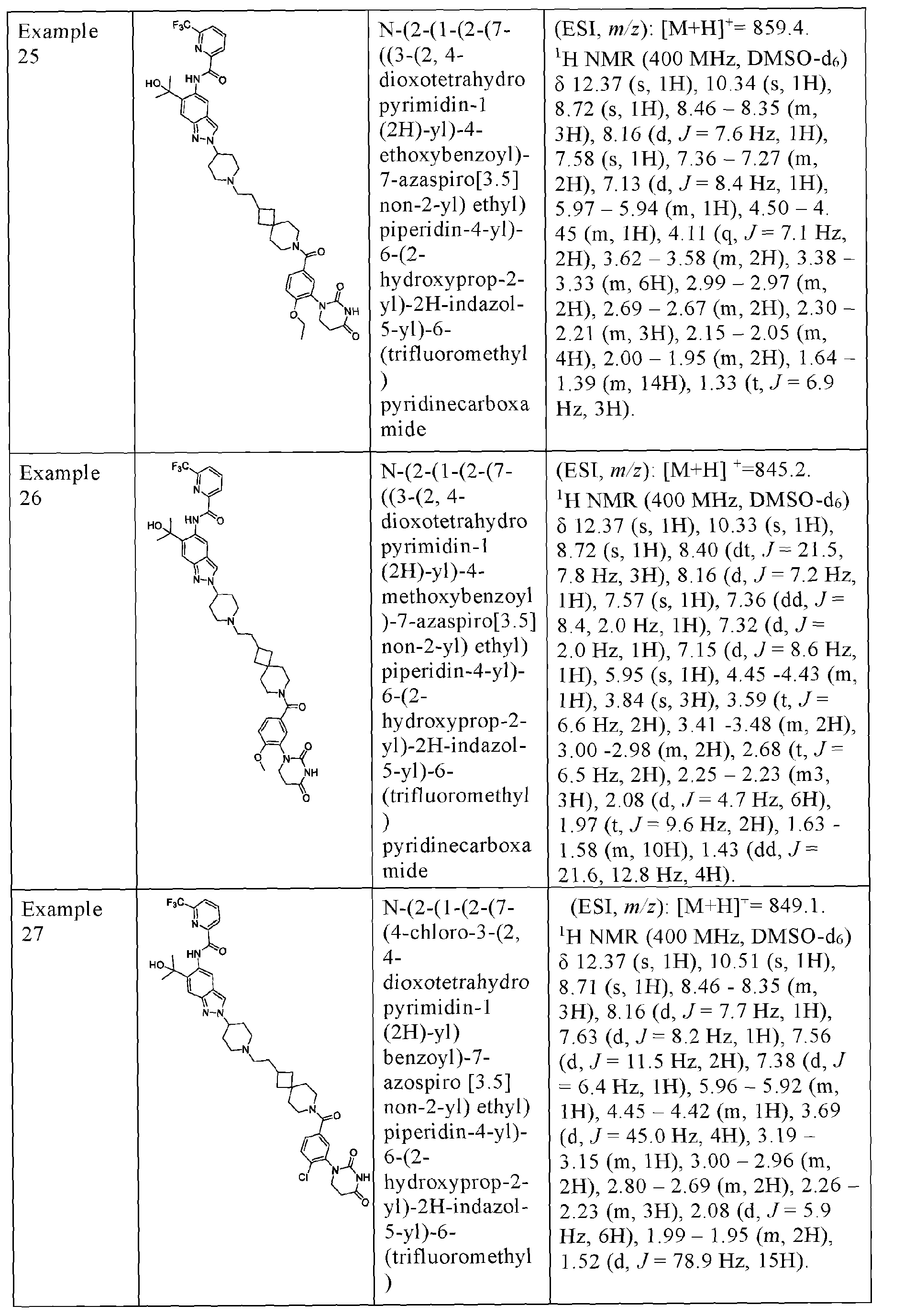

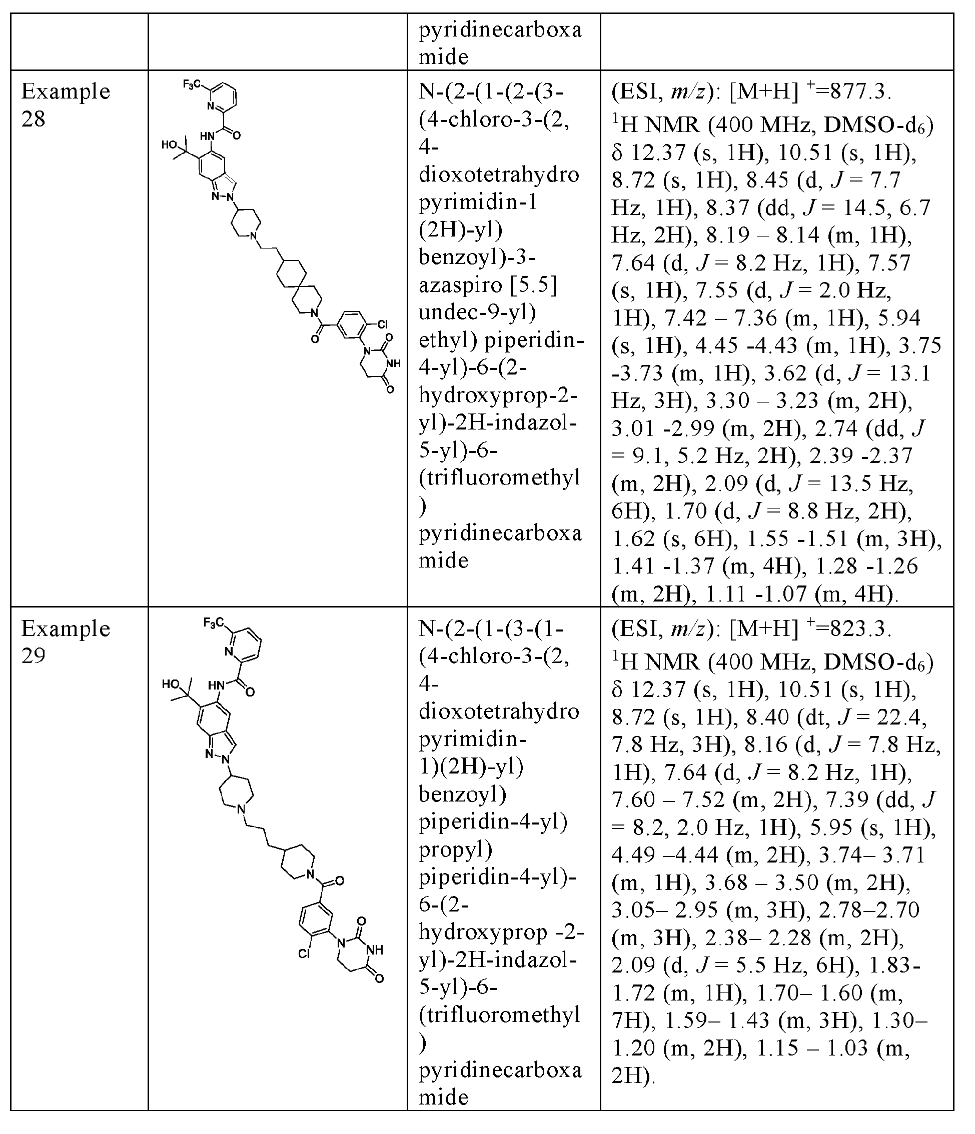

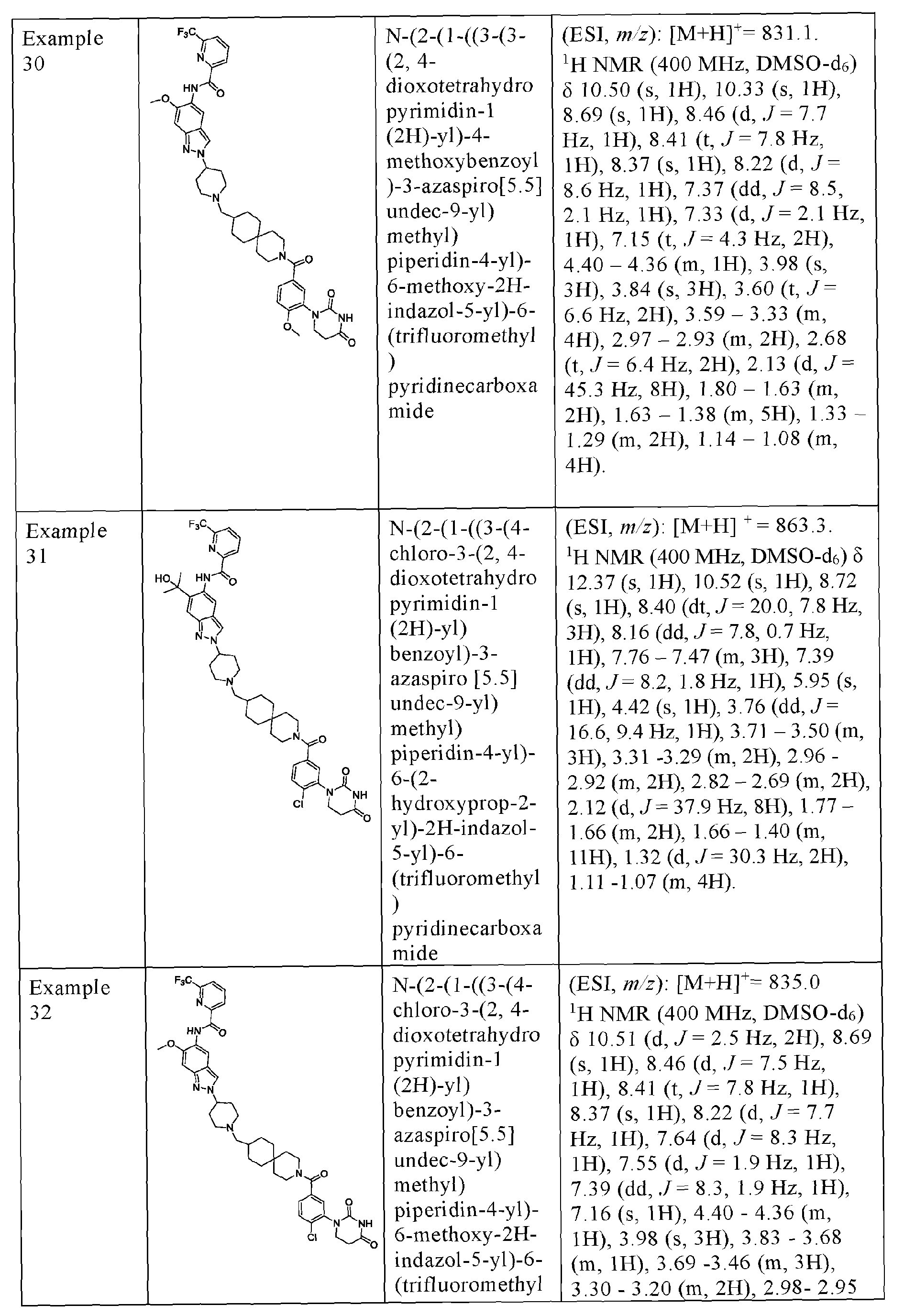

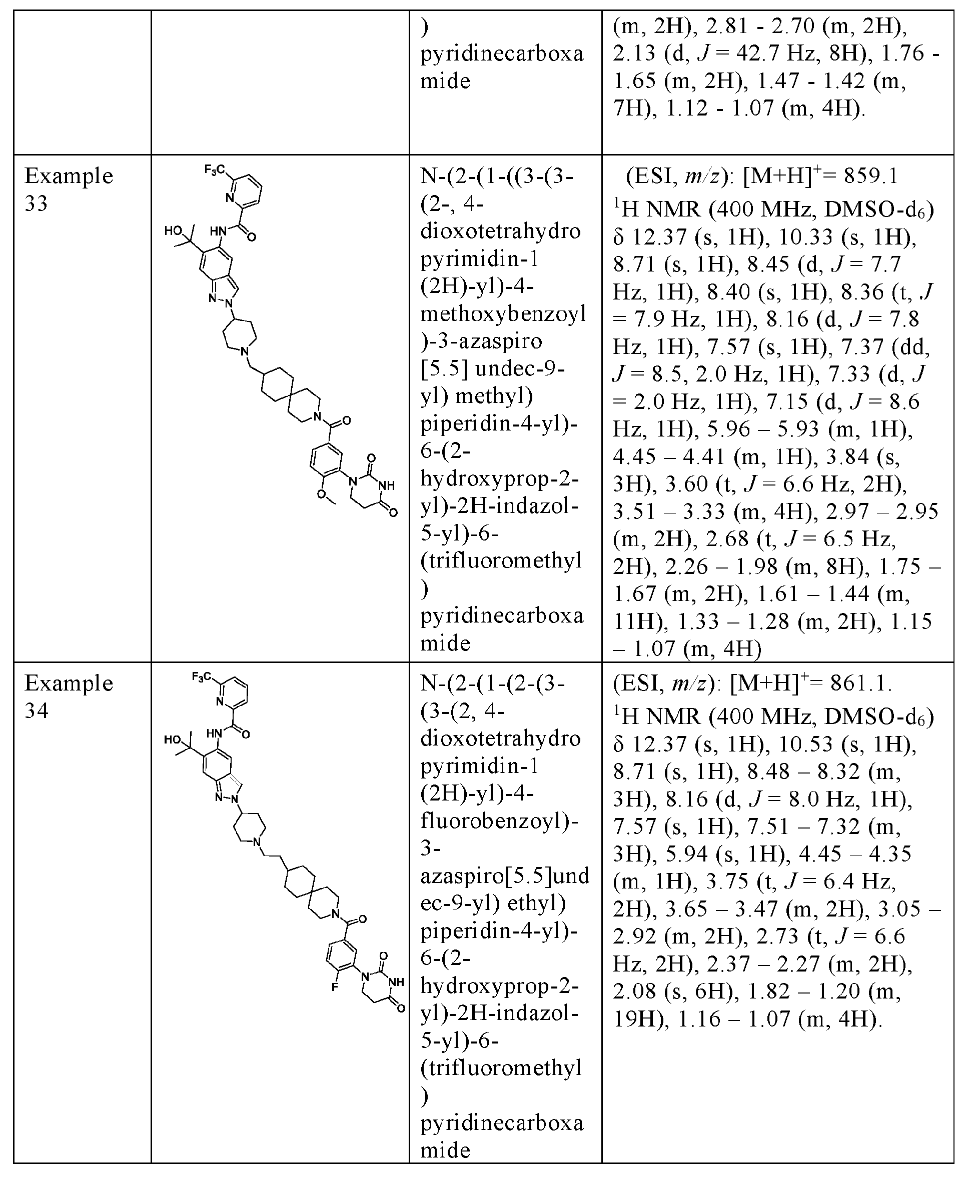

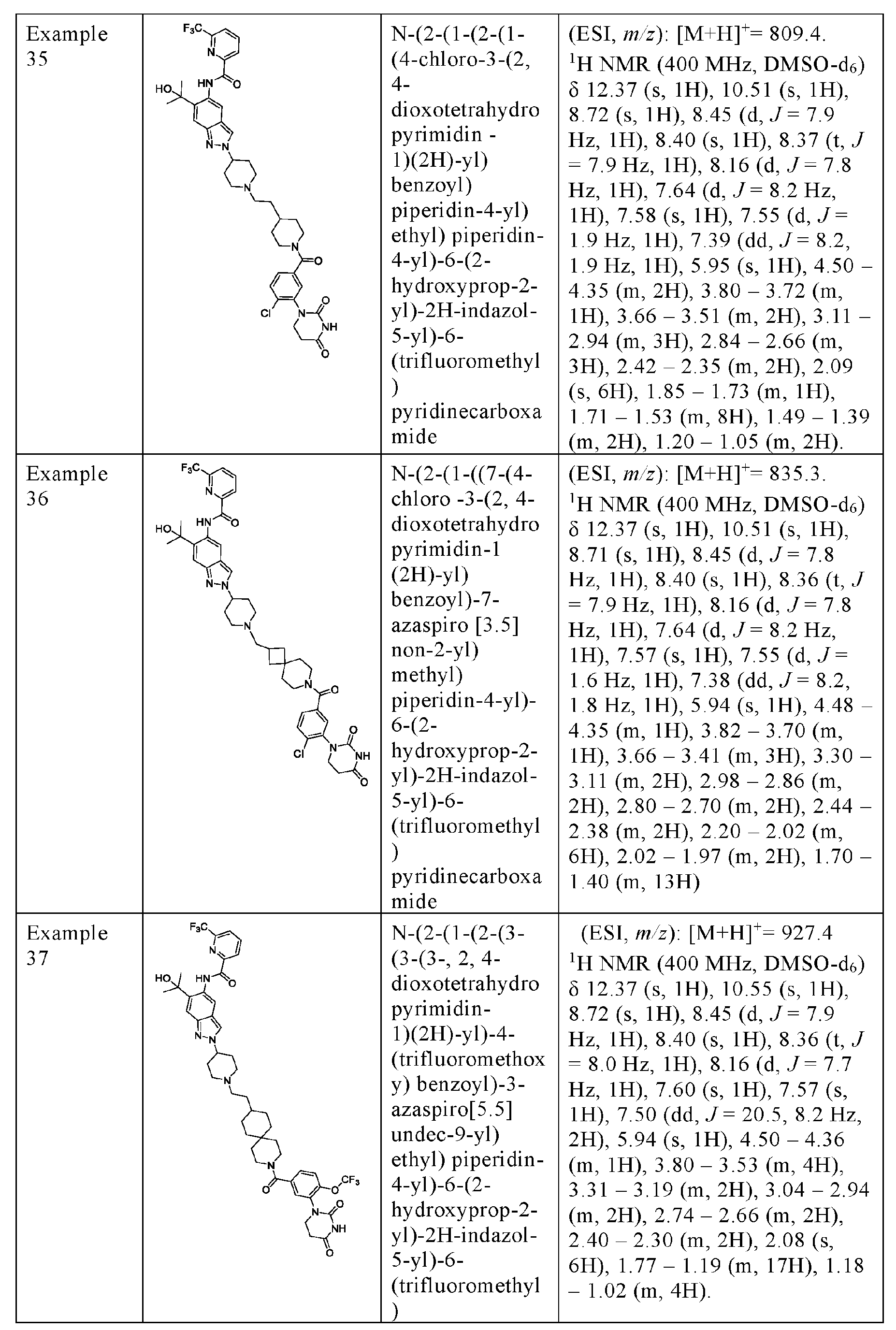

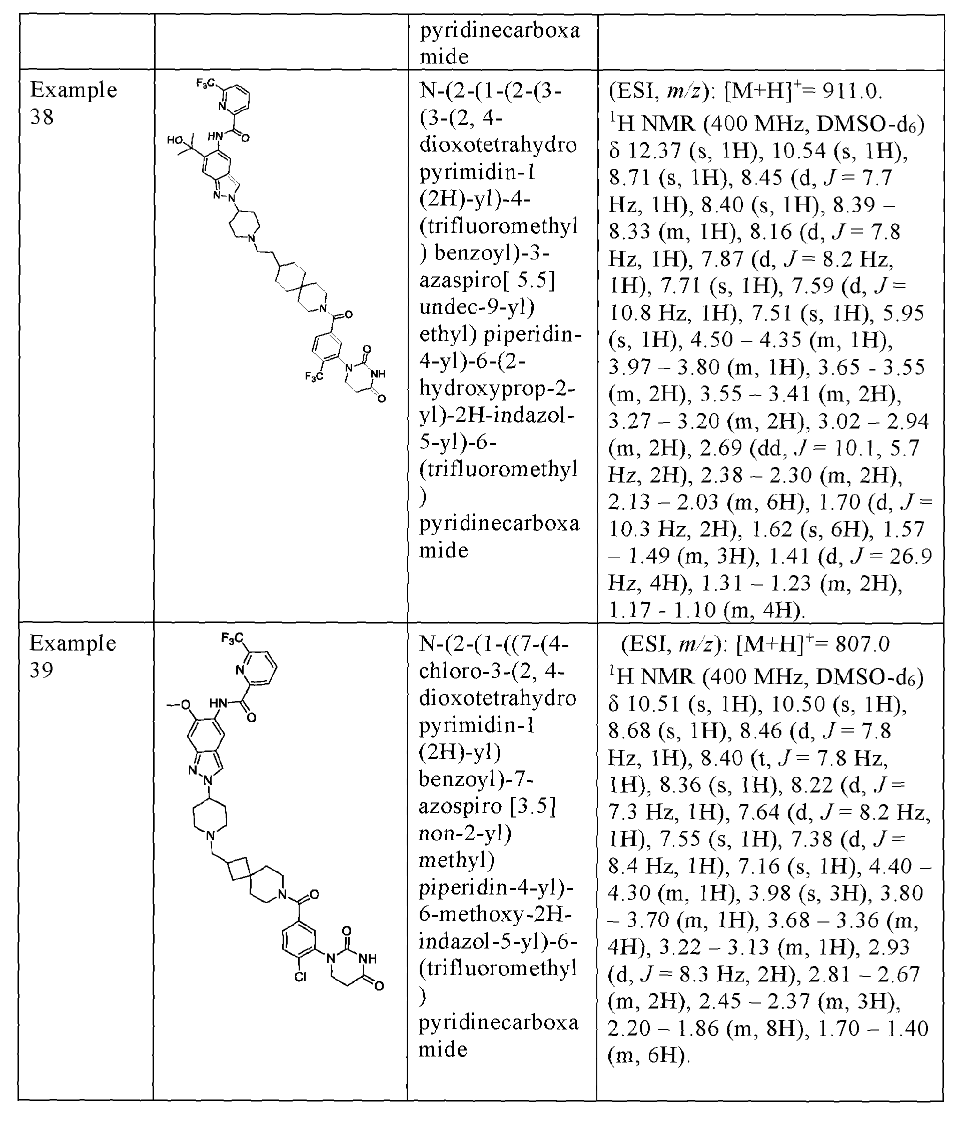

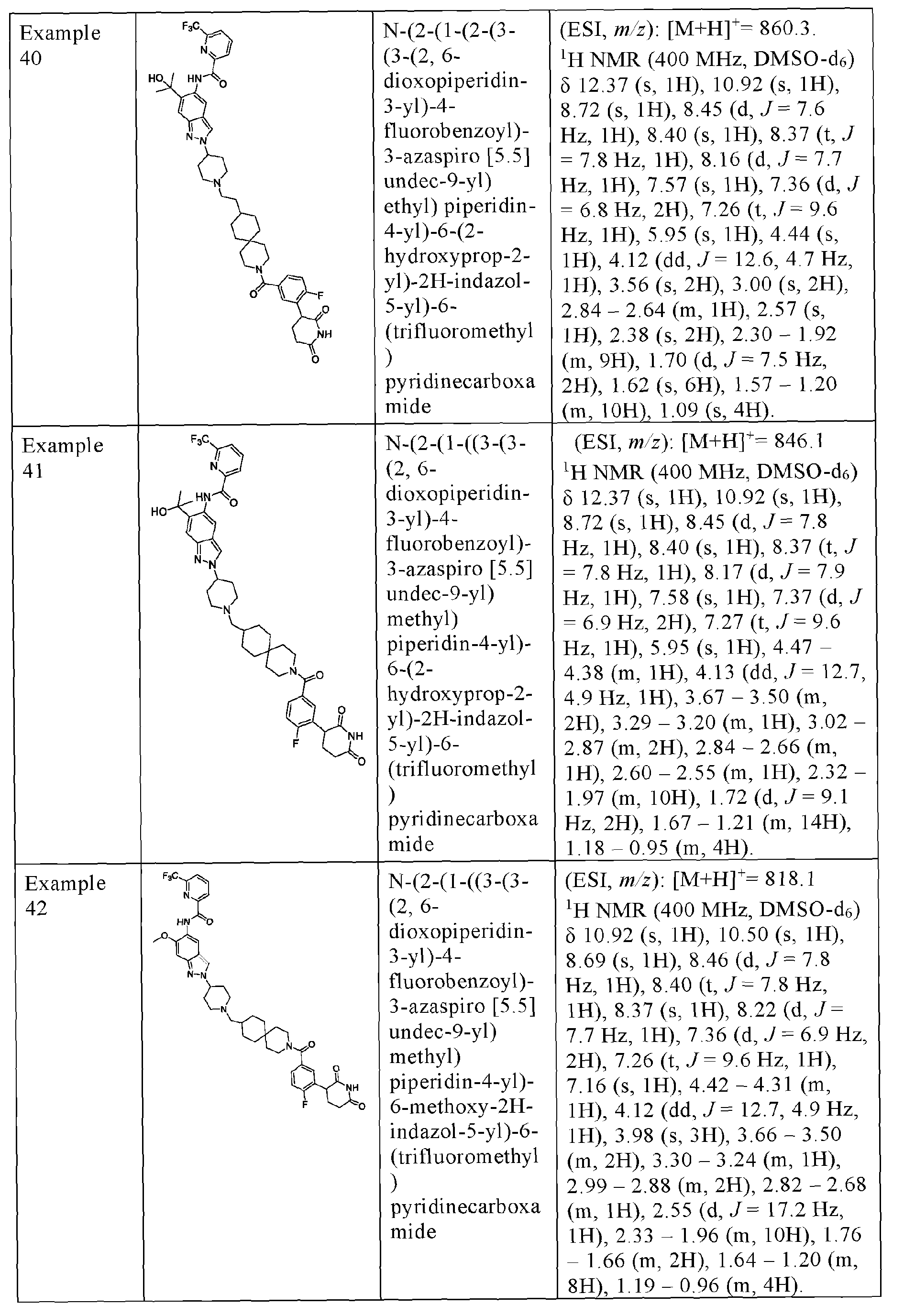

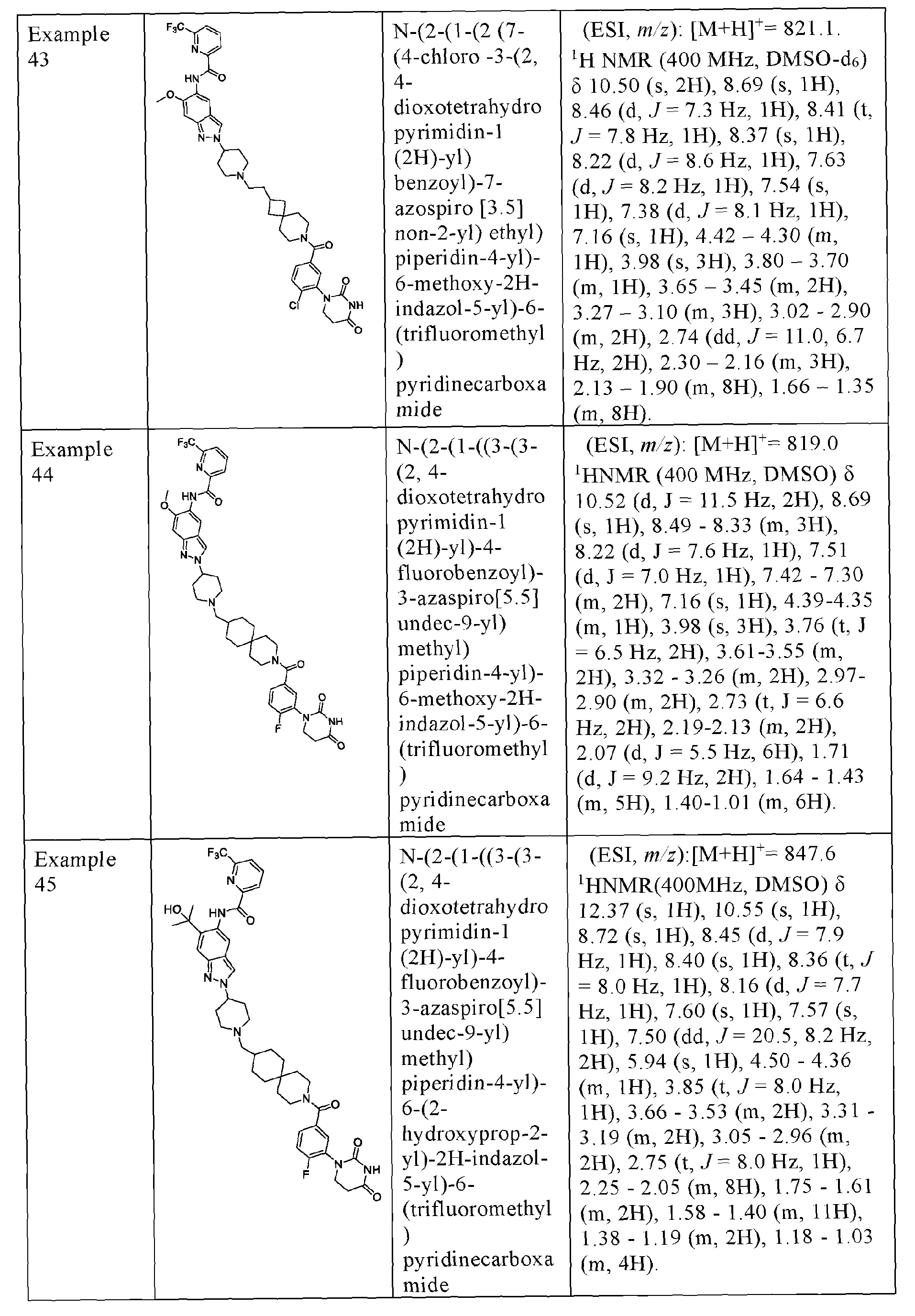

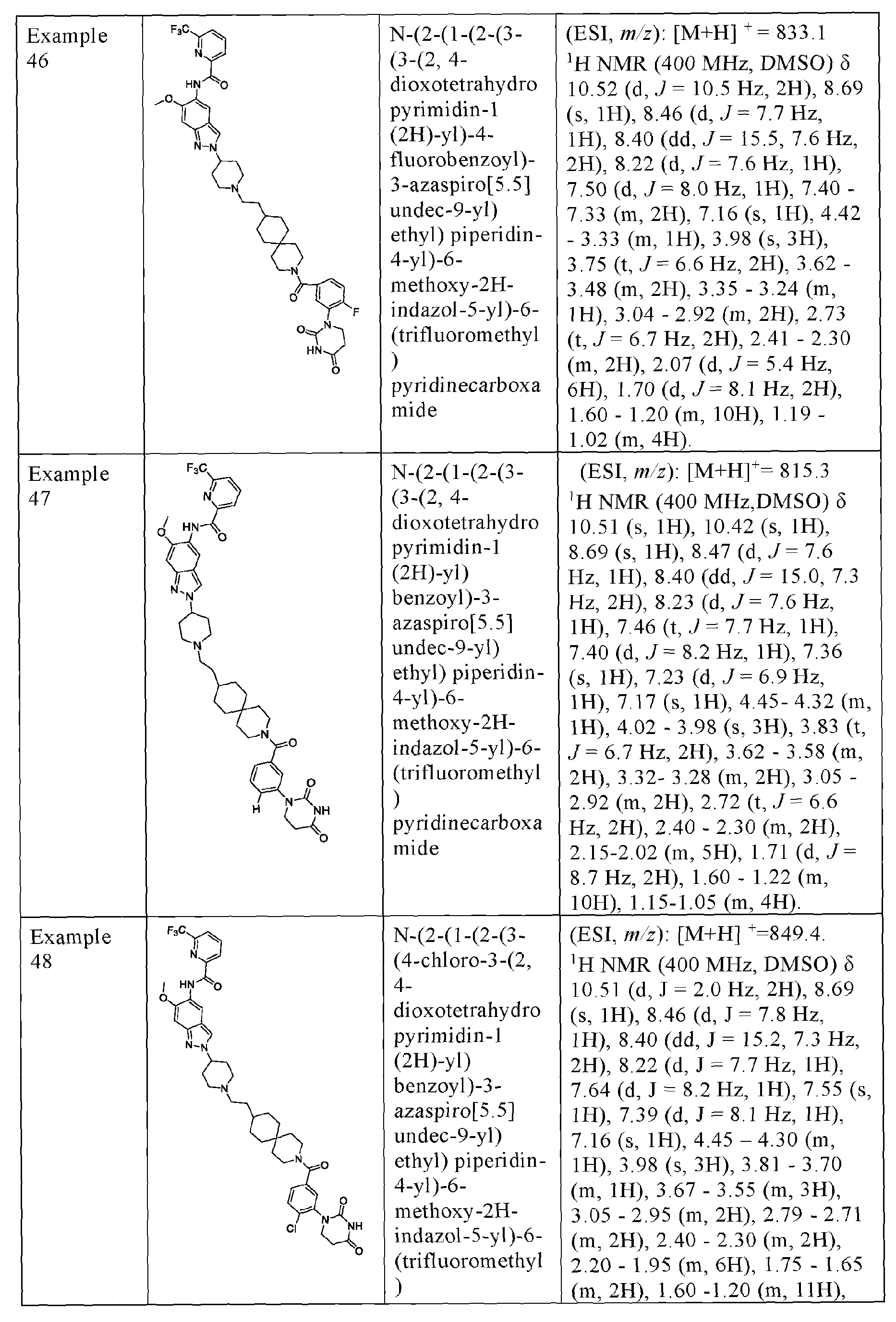

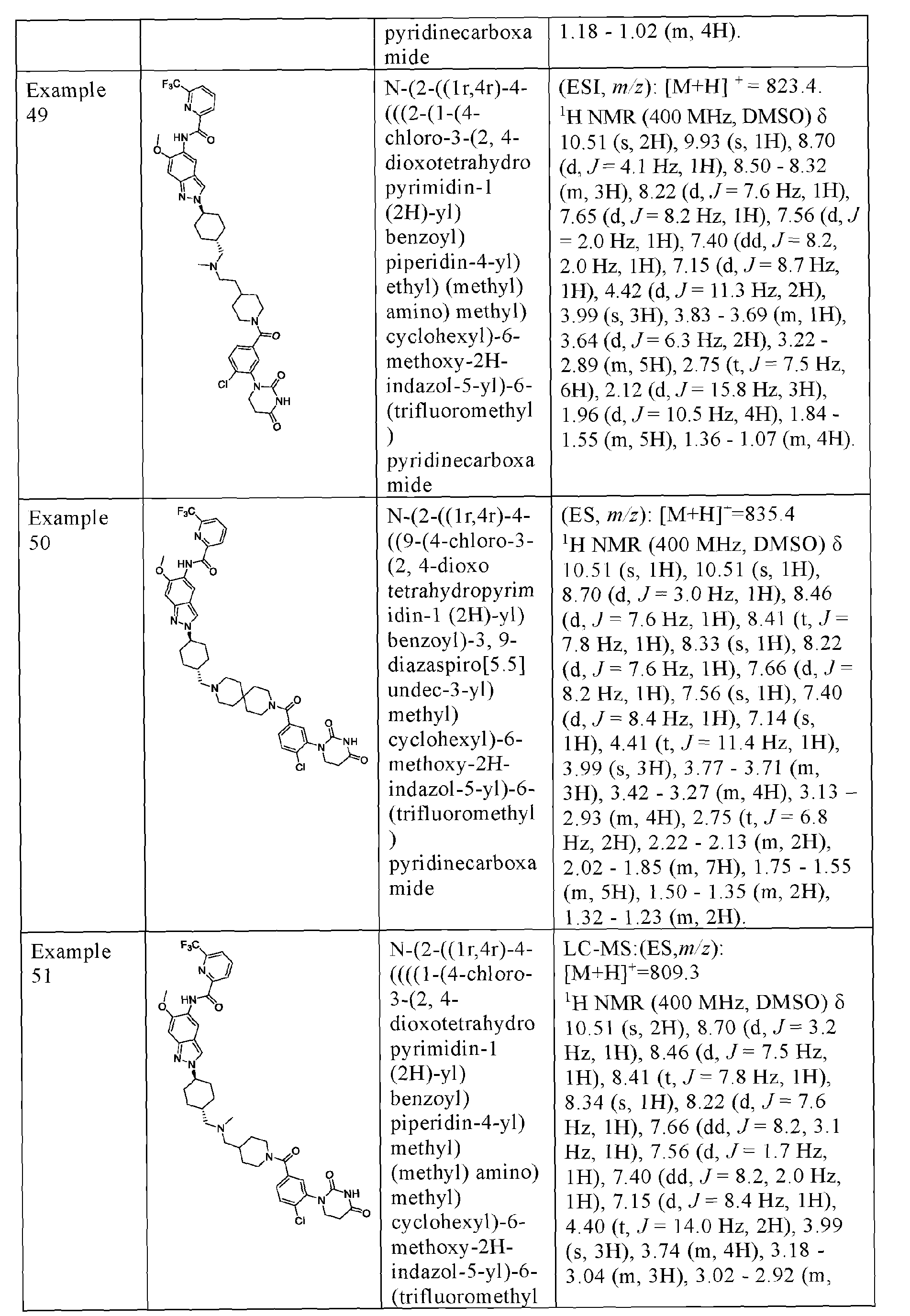

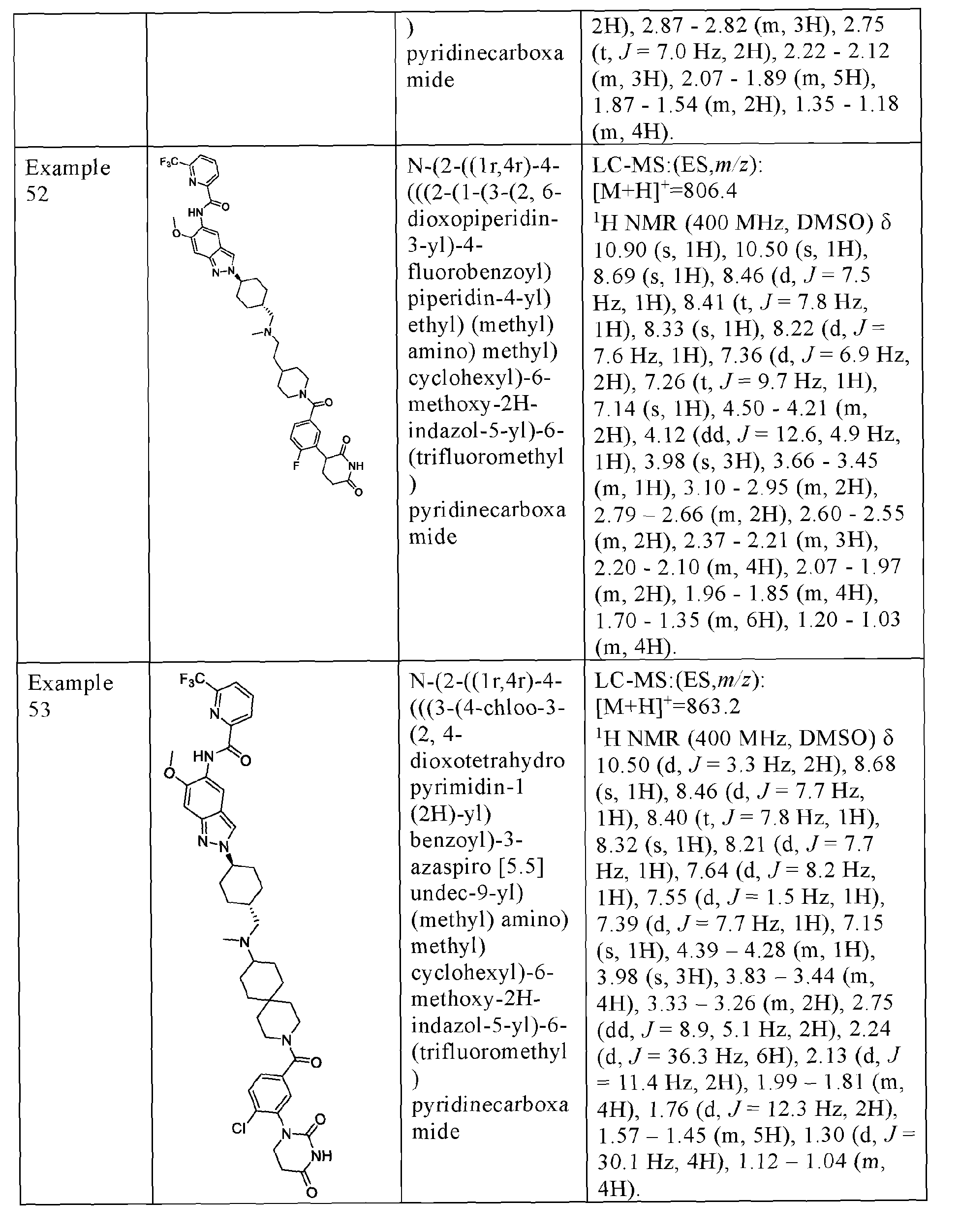

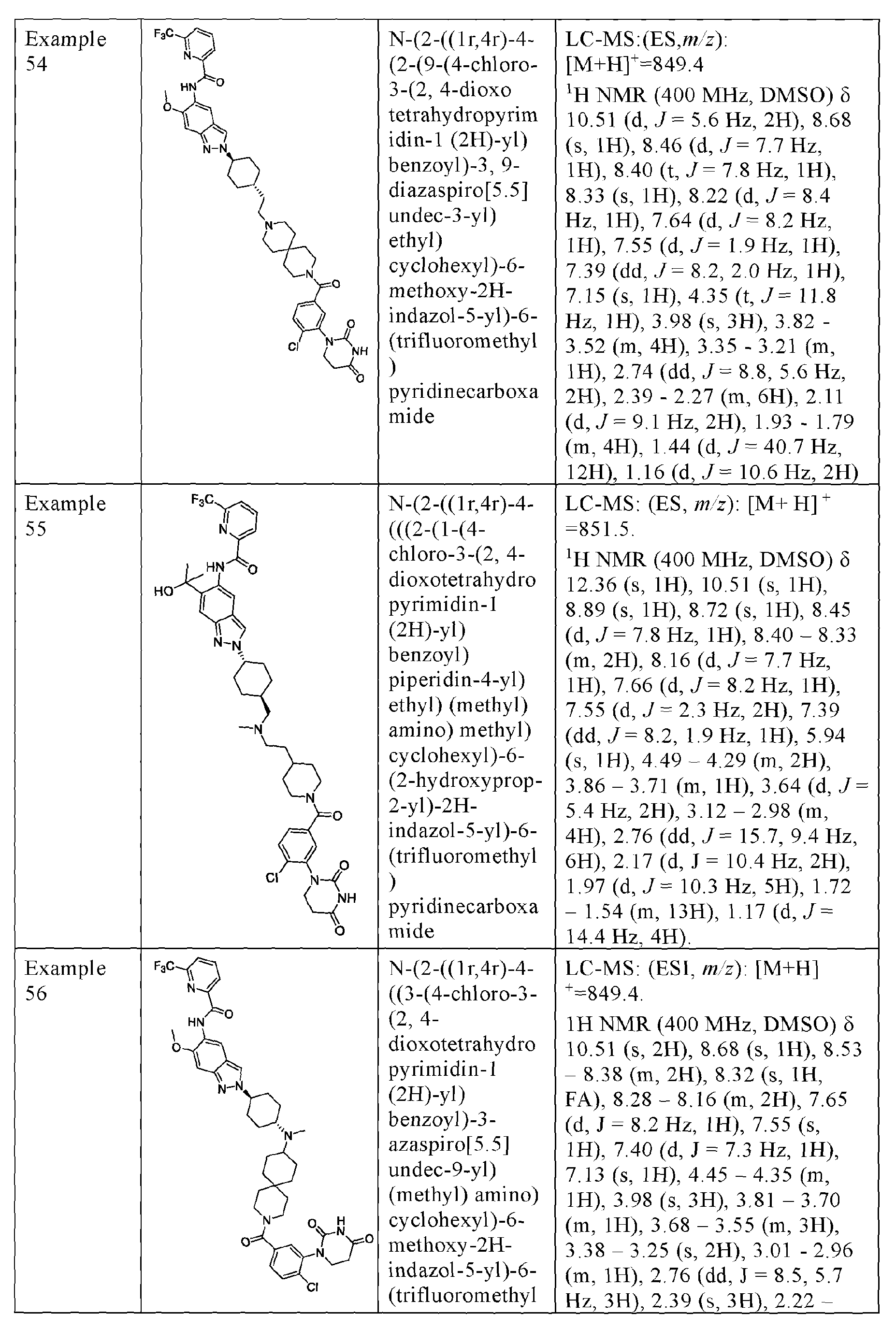

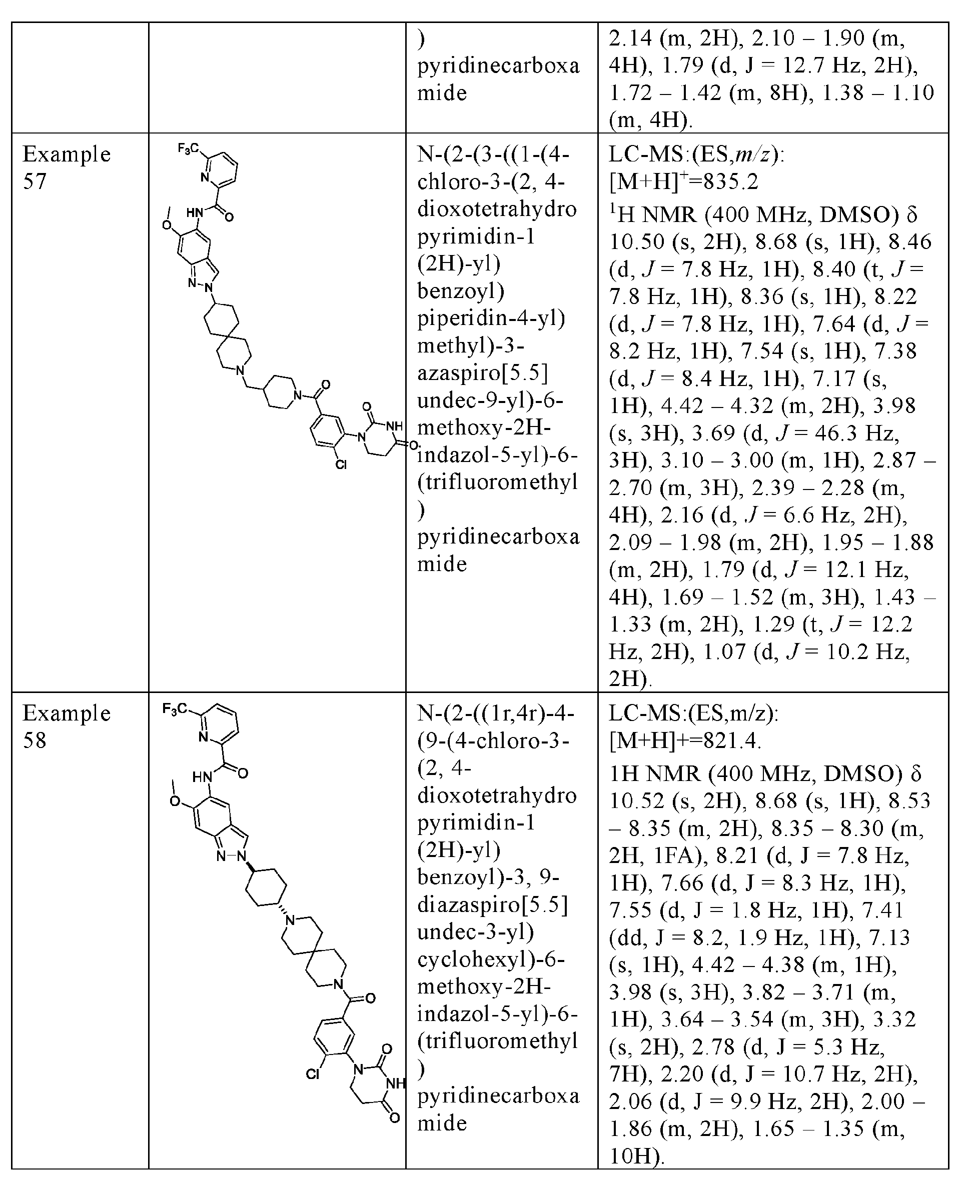

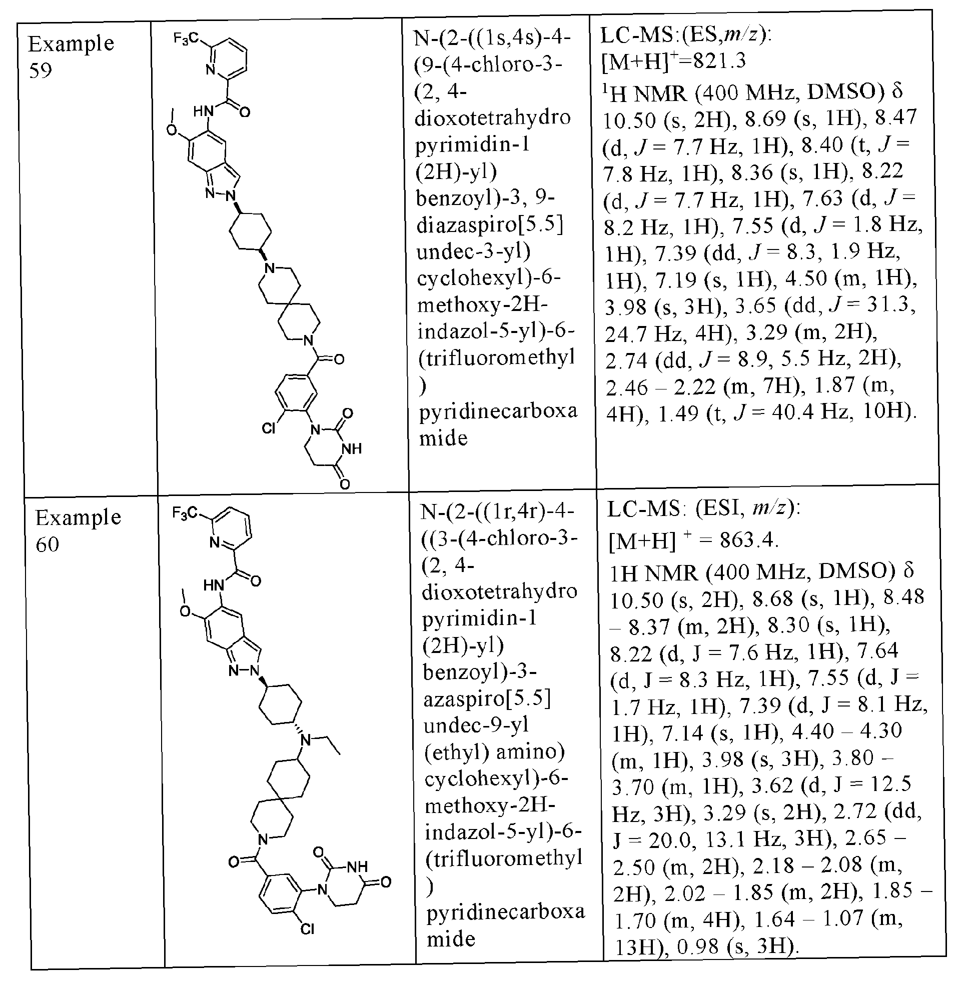

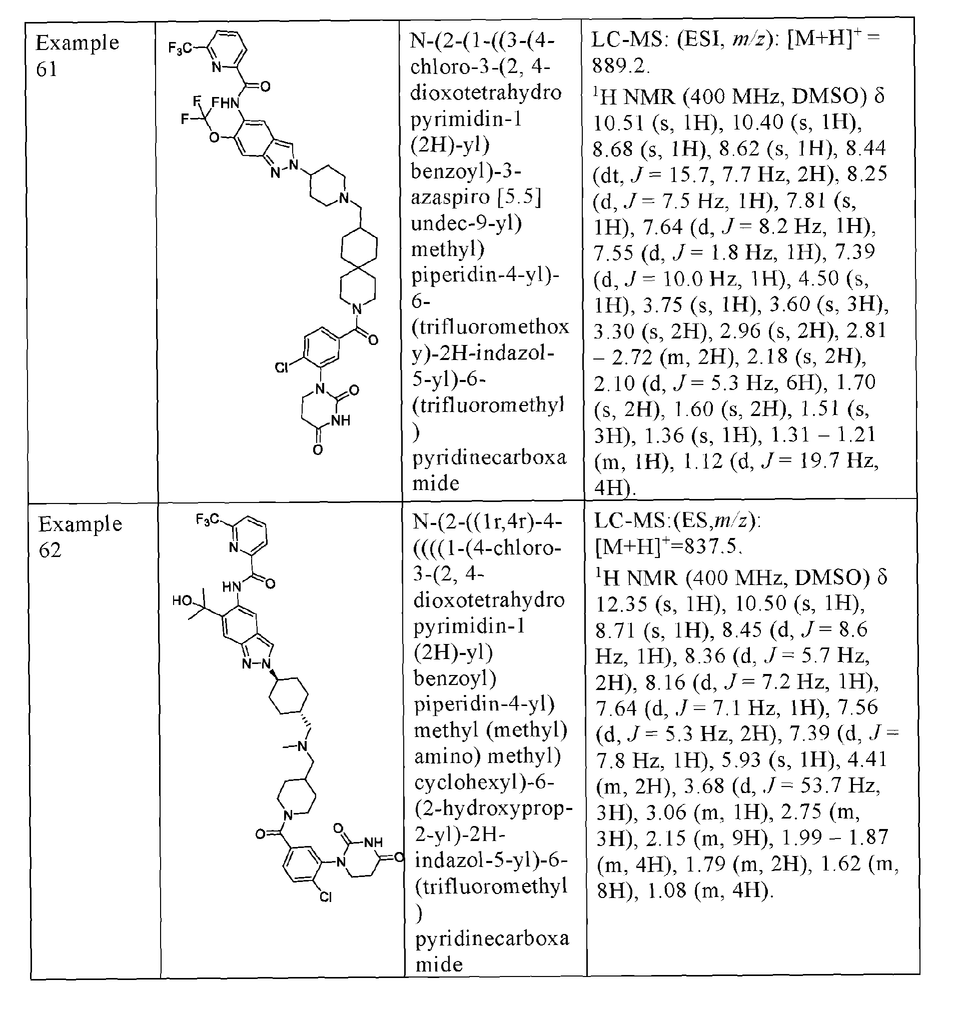

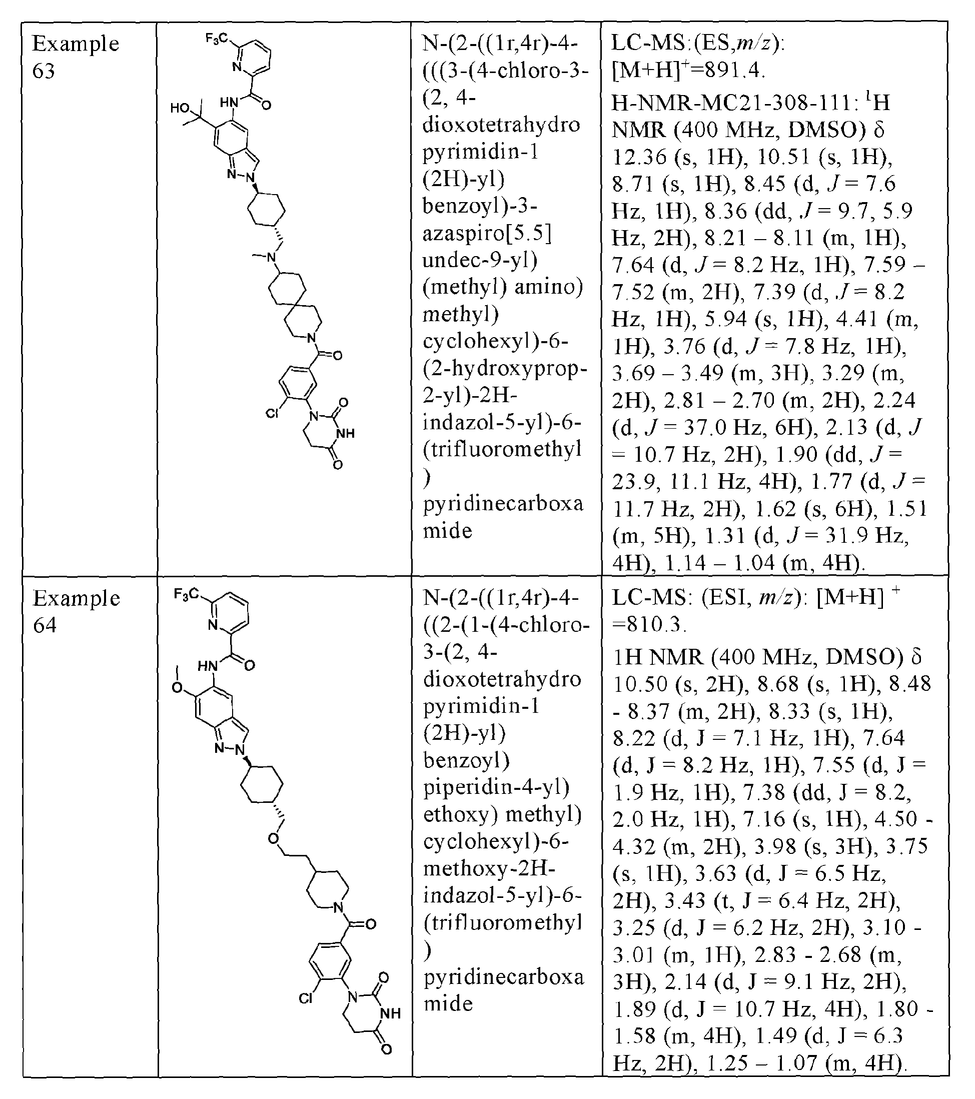

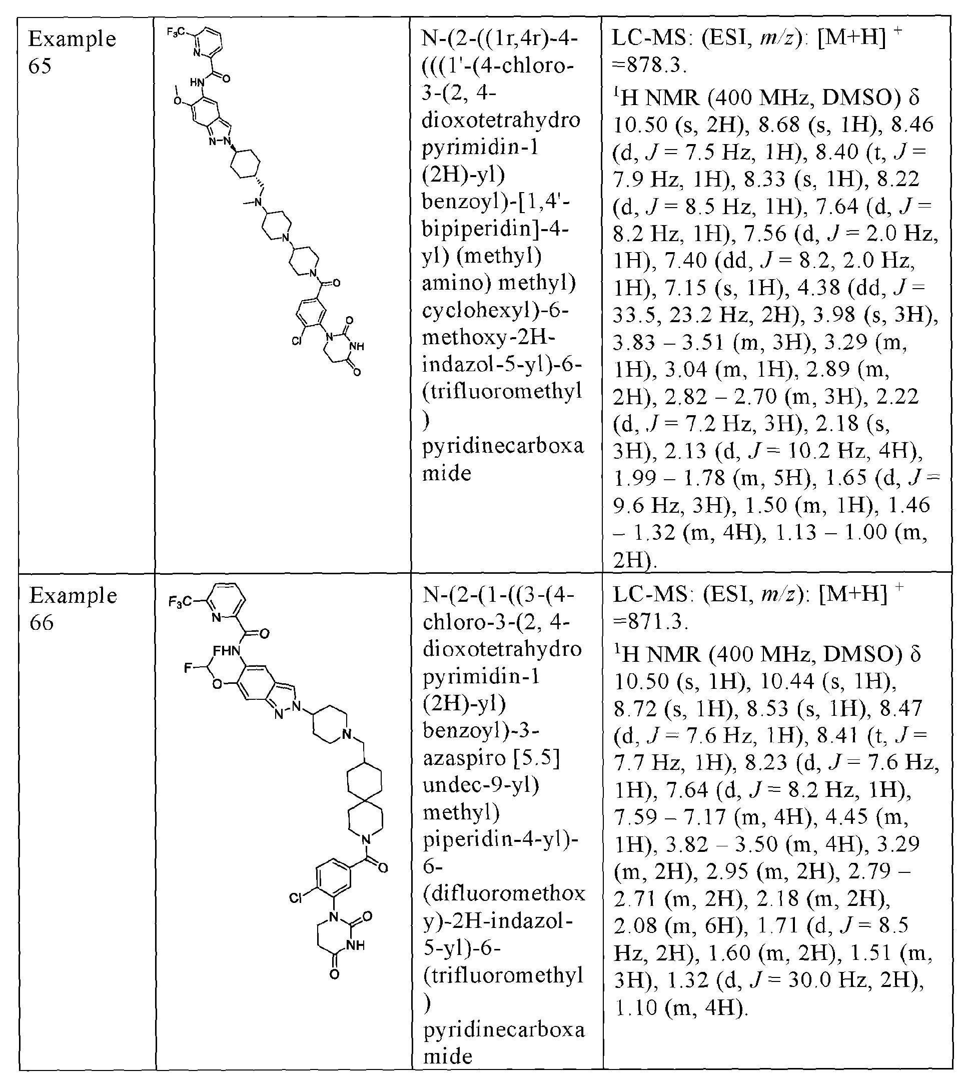

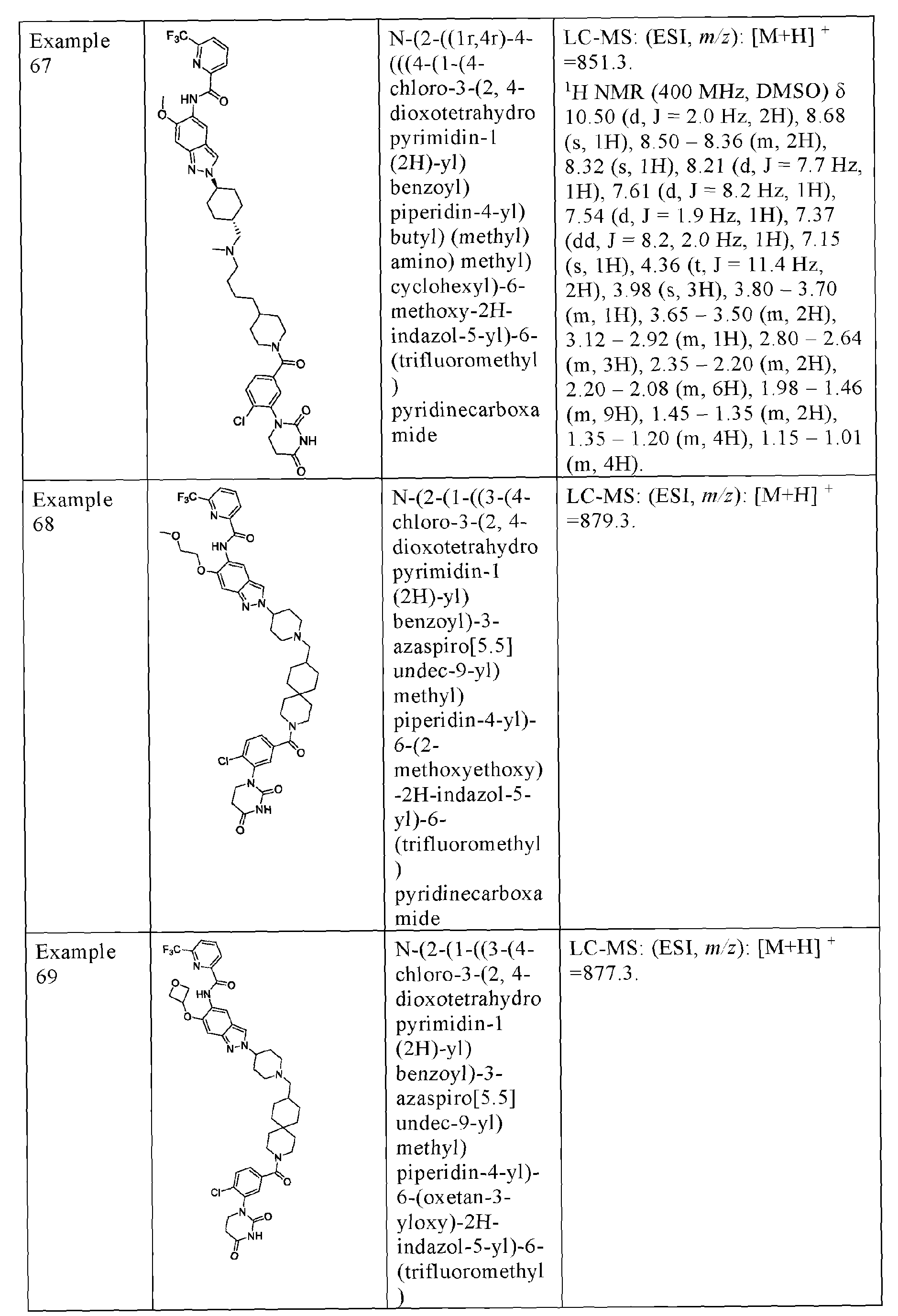

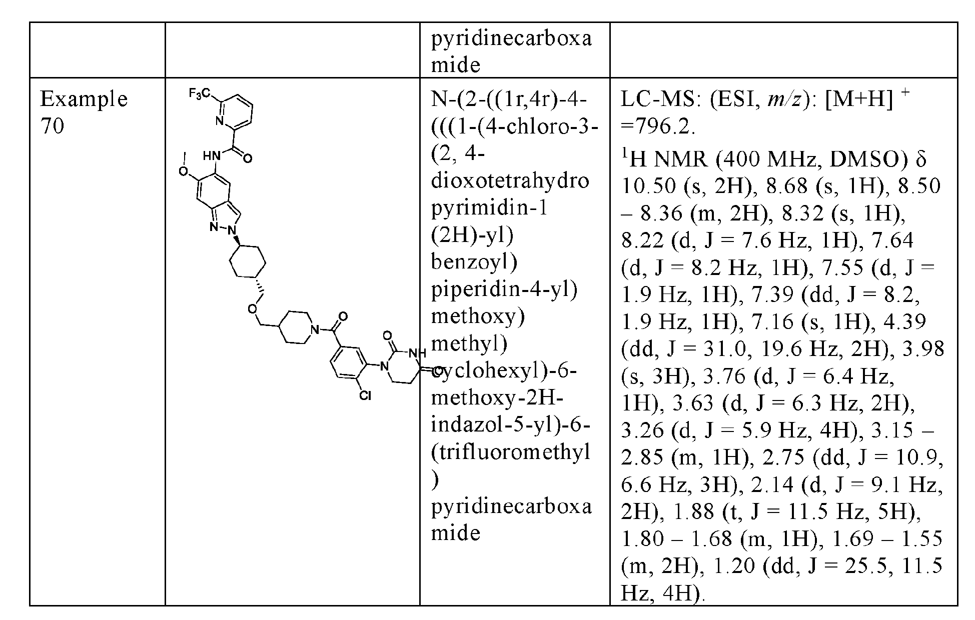

### Example 71:methyl (3-(2-chloro-5-(9-(2-(4-(6-(2-hydroxyprop-2-yl)-5-(6-(trifluoromethyl) pyridinecarboxamido)-2H-indazol-2-yl) piperidin-1-yl) ethyl)-3-azaspiro[5.5] undecyl-3-carbonyl) phenyl)-2, 6-dioxotetrahydropyrimidin-1 (2H)-yl) pivalate

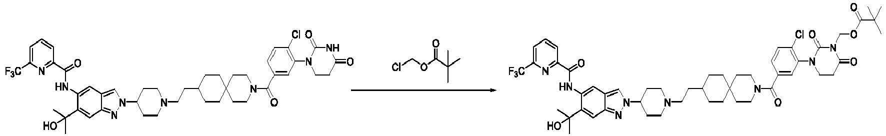

Cesium carbonate (82 mg,0.249mmol) and chloromethyl pivalate (63mg,0.416mmol) were added to a mixture of N-(2-(1-(2-(3-(4-chloro-3-(2, 4-dioxotetrahydropyrimidin-1 (2H)-yl) benzoyl)-3-azaspiro [5.5] undec-9-yl) ethyl) piperidin-4-yl)-6-(2-hydroxyprop-2-yl)-2H-indazol-5-yl)-6-(trifluoromethyl) pyridinecarboxamide (182 mg,0.208 mmol) in acetonitrile (5mL). The reaction mixture was stirred at room temperature for 4h,. filtered, then the filtrate was dried over anhydrous sodium sulfate and concentrated under reduced pressure. The concentrate was purified by preparative HPLC to obtain 50mg of target product as a light yellow solid.

LC-MS: (ESI, *m*/*z*): [M+H] ⁺= 991.1.

¹H NMR (400 MHz, DMSO) δ 12.37 (s, 1H), 8.71 (s, 1H), 8.45 (d, *J* = 7.9 Hz, 1H), 8.40-8.33 (m, 2H), 8.16 (d, *J* = 7.8 Hz, 1H), 7.66 (d, *J* = 8.2 Hz, 1H), 7.61 - 7.54 (m, 2H), 7.42 (d, *J* = 8.3 Hz, 1H), 5.94 (s, 1H), 5.69 (s, 2H), 4.49-4.36 (m, 1H), 3.84 - 3.63 (m, 2H), 3.62-3.50 (m, 2H), 3.30-3.20 (m, 2H), 2.97 (dt, *J* = 16.7, 7.9 Hz, 4H), 2.40-2.35 (m, 2H), 2.10-1.96 (m, 6H), 1.75-1.67 (m, 2H), 1.62 (s, 6H), 1.58-1.47 (m, 3H), 1.47-1.32 (m, 4H), 1.31-1.22 (m, 2H), 1.18-1.04 (m, 13H).

### Control group 1:N-(2-(1-(2-(2-((2-(2, 6-dioxopiperidin-3-yl)-1, 3-dioxoisoindol-5-yl) oxy) ethoxy) ethyl) piperidin-4-yl)-6-methoxy-2H-indazol-5-yl)-6-(trifluoromethyl) pyridinecarboxamide

### Step 1: Preparation of 2-(2, 6-dioxopiperidin-3-yl)-5-(2-(2-hydroxyethoxy) ethoxy) isoindolin-1, 3-dione

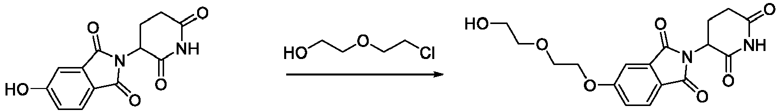

N,N-diisopropylethylamine(706.2mg,0.0055mol) and potassium iodide (30mg,0.0002mol) were added dropwise to a mixture of 2-(2,6-dioxopiperidin-3-yl)-5-hydroxyisoindolin-1,3-dione (500 mg, 0.0018 mol) and 2-(2-chloroethoxy)ethyl-1-ol (227 mg, 0.0018 mol) in dimethylsulfoxide (20 mL). The reaction solution was stirred at 100 °C for 10h. Water (30ml) and ethyl acetate (100ml) were added, the water layer was separated, and extracted with ethyl acetate (100ml × 2). The organic phases were combined and washed with saline (100mL × 2), dried over anhydrous sodium sulfate, filtered and concentrated under reduced pressure. The concentrate was purified by column chromatography to obtain 612 mg of target product as a white solid.

LC-MS: (ES, *m*/*z*): [M+H]⁺= 363.0

### Step 2: Preparation of 2-(2-((2-(2, 6-dioxopiperidin-3-yl)-1, 3-dioxoisoindolin-5-yl) oxy) ethoxy) acetaldehyde

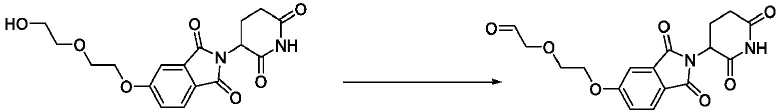

Dess-martin(393mg,0.88mmol) was added to a mixture of 2-(2, 6-dioxopiperidin-3-yl)-5-(2-(2-hydroxyethoxy) ethoxy) isoindolin-1, 3-dione (162 mg,0.44 mmol) in tetrahydrofuran (10 mL) at 0 °C, and the obtained reaction solution was stirred for 16 hours at room temperature. The reaction solution was quenched with water (30ml) and dichloromethane (50ml). The water layer was separated and extracted with dichloromethane (50ml × 2). The organic phases were combined, washed with saturated saline (50mL × 2), dried over anhydrous sodium sulfate, filtered and concentrated under reduced pressure. The concentrate was purified by column chromatography to obtain 32 mg of target product as a white solid.

LC-MS: (ES, *m*/*z*): [M+H]⁺= 361.0

### Step 3:preparation of N-(2-(1-(2-(2-((2-(2, 6-dioxopiperidin-3-yl)-1, 3-dioxoisoindol-5-yl) oxy) ethoxy) ethyl) piperidin-4-yl)-6-methoxy-2H-indazol-5-yl)-6-(trifluoromethyl) pyridinecarboxamide

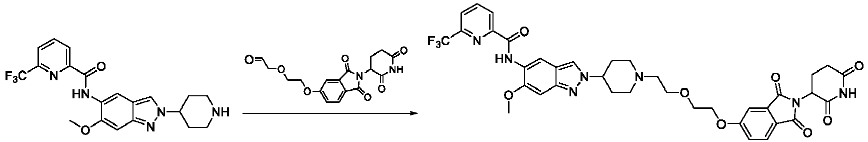

Sodium triacetoxyborohydride(36mg,0.17mmol) was added to a mixture of N-(6-methoxy-2-(piperidin-4-yl)-2H-indazol-5-yl)-6-(trifluoromethyl) pyridinecarboxamide (24 mg,0.05 mmol) and 2-(2-((2-(2, 6-dioxopiperidin-3-yl)-1, 3-dioxoisoindol-5-yl) oxy) ethoxy) acetaldehyde (40 mg,0.11 mmol) in 1, 2-dichloroethane (5 ml). The reaction solution was stirred at room temperature for 2h. The solvent was removed by concentration under reduced pressure, and the concentrate was purified by HPLC to obtain 3.25 mg of target product as a white solid.

LC-MS: (ES, *m*/*z*): [M+H]⁺=764.3

¹H-NMR-LT-002-007: ¹H NMR (400 MHz, CD₃OD_SPE) δ 8.77 (s, 1H), 8.47 (d, *J* = 8.0 Hz, 3H), 8.31 (t, *J* = 7.8 Hz, 1H), 8.18 (s, 1H), 8.05 (d, *J* = 7.4 Hz, 1H), 7.81 (d, *J* = 8.3 Hz, 1H), 7.47 (d, *J =* 1.9 Hz, 1H), 7.35 (d, *J* = 8.3 Hz, 1H), 7.07 (s, 1H), 5.12 - 5.07 (m, 1H), 4.51 (s, 1H), 4.37 (s, 2H), 4.05 (s, 3H), 3.91 (s, 2H), 3.82 (t, *J =* 5.0 Hz, 2H), 3.37 (s, 2H), 2.96 (s, 2H), 2.90 - 2.77 (m, 1H), 2.77 - 2.60 (m, 4H), 2.26 (s, 4H), 2.08 (d, *J* = 5.2 Hz, 1H).

### Control group 2:N-(2-(1-((1-(2-((2-(2, 6-dioxopiperidin-3-yl)-1, 3-dioxoisoindol-5-yl) oxy) ethyl) piperidin-4-yl) methyl) piperidin-4-yl)-6-methoxy-2H-indazol-5-yl)-6-(trifluoromethyl) pyridinecarboxamide

### Step 1: Preparation of 5-(allyloxy)-2-(2, 6-dioxopiperidin-3-yl) isoindolin-1, 3-dione

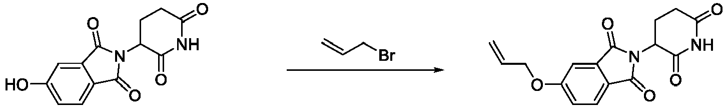

Under nitrogen protection, 3-bromoprop-1-ene (470mg,3.89mmol) was added to a mixture of 2-(2, 6-dioxopiperidin-3-yl) -5-hydroxyisoindolin-1, 3-dione (1g,3.65mmol) and potassium carbonate (1.01g,7.29mmol) in N,N-dimethylformamide (10mL). The reaction mixture was stirred overnight at 50°C. At 0 °C, the reaction was quenched with water (60mL) and extracted with ethyl acetate (30mL × 3). The organic phase was collected and washed with saturated saline, dried over anhydrous sodium sulfate, filtered and concentrated under reduced pressure. The concentrate was purified by column chromatography to obtain 0.85g of the target product.

LC-MS: (ES, *m*/*z*): [M+H]⁺=315.1

### Step 2:2-((2-(2,6-dioxopiperidin-3-yl)-1, 3-dioxoisoindolin-5-yl) oxy) acetaldehyde

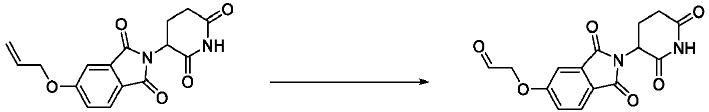

Ozone was introduced into a solution of 5-(allyloxy)-2-(2, 6-dioxopiperidin-3-yl) isoindolin-1, 3-dione (0.7g,2.23mmol) in methylene chloride (200ml) at -78°C until the reaction solution turned to be blue. Then nitrogen was introduced until the reaction solution became colorless. Dimethyl sulfide (7.61g,123mmol) was added at -78 °C under nitrogen protection, and the reaction mixture was stirred at room temperature overnight. The reaction solution was concentrated, and the concentrate was purified by column chromatography to obtain the target product.

LC-MS: (ES, *m*/*z*): [M+H]⁺=317.1

### Step 3:Preparation of N-(2-(1-((1-(2-((2-(2, 6-dioxopiperidin-3-yl)-1, 3-dioxoisoindol-5-yl) oxy) ethyl) piperidin-4-yl) methyl) piperidin-4-yl)-6-methoxy-2H-indazol-5-yl)-6-(trifluoromethyl) pyridinecarboxamide

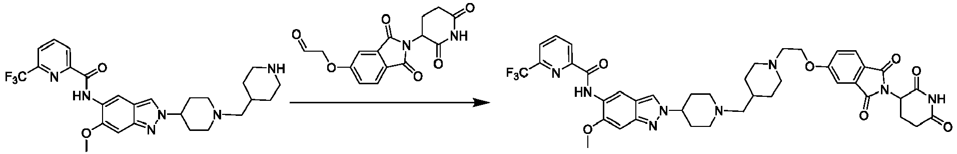

It was synthesized with reference to the method of step 3 of control group 1.

LC-MS: (ESI, *m*/*z*): [M+H]⁺= 817.1.

¹H NMR (400 MHz, DMSO-d₆) δ 11.11 (s, 1H), 10.50 (s, 1H), 8.69 (s, 1H), 8.46 (d, *J* = 7.6 Hz, 1H), 8.41 (t, *J* = 7.8 Hz, 1H), 8.37 (s, 1H), 8.22 (d, *J* = 8.6 Hz, 1H), 7.83 (d, *J* = 8.3 Hz, 1H), 7.47 (d, *J* = 2.2 Hz, 1H), 7.37 (dd, *J* = 8.3, 2.3 Hz, 1H), 7.16 (s, 1H), 5.12 (dd, *J =* 12.8, 5.3 Hz, 1H), 4.43 - 4.34 (m, 1H), 4.29 (t, *J* = 5.5 Hz, 2H), 3.00 - 2.91 (m, 5H), 2.78 - 2.70 (m, 2H), 2.64 - 2.54 (m, 2H), 2.22 - 2.16 (m, 3H), 2.13 - 2.02 (m, 9H), 1.74 - 1.66 (m, 2H), 1.58 - 1.44 (m, 1H), 1.18 - 1.08 (m, 2H).

### Control group 3:N-(2-((1r,4r)-4-((((1-(2-(2, 6-dioxopiperidin-3-yl)-1, 3-dioxoisoindolin-4-yl) piperidin-4-yl) methyl) (methyl) amino) methyl) cyclohexyl)-6-(2-hydroxyprop-2-yl)-2H-indazol-5-yl)-6-(trifluoromethyl) pyridinecarboxamide

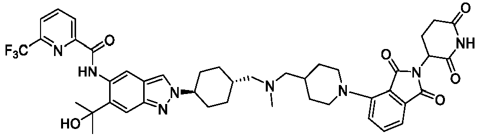

It was prepared with reference to the method of compound I-317 in WO2020113233.

LC-MS: (ESI, *m*/*z*): [M+H]⁺= 843.3

¹H NMR (400 MHz, DMSO-d₆) δ 12.36 (s, 1H), 11.08 (s, 1H), 8.71 (s, 1H), 8.45 (d, *J =* 7.8 Hz, 1H), 8.41 - 8.32 (m, 2H), 8.16 (d, *J* = 7.8 Hz, 1H), 7.74 - 7.62 (m, 1H), 7.57 (s, 1H), 7.42 - 7.17 (m, 2H), 5.94 (s, 1H), 5.09 (dd, *J =* 12.8, 5.3 Hz, 1H), 4.47 - 4.36 (m, 1H), 3.70 (d, *J* = 10.0 Hz, 2H), 2.90 (d, *J* = 12.7 Hz, 3H), 2.65 - 2.52 (m, 2H), 2.25 - 2.10 (m, 9H), 2.05- 1.81 (m, 7H), 1.74 - 1.55 (m, 8H), 1.32 (d, *J =* 11.6 Hz, 2H), 1.11 (d, *J =* 11.9 Hz, 2H)

### II Biological Activity Test Example

### Test Example 1: IRAK4 kinase activity test

KinEASE-STK S1 serine/threonine kinase kit (Cisbio) was used to detect the inhibitory effect of the compound on IRAK4 kinase activity. The specific method was: the compound was dissolved in dimethyl sulfoxide, and then it was diluted with the buffer solution of the kit by equal gradient to make the final concentration range of the tested compound in the reaction system 10000 nM-0.038 nM, then 2.5 nM kinase, 1 µM biotinylated polypeptide substrate and 7 µM adenosine triphosphate (ATP) were added in sequence and incubated at 37 °C for 120 min. Subsequently, anti-phosphorylated serine/threonine antibody coupled with europium element compound and modified XL665 streptavidin were added to the reaction system to terminate the reaction. After incubating at room temperature for 1h, the fluorescence intensity of each well at the emission wavelength of 620 nm and 665 nm and the excitation wavelength of 337nm was determined in HTRF mode on the microplate reader EnVision(PerkinElmer), the ratio value was calculated using the formula Ratio =(665 nm/620 nm)× 10⁴. By comparing with the fluorescence intensity ratio of the control group, the inhibition rate of the compound at each concentration was calculated, and then the IC₅₀ value of the compound was obtained by fitting the nonlinear curve with the logarithmic concentration-inhibition rate by GraphPad Prism 7.

**Table 1:**

| Example | IC₅₀(nM) |
|---|---|
| Example 1 | 2.5 |
| Example 2 | 2.9 |
| Example 3 | 2.7 |
| Example 4 | 12.2 |
| Example 5 | 9.1 |
| Example 6 | 7.6 |
| Example 7 | 5.9 |
| Example 8 | 10.5 |
| Example 9 | 18.0 |
| Example 10 | 6.5 |
| Example 11 | 6.0 |
| Example 12 | 7.9 |
| Example 13 | 7.9 |
| Example 14 | 6.9 |
| Example 15 | 6.0 |
| Example 16 | 9.2 |
| Example 17 | 7.9 |
| Example 18 | 9.6 |
| Example 19 | 16.9 |
| Example 20 | 9.5 |
| Example 21 | 18.0 |
| Example 22 | 11.7 |
| Example 23 | 8.5 |
| Example 24 | 34.6 |
| Example 25 | 13.7 |
| Example 26 | 6.5 |
| Example 27 | 13.8 |
| Example 28 | 16.2 |
| Example 29 | 8.6 |
| Example 30 | 16.0 |
| Example 31 | 14.0 |
| Example 32 | 28.5 |
| Example 33 | 17.2 |
| Example 34 | 16.5 |
| Example 35 | 10.3 |
| Example 36 | 11.8 |
| Example 37 | 30.5 |
| Example 38 | 34.5 |
| Example 39 | 16.7 |
| Example 40 | 35.1 |
| Example 41 | 28.1 |
| Example 42 | 39.2 |
| Example 43 | 24.2 |
| Example 44 | 52.4 |
| Example 45 | 23.5 |
| Example 46 | 43.1 |
| Example 47 | 34.2 |
| Example 48 | 61.8 |
| Example 49 | 3.4 |
| Example 50 | 7.2 |
| Example 51 | 11.3 |
| Example 52 | 9.2 |
| Example 53 | 6.3 |
| Example 54 | 7.5 |
| Example 55 | 10.6 |
| Example 56 | 3.6 |
| Example 57 | 3.8 |
| Example 58 | 4.3 |
| Example 59 | 11.04 |
| Example 60 | 6.43 |
| Example 61 | 128.2 |
| Example 62 | 6.9 |
| Example 63 | 4.4 |
| Example 64 | 48.0 |
| Example 65 | 1.6 |
| Example 66 | 21.4 |
| Example 67 | 5.13 |
| Example 71 | 114.0 |
| Control group 1 | 44.2 |
| Control group 2 | 15.2 |
| Control group 3 | 16.2 |

Experimental results: The compound of the invention can effectively bind with the target protein and inhibit the IRAK4 kinase activity.

### Test Example 2: Degradation of IRAK4 in THP-1 Cells by Compounds

Each well of the 24-well cell culture plate was inoculated with 0.95 mL THP-1 cells (stem cell bank of Chinese academy of sciences) with a cell density of 5 × 10⁵cells/well. The cell plate was placed in a 5% carbon dioxide incubator and cultured overnight at 37 °C, then 50 µL compound in dimethyl sulfoxide was added. The final concentration of the compound was in the range of 1-3000 nM. After continuous cultivation for 24 hours, the cells were collected into a 1.5 mL centrifuge tube, centrifuged at 1000 rpm and 4 °C for 5 minutes. Cell precipitation was cleaned twice with 1 ×DPBS, resuspended cells were lysed with 200 µL lysate (cell lysate was Western and IP cell lysate (Beyotime), supplemented with 1 mM mixture of phenylmethylsulfonyl fluoride and protease inhibitor (Beyotime)), left on ice for 30 minutes, then centrifuged at 14000g at 4 °C for 10 minutes, and the supernatant was taken to detect IRAK4 protein level by Western blot.

Western blot method: The total protein concentration in the supernatant of cell lysis was determined by BCA protein quantitative kit (Tiangen). According to the concentration of total protein detected by BCA, the supernatant was adjusted to 0.2 µg/µL with PBS and 5 × SDS-PAGE protein loading buffer (Beyotime), bathed in water at 100 °C for 10 minutes, then placed in ice bath for 5 minutes, centrifuged at 14000g and 4 °C for 5 minutes, and then collected as WB loading sample. Prefabricated glue (KeyGEN) was used for protein electrophoresis with a loading amount of 10 µL (total protein 2 µg). After Tris-MOPS-SDS electrophoresis solution (Adamas), 120V constant pressure electrophoresis was performed. After electrophoresis, the protein on the adhesive strip was transferred to PVDF membrane with a constant current of 250V for 50 minutes. After the membrane was transferred, the membrane was placed in 1 × QuickBlock blocking buffer (Beyotime) and incubated for 30 minutes at room temperature. After blocking, PVDF membranes were incubated with IRAK4 primary antibody (Abcam) overnight at 4°, the membranes were washed with TBST buffer (2.4g Tris,8.8g NaCl,1.5 mL Tween 20, pH adjusted to 7.4, constant volume to 1L) for 30 minutes, incubated with secondary antibody (Abcam) for 2 hours at room temperature, finally incubated with Clarity Western ECL Substrate(BIO-RAD) for 5 minutes for luminescence development, chemiluminescence imaging system (Clinx, chemiScope 6200 Touch) for development and protein mapping photography. The protein map was analyzed by Clinx chemiluminescence analysis software for grayscale values. Using the formula: grayscale correction value = (target protein grayscale value/corresponding internal reference grayscale value) × 10³, the grayscale correction value of each sample was calculated. The degradation rate was calculated by comparing with the grayscale correction value of the control group. Furthermore, DC₅₀ and Dₘₐₓ values of the compound were obtained by the nonlinear curve fitting with the logarithmic concentration-inhibition rate by GraphPad Prism 7.

**Table 2:**

| Example | DC₅₀(nM) | Dₘₐₓ(%) |
|---|---|---|
| Example 1 | 3.0 | 94.6 |
| Example 2 | 7.4 | 99.1 |
| Example 3 | 23.1 | 90.5 |
| Example 6 | 24.0 | 91.7 |
| Example 7 | 9.4 | 95.5 |
| Example 9 | 16.4 | 94.3 |
| Example 10 | 2.3 | 100.0 |
| Example 11 | 27.7 | 99.6 |
| Example 12 | 1.4 | 96.9 |
| Example 16 | 7.1 | 99.4 |
| Example 17 | 5.3 | 96.9 |
| Example 18 | 7.1 | 99.4 |
| Example 19 | 13.4 | 92.8 |
| Example 20 | 12.2 | 93.7 |
| Example 21 | 4.0 | 90.1 |
| Example 22 | 16.4 | 94.3 |
| Example 23 | 32.4 | 97.6 |
| Example 25 | 0.82 | 98.0 |
| Example 26 | 1.0 | 86.9 |
| Example 27 | 3.5 | 96.3 |
| Example 28 | 0.8 | 99.4 |
| Example 30 | 2.3 | 98.4 |
| Example 31 | 2.6 | 97.1 |
| Example 32 | 5.1 | 96.9 |
| Example 33 | 2.1 | 96.6 |
| Example 34 | 2.5 | 98.1 |
| Example 35 | 8.4 | 95.2 |
| Example 36 | 4.0 | 92.5 |
| Example 39 | 10.5 | 89.4 |
| Example 40 | 4.8 | 98.0 |
| Example 41 | 2.9 | 97.7 |
| Example 42 | 7.8 | 100.0 |
| Example 43 | 4.9 | 98.7 |
| Example 44 | 2.3 | 98.5 |
| Example 45 | 1.9 | 98.2 |
| Example 46 | 4.0 | 97.8 |
| Example 47 | 5.4 | 98.3 |
| Example 48 | 6.0 | 98.0 |
| Example 49 | 1.8 | 99.2 |
| Example 50 | 5.6 | 97.0 |
| Example 51 | 27.5 | 95.4 |
| Example 53 | 5.9 | 96.3 |
| Example 54 | 1.4 | 98.9 |
| Example 55 | 2.70 | 98.1 |
| Example 56 | 3.0 | 97.0 |
| Example 58 | 7.6 | 92.1 |
| Example 60 | 5.1 | 93.2 |
| Example 62 | 5.6 | 92.3 |
| Example 64 | 13.9 | 95.6 |
| Example 65 | 13.4 | 98.3 |
| Example 66 | 16.1 | 95.3 |
| Example 67 | 11.5 | 99.2 |
| Example 71 | 3.70 | 97.0 |
| Control group 1 | 38.1 | 73.9 |
| Control group 2 | 40.6 | 85.1 |
| Control group 3 | 19.8 | 75.3 |

Experimental results: The compound of the present invention can effectively degrade IRAK4 kinase protein in THP-1 cells and has excellent degradation activity.

### Test Example 3: Degradation of IKZF1 and IKZF3 in L363 Cells by Compound

The 24-well cell culture plate was inoculated with 0.95 mL L363 cells (Nanjing Co-bioer) per well with a cell density of 6 × 10⁵ cells/well. The cell plate was placed in a 5% carbon dioxide incubator and cultured at 37 °C for 8 hours, then 50 µL of compound solution was added. The final concentration of the compound was 1000 nM. After continuing to culture for 16 hours, the cells were collected into a 1.5 mL centrifuge tube and centrifuged at 3000 rpm and 4 °C for 5 minutes. Cell precipitation was cleaned twice with 1 ×DPBS, resuspended cells were lysed with 100 µL lysate (cell lysate was Western and IP cell lysate (Beyotime), supplemented with 1 mM mixture of phenylmethylsulfonyl fluoride and protease inhibitor (Beyotime)), left on ice for 30 minutes, then centrifuged at 14000g at 4 °C for 10 minutes, and the supernatant was taken to detect IKZF1 and IKZF3 protein level by Western blot.

The total protein concentration in the supernatant of cell lysis was determined by BCA protein quantitative kit (Tiangen). According to the concentration of total protein detected by BCA, the supernatant was adjusted to 0.2 µg/µL with PBS and 5 × SDS-PAGE protein loading buffer (Beyotime), bathed in water at 100 °C for 10 minutes, then placed in ice bath for 5 minutes, centrifuged at 14000g and 4 °C for 5 minutes, and then collected as WB loading sample. Prefabricated glue (KeyGEN) was used for protein electrophoresis with a loading amount of 10 µL (total protein 2 µg). After Tris-MOPS-SDS electrophoresis solution (Adamas), 120V constant pressure electrophoresis was performed. After electrophoresis, the protein on the adhesive strip was transferred to PVDF membrane with a constant current of 250V for 50 minutes. After the membrane was transferred, the membrane was incubated in 5% bovine serum albumin solution for 2 hours at room temperature. After blocking, PVDF membranes were incubated with IKZF1 primary antibody (CST) and IKZF3 primary antibody (Abcam) overnight at 4°, the membranes were washed with TBST buffer (2.4g Tris,8.8g NaCl,1.5 mL Tween 20, pH adjusted to 7.4, constant volume to 1L) for 30 minutes, incubated with secondary antibody (Abcam) for 2 hours at room temperature, finally incubated with Clarity Western ECL Substrate(BIO-RAD) for 5 minutes for luminescence development, chemiluminescence imaging system (Clinx, chemiScope 6200 Touch) for development and protein mapping photography. The protein map was analyzed by Clinx chemiluminescence analysis software for grayscale values. Using the formula: grayscale correction value = (target protein grayscale value/corresponding internal reference grayscale value) × 10³, the grayscale correction value of each sample was calculated. The degradation rate was calculated by comparing with the grayscale correction value of the control group.

**Table 3:**

| **Compound** | **Concentration (nM)** | **IKZF1 degradation rate (%)** | **IKZF3 degradation rate (%)** |
|---|---|---|---|
| Pomalidomide | 1000 | 99.7±0.2 | 98.6±0.6 |
| Control group 2 | 1000 | 47.5±9.7 | 71.2±5.7 |
| Control group 3 | 1000 | 97.2±2.6 | 92.8±5.0 |
| Example 34 | 1000 | 10.2±1.8 | -2.2±2.8 |
| Example 31 | 1000 | 8.4±13.9 | 17.8±3.6 |
| Example 40 | 1000 | 53.8±2.0 | 9.8±2.0 |

| | | | |
|---|---|---|---|
| Note: In Table 3, "-" refers to no degradation of IKZF1 and IKZF3. | | | |

The experimental results show that the compound of the present invention has good degradation selectivity, no or a small amount of degradation to IKZF1 and IKZF3, and has small toxic side effects, which is beneficial to the patent medicine.

### Test Example 4: Determination of Cytokine Concentration of LPS-Induced Human PBMC Secretion

Frozen human PBMC (Milestone, PB010C) was resuspended to RPMI 1640 medium (Gibco,A1049101) after resuscitation, supplemented with 10% fetal bovine serum (Gibco,10099141),100 U/mL penicillin and 100 µg/mL streptomycin (Gibco,15140122). On the same day, 150 µL of PBMC was spread in each well of a 96-well transparent cell plate (Labserv,310109008), so that the cell density was 2 × 105 cells/well. Then 50 µL diluted compound solution was added to the corresponding cell-covered wells to make the final concentration of the compound in the range of 0.026-10000 nM. The final concentration of DMSO was 0.25%. After the drug-treated cell plate was cultured at 37 °C for 1 hour or 20 hours in a 5% CO2 incubator, 10 µL LPS (0111:B4) (Sigma,L4391) was added with a final concentration of 100 ng/mL. The cell plate was again placed in a 5% CO2 incubator at 37 °C for 5 hours, centrifuged at 2000 rpm for 10 minutes, and 100 µL cell supernatant was taken from each well and transferred to a new 96-well transparent cell plate for cytokine concentration test, which was frozen at -80 °C to be tested.

### Interleukin-6 (IL-6) AlphaLISA Detection

The concentration of IL-6 in the supernatant of LPS-induced human PBMC cells was determined using interleukin-6 (IL-6)AlphaLISA detection kit (PerkinElmer,AL223 C).

According to the product instructions, IL-6 standard solutions with different concentrations were prepared in the concentration range of 0-100000 pg/mL. 2 µL of IL-6 standard solution at various concentrations and the cell supernatant sample to be tested were taken, respectively added to white 384 well plates (PerkinElmer,6007299), then a mixed solution of 8 µL of 5 × anti-IL-6 receptor beads (final concentration: 10 µg/mL) and biotin-labeled anti-IL-6 antibody (final concentration 1 nM) was added to each well, and incubated at 23 °C for 60 minutes. Finally, 10 µL of 2 × streptavidin-labeled donor beads (final concentration: 40 µg/mL) were added to each well and incubated at 23°C for 30 minutes in the absent of light. After the incubation, the AlphaLISA signal value was determined in the AlphaScreen standard setting mode on the microplate reader EnVision(PerkinElmer,2105). The standard curve was drawn by the AlphaLISA signal value of each concentration IL-6 standard solution, and then based on the corresponding concentration on the standard curve for the AlphaLISA signal value of the sample to be tested, the IL-6 concentration of each cell lysis supernatant was determined. Compared with the IL-6 concentration of the control group, the inhibition rate of the compound at each concentration was calculated, and then IC₅₀ value of the compound was calculated by fitting the nonlinear curve with the logarithmic concentration-inhibition rate by GraphPad Prism 7.

**Table 4:**

| Example | IC₅₀(nM) |
|---|---|
| Control group 2 | 2046 |
| Control group 3 | 47.5 |
| Example 1 | 4.9 |
| Example 28 | 4.7 |
| Example 31 | 1.8 |
| Example 32 | 9.7 |
| Example 41 | 5.1 |
| Example 55 | 0.8 |
| Example 53 | 2.8 |
| Example 49 | 3.7 |
| Example 54 | 2.6 |
| Example 56 | 3.2 |

Experimental results: The compounds of the present invention have a good inhibitory effect on IL-6 production by immune cells.

## Claims

1. A compound of formula I, and/or a stereoisomer, an enantiomer, a diastereomer, a deuterate, a hydrate, a solvate, a prodrug and/or a pharmaceutically acceptable salt thereof:
PTM-L-ULM I
wherein:
PTM is a small molecule compound that can inhibit IRAK4 kinase protein or bind to IRAK4 kinase protein;
L is a connecting chain, which connects PTM and ULM through a covalent bond;
ULM is a small molecule ligand in E3 ubiquitin ligase complex, and the ULM has the following structure: 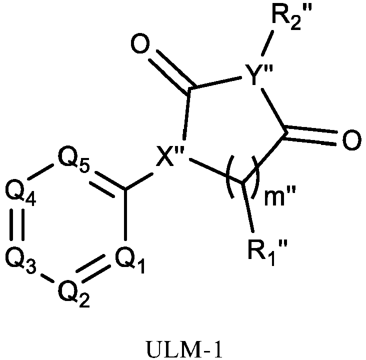
wherein, in ULM-1:
X" is CH or N;
Y" is CH, N, O or S;
Q₁, Q₂, Q₃, Q₄ and Q₅ are each independently CR₃" or N;
R₃" are each independently hydrogen, deuterium, hydroxyl, amino, cyano, halogen, C1-C6 alkyl, C3-C8 cycloalkyl, 3-8-membered heterocycloalkyl, 6-10-membered aryl, 5-10-membered heteroaryl, -O(C1-C6 alkyl), -O-(C3-C8 cycloalkyl), -O-(3-8-membered heterocycloalkyl), -N(C1-C6 alkyl)₂, -NH(C3-C8 cycloalkyl)), -NH(3-8-membered heterocycloalkyl), -O-(6-10-membered aryl),or -O-(5-10-membered heteroaryl); and the alkyl, cycloalkyl, heterocycloalkyl, aryl and heteroaryl are optionally substituted by 1-3 groups independently selected from hydroxyl, halogen, cyano, or amino; or R₃" together with its attached atoms form a cycloalkyl, heterocycloalkyl, aryl or heteroaryl;
m" is 1, 2 or 3;
Ri" are each independently hydrogen, deuterium, hydroxyl, amino, cyano, halogen, C1-C6 alkyl, C3-C8 cycloalkyl, 3-8-membered heterocycloalkyl, 6-10-membered aryl, 5-10-membered heteroaryl, or -O(C1-C6 alkyl); and the alkyl, cycloalkyl, heterocycloalkyl, aryl and heteroaryl are optionally substituted by 1-3 groups independently selected from hydroxyl, halogen, cyano, or amino;
R₂" is absent, hydrogen, deuterium, C1-C6 alkyl, or C3-C6 cycloalkyl, the C1-C6 alkyl and C3-C6 cycloalkyl are optionally substituted by 1-3 groups independently selected from hydroxyl, halogen, -O-(C=O)-(C1-C6 alkyl), cyano or amino.

2. The compound of formula I according to claim 1, and/or the stereoisomer, the enantiomer, the diastereomer, the deuterate, the hydrate, the solvate, the prodrug and/or the pharmaceutically acceptable salt thereof, wherein one or two of Q₁, Q₂, Q₃, Q₄, and Q₅ in ULM-1 is N, the rest are each independently CR₃".

3. The compound of formula I according to claim 1, and/or the stereoisomer, the enantiomer, the diastereomer, the deuterate, the hydrate, the solvate, the prodrug and/or the pharmaceutically acceptable salt thereof, wherein Q₁, Q₂, Q₃, Q₄, and Q₅ in ULM-1 are each independently CR₃".

4. The compound of formula I according to claim 1, and/or the stereoisomer, the enantiomer, the diastereomer, the deuterate, the hydrate, the solvate, the prodrug and/or the pharmaceutically acceptable salt thereof, wherein Y" in ULM-1 is N.

5. The compound of formula I according to claim 1, and/or the stereoisomer, the enantiomer, the diastereomer, the deuterate, the hydrate, the solvate, the prodrug and/or the pharmaceutically acceptable salt thereof, wherein R₁" in ULM-1 are each independently hydrogen, deuterium, -F, -Cl, or C1-C6 alkyl, the alkyl is optionally substituted by 1-3 halogens; preferably R₁" is hydrogen.

6. The compound of formula I according to claim 1, and/or the stereoisomer, the enantiomer, the diastereomer, the deuterate, the hydrate, the solvate, the prodrug and/or the pharmaceutically acceptable salt thereof, wherein R₂"in ULM-1 is hydrogen or C1-C6 alkyl, and the alkyl is optionally substituted by 1-3 halogens; preferably R₂" is hydrogen.

7. The compound of formula I according to claim 1, and/or the stereoisomer, the enantiomer, the diastereomer, the deuterate, the hydrate, the solvate, the prodrug and/or the pharmaceutically acceptable salt thereof, wherein R₃" in ULM-1 is each independently hydrogen, deuterium, halogen, -O(C1-C6 alkyl), or C1-C6 alkyl, and the alkyl is optionally substituted by 1 to 3 halogens; preferably R₃" is each independently hydrogen, deuterium, F, Cl, methyl, methoxy, ethoxy, trifluoromethoxy, 2-hydroxypropyl-2-yl or trifluoromethyl.

8. The compound of formula I according to claim 1, and/or the stereoisomer, the enantiomer, the diastereomer, the deuterate, the hydrate, the solvate, the prodrug and/or the pharmaceutically acceptable salt thereof, wherein ULM is selected from

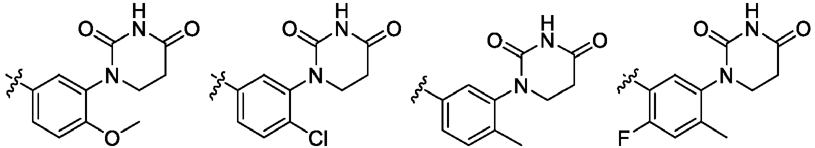

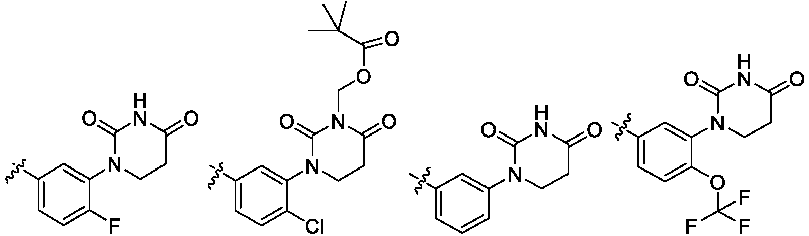

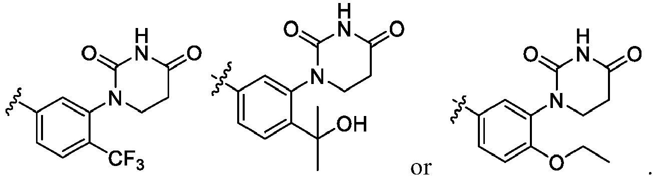

9. The compound of formula I according to claim 1, and/or the stereoisomer, the enantiomer, the diastereomer, the deuterate, the hydrate, the solvate, the prodrug and/or the pharmaceutically acceptable salt thereof, wherein ULM is selected from

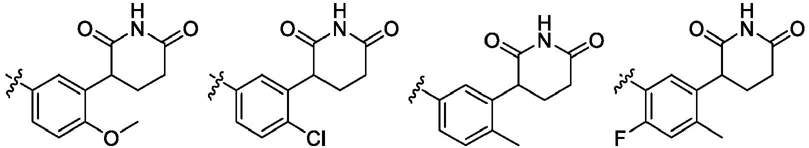

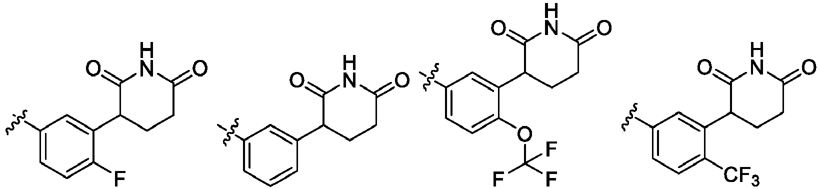

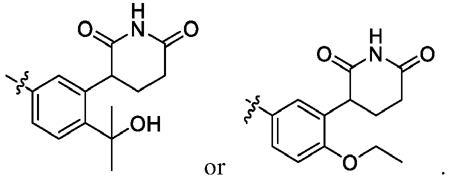

10. The compound of formula I according to claim 1, and/or the stereoisomer, the enantiomer, the diastereomer, the deuterate, the hydrate, the solvate, the prodrug and/or the pharmaceutically acceptable salt thereof, wherein PTM is 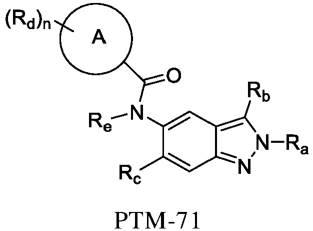 wherein, in PTM-71:
ring A is 6-10 membered aryl or 5-10 membered heteroaryl;
R_{d} is each independently hydrogen, deuterium, halogen, cyano, C1-C6 alkyl, C3-C6 cycloalkyl, or 5-10-membered heteroaryl; and the alkyl, cycloalkyl, and heteroaryl are optionally substituted by one or more groups selected from halogen, hydroxyl, or amino;
n is 1, 2, 3 or 4;
Rₑ is hydrogen or C1-C6 alkyl;
R_{c} is hydrogen, deuterium, -O-(C1-C6 alkyl), -O-(C3-C8 cycloalkyl), -O-(3-8-membered heterocycloalkyl), -O-(6-10-membered aryl), -O-(5-10-membered heteroaryl), - N(C1-C6 alkyl)₂, -NH(C3-C8 cycloalkyl), -NH(3-8-membered heterocycloalkyl), -NH-(6-10 membered aryl), -NH-(5-10 membered heteroaryl),C1-C6 alkyl, C2-C6 alkenyl, C2-C6 alkynyl, C3-C8 cycloalkyl, 3-8-membered heterocycloalkyl, 6-10 membered aryl or 5-10 membered heteroaryl; and the alkyl, cycloalkyl, heterocycloalkyl, aryl and heteroaryl are optionally substituted by one or more groups independently selected from hydroxyl, amino, halogen, cyano or -O-(C1-C6 alkyl);
R_{b} is hydrogen, deuterium, -O-(C1-C6 alkyl), -O-(C3-C8 cycloalkyl), -O-(3-8-membered heterocycloalkyl), -O-(6-10-membered aryl), -O-(5-10-membered heteroaryl), - N(C1-C6 alkyl)₂, -NH(C3-C8 cycloalkyl), -NH(3-8-membered heterocycloalkyl), -NH-(6-10 membered aryl), -NH-(5-10 membered heteroaryl), C1-C6 alkyl, C2-C6 alkenyl, C2-C6 alkynyl, C3-C8 cycloalkyl, 3-8-membered heterocycloalkyl, 6-10 membered aryl or 5-10 membered heteroaryl; and the alkyl, cycloalkyl, heterocycloalkyl, aryl, and heteroaryl are optionally substituted by one or more groups independently selected from hydroxyl, amino, halogen, or cyano;
Rₐ is hydrogen, deuterium, 3-8-membered heterocycloalkyl, C3-C8 cycloalkyl, 6-10 membered aryl, 5-10 membered heteroaryl, C1-C6 alkyl, 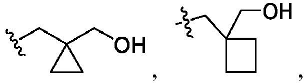 or 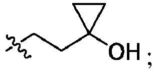 the alkyl is optionally substituted by one or more substituents selected from halogen, hydroxyl, C1-C6 alkoxy, halogenated C1-C6 alkoxy, S(O)₁₋₂(C1-C6 alkyl), S(O)₁₋₂(C3-C6 cycloalkyl), unsubstituted or mono- or polyhalogen-substituted C3-C6 cycloalkyl, unsubstituted or mono- or di-methyl substituted monocyclic saturated heterocycloalkyl having 4-6 ring atoms and containing heteroatoms or heterogroups selected from O, S, SO or SO₂; and the cycloalkyl, heterocycloalkyl, aryl, and heteroaryl are optionally substituted by one or more groups selected from hydroxyl, halogen, amino, cyano, C1-C6 alkyl, -O(C1-C6 alkyl), halogenated C1-C6 alkyl, hydroxyl C1-C6 alkyl, or amino C1-C6 alkyl.

11. The compound of formula I according to claim 10, and/or the stereoisomer, the enantiomer, the diastereomer, the deuterate, the hydrate, the solvate, the prodrug and/or the pharmaceutically acceptable salt thereof, wherein PTM is 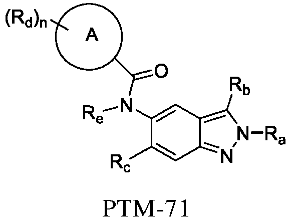 wherein, in PTM-71:
ring A is phenyl or pyridyl;
R_{d} is each independently hydrogen, deuterium, halogen, cyano, C1-C6 alkyl, or C3-C6 cycloalkyl; and the alkyl and cycloalkyl are optionally substituted by one or more groups selected from halogen, hydroxyl or amino;
n is 1 or 2;
Rₑ is hydrogen;
R_{c} is hydrogen, deuterium, -O(C1-C6 alkyl), -N(C1-C6 alkyl)₂,C1-C6 alkyl, -O(C3-C6 cycloalkyl), -N(C3-C6 cycloalkyl)₂, -O(3-6-membered heterocycloalkyl), -N (3-6-membered heterocycloalkyl)₂; and the alkyl, cycloalkyl and heterocycloalkyl are optionally substituted by one or more groups independently selected from hydroxyl, amino, halogen, cyano or -O-(C1-C6 alkyl);
R_{b} is hydrogen or C1-C6 alkyl, and the alkyl is optionally substituted by one or more groups independently selected from hydroxyl, amino, halogen or cyano;
Rₐ is hydrogen, deuterium, 3-8-membered heterocycloalkyl, C3-C8 cycloalkyl, C1-C6 alkyl, 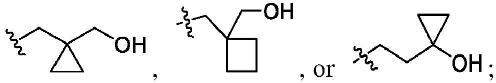 the alkyl is optionally substituted by one or more substituents selected from halogen, hydroxyl, C1-C6 alkoxy, halogenated C1-C6 alkoxy, S(O)₁₋₂(C1-C6 alkyl), S(O)₁₋₂(C3-C6 cycloalkyl), unsubstituted or mono- or polyhalogen-substituted C3-C6 cycloalkyl, unsubstituted or mono- or di-methyl substituted monocyclic saturated heterocycloalkyl having 4-6 ring atoms and containing heteroatoms or heterogroups selected from O, S, SO or SO₂; the cycloalkyl and the heterocycloalkyl are optionally substituted by one or more groups selected from hydroxyl, halogen, amino, cyano, C1-C6 alkyl, -O(C1-C6 alkyl), halogenated C1-C6 alkyl, hydroxyl C1-C6 alkyl, or amino C1-C6 alkyl.

12. The compound of formula I according to claim 11, and/or the stereoisomer, the enantiomer, the diastereomer, the deuterate, the hydrate, the solvate, the prodrug and/or the pharmaceutically acceptable salt thereof, wherein R_{d} in PTM-71 is each independently hydrogen, deuterium, halogen or C1-C6 alkyl; and the alkyl is optionally substituted by one or more groups selected from halogen or hydroxyl; preferably R_{d} is hydrogen, deuterium, F, methyl, difluoromethyl, trifluoromethyl or 2-hydroxypropyl.

13. The compound of formula I according to claim 11, and/or the stereoisomer, the enantiomer, the diastereomer, the deuterate, the hydrate, the solvate, the prodrug and/or the pharmaceutically acceptable salt thereof, wherein R_{c} in PTM-71 is C1-C6 alkyl, and the alkyl is optionally substituted by one or more groups independently selected from hydroxyl and halogen; preferably R_{c} is difluoromethyl or 2-hydroxypropyl.

14. The compound of formula I according to claim 11, and/or the stereoisomer, the enantiomer, the diastereomer, the deuterate, the hydrate, the solvate, the prodrug and/or the pharmaceutically acceptable salt thereof, wherein R_{c} in PTM-71 is -O(C1-C6 alkyl), and the alkyl is optionally substituted by one or more groups independently selected from hydroxyl, halogen or -OCH₃; preferably R_{c} is methoxy, ethoxy, isopropoxy, -OCH₂CH₂OCH₃, difluoromethoxy or trifluoromethoxy.

15. The compound of formula I according to claim 11, and/or the stereoisomer, the enantiomer, the diastereomer, the deuterate, the hydrate, the solvate, the prodrug and/or the pharmaceutically acceptable salt thereof, wherein R_{c} in PTM-71 is -O-(3-6-membered heterocycloalkyl containing one or two heteroatoms selected from N or O); preferably

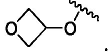

16. The compound of formula I according to claim 11, and/or the stereoisomer, the enantiomer, the diastereomer, the deuterate, the hydrate, the solvate, the prodrug and/or the pharmaceutically acceptable salt thereof, wherein R_{b} in PTM-71 is hydrogen or methyl.

17. The compound of formula I according to claim 11, and/or the stereoisomer, the enantiomer, the diastereomer, the deuterate, the hydrate, the solvate, the prodrug and/or the pharmaceutically acceptable salt thereof, wherein Rₐ in PTM-71 is 3-12 membered heterocycloalkyl containing one or two heteroatoms selected from N, O or S, and the heterocycloalkyl is optionally substituted by one or more groups selected from hydroxyl, halogen, amino, cyano, C1-C6 alkyl, -O(C1-C6 alkyl), halogenated C1-C6 alkyl, hydroxyl C1-C6 alkyl, or amino C1-C6 alkyl; preferably Rₐ is 3-12-membered heterocycloalkyl containing one or two heteroatoms selected from N or O, and the heterocycloalkyl is optionally substituted by one or more groups selected from hydroxyl, halogen, amino, cyano, C1-C6 alkyl, -O(C1-C6 alkyl), halogenated C1-C6 alkyl, hydroxyl C1-C6 alkyl, or amino C1-C6 alkyl; more preferably Rₐ is piperidinyl or piperazinyl, the piperidinyl and the piperazinyl are optionally substituted by one or more groups selected from hydroxyl, halogen, amino, cyano, C1-C6 alkyl, -O(C1-C6 alkyl), halogenated C1-C6 alkyl, hydroxyl C1-C6 alkyl, or amino C1-C6 alkyl; most preferably is piperidinyl which is optionally substituted by one or more groups selected from hydroxyl, amino, methyl, methoxy, hydroxymethyl, or trifluoromethyl.

18. The compound of formula I according to claim 11, and/or the stereoisomer, the enantiomer, the diastereomer, the deuterate, the hydrate, the solvate, the prodrug and/or the pharmaceutically acceptable salt thereof, wherein Rₐ in PTM-71 is cyclohexane, and the cyclohexane is optionally substituted by one or more groups selected from hydroxyl, halogen, amino, cyano, C1-C6 alkyl, -O(C1-C6 alkyl), halogenated C1-C6 alkyl, hydroxyl C1-C6 alkyl, or amino C1-C6 alkyl, preferably optionally substituted by one or more groups selected from hydroxyl, amino, methyl, methoxy, hydroxymethyl, or trifluoromethyl.

19. The compound of formula I according to claim 1, and/or the stereoisomer, the enantiomer, the diastereomer, the deuterate, the hydrate, the solvate, the prodrug and/or the pharmaceutically acceptable salt thereof, wherein PTM is

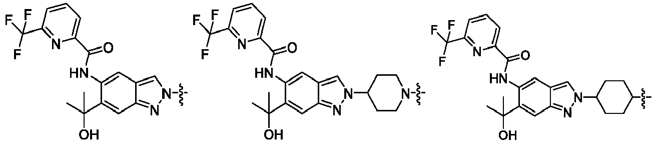

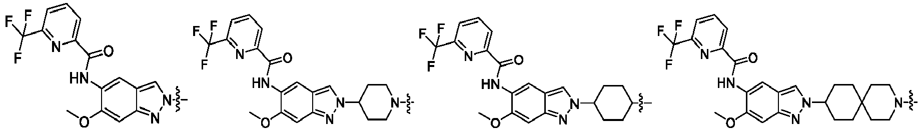

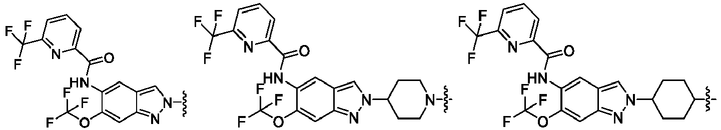

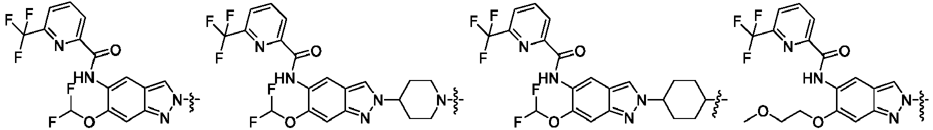

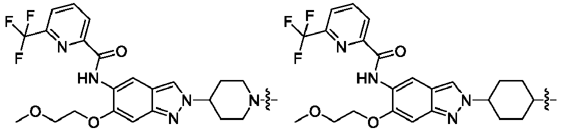

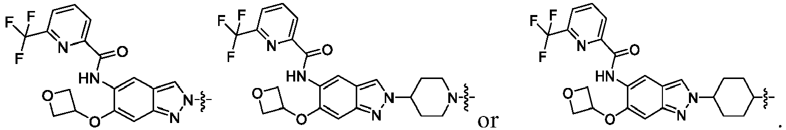

20. The compound of formula I according to claim 1, and/or the stereoisomer, the enantiomer, the diastereomer, the deuterate, the hydrate, the solvate, the prodrug and/or the pharmaceutically acceptable salt thereof, wherein L is a bond.

21. The compound of formula I according to claim 1, and/or the stereoisomer, the enantiomer, the diastereomer, the deuterate, the hydrate, the solvate, the prodrug and/or the pharmaceutically acceptable salt thereof, wherein L is -(CH₂)ⱼ-, and one or more methylenes in -(CH₂)ⱼ- are optionally replaced by a group selected from -NR^{3'}-, -O-, -S-, -S(O)-, - S(O)NR^{3'}-, -NR^{3'}S(O)-, -S(O)₂-, -S(O)₂NR^{3'}-, -NR^{3 '}S(O)₂-, -NR^{4'}S(O)₂NR^{3'}-, -CR^{1'}R^{2'}-, - C(O)-, -C(O)O-, -OC(O)-, -NR^{3'}C(O)O-, -OC(O)NR^{3'}-, -C(O)NR^{3'}-, -NR^{3 '}C(O)-, - NR^{4'}C(O)NR^{3'}-, -P(O)-, -P(O)O-, -OP(O)-, -OP(O)O-, vinylidene, ethynylene,C3-C12 cycloalkylene, 3-12 membered heterocycloalkylene containing one or more heteroatoms selected from N, O or S, 6-10 membered arylene or 5-10 membered heteroarylene; and the vinylidene, cycloalkylene, heterocycloalkylene, arylene, and heteroarylene are each independently optionally substituted by one or more substituents selected from halogen, - OR^{3'}, -NR^{3'}R^{4'}, oxo, nitro, cyano, C1-C6 alkyl, -S(C1-C6 alkyl), C3-C10 cycloalkyl, 3-10 membered heterocycloalkyl, 6-10 membered aryl, 5-10 membered heteroaryl, -C(O)R^{1'}, - C(O)OR^{3'}, -OC(O)R^{1'}, -C(O)NR^{3'}, -NR^{3 '}C(O)R^{1'}, -S(O)R^{1'}, -S(O)NR^{3'}, -S(O))₂R^{1'}, - S(O)₂NR^{3'}, -NR^{3 '}S(O)₂R^{1'}, -NR^{4'}S(O)₂NR^{3'}, -OC(O)NR^{3'}, -NR^{4'}C(O)NR^{3'}, and the alkyl, cycloalkyl, heterocycloalkyl, aryl, and heteroaryl are each independently optionally substituted by one or more substituents selected from halogen, -OH, -NR³R^{4'}, oxo, nitro, cyano, C1-C6 alkyl, C3-C10 cycloalkyl, 3-10 membered heterocycloalkyl, 6-10 membered aryl, or 5-10 membered heteroaryl; R^{1'} and R^{2'} are each independently halogen, -OH, - NR^{3'}R^{4'}, C1-C6 alkyl, chlorinated C1-C6 alkyl, hydroxyl C1-C6 alkyl, -O(C1-C6 alkyl), - NH(C1-C6 alkyl), -NH(C1-C6 alkyl), C3-C10 cycloalkyl, -O(C3-C10 cycloalkyl), -NH(C3-C10 cycloalkyl), 3-10 membered heterocycloalkyl, -0(3-10 membered heterocycloalkyl), - NH(3-10 membered heterocycloalkyl), 6-10 membered aryl, -0(6-10 membered aryl), - NH(6-10 membered aryl), 5-10 membered heteroaryl, -0(5-10 membered heteroaryl), or - NH(5-10 membered heteroaryl); R^{3'} and R^{4'} are each independently hydrogen, deuterium, C1-C6 alkyl, C3-C10 cycloalkyl, 3-10 membered heterocycloalkyl, 6-10 membered aryl, or 5-10 membered heteroaryl; j is 1, 2, 3, 4, 5, 6, 7, 8, 9, 10, 11, 12, 13, 14, 15, 16, 17, 18, 19, 20, 21, 22, 23, 24 or 25.

22. The compound of formula I according to claim 21, and/or the stereoisomer, the enantiomer, the diastereomer, the deuterate, the hydrate, the solvate, the prodrug and/or the pharmaceutically acceptable salt thereof, wherein L is -(CH₂)ⱼ, 1, 2, 3 or 4 methylenes in - (CH₂)ⱼ- are optionally replaced by a group selected from -NH-, -NCH₃-, -NCH₂CH₃-, -O-, - C(CH₃)₂-, -CHF-, -CHCF₃-, -C(O)-, -C(O)O-, -OC(O)-, -C(O)NH-, -C(O)NCH₃-, -NHC(O)-, -NCH₃C(O)-, vinylidene, ethynylene, cyclopropylene, cyclobutylene, cyclopentylene, cyclohexylidene, oxiranylene, oxetanylene, oxolanylene, oxanilene, azridinylene, azetidinylene, azacyclopentylene, piperidinylidene, piperazinylidene, morpholinylidene, perhomomorpholinylidene, phenylene, pyrrolylidene, thienylidene, furanylidene, imidazolylidene, pyrazolylidene, triazolylidene, tetrazolylidene, oxazolylidene, isoxazolylidene, thiazolylidene, isothiazolylidene, pyridylidene, pyrimidinylidene, pyridazinylidene, pyrazinylidene, 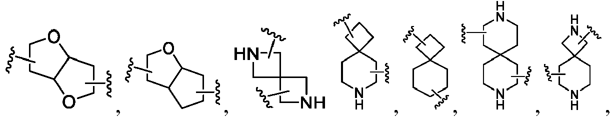 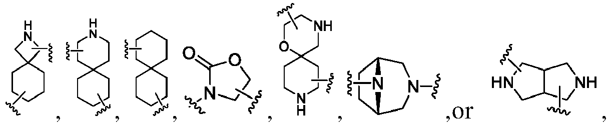 and the group is optionally substituted by one or more substituents selected from halogen, oxo, -NR^{3'} R^{4'}, -OR^{3'}, or C1-C4 alkyl, the alkyl is optionally substituted by one or more substituents selected from halogen, -OH, or -NH₂, R^{3'} and R^{4'} are each independently hydrogen, deuterium, C1-C4 alkyl, and j is 2, 3, 4, 5, 6, 7 or 8.

23. The compound of formula I according to claim 21, and/or the stereoisomer, the enantiomer, the diastereomer, the deuterate, the hydrate, the solvate, the prodrug and/or the pharmaceutically acceptable salt thereof, wherein L is -(CH₂)ⱼ-, 1, 2 or 3 methylenes in - (CH₂)ⱼ- are optionally substituted by a group selected from -O-, -NH-, -NCH₃-, -NCH₂CH₃-, - C(O)-, -C(O)NH-, -NHC(O)-, -NCH₃C(O)-, -C(O)NCH₃-, azridinylene, azetidinylene, azacyclopentylene, piperidinylidene, piperazinylidene, 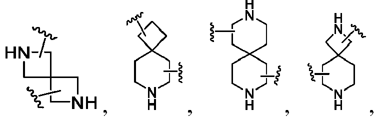 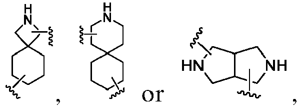 j is 2, 3, 4, 5, 6, 7, or 8.

24. The compound of formula I according to claim 21, and/or the stereoisomer, the enantiomer, the diastereomer, the deuterate, the hydrate, the solvate, the prodrug and/or the pharmaceutically acceptable salt thereof, wherein L is -(CH₂)ⱼ₋₁-C(O)-, the methylene in - (CH₂)ⱼ₋₁-C(O)- is as defined in claim 21, optionally substituted by one or more groups, j is as defined in claim 21.

25. The compound of formula I according to claim 21, and/or the stereoisomer, the enantiomer, the diastereomer, the deuterate, the hydrate, the solvate, the prodrug and/or the pharmaceutically acceptable salt thereof, wherein L is

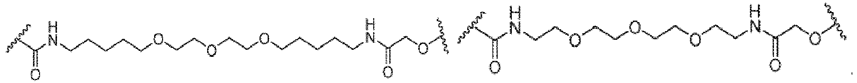

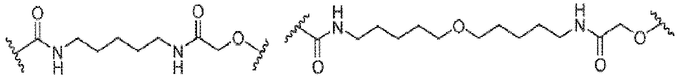

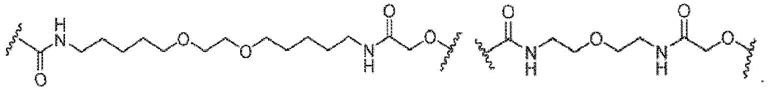

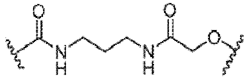

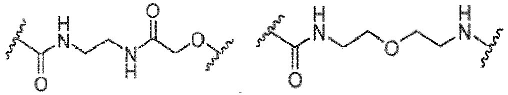

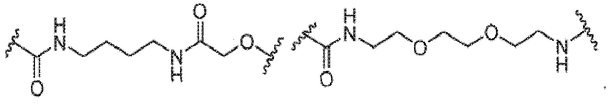

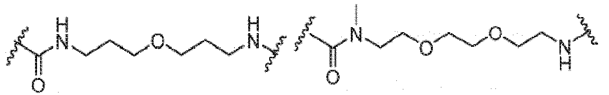

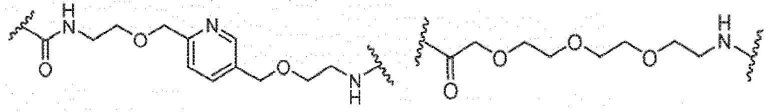

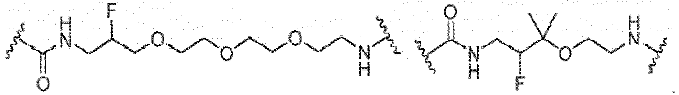

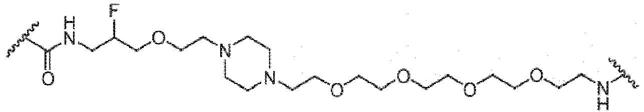

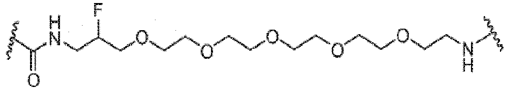

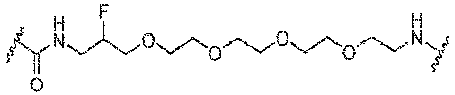

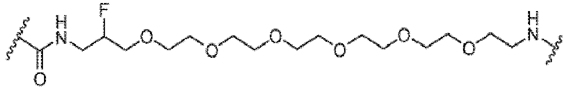

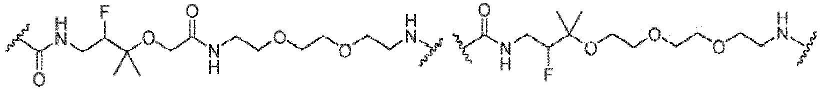

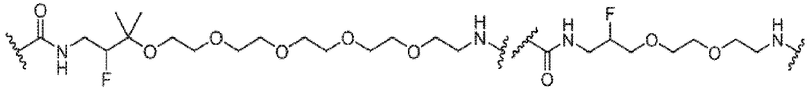

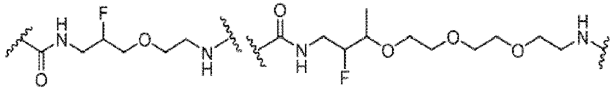

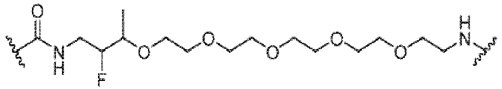

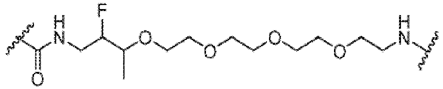

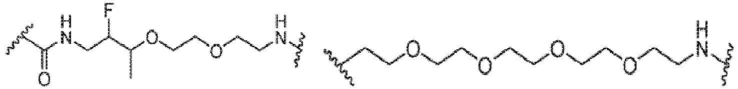

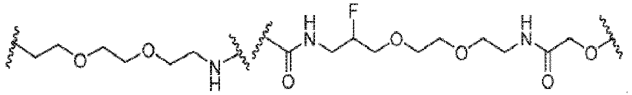

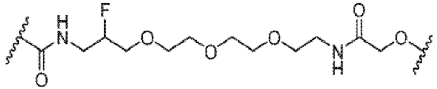

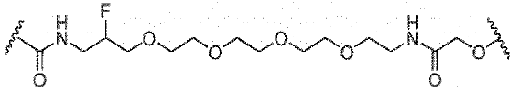

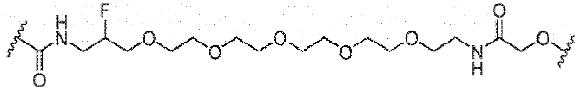

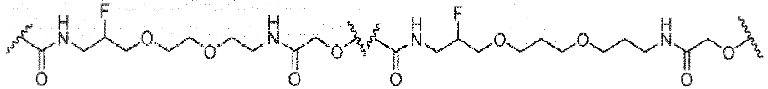

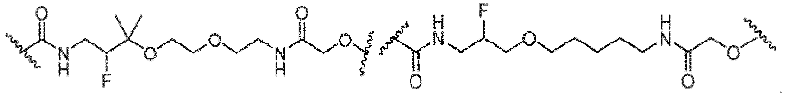

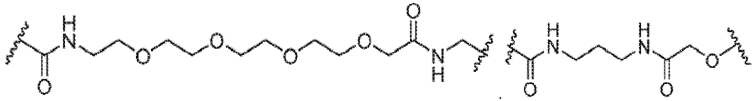

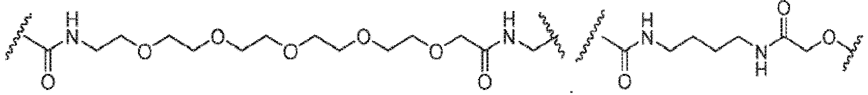

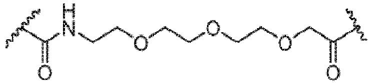

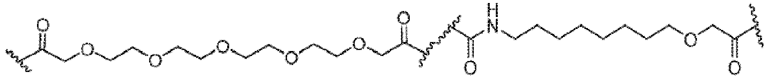

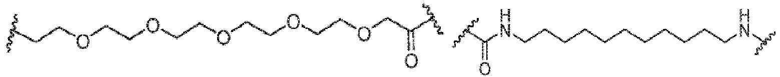

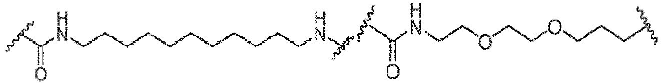

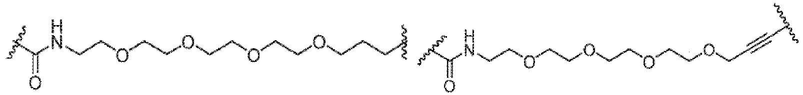

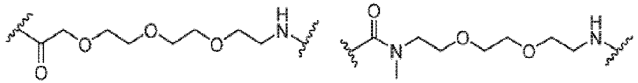

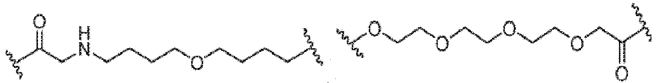

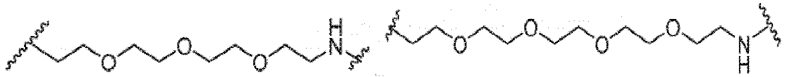

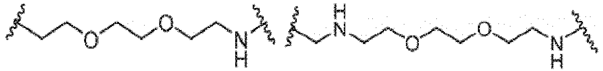

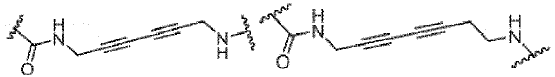

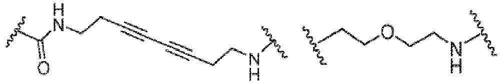

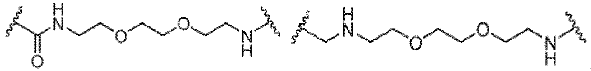

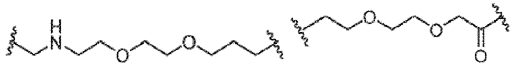

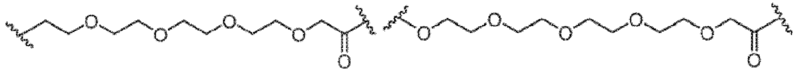

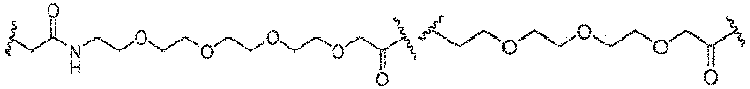

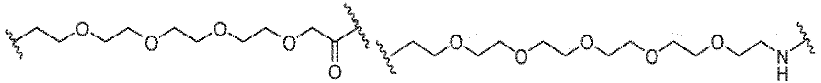

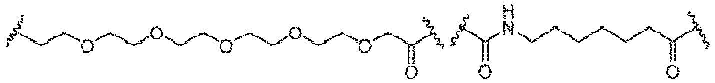

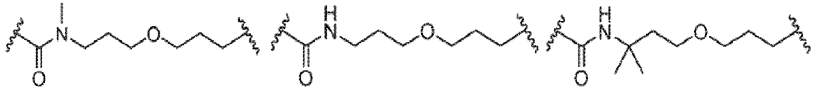

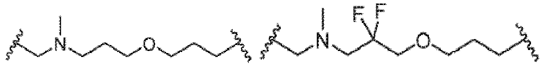

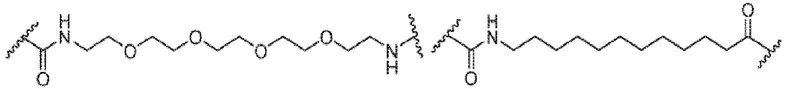

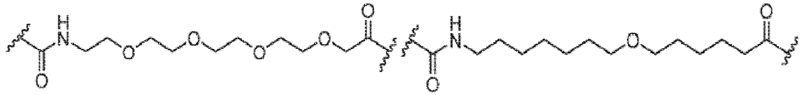

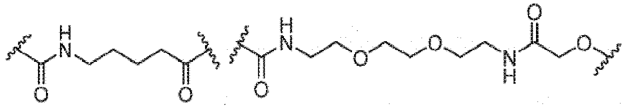

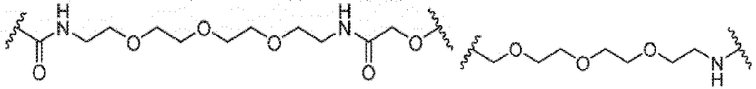

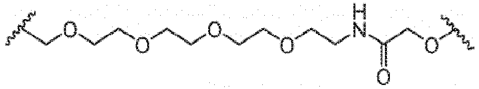

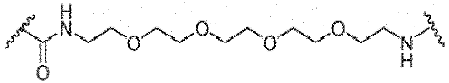

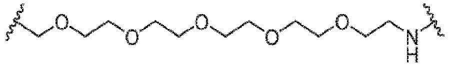

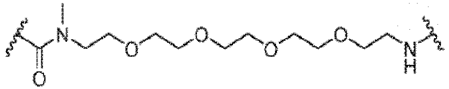

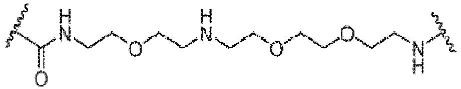

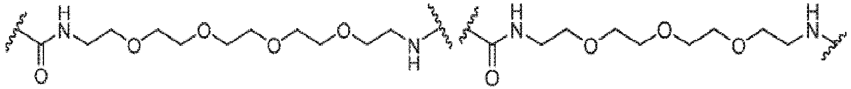

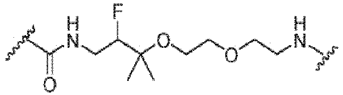

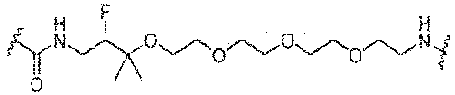

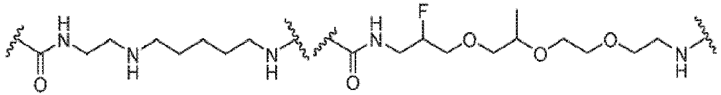

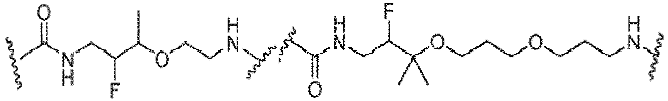

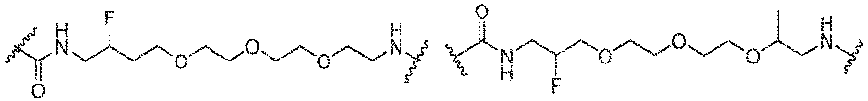

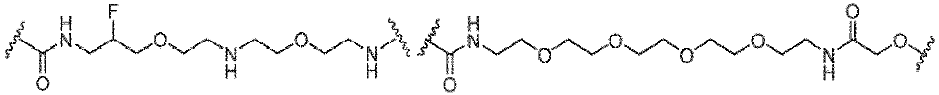

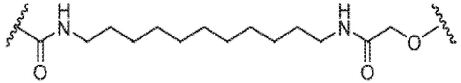

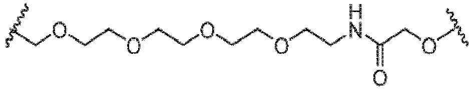

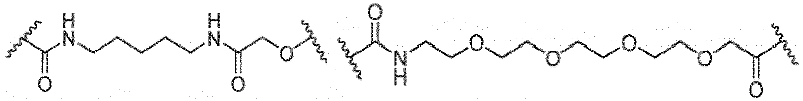

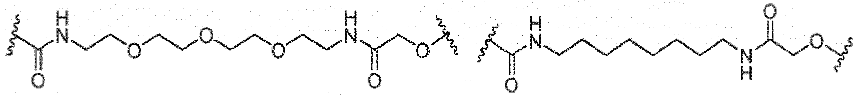

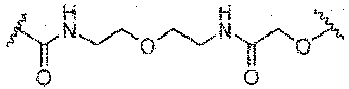

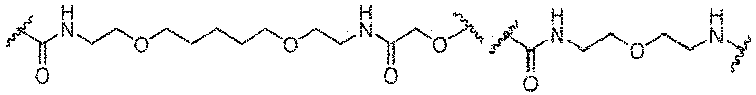

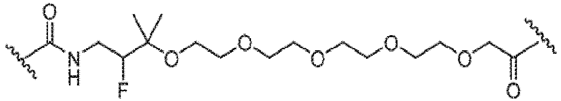

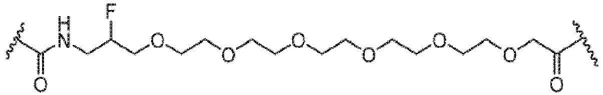

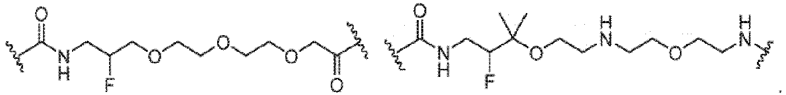

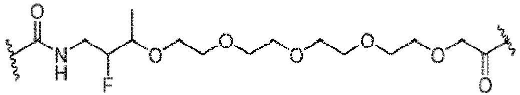

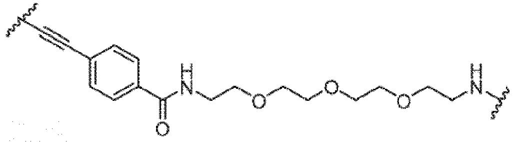

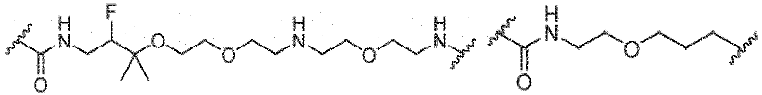

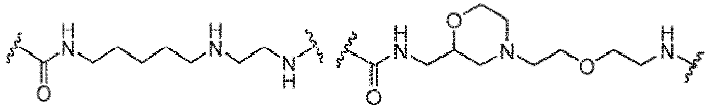

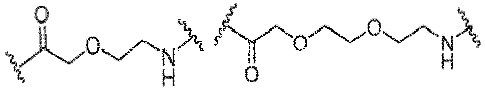

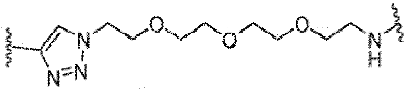

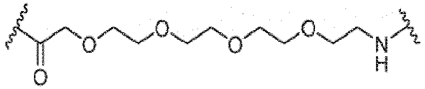

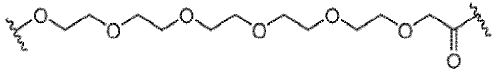

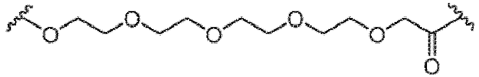

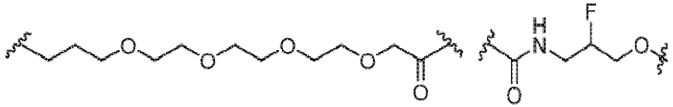

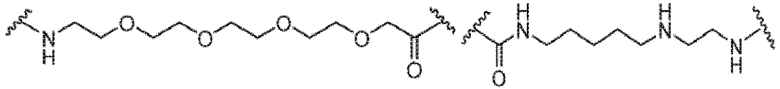

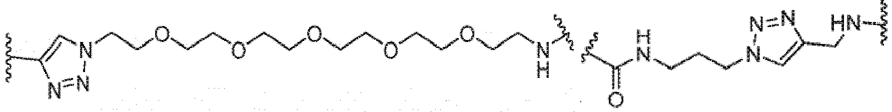

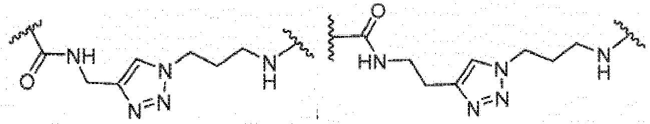

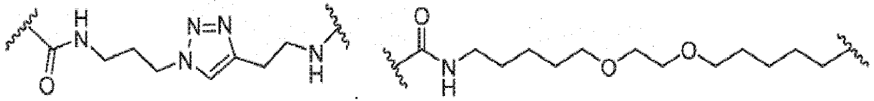

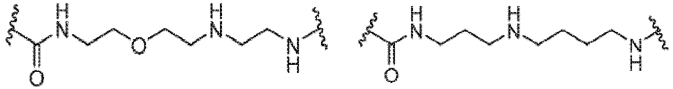

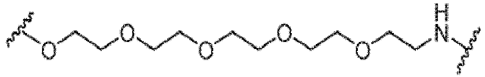

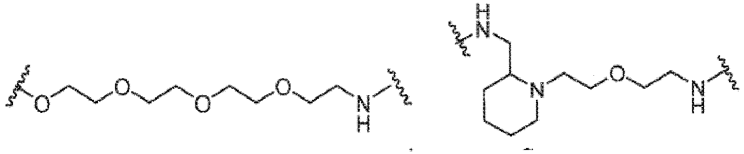

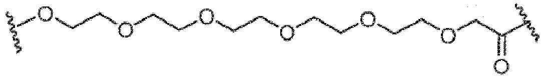

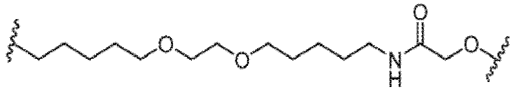

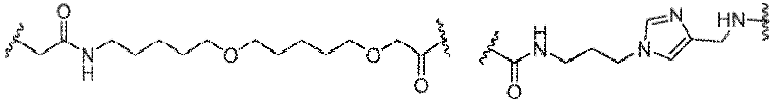

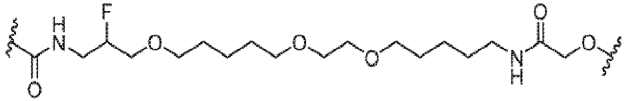

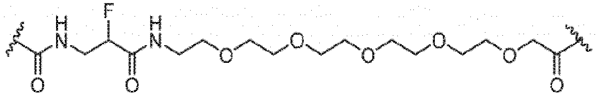

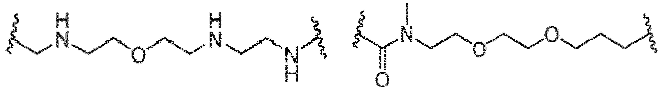

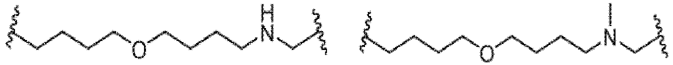

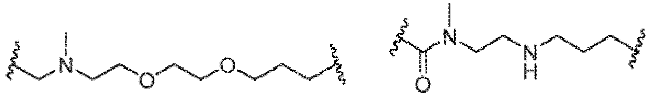

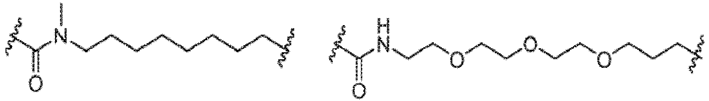

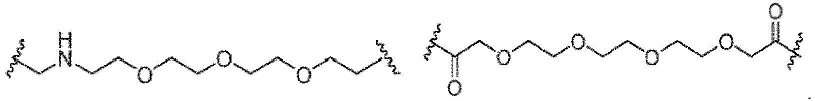

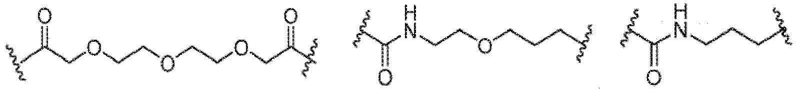

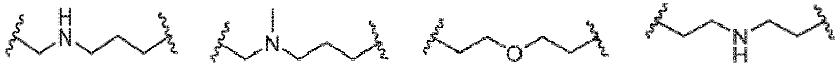

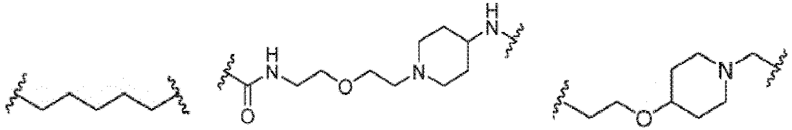

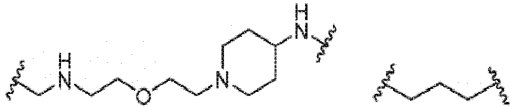

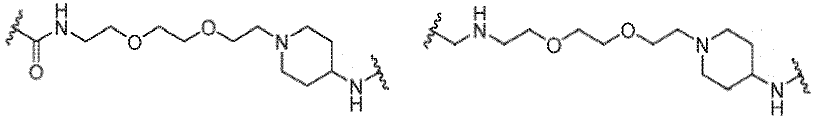

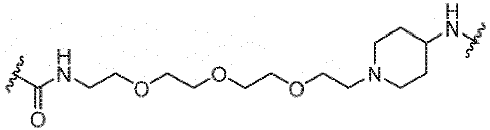

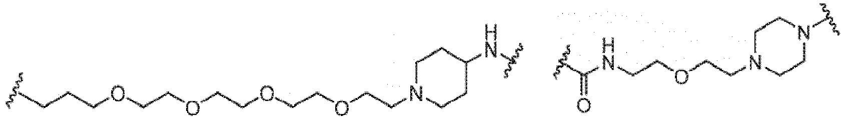

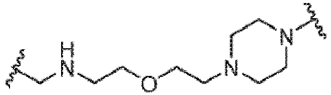

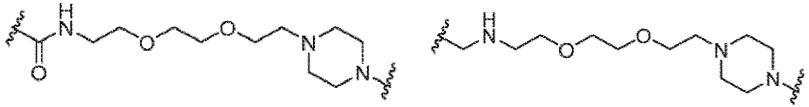

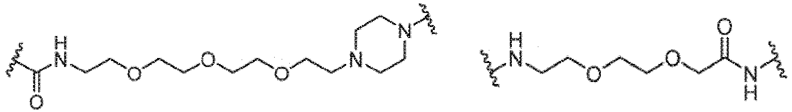

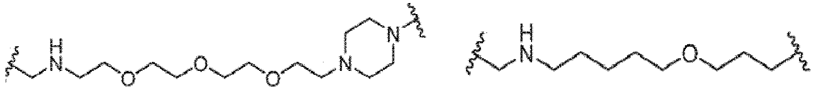

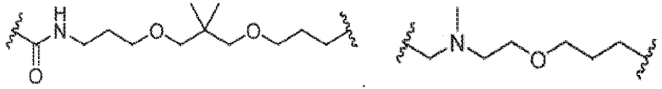

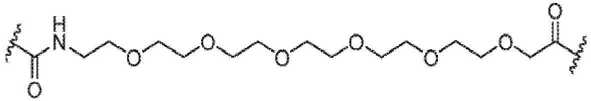

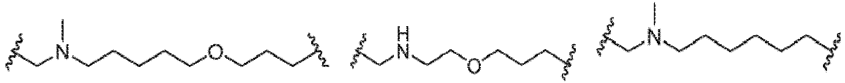

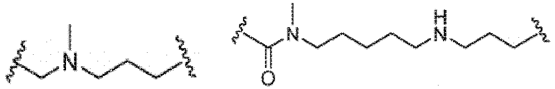

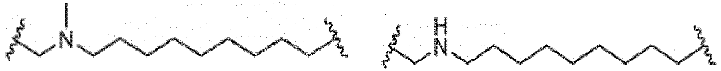

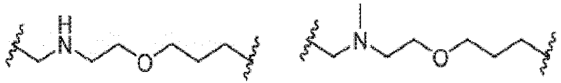

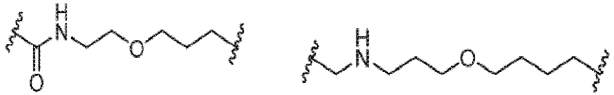

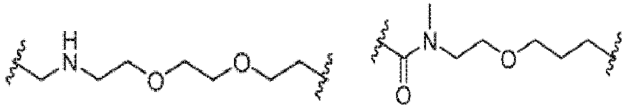

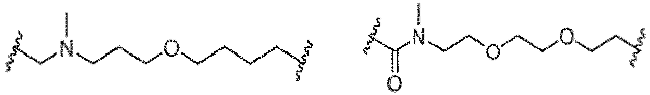

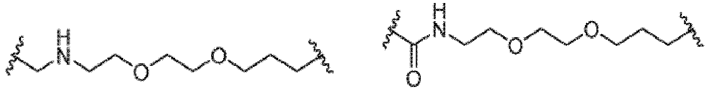

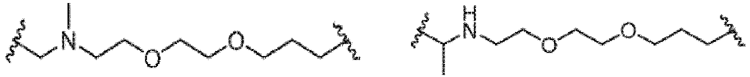

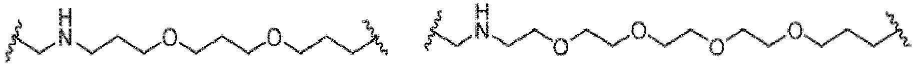

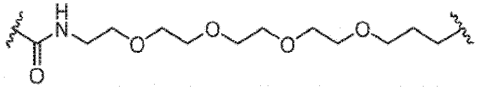

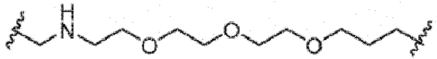

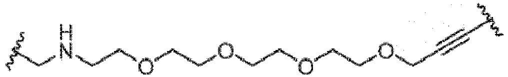

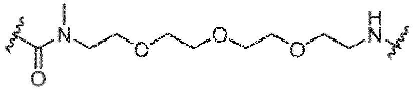

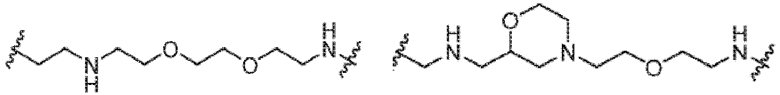

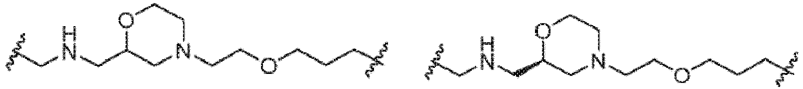

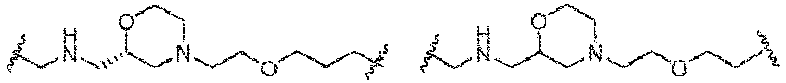

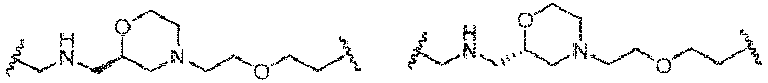

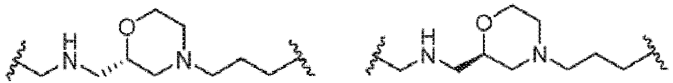

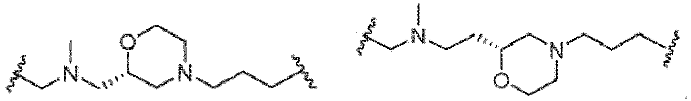

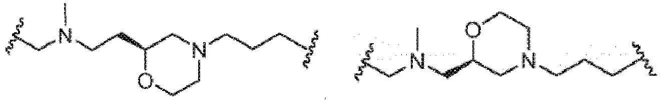

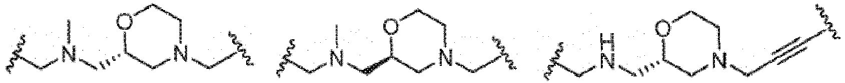

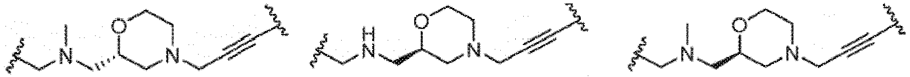

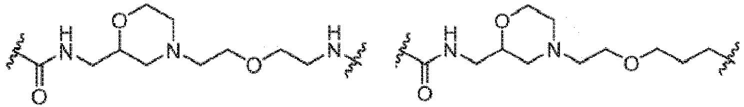

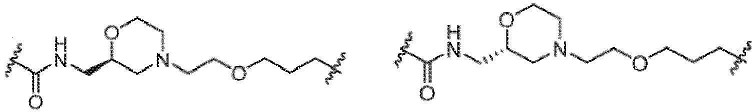

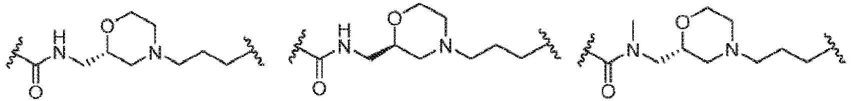

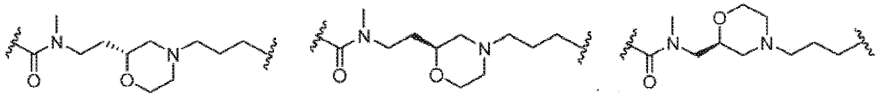

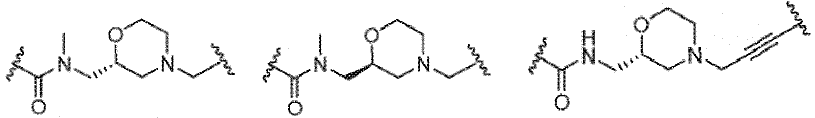

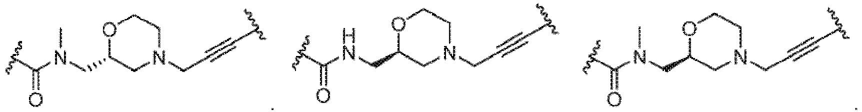

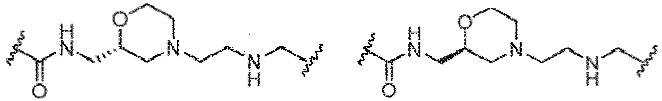

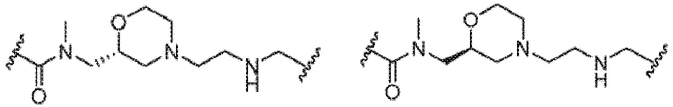

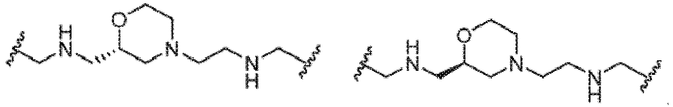

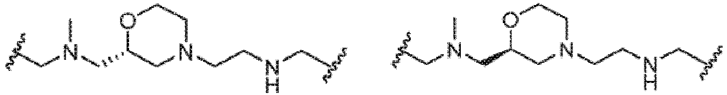

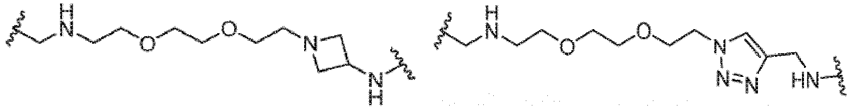

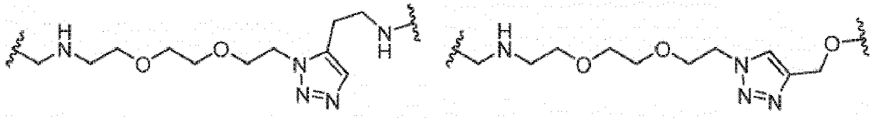

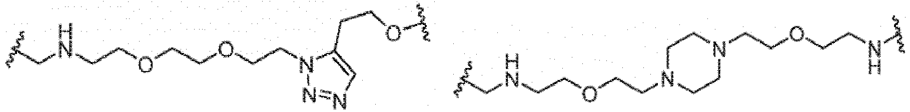

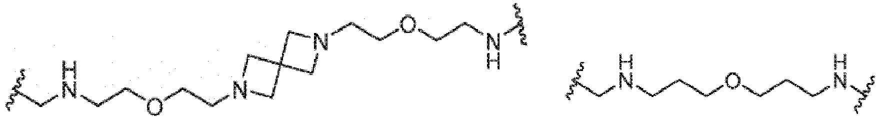

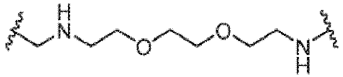

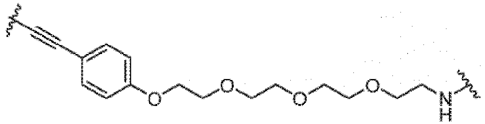

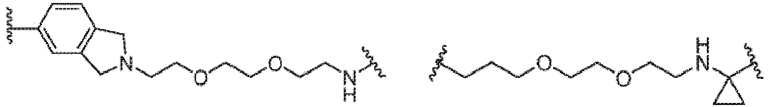

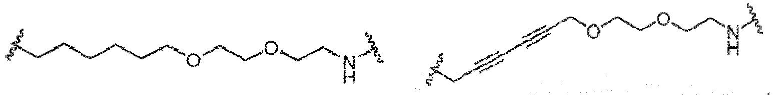

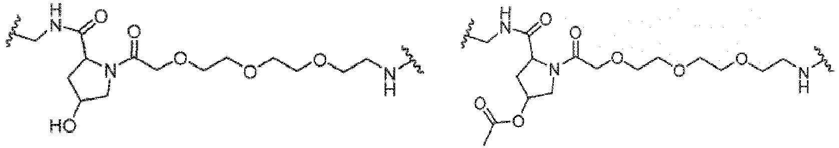

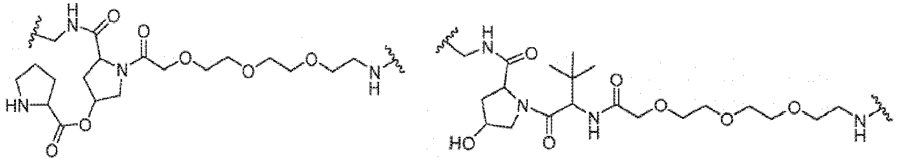

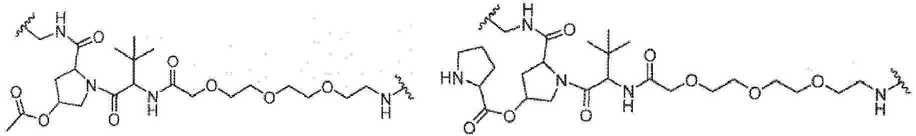

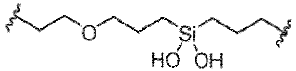

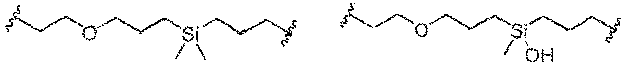

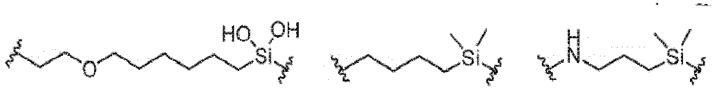

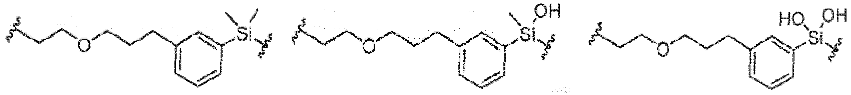

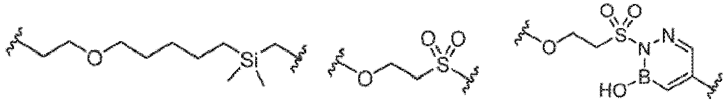

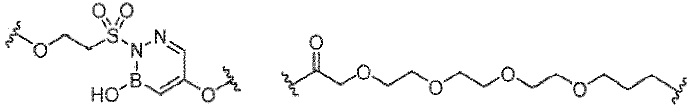

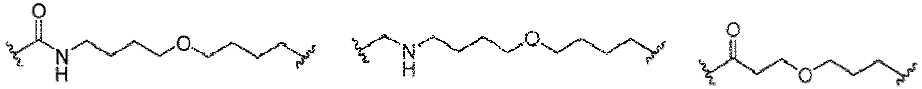

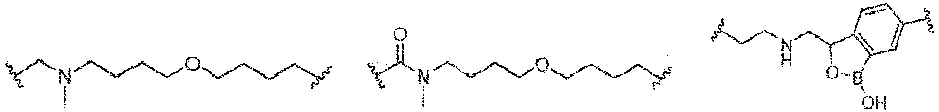

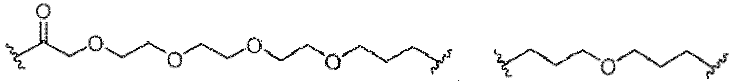

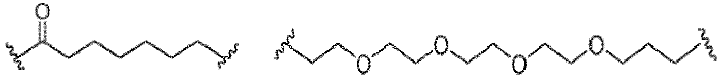

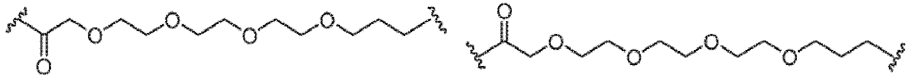

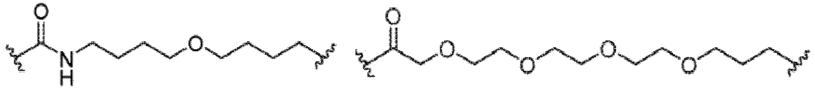

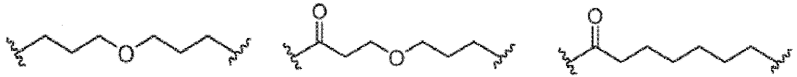

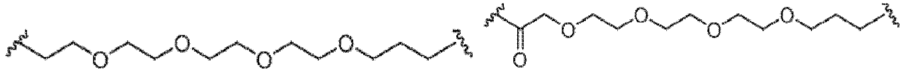

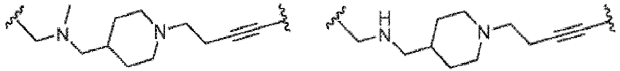

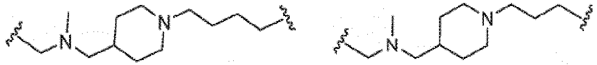

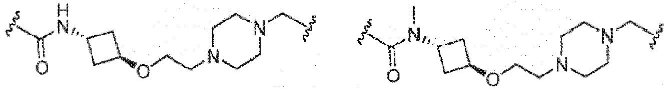

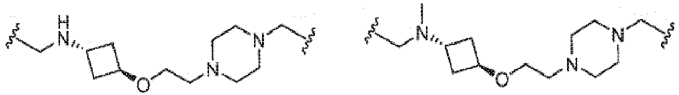

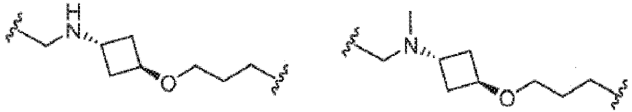

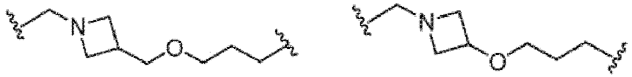

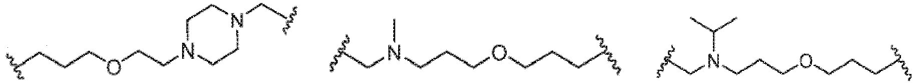

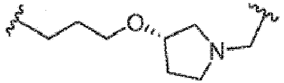

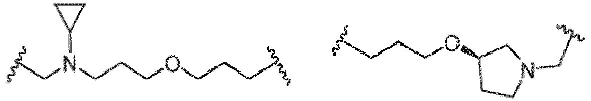

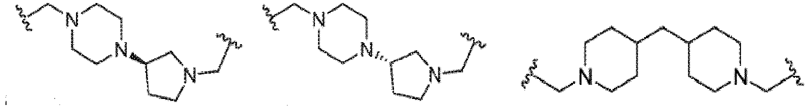

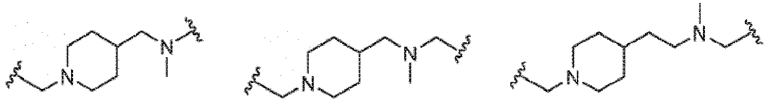

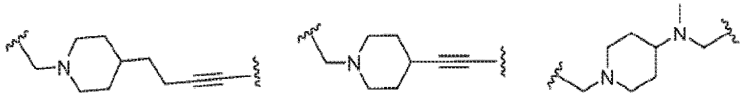

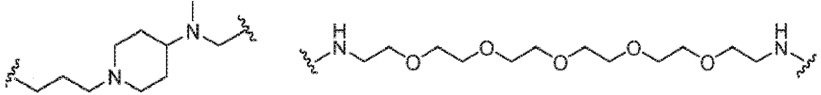

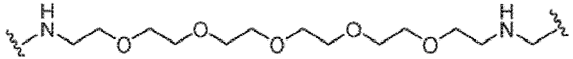

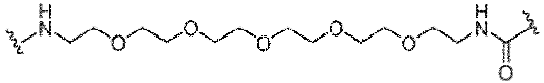

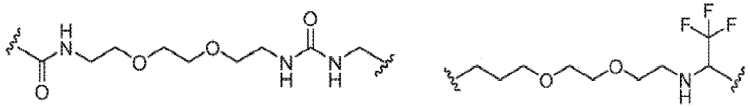

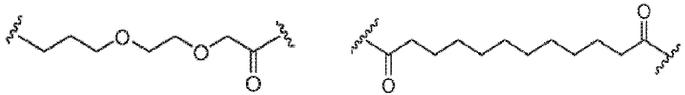

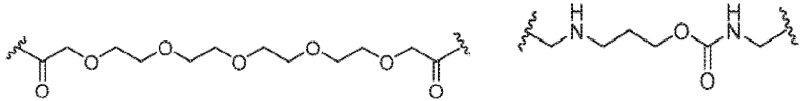

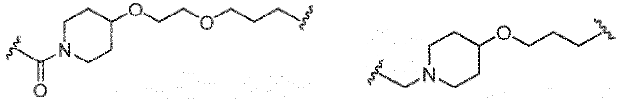

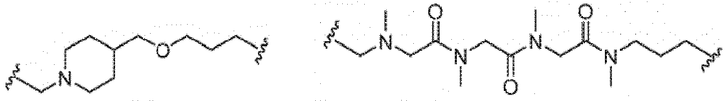

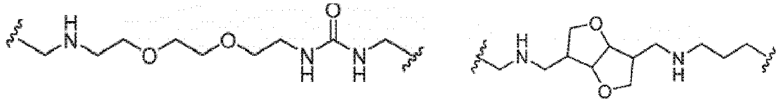

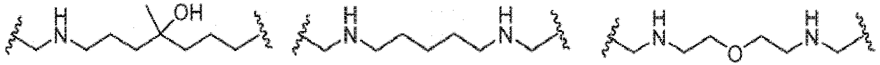

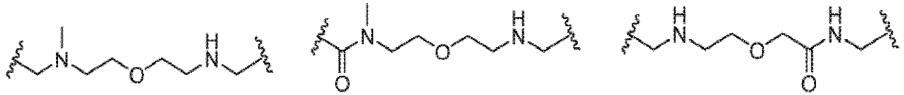

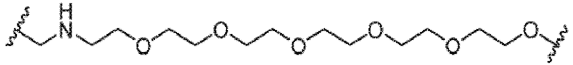

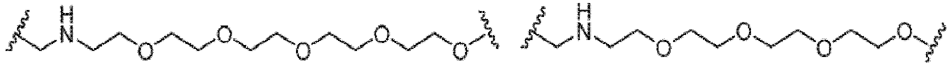

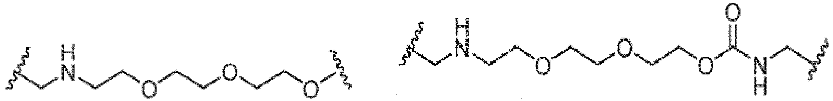

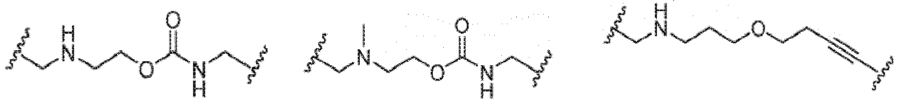

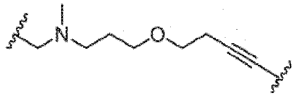

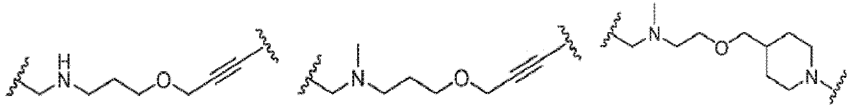

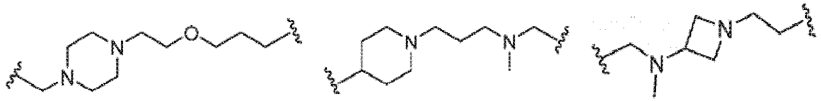

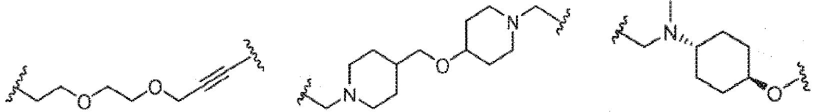

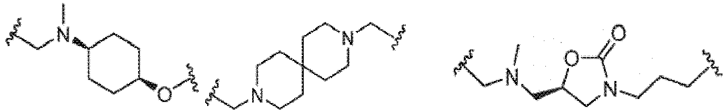

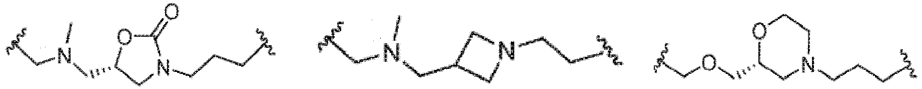

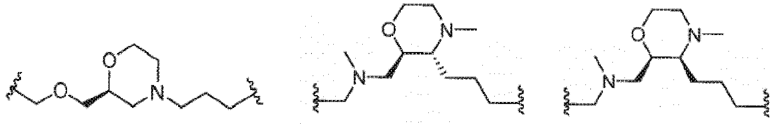

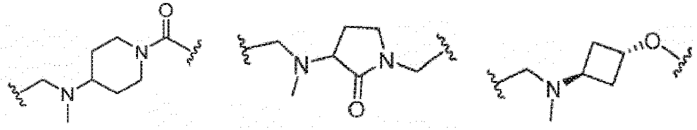

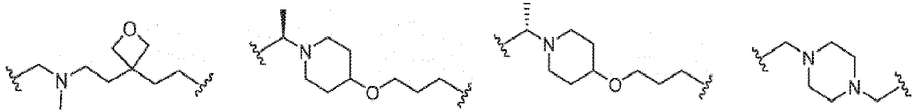

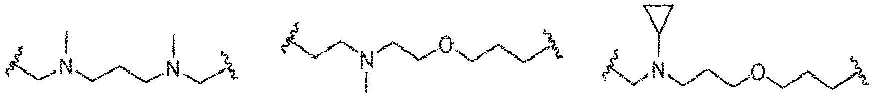

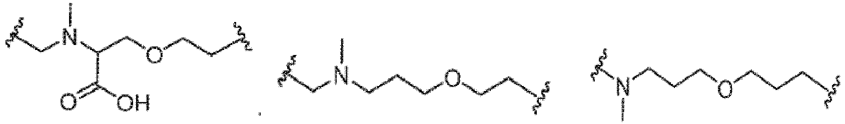

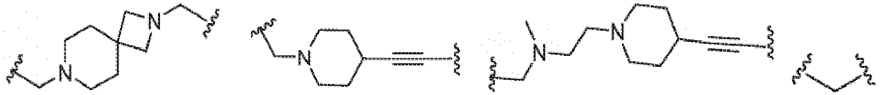

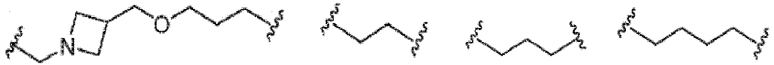

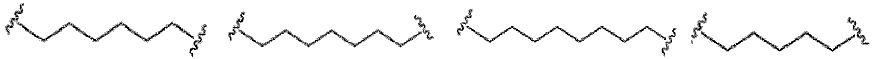

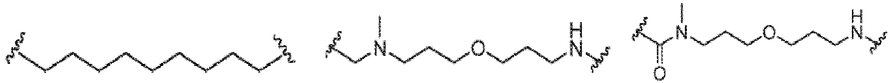

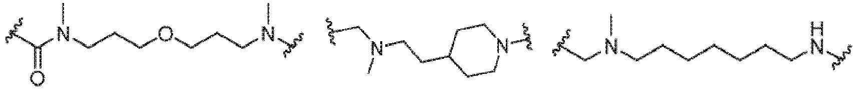

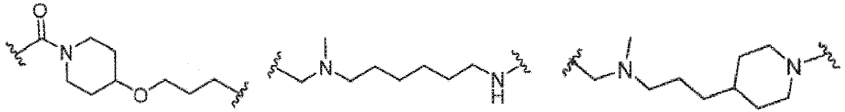

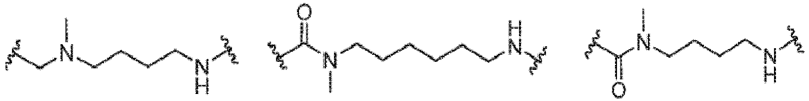

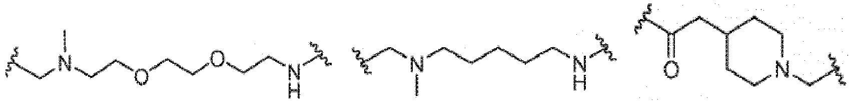

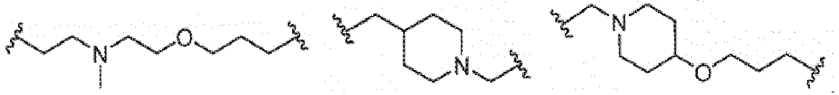

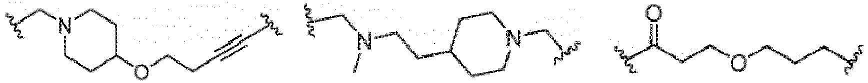

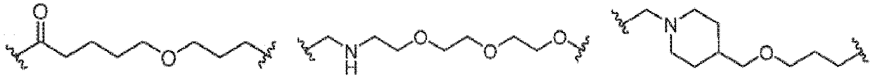

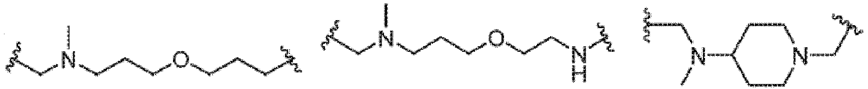

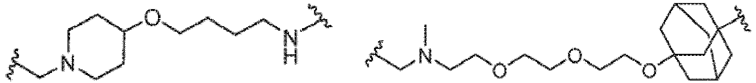

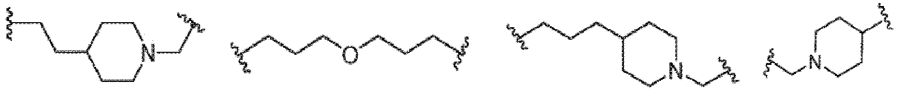

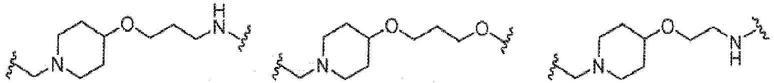

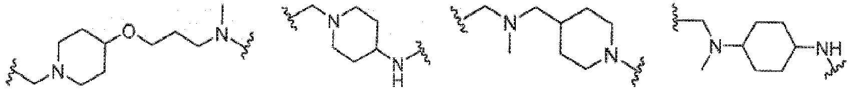

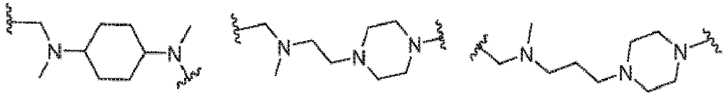

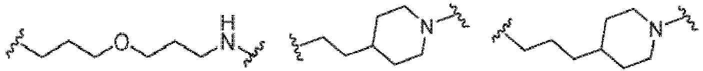

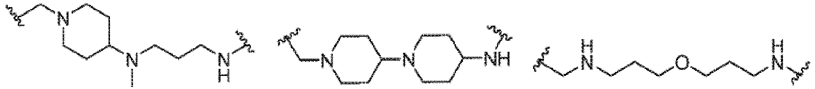

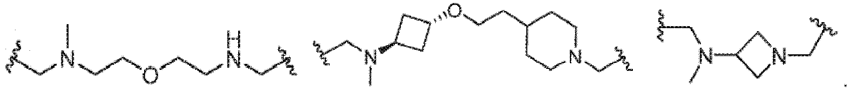

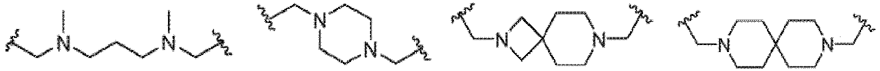

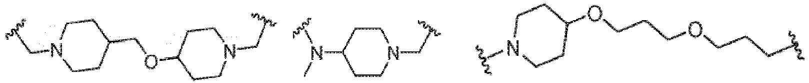

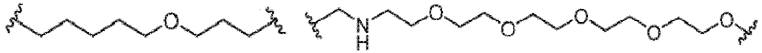

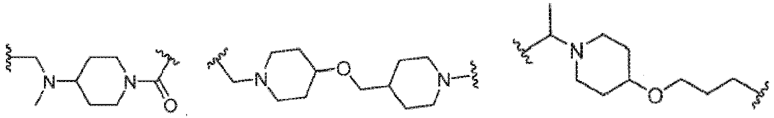

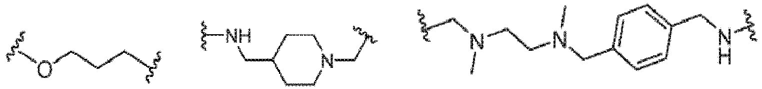

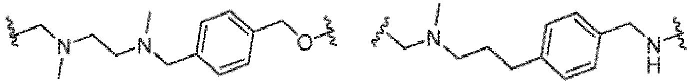

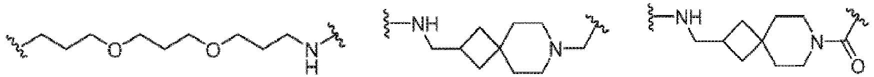

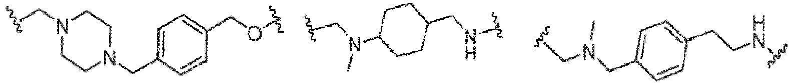

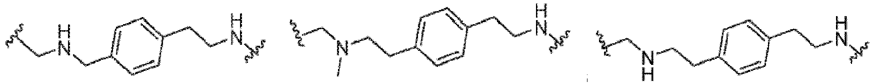

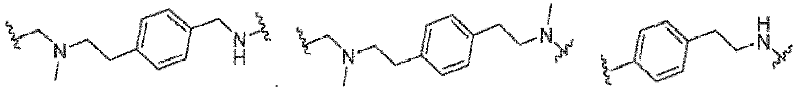

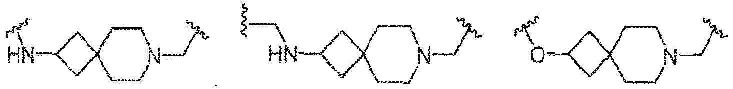

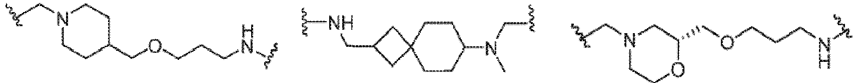

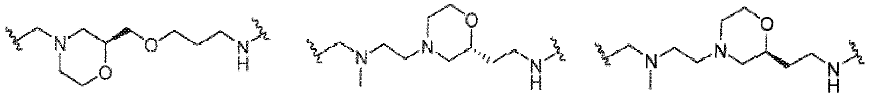

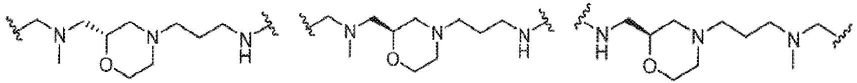

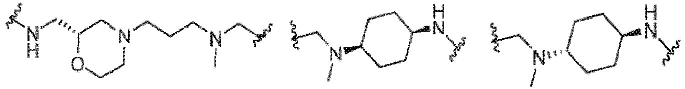

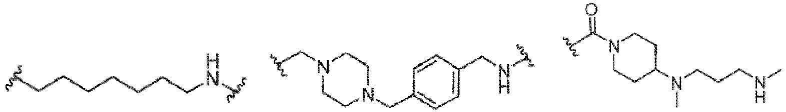

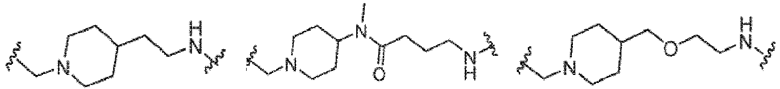

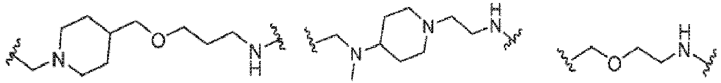

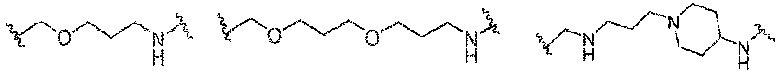

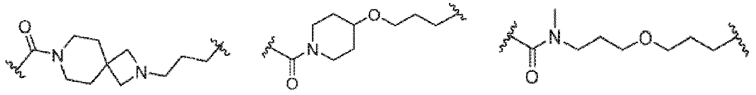

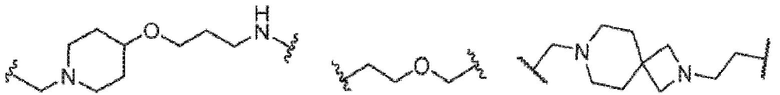

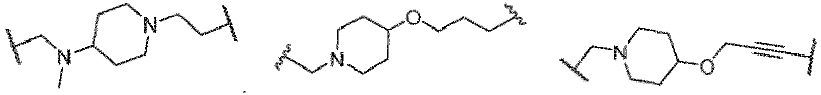

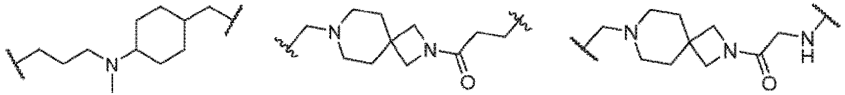

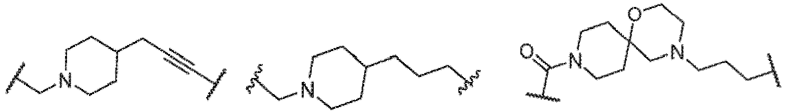

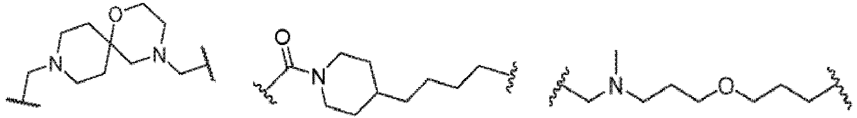

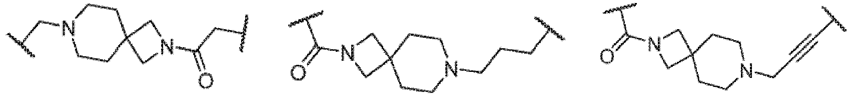

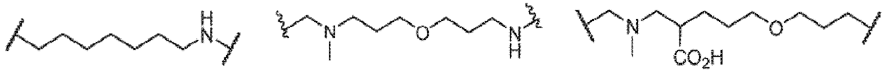

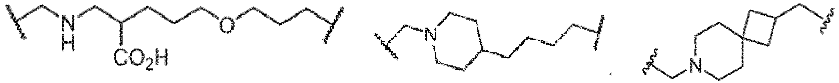

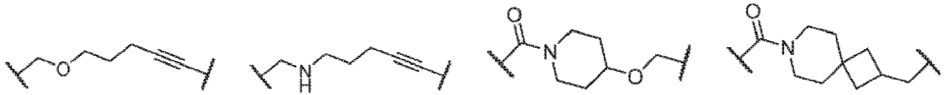

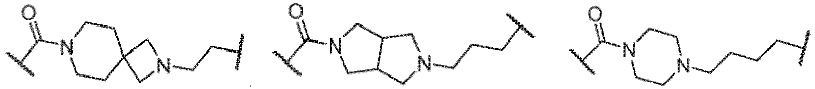

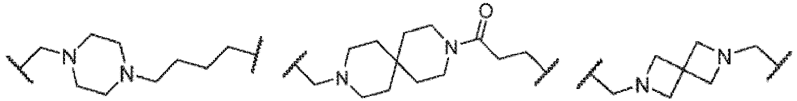

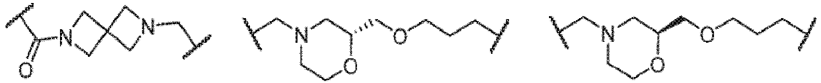

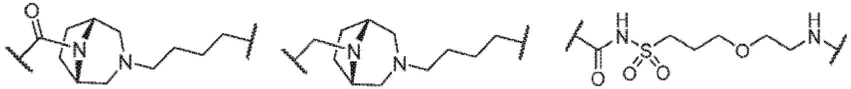

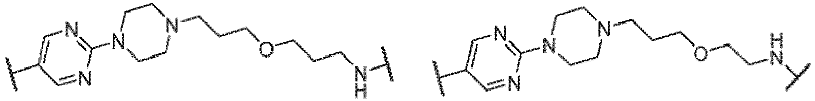

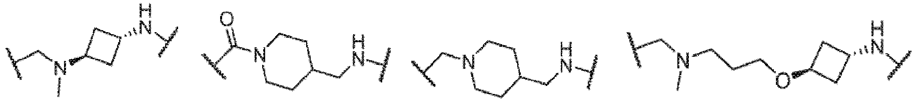

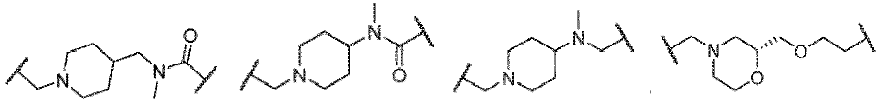

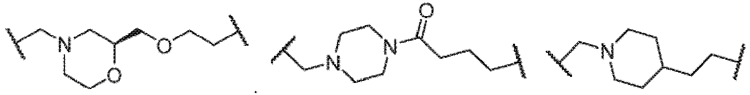

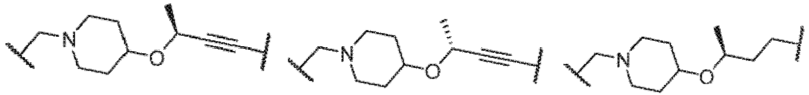

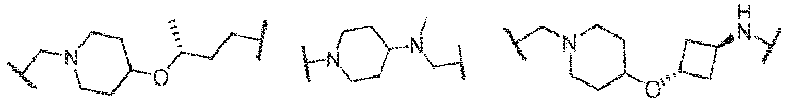

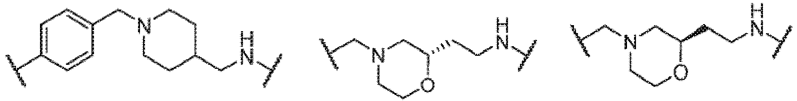

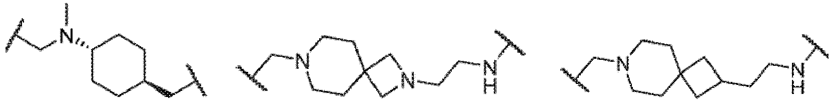

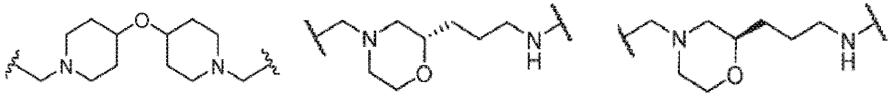

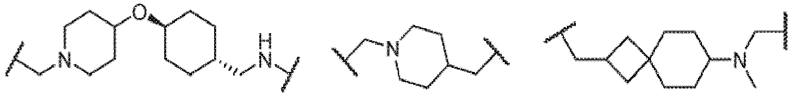

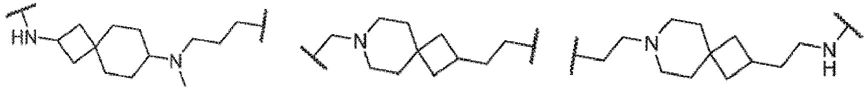

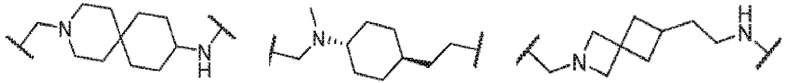

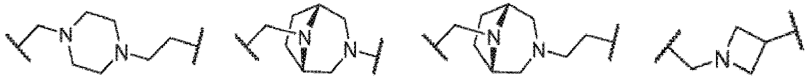

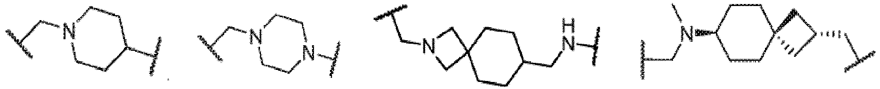

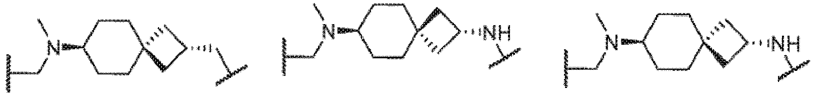

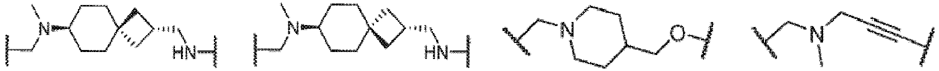

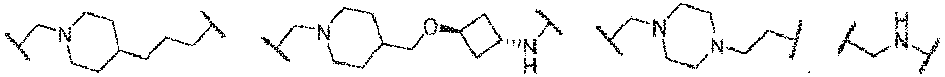

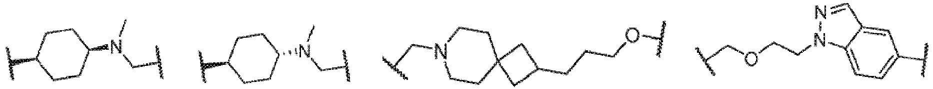

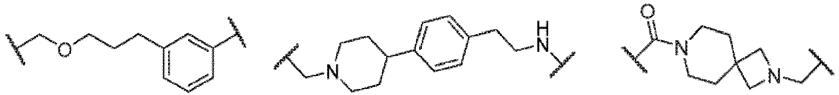

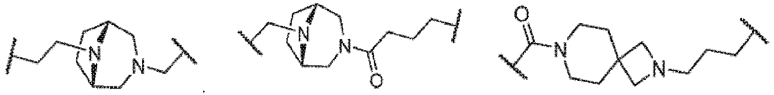

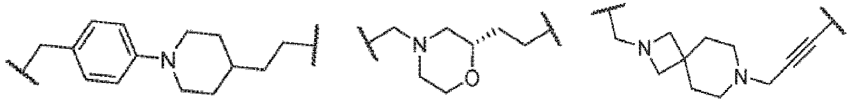

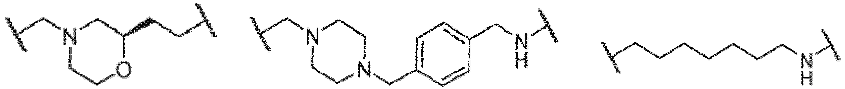

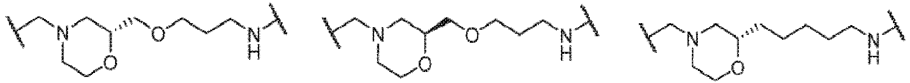

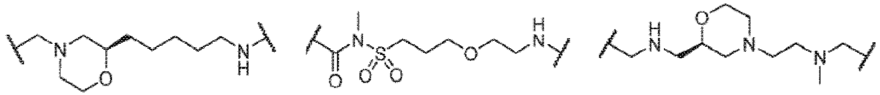

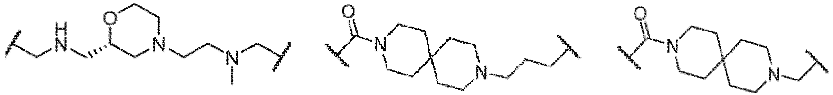

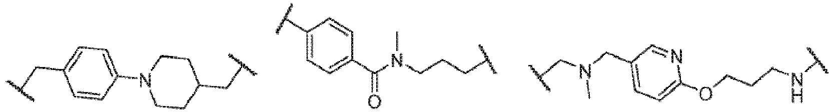

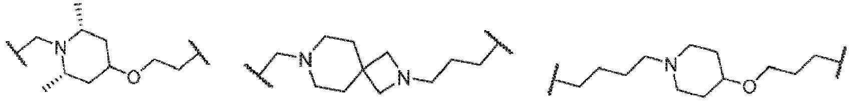

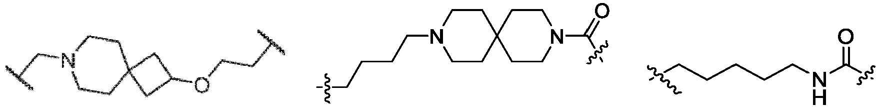

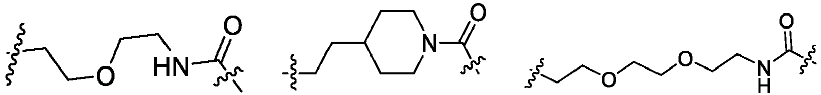

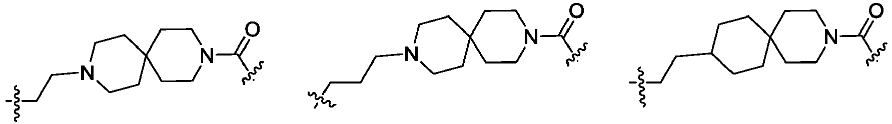

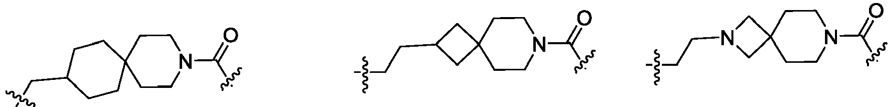

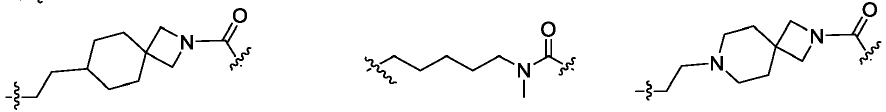

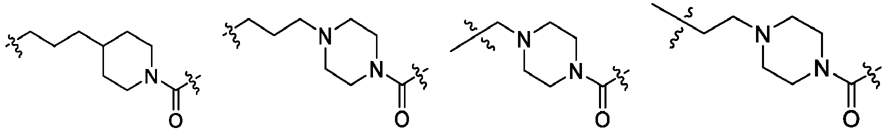

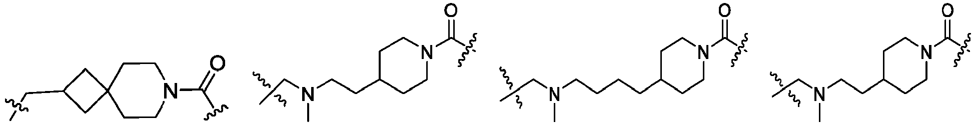

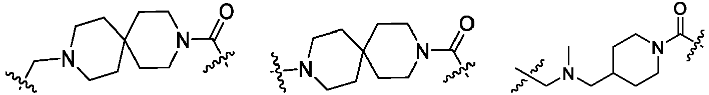

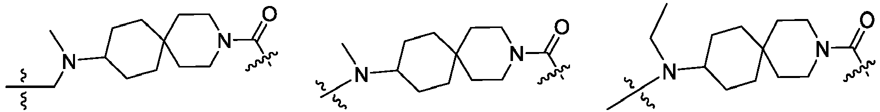

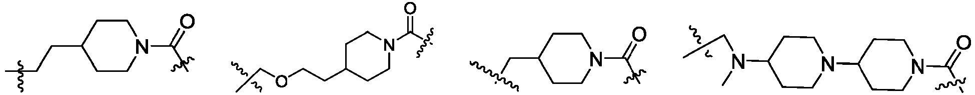

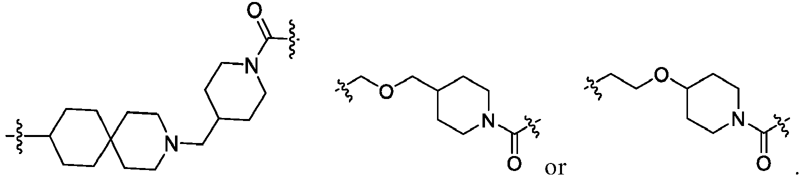

26. The compound of formula I according to claim 21, and/or the stereoisomer, the enantiomer, the diastereomer, the deuterate, the hydrate, the solvate, the prodrug and/or the pharmaceutically acceptable salt thereof, wherein L is LA, 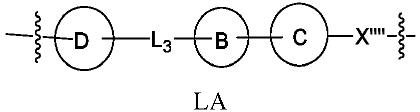 wherein, in LA,
ring D is absent or is C3-C12 cycloalkylene or 3-12 membered heterocycloalkylene containing 1-2 heteroatoms selected from N, O or S, the cycloalkylene and the heterocycloalkylene are optionally substituted by a substituent selected from halogen, oxo, cyano, amino, hydroxyl, C1-C6 alkyl, C1-C6 haloalkyl, C1-C6 hydroxyalkyl or -O-(C1-C6 alkyl);
ring B is absent or is C3-C12 cycloalkylene or 3-12 membered heterocycloalkylene containing 1-2 heteroatoms selected from N, O or S, the cycloalkylene and the heterocycloalkylene are optionally substituted by a substituent selected from halogen, oxo, cyano, amino, hydroxyl, C1-C6 alkyl, C1-C6 haloalkyl, C1-C6 hydroxyalkyl or -O-(C1-C6 alkyl);
ring C is absent, C3-C12 cycloalkylene or 3-12 membered heterocycloalkylene containing 1-2 heteroatoms selected from N, O or S, the cycloalkylene and the heterocycloalkylene are optionally substituted by a substituent selected from halogen, oxo, cyano, amino, hydroxyl, C1-C6 alkyl, C1-C6 haloalkyl, C1-C6 hydroxyalkyl or -O-(C1-C6 alkyl);
Xʺʺ is a bond, -NH-, -NCH₃-, -O-, -C(CH₃)₂-, -S-, -C=C- , -C≡C-, -CHF-, -CHCF₃-, - C(O)-, -S(O)-, -S(O)₂-, -C(O)O-, -OC(O)-, -C(O)NH-, -C(O)NCH₃-, -NHC(O)- or - NCH₃C(O)-;
L₃ is -(CH₂)ₖ, one or two methylenes in L₃ are optionally substituted by a substituent selected from -O-, -NH-, -N(C1-C6 alkyl)-, -N(C1-C6 haloalkyl)-, -N(hydroxyC1-C6 alkyl)-or -N(C3-C8 cycloalkyl)-, k is 0, 1, 2, 3, 4, 5, 6, 7, 8, 9 or 10.

27. The compound of formula I according to claim 21, and/or the stereoisomer, the enantiomer, the diastereomer, the deuterate, the hydrate, the solvate, the prodrug and/or the pharmaceutically acceptable salt thereof, wherein L is LA, 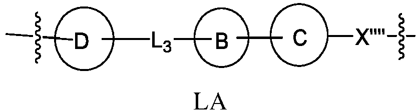 wherein, in LA,
ring D and ring B are absent, ring C is 4-7-membered saturated monocyclic heterocycloalkylene containing 1 or 2 nitrogen heteroatoms, or 7-11-membered spiroheterocycloalkylene containing 1 or 2 nitrogen heteroatoms; Xʺʺ is -C(O)-; L₃ is - (CH₂)ₖ, one methylene in L₃ is optionally replaced by a group selected from -O-, -NH-, - NCH₃- or -NCH₂CH₃-; and k is 2, 3 or 4.

28. The compound of formula I according to claim 21, and/or the stereoisomer, the enantiomer, the diastereomer, the deuterate, the hydrate, the solvate, the prodrug and/or the pharmaceutically acceptable salt thereof, wherein L is

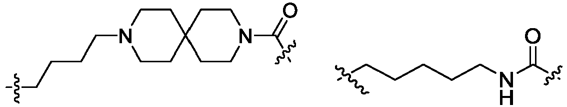

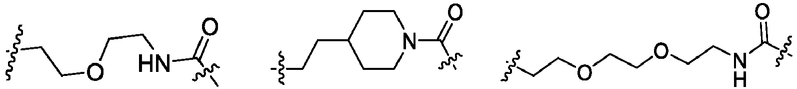

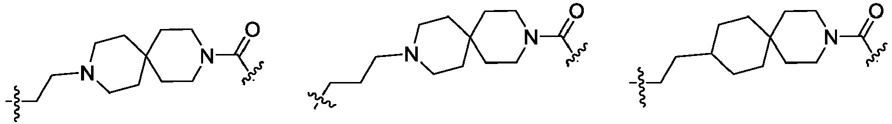

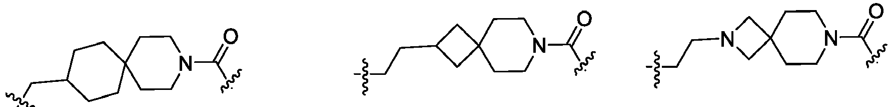

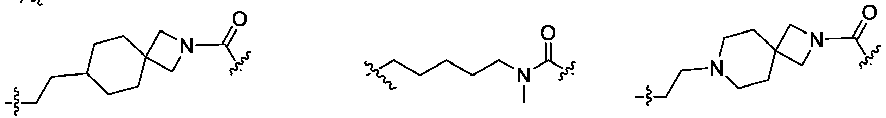

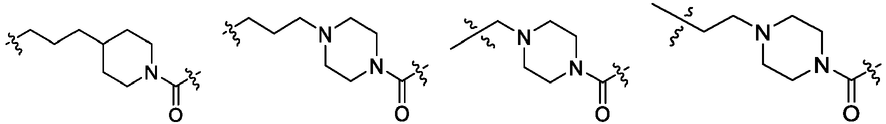

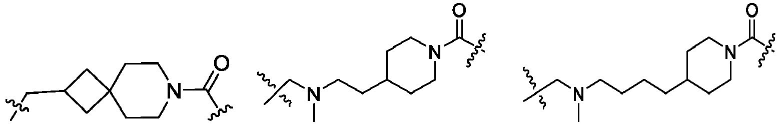

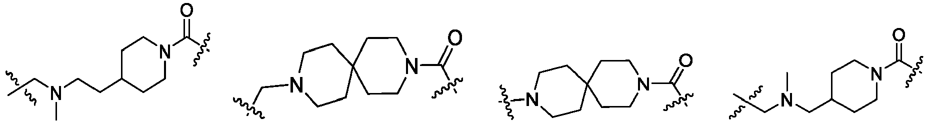

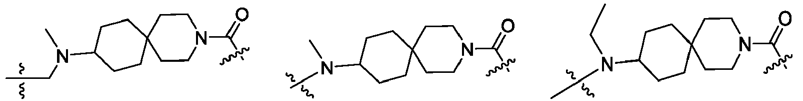

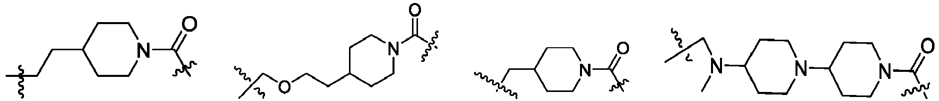

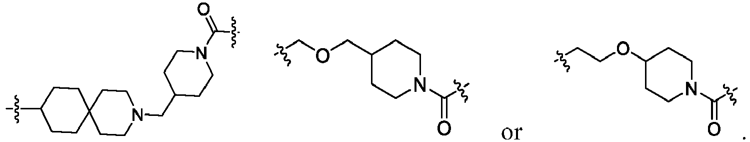

29. The compound of formula I according to claim 1, and/or the stereoisomer, the enantiomer, the diastereomer, the deuterate, the hydrate, the solvate, the prodrug and/or the pharmaceutically acceptable salt thereof, wherein the compound of formula I is

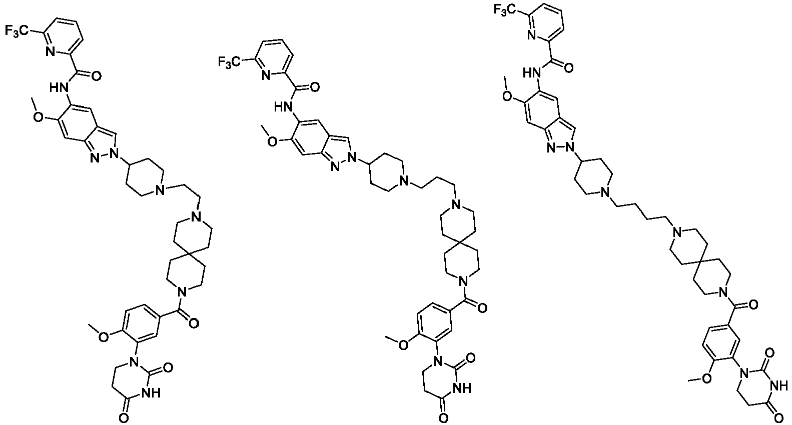

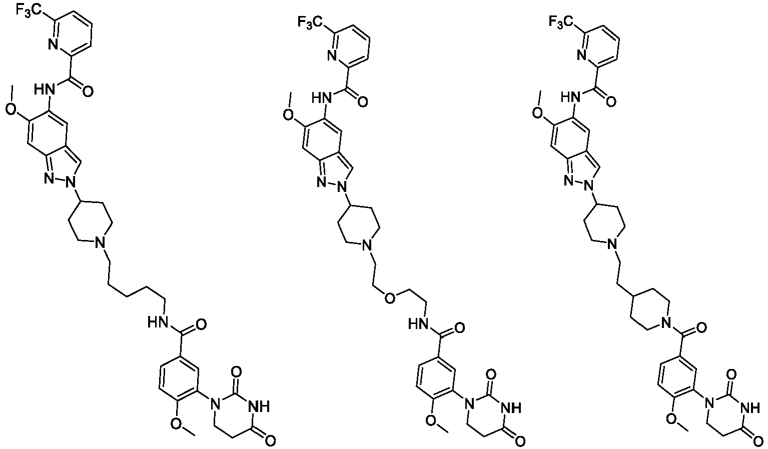

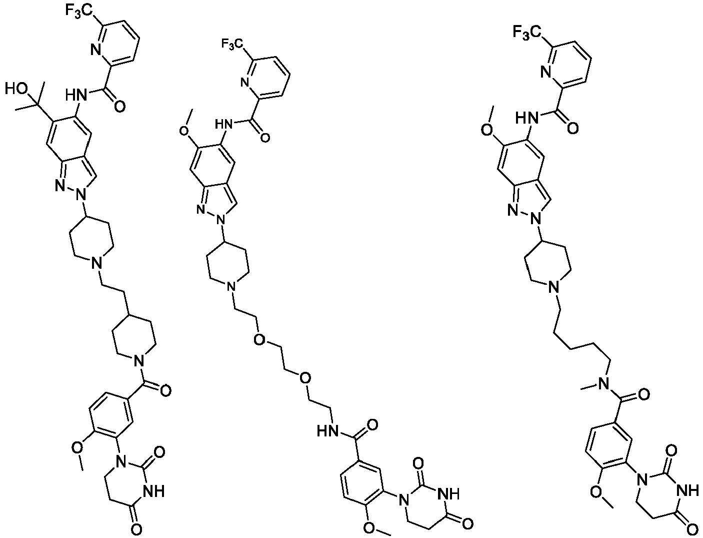

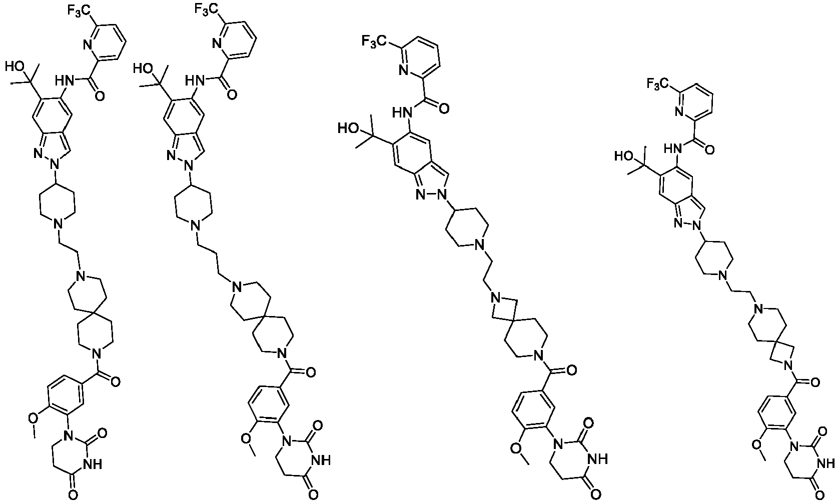

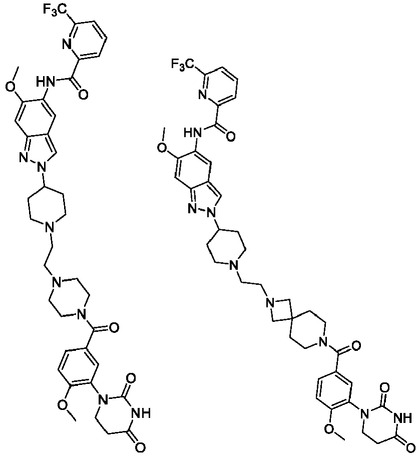

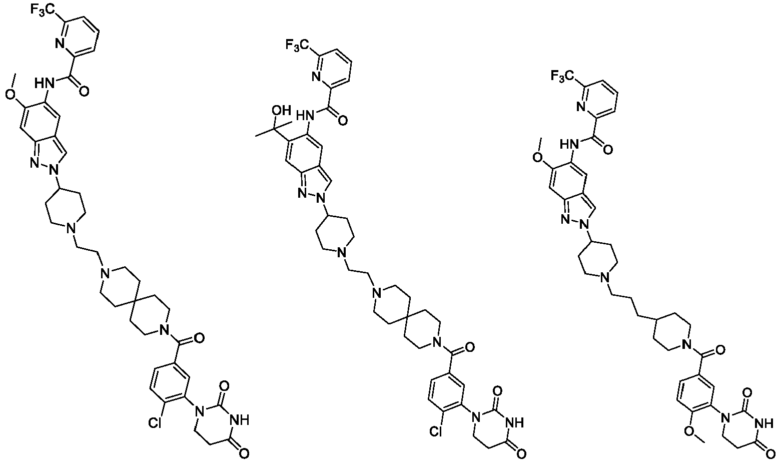

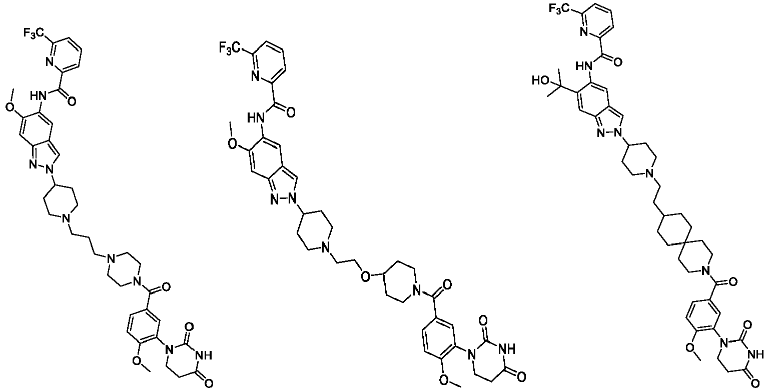

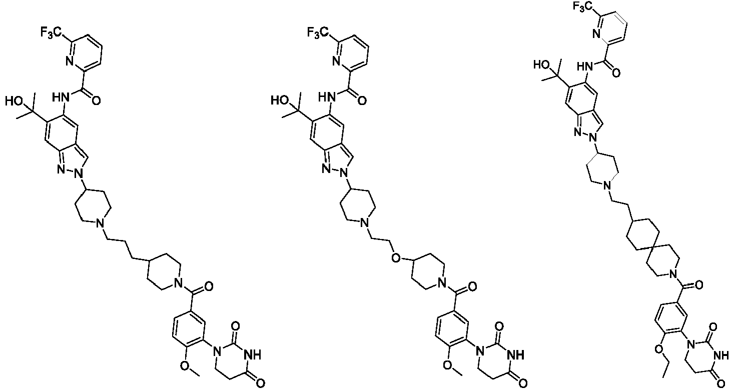

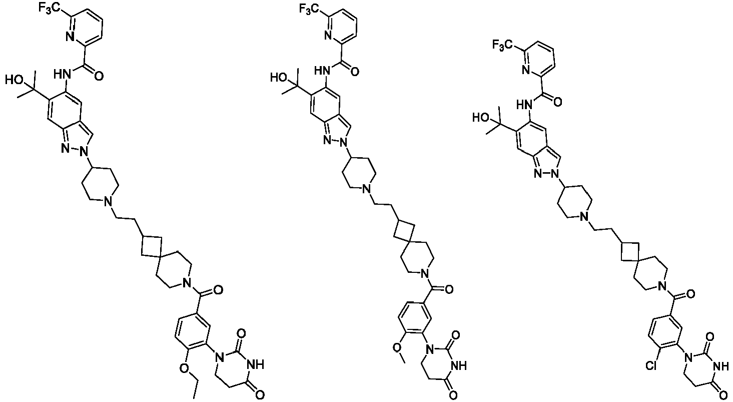

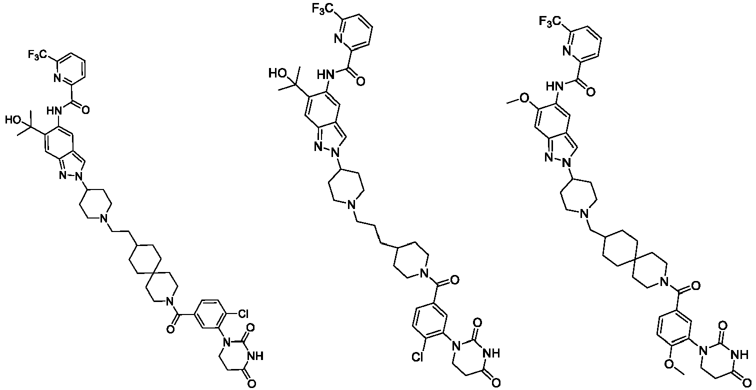

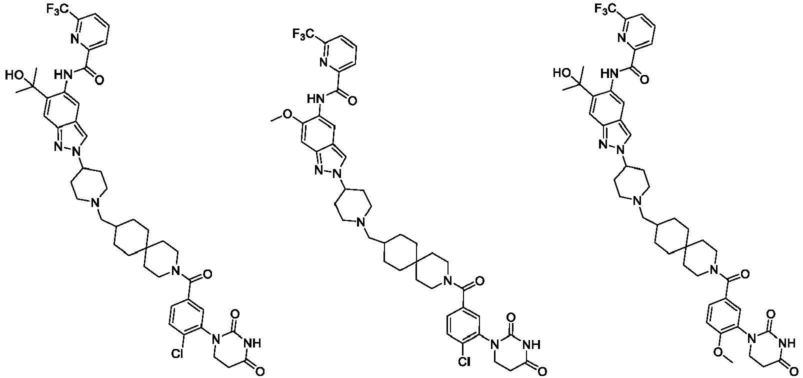

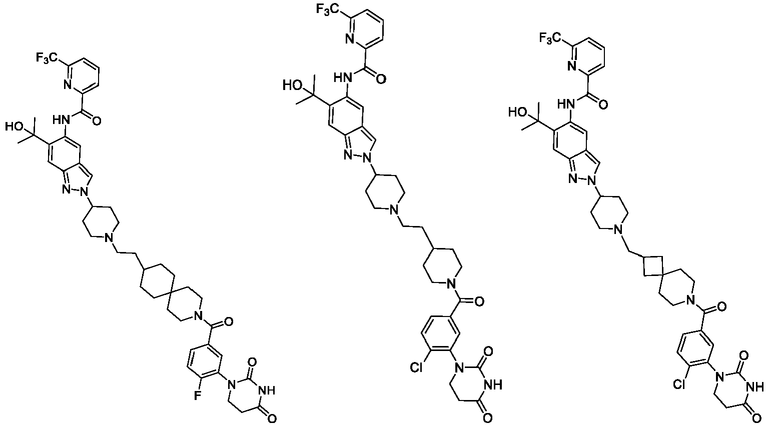

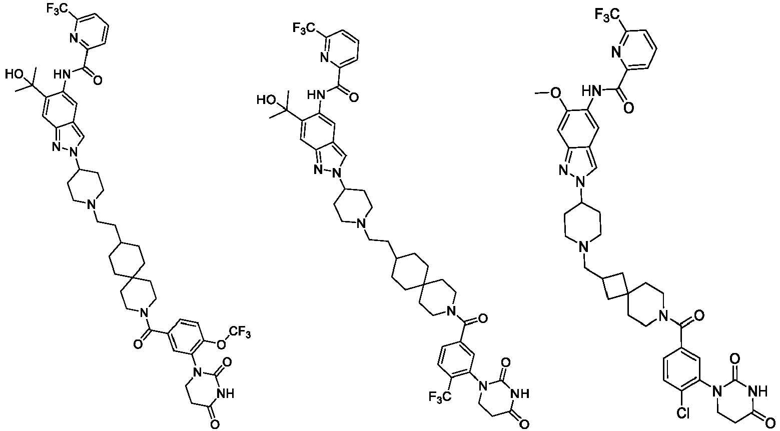

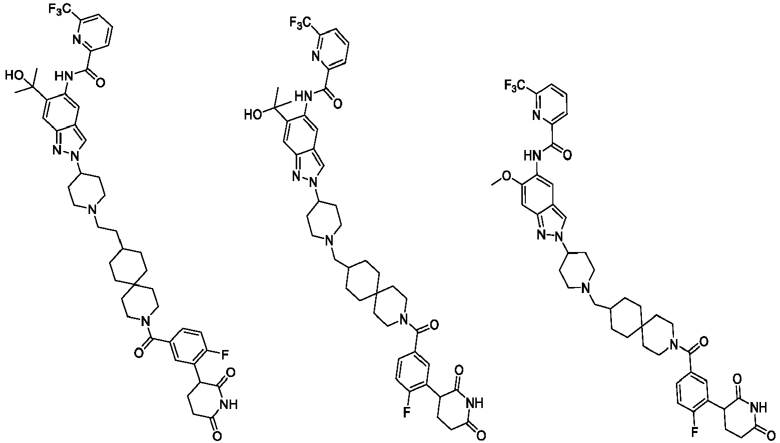

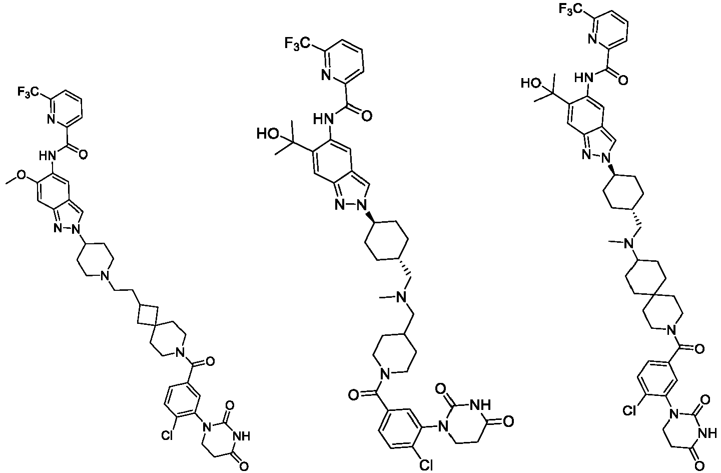

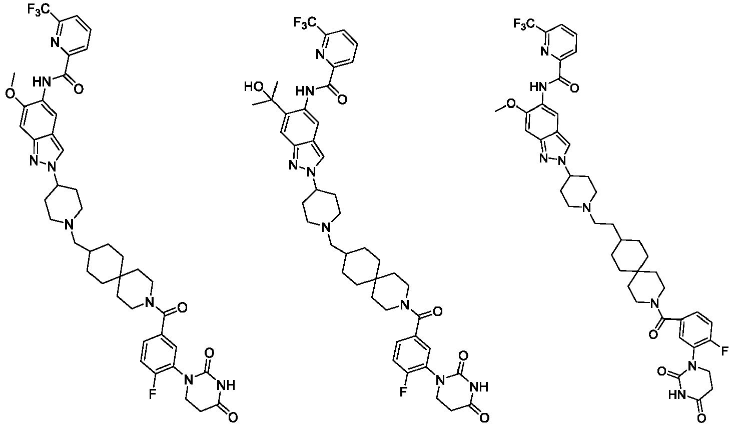

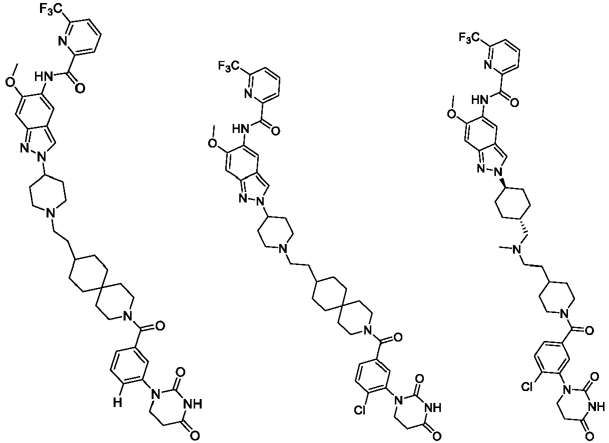

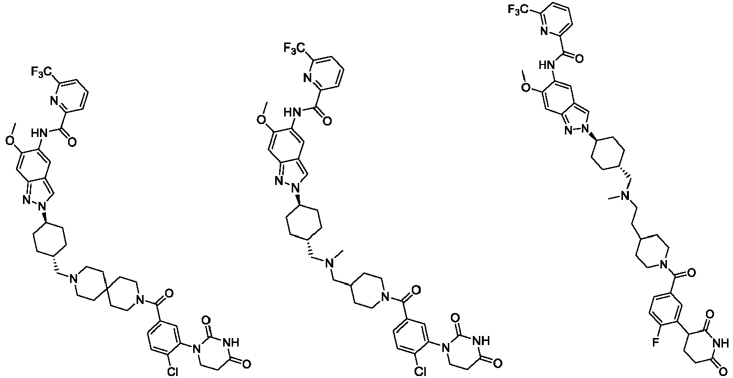

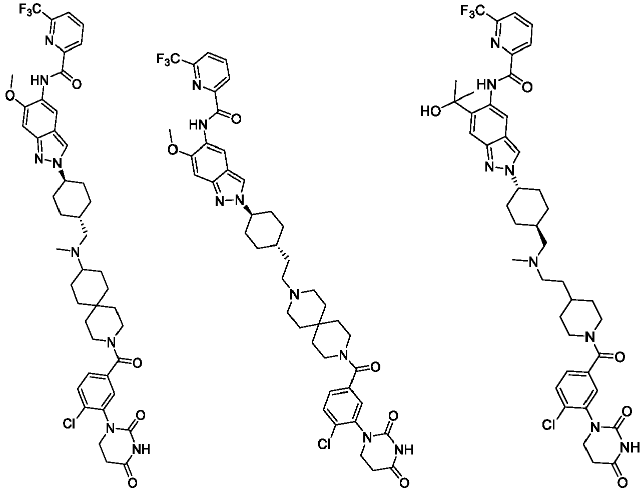

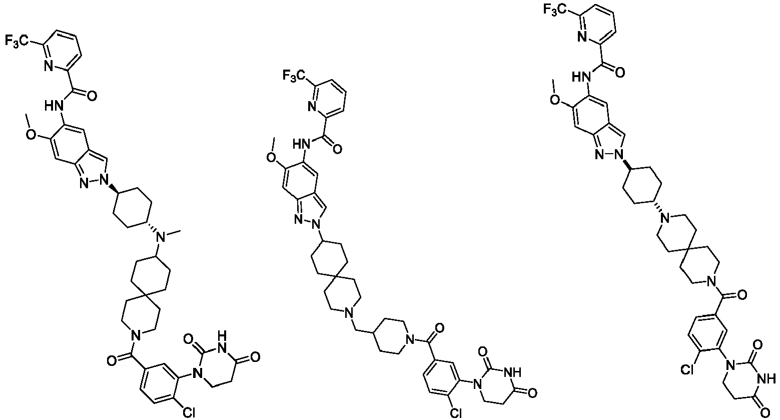

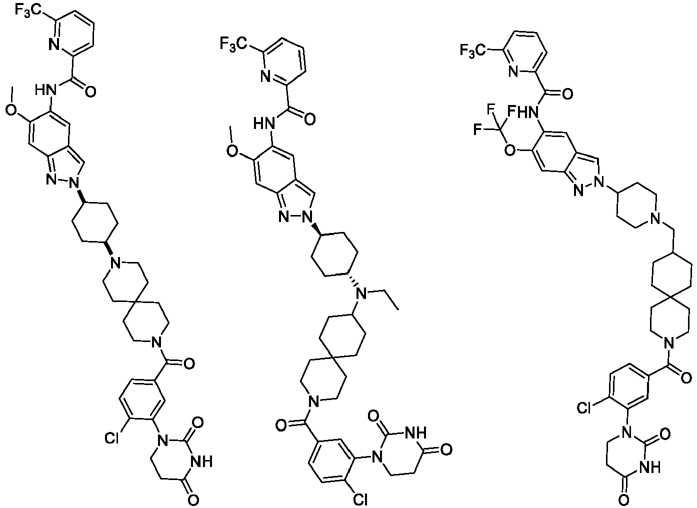

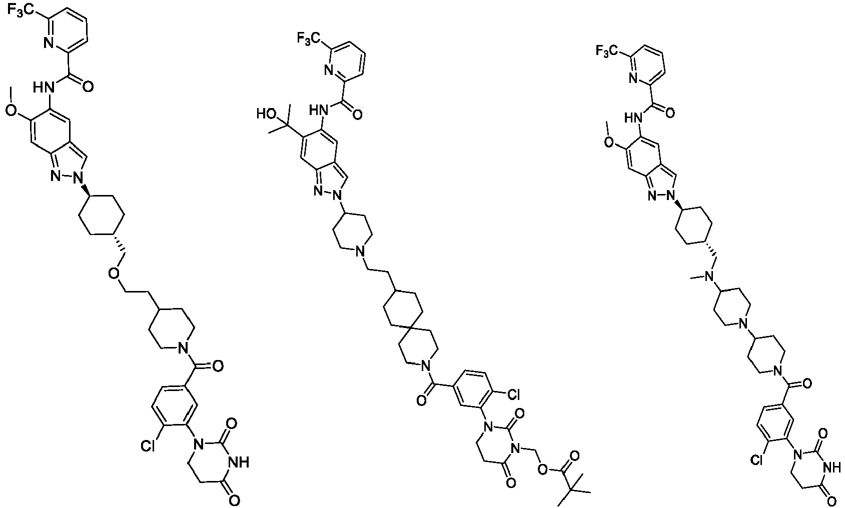

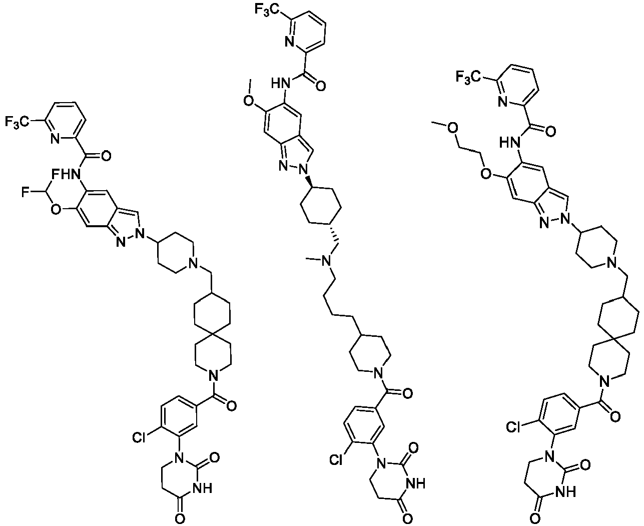

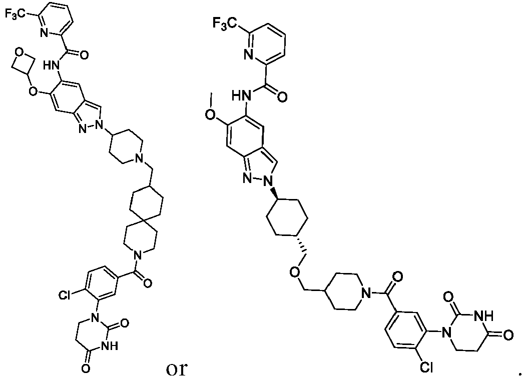

30. A pharmaceutical composition comprising the compound according to any one of claims 1 to 29, and/or the stereoisomer, the enantiomer, the diastereomer, the deuterate, the hydrate, the solvate, the prodrug and/or the pharmaceutically acceptable salt thereof, and a pharmaceutically acceptable carrier, diluent or excipient.

31. Use of the compound according to any one of claims 1 to 29, and/or the stereoisomer, the enantiomer, the diastereomer, the deuterate, the hydrate, the solvate, the prodrug and/or the pharmaceutically acceptable salt thereof in the preparation of drugs for the treatment and/or prevention of IRAK4-mediated diseases or conditions, or TLR (other than TLR3R), IL-1α or IL-1β receptor family mediated diseases or conditions.

32. Use of the compound according to any one of claims 1 to 29, and/or the stereoisomer, the enantiomer, the diastereomer, the deuterate, the hydrate, the solvate, the prodrug and/or the pharmaceutically acceptable salt thereof in the preparation of drugs for the treatment and/or prevention of cancer, neurodegenerative diseases, viral diseases, autoimmune diseases, inflammatory diseases, hereditary diseases, hormone-related diseases, metabolic diseases, organ transplantation-related diseases, immunodeficiency diseases, destructive bone diseases, proliferative disorders, infectious diseases, conditions related to cell death, thrombin-induced platelet aggregation, liver diseases, pathological immune conditions involving T cell activation, cardiovascular diseases or CNS diseases.

33. Use of the compound according to any one of claims 1 to 29, and/or the stereoisomer, the enantiomer, the diastereomer, the deuterate, the hydrate, the solvate, the prodrug and/or the pharmaceutically acceptable salt thereof in the preparation of drugs for the treatment and/or prevention of brain cancer, kidney cancer, liver cancer, adrenal cancer, bladder cancer, breast cancer, gastric cancer, ovarian cancer, colon cancer, rectal cancer, prostate cancer, pancreatic cancer, lung cancer, vaginal cancer, cervical cancer, testicular cancer, genitourinary cancer, esophageal cancer, laryngeal cancer, skin cancer, bone cancer, thyroid cancer, sarcoma, glioblastoma, neuroblastoma, multiple myeloma, gastrointestinal cancer, neck or head tumor, epidermal hyperhyperplasia, bovine skin moss, prostate hyperplasia, Adenoma, adenocarcinoma, keratoacanthoma, epidermoid cancer, large cell carcinoma, non-small cell lung cancer, lymphoma, Hodgkin's and non-Hodgkin's, breast cancer, follicular cancer, undifferentiated tumor, papillary tumor, seminoma, melanoma, ABC DLBCL, Hodgkin's lymphoma, primary cutaneous T-cell lymphoma, chronic lymphocytic leukemia, smoking indolent multiple myeloma, leukemia, diffuse large B - cell lymphoma DLBCL, chronic lymphocytic leukemia CLL, chronic lymphocytic lymphoma, primary exudative lymphoma, Burkitt lymphoma/leukemia, acute lymphocytic leukemia, pre-B cell lymphocytic leukemia, lymphoplasmic lymphoma, Waldenstroms's macroglobulinemia WM, splenic marginal zone lymphoma, multiple myeloma, or plasmacytoma or intravascular large B- cell lymphoma, Alzheimer's disease, Parkinson's disease, amyotrophic lateral sclerosis, Huntington's disease, cerebral ischemia or traumatic injury, glutamate neurotoxicity, hypoxia, epilepsy, diabetes treatment, metabolic syndrome, obesity, neurodegenerative diseases caused by organ transplantation or graft-versus-host disease, eye disease, such as eye allergy, conjunctivitis, dry eye or spring conjunctivitis, diseases affecting the nose, including allergic rhinitis; autoimmune hematological diseases, such as hemolytic anemia, aplastic anemia, pure red blood cell anemia and idiopathic thrombocytopenia, systemic lupus erythematosus, rheumatoid arthritis, polychondritis, scleroderma, dermatomyositis, polymyositis, chronic active hepatitis, myasthenia gravis, Stephen-Johnson syndrome, idiopathic stomatitis diarrhea, autoimmune inflammatory bowel disease, bowel syndrome, celiac disease, root periostitis, lung hyaline membrane disease, nephropathy, glomerular disease, Alcoholic liver disease, multiple sclerosis, endocrine ophthalmopathy, Grave's disease, Sarcomatosis, dry eye, spring conjunctival keratitis, interstitial pulmonary fibrosis, bovine moss arthritis, systemic juvenile idiopathic arthritis, nephritis, vasculitis, interstitial cystitis, diverticulitis, Glomerulonephritis, chronic granulomatous disease, endometriosis, leptospirosis nephropathy, glaucoma, retinal disease, aging, headache, pain, complex regional pain syndrome, cardiac hypertrophy, muscle atrophy, catabolism, obesity, slow fetal growth, hypercholesterolemia, heart disease, chronic heart failure, mesothelioma, anhidromic ectodermal dysplasia, Behcet's disease, pigment incontinence, Paget's disease, pancreatitis, hereditary periodic fever syndrome, asthma, acute lung injury, acute respiratory distress syndrome, eosinophilia, allergic reaction, systemic allergic reaction, sinusitis, eye allergy, silica-induced diseases, COPD, lung disease, cystic fibrosis, acid-induced lung injury, pulmonary hypertension, polyneuropathy, Cataract, muscle inflammation combined with systemic sclerosis, inclusion body myositis, myasthenia gravis, thyroiditis, Addison's disease, lichen planus, type 1 diabetes, type 2 diabetes, appendicitis, atopic dermatitis, asthma, allergies, bronchiolitis, bronchitis, bursitis, cervicitis, cholangitis, cholecystitis, chronic transplant rejection, colitis, conjunctivitis, cystitis, lacrimal gland inflammation, dermatitis, dermatomyositis, polymyositis, encephalitis, endocarditis, endometritis, enteritis, enterocolitis, upper ankle inflammation, epididymitis, fasciitis, fibrous tissue inflammation, gastritis, gastroenteritis, Henoch-Schonlein purpura, hepatitis, suppurative sweat Inflammation, immunoglobulin A nephropathy, interstitial lung disease, laryngitis, mastitis, meningitis, myelitis, myocarditis, myositis, nephritis, liver fibrosis, renal fibrosis, alcoholic fatty liver, non-alcoholic fatty liver, heart fibrosis, psoriasis, Crohn's disease, inflammatory bowel disease, oophoritis, orchitis, osteitis, otitis, pancreatitis, mumps, pericarditis, peritonitis, pharyngitis, pleurisy, phlebitis, local pneumonia, pneumonia, polymyositis, proctitis, prostatitis, pyelonephritis, rhinitis, salpingitis, sinusitis, stomatitis, articular inflammation, tendinitis, tonsillitis, ulcerative colitis, uveitis, vaginitis, vasculitis, vulvitis, alopecia areata, erythema multiforme, dermatitis herpetiformis, sclerosis, vitiligo, hypersensitivity vasculitis, urticaria, bullous pemphigoid, pemphigus vulgaris, deciduous pemphigus, paraneoplastic pemphigus, acquired bullous epidermal laxity, acute and chronic gout, chronic gouty arthritis, bovine skin moss, bovine skin arthritis, rheumatoid arthritis, juvenile rheumatoid arthritis, cryopyrin-associated periodic syndrome or osteoarthritis diseases.
